# EUROPEAN PATENT APPLICATION

(11) **EP 3 808 760 A1**
(43) Date of publication of application: **21.04.2021**
(21) Application number: 19818738.7
(22) Date of filing: 14.06.2019
(51) Int. Cl.: C07K 7/08, A61K 47/68, A61P 35/00, C07K 7/06, C07K 7/50, C07K 16/00, C12P 21/08

(54) **COMPOUND COMPRISING SUBSTANCE HAVING AFFINITY FOR ANTIBODY, CLEAVAGE SITE AND REACTIVE GROUP, OR SALT THEREOF**

(30) Priority: 14.06.2018 JP 2018113953; 31.10.2018 JP 2018205446
(71) Applicant: AJINOMOTO CO., INC., Chuo-ku Tokyo 104-8315 (JP)
(72) Inventor: YAMADA, Kei, Kawasaki-shi, Kanagawa 210-8681 (JP); FUJII, Tomohiro, Kawasaki-shi, Kanagawa 210-8681 (JP); SHIKIDA, Natsuki, Kawasaki-shi, Kanagawa 210-8681 (JP); SHIMBO, Kazutaka, Kawasaki-shi, Kanagawa 210-8681 (JP)
(74) Representative: Mewburn Ellis LLP
(86) International application number: PCT/JP2019/023778
(87) International publication number: WO 2019/240287

(57) **Abstract**

The present invention provides a technique enabling modification of an antibody, particularly regioselective modification of an antibody. More specifically, the present invention provides a compound having an affinity substance to an antibody, a cleavable portion, and a reactive group, represented by the following Formula (I):

A-L-B-R (I)

wherein
A is the affinity substance an antibody,
L is a cleavable linker which is a divalent group comprising the cleavable portion,
B is (a) a divalent group comprising a bioorthogonal functional group or (b) a divalent group comprising no bioorthogonal functional group, and
R is the reactive group to the antibody, in which
the affinity substance to an antibody is a certain peptide, or a salt thereof.

## Description

### TECHNICAL FIELD

The present invention relates to a compound having an affinity substance to an antibody, a cleavable portion, and a reactive group, or a salt thereof, and the like.

### BACKGROUND ART

In recent years, research and development of an antibody-drug conjugate (ADC) have been actively conducted. An ADC, as implied by the name, is a medicine in which a drug (e.g., an anti-cancer agent) is conjugated with an antibody and has a direct cytotoxic activity on cancer cells and the like. A typical ADC is T-DM1 (trade name: Kadcyla (registered trademark)) (Non-Patent Literatures 1 to 3).

ADCs including T-DM1 have had the problem of their nonuniformity from the beginning of their development. That is, a small compound drug is randomly reacted with about 70 to 80 Lys residues in an antibody, and thus a drug/antibody ratio (DAR) and a conjugation position are not constant. It is known that such a random conjugation method normally provides a DAR within a range of 0 to 8, producing a plurality of medicines having different numbers of bonds of a drug. In recent years, it has been reported that when the number of bonds and the bond positions of a drug of an ADC are changed, pharmacokinetics, and a releasing rate and effects of the drug change. Given these circumstances, next-generation ADCs are required to control the number and positions of a drug to be conjugated. It is believed that when the number and positions are fixed, the problems of expected efficacy, variations in conjugation medicines, and lot difference, or what is called regulation, will be solved (Non-Patent Literature 4).

Although methods for regioselectively modifying antibodies are being investigated worldwide, most of them are methods of modification using genetic engineering techniques or enzymes. For the genetic engineering methods of modification, problems have been pointed out such as reductions in the expression efficiency of antibodies themselves (reductions in total yield when ADCs are prepared), although regioselectivity and number selectivity can be controlled. In addition, there is a problem in that it takes long years to construct an antibody expression system and the like (Non-Patent Literatures 5 to 7).

In recent years, methods that chemically modify proteins under complicated environments such as intracellular ones using a small molecule probe have been reported. The methods are used for imaging or identification of receptors in repositioning small compound drugs. In the field of chemical biology, organic chemical methods of protein modification using a synthesized small molecule probe are attracting attention (Non-Patent Literature 8 to 10).

A chemical conjugation by affinity peptide (CCAP) method has recently been developed. This method has succeeded in regioselective modification of antibodies by a method that reacts a peptide reagent in which an NHS-activated ester and a drug are coupled with an affinity peptide with an antibody (that is, a method for producing an ADC through a linker comprising a peptide portion). This method has succeeded in regioselectively modifying an antibody Fc region with a drug by a chemical synthetic technique first in the world, and besides, practically favorable results [reaction time: 30 minutes, yield: 70% (for DAR 1), and regioselectivity: 100%] have been determined. It has been demonstrated that control with a DAR of 2 can be achieved by adding about five equivalents of the peptide reagent, which is epoch-making in that a modified position can also be controlled (Patent Literature 1) .

### PRIOR ART REFERENCES

### Patent Literature

Patent Literature 1: WO 2016/186206

### Non-Patent Literature

Non-Patent Literature 1: Reichert JM et al., Nat Biotechnol 2005; 23: 1073-8
Non-Patent Literature 2: Kubota T et al., Cancer Sci 2009; 100: 1566-72
Non-Patent Literature 3: Wu AM et al., Nat Biotechnol 2005; 23: 1137-46
Non-Patent Literature 4: Junutula JR et al., Nat Biotechnol 2008; 26: 925-32
Non-Patent Literature 5: Shen BQ et al., Nat Biotechnol 2012; 30: 184-9
Non-Patent Literature 6: Hofer T et al., Biochemistry 2009; 48: 12047-57
Non-Patent Literature 7: Liu W et al., Nat Methods 2007; 4: 239-44
Non-Patent Literature 8: S. T. Laughlin et al., Science 2008; 320,664
Non-Patent Literature 9: A. E. Speers et al., ChemBioChem 2004; 5, 41
Non-Patent Literature 10: Y. Takaoka et al., Angew. Chem. Int. Ed. 2013; 52, 4088

### DISCLOSURE OF INVENTION

### PROBLEM TO BE SOLVED BY THE INVENTION

The object of the present invention is to develop a technique enabling modification of an antibody, particularly regioselective modification of an antibody.

### MEANS FOR SOLVING PROBLEM

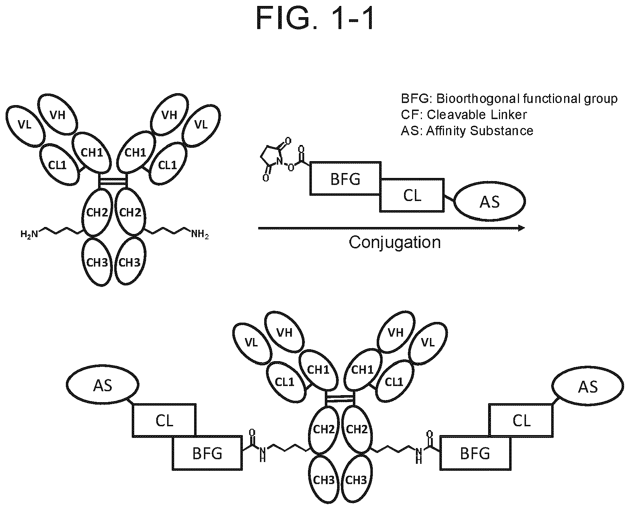

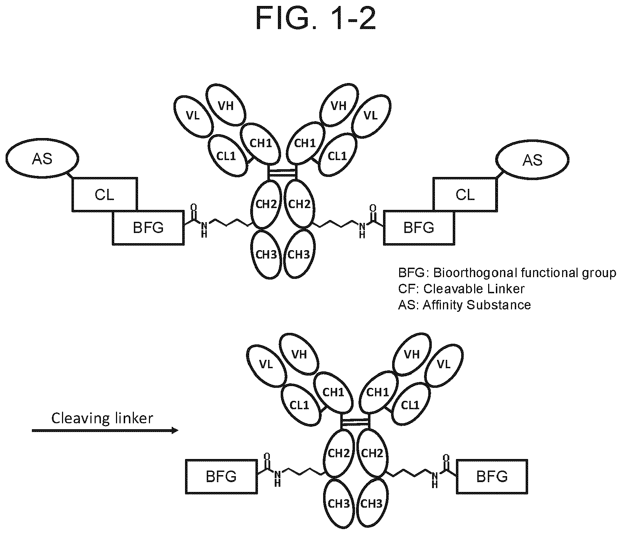

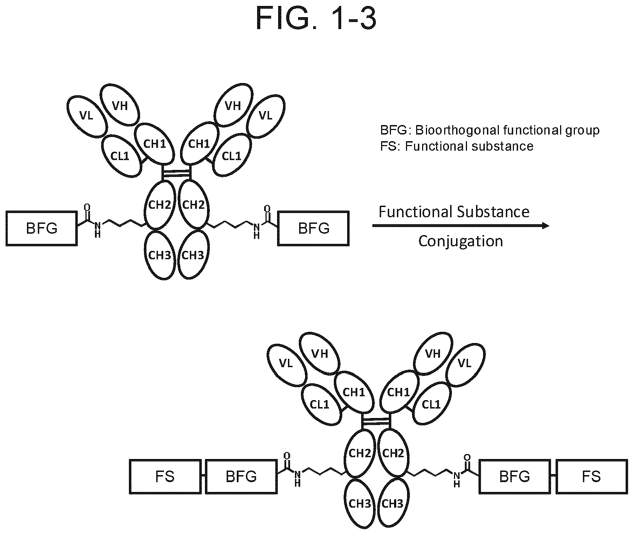

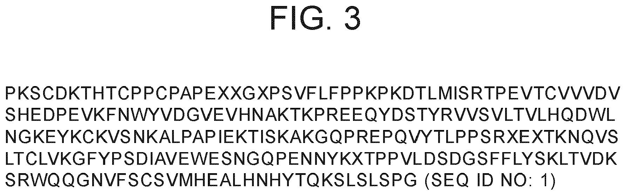

Through dedicated study, the inventors of the present invention have found out that a compound developed based on a novel and original design concept having a structural feature comprising (1) an affinity substance to an antibody, (2) a reactive group to an amino acid residue constituting the antibody, and (3) a cleavable portion between the affinity substance and the reactive group, and capable of producing (4) a structural unit having a bioorthogonal functional group or bioorthogonal functional groups on a reactive group side (that is, a structural unit comprising a bioorthogonal functional group and a reactive group) by cleavage at the cleavable portion is useful for regiospecific modification of an antibody (e.g., FIGS. 1-1, 1-2, 1-3, and 2). The inventors of the present invention have also found out that using such a compound can prepare an antibody regioselectively having a functional substance or functional substances (e.g., a drug) and comprising no peptide portion as a linker (e.g., an antibody drug conjugate (ADC)). Avoidance of use of a linker comprising a peptide portion, which has potential immunogenicity and is easily hydrolyzed in the blood, is desirable in the clinical application of ADC. That is, it can be said that the method developed by the inventors of the present invention has succeeded first in the world in regioselectively modifying an antibody Fc region with a drug by a chemical synthetic technique, and besides, without using any linker comprising a peptide portion. The inventors of the present invention have also succeeded in developing various compounds having the above (1) to (4) structural features (e.g., FIGS. 1-1, 1-2, 1-3, and 2) to complete the present invention.

Specifically, the present invention is as follows.

In a first embodiment, the present invention provides a compound having an affinity substance to an antibody, a cleavable portion, and a reactive group, or a salt thereof, and a reagent of regioselectively modifying an antibody, comprising the compound or salt thereof.
[1] A compound having an affinity substance to an antibody, a cleavable portion, and a reactive group, or a salt thereof, the compound being represented by the following formula (I):

   A-L-B-R (I)

   wherein
   A is the affinity substance an antibody,
   L is a cleavable linker which is a divalent group comprising the cleavable portion,
   B is (a) a divalent group comprising a bioorthogonal functional group or (b) a divalent group comprising no bioorthogonal functional group, and
   R is the reactive group to the antibody, wherein
   the affinity substance to an antibody is a peptide comprising any of the following amino acid sequences of
   Formula 1-1: (X₀₋₃)ₐ-C-Xaa1-Xaa2-Xaa3-Xaa4-Xaa5-Xaa6-I-I-W-C- (X₀₋₃)_{b}(SEQ ID NO: 58)
   Formula 1-2: (X₀₋₃)ₐ-C-Xaa1-Xaa2-Xaa3-Xaa4-Xaa5-Xaa6-I-V-W-C-(X₀₋₃)_{b} (SEQ ID NO: 59)
   Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
   Formula 1-4: (X₀₋₃)ₐ-C-Xaa1-Xaa2-Xaa3-Xaa4-Xaa5-Xaa6-A-V-W-C- (X₀₋₃)_{b}(SEQ ID NO: 61)
   Formula 1-5: (X₀₋₃)ₐ-C-Xaa1-Xaa2-Xaa3-Xaa4-Xaa5-Xaa6-L-L-W-C- (X₀₋₃)_{b}(SEQ ID NO: 62)
   Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
   Formula 1-7: (X₀₋₃)ₐ-C-Xaa1-Xaa2-Xaa3-Xaa4-Xaa5-Xaa6-L-V-F-C- (X₀₋₃)_{b}(SEQ ID NO: 64)
   Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
   Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
   wherein
   (X₀₋₃)ₐ is absent or one to three consecutive arbitrary amino acid residues (other than a lysine residue and a cysteine residue) which are the same or different,
   (X₀₋₃)_{b} is absent or one to three consecutive arbitrary amino acid residues (other than a lysine residue and a cysteine residue) which are the same or different,
   Xaa1 is an alanine residue, a glycine residue, a leucine residue, a proline residue, an arginine residue, a valine residue, an asparagine residue, a glutamic acid residue, or a phenylalanine residue,
   Xaa2 is a tyrosine residue, a tryptophan residue, a histidine residue, or a phenylalanine residue,
   Xaa3 is a histidine residue, a phenylalanine residue, a tyrosine residue, a tryptophan residue, an arginine residue, or a glycine residue,
   Xaa4 is a lysine residue,
   Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
   Xaa6 is a glutamine residue, a glutamic acid residue, an asparagine residue, an aspartic acid residue, a proline residue, a glycine residue, an arginine residue, a phenylalanine residue, or a histidine residue, and
   Formula 2-1: (X₀₋₃')ₐ-C-Xaa1'-Xaa2'-Xaa3'-Xaa4'-Xaa5'-Xaa6'-L-V-W-C-(X₀₋₃')_{b} (SEQ ID NO: 67)
   wherein
   (X₀₋₃')ₐ and (X₀₋₃')_{b} are the same as the above (X₀₋₃)ₐ and (X₀₋₃)_{b}, respectively, and
   Xaa1', Xaa2', Xaa3', Xaa4', Xaa5', and Xaa6' are the same as the above Xaa1, Xaa2, Xaa3, Xaa4, Xaa5, and Xaa6, respectively,
   except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.
[2] The compound or salt thereof according to [1], wherein
   (X₀₋₃)ₐ is absent, an arginine residue-glycine residue-asparagine residue, an aspartic acid residue, or an asparagine residue,
   (X₀₋₃)_{b} is absent, a threonine residue-tyrosine residue-histidine residue, or a threonine residue,
   Xaa1 is an alanine residue,
   Xaa2 is a tyrosine residue, a tryptophan residue, or a histidine residue, and
   Xaa6 is a glutamine residue, a glutamic acid residue, an asparagine residue, or an aspartic acid residue.
[3] The compound or salt thereof according to [1], wherein
   (X₀₋₃)ₐ is a glycine residue-asparagine residue, a glycine residue, a glycine residue-glycine residue, or a glycine residue-glycine residue-glycine residue,
   (X₀₋₃)_{b} is a threonine residue-tyrosine residue, a glycine residue, a glycine residue-glycine residue, or a glycine residue-glycine residue-glycine residue,
   Xaa1 is a glycine residue, a leucine residue, a proline residue, an arginine residue, a valine residue, an asparagine residue, a glutamic acid residue, or a phenylalanine residue,
   Xaa2 is a phenylalanine residue, and
   Xaa6 is a proline residue, a glycine residue, an arginine residue, a phenylalanine residue, or a histidine residue.
[4] The compound or salt thereof according to any one of [1] to [3], wherein the affinity substance to an antibody is a peptide comprising an amino acid sequence selected from the group consisting of the following:
   (1) RGNCAYHKGQIIWCTYH (SEQ ID NO: 5);
   (2) RGNCAYHKGQIVWCTYH (SEQ ID NO: 8);
   (3) RGNCAYHKGQVVWCTYH (SEQ ID NO: 9);
   (4)RGNCAYHKGQAVWCTYH(SEQ ID NO: 10);
   (5)RGNCAYHKGQLLWCTYH(SEQ ID NO: 11);
   (6)RGNCAYHKGQLIWCTYH(SEQ ID NO: 12);
   (7) DCAYHKGQIVWCT(SEQ ID NO: 13);
   (8) DCAYHKGQVVWCT (SEQ ID NO: 14);
   (9) DCAYHKGQAVWCT (SEQ ID NO: 15);
   (10) RGNCAYHKSQIIWCTYH (SEQ ID NO: 16);
   (11) RGNCAYHKNQIIWCTYH (SEQ ID NO: 17);
   (12)RGNCAYHKDQIIWCTYH(SEQ ID NO: 18);
   (13)RGNCAYHKQQIIWCTYH(SEQ ID NO: 19);
   (14)RGNCAYHKEQIIWCTYH(SEQ ID NO: 20);
   (15)RGNCAYHKFQIIWCTYH(SEQ ID NO: 21);
   (16)RGNCAYHKYQIIWCTYH(SEQ ID NO: 22);
   (17)RGNCAYHKWQIIWCTYH(SEQ ID NO: 23);
   (18)RGNCAYHKHQIIWCTYH(SEQ ID NO: 24);
   (19)RGNCAYHKTQIIWCTYH(SEQ ID NO: 25);
   (20)RGNCAYHKLQIIWCTYH(SEQ ID NO: 26);
   (21)CAYHKLQIVWC(SEQ ID NO: 27);
   (22)CAYHKLQLIWC(SEQ ID NO: 28);
   (23)CAYHKSQIVWC(SEQ ID NO: 29);
   (24)RGNCAYHKGQLVFCTYH(SEQ ID NO: 30);
   (25)RGNCAYHKGQQVWCTYH(SEQ ID NO: 31);
   (26)RGNCAYHKGQEVWCTYH(SEQ ID NO: 32);
   (27)CAYHKGQLVWC(SEQ ID NO: 33);
   (28)RGNCAYHKAQLVWCTYH(SEQ ID NO: 34);
   (29)RGNCAYHKVQLVWCTYH(SEQ ID NO: 35);
   (30)RGNCAYHKLQLVWCTYH(SEQ ID NO: 36);
   (31)RGNCAYHKIQLVWCTYH(SEQ ID NO: 37);
   (32)RGNCAYHKSQLVWCTYH(SEQ ID NO: 38);
   (33)RGNCAYHKTQLVWCTYH(SEQ ID NO: 39);
   (34)RGNCAYHKNQLVWCTYH(SEQ ID NO: 40);
   (35)RGNCAYHKDQLVWCTYH(SEQ ID NO: 41);
   (36)RGNCAYHKQQLVWCTYH(SEQ ID NO: 42);
   (37)RGNCAYHKEQLVWCTYH(SEQ ID NO: 43);
   (38)RGNCAYHKFQLVWCTYH(SEQ ID NO: 44);
   (39)RGNCAYHKRQLVWCTYH(SEQ ID NO: 45);
   (40)RGNCAYHKHQLVWCTYH(SEQ ID NO: 46);
   (41)RGNCAYHKWQLVWCTYH(SEQ ID NO: 47);
   (42)RGNCAYHKYQLVWCTYH(SEQ ID NO: 48);
   (43)RGNCAYFKGQLVWCTYH(SEQ ID NO: 49);
   (44)RGNCAYYKGQLVWCTYH(SEQ ID NO: 50);
   (45)RGNCAYWKGQLVWCTYH(SEQ ID NO: 51);
   (46)RGNCAYRKGQLVWCTYH(SEQ ID NO: 52);
   (47)RGNCAYGKGQLVWCTYH(SEQ ID NO: 53);
   (48)DCAYHKGQLVWC(SEQ ID NO: 54);
   (49)NCAYHKGQLVWC(SEQ ID NO: 55);
   (50)CAYHKGQLVWCT(SEQ ID NO: 56);
   (51)CAYHKSQLVWC(SEQ ID NO: 57);
   (52)RGNCAWHKGQIIWCTYH(SEQ ID NO: 68);
   (53)RGNCAFHKGQIIWCTYH(SEQ ID NO: 69);
   (54)RGNCAHHKGQIIWCTYH(SEQ ID NO: 70);
   (55)RGNCGYHKGQIIWCTYH(SEQ ID NO: 71);
   (56)RGNCLYHKGQIIWCTYH(SEQ ID NO: 72);
   (57)RGNCPYHKGQIIWCTYH(SEQ ID NO: 73);
   (58)RGNCRYHKGQIIWCTYH(SEQ ID NO: 74);
   (59)RGNCVYHKGQIIWCTYH(SEQ ID NO: 75);
   (60)RGNCNYHKGQIIWCTYH(SEQ ID NO: 76);
   (61)RGNCEYHKGQIIWCTYH(SEQ ID NO: 77);
   (62)RGNCFYHKGQIIWCTYH(SEQ ID NO: 78);
   (63)RGNCAYHKGEIIWCTYH(SEQ ID NO: 79);
   (64)RGNCAYHKGNIIWCTYH(SEQ ID NO: 80);
   (65)RGNCAYHKGPIIWCTYH(SEQ ID NO: 81);
   (66)RGNCAYHKGGIIWCTYH(SEQ ID NO: 82);
   (67)RGNCAYHKGDIIWCTYH(SEQ ID NO: 83);
   (68)RGNCAYHKGRIIWCTYH(SEQ ID NO: 84);
   (69)RGNCAYHKGFIIWCTYH(SEQ ID NO: 85);
   (70)RGNCAYHKGHIIWCTYH(SEQ ID NO: 86);
   (71)DCAYHKGQIIWCT(SEQ ID NO: 87);
   (72)NCAYHKGQIIWCT(SEQ ID NO: 88);
   (73)GNCAYHKGQIIWCTY(SEQ ID NO: 89);
   (74)GCAYHKGQIIWCG(SEQ ID NO: 90);
   (75)GGCAYHKGQIIWCGG(SEQ ID NO: 91); and
   (76) GGGCAYHKGQIIWCGGG (SEQ ID NO: 92).
[5] The compound or salt thereof according to any one of [1] to [4], wherein L is (i) a cleavable linker which is a divalent group comprising a cleavable portion having an ability to form a bioorthogonal functional group on a reactive group side by cleavage or (ii) a cleavable linker which is a divalent group comprising a cleavable portion having no ability to form a bioorthogonal functional group on a reactive group side by cleavage.
[6] The compound or salt thereof according to [5], wherein L is the cleavable linker (i).
[7] The compound or salt thereof according to [5] or [6], wherein L is the cleavable linker (i), and B is the divalent group (b).
[8] The compound or salt thereof according to [5], wherein L is the cleavable linker (ii); and B is the divalent group (a) .
[9] The compound or salt thereof according to any one of [1] to [8], wherein the peptide is a binding peptide to an Fc region of a monoclonal antibody.
[10] The compound or salt thereof according to [9], wherein the peptide is a binding peptide to an Fc region of IgG.
[11] The compound or salt thereof according to any one of [1] to [10], wherein the affinity substance is an affinity substance to an antibody, comprising any one Fc region protein selected from the group consisting of the following (A) to (C) and having antigen-binding ability:
   (A) an Fc region protein comprising the amino acid sequence of SEQ ID NO: 1;
   (B) an Fc region protein comprising an amino acid sequence with one or several amino acid residues inserted, added, deleted, or substituted in the amino acid sequence of SEQ ID NO: 1; and
   (C) an Fc region protein comprising an amino acid sequence having 90% or more identity to the amino acid sequence of SEQ ID NO: 1.
[12] The compound or salt thereof according to any one of [1] to [11], wherein the peptide is capable of binding to human IgG.
[13] The compound or salt thereof according to any one of [1] to [12], wherein the cleavable portion is a portion cleavable by any of (a) treatment with one or more substances selected from the group consisting of an acidic substance, a basic substance, a reducing agent, an oxidizing agent, and an enzyme, (b) treatment by physicochemical stimulus selected from the group consisting of light, and (c) being left when a cleavable linker comprising a self-decomposing cleavable portion is used.
[14] The compound or salt thereof according to any one of [1] to [13], wherein the cleavable portion is selected from the group consisting of a disulfide residue, an acetal residue, a ketal residue, an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.
[15] The compound or salt thereof according to any one of [5] to [14], wherein the cleavable portion of (i) is selected from the group consisting of a disulfide residue, an ester residue, an acetal residue, a ketal residue, an imine residue, and a vicinal diol residue.
[16] The compound or salt thereof according to any one of [5] to [14], wherein the cleavable portion of (ii) is selected from the group consisting of an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.
[17] The compound or salt thereof according to any one of [1] to [16], wherein the cleavable portion corresponds to any one chemical structure selected from the group consisting of the following: 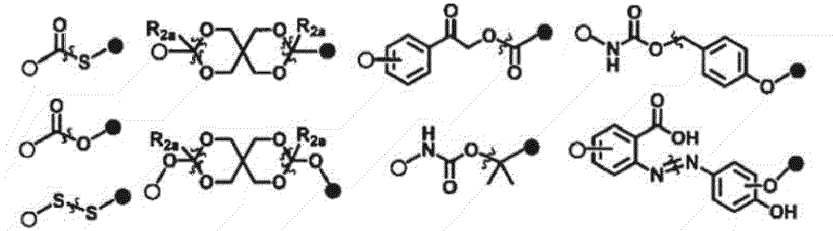 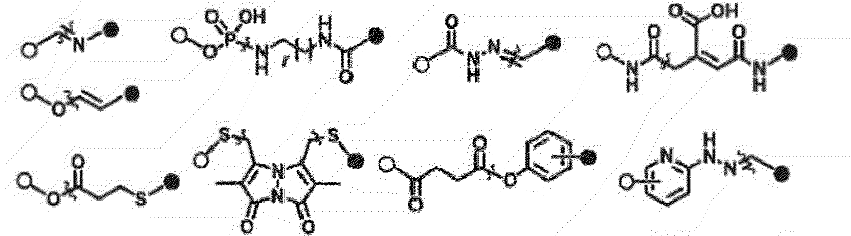 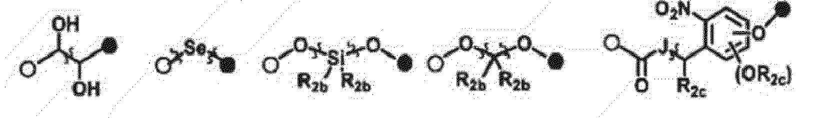 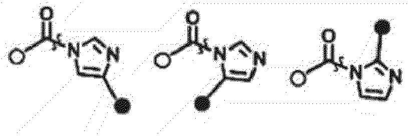
   where a wavy line orthogonal to a bond indicates a cleavage site;
   a plurality of R₂ₐs, a plurality of R_{2b}s, and a plurality of R_{2c}s are the same as or different from each other, and are each selected from the group consisting of:
      (i) a hydrogen atom or a halogen atom;
      (ii) a monovalent hydrocarbon group;
      (iii) aralkyl;
      (iv) a monovalent heterocyclic group;
      (v) R_{c}-O-, R_{c}-C(=O)-, R_{c}-O-C(=O)-, or R_{c}-C(=O)-O- (R_{c} indicates a hydrogen atom or a monovalent hydrocarbon group);
      (vi) NR_{d}Rₑ-, NR_{d}Rₑ-C(=O)-, NR_{d}Rₑ-C(=O)-O-, or R_{d}-C(=O)-NRₑ- (R_{d} and Rₑ are the same as or different from each other, and each indicate a hydrogen atom or a monovalent hydrocarbon group); and
      (vii) a nitro group, a sulfuric acid group, a sulfonic acid group, a cyano group, or a carboxy group,
   J is -CH₂-, -O-, or -S-,
   r is any integer of 1 to 4,
   a symbol of "white circle" indicates a bond to A, and a symbol of "black circle" indicates a bond to B, and
   when the chemical structure is asymmetrical with respect to the cleavage site, the symbol of "black circle" may indicate a bond to A, and the symbol of "white circle" may indicate a bond to B.
[18] The compound or salt thereof according to any one of [5] to [13], [15], and [17], wherein
   the cleavable portion of (i) corresponds to any one chemical structure selected from the group consisting of the following: 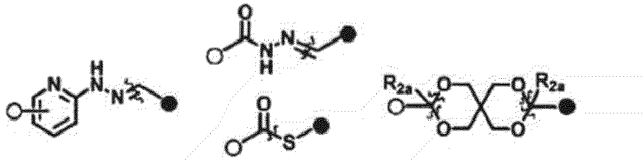 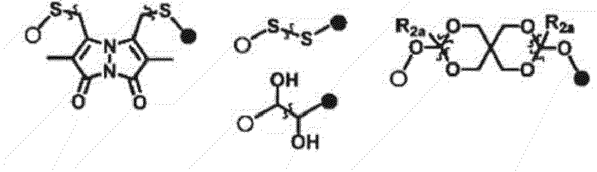
   where a wavy line orthogonal to a bond indicates a cleavage site,
   R₂ₐ is the same as that in [23],
   a symbol of "white circle indicates a bond to A, and a symbol of "black circle indicates a bond to B, and
   when the chemical structure is asymmetrical with respect to the cleavage site, the symbol of "black circle" may indicate a bond to A, and the symbol of "white circle" may indicate a bond to B.
[19] The compound or salt thereof according to any one of [5] to [13], [16], and [17], wherein
   the cleavable portion of (ii) corresponds to any one chemical structure selected from the group consisting of the following: 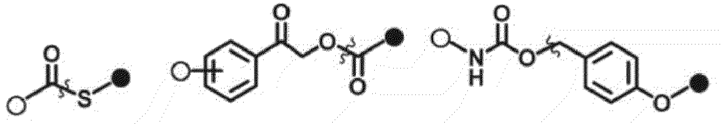 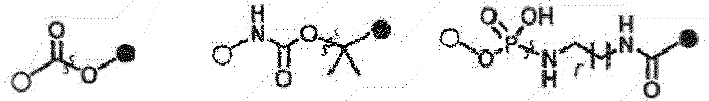 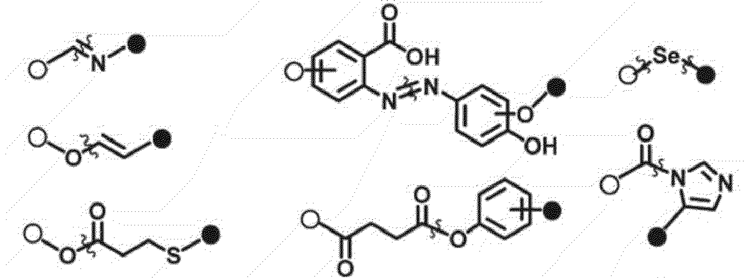 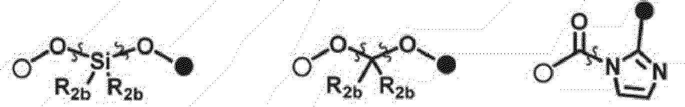 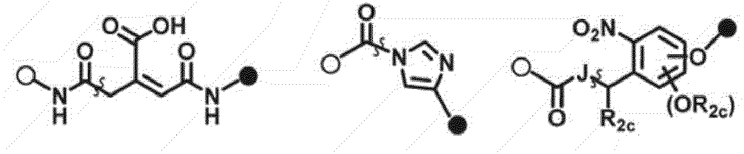
   where a wavy line orthogonal to a bond indicates a cleavage site,
   R_{2b}, R_{2c}, J, and r are the same as those in [23],
   a symbol of "white circle indicates a bond to A, and a symbol of "black circle indicates a bond to B, and
   when the chemical structure is asymmetrical with respect to the cleavage site, the symbol of "black circle" may indicate a bond to A, and the symbol of "white circle" may indicate a bond to B.
[20] The compound or salt thereof according to any one of [1] to [19], wherein L is represented by any one of the following Formulae (L1) to (L3):

   La-C-Lb (L1)

   La-C (L2)

   C-Lb (L3)

   wherein La and Lb are each a divalent group, and
   C is a cleavable portion.
[21] The compound or salt thereof according to [20], wherein
   the La and Lb are the following (La') and (Lb'), respectively: 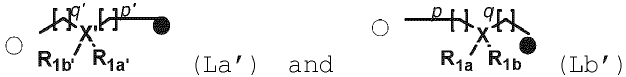 wherein
   p and p' are the same as or different from each other, and are each any integer of 0 to 10,
   q and q' are the same as or different from each other, and are each any integer of 0 to 10,
   X and X' are the same as or different from each other, and are each a carbon atom, a nitrogen atom, or a single bond (when X is a nitrogen atom, R_{1b} is absent, when X' is a nitrogen atom, R_{1b'} is absent, when X is a single bond, R₁ₐ and R_{1b} are absent, and when X' is a single bond, R_{1a'} and R_{1b'} are absent), and
   R₁ₐ, R_{1b}, R_{1a'}, and R_{1b}, are the same as or different from each other, and are each an atom or a group selected from the group consisting of the above (i) to (vii).
[22] The compound or salt thereof according to any one of [1] to [21], wherein the divalent group comprising a bioorthogonal functional group is a divalent group comprising a bioorthogonal functional group selected from the group consisting of an azide residue, an aldehyde residue, a thiol residue, an alkyne residue, an alkene residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue in a main chain thereof.
[23] The compound or salt thereof according to any one of [1] to [21], wherein the divalent group comprising a bioorthogonal functional group is a divalent group comprising a bioorthogonal functional group selected from the group consisting of an azide residue, an aldehyde residue, a thiol residue, an alkyne residue, an alkene residue, a halogen residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, an α-halocarbonyl residue, an isonitrile residue, a sydnone residue, and a selenium residue in a side chain thereof.
[24] The compound or salt thereof according to any one of [1] to [23], wherein
   the bioorthogonal functional group is any one represented by the following: 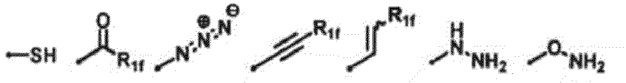 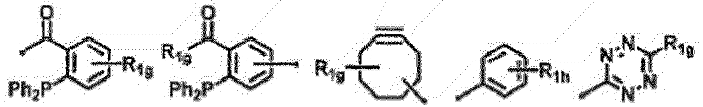 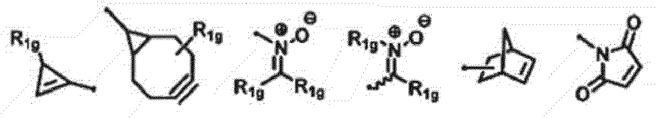 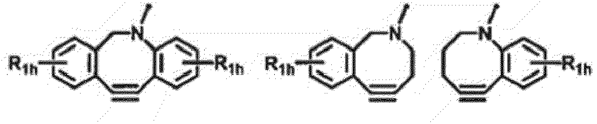 wherein
   R_{1f}, one or a plurality of R_{1g}s, and one or a plurality of R₁ₕs are the same as or different from each other, and are each an atom or a group selected from the group consisting of the above (i) to (vii) or an electron-withdrawing group, and · is a bond.
[25] The compound or salt thereof according to any one of [1] to [24], wherein the divalent group (b) is selected from the group consisting of optionally substituted alkylene, optionally substituted cycloalkylene, optionally substituted aryl, an optionally substituted divalent heterocyclic group, -NRₐ- (Rₐ indicates a hydrogen atom or a substituent), -O-, and a combination of two or more of these.
[26] The compound or salt thereof according to any one of [1] to [25], wherein
   B is represented by the following Formula (B-1): 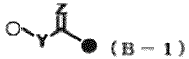 wherein
   Y is -NH-, -O-, -CH₂-, or the following Formula (B-2): 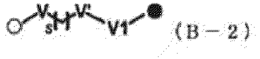 wherein
   V and V' are the same as or different from each other, and are each -NH-, -O-, -CH₂-, or a single bond,
   V1 is a divalent group comprising a bioorthogonal functional group,
   s is any integer of 0 to 10,
   a symbol of "white circle" and a symbol of "black circle" in Formula (B-2) have the same orientation as a symbol of "white circle" and a symbol of "black circle" in Formula (B-1), respectively,
   Z is an oxygen atom, a sulfur atom, or a hydrogen atom (when Z is a hydrogen atom, -C(=Z)- indicates -CH₂-), and
   in Formula (B-1), a symbol of "white circle" indicates a bond to an L-side portion, and a symbol of "black circle" indicates a bond to an R-side portion.
[27] The compound or salt thereof according to any one of [1] to [26], wherein the reactive group is a reactive group specific to any one side chain of a lysine residue, a tyrosine residue, and a tryptophan residue.
[28] The compound or salt thereof according to [27], wherein the reactive group is a reactive group specific to a side chain of a lysine residue.
[29] The compound or salt thereof according to any one of [1] to [28], wherein the reactive group corresponds to any one chemical structure selected from the group consisting of the following: 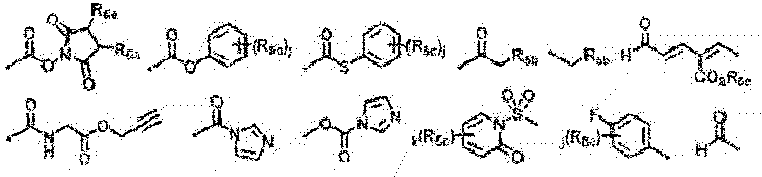 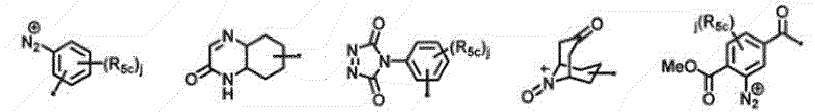 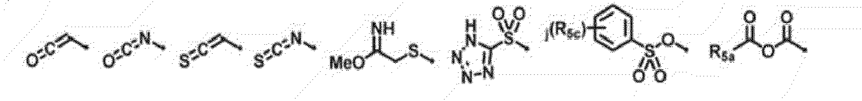
   where R₅ₐ and R_{5c} are each an atom or a group selected from the group consisting of the above (i) to (vii),
   R_{5b} is an electron-withdrawing group,
   j is any integer of 1 to 5, and
   k is any integer of 1 to 4.
[30] The compound or salt thereof according to any one of [1] to [29], wherein a main chain linking A and R has 4 to 20 atoms.
[31] The compound or salt thereof according to any one of [1] to [30], wherein a main chain linking A and R comprises no cyclic structure.
[32] The compound or salt thereof according to any one of [1] to [31], wherein a partial structure represented by L-B comprises no peptide portion.
[33] The compound or salt thereof according to any one of [1] to [32], wherein
   the compound represented by the above Formula (I) is a compound represented by the following Formula (I'): A-B2-L'-B1-R (I')
   wherein
   A and R are the same as those of the above Formula (I),
      L' is a cleavable linker which is a divalent group comprising a cleavable portion,
   B1 and B2 are the same as or different from each other, and are each (a) a divalent group comprising a bioorthogonal functional group or (b) a divalent group comprising no bioorthogonal functional group, and
      B1 and B2 may have a symmetrical structure with respect to L'.
[34] The compound or salt thereof according to [33], wherein
   the compound represented by the above Formula (I') is represented by the following Formula (I"): 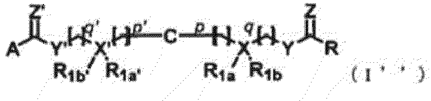 wherein
   A and R are the same as those of Formula (I) according to [1],
   C is a cleavable portion,
   p, p', q, q', X, X', R₁ₐ, R_{1a'}, R_{1b}, and R_{1b'} are the same as those of Formulae (La') and (Lb') according to [19],
   Y and Y' are the same as or different from each other, and are the same as Y of Formula (B-1) according to [24], and
   Z and Z' are the same as or different from each other, and are the same as Z of the above Formula (B-1).
[35] A reagent of regioselectively modifying an antibody, the reagent comprising a compound having an affinity substance to an antibody, a cleavable portion, and a reactive group, represented by the following Formula (I):

   A-L-B-R (I)

   wherein
   A is the affinity substance to an antibody,
   L is a cleavable linker which is a divalent group comprising the cleavable portion,
   B is (a) a divalent group comprising a bioorthogonal functional group or (b) a divalent group comprising no bioorthogonal functional group, and
   R is the reactive group to the antibody, or
   a salt thereof, wherein
   the affinity substance to an antibody is a peptide comprising any of the amino acid sequences of the above Formulae 1-1 to 1-9 and 2-1 [preferably a peptide comprising an amino acid sequence selected from the group consisting of the above (1) to (76), hereinafter the same].
[36] A compound having an affinity substance to an antibody, a cleavable portion, and a reactive group, or a salt thereof, represented by the following Formula (I):

   A-L-B-R (I)

   wherein
   A is the affinity substance to an antibody,
   L is a cleavable linker which is a divalent group comprising the cleavable portion,
   B is (a) a divalent group comprising a bioorthogonal functional group or (b) a divalent group comprising no bioorthogonal functional group, and
   R is the reactive group specific to a side chain of a lysine residue, wherein
   the affinity substance to an antibody is a peptide comprising any of the amino acid sequences of the above Formulae 1-1 to 1-9 and 2-1.
[37] A reagent of regioselectively modifying an antibody, the reagent comprising a compound having an affinity substance to an antibody, a cleavable portion, and a reactive group, represented by the following Formula (I):

   A-L-B-R (I)

   wherein
   A is the affinity substance to an antibody,
   L is a cleavable linker which is a divalent group comprising the cleavable portion,
   B is (a) a divalent group comprising a bioorthogonal functional group or (b) a divalent group comprising no bioorthogonal functional group, and
   R is the reactive group specific to a side chain of a lysine residue, or
   a salt thereof, wherein
   the affinity substance to an antibody is a peptide comprising any of the amino acid sequences of the above Formulae 1-1 to 1-9 and 2-1.

Second, the present invention provides an antibody having an affinity substance to the antibody and a cleavable portion, or a salt thereof, and a method for producing the same.

### (Antibody Having Affinity Substance to Antibody and Cleavable Portion, or Salt thereof)

[38] An antibody having an affinity substance to the antibody and a cleavable portion, or a salt thereof, represented by the following Formula (II):

   A-L-B-R'-T (II)

   wherein
   A is the affinity substance to the antibody,
   L is a cleavable linker which is a divalent group comprising a cleavable portion,
   B is (a) a divalent group comprising a bioorthogonal functional group or (b) a divalent group comprising no bioorthogonal functional group,
   R' is a portion formed by a reaction between the antibody and a reactive group, and
   T is the antibody, wherein
   the affinity substance to the antibody is a peptide comprising any of the amino acid sequences of the above Formulae 1-1 to 1-9 and 2-1.
[39] The antibody or salt thereof according to [38], wherein the antibody is a monoclonal antibody.
[40] The antibody or salt thereof according to [38] or [39], wherein the antibody is an IgG antibody.
[41] The antibody or salt thereof according to any one of [38] to [40], wherein the antibody is derived from a human.
[42] The antibody or salt thereof according to any one of [38] to [41], wherein the antibody is an antibody comprising any one Fc region protein selected from the group consisting of the following (A) to (C) and having antigen-binding ability:
   (A) an Fc region protein comprising the amino acid sequence of SEQ ID NO: 1;
   (B) an Fc region protein comprising an amino acid sequence with one or several amino acid residues inserted, added, deleted, or substituted in the amino acid sequence of SEQ ID NO: 1; and
   (C) an Fc region protein comprising an amino acid sequence having 90% or more identity to the amino acid sequence of SEQ ID NO: 1.
[43] The antibody or salt thereof according to any one of [38] to [42], wherein the antibody comprises one or more specific amino acid residues in a target region consisting of 1 to 50 consecutive amino acid residues, and five or more of the specific amino acid residues in a non-target region other than the target region, and
   a structural unit represented by A-L-B-R' binds to one or more of the specific amino acid residues contained in the target region with 30% or more regioselectivity.
[44] The antibody or salt thereof according to [43], wherein the target region is a region consisting of one to ten consecutive amino acid residues.
[45] The antibody or salt thereof according to [44], wherein the target region is a region consisting of one to three consecutive amino acid residues.
[46] The antibody or salt thereof according to [45], wherein the target region is a region consisting of amino acid residues at positions 246 to 248 in a human IgG Fc region.
[47] The antibody or salt thereof according to any one of [43] to [46], wherein the regioselectivity is 50% or more.
[48] The antibody or salt thereof according to [47], wherein the regioselectivity is 70% or more.
[49] The antibody or salt thereof according to [48], wherein the regioselectivity is 90% or more.
[50] The antibody or salt thereof according to any one of [43] to [49], wherein the specific amino acid residue does not comprise the same kind of amino acid residue as the specific amino acid residue other than the specific amino acid residue present at the specific position in a region up to a remote position of "a" (where "a" is any integer of 1 to 10) amino acid residues to an N-terminal side and a C-terminal side each with respect to the specific amino acid present at the specific position.
[51] The antibody or salt thereof according to any one of [38] to [50], wherein
   the antibody is an antibody comprising a plurality of heavy chains, and
   T has a structural unit represented by A-L-B-R' in a plurality of corresponding target regions in the heavy chains such that the antibody has a plurality of structural units represented by A-L-B-R'.
[52] The antibody or salt thereof according to [51], having two heavy chains.
[53] The antibody or salt thereof according to any one of [38] to [52], wherein the portion formed by a reaction between the antibody and a reactive group is a portion formed by a reaction of a reactive group specific to any one side chain of a lysine residue, a tyrosine residue, and a tryptophan residue to a lysine residue, a tyrosine residue, or a tryptophan residue.
[54] The antibody or salt thereof according to any one of [38] to [53], wherein the portion formed by a reaction between the antibody and a reactive group is a portion formed by a reaction between a lysine residue and a reactive group specific to a side chain of the lysine residue.
[55] The antibody or salt thereof according to any one of [38] to [54], wherein the portion formed by the reaction corresponds to any one chemical structure selected from the group consisting of the following: 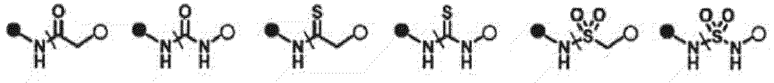 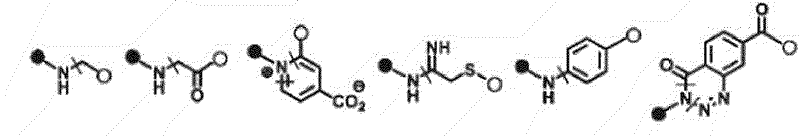 where a symbol of "black circle" indicates a bond to a T-side portion, and a symbol of "white circle" indicates a bond to a B-side portion, and a straight line orthogonal to a bond indicates a bond formed by the reaction.
[56] The antibody or salt thereof according to any one of [38] to [55], wherein a main chain linking A and R' has 4 to 20 atoms.
[57] The antibody or salt thereof according to any one of [38] to [56], wherein a main chain linking A and R comprises no cyclic structure.
[58] The antibody or salt thereof according to any one of [38] to [57], wherein a partial structure represented by L-B comprises no peptide portion.
[59] The antibody or salt thereof according to any one of [38] to [58], wherein the compound represented by the above Formula (II) is a compound represented by the following (II'): A-B2-L'-B1-R'-T (II')
   wherein
   A, R', and T are the same as those of the above Formula (II),
   L' is a cleavable linker which is a divalent group comprising a cleavable portion,
   B1 and B2 are the same as or different from each other, and are each (a) a divalent group comprising a bioorthogonal functional group or (b) a divalent group comprising no bioorthogonal functional group, and
   B1 and B2 may have a symmetrical structure with respect to L'.
[60] The antibody or salt thereof according to [59], wherein
   the compound represented by the above Formula (II') is represented by the following (II''): 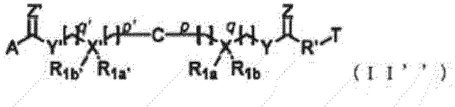 wherein
   A, R', and T are the same as those of Formula (II) according to [36],
   C is a cleavable portion,
   p and p' are the same as or different from each other, and are each any integer of 0 to 10,
   q and q' are the same as or different from each other, and are each any integer of 0 to 10,
   X and X' are the same as or different from each other, and are each a carbon atom, a nitrogen atom, or a single bond (when X is a nitrogen atom, R_{1b} is absent, when X' is a nitrogen atom, R_{1b'} is absent, when X is a single bond, R₁ₐ and R_{1b} are absent, and when X' is a single bond, R_{1a'} and R_{1b}, are absent), and
   R₁ₐ, R_{1b}, R_{1a'}, and R_{1b}, are the same as or different from each other, and are each selected from the group consisting of:
      (i) a hydrogen atom or a halogen atom;
      (ii) a monovalent hydrocarbon group;
      (iii) aralkyl;
      (iv) a monovalent heterocyclic group;
      (v) R_{c}-O-, R_{c}-C(=O)-, R_{c}-O-C(=O)-, or R_{c}-C(=O)-O- (R_{c} indicates a hydrogen atom or a monovalent hydrocarbon group);
      (vi) NR_{d}Rₑ-, NR_{d}Rₑ-C(=O)-, NR_{d}Rₑ-C(=O)-O-, or R_{d}-C(=O)-NRₑ- (R_{d} and Rₑ are the same as or different from each other, and each indicate a hydrogen atom or a monovalent hydrocarbon group); and
      (vii) a nitro group, a sulfuric acid group, a sulfonic acid group, a cyano group, or a carboxy group,
   Y and Y' are the same as or different from each other, and are the same as Y of Formula (B-1) according to [24], and
   Z and Z' are the same as or different from each other, and are the same as Z of the above Formula (B-1).
[61] An antibody having an affinity substance to the antibody and a cleavable portion, or a salt thereof, represented by the following Formula (II):

   A-L-B-R'-T (II)

   wherein
   A is the affinity substance to the antibody,
   L is a cleavable linker which is a divalent group comprising the cleavable portion,
   B is (a) a divalent group comprising a bioorthogonal functional group or (b) a divalent group comprising no bioorthogonal functional group, and
   R' is a portion formed by a reaction between the antibody and a reactive group specific to a side chain of a lysine residue, and
   T is the antibody, wherein
   the affinity substance to the antibody is a peptide comprising any of the amino acid sequences of the above Formulae 1-1 to 1-9 and 2-1.

### (Method for producing Antibody Having Affinity Substance to Antibody and Cleavable Portion, or Salt thereof)

[62] A method for producing an antibody having an affinity substance to the antibody and a cleavable portion, or a salt thereof, the method comprising
   reacting a compound having an affinity substance to an antibody, a cleavable portion, and a reactive group, represented by the following Formula (I):

   A-L-B-R (I)

   wherein
   A is the affinity substance to an antibody,
   L is a cleavable linker which is a divalent group comprising the cleavable portion,
   B is (a) a divalent group comprising a bioorthogonal functional group or (b) a divalent group comprising no bioorthogonal functional group, and
   R is a reactive group to the antibody, or a salt thereof with an antibody to form an antibody having an affinity substance to the antibody and a cleavable portion, represented by the following Formula (II):

      A-L-B-R'-T (II)

      wherein
      A, L, and B are the same as those of the above Formula (I),
      R' is a portion formed by a reaction between the antibody and the reactive group, and
      T is the antibody, or
      a salt thereof, wherein
      the affinity substance to an antibody is a peptide comprising any of the amino acid sequences of the above Formulae 1-1 to 1-9 and 2-1.
[63] The method according to [62], wherein the reactive group is a reactive group specific to a side chain of a lysine residue.

Third, the present invention provides a conjugate having an affinity substance to an antibody, a cleavable portion, a functional substance, and an antibody, or a salt thereof, and a method for producing the same.

### (Conjugate Having Affinity Substance to Antibody, Cleavable portion, Functional Substance, and Antibody, or Salt thereof)

[64] A conjugate having an affinity substance to an antibody, a cleavable portion, a functional substance, and an antibody, or a salt thereof, represented by the following Formula (III):

   A-L-B' (-F)-R'-T (III)

   wherein
   A is the affinity substance to an antibody,
   L is a cleavable linker which is a divalent group comprising a cleavable portion,
   B' is a trivalent group comprising a portion formed by a reaction between the functional substance and a bioorthogonal functional group,
   F is the functional substance,
   R' is a portion formed by a reaction between the antibody and a reactive group, and
   T is the antibody, wherein
   the affinity substance to an antibody is a peptide comprising any of the amino acid sequences of the above Formulae 1-1 to 1-9 and 2-1.
[65] The conjugate or salt thereof according to [64], wherein the antibody is a monoclonal antibody.
[66] The conjugate or salt thereof according to [64] or [65], wherein the antibody is an IgG antibody.
[67] The conjugate or salt thereof according to any one of [64] to [66], wherein the antibody is derived from a human.
[68] The conjugate or salt thereof according to any one of [64] to [67], wherein the antibody is an antibody comprising any one Fc region protein selected from the group consisting of the following (A) to (C) and having antigen-binding ability:
   (A) an Fc region protein comprising the amino acid sequence of SEQ ID NO: 1;
   (B) an Fc region protein comprising an amino acid sequence with one or several amino acid residues inserted, added, deleted, or substituted in the amino acid sequence of SEQ ID NO: 1; and
   (C) an Fc region protein comprising an amino acid sequence having 90% or more identity to the amino acid sequence of SEQ ID NO: 1.
[69] The conjugate or salt thereof according to any one of [64] to [68], wherein the antibody comprises one or more specific amino acid residues in a target region consisting of 1 to 50 consecutive amino acid residues, and five or more of the specific amino acid residues in a non-target region other than the target region, and
   a structural unit represented by A-L-B'(-F)-R' binds to the one or more of the specific amino acid residues contained in the target region with 30% or more regioselectivity.
[70] The conjugate or salt thereof according to [69], wherein the specific amino acid residue does not comprise the same kind of amino acid residue as the specific amino acid residue other than the specific amino acid residue present at the specific position in a region up to a remote position of "a" (where "a" is any integer of 1 to 10) amino acid residues to an N-terminal side and a C-terminal side each with respect to the specific amino acid present at the specific position.
[71] The conjugate or salt thereof according to any one of [64] to [70], wherein
   the antibody is an antibody comprising a plurality of heavy chains, and
   T has a structural unit represented by A-L-B'(-F)-R' in a plurality of corresponding target regions in the heavy chains such that the antibody has a plurality of structural units represented by A-L-B'(-F)-R'.
[72] The conjugate or salt thereof according to any one of [64] to [71], wherein when the functional substance has a functional group easily reacting with a bioorthogonal functional group, or when the functional substance is derivatized so as to have a functional group easily reacting with the bioorthogonal functional group, the functional group easily reacting with the bioorthogonal functional group is a group selected from the group consisting of an azide residue, an aldehyde residue, a thiol residue, an alkyne residue, an alkene residue, a halogen residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue.
[73] The conjugate or salt thereof according to any one of [64] to [71], wherein when the functional substance has a functional group easily reacting with a bioorthogonal functional group, or when the functional substance is derivatized so as to have a functional group easily reacting with the bioorthogonal functional group, the functional group easily reacting with the bioorthogonal functional group is a group selected from the group consisting of the groups represented by the following: 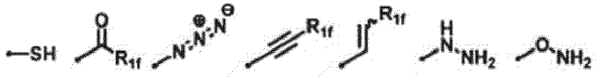 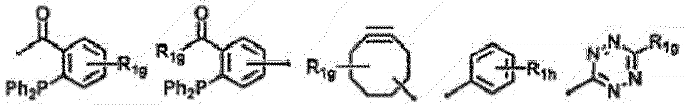 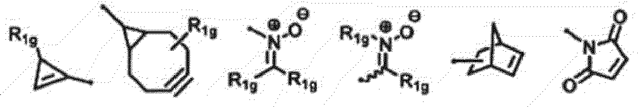 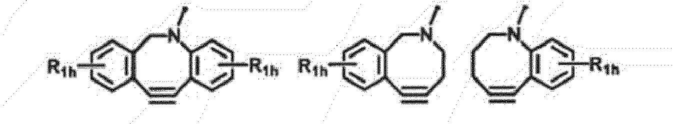 wherein
   R_{1f}, one or a plurality of R_{1g}s, and one or a plurality of R₁ₕs are the same as or different from each other, and are each an atom or a group selected from the group consisting of the above (i) to (vii) or an electron-withdrawing group, and · is a bond to the functional substance.
[74] The conjugate or salt thereof according to any one of [64] to [73], wherein the portion formed by a reaction between the antibody and a reactive group is a portion formed by a reaction between a lysine residue, a tyrosine residue, or a tryptophan residue and a reactive group specific to any one side chain of a lysine residue, a tyrosine residue, and a tryptophan residue.
[75] The conjugate or salt thereof according to any one of [64] to [74], wherein the portion formed by a reaction between the antibody and a reactive group is a portion formed by a reaction between a lysine residue and a reactive group specific to a side chain of the lysine residue.
[76] The conjugate or salt thereof according to any one of [64] to [75], wherein the portion formed by the reaction corresponds to any one chemical structure selected from the group consisting of the following: 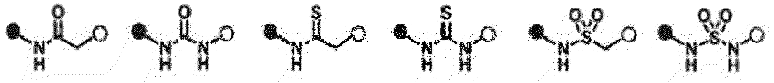 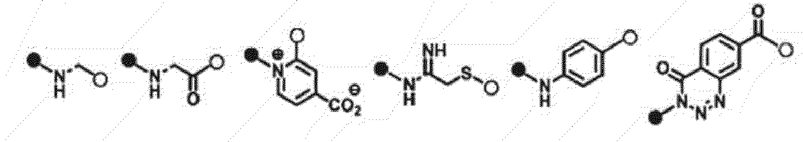 where a symbol of "black circle" indicates a bond to a T-side portion, and a symbol of "white circle" indicates a bond to a B-side portion.
[77] The conjugate or salt thereof according to any one of [64] to [76], wherein the functional substance is a drug or a labelling substance.
[78] The conjugate or salt thereof according to any one of [64] to [77], wherein the functional substance is a small compound.
[79] The conjugate or salt thereof according to [77] or [78], wherein the drug is an anti-cancer agent.
[80] The conjugate or salt thereof according to any one of [64] to [79], wherein a main chain linking A and R' has 4 to 20 atoms.
[81] The conjugate or salt thereof according to any one of [64] to [80], wherein a main chain linking A and R comprises no cyclic structure.
[82] The conjugate or salt thereof according to any one of [64] to [81], wherein a partial structure represented by L-B comprises no peptide portion.
[83] The conjugate or salt thereof according to any one of [64] to [82], wherein the compound represented by the above Formula (III) is represented by the following Formula (III'): A-B2'(-F2)-L'-B1'(-F1)-R'-T (III')
   wherein
   A, R', and T are the same as those of the above Formula (II),
   L' is a cleavable linker which is a divalent group comprising the cleavable portion,
   B1' and B2' are the same as or different from each other, and are each a trivalent group comprising a portion formed by a reaction between the functional substance and a bioorthogonal functional group,
   F1 and F2 are the same as or different from each other, and are each the functional substance, and
   B1'(-F1) and B2'(-F2) may have a symmetrical structure with respect to L'.
[84] The conjugate or salt thereof according to [83], wherein
   the compound represented by the above Formula (III') is represented by the following (III"): 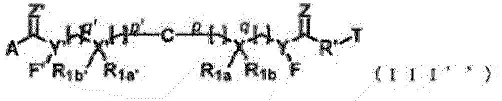 wherein
   A, R', and T are the same as those of Formula (III) according to [62],
   C is the cleavable portion,
   p and p' are the same as or different from each other, and are each any integer of 0 to 10,
   q and q' are the same as or different from each other, and are each any integer of 0 to 10,
   X and X' are the same as or different from each other, and are each a carbon atom, a nitrogen atom, or a single bond (when X is a nitrogen atom, R_{1b} is absent, when X' is a nitrogen atom, R_{1b'} is absent, when X is a single bond, R₁ₐ and R_{1b} are absent, and when X' is a single bond, R_{1a'} and R_{1b}, are absent),
   R₁ₐ, R_{1b}, R_{ia'}, and R_{1b}, are the same as or different from each other, and are each selected from the group consisting of the above (i) to (vii),
   Y and Y' are the same as or different from each other, and are each a residue obtained by removing one hydrogen atom from Y of the above Formula (B-1),
   Z and Z' are the same as or different from each other, and are the same as Z of the above Formula (B-1), and
   F and F' are the same as or different from each other, and are each the functional substance.
[85] A conjugate having an affinity substance, a functional substance, and an antibody, or a salt thereof, represented by the following Formula (III):

   A-L-B' (-F)-R'-T (III)

   wherein
   A is the affinity substance to the antibody,
   L is a cleavable linker which is a divalent group comprising a cleavable portion,
   B' is a trivalent group comprising a portion formed by a reaction between the functional substance and a bioorthogonal functional group,
   F is the functional substance,
   R' is a portion formed by a reaction between the antibody and a reactive group specific to a side chain of a lysine residue, and
   T is the antibody, wherein
   the affinity substance to the antibody is a peptide comprising any of the amino acid sequences of the above Formulae 1-1 to 1-9 and 2-1.
   (Method for Producing Conjugate Having Affinity Substance to Antibody, Cleavable portion, Functional substance, and Antibody, or Salt thereof)
[86] A method for producing a conjugate having an affinity substance to an antibody, a cleavable portion, a functional substance, and an antibody, or a salt thereof, the method comprising
   reacting an antibody having an affinity substance to the antibody and a cleavable portion, represented by the following Formula (II):

   A-L-B-R'-T (II)

   wherein
   A is the affinity substance to the antibody,
   L is a cleavable linker which is a divalent group comprising the cleavable portion,
   B is (a) a divalent group comprising a bioorthogonal functional group,
   R' is a portion formed by a reaction between the antibody and a reactive group, and
   T is the antibody, or a salt thereof with a functional substance to form a conjugate having an affinity substance to an antibody, a cleavable portion, a functional substance, and an antibody, represented by the following Formula (III) :

      A-L-B' (-F)-R'-T (III)

      wherein
      A, L, R', and T are the same as those of the above Formula (II),
      B' is a trivalent group comprising a portion formed by a reaction between the functional substance and a bioorthogonal functional group, and
      F is the functional substance, or
      a salt thereof, wherein
      the affinity substance to an antibody is a peptide comprising any of the amino acid sequences of the above Formulae 1-1 to 1-9 and 2-1.
[87] The method according to [86], wherein the reactive group is a reactive group specific to a side chain of a lysine residue.
[88] A method for producing a conjugate having an affinity substance to an antibody, a cleavable portion, a functional substance, and an antibody, or a salt thereof, the method comprising:
   (A) reacting a compound represented by the following Formula (I):

      A-L-B-R (I)

      wherein
      A is an affinity substance to an antibody,
      L is a cleavable linker which is a divalent group comprising a cleavable portion,
      B is (a) a divalent group comprising a bioorthogonal functional group, and
      R is a reactive group to the antibody, or a salt thereof with an antibody to form an antibody having an affinity substance to the antibody and a cleavable portion, represented by the following Formula (II):

         A-L-B-R'-T (II)

         wherein
         A, L, and B are the same as those of the above Formula (I),
         R' is a portion formed by a reaction between the antibody and a reactive group, and
         T is the antibody, or a salt thereof; and
   (B) reacting the antibody having an affinity substance to the antibody and a cleavable portion, or a salt thereof with a functional substance to form a conjugate having an affinity substance to an antibody, a cleavable portion, a functional substance, and an antibody, represented by the following Formula (III):

      A-L-B' (-F)-R'-T (III)

      wherein
      A and L are the same as those of the above Formula (I), R' and T are the same as those of the above Formula (II),
      B' is a trivalent group comprising a portion formed by a reaction between the functional substance and a bioorthogonal functional group, and
      F is the functional substance, or a salt thereof, wherein
      the affinity substance to an antibody is a peptide comprising any of the amino acid sequences of the above Formulae 1-1 to 1-9 and 2-1.

Fourth, the present invention provides a method for producing an antibody having a bioorthogonal functional group or bioorthogonal functional groups.
[89] A method for producing an antibody having a bioorthogonal functional group or bioorthogonal functional groups, or a salt thereof, the method comprising
   cleaving a cleavable portion of an antibody having an affinity substance to the antibody and a cleavable portion, represented by the following Formula (II):

   A-L-B-R'-T (II)

   wherein
   A is the affinity substance to the antibody,
   L is a cleavable linker which is a divalent group comprising a cleavable portion,
   B is (a) a divalent group comprising a bioorthogonal functional group or (b) a divalent group comprising no bioorthogonal functional group,
   R' is a portion formed by a reaction between the antibody and a reactive group, and
   T is the antibody, or a salt thereof to form an antibody having a bioorthogonal functional group or bioorthogonal functional groups, represented by the following Formula (IV):

      L1-B-R'-T (IV)

      wherein
      B, R', and T are the same as those of the above Formula (II), and
      L1 is (i') a monovalent group comprising a bioorthogonal functional group or (ii') a monovalent group comprising no bioorthogonal functional group, or a salt thereof, wherein
      the affinity substance to an antibody is a peptide comprising any of the amino acid sequences of the above Formulae 1-1 to 1-9 and 2-1.
[90] The method according to [89], wherein L is (i) a cleavable linker which is a divalent group comprising a cleavable portion having an ability to form a bioorthogonal functional group on a reactive group side by cleavage or (ii) a cleavable linker which is a divalent group comprising a cleavable portion having no ability to form a bioorthogonal functional group on a reactive group side by cleavage.
[91] The method according to [89], wherein
   L is the cleavable linker (i),
   L1 is (i') a monovalent group comprising a bioorthogonal functional group, and
   B is the divalent group (a) or (b).
[92] The method according to [90] or [91], wherein
   L is the cleavable linker (i),
   L1 is (i') a monovalent group comprising a bioorthogonal functional group, and
   B is the divalent group (b).
[93] The method according to [90], wherein
   L is (ii) the cleavable linker,
   L1 is (i') a monovalent group comprising no bioorthogonal functional group, and
   B is the divalent group (a).
[94] The method according to any one of [89] to [93], wherein the reactive group is a reactive group specific to a side chain of a lysine residue.
[95] A method for producing an antibody having a bioorthogonal functional group or bioorthogonal functional groups, or a salt thereof, the method comprising:
   (A) reacting a compound represented by the following Formula (I):

      A-L-B-R (I)

      wherein
      A is an affinity substance to an antibody,
      L is a cleavable linker which is a divalent group comprising a cleavable portion,
      B is (a) a divalent group comprising a bioorthogonal functional group or (b) a divalent group comprising no bioorthogonal functional group, and
      R is a reactive group to the antibody, or a salt thereof with an antibody to form an antibody having an affinity substance to the antibody and a cleavable portion, represented by the following Formula (II):

         A-L-B-R'-T (II)

         wherein
         A, L, and B are the same as those of the above Formula (I),
         R' is a portion formed by a reaction between the antibody and a reactive group, and
         T is the antibody, or a salt thereof; and
   (B) cleaving a cleavable portion of the antibody having an affinity substance to the antibody and a cleavable portion, or a salt thereof to form an antibody having a bioorthogonal functional group or bioorthogonal functional groups, represented by the following Formula (IV) :

      L1-B-R'-T (IV)

      wherein
      B, R', and T are the same as those of the above Formula (II), and
      L1 is (i') a monovalent group comprising a bioorthogonal functional group or (ii') a monovalent group comprising no bioorthogonal functional group, or a salt thereof, wherein
      the affinity substance to an antibody is a peptide comprising any of the amino acid sequences of the above Formulae 1-1 to 1-9 and 2-1.

Fifth, the present invention provides a method for producing an antibody having a functional substance or functional substances, or a salt thereof.
[96] A method for producing an antibody having a functional substance or functional substances, or a salt thereof, the method comprising:
   cleaving a cleavable portion of a conjugate having an affinity substance to an antibody, a cleavable portion, a functional substance, and an antibody, represented by the following Formula (III) :

   A-L-B' (-F)-R'-T (III)

   wherein
   A is the affinity substance to an antibody,
   L is a cleavable linker which is a divalent group comprising the cleavable portion,
   B' is a trivalent group comprising a portion formed by a reaction between the functional substance and a bioorthogonal functional group,
   F is the functional substance,
   R' is a portion formed by a reaction between the antibody and a reactive group, and
   T is the antibody, or a salt thereof to form an antibody having a functional substance or functional substances, represented by the following Formula (V):

      F-(L1-B) '-R'-T (V)

      wherein
      L1 is (i') a monovalent group comprising a bioorthogonal functional group or (ii') a monovalent group comprising no bioorthogonal functional group,
      B is (a) a divalent group comprising a bioorthogonal functional group or (b) a divalent group comprising no bioorthogonal functional group,
      a structural unit represented by (L1-B)' is a divalent structural unit comprising a portion formed by a reaction between the functional substance and either one or both of the bioorthogonal functional groups in (i') and (a),
      F is the functional substance,
      R' is a portion formed by a reaction between the antibody and a reactive group, and
      T is the antibody, wherein
      the affinity substance to an antibody is a peptide comprising any of the amino acid sequences of the above Formulae 1-1 to 1-9 and 2-1.
[97] The method according to [96], comprising
   cleaving a cleavable portion of the conjugate having an affinity substance to an antibody, a cleavable portion, a functional substance, and an antibody, or a salt thereof to form an antibody having a functional substance or functional substances, represented by the following Formula (V1) :

   L1-B'(-F)-R'-T (V1)

   wherein
   L1 is (i') a monovalent group comprising a bioorthogonal functional group or (ii') a monovalent group comprising no bioorthogonal functional group, and
   B', F, R', and T are the same as those of the above Formula (III), or a salt thereof.
[98] The method according to [96], comprising
   reacting the antibody having a bioorthogonal functional group or bioorthogonal functional groups, or a salt thereof with one or two functional substances to form an antibody having a functional substance or functional substances, represented by the following Formula (V2):

   F-L1'-B-R'-T (V2)

   wherein
   B, R', and T are the same as those of the above Formula (IV),
   L1' is a divalent group comprising a portion formed by a reaction between the functional substance(s) and (i') a monovalent group comprising a bioorthogonal functional group, and
   F is the functional substance(s), or
   the following Formula (V3):

      Fa-L1'-B'(-Fb)-R'-T (V3)

      wherein
      R' and T are the same as those of the above Formula (IV),
      L1' is the same as that of the above Formula (V2),
      B' is a trivalent group comprising a portion formed by a reaction between the functional substance(s) and a bioorthogonal functional group, and
      Fa and Fb are functional substances which are the same as or different from each other, or a salt thereof.
[99] The method according to any one of [96] to [98], wherein the reactive group is a reactive group specific to a side chain of a lysine residue.
[100] A method for producing an antibody having a functional substance or functional substances, or a salt thereof, the method comprising:
   (A) reacting an antibody having an affinity substance to the antibody and a cleavable portion, represented by the following Formula (II):

      A-L-B-R'-T (II)

      wherein
      A is the affinity substance to the antibody,
      L is a cleavable linker which is a divalent group comprising the cleavable portion,
      B is (a) a divalent group comprising a bioorthogonal functional group,
      R' is a portion formed by a reaction between the antibody and a reactive group, and
      T is the antibody, or a salt thereof with the functional substance to form a conjugate having an affinity substance to an antibody, a cleavable portion, a functional substance, and an antibody, represented by the following Formula (III):

         A-L-B' (-F)-R'-T (III)

         wherein
         A, L, R', and T are the same as those of the above Formula (II),
         B' is a trivalent group comprising a portion formed by a reaction between the functional substance and a bioorthogonal functional group, and
         F is the functional substance, or a salt thereof; and
   (B) cleaving a cleavable portion of the conjugate having an affinity substance to an antibody, a cleavable portion, a functional substance, and an antibody, or a salt thereof to form an antibody having a functional substance or functional substances, represented by the following Formula (V1):

      L1-B'(-F)-R'-T (V1)

      wherein
      L1 is (i') a monovalent group comprising a bioorthogonal functional group or (ii') a monovalent group comprising no bioorthogonal functional group, and
      B', F, R', and T are the same as those of the above Formula (III), or a salt thereof, wherein
      the affinity substance to an antibody is a peptide comprising any of the amino acid sequences of the above Formulae 1-1 to 1-9 and 2-1.
[101] A method for producing an antibody having a functional substance or functional substances, or a salt thereof, the method comprising:
   (A) reacting a compound having an affinity substance to an antibody, a cleavable portion, and a reactive group, represented by the following Formula (I):

      A-L-B-R (I)

      wherein
      A is the affinity substance to an antibody,
      L is a cleavable linker which is a divalent group comprising the cleavable portion,
      B is (a) a divalent group comprising a bioorthogonal functional group, and
      R is a reactive group to the antibody, or a salt thereof with an antibody to form an antibody having an affinity substance to the antibody and a cleavable portion, represented by the following Formula (II):

         A-L-B-R'-T (II)

         wherein
         A, L, and B are the same as those of the above Formula (I),
         R' is a portion formed by a reaction between the antibody and a reactive group, and
         T is the antibody, or a salt thereof;
   (B) reacting the antibody having an affinity substance to the antibody and a cleavable portion, or a salt thereof with a functional substance to form a conjugate having an affinity substance to an antibody, a cleavable portion, a functional substance, and an antibody, represented by the following Formula (III) :

      A-L-B' (-F)-R'-T (III)

      wherein
      A and L are the same as those of the above Formula (I), R' and T are the same as those of the above Formula (II),
      B' is a trivalent group comprising a portion formed by a reaction between the functional substance and a bioorthogonal functional group, and
      F is the functional substance, or a salt thereof; and
   (C) cleaving a cleavable portion of the conjugate having an affinity substance to an antibody, a cleavable portion, a functional substance, and an antibody, or a salt thereof to form an antibody having a functional substance or functional substances, represented by the following Formula (V1):

      L1-B'(-F)-R'-T (V1)

      wherein
      L1 is (i') a monovalent group comprising a bioorthogonal functional group or (ii') a monovalent group comprising no bioorthogonal functional group, and
      B', F, R', and T are the same as those of the above Formula (III), or a salt thereof, wherein
      the affinity substance to an antibody is a peptide comprising any of the amino acid sequences of the above Formulae 1-1 to 1-9 and 2-1.
[102] A method for producing an antibody having a functional substance or functional substances, or a salt thereof, the method comprising:
   (A) cleaving a cleavable portion of an antibody having an affinity substance to the antibody and a cleavable portion, represented by the following Formula (II):

      A-L-B-R'-T (II)

      wherein
      A is the affinity substance to an antibody,
      L is a cleavable linker which is a divalent group comprising the cleavable portion,
      B is (a) a divalent group comprising a bioorthogonal functional group or (b) a divalent group comprising no bioorthogonal functional group, and
      R' is a portion formed by a reaction between the antibody and a reactive group, and
      T is the antibody, or a salt thereof to form an antibody having a bioorthogonal functional group or bioorthogonal functional groups, represented by the following Formula (IV):

         L1-B-R'-T (IV)

         wherein
         B, R', and T are the same as those of the above Formula (II), and
         L1 is (i') a monovalent group comprising a bioorthogonal functional group or (ii') a monovalent group comprising no bioorthogonal functional group, or a salt thereof; and
   (B) reacting the antibody having a bioorthogonal functional group or bioorthogonal functional groups, or a salt thereof with one or more functional substances to form an antibody having a functional substance or functional substances, represented by the following Formula (V2):

      F-L1'-B-R'-T (V2)

      wherein
      B, R', and T are the same as those of the above Formula (IV),
      L1' is a divalent group comprising a portion formed by a reaction between the functional substance and (i') the monovalent group comprising a bioorthogonal functional group, and
      F is the functional substance, or the following Formula (V3):

         Fa-L1'-B'(-Fb)-R'-T (V3)

         wherein
         R' and T are the same as those of the above Formula (IV),
         L1' is the same as that of the above Formula (V2),
         B' is a trivalent group comprising a portion formed by a reaction between the functional substance and a bioorthogonal functional group, and
         Fa and Fb are functional substances which are the same as or different from each other, or a salt thereof, wherein
         the affinity substance to an antibody is a peptide comprising any of the amino acid sequences of the above Formulae 1-1 to 1-9 and 2-1.
[103] A method for producing an antibody having a functional substance or functional substances, or a salt thereof, the method comprising:
   (A) reacting a compound represented by the following Formula (I):

      A-L-B-R (I)

      wherein
      A is an affinity substance to the antibody,
      L is a cleavable linker which is a divalent group comprising a cleavable portion,
      B is (a) a divalent group comprising a bioorthogonal functional group or (b) a divalent group comprising no bioorthogonal functional group, and
      R is a reactive group to the antibody, or a salt thereof with an antibody to form an antibody having an affinity substance to the antibody and a cleavable portion, represented by the following Formula (II):

         A-L-B-R'-T (II)

         wherein
         A, L, and B are the same as those of the above Formula (I),
         R' is a portion formed by a reaction between the antibody and a reactive group, and
         T is the antibody, or a salt thereof;
   (B) cleaving a cleavable portion of the antibody having an affinity substance to the antibody and a cleavable portion, or a salt thereof to form an antibody having a bioorthogonal functional group or bioorthogonal functional groups, represented by the following Formula (IV) :

      L1-B-R'-T (IV)

      wherein
      B, R', and T are the same as those of the above Formula (II), and
      L1 is (i') a monovalent group comprising a bioorthogonal functional group or (ii') a monovalent group comprising no bioorthogonal functional group, or a salt thereof; and
   (C) reacting the antibody having a bioorthogonal functional group or bioorthogonal functional groups, or a salt thereof with one or more functional substances to form an antibody having a functional substance or functional substances, represented by the following Formula (V2):

      F-L1'-B-R'-T (V2)

      wherein
      B, R', and T are the same as those of the above Formula (IV),
      L1' is a divalent group comprising a portion formed by a reaction between the functional substance and (i') the monovalent group comprising a bioorthogonal functional group, and
      F is the functional substance, or the following Formula (V3):

         Fa-L1'-B'(-Fb)-R'-T (V3)

         wherein
         R' and T are the same as those of the above Formula (IV),
         L1' is the same as that of the above Formula (V2),
         B' is a trivalent group comprising a portion formed by a reaction between the functional substance and a bioorthogonal functional group, and
         Fa and Fb are the functional substances which are the same as or different from each other, or a salt thereof, wherein
         the affinity substance to an antibody is a peptide comprising any of the amino acid sequences of the above Formulae 1-1 to 1-9 and 2-1.

### Effect of Invention

(I) The compound or salt thereof of the present invention having an affinity substance to an antibody, a cleavable portion, and a reactive group is useful for regioselective modification of an antibody, for example.

(I) The compound or salt thereof of the present invention, (II) the antibody or salt thereof of the present invention (regioselectively) having an affinity substance to an antibody and a cleavable portion, and (III) the conjugate or salt thereof of the present invention (regioselectively) having an affinity substance to an antibody, a cleavable portion, a functional substance, and an antibody are useful as intermediates for preparing an antibody (regioselectively) having a bioorthogonal functional group or bioorthogonal functional groups, or a salt thereof, and an antibody (regioselectively) having a functional substance or functional substances, or a salt thereof, for example. The antibody or salt thereof (regioselectively) having a functional substance or functional substances is useful as pharmaceuticals or reagents (e.g., diagnostic reagents and reagents for research), for example. The antibody or salt thereof (regioselectively) having a bioorthogonal functional group or bioorthogonal functional groups is useful as an intermediate for preparing an antibody (regioselectively) having a functional substance or functional substances, or a salt thereof, for example. Consequently, (I) the compound or salt thereof of the present invention, (II) the antibody or salt thereof of the present invention, and (III) the conjugate or salt thereof of the present invention are useful as synthetic intermediates of pharmaceuticals or reagents, for example.

### BRIEF DESCRIPTION OF DRAWINGS

FIG. 1-1 is a schematic diagram (No. 1) of the concept of regioselective modification of an antibody with a compound of the present invention [a compound having an affinity substance to an antibody, a cleavable portion, and a reactive group: A-L-B-R(I)]. First, the compound of the present invention associates with an antibody (T) through an affinity substance to an antibody (A). Next, the compound of the present invention reacts with a side chain of a specific amino acid residue (a side chain of a lysine residue in the drawing) in a target region present near an association site of the affinity substance and the antibody through a reactive group (R) (an activated ester in the drawing) to form a conjugate between the compound of the present invention and the antibody [an antibody regioselectively having a structural unit comprising an affinity substance to an antibody and a cleavable portion: A-L-B-R'-T (II)].
FIG. 1-2 is a schematic diagram (No. 2) of the concept of regioselective modification of an antibody with the compound of the present invention. Cleavage of a cleavable portion in a linker (L) forms an antibody regiospecifically modified with a bioorthogonal functional group.
FIG. 1-3 is a schematic diagram (No. 3) of the concept of regioselective modification of an antibody with the compound of the present invention. A reaction between a bioorthogonal functional group and a functional substance (e.g., a drug) forms an antibody regiospecifically modified with the functional substance.

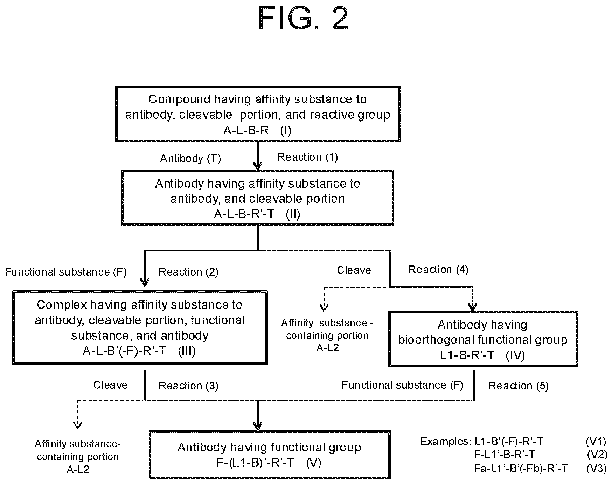

FIG. 2 is a diagram illustrating a relation among the aspects of the present invention (the expression of a salt is omitted). In Reaction (1), a compound having an affinity substance to an antibody, a cleavable portion, and a reactive group is reacted with an antibody to form an antibody having an affinity substance to the antibody and a cleavable portion. In Reaction (2), the antibody having an affinity substance to the antibody and a cleavable portion is reacted with a functional substance to form a conjugate having an affinity substance to an antibody, a cleavable portion, a functional substance, and an antibody. In Reaction (3), a cleavable portion of the conjugate having an affinity substance to an antibody, a cleavable portion, a functional substance, and an antibody is cleaved to form an antibody having a functional substance or functional substances (in this process, an affinity substance-containing portion is formed as a by-product). In Reaction (4), a cleavable portion of the antibody having an affinity substance to the antibody and a cleavable portion is cleaved to form an antibody having a bioorthogonal functional group or bioorthogonal functional groups (in this process, an affinity substance-containing portion is formed as a by-product). In Reaction (5), the antibody having a bioorthogonal functional group or bioorthogonal functional groups is reacted with a functional substance to form an antibody having a functional substance or functional substances. Reactions (2) and (5) can be conducted in a similar manner. Reactions (3) and (5) can also be conducted in a similar manner.
FIG. 3 is a diagram illustrating a consensus amino acid sequence between an Fc region in a heavy chain of trastuzumab and an IgG1 Fc region (SEQ ID NO: 1).
FIG. 4 is a diagram illustrating (1) an amino acid sequence of a heavy chain of trastuzumab (SEQ ID NO: 2), (2) an amino acid sequence of an IgG1 Fc region with a sugar chain cleaved with PNGase (SEQ ID NO: 3), and (3) an amino acid sequence of a light chain of trastuzumab (SEQ ID NO: 4).
FIG. 5 is diagram illustrating a result of Hydrophobic Interaction Chromatography (HIC)-UPLC analysis of specific modification of trastuzumab (detection wavelength: UV280 nm) (Example 1). AU on the vertical axis indicates absorbance (the same for the drawings below).
FIG. 6 is a diagram of an MS spectrum (measured value: m/z 1269.30717; theoretical value: 1269.30273; and trivalent) of a peptide fragment of the peptide THTCPPCPAPELLGGPSVFLFPPKPKDTLMISR (SEQ ID NO: 6) consisting of 33 amino acid residues comprising a modified site to a lysine residue by trypsin digestion of trastuzumab (a thiol-introduced portion subjected to carbamidomethylation with iodoacetamide (+145.019 Da)) (Example 2).
FIG. 7-1 is a diagram illustrating a CID spectrum of a product ion of m/z 603.29 (theoretical value: 603.30) corresponding to divalent y9, indicating modification of a lysine residue at position 248 of a human IgG heavy chain in EU numbering (Example 2).

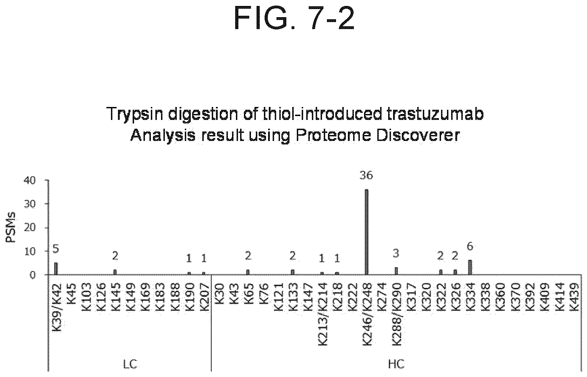

FIG. 7-2 is a diagram illustrating a result of searching for a peptide fragment comprising a modified lysine residue (a thiol-introduced portion subjected to carbamidomethylation with iodoacetamide (+145.019 Da) with respect to a trypsin digestion of trastuzumab using Proteome Discoverer (Thermo Fisher Scientific) (Example 2). The horizontal axis indicates an identified lysine residue, and the vertical axis indicates Peptide Spectrum Matches (PSMs). The residue number of the lysine residue on a heavy chain VH domain and a light chain was expressed by the number in the sequence (that is, the N-terminal amino acid is the first, hereinafter the same), and the residue number of the lysine residue on heavy chain CH1, CH2, and CH3 domains are expressed by EU numbering.
FIG. 8 is a diagram of an MS spectrum (measured value: m/z 619.67299; theoretical value: 619.67112; and trivalent) of a peptide fragment of the peptide LLGGPSVFLFPPKPKD (SEQ ID NO: 7) consisting of 16 amino acid residues comprising a modified site to a lysine residue by Glu-C protease digestion of trastuzumab (a thiol-introduced portion subjected to carbamidomethylation with iodoacetamide (+145.019 Da)) (Example 2).
FIG. 9-1 is a diagram illustrating a CID spectrum of a product ion of m/z 729.49 (theoretical value: 729.36) corresponding to monovalent y5, indicating modification of a lysine residue at position 246 or 248 of a human IgG heavy chain in EU numbering (Example 2).

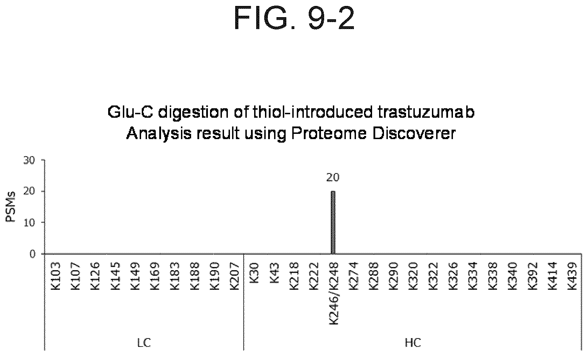

FIG. 9-2 is a diagram illustrating a result of searching for a peptide fragment comprising a modified lysine residue (a thiol-introduced portion subjected to carbamidomethylation with iodoacetamide (+145.019 Da) with respect to a Glu-C protease digestion of trastuzumab using Proteome Discoverer (Thermo Fisher Scientific) (Example 2). The horizontal axis indicates an identified lysine residue, and the vertical axis indicates Peptide Spectrum Matches (PSMs). The residue number of the lysine residue on a heavy chain VH domain and a light chain are expressed by the number in the sequence, and the residue number of the lysine residue on heavy chain CH1, CH2, and CH3 domains are expressed by EU numbering.
FIG. 10 is a diagram of a result of HIC-UPLC analysis of specific modification of trastuzumab (detection wavelength: UV 280 nm) (Example 3).
FIG. 11 is a diagram of a result of HIC-UPLC analysis of specific modification of trastuzumab (detection wavelength: UV 280 nm) (Example 4).
FIG. 12 is a diagram of a result of HIC-UPLC analysis of specific modification of trastuzumab (detection wavelength: UV 280 nm) (Example 5).
FIG. 13 is a diagram of an MS spectrum (measured value: m/z 952.23170; theoretical value: 952.22900; and tetravalent) of a peptide fragment of the peptide THTCPPCPAPELLGGPSVFLFPPKPKDTLMISR (SEQ ID NO: 6) consisting of 33 amino acid residues comprising a modified site to a lysine residue by trypsin digestion of trastuzumab (a thiol-introduced portion subjected to carbamidomethylation with iodoacetamide (+145.019 Da)) (Example 6).
FIG. 14-1 is a diagram illustrating a CID spectrum of a product ion of m/z 1166.88 (theoretical value: 1166.61) corresponding to divalent y20, indicating modification of a lysine residue at position 246 or 248 of a human IgG heavy chain in EU numbering (Example 6).

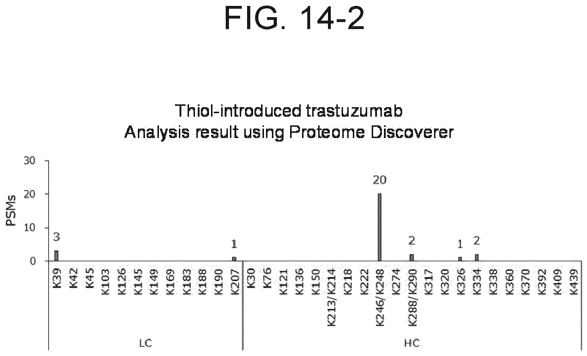

FIG. 14-2 is a diagram illustrating a result of searching for a peptide fragment comprising a modified lysine residue (a thiol-introduced portion subjected to carbamidomethylation with iodoacetamide (+145.019 Da) with respect to a trypsin digestion of trastuzumab using Proteome Discoverer (Thermo Fisher Scientific) (Example 6). The horizontal axis indicates an identified lysine residue, and the vertical axis indicates Peptide Spectrum Matches (PSMs). The residue number of the lysine residue on a heavy chain VH domain and a light chain are expressed by the number in the sequence, and the residue number of the lysine residue on heavy chain CH1, CH2, and CH3 domains are expressed by EU numbering.
FIG. 15 is a diagram of a result of HIC-UPLC analysis of specific modification of trastuzumab (detection wavelength: UV 280 nm) (Example 7).
FIG. 16 is a diagram of a result of HIC-UPLC analysis of specific modification of trastuzumab (detection wavelength: UV 280 nm) (Example 8).
FIG. 17 is a diagram of a result of HIC-UPLC analysis of specific modification of trastuzumab (detection wavelength: UV 280 nm) (Example 9).
FIG. 18 is a diagram of a result of HIC-UPLC analysis of specific modification of trastuzumab (detection wavelength: UV 280 nm) (Example 10).
FIG. 19 is a diagram of a result of HIC-UPLC analysis of specific modification of trastuzumab (detection wavelength: UV 280 nm) (Example 11).
FIG. 20 is a diagram of a result of HIC-UPLC analysis of specific modification of trastuzumab (detection wavelength: UV 280 nm) (Example 12).
FIG. 21 is a diagram of a result of HIC-UPLC analysis of specific modification of trastuzumab (detection wavelength: UV 280 nm) (Example 13).
FIG. 22 is a diagram of a result of HIC-UPLC analysis of specific modification of trastuzumab (detection wavelength: UV 280 nm) (Example 14).
FIG. 23 is a diagram of a result of HIC-UPLC analysis of specific modification of trastuzumab (detection wavelength: UV 280 nm) (Example 15).
FIG. 24 is a diagram of a result of HIC-UPLC analysis of specific modification of trastuzumab (detection wavelength: UV 280 nm) (Example 16).
FIG. 25 is a diagram of a result of HIC-UPLC analysis of specific modification of trastuzumab (detection wavelength: UV 280 nm) (Example 17).
FIG. 26 is a diagram of a result of HIC-UPLC analysis of specific modification of trastuzumab (detection wavelength: UV 280 nm) (Example 18).
FIG. 27 is a diagram of a result of HIC-UPLC analysis of specific modification of trastuzumab (detection wavelength: UV 280 nm) (Example 19).
FIG. 28 is a diagram of a result of HIC-UPLC analysis of specific modification of trastuzumab (detection wavelength: UV 280 nm) (Example 20).
FIG. 29 is a diagram of a result of HIC-UPLC analysis of specific modification of trastuzumab (detection wavelength: UV 280 nm) (Example 21).
FIG. 30 is a diagram of a result of HIC-UPLC analysis of specific modification of trastuzumab (detection wavelength: UV 280 nm) (Example 22).
FIG. 31 is a diagram of a result of HIC-UPLC analysis of specific modification of trastuzumab (detection wavelength: UV 280 nm) (Example 23).
FIG. 32 is a diagram of a result of HIC-UPLC analysis of specific modification of trastuzumab (detection wavelength: UV 280 nm) (Example 24).
FIG. 33 is a diagram of a result of HIC-UPLC analysis of specific modification of trastuzumab (detection wavelength: UV 280 nm) (Example 25).
FIG. 34 is a diagram of a result of HIC-UPLC analysis of specific modification of trastuzumab (detection wavelength: UV 280 nm) (Example 26).
FIG. 35 is a diagram of a result of HIC-UPLC analysis of specific modification of trastuzumab (detection wavelength: UV 280 nm) (Example 27).
FIG. 36 is a diagram of a result of HIC-UPLC analysis of specific modification of trastuzumab (detection wavelength: UV 280 nm) (Example 28).
FIG. 37 is a diagram of a result of HIC-UPLC analysis of specific modification of trastuzumab (detection wavelength: UV 280 nm) (Example 29).
FIG. 38 is a diagram of a result of HIC-UPLC analysis of specific modification of trastuzumab (detection wavelength: UV 280 nm) (Example 30).
FIG. 39 is a diagram of a result of HIC-UPLC analysis of specific modification of trastuzumab (detection wavelength: UV 280 nm) (Example 31).
FIG. 40 is a diagram of a result of HIC-UPLC analysis of specific modification of trastuzumab (detection wavelength: UV 280 nm) (Example 32).
FIG. 41 is a diagram of a result of HIC-UPLC analysis of specific modification of trastuzumab (detection wavelength: UV 280 nm) (Example 33).
FIG. 42 is a diagram of a result of HIC-UPLC analysis of specific modification of trastuzumab (detection wavelength: UV 280 nm) (Example 34).
FIG. 43 is a diagram of a result of HIC-UPLC analysis of specific modification of trastuzumab (detection wavelength: UV 280 nm) (Example 35).
FIG. 44 is a diagram of a result of HIC-UPLC analysis of specific modification of trastuzumab (detection wavelength: UV 280 nm) (Example 36).
FIG. 45 is a diagram of a result of HIC-UPLC analysis of specific modification of trastuzumab (detection wavelength: UV 280 nm) (Example 37).
FIG. 46 is a diagram of a result of HIC-UPLC analysis of specific modification of trastuzumab (detection wavelength: UV 280 nm) (Example 38).
FIG. 47 is a diagram of a result of HIC-UPLC analysis of specific modification of trastuzumab (detection wavelength: UV 280 nm) (Example 39).
FIG. 48 is a diagram of a result of HIC-UPLC analysis of specific modification of trastuzumab (detection wavelength: UV 280 nm) (Example 40).
FIG. 49 is a diagram of a result of HIC-UPLC analysis of specific modification of trastuzumab (detection wavelength: UV 280 nm) (Example 41).
FIG. 50 is a diagram of a result of HIC-UPLC analysis of specific modification of trastuzumab (detection wavelength: UV 280 nm) (Example 42).
FIG. 51 is a diagram of a result of HIC-UPLC analysis of specific modification of trastuzumab (detection wavelength: UV 280 nm) (Example 43).
FIG. 52 is a diagram of a result of HIC-UPLC analysis of specific modification of trastuzumab (detection wavelength: UV 280 nm) (Example 44).
FIG. 53 is a diagram of a result of HIC-UPLC analysis of specific modification of trastuzumab (detection wavelength: UV 280 nm) (Example 45).
FIG. 54 is a diagram of a result of HIC-UPLC analysis of specific modification of trastuzumab (detection wavelength: UV 280 nm) (Example 46).
FIG. 55 is a diagram of a result of HIC-UPLC analysis of specific modification of trastuzumab (detection wavelength: UV 280 nm) (Example 47).
FIG. 56 is a diagram of a result of HIC-UPLC analysis of specific modification of trastuzumab (detection wavelength: UV 280 nm) (Example 48).
FIG. 57 is a diagram of a result of HIC-UPLC analysis of specific modification of trastuzumab (detection wavelength: UV 280 nm) (Example 49).
FIG. 58 is a diagram of a result of HIC-UPLC analysis of specific modification of trastuzumab (detection wavelength: UV 280 nm) (Example 50).
FIG. 59 is a diagram of a result of HIC-UPLC analysis of specific modification of trastuzumab (detection wavelength: UV 280 nm) (Example 51).
FIG. 60 is a diagram of a result of HIC-UPLC analysis of specific modification of trastuzumab (detection wavelength: UV 280 nm) (Example 52).
FIG. 61 is a diagram of a result of HIC-UPLC analysis of specific modification of trastuzumab (detection wavelength: UV 280 nm) (Example 53).
FIG. 62 is a diagram of a result of HIC-UPLC analysis of specific modification of trastuzumab (detection wavelength: UV 280 nm) (Example 54).
FIG. 63 is a diagram of a result of HIC-UPLC analysis of specific modification of trastuzumab (detection wavelength: UV 280 nm) (Example 55).
FIG. 64 is a diagram of a result of HIC-UPLC analysis of specific modification of trastuzumab (detection wavelength: UV 280 nm) (Example 56).
FIG. 65 is a diagram of a result of HIC-UPLC analysis of specific modification of trastuzumab (detection wavelength: UV 280 nm) (Example 57).
FIG. 66 is a diagram of a result of HIC-UPLC analysis of specific modification of trastuzumab (detection wavelength: UV 280 nm) (Example 58).
FIG. 67 is a diagram of a result of HIC-UPLC analysis of specific modification of trastuzumab (detection wavelength: UV 280 nm) (Example 59).
FIG. 68 is a diagram of a result of HIC-UPLC analysis of specific modification of trastuzumab (detection wavelength: UV 280 nm) (Example 60).
FIG. 69 is a diagram of a result of HIC-UPLC analysis of specific modification of trastuzumab (detection wavelength: UV 280 nm) (Example 61).
FIG. 70 is a diagram of a result of HIC-UPLC analysis of specific modification of trastuzumab (detection wavelength: UV 280 nm) (Example 62).
FIG. 71 is a diagram of a result of HIC-UPLC analysis of specific modification of trastuzumab (detection wavelength: UV 280 nm) (Example 63).
FIG. 72 is a diagram of a result of HIC-UPLC analysis of specific modification of trastuzumab (detection wavelength: UV 280 nm) (Example 64).
FIG. 73 is a diagram of a result of HIC-UPLC analysis of specific modification of trastuzumab (detection wavelength: UV 280 nm) (Example 65).
FIG. 74 is a diagram of a result of HIC-UPLC analysis of specific modification of trastuzumab (detection wavelength: UV 280 nm) (Example 66).
FIG. 75 is a diagram of a result of HIC-UPLC analysis of specific modification of trastuzumab (detection wavelength: UV 280 nm) (Example 67).
FIG. 76 is a diagram of a result of HIC-UPLC analysis of specific modification of trastuzumab (detection wavelength: UV 280 nm) (Example 68).
FIG. 77 is a diagram of a result of HIC-UPLC analysis of specific modification of trastuzumab (detection wavelength: UV 280 nm) (Example 69).
FIG. 78 is a diagram of a result of HIC-UPLC analysis of specific modification of trastuzumab (detection wavelength: UV 280 nm) (Example 70).
FIG. 79 is a diagram of a result of HIC-UPLC analysis of specific modification of trastuzumab (detection wavelength: UV 280 nm) (Example 71).
FIG. 80 is a diagram of a result of HIC-UPLC analysis of specific modification of trastuzumab (detection wavelength: UV 280 nm) (Example 72).
FIG. 81 is a diagram of a result of HIC-UPLC analysis of specific modification of trastuzumab (detection wavelength: UV 280 nm) (Example 73).
FIG. 82 is a diagram of a result of HIC-UPLC analysis of specific modification of trastuzumab (detection wavelength: UV 280 nm) (Example 74).
FIG. 83 is a diagram of a result of HIC-UPLC analysis of specific modification of trastuzumab (detection wavelength: UV 280 nm) (Example 75).
FIG. 84 is a diagram of a result of HIC-UPLC analysis of specific modification of trastuzumab (detection wavelength: UV 280 nm) (Example 76).
FIG. 85 is a diagram of a result of HIC-UPLC analysis of specific modification of trastuzumab (detection wavelength: UV 280 nm) (Example 77).
FIG. 86 is a diagram of a result of HIC-UPLC analysis of specific modification of trastuzumab (detection wavelength: UV 280 nm) (Example 78).
FIG. 87 is a diagram of a result of HIC-UPLC analysis of specific modification of trastuzumab (detection wavelength: UV 280 nm) (Example 79).
FIG. 88 is a diagram illustrating determination of heavy chain selectivity of specifically modified compound of trastuzumab under reduction conditions by ESI-TOFMS analysis (Example 80).
FIG. 89 is a diagram of a result of HIC-UPLC analysis of specific modification of trastuzumab (detection wavelength: UV 280 nm) (Example 80).
FIG. 90 is a diagram illustrating determination of heavy chain selectivity of a specifically modified compound of thiol-introduced trastuzumab under reduction conditions by ESI-TOFMS analysis (Example 80).
FIG. 91 is a diagram of a result of HIC-UPLC analysis of specific modification of trastuzumab (detection wavelength: UV 280 nm) (Example 80).
FIG. 92 is a diagram illustrating determination of heavy chain selectivity of fluorescently labeled trastuzumab under reduction conditions by ESI-TOFMS analysis (Example 80).
FIG. 93 is a diagram illustrating determination of heavy chain selectivity of ADC mimic under reduction conditions by ESI-TOFMS analysis (Example 80).
FIG. 94 is a diagram illustrating a summary of results of Example 80.
FIG. 95 is a diagram of a result of HIC-UPLC analysis of specific modification of trastuzumab (detection wavelength: UV 280 nm) (Example 81).
FIG. 96 is a diagram of an MS spectrum (measured value: m/z 1269.30359; theoretical value: 1269.30273; and trivalent) of a peptide fragment of the peptide THTCPPCPAPELLGGPSVFLFPPKPKDTLMISR (SEQ ID NO: 6) consisting of 33 amino acid residues comprising a modified site to a lysine residue by trypsin digestion of trastuzumab (a thiol-introduced portion subjected to carbamidomethylation with iodoacetamide (+145.019 Da)) (Example 81).
FIG. 97-1 is a diagram illustrating a CID spectrum of a product ion of m/z 1205.86 (theoretical value: 1205.60) corresponding to monovalent y9, indicating modification of a lysine residue at position 246 or 248 of a human IgG heavy chain in EU numbering (Example 81).

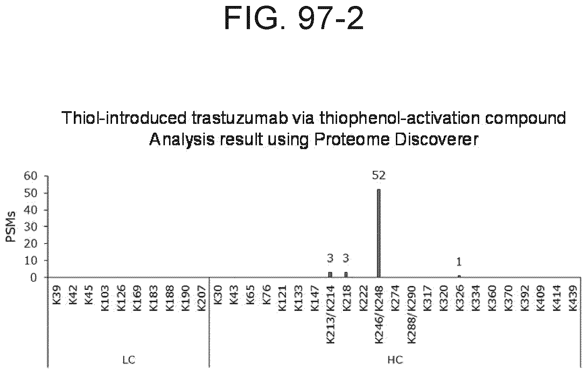

FIG. 97-2 is a diagram illustrating a result of searching for a peptide fragment comprising a modified lysine residue (a thiol-introduced portion subjected to carbamidomethylation with iodoacetamide (+145.019 Da) with respect to a trypsin digestion of trastuzumab using Proteome Discoverer (Thermo Fisher Scientific) (Example 81). The horizontal axis indicates an identified lysine residue, and the vertical axis indicates Peptide Spectrum Matches (PSMs). The residue number of the lysine residue on a heavy chain VH domain and a light chain are expressed by the number in the sequence, and the residue number of the lysine residue on heavy chain CH1, CH2, and CH3 domains are expressed by EU numbering.
FIG. 98 is a diagram illustrating an analysis result of an antibody-nucleic acid conjugate by SDS-PAGE (Example 82) .
FIG. 99 is a diagram illustrating determination of heavy chain selectivity of azide-introduced trastuzumab under reduction conditions by ESI-TOFMS analysis (Example 83) .
FIG. 100 is a diagram illustrating determination of heavy chain selectivity of ADC mimic under reduction conditions by ESI-TOFMS analysis (Example 83).
FIG. 101 is a diagram illustrating determination of heavy chain selectivity of specifically modified compound of trastuzumab under reduction conditions by ESI-TOFMS analysis (Example 84).
FIG. 102 is a diagram of a result of HIC-UPLC analysis of specific modification of trastuzumab (detection wavelength: UV 280 nm) (Example 84).
FIG. 103 is a diagram illustrating determination of heavy chain selectivity of a specifically modified compound of azide-introduced trastuzumab under reduction conditions by ESI-TOFMS analysis (Example 84).
FIG. 104 is a diagram illustrating a result of HIC-UPLC analysis of specific modification of azide-introduced trastuzumab (detection wavelength: UV 280 nm) (Example 84).
FIG. 105 is a diagram of an MS spectrum (measured value: m/z 1300.99241; theoretical value: 1300.99173; and trivalent) of a peptide fragment of the peptide THTCPPCPAPELLGGPSVFLFPPKPKDTLMISR (SEQ ID NO: 6) consisting of 33 amino acid residues comprising a modified site to a lysine residue by trypsin digestion of trastuzumab (an azidocarboxylic acid-introduced portion (+240.086)) (Example 84).
FIG. 106-1 is a diagram illustrating a CID spectrum of a product ion of m/z 1622.92 (theoretical value: 1622.87) corresponding to monovalent y12, indicating modification of a lysine residue at position 246 or 248 in EU numbering (Example 84).

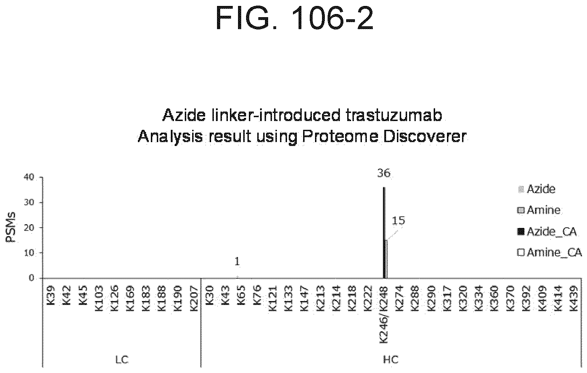

FIG. 106-2 is a diagram illustrating a result of searching for a peptide fragment comprising a modified lysine residue (an azide-introduced portion (+255.097Da), an amine-introduced portion (+229.106), an azidocarboxylic acid-introduced portion (+240.086), and an amine carboxylic acid-introduced portion (+214.095)) with respect to a trypsin digestion of trastuzumab using Proteome Discoverer (Thermo Fisher Scientific) (Example 84). The horizontal axis indicates an identified lysine residue, and the vertical axis indicates Peptide Spectrum Matches (PSMs). The residue number of the lysine residue on a heavy chain VH domain and a light chain are expressed by the number in the sequence, and the residue number of the lysine residue on heavy chain CH1, CH2, and CH3 domains are expressed by EU numbering.
FIG. 107 is a diagram illustrating determination of heavy chain selectivity of ADC mimic under reduction conditions by ESI-TOFMS analysis (Example 85).
FIG. 108 is a diagram illustrating HIC-UPLC analysis of specific modification of azide-introduced trastuzumab (detection wavelength: UV 280 nm) (Example 85).
FIG. 109 is a diagram of an MS spectrum (measured value: m/z 1055.77631; theoretical value: 1055.77600; and tetravalent) of a peptide fragment of the peptide THTCPPCPAPELLGGPSVFLFPPKPKDTLMISR (SEQ ID NO: 6) consisting of 33 amino acid residues comprising a modified site to a lysine residue by trypsin digestion of trastuzumab (a DBCO-Acid carboxylic acid-introduced portion (+559.207)) (Example 85).
FIG. 110-1 is a diagram illustrating a CID spectrum of a product ion of m/z 971.71 (theoretical value: 971.50) corresponding to divalent y12, indicating modification of a lysine residue at position 246 or 248 in EU numbering (Example 85).

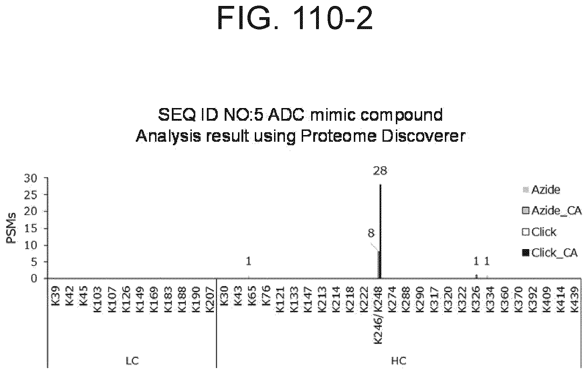

FIG. 110-2 is a diagram illustrating a result of searching for a peptide fragment comprising a modified lysine residue (a DBCO-Acid-introduced portion (+574.218), a DBCO-Acid carboxylic acid-introduced portion (+559.207), an azide-introduced portion (+255.097), and an azidocarboxylic acid-introduced portion (+240.086)) with respect to a trypsin digestion of trastuzumab using Proteome Discoverer (Thermo Fisher Scientific) (Example 85). The horizontal axis indicates an identified lysine residue, and the vertical axis indicates Peptide Spectrum Matches (PSMs). The residue number of the lysine residue on a heavy chain VH domain and a light chain are expressed by the number in the sequence, and the residue number of the lysine residue on heavy chain CH1, CH2, and CH3 domains are expressed by EU numbering.
FIG. 111 is a diagram illustrating specific modification of an anti-TNF-α IgG1 antibody adalimumab and analysis by ESI-TOFMS (Example 86).
FIG. 112 is a diagram illustrating HIC-UPLC analysis of specific modification of adalimumab (detection wavelength: UV 280 nm) (Example 86).
FIG. 113 is a diagram illustrating linker cleavage of an adalimumab-peptide conjugate (Example 86).
FIG. 114 is a diagram illustrating HIC-UPLC analysis of thiol-introduced adalimumab (detection wavelength: UV 280 nm) (Example 86).
FIG. 115 is a diagram illustrating fluorescent labeling of thiol-introduced adalimumab (Example 86).
FIG. 116 is a diagram illustrating specific modification of an anti-RANKL IgG2 antibody denosumab and analysis by ESI-TOFMS (Example 87).
FIG. 117 is a diagram illustrating HIC-UPLC analysis of specific modification of denosumab (detection wavelength: UV 280 nm) (Example 87).
FIG. 118 is a diagram illustrating linker cleavage of a denosumab-peptide conjugate (Example 87).
FIG. 119 is a diagram illustrating HIC-UPLC analysis of thiol-introduced denosumab (detection wavelength: UV 280 nm) (Example 87).
FIG. 120 is a diagram illustrating fluorescent labeling of thiol-introduced denosumab (Example 87).
FIG. 121 is a diagram illustrating specific modification of an anti-IL-4/13 receptor IgG4 antibody dupilumab and analysis by ESI-TOFMS (Example 88).
FIG. 122 is a diagram illustrating HIC-UPLC analysis of specific modification of dupilumab (detection wavelength: UV 280 nm) (Example 88).
FIG. 123 is a diagram illustrating linker cleavage of a dupilumab-peptide conjugate (Example 88).
FIG. 124 is a diagram illustrating HIC-UPLC analysis of thiol-introduced dupilumab (detection wavelength: UV 280 nm) (Example 88).
FIG. 125 is a diagram illustrating determination of heavy chain selectivity of fluorescently labeled dupilumab under reduction conditions by ESI-TOFMS analysis (Example 88) .

### EMBODIMENTS FOR CARRYING OUT THE INVENTION

### 1. Compound Having Affinity Substance to Antibody, Cleavable Portion, and Reactive Group, or Salt thereof

### 1-1. Outline

The present invention provides a compound having an affinity substance to an antibody, a cleavable portion, and a reactive group, represented by Formula (I), or a salt thereof.

A-L-B-R (I)

wherein
A is the affinity substance to an antibody,
L is a cleavable linker which is a divalent group comprising the cleavable portion,
B is (a) a divalent group comprising a bioorthogonal functional group or (b) a divalent group comprising no bioorthogonal functional group, and
R is the reactive group to the antibody.

In Formula (I) and other formulae presented in relation to the present invention, - (a hyphen) indicates that two units present on both sides thereof covalently bind to each other. Consequently, in Formula (I), A covalently binds to L, L covalently binds to A and B, B covalently binds to L and R, and R covalently binds to B.

### 1-2. Affinity Substance to Antibody (A)

In Formula (I), A is an affinity substance to an antibody. The affinity substance is a substance having binding ability through a noncovalent bond to an antibody.

The affinity substance used in the present invention targets an antibody. The antibody may be a protein modified with a biomolecule (e.g., a sugar) (e.g., a glycoprotein) or a protein unmodified with a biomolecule. As the antibody, any antibody to any component such as a bio-derived component, a virus-derived component, or a component found in an environment can be used, but an antibody to a bio-derived component or a virus-derived component is preferable. Examples of the bio-derived component include components derived from animals such as mammals and birds (e.g., chickens), insects, microorganisms, plants, fungi, and fishes (e.g., protein). The bio-derived component is preferably a component derived from mammals. Examples of the mammals include primates (e.g., humans, monkeys, and chimpanzees), rodents (e.g., mice, rats, guinea pigs, hamsters, and rabbits), pets (e.g., dogs and cats), domestic animals (e.g., cows, pigs, and goats), and work animals (e.g., horses and sheep). The bio-derived component is more preferably a component derived from primates or rodents (e.g., protein), and even more preferably a human-derived component (e.g., protein) in view of the clinical application of the present invention. Examples of the virus-derived component include components derived from influenza viruses (e.g., avian influenza viruses and swine influenza viruses), AIDS virus, Ebola virus, and phage viruses (e.g., protein).

The antibody is a polyclonal antibody or a monoclonal antibody, and is preferably a monoclonal antibody. Examples of the monoclonal antibody include chimeric antibodies, humanized antibodies, human antibodies, antibodies with a certain sugar chain added (e.g., an antibody modified so as to have a sugar chain-binding consensus sequence such as an N-type sugar chain-binding consensus sequence), bi-specific antibodies, scFv antibodies, Fab antibodies, F(ab')₂ antibodies, VHH antibodies, Fc region proteins, and Fc-fusion proteins. The antibody may be a divalent antibody (e.g., IgG, IgD, or IgE) or a tetravalent or higher antibody (e.g., IgA antibody or IgM antibody).

The antibody as a target of the affinity substance may comprise any amino acid residues and preferably comprises 20 natural L-α-amino acid residues normally contained in proteins. Examples of such amino acid residues include L-alanine (A), L-asparagine (N), L-cysteine (C), L-glutamine (Q), L-isoleucine (I), L-leucine (L), L-methionine (M), L-phenylalanine (F), L-proline (P), L-serine (S), L-threonine (T), L-tryptophan (W), L-tyrosine (Y), L-valine (V), L-aspartic acid (D), L-glutamic acid (E), L-arginine (R), L-histidine (H), L-lysine (K), and glycine (G) (hereinafter, the expression of L is omitted). The antibody may comprise e.g., 100 or more, preferably 120 or more, more preferably 150 or more, even more preferably 180 or more, and particularly preferably 200 or more amino acid residues. The antibody may comprise e.g., 1,000 or less, preferably 900 or less, more preferably 800 or less, even more preferably 700 or less, and particularly preferably 600 or less amino acid residues. More specifically, the antibody may comprise e.g., 100 to 1,000, preferably 120 to 900, more preferably 150 to 800, even more preferably 180 to 700, and particularly preferably 200 to 600 amino acid residues. When the antibody is an antibody (e.g., the monoclonal antibody described above), the above number of amino acid residues may correspond to amino acid residues of a heavy chain of the antibody.

The antibody as the target of the affinity substance is further a protein comprising specific amino acid residues having a side chain or a terminal (an N-terminal and/or a C-terminal), preferably a side chain, with which a reactive group described below is capable of reacting at one position or a plurality of positions (preferably a plurality of positions). Examples of such specific amino acid residues include 14 amino acid residues described below; preferred are amino acid residues selected from the group consisting of a lysine residue, a tyrosine residue, a tryptophan residue, and a cysteine residue. Considering that the compound of the present invention can regioselectively modify an antibody, preferred is an antibody comprising such specific amino acid residues at a plurality of positions. The positions are not limited to particular positions so long as they are two or more positions and may be e.g., three or more positions, preferably five or more positions, more preferably ten or more positions, even more preferably 20 or more positions, and particularly preferably 30 or more positions. The positions may be e.g., 200 or less positions, preferably 180 or less positions, more preferably 150 or less positions, even more preferably 120 or less positions, and particularly preferably 100 or less positions. More specifically, the positions may be e.g., 3 to 200 positions, preferably 5 to 180 positions, more preferably 10 to 150 positions, even more preferably 20 to 120 positions, and particularly preferably 30 to 100 positions. Even for an antibody comprising such specific amino acid residues at a plurality of positions, the compound of the present invention can regioselectively modify a specific amino acid residue present at one specific position. It is said that the number of lysine residues of human IgG1 is generally about 70 to 90, for example, although it depends on an amino acid composition in a variable region. The present invention has succeeded in regioselectively modifying such lysine residues present at specific positions of human IgG1.

More specifically, in the present invention, in view of, while maintaining the function of an antibody (that is, while maintaining native folding without denaturing the antibody), modifying amino acid residues present at specific positions in the antibody, preferred is regioselective modification of amino acid residues exposed to the surface of the antibody. In human IgG such as human IgG1, for example, exposed lysine residues and exposed tyrosine residues are present at the following positions (refer to http://www.imgt.org/IMGTScientificChart/Numbering/Hu_IGHGnb er.html by EU numbering). In the present application, for convenience of explanation, the residue number on heavy chain CH1, CH2, and CH3 domains may be expressed by EU numbering, and the residue number on a heavy chain VH domain and a light chain may be expressed by the number in the sequence (that is, the N-terminal amino acid is the first).

### (1) Exposed lysine residues

CH2 domain (position 246, position 248, position 274, position 288, position 290, position 317, position 320, position 322, and position 338)
CH3 domain (position 360, position 414, and position 439)

### (2) Exposed tyrosine residues

CH2 domain (position 278, position 296, and position 300)
CH3 domain (position 436)
Consequently, when human IgG such as human IgG1 is modified with a lysine residue or a tyrosine residue, modification at the above positions is preferred.

When human IgG such as human IgG1 is modified with a lysine residue or a tyrosine residue, among the positions of (1) and (2), lysine residues or tyrosine residues present at the following positions, which are high in the degree of exposure to the surface, may be preferably modified.

### (1') Exposed lysine residues

CH2 domain (position 246, position 248, position 274, position 288, position 290, position 317, position 320, and position 322)
CH3 domain (position 360, position 414, and position 439)

### (2') Exposed tyrosine residues

CH2 domain (position 278, position 296, and position 300)
CH3 domain (position 436)

Consequently, when human IgG such as human IgG1 is modified with a lysine residue or a tyrosine residue, modification at the above positions is more preferred.

When human IgG such as human IgG1 is modified with a lysine residue, among the positions of (1), lysine residues present at certain positions (e.g., position 246, position 248, position 288, position 290, and position 317) in the CH2 domain, which can be efficiently modified in the present invention, may be more preferably modified.

In a specific embodiment, the antibody as the target of the affinity substance, when comprising the specific amino acid residues at a plurality of positions as described above, may comprise one or more specific amino acid residues in a target region consisting of 1 to 50 consecutive amino acid residues and comprise five or more of the specific amino acid residues in a non-target region other than the target region. The target region may consist of preferably 1 to 30, more preferably 1 to 20, and even more preferably one to ten, one to five, or one to three (that is, one, two, or three) amino acid residues. The target region may be particularly preferably a region consisting of a specific amino acid residue present at a specific position. Such a specific position, which varies depending on the types of the target protein and the affinity substance and the like, may be e.g., a specific position in a specific region of a constant region of an antibody (e.g., CH1, CH2, and CH3) and preferably a position in CH2 of an antibody. The target region may be more specifically the following residues following Eu numbering in human IgG Fc: (1) a Lys248 residue (hereinafter, also referred to simply as "Lys248" in the present specification and corresponding to the 18th residue in a human IgG CH2 region (SEQ ID NO: 1)) or a Lys246 residue (hereinafter, also referred to simply as "Lys246" in the present specification and corresponding to the 16th residue in the human IgG CH2 region (SEQ ID NO: 1)); (2) a Lys288 residue (hereinafter, also referred to simply as "Lys288" in the present specification and corresponding to the 58th residue in the human IgG CH2 region (SEQ ID NO: 1)) or a Lys290 residue (hereinafter, also referred to simply as "Lys290" in the present specification and corresponding to the 60th residue in the human IgG CH2 region (SEQ ID NO: 1)); and (3) a Lys317 residue (hereinafter, also referred to simply as "Lys317" in the present specification and corresponding to the 87th residue in the human IgG CH2 region (SEQ ID NO: 1)).

The present invention can modify the specific amino acid residue in the target region highly regioselectively. Such regioselectivity may be e.g., 30% or more, preferably 40% or more, more preferably 50% or more, even more preferably 60% or more, and particularly preferably 70% or more, 80% or more, 90% or more, 95% or more, 96% or more, 97% or more, 98% or more, 99% or more, or 100% or more.

The target region does not necessarily comprise the same kind of amino acid residue as the specific amino acid residue other than the specific amino acid residue present at the specific position in a region up to a remote position of "a" (where "a" is any integer of 1 to 10) amino acid residues to an N-terminal side and a C-terminal side each with respect to the specific amino acid present at the specific position. The symbol "a" is preferably an integer of 1 to 5, more preferably an integer of 1 to 3, even more preferably 1 or 2, and particularly preferably 1.

In a preferred embodiment, the antibody is a monoclonal antibody. Examples of the isotype of the monoclonal antibody and the like include IgG (e.g., IgG1, IgG2, IgG3, and IgG4), IgM, IgA, IgD, IgE, and IgY. The monoclonal antibody is a full-length antibody or an antibody fragment (e.g., F(ab')₂, Fab', Fab, Fv, and a single-chain antibody); the full-length antibody is preferred.

The antibody is an antibody to any antigen. Such an antigen may be a component found in organisms and viruses described above, for example. Examples of such an antigen include a protein [comprising an oligopeptide and a polypeptide, which may be a protein modified with a biomolecule such as sugar (e.g., glycoprotein)], a sugar chain, a nucleic acid, and a small compound.

The antibody may be preferably an antibody with a protein as an antigen. Examples of the protein include cell membrane receptors, cell membrane proteins other than cell membrane receptors (e.g., extracellular matrix proteins), ligands, and soluble receptors.

More specifically, the protein as the antigen of the antibody may be a disease target protein. Examples of the disease target protein include the following.

### (1) Cancerous Region

PD-L1, GD2, PDGFRα (a platelet-derived growth factor receptor), CD22, HER2, phosphatidyl serine (PS), EpCAM, fibronectin, PD-1, VEGFR-2, CD33, HGF, gpNMB, CD27, DEC-205, folic acid receptors, CD37, CD19, Trop2, CEACAM5, S1P, HER3, IGF-1R, DLL4, TNT-1/B, CPAAs, PSMA, CD20, CD105 (Endoglin), ICAM-1, CD30, CD16A, CD38, MUC1, EGFR, KIR2DL1, KIR2DL2, NKG2A, tenascin-C, IGF (insulin-like growth factor), CTLA-4, mesothelin, CD138, c-Met, Ang2, VEGF-A, CD79b, ENPD3, folic acid receptor α, TEM-1, GM2, Glypican 3, macrophage inhibitory factor, CD74, Notch1, Notch2, Notch3, CD37, TLR-2, CD3, CSF-1R, FGFR2b, HLA-DR, GM-CSF, EphA3, B7-H3, CD123, gpA33, Frizzled7 receptor, DLL4, VEGF, RSPO, LIV-1, SLITRK6, Nectin-4, CD70, CD40, CD19, SEMA4D (CD100), CD25, MET, Tissue Factor, IL-8, EGFR, cMet, KIR3DL2, Bst1 (CD157), P-Cadherin, CEA, GITR, TAM (tumor associated macrophage), CEA, DLL4, Ang2, CD73, FGFR2, CXCR4, LAG-3, GITR, Fucosyl GM1, IGF-1, Angiopoietin 2, CSF-1R, FGFR3, OX40, BCMA, ErbB3, CD137 (4-1BB), PTK7, EFNA4, FAP, DR5, CEA, Ly6E, CA6, CEACAM5, LAMP1, tissue factor, EPHA2, DR5, B7-H3, FGFR4, FGFR2, α2-PI, A33, GDF15, CAIX, CD166, ROR1, GITR, BCMA, TBA, LAG-3, EphA2, TIM-3, CD-200, EGFRvIII, CD16A, CD32B, PIGF, Axl, MICA/B, Thomsen-Friedenreich, CD39, CD37, CD73, CLEC12A, Lgr3, transferrin receptors, TGFβ, IL-17, 5T4, RTK, Immune Suppressor Protein, NaPi2b, Lewis blood group B antigen, A34, Lysil-Oxidase, DLK-1, TROP-2, α9 Integrin, TAG-72 (CA72-4), and CD70.

### (2) Autoimmune Diseases and Inflammatory Diseases

IL-17, IL-6R, IL-17R, INF-α, IL-5R, IL-13, IL-23, IL-6, ActRIIB, β7-Integrin, IL-4αR, HAS, Eotaxin-1, CD3, CD19, TNF-α, IL-15, CD3ε, Fibronectin, IL-1β, IL-1α, IL-17, TSLP (Thymic Stromal Lymphopoietin), LAMP (Alpha4 Beta 7 Integrin), IL-23, GM-CSFR, TSLP, CD28, CD40, TLR-3, BAFF-R, MAdCAM, IL-31R, IL-33, CD74, CD32B, CD79B, IgE (immunoglobulin E), IL-17A, IL-17F, C5, FcRn, CD28, TLR4, MCAM, B7RP1, CXCR1/2 Ligands, IL-21, Cadherin-11, CX3CL1, CCL20, IL-36R, IL-10R, CD86, TNF-α, IL-7R, Kv1.3, α9 Integrin, and LIFHT.

### (3) Brain or Nerve Diseases

CGRP, CD20, β amyloid, β amyloid protofibril, Calcitonin Gene-Related Peptide Receptor, LINGO (Ig Domain Containing 1), α Synuclein, extracellular tau, CD52, insulin receptors, tau protein, TDP-43, SOD1, TauC3, and JC virus.

### (4) Infectious Diseases

Clostridium Difficile toxin B, cytomegalovirus, RS viruses, LPS, S. Aureus Alpha-toxin, M2e protein, Psl, PcrV, S. Aureus toxin, influenza A, Alginate, Staphylococcus aureus, PD-L1, influenza B, Acinetobacter, F-protein, Env, CD3, enteropathogenic Escherichia coli, Klebsiella, and Streptococcus pneumoniae.

### (5) Hereditary Rare Diseases

amyloid AL, SEMA4D (CD100), insulin receptors, ANGPTL3, IL4, IL13, FGF23, adrenocorticotropic hormone, transthyretin, and huntingtin.

### (6) Eye Diseases

Factor D, IGF-1R, PGDFR, Ang2, VEGF-A, CD-105 (Endoglin), IGF-1R, and β amyloid.

### (7) Bone and Orthopedic Region

Sclerostin, Myostatin, Dickkopf-1, GDF8, RNAKL, HAS, Siglec-15

### (8) Blood Diseases

vWF, Factor IXa, Factor X, IFNγ, C5, BMP-6, Ferroportin, TFPI

### (9) Other Diseases

BAFF (B cell activating factor), IL-1β, PCSK9, NGF, CD45, TLR-2, GLP-1, TNFR1, C5, CD40, LPA, prolactin receptors, VEGFR-1, CB1, Endoglin, PTH1R, CXCL1, CXCL8, IL-1β, AT2-R, and IAPP.

In a more preferred embodiment, the affinity substance to an antibody is an affinity substance to a monoclonal antibody. The isotype of the monoclonal antibody is similar to those described above for the antibody; IgG (e.g., IgG1, IgG2, IgG3, and IgG4) is preferred. The monoclonal antibody is preferably a full-length monoclonal antibody.

In an even more preferred embodiment, the affinity substance to an antibody is an affinity substance to a chimeric antibody, a humanized antibody, or a human antibody (e.g., IgG including IgG1, IgG2, IgG3, and IgG4) as a full-length monoclonal antibody.

In a particularly preferred embodiment, the affinity substance to an antibody is an affinity substance to an antibody, comprising any one Fc region protein selected from the group consisting of the following (A) to (C) and having antigen-binding ability:
(A) an Fc region protein comprising the amino acid sequence of SEQ ID NO: 1;
(B) an Fc region protein comprising an amino acid sequence with one or several amino acid residues inserted, added, deleted, or substituted in the amino acid sequence of SEQ ID NO: 1; and
(C) an Fc region protein comprising an amino acid sequence having 90% or more identity to the amino acid sequence of SEQ ID NO: 1.

The amino acid sequence of SEQ ID NO: 1 is an Fc region protein. It is known that such an Fc region protein has secretion ability. Consequently, the Fc region proteins of (A) to (C) can have secretion ability. An antibody comprising such an Fc region protein can have antigen-binding ability. The amino acid residue at position 18 in SEQ ID NO: 1 is any amino acid residue, preferably a neutral amino acid residue, more preferably an amino acid residue having a nonpolar side chain described below, and even more preferably leucine, isoleucine, or alanine, and particularly preferably leucine or alanine. The amino acid residue at position 19 in SEQ ID NO: 1 is any amino acid residue, preferably a neutral amino acid residue or an acidic amino acid residue, more preferably an amino acid residue having a nonpolar side chain or an acidic amino acid residue, and even more preferably leucine or glutamic acid. The amino acid residue at position 21 in SEQ ID NO: 1 is any amino acid residue, preferably a neutral amino acid residue, more preferably an amino acid residue having a nonpolar side chain, and even more preferably glycine or alanine. The amino acid residue at position 140 in SEQ ID NO: 1 is any amino acid residue, preferably an acidic amino acid residue, and more preferably glutamic acid or aspartic acid. The amino acid residue at position 142 in SEQ ID NO: 1 is any amino acid residue, preferably a neutral amino acid residue, more preferably an amino acid residue having a nonpolar side chain, even more preferably methionine, leucine, or isoleucine, and particularly preferably methionine or leucine. The amino acid residue at position 177 in SEQ ID NO: 1 is any amino acid residue, preferably a neutral amino acid residue, more preferably an amino acid residue having an uncharged polar side chain or an amino acid residue having a nonpolar side chain described below, even more preferably threonine, alanine, or glycine, and particularly preferably threonine or alanine.

In a preferred embodiment, the amino acid sequence of SEQ ID NO: 1 may be an amino acid sequence consisting of the amino acid residues at positions 220 to 449 in the amino acid sequence of SEQ ID NO: 2.

In another preferred embodiment, the amino acid sequence of SEQ ID NO: 1 may be an amino acid sequence consisting of the amino acid residues at positions 7 to 236 in the amino acid sequence of SEQ ID NO: 3.

In a specific embodiment, the antibody comprising the Fc region protein comprising the amino acid sequence described above may be an antibody comprising the Fc region protein comprising the amino acid sequence described above and a constant region of an antibody. Such a constant region of an antibody may be a constant region of a chimeric antibody, a humanized antibody, or a human antibody (e.g., IgG including IgG1, IgG2, IgG3, and IgG4).

In (B) the Fc region protein, one or several amino acid residues can be modified by one, two, three, or four variations selected from the group consisting of deletion, substitution, addition, and insertion of amino acid residues. The variations of amino acid residues may be introduced to one region in the amino acid sequence or intruded to a plurality of different regions. The term "one or several" indicates a number that does not significantly impair protein activity. The number indicated by the term "one or several" is e.g., 1 to 100, preferably 1 to 80, more preferably 1 to 50, 1 to 30, 1 to 20, 1 to 10, or 1 to 5 (e.g., one, two, three, four, or five).

In (C) the Fc region protein, the percent identity to the amino acid sequence of SEQ ID NO: 1 may be 70% or more, 75% or more, 80% or more, 85% or more, 90% or more, 91% or more, 92% or more, 93% or more, 94% or more, 95% or more, 96% or more, 97% or more, 98% or more, or 99% or more. In the present invention, calculation of the percent identity of peptides and polypeptides (proteins) can be performed by Algorithm blastp. More specifically, calculation of the percent identity of polypeptides can be performed using Scoring Parameters (Matrix: BLOSUM62; Gap Costs: Existence = 11 Extension = 1; Compositional Adjustments: Conditional compositional score matrix adjustment) as default settings in Algorithm blastp provided in National Center for Biotechnology Information (NCBI). Calculation of the percent identity of polynucleotides (genes) can be performed by Algorithm blastn. More specifically, calculation of the percent identity of polynucleotides can be performed using Scoring Parameters (Match/Mismatch Scores = 1, -2; Gap Costs = Linear) as default settings in Algorithm blastn provided in NCBI.

Secretion in secretion ability has the same meaning as the secretion (what is called solubility) of the secretory protein. Consequently, "having secretion ability" means functioning as an antibody in a manner similar to normal antibodies.

In the antibody comprising the Fc region protein, a variation may be introduced to a specific site so long as target characteristics (e.g., secretion ability and antigen-binding ability) are maintained. The position of an amino acid residue to which a variation may be introduced that can maintain the target characteristics is obvious to a person skilled in the art. Specifically, a person skilled in the art can 1) compare amino acid sequences of a plurality of proteins having homogeneous characteristics with each other, 2) clarify a relatively preserved region and a relatively non-preserved region, and then 3) predict a region capable of playing an important role for a function and a region incapable of playing an important role for a function from the relatively preserved region and the relatively non-preserved region each and can thus recognize structure-function correlation. Consequently, a person skilled in the art can identify the position of an amino acid residue to which a variation may be introduced in the amino acid sequence of the antibody comprising the Fc region protein.

When an amino acid residue is varied by substitution, the substitution of the amino acid residue may be preservative substitution. The term "preservative substitution" when used in the present specification refers to substituting a certain amino acid residue with an amino acid residue having a similar side chain. Families of amino acid residues having a similar side chain are known in the field concerned. Examples of such families include amino acids having a basic side chain (e.g., lysine, arginine, and histidine), amino acids having an acidic side chain (e.g., aspartic acid, and glutamic acid), amino acids having an uncharged polar side chain (e.g., asparagine, glutamine, serine, threonine, tyrosine, and cysteine), amino acids having a nonpolar side chain (e.g., glycine, alanine, valine, leucine, isoleucine, proline, phenylalanine, methionine, and tryptophan), amino acids having a β-position-branched side chain (e.g., threonine, valine, and isoleucine), amino acids having an aromatic side chain (e.g., tyrosine, phenylalanine, tryptophan, and histidine), amino acids having a hydroxy group (e.g., alcoholic and phenolic)-containing side chain (e.g., serine, threonine, tyrosine), and amino acids having a sulfur-containing side chain (e.g., cysteine and methionine). The preservative substitution of the amino acid may be preferably substitution between aspartic acid and glutamic acid, substation among arginine, lysine, and histidine, substitution between tryptophan and phenylalanine, substitution between phenylalanine and valine, substitution among leucine, isoleucine, and alanine, and substitution between glycine and alanine.

Examples of the antibody comprising any one Fc region selected from the group consisting of (A) to (C) include chimeric antibodies (e.g., rituximab, basiliximab, infliximab, cetuximab, siltuximab, dinutuximab, and altertoxaximab), humanized antibodies (e.g., daclizumab, palivizumab, trastuzumab, alemtuzumab, omalizumab, efalizumab, bevacizumab, natalizumab (IgG4), tocilizumab, eculizumab (IgG2), mogamulizumab, pertuzumab, obinutuzumab, vedolizumab, pembrolizumab (IgG4), mepolizumab, elotuzumab, daratumumab, ixekizumab (IgG4), reslizumab (IgG4), and atezolizumab), and human antibodies (e.g., adalimumab (IgG1), panitumumab, golimumab, ustekinumab, canakinumab, ofatumumab, denosumab (IgG2), ipilimumab, belimumab, raxibacumab, ramucirumab, nivolumab, dupilumab (IgG4), secukinumab, evolocumab (IgG2), alirocumab, necitumumab, brodalumab (IgG2), and olaratumab) (cases not referring to the IgG subtype indicate that they are IgG1).

The affinity substance to an antibody used in the present invention is a peptide comprising any of the following amino acid sequences.
Formula 1-1: (X₀₋₃)ₐ-C-Xaa1-Xaa2-Xaa3-Xaa4-Xaa5-Xaa6-I-I-W-C- (X₀₋₃)_{b}(SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b}(SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
   wherein
   (X₀₋₃)ₐ is absent or one to three consecutive arbitrary amino acid residues (other than a lysine residue and a cysteine residue) which are the same or different,
   (X₀₋₃)_{b} is absent or one to three consecutive arbitrary amino acid residues (other than a lysine residue and a cysteine residue) which are the same or different,
   Xaa1 is an alanine residue, a glycine residue, a leucine residue, a proline residue, an arginine residue, a valine residue, an asparagine residue, a glutamic acid residue, or a phenylalanine residue,
   Xaa2 is a tyrosine residue, a tryptophan residue, a histidine residue, or a phenylalanine residue,
   Xaa3 is a histidine residue, a phenylalanine residue, a tyrosine residue, a tryptophan residue, an arginine residue, or a glycine residue,
   Xaa4 is a lysine residue,
   Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
   Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1'-Xaa2'-Xaa3'-Xaa4'-Xaa5'-Xaa6'-L-V-W-C-(X₀₋₃')_{b} (SEQ ID NO: 67)
   wherein
   (X₀₋₃')ₐ and (X₀₋₃')_{b} are the same as the above (X₀₋₃)ₐ and (X₀₋₃)_{b}, respectively, and
   Xaa1', Xaa2', Xaa3', Xaa4', Xaa5', and Xaa6' are the same as the above Xaa1, Xaa2, Xaa3, Xaa4, Xaa5, and Xaa6, respectively,
   except for a case where (X₀₋₃')ₐ is one to three amino acid residues, (X₀₋₃')_{b} is one to three amino acid residues, Xaa3' is a histidine residue, and Xaa5' is a glycine residue.

(X₀₋₃), (X₀₋₃)_{b}, (X₀₋₃')ₐ, and (X₀₋₃')_{b} are each independently absent or one to three consecutive arbitrary amino acid residues (other than a lysine residue and a cysteine residue) which are the same or different. Consequently, such an arbitrary amino acid residue is an alanine residue, an asparagine residue, a glutamine residue, a glycine residue, an isoleucine residue, a leucine residue, a methionine residue, a phenylalanine residue, a proline residue, a serine residue, a threonine residue, a tryptophan residue, a tyrosine residue, a valine residue, an aspartic acid residue, a glutamic acid residue, an arginine residue, or a histidine residue.

In a specific embodiment, (X₀₋₃)ₐ may be absent, an arginine residue-glycine residue-asparagine residue, an aspartic acid residue, an asparagine residue, a glycine residue-asparagine residue, a glycine residue, a glycine residue-glycine residue, or a glycine residue-glycine residue-glycine residue.

In a specific embodiment, (X₀₋₃)_{b} may be absent, a threonine residue-tyrosine residue-histidine residue, a threonine residue, a threonine residue-tyrosine residue, a glycine residue, a glycine residue-glycine residue, or a glycine residue-glycine residue-glycine residue.

In a specific embodiment, in the amino acid sequences represented by the above Formulae 1-1 to 1-9 and 2-1, the following may be possible:
(X₀₋₃)ₐ is absent, an arginine residue-glycine residue-asparagine residue, an aspartic acid residue, or an asparagine residue,
(X₀₋₃)_{b} is absent, a threonine residue-tyrosine residue-histidine residue, or a threonine residue,
Xaa1 is an alanine residue,
Xaa2 is a tyrosine residue, a tryptophan residue, or a histidine residue, and
Xaa6 is a glutamine residue, a glutamic acid residue, an asparagine residue, or an aspartic acid residue.

In another specific embodiment, in the amino acid sequences represented by the above Formulae 1-1 to 1-9 and 2-1, the following may be possible:
(X₀₋₃)ₐ is a glycine residue-asparagine residue, a glycine residue, a glycine residue-glycine residue, or a glycine residue-glycine residue-glycine residue,
(X₀₋₃)_{b} is a threonine residue-tyrosine residue, a glycine residue, a glycine residue-glycine residue, or a glycine residue-glycine residue-glycine residue,
Xaa1 is a glycine residue, a leucine residue, a proline residue, an arginine residue, a valine residue, an asparagine residue, a glutamic acid residue, or a phenylalanine residue,
Xaa2 is a phenylalanine residue, and
Xaa6 is a proline residue, a glycine residue, an arginine residue, a phenylalanine residue, or a histidine residue.

In the amino acid sequences represented by the above formulae 1-1 to 1-9 and formula 2-1, Xaa3 is a histidine residue, a phenylalanine residue, a tyrosine residue, a tryptophan residue, an arginine residue, or a glycine residue, and preferably a histidine residue.

In the amino acid sequences represented by the above formulae 1-1 to 1-9 and formula 2-1, Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and preferably a glycine residue, a threonine residue, or a leucine residue.

The peptide comprising any of the amino acid sequences represented by the above Formulae 1-1 to 1-9 and 2-1 is preferably a peptide comprising an amino acid sequence selected from the group consisting of the following:
(1)RGNCAYHKGQIIWCTYH(SEQ ID NO: 5);
(2)RGNCAYHKGQIVWCTYH(SEQ ID NO: 8);
(3)RGNCAYHKGQVVWCTYH(SEQ ID NO: 9);
(4)RGNCAYHKGQAVWCTYH(SEQ ID NO: 10);
(5)RGNCAYHKGQLLWCTYH(SEQ ID NO: 11);
(6)RGNCAYHKGQLIWCTYH(SEQ ID NO: 12);
(7)DCAYHKGQIVWCT(SEQ ID NO: 13);
(8)DCAYHKGQVVWCT (SEQ ID NO: 14);
(9)DCAYHKGQAVWCT(SEQ ID NO: 15);
(10)RGNCAYHKSQIIWCTYH(SEQ ID NO: 16);
(11)RGNCAYHKNQIIWCTYH(SEQ ID NO: 17);
(12)RGNCAYHKDQIIWCTYH(SEQ ID NO: 18);
(13)RGNCAYHKQQIIWCTYH(SEQ ID NO: 19);
(14)RGNCAYHKEQIIWCTYH(SEQ ID NO: 20);
(15)RGNCAYHKFQIIWCTYH(SEQ ID NO: 21);
(16)RGNCAYHKYQIIWCTYH(SEQ ID NO: 22);
(17)RGNCAYHKWQIIWCTYH(SEQ ID NO: 23);
(18)RGNCAYHKHQIIWCTYH(SEQ ID NO: 24);
(19)RGNCAYHKTQIIWCTYH(SEQ ID NO: 25);
(20)RGNCAYHKLQIIWCTYH(SEQ ID NO: 26);
(21)CAYHKLQIVWC(SEQ ID NO: 27);
(22)CAYHKLQLIWC(SEQ ID NO: 28);
(23)CAYHKSQIVWC(SEQ ID NO: 29);
(24)RGNCAYHKGQLVFCTYH(SEQ ID NO: 30);
(25)RGNCAYHKGQQVWCTYH(SEQ ID NO: 31);
(26)RGNCAYHKGQEVWCTYH(SEQ ID NO: 32);
(27)CAYHKGQLVWC(SEQ ID NO: 33);
(28)RGNCAYHKAQLVWCTYH(SEQ ID NO: 34);
(29)RGNCAYHKVQLVWCTYH(SEQ ID NO: 35);
(30)RGNCAYHKLQLVWCTYH(SEQ ID NO: 36);
(31)RGNCAYHKIQLVWCTYH(SEQ ID NO: 37);
(32)RGNCAYHKSQLVWCTYH(SEQ ID NO: 38);
(33)RGNCAYHKTQLVWCTYH(SEQ ID NO: 39);
(34)RGNCAYHKNQLVWCTYH(SEQ ID NO: 40);
(35)RGNCAYHKDQLVWCTYH(SEQ ID NO: 41);
(36)RGNCAYHKQQLVWCTYH(SEQ ID NO: 42);
(37)RGNCAYHKEQLVWCTYH(SEQ ID NO: 43);
(38)RGNCAYHKFQLVWCTYH(SEQ ID NO: 44);
(39)RGNCAYHKRQLVWCTYH(SEQ ID NO: 45);
(40)RGNCAYHKHQLVWCTYH(SEQ ID NO: 46);
(41)RGNCAYHKWQLVWCTYH(SEQ ID NO: 47);
(42)RGNCAYHKYQLVWCTYH(SEQ ID NO: 48);
(43)RGNCAYFKGQLVWCTYH(SEQ ID NO: 49);
(44)RGNCAYYKGQLVWCTYH(SEQ ID NO: 50);
(45)RGNCAYWKGQLVWCTYH(SEQ ID NO: 51);
(46)RGNCAYRKGQLVWCTYH(SEQ ID NO: 52);
(47)RGNCAYGKGQLVWCTYH(SEQ ID NO: 53);
(48)DCAYHKGQLVWC(SEQ ID NO: 54);
(49)NCAYHKGQLVWC(SEQ ID NO: 55);
(50)CAYHKGQLVWCT(SEQ ID NO: 56);
(51)CAYHKSQLVWC(SEQ ID NO: 57);
(52)RGNCAWHKGQIIWCTYH(SEQ ID NO: 68);
(53)RGNCAFHKGQIIWCTYH(SEQ ID NO: 69);
(54)RGNCAHHKGQIIWCTYH(SEQ ID NO: 70);
(55)RGNCGYHKGQIIWCTYH(SEQ ID NO: 71);
(56)RGNCLYHKGQIIWCTYH(SEQ ID NO: 72);
(57)RGNCPYHKGQIIWCTYH(SEQ ID NO: 73);
(58)RGNCRYHKGQIIWCTYH(SEQ ID NO: 74);
(59)RGNCVYHKGQIIWCTYH(SEQ ID NO: 75);
(60)RGNCNYHKGQIIWCTYH(SEQ ID NO: 76);
(61)RGNCEYHKGQIIWCTYH(SEQ ID NO: 77);
(62)RGNCFYHKGQIIWCTYH(SEQ ID NO: 78);
(63)RGNCAYHKGEIIWCTYH(SEQ ID NO: 79);
(64)RGNCAYHKGNIIWCTYH(SEQ ID NO: 80);
(65)RGNCAYHKGPIIWCTYH(SEQ ID NO: 81);
(66)RGNCAYHKGGIIWCTYH(SEQ ID NO: 82);
(67)RGNCAYHKGDIIWCTYH(SEQ ID NO: 83);
(68)RGNCAYHKGRIIWCTYH(SEQ ID NO: 84);
(69)RGNCAYHKGFIIWCTYH(SEQ ID NO: 85);
(70)RGNCAYHKGHIIWCTYH(SEQ ID NO: 86);
(71)DCAYHKGQIIWCT(SEQ ID NO: 87);
(72)NCAYHKGQIIWCT(SEQ ID NO: 88);
(73)GNCAYHKGQIIWCTY(SEQ ID NO: 89);
(74)GCAYHKGQIIWCG(SEQ ID NO: 90);
(75)GGCAYHKGQIIWCGG(SEQ ID NO: 91); and
(76)GGGCAYHKGQIIWCGGG(SEQ ID NO: 92).

At least two cysteine residues separated from each other in each amino acid sequence of the peptide can form a cyclic peptide through a disulfide bond. Alternatively, in the peptide, the sulfide groups in the two cysteine residues may be coupled with each other through a carbonyl group-containing linker represented by the following.

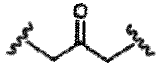

The broken line portions of the carbonyl group-containing linker represented by the above mean bond portions with the sulfide groups. The linker is more stable than a normal disulfide bond against a reduction reaction and the like. Such a peptide can be prepared by a method described in WO 2016/186206, for example.

The novel peptide having such a specific structure is useful for regioselective modification of the Lys248 residue or the Lys246 residue or other amino acid residues other than the Lys248 residue or the Lys246 residue following Eu numbering in human IgG Fc (Examples). An amino acid forming the peptide may each be an L-body or a D-body; an L-body is preferred (in Examples, the amino acid residues forming the peptides are all L-bodies).

The peptide may have a specific amino acid residue modified with a cross-linking agent. Examples of such a specific amino acid residue include a lysine residue, an aspartic acid residue, and a glutamic acid residue; preferred is a lysine residue. Examples of the cross-linking agent include cross-linking agents comprising preferably two or more succinimidyl groups such as disuccinimidyl glutarate (DSG) and disuccinimidyl suberate (DSS); cross-linking agents comprising preferably two or more imide acid portions such as dimethyl adipimidate·2HCl (DMA), dimethyl pimelimidate·2HCl (DMP), and dimethyl suberimidate·2HCl (DMS); and cross-linking agents having an SS bond such as dimethyl 3,3'-dithiobispropionimidate·2HCl (DTBP) and dithiobis(succinimidyl propionate) (DSP) (e.g., WO 2016/186206).

A terminal amino group and a terminal carboxy group of the peptide may be protected. Examples of a protecting group for the N-terminal amino group include an alkylcarbonyl group (an acyl group) (e.g., an acetyl group, a propoxy group, and a butoxycarbonyl group such as a tert-butoxycarbonyl group), an alkyloxycarbonyl group (e.g., a fluorenylmethoxycarbonyl group), an aryloxycarbonyl group, and an arylalkyl(aralkyl)oxycarbonyl group (e.g., a benzyloxycarbonyl group). The protecting group for the N-terminal amino group is preferably an acetyl group. Examples of a protecting group for the C-terminal carboxy group include a group capable of forming an ester or an amide. Examples of the group capable of forming an ester or an amide include an alkyloxy group (e.g., methyloxy, ethyloxy, propyloxy, butyloxy, pentyloxy, and hexyloxy), an aryloxy group (e.g., phenyloxy and naphthyloxy), an aralkyloxy group (e.g., benzyloxy), and an amino group. The protecting group for the C-terminal carboxy group is preferably an amino group.

### 1-3. Linker (L)

In Formula (I), L is a cleavable linker which is a divalent group comprising a cleavable portion.

The cleavable linker represented by L is a divalent group comprising a cleavable portion. The cleavable portion is a site cleavable by specific treatment under a condition incapable of causing denaturation or decomposition (e.g., cleavage of an amide bond) of proteins (a mild condition). Consequently, it can be said that the cleavable portion is a site cleavable by specific cleaving treatment under a mild condition (a bond other than the amide bond). Examples of such specific treatment include (a) treatment with one or more substances selected from the group consisting of an acidic substance, a basic substance, a reducing agent, an oxidizing agent, and an enzyme, (b) treatment by physicochemical stimulus selected from the group consisting of light, and (c) being left when a cleavable linker comprising a self-decomposing cleavable portion is used. Such a cleavable linker and a cleavage condition thereof are a common technical knowledge in the field concerned (e.g., G. Leriche, L. Chisholm, A. Wagner, Bioorganic & Medicinal Chemistry 20,571 (2012); Feng P. et al., Journal of American Chemical Society. 132,1500 (2010); Bessodes M. et al., Journal of Controlled Release, 99,423 (2004); DeSimone, J. M., Journal of American Chemical Society 132,17928 (2010); Thompson, D. H., Journal of Controlled Release, 91,187 (2003); and Schoenmarks, R. G., Journal of Controlled Release, 95,291 (2004)). Examples of such a cleavable portion include a disulfide residue, an acetal residue, a ketal residue, an ester residue, a carbamoyl residue, an alkoxyalkyl residue, an imine residue, a tertiary alkyloxy carbamate residue (e.g., a tert-butyloxy carbamate residue), a silane residue, a hydrazone-containing residue (e.g., a hydrazone residue, an acyl hydrazone residue, and a bisaryl hydrazone residue), a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.

The aromatic ring group having an electron-withdrawing group preferably has an aromatic ring group selected from the group consisting of aryl, aralkyl, an aromatic heterocyclic group, and alkyl having an aromatic heterocyclic group and more preferably aralkyl and alkyl having an aromatic heterocyclic group. The electron-withdrawing group preferably binds to the 2-position of the ring. The aromatic ring-containing residue having an electron-withdrawing group is even more preferably aralkyl having an electron-withdrawing group at the 2-position thereof (e.g., benzyl), for example. Examples of the electron-withdrawing group include a halogen atom, halogen atom-substituted alkyl (e.g., trifluoromethyl), a boronic acid residue, mesyl, tosyl, triflate, nitro, cyano, a phenyl group, and a keto group (e.g., acyl).

The definitions, examples, and preferred examples of groups such as alkyl, acyl (that is, alkylcarbonyl), alkoxy (that is, alkyloxy), aryl, and aralkyl found as terms such as a prefix and a suffix in relation to the names of the residues as the cleavable portion are similar to those described below.

Examples of the ester residue include normal ester residues comprising carbon atoms and oxygen atoms [e.g., alkyl esters (e.g., tertiary alkyl oxycarbonyls such as tert-butyl oxycarbonyl), aryl eaters (e.g., phenacyl ester, 2-(diphenylphosphino)benzoate)], a glycosyl ester residue, an orthoester residue], ester residues comprising a sulfur atom and an oxygen atom (e.g., thioester residues such as an α-thiophenyl ester residue and an alkyl thioester residue), ester residues comprising a phosphorous atom and an oxygen atom (e.g., a phosphodiester residue and a phosphotriester residue), and an activated ester residue (e.g., an N-hydroxysuccinimide residue).

Examples of the sulfone-containing residue include a sulfone residue and a quinolinyl benzenesulfonate residue.

The silane residue is preferably a silane residue having a group selected from the group consisting of alkyl, aryl, aralkyl, and alkoxy. Examples of such a silane residue include a dialkyldialkoxysilane residue (e.g., dimethyldialkoxysilane and diethyldialkoxysilane) and a diaryldialkoxysilane residue (e.g., diphenyldialkoxysilane).

The alkoxyalkyl (that is, alkyloxyalkyl) residue is a group obtained by combining alkyloxy and alkyl described below (the definitions, examples, and preferred examples of alkyloxy and alkyl are similar to those described below); examples thereof include, but are not limited to, a methoxymethyl residue, an ethoxymethyl residue, a methoxyethyl residue, and an ethoxyethyl residue.

The unsaturated bond-containing chain residue is a residue comprising an unsaturated bond portion consisting of only carbon atoms (e.g., vinyl (ethenyl) as the minimum unit having a carbon-carbon double bond or acetylenyl (ethynyl) as the minimum unit having a carbon-carbon triple bond) or a residue comprising an unsaturated bond portion consisting of a carbon atom and a hetero atom (e.g., a nitrogen atom, a sulfur atom and an oxygen atom) (e.g., aldehyde and cyano). Examples of the unsaturated bond-containing chain residue include a vinyl ether residue, a cyanoethyl residue, an ethylene residue, and a malondialdehyde residue.

Examples of the acidic substance (also referred to as an electrophilic reagent) include inorganic acidic substances such as hydrochloric acid, sulfuric acid, and nitric acid; and organic acidic substances such as formic acid, acetic acid, 4-(2-hydroxyethyl)-1-piperazinepropane sulfonic acid, 3-morpholinopropane sulfonic acid, sodium dihydrogenphosphate, citric acid, dodecyl sulfuric acid, N-dodecanoyl sarcosine acid, and trifluoroacetic acid. Examples of a site cleavable with the acidic substance include an alkyloxyarylalkyl residue, a tertiary alkyloxy carbamate residue, an acetal residue, a silane residue, an imine residue, a vinyl ether residue, a β-thiopropionate residue, a trityl residue, a hydrazone residue, an aconityl residue, an orthoester residue, a carbamoyl residue, a 2-(diphenylphosphino)benzoate residue.

Examples of the basic substance (also referred to as a nucleophilic reagent) include inorganic basic substances such as sodium hydroxide, potassium hydroxide, sodium acetate, potassium acetate, and ammonium acetate; and organic basis substances such as hydroxyamine, triethylamine, and N,N'-diisopropylamine. Examples of a site cleavable with the basic substance include a silane residue, a cyanoethyl residue, a sulfone residue, an ethylene residue, a glycosyl disuccinate residue, an α-thiophenyl ester residue, an unsaturated vinylsulfide residue, a malondialdehyde residue, an acylhydrazone residue, and an alkyl thioester residue.

Examples of the reducing agent include cysteine, dithiothreitol, reduced glutathione, and β-mercaptoethanol. Examples of a site cleavable with the reducing agent include a disulfide residue, an alkoxyalkyl residue, and an azo residue.

Examples of the oxidizing agent include sodium periodate and oxidized glutathione. Examples of a site cleavable with the oxidizing agent include a vicinal diol residue and a selenium residue.

Examples of the enzyme include trypsin, papain, TEV, thrombin, cathepsin B, cathepsin D, cathepsin K, caspase, protease, matrix metalloproteinase, lipase, endoglycosidase, and PN Gase F. Examples of a site cleavable with the enzyme include an ester residue, a phosphodiester residue, and a glycosyl residue.

Examples of a site cleavable with light include a 2-nitrobenzyl residue, a phenacyl ester residue, an 8-quinoline benzenesulfonate residue, a coumarin residue, a phosphotriester residue, a bisarylhydrazone residue, and a bimane dithiopropionic acid residue.

Examples of the self-decomposing cleavable portion include an activated ester residue (e.g., an N-hydroxysuccinimide residue).

More specifically, the cleavable portion may correspond to any one chemical structure selected from the group consisting of the following: 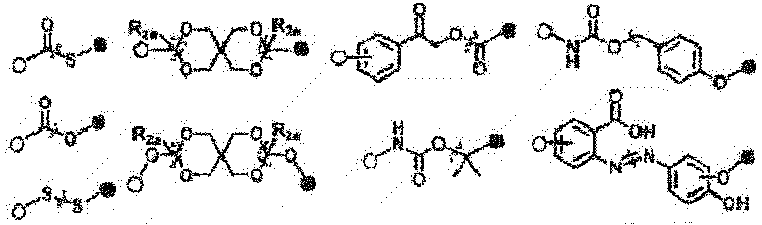 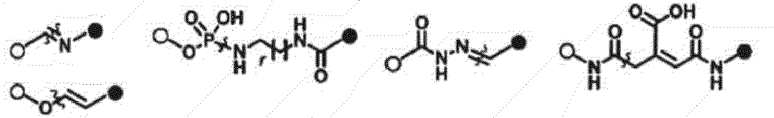 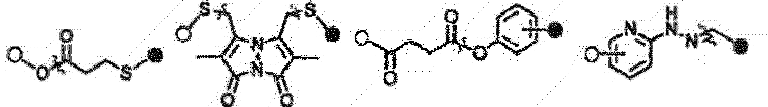 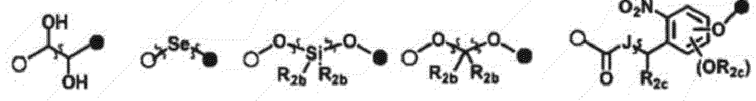 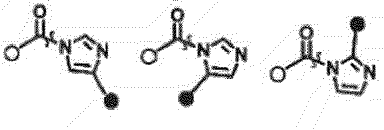
where a wavy line orthogonal to a bond indicates a cleavage site,
a plurality of R₂ₐS, a plurality of R_{2b}S, and a plurality of R_{2c}S are the same as or different from each other, and are each a hydrogen atom or selected from substituents described later,
J is -CH₂-, -O-, or -S-,
r is any integer of 1 to 4,
a symbol of "white circle" indicates a bond to A (or La described later), and a symbol of "black circle" indicates a bond to B (or Lb described later), and
when the chemical structure is asymmetrical with respect to the cleavage site, a symbol of "black circle" may indicate a bond to A (or La described later), and a symbol of "white circle" may indicate a bond to B (or Lb described later).

J is -CH₂-, -O-, or -S-. The letter j is preferably -CH₂- or -O- and more preferably -CH₂-.

The letter r is any integer of 1 to 4, preferably any integer of 1 to 3, and more preferably 1 or 2.

In an embodiment, the cleavable linker may be (i) a cleavable linker which is a divalent group comprising a cleavable portion having the ability to form a bioorthogonal functional group on a reactive group side by cleavage or (ii) a cleavable linker which is a divalent group comprising a cleavable portion having no ability to form a bioorthogonal functional group on a reactive group side by cleavage.

Examples of the cleavable portion of (i) include a disulfide residue, an ester residue, an acetal residue, a ketal residue, an imine residue, and a vicinal diol residue.

More specifically, the cleavable portion of (i) may, for example, correspond to any one chemical structure selected from the group consisting of the following: 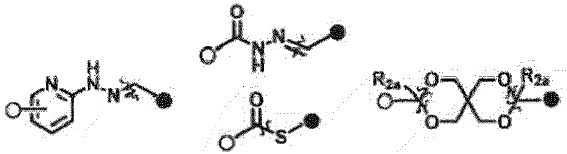 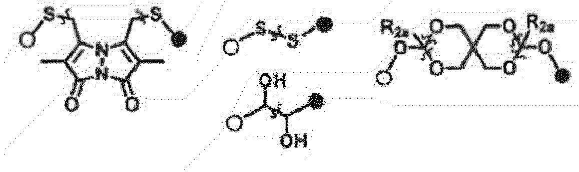
where a wavy line orthogonal to a bond indicates a cleavage site,
a plurality of R₂ₐs are the same as or different from each other, and are hydrogen atoms or selected from substituents described later,
a symbol of "white circle" indicates a bond to A (or La described later), and a symbol of "black circle" indicates a bond to B (or Lb described later), and
when the chemical structure is asymmetrical with respect to the cleavage site, a symbol of "black circle" may indicate a bond to A (or La described later), and a symbol of "white circle" may indicate a bond to B (or Lb described later).

Examples of the cleavable portion of (ii) include an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.

More specifically, the cleavable portion of (ii) may, for example, correspond to any one chemical structure selected from the group consisting of the following: 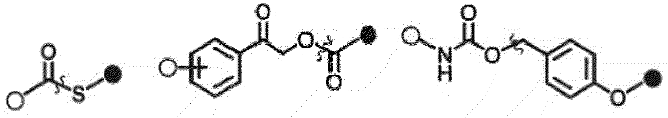 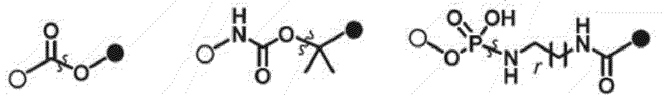 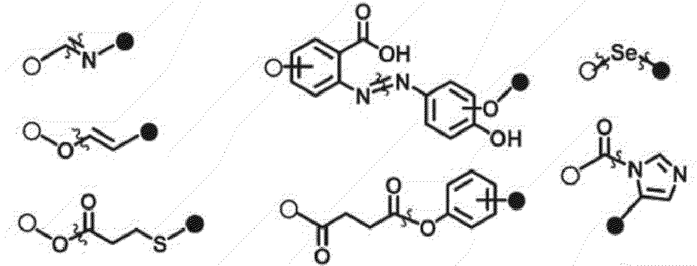 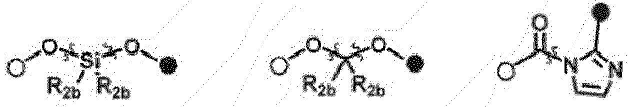 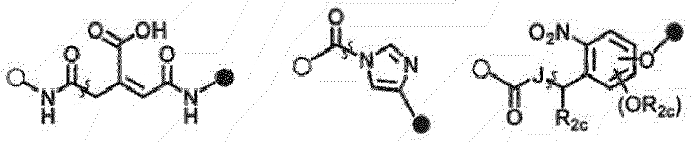
where a wavy line orthogonal to a bond indicates a cleavage site,
a plurality of R_{2b}s, a plurality of R_{2c}s, J, and r are hydrogen atoms or selected from substituents described later,
a symbol of "white circle" indicates a bond to A (or La described later), and a symbol of "black circle" indicates a bond to B (or Lb described later), and
when the chemical structure is asymmetrical with respect to the cleavage site, a symbol of "black circle" may indicate a bond to A (or La described later), and a symbol of "white circle" may indicate a bond to B (or Lb described later).

In a specific embodiment, the cleavable linker (L) may be represented by any one of the following Formulae (L1) to (L3):

La-C-Lb (L1)

La-C (L2)

C-Lb (L3)

wherein
La and Lb are each a divalent group, and
C is a cleavable portion.

Examples of the divalent group include a divalent hydrocarbon group optionally having a substituent, a divalent heterocyclic group optionally having a substituent, -C(=O)-, -NRₐ- (Rₐ indicates a hydrogen atom or a substituent), -O-, -S-, -C(=S)-, and a group consisting of a combination of two or more (e.g., two to eight, preferably two to six, and more preferably two to four) of these.

The divalent hydrocarbon group is a linear, branched, or cyclic divalent hydrocarbon group and preferably a linear or branched divalent hydrocarbon group. Examples of the divalent hydrocarbon group include alkylene, alkenylene, alkynylene, and arylene.

The alkylene is preferably C1-12 alkylene, more preferably C1-6 alkylene, and particularly preferably C1-4 alkylene. The number of carbon atoms does not comprise the number of carbon atoms of the substituent. Alkylene may be any of linear, branched, or cyclic one and is preferably linear alkylene. Examples of such an alkylene include methylene, ethylene, propylene, butylene, pentylene, and hexylene.

The alkenylene is preferably C2-12 alkenylene, more preferably C2-6 alkenylene, and particularly preferably C2-4 alkenylene. The number of carbon atoms does not comprise the number of carbon atoms of the substituent. Alkenylene may be any of linear, branched, or cyclic one and is preferably linear alkenylene. Examples of such an alkenylene include ethylenylene, propynylene, butenylene, pentenylene, and hexenylene.

The alkynylene is preferably C2-12 alkynylene, more preferably C2-6 alkynylene, and particularly preferably C2-4 alkynylene. The number of carbon atoms does not comprise the number of carbon atoms of the substituent. Alkynylene may be any of linear, branched, or cyclic one and is preferably linear alkynylene. Examples of such an alkynylene include ethynylene, propynylene, butynylene, pentynylene, and hexynylene.

The arylene is preferably C6-24 arylene, more preferably C6-18 arylene, even more preferably C6-14 arylene, and still even more preferably C6-10 arylene. The number of carbon atoms does not comprise the number of carbon atoms of the substituent. Examples of the arylene include phenylene, naphthylene, and anthracenylene.

The divalent heterocyclic group is a divalent aromatic heterocyclic group or a divalent nonaromatic heterocyclic group. The divalent heterocyclic group preferably comprises, as a hetero atom forming a heterocycle, one or more selected from the group consisting of an oxygen atom, a sulfur atom, a nitrogen atom, a phosphorous atom, a boron atom, and a silicon atom and more preferably comprises one or more selected from the group consisting of an oxygen atom, a sulfur atom, and a nitrogen atom.

The divalent aromatic heterocyclic group is preferably a C1-21 divalent aromatic heterocyclic group, more preferably a C1-15 divalent aromatic heterocyclic group, even more preferably a C1-9 divalent aromatic heterocyclic group, and still even more preferably a C1-6 divalent aromatic heterocyclic group. The number of carbon atoms does not comprise the number of carbon atoms of the substituent. More specific examples of the divalent aromatic heterocyclic group include pyrrolediyl, furandiyl, thiophenediyl, pyridinediyl, pyridazinediyl, pyrimidinediyl, pyrazinediyl, triazinediyl, pyrazolediyl, imidazolediyl, thiazolediyl, isothiazolediyl, oxazolediyl, isoxazolediyl, triazolediyl, tetrazolediyl, indolediyl, purinediyl, anthraquinonediyl, carbazolediyl, fluorenediyl, quinolinediyl, isoquinolinediyl, quinazolinediyl, and phthalazinediyl.

The divalent nonaromatic heterocyclic group is preferably a C2-21 nonaromatic heterocyclic group, more preferably a C2-15 nonaromatic heterocyclic group, even more preferably a C2-9 nonaromatic heterocyclic group, and still even more preferably a C2-6 nonaromatic heterocyclic group. The number of carbon atoms does not comprise the number of carbon atoms of the substituent. More specifically, examples of the divalent nonaromatic heterocyclic group include pyrroldionediyl, pyrrolinedionediyl, oxiranediyl, aziridinediyl, azetidinediyl, oxetanediyl, thietanediyl, pyrrolidinediyl, dihydrofurandiyl, tetrahydrofurandiyl, dioxolanediyl, tetrahydrothiophenediyl, imidazolidinediyl, oxazolidinediyl, piperidinediyl, dihydropyrandiyl, tetrahydropyrandiyl, tetrahydrothiopyrandiyl, morpholinediyl, thiomorpholinediyl, piperazinediyl, dihydrooxazinediyl, tetrahydrooxazinediyl, dihydropyrimidinediyl, and tetrahydropyrimidinediyl.

The divalent group represented by La and Lb may have e.g., one to five, preferably one to three, and more preferably one or two substituents. Such a substituent is similar to the substituent represented by Rₐ and R_{b}.
Examples of such a substituent include the following:
(i) a halogen atom;
(ii) a monovalent hydrocarbon group;
(iii) aralkyl;
(iv) a monovalent heterocyclic group;
(v) R_{c}-O-, R_{c}-C(=O)-, R_{c}-O-C(=O)-, and R_{c}-C(=O)-O- (R_{c} indicates a hydrogen atom or a monovalent hydrocarbon group);
(vi) NR_{d}Rₑ-, NR_{d}Rₑ-C(=O)-, NR_{d}Rₑ-C(=O)-O-, and R_{d}-C(=O)-NRₑ- (R_{d} and Rₑ are the same as or different from each other, and each indicate a hydrogen atom or a monovalent hydrocarbon group); and
(vii) a nitro group, a sulfuric acid group, a sulfonic acid group, a cyano group, and a carboxy group.

Examples of the halogen atom include a fluorine atom, a chlorine atom, a bromine atom, and an iodine atom.

Examples of the monovalent hydrocarbon group include a monovalent chain hydrocarbon group, a monovalent alicyclic hydrocarbon group, and a monovalent aromatic hydrocarbon group.

The monovalent chain hydrocarbon group means a hydrocarbon group comprising only a chain structure and does not comprise any cyclic structure in a main chain thereof. Note that the chain structure may be linear or branched. Examples of the monovalent chain hydrocarbon group include alkyl, alkenyl, and alkynyl. Alkyl, alkenyl, and alkynyl may be linear or branched.

The alkyl is preferably C₁₋₁₂ alkyl, more preferably C₁₋₆ alkyl, and even more preferably C₁₋₄ alkyl. The number of carbon atoms does not comprise the number of carbon atoms of the substituent. Examples of C₁₋₁₂ alkyl include methyl, ethyl, n-propyl, i-propyl, n-butyl, s-butyl, isobutyl, t-butyl, pentyl, hexyl, heptyl, octyl, nonyl, decyl, and dodecyl.

The alkenyl is preferably C₂₋₁₂ alkenyl, more preferably C₂₋₆ alkenyl, and even more preferably C₂₋₄ alkenyl. The number of carbon atoms does not comprise the number of carbon atoms of the substituent. Examples of C₂₋₁₂ alkenyl include vinyl, propenyl, and n-butenyl.

The alkynyl is preferably C₂₋₁₂ alkynyl, more preferably C₂₋₆ alkynyl, and even more preferably C₂₋₄ alkynyl. The number of carbon atoms does not comprise the number of carbon atoms of the substituent. Examples of C₂₋₁₂ alkynyl include ethynyl, propynyl, and n-butynyl.

The monovalent chain hydrocarbon group is preferably alkyl.

The monovalent alicyclic hydrocarbon group means a hydrocarbon group comprising only alicyclic hydrocarbon as a cyclic structure and not comprising any aromatic ring, in which the alicyclic hydrocarbon may be monocyclic or polycyclic. Note that the monovalent alicyclic hydrocarbon group is not necessarily required to comprise only an alicyclic hydrocarbon but may comprise a chain structure in part thereof. Examples of the monovalent alicyclic hydrocarbon group include cycloalkyl, cycloalkenyl, and cycloalkynyl, which may be monocyclic or polycyclic.

Cycloalkyl is preferably C₃₋₁₂ cycloalkyl, more preferably C₃₋₆ cycloalkyl, and even more preferably C₅₋₆ cycloalkyl. The number of carbon atoms does not comprise the number of carbon atoms of the substituent. Examples of C₃₋₁₂ cycloalkyl include cyclopropyl, cyclobutyl, cyclopentyl, and cyclohexyl.

The cycloalkenyl is preferably C₃₋₁₂ cycloalkenyl, more preferably C₃₋₆ cycloalkenyl, and even more preferably C₅₋₆ cycloalkenyl. The number of carbon atoms does not comprise the number of carbon atoms of the substituent. Examples of C₃₋₁₂ cycloalkenyl include cyclopropenyl, cyclobutenyl, cyclopentenyl, and cyclohexenyl.

The cycloalkynyl is preferably C₃₋₁₂ cycloalkynyl, more preferably C₃₋₆ cycloalkynyl, and even more preferably C₅₋₆ cycloalkynyl. The number of carbon atoms does not comprise the number of carbon atoms of the substituent. Examples of C₃₋₁₂ cycloalkynyl include cyclopropynyl, cyclobutynyl, cyclopentynyl, and cyclohexynyl.

The monovalent alicyclic hydrocarbon group is preferably cycloalkyl.

The monovalent aromatic hydrocarbon group means a hydrocarbon group comprising an aromatic cyclic structure. Note that the monovalent aromatic hydrocarbon group is not necessarily required to comprise only an aromatic ring and may comprise a chain structure or alicyclic hydrocarbon in part thereof, in which the aromatic ring may be monocyclic or polycyclic. The monovalent aromatic hydrocarbon group is preferably C₆₋₁₂ aryl, more preferably C₆₋₁₀ aryl, and even more preferably C₆ aryl. The number of carbon atoms does not comprise the number of carbon atoms of the substituent. Examples of C₆₋₁₂ aryl include phenyl and naphthyl.

The monovalent aromatic hydrocarbon group is preferably phenyl.

Among these, the monovalent hydrocarbon group is preferably alkyl, cycloalkyl, and aryl and more preferably alkyl.

Aralkyl refers to arylalkyl. The definitions, examples, and preferred examples of aryl and alkyl in arylalkyl are as described above. The aralkyl is preferably C₃₋₁₅ aralkyl. Examples of such an aralkyl include benzoyl, phenethyl, naphthylmethyl, and naphthylethyl.

The monovalent heterocyclic group refers to a group obtained by removing one hydrogen atom from a heterocycle of a heterocyclic compound. The monovalent heterocyclic group is a monovalent aromatic heterocyclic group or a monovalent nonaromatic heterocyclic group. The monovalent heterocyclic group preferably comprises one or more selected from the group consisting of an oxygen atom, a sulfur atom, a nitrogen atom, a phosphorus atom, a boron atom, and a silicon atom and more preferably comprises one or more selected from the group consisting of an oxygen atom, a sulfur atom, and a nitrogen atom as a hetero atom contained in the heterocyclic group.

The monovalent aromatic heterocyclic group is preferably a C₁₋₁₅ aromatic heterocyclic group, more preferably a C₁₋₉ aromatic heterocyclic group, and even more preferably a C₁₋₆ aromatic heterocyclic group. The number of carbon atoms does not comprise the number of carbon atoms of the substituent. Examples of the monovalent aromatic heterocyclic group include pyrrolyl, furanyl, thiophenyl, pyridinyl, pyridazinyl, pyrimidinyl, pyrazinyl, triazinyl, pyrazolyl, imidazolyl, thiazolyl, isothiazolyl, oxazolyl, isoxazolyl, triazolyl, tetrazolyl, indolyl, purinyl, anthraquinolyl, carbazonyl, fluorenyl, quinolinyl, isoquinolinyl, quinazolinyl, and phthalazinyl.

The monovalent nonaromatic heterocyclic group is preferably a C₂₋₁₅ nonaromatic heterocyclic group, more preferably a C₂₋₉ nonaromatic heterocyclic group, and even more preferably a C₂₋₆ nonaromatic heterocyclic group. The number of carbon atoms does not comprise the number of carbon atoms of the substituent. Examples of the monovalent nonaromatic heterocyclic group include oxiranyl, aziridinyl, azetidinyl, oxetanyl, thietanyl, pyrrolidinyl, dihydrofuranyl, tetrahydrofuranyl, dioxolanyl, tetrahydrothiophenyl, pyrolinyl, imidazolidinyl, oxazolidinyl, piperidinyl, dihydropyranyl, tetrahydropyranyl, tetrahydrothiopyranyl, morpholinyl, thiomorpholinyl, piperazinyl, dihydrooxazinyl, tetrahydrooxazinyl, dihydropyrimidinyl, and tetrahydropyrimidinyl.

Among these, the monovalent heterocyclic group is preferably a five-membered or six-membered heterocyclic group.

The substituent may be preferably the following:
(i') a halogen atom;
(ii') a C₁₋₁₂ alkyl, phenyl, or naphthyl;
(iii') C₃₋₁₅ aralkyl;
(iv') a five-membered or six-membered heterocyclic group;
(v') R_{c}-O-, R_{c}-C(=O)-, R_{c}-O-C(=O)-, or R_{c}-C(=O)-O- (R_{c} indicates a hydrogen atom or C₁₋₁₂ alkyl) ;
(vi') NR_{d}Rₑ-, NR_{d}Rₑ-C(=O)-, NR_{d}Rₑ-C(=O)-O-, or R_{d}-C(=O)-NRₑ- (R_{d} and Rₑ are the same as or different from each other, and each indicate a hydrogen atom or C₁₋₁₂ alkyl); or
(vii') the same groups as those enumerated in (vii).

The substituent may be more preferably the following:
(i") a halogen atom;
(ii") C₁₋₁₂ alkyl;
(iii") R_{c}-O-, R_{c}-C(=O)-, R_{c}-O-C(=O)-, or R_{c}-C(=O)-O-(R_{c} indicates a hydrogen atom or C₁₋₁₂ alkyl);
(iv") NR_{d}Rₑ-, NR_{d}Rₑ-C(=O)-, NR_{d}Rₑ-C(=O) -O-, or R_{d}-C(=O)-NRₑ-(R_{d} and Rₑ are the same as or different from each other, and each indicate a hydrogen atom or C₁₋₁₂ alkyl); or (v") the same groups as those enumerated in (vii).

The substituent may be even more preferably the following:
(i''') a halogen atom;
(ii''') C₁₋₆ alkyl;
(iii''') R_{c}-O-, R_{c}-C(=O)-, R_{c}-O-C(=O)-, or R_{c}-C(=O)-O-(R_{c} indicates a hydrogen atom or C₁₋₆ alkyl);
(iv''') NR_{d}Rₑ-, NR_{d}Rₑ-C(=O) -, NR_{d}Rₑ-C(=O) -O-, or R_{d}-C(=O)-NRₑ- (R_{d} and Rₑ are the same as or different from each other, and each indicate a hydrogen atom or C₁₋₆ alkyl); or (v''') the same groups as those enumerated in (vii).

The substituent may be particularly preferably the following:
(i'''') a halogen atom;
(ii'''') C₁₋₄ alkyl;
(iii'''') R_{c}-O-, R_{c}-C(=O)-, R_{c}-O-C(=O)-, or R_{c}-C(=O)-O-(R_{c} indicates a hydrogen atom or C₁₋₄ alkyl);
(iv'''') NR_{d}Rₑ-, NR_{d}Rₑ-C(=O)-, NR_{d}Rₑ-C(=O)-O-, or R_{d}-C(=O)-NRₑ- (R_{d} and Rₑ are the same as or different from each other, and each indicate a hydrogen atom or C₁₋₄ alkyl); or (v'''') the same groups as those enumerated in (vii).

In a specific embodiment, La and Lb may be represented by the following (La') and (Lb'), respectively: 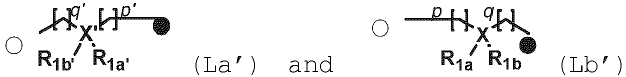 wherein
p and p' are the same as or different from each other, and are each any integer of 0 to 10,
q and q' are the same as or different from each other, and are each any integer of 0 to 10,
X and X' are the same as or different from each other, and are each a carbon atom, a nitrogen atom, or a single bond (when X is a nitrogen atom, R_{1b} is absent, when X' is a nitrogen atom, R_{1b'} is absent, when X is a single bond, R₁ₐ and R_{1b} are absent, and when X' is a single bond, R_{1a'} and R_{ib'} are absent), and
R₁ₐ, R_{1b}, R_{1a'}, and R_{1b'} are the same as or different from each other, and are each a hydrogen atom or selected from the group consisting of the above-described substituents.

The letters p and p' are the same as or different from each other, and are each any integer of 0 to 10, preferably an integer of 0 to 8, more preferably an integer of 0 to 6, even more preferably an integer of 0 to 4, and particularly preferably 0, 1, or 2. The letters p and p' are preferably the same as each other.

The letters q and q' are the same as or different from each other, and are each any integer of 0 to 10, preferably an integer of 0 to 8, more preferably an integer of 0 to 6, even more preferably an integer of 0 to 4, and particularly preferably 0, 1, or 2. The letters q and q' are preferably the same.

X and X' are the same as or different from each other, and are each a carbon atom, a nitrogen atom, or a single bond and preferably a carbon atom or a single bond. X and X' are preferably the same.

R₁ₐ, R_{1b}, R_{1a'}, and R_{1b'} are the same as or different from each other, and are selected from a hydrogen atom or the group consisting of the following substituents. The definition, examples, and preferred examples of the substituent are as described above. R₁ₐ, R_{1b'} R_{1a'}, and R_{1b'} are each preferably a hydrogen atom.

### 1-4. (a) Divalent Group Comprising Bioorthogonal functional group or (b) Divalent Group Comprising No Bioorthogonal functional group (B)

In Formula (I), B is (a) a divalent group comprising a bioorthogonal functional group or (b) a divalent group comprising no bioorthogonal functional group.

The bioorthogonal functional group refers to a group that does not react with biological components (e.g., amino acids, nucleic acids, lipids, sugars, and phosphoric acids) or has a low reaction rate to biological components but selectively reacts with components other than biological components. The bioorthogonal functional group is well known in the technical field concerned (e.g., refer to Sharpless K. B. et al., Angew. Chem. Int. Ed. 40, 2004 (2015); Bertozzi C. R. et al., Science 291, 2357 (2001); Bertozzi C. R. et al., Nature Chemical Biology 1, 13 (2005)).

When the target of the affinity substance is an antibody, the bioorthogonal functional group is a bioorthogonal functional group to a protein. The bioorthogonal functional group to proteins is a group that does not react with side chains of 20 natural amino acid residues forming proteins and reacts with certain functional groups. The 20 natural amino acid residues forming proteins are alanine (A), asparagine (N), cysteine (C), glutamine (Q), glycine (G), isoleucine (I), leucine (L), methionine (M), phenylalanine (F), proline (P), serine (S), threonine (T), tryptophan (W), tyrosine (Y), valine (V), aspartic acid (D), glutamic acid (E), arginine (R), histidine (H), and lysine (L). Among these 20 natural amino acid residues, glycine, which has no side chain (that is, has a hydrogen atom), and alanine, isoleucine, leucine, phenylalanine, and valine, which have a hydrocarbon group as a side chain (that is, comprise no hetero atom selected from the group consisting of a sulfur atom, a nitrogen atom, and an oxygen atom in their side chains) are inactive to normal reactions. Consequently, the bioorthogonal functional group to proteins is a functional group incapable of reacting with, in addition to the side chains of these amino acids having side chains inactive to normal reactions, side chains of asparagine, glutamine, methionine, proline, serine, threonine, tryptophan, tyrosine, aspartic acid, glutamic acid, arginine, histidine, and lysin.

Examples of such a bioorthogonal functional group that cannot react with proteins include an azide residue, an aldehyde residue, a thiol residue, an alkene residue (in other words, it is only required to have a vinylene (ethenylene) portion, which is a minimum unit having a carbon-carbon double bond. Hereinafter the same), an alkyne residue (in other words, it is only required to have an ethynylene portion, which is a minimum unit having a carbon-carbon triple bond. Hereinafter the same), a halogen residue, a tetrazine residue, a nitrone residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue (e.g., a carbonyl residue having a fluorine atom, a chlorine atom, a bromine atom, or an iodine atom at an α-position. Hereinafter the same), an isonitrile residue, a sydnone residue, and a selenium residue. The protein includes proteins capable of comprising a free thiol (cysteine) (e.g., proteins other than antibodies) and proteins incapable of comprising a free thiol (e.g., antibodies). In the protein that cannot comprise a free thiol, a thiol functions as a bioorthogonal functional group. Consequently, a target of the affinity substance is an antibody incapable of comprising a free thiol, and therefore the bioorthogonal functional group comprises a thiol. The divalent group may comprise one or more (e.g., two, three, or four) bioorthogonal functional groups; the divalent group may preferably comprise one bioorthogonal functional group.

In an embodiment, the divalent group comprising a bioorthogonal functional group may be a divalent group comprising a bioorthogonal functional group selected from the group consisting of an azide residue, an aldehyde group, a thiol residue, an alkyne residue, an alkene residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue in a main chain thereof.

In another embodiment, the divalent group comprising a bioorthogonal functional group may be a divalent group comprising a bioorthogonal functional group selected from the group consisting of an azide residue, an aldehyde residue, a thiol residue, an alkyne residue, an alkene residue, a halogen residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, an α-halocarbonyl residue, an isonitrile residue, a sydnone residue, and a selenium residue in a side chain thereof.

More specifically, the bioorthogonal functional group may correspond to any one chemical structure selected from the group consisting of the following: 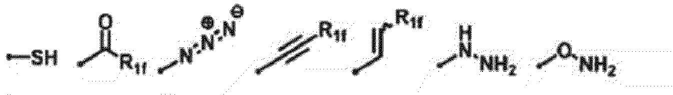 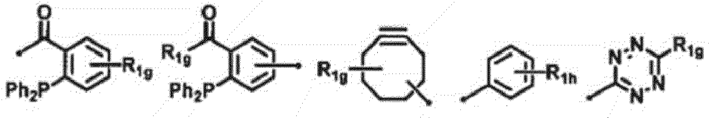 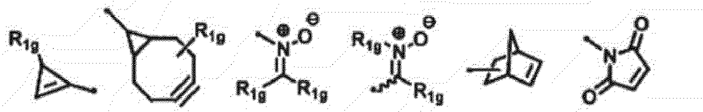 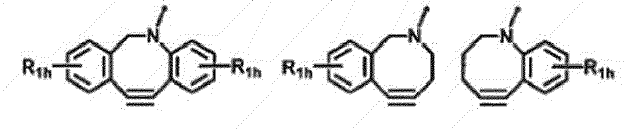 wherein
R_{1f}, one or a plurality of R_{1g}, and one or a plurality of R₁ₕ are the same as or different from each other, and are each an atom or a group selected from the group consisting of above (i) to (vii) or an electron-withdrawing group, and · is a bond.

Examples of the electron-withdrawing group include those described above, in which preferred are a halogen atom, a boronic acid residue, mesyl, tosyl, and triflate.

In an embodiment, B may be (a) the divalent group comprising a bioorthogonal functional group. The divalent group comprises one or a plurality of bioorthogonal functional groups; the number is e.g., one to five, preferably one to three, more preferably one or two, and even more preferably one. When the divalent group comprises a plurality of bioorthogonal functional groups, the bioorthogonal functional groups may be homogeneous or heterogeneous and are preferably homogeneous in view of employing a simple structure and the like.

In a specific embodiment, B may be (a1) a divalent group comprising a bioorthogonal functional group in a main chain thereof. The divalent group comprising a bioorthogonal functional group in a main chain thereof is a bioorthogonal functional group itself selected from the group consisting of an azide residue, an aldehyde group, a thiol residue, an alkyne residue, an alkene residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue as a divalent group or a group in which the divalent group described above is linked to either one terminal or both terminals of such a divalent orthogonal functional group. The definition, examples, and preferred examples of the divalent group to be linked are similar to those of the divalent group described above.

In another specific embodiment, B may be (a2) a divalent group comprising a bioorthogonal functional group in a side chain thereof. The divalent group comprising a bioorthogonal functional group in a side chain thereof is a divalent group substituted with a bioorthogonal functional group selected from the group consisting of an azide residue, an aldehyde residue, a thiol residue, an alkyne residue, an alkene residue, a halogen residue, a tetrazine residue, a nitron residue, a hydroxyamine residue, a nitrile residue, a hydrazine residue, a ketone residue, a boronic acid residue, a cyanobenzothiazole residue, an allyl residue, a phosphine residue, a maleimide residue, a disulfide residue, an α-halocarbonyl residue, an isonitrile residue, a sydnone residue, and a selenium residue or a group comprising the bioorthogonal functional group. The definition, examples, and preferred examples of the divalent group to be substituted are similar to those of the divalent group described above.

In another embodiment, B may be (b) the divalent group comprising no bioorthogonal functional group. Such a divalent group may be optionally substituted alkylene, optionally substituted cycloalkylene, optionally substituted aryl, an optionally substituted divalent heterocyclic group, -NRₐ- (Rₐ indicates a hydrogen atom or a substituent), -O-, or a group consisting of a combination of two or more (e.g., two to eight, preferably two to six, and more preferably two to four) of these. The substituent in the case of being optionally substituted and the substituent of Rₐ are each a substituent other than the bioorthogonal functional group. Examples of such a substituent include alkyl, cycloalkyl, aralkyl, a monovalent heterocyclic group, hydroxy, amino, alkyloxy (alkoxy), cycloalkyloxy, and aralkyloxy. The number of such a substituent is e.g., one to five, preferably one to three, more preferably one or two, and even more preferably one.

As to the substituent other than the bioorthogonal functional group, the definitions, examples, and preferred examples of alkyl, cycloalkyl, aralkyl, and the monovalent heterocyclic group are as described above.

As to the substituent other than the bioorthogonal functional group, the definition, examples, and preferred examples of alkyl in alkyloxy (alkoxy), cycloalkyl in cycloalkyloxy, and aralkyl in aralkyloxy are as described above. More specifically, examples of alkyloxy include methyloxy, ethyloxy, propyloxy (e.g., n-propyloxy and iso-propyloxy), butyloxy (e.g., n-butyloxy, iso-butyloxy, sec-butyloxy, and tert-butyloxy), pentyloxy (e.g., n-pentyloxy), and hexyloxy (e.g., n-hexyloxy). Examples of cycloalkyloxy include cyclopropyloxy, cyclobutyloxy, cyclopentyloxy, and cyclohexyloxy. Examples of aralkyloxy include benzoyloxy, phenethyloxy, naphthylmethyloxy, and naphthylethyloxy.

The divalent group comprising no bioorthogonal functional group may be a group highly inactive to reactions. Consequently, such a divalent group may be a group comprising only carbon atoms and hydrogen atoms. Such a divalent group is alkylene, cycloalkylene, or aryl, and a combination of two or more (e.g., two or three) of these. When the divalent group comprising no bioorthogonal functional group is a group highly inactive to reactions, such a divalent group may have a substituent selected from the group consisting of alkylene, cycloalkylene, and aryl as a substituent highly inactive to reactions. The number of the substituent highly inactive to reactions is e.g., one to five, preferably one to three, and more preferably one or two.

In a specific embodiment, B may be represented by the following Formula (B-1): 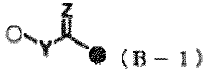 wherein
Y is -NH-, -O-, -CH₂-, or the following Formula (B-2): 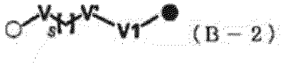 wherein
V and V' are the same as or different from each other, and are each -NH-, -O-, -CH₂-, or a single bond,
V1 is (a) a divalent group comprising a bioorthogonal functional group or (b) a divalent group comprising no bioorthogonal functional group,
s is any integer of 0 to 10,
a symbol of "white circle" and a symbol of "black circle" in Formula (B-2) have the same orientation as a symbol of "white circle" and a symbol of "black circle" in Formula (B-1), respectively,
Z is an oxygen atom, a sulfur atom, or a hydrogen atom (when Z is a hydrogen atom, -C(=Z)- indicates -CH₂-.), and in Formula (B-1), a symbol of "white circle" indicates a bond to an L-side portion and a symbol of "black circle" indicates a bond to an R-side portion.

Y is -NH-, -O-, -CH₂-, or the group represented by Formula (B-2). In view of simplifying the structure and the like, Y may be -NH-, -O-, or -CH₂-. Alternatively, in view of designing a carbon atom-based structure and the like, Y may be -CH₂- or the group represented by Formula (B-2).

Z is an oxygen atom, a sulfur atom, or a hydrogen atom and is preferably an oxygen atom or a sulfur atom.

V and V' are each -NH-, -O-, -CH₂-, or a single bond and preferably -CH₂- or a single bond.

V1 is (a) a divalent group comprising a bioorthogonal functional group or (b) a divalent group comprising no bioorthogonal functional group. Such a divalent group is similar to that described above.

The divalent group in V1 is preferably an optionally substituted divalent hydrocarbon group or an optionally substituted divalent heterocyclic group. The definition, examples, and preferred examples of the divalent hydrocarbon group are similar to those described above; for V1, preferred are alkylene, alkenylene, alkynylene, cycloalkylene, cycloalkenylene, cycloalkynylene, and arylene. In the case of not being substituted at the portion comprising the bioorthogonal functional group, for example, preferred are alkenylene, alkynylene, cycloalkenylene, and cycloalkynylene. On the other hand, in the case of being substituted at the portion comprising the bioorthogonal functional group, preferred are alkylene, cycloalkylene, and arylene. Examples and preferred examples of these groups are as described above. The definition, examples, and preferred examples of the divalent heterocyclic group are similar to those described above; for V1, a five-membered or six-membered heterocyclic group is preferred. Examples and preferred examples of the five-membered or six-membered heterocyclic group are similar to those described above. The definition, examples, and preferred examples of the substituent are as described above. V1 may have e.g., one to five, more preferably one to three, even more preferably one or two, and still even more preferably one of (a) the bioorthogonal functional groups. When the divalent group comprises a plurality of bioorthogonal functional groups, the bioorthogonal functional groups may be homogeneous or heterogeneous. In view employing a simple structure, improving reactivity, and the like, they are preferably homogeneous. In view of ensuring a differentiated reaction and the like, they are preferably heterogeneous. V1 may also have one to five, preferably one to three, and more preferably one or two (of b) the substituents.

The letter s is any integer of 0 to 10, preferably an integer of 0 to 8, more preferably an integer of 0 to 6, even more preferably an integer of 0 to 4, and particularly preferably 0, 1, or 2.

In a more specific embodiment, V1 may be a divalent group having, as a side chain, a group represented by the following Formula (B-3): 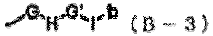 wherein
G and G' are the same as or different from each other, and are each -NH-, -O-, -CH₂-, a single bond, or the following formula (B-4): 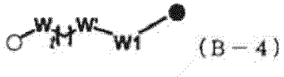 wherein
W and W' are the same as or different from each other, and are each -NH-, -O-, -CH₂-, or a single bond,
W1 is (a) a divalent group comprising a bioorthogonal functional group or (b) a divalent group comprising no bioorthogonal functional group,
t is an any integer of 0 to 10,
in Formula (B-4), a symbol of "white circle" indicates a bond to a direction of a bond (·) in Formula (B-3), and a symbol of "black circle" indicates a bond to a direction of a b side,
H is -CH₂-, -C=O-, -C=S-, -NH-, or a single bond,
I is a divalent hydrocarbon group, a divalent heterocycle, or a single bond,
b is any one group represented by the following: 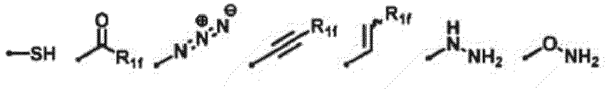 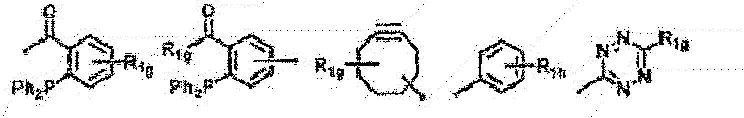 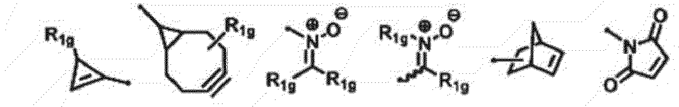 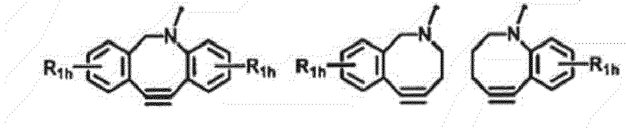 wherein
   R_{1f}, one or a plurality of R_{1g}, and one or a plurality of R₁ₕ are the same as or different from each other, and are each an atom or a group selected from the group consisting of the above (i) to (vii) or an electron-withdrawing group, and · is a bond. Such a divalent group is a divalent hydrocarbon group or a divalent heterocyclic group, preferably an optionally substituted divalent hydrocarbon group, more preferably alkylene, alkenylene, alkynylene, cycloalkylene, cycloalkenylene, cycloalkynylene, or arylene, even more preferably alkylene, cycloalkylene, or arylene, and particularly preferably alkylene. Examples and preferred examples of these groups are as described above. These groups may be substituted with a substituent other than the side chain. The number of such a substituent is one to five, preferably one to three, and more preferably one or two. Examples and preferred examples of the substituent are as described above.

G and G' are the same as or different from each other, and are each -NH-, -O-, -CH₂-, a single bond, or the group represented by Formula (B-4). In view of simplifying the structure and the like, G and G' may be -NH-, -O-,-CH₂-, or a single bond. Alternatively, in view of designing a carbon atom-based structure and the like, G and G' may be -CH₂-, a single bond, or the group represented by Formula (B-4).

H is -CH₂-, -C=O-, -C=S-, -NH-, or a single bond. H is preferably -CH₂- or a single bond.

I is a divalent hydrocarbon group, a divalent heterocycle, or a single bond. The divalent hydrocarbon group and the divalent heterocycle may be substituted or are not necessarily substituted with a substituent. The definitions, examples, and preferred examples of the divalent hydrocarbon group, the divalent heterocycle, and the substituent are similar to those described above for V1.

W and W' are the same as or different from each other, and are each -NH-, -O-, -CH₂-, or a single bond and preferably -CH₂- or a single bond.

W1 is (a) a divalent group comprising a bioorthogonal functional group or (b) a divalent group comprising no bioorthogonal functional group. Such a divalent group is similar to that described above.

The letter t is any integer of 0 to 10, preferably an integer of 0 to 8, more preferably an integer of 0 to 6, even more preferably an integer of 0 to 4, and particularly preferably 0, 1, or 2.

### 1-5. Reactive Group (R)

In Formula (I), R is a reactive group to an antibody. Such a reactive group is a common technical knowledge in the technical field concerned.

The reactive group is a group homogeneous or heterogeneous with respect to the bioorthogonal functional group.

When B is (a) the divalent group comprising a bioorthogonal functional group, for example, the reactive group may be a group heterogeneous with respect to the bioorthogonal functional group. This is because the reactive group being a group homogeneous with respect to the bioorthogonal functional group does not ensure the reaction specificity of the reactive group to the antibody. In addition, this is because the bioorthogonal functional group in the first place is a group incapable of reacting with the side chains of the 20 natural amino acid residues forming the antibody.

More specifically, among the 20 natural amino acids described above forming proteins, glycine, which has no side chain, and alanine, isoleucine, leucine, phenylalanine, and valine, which have a hydrocarbon group as a side chain, are inactive to normal reactions. Consequently, the reactive group to the protein is a group capable of reacting with side chains of any one or more (e.g., two, three, or four) of 14 amino acids consisting of asparagine, glutamine, methionine, proline, serine, threonine, tryptophan, tyrosine, aspartic acid, glutamic acid, arginine, histidine, and lysin. The compound represented by Formula (I) may comprise one or more (e.g., two, three, or four) reactive groups in accordance with conditions such as the amino acid composition of the protein; the compound represented by Formula (I) may preferably comprise one reactive group.

The reactive group is preferably a group capable of reacting with a side chain of any one amino acid among the 14 amino acids described above forming proteins.

The reactive group is more preferably a reactive group specific to a side chain of any one amino acid of lysine, tyrosine, tryptophan, and cysteine.

The reactive group is even more preferably a reactive group specific to a side chain of any one amino acid of lysine, tyrosine, and tryptophan.

When the protein is human IgG such as human IgG1, the reactive group is preferably a reactive group specific to a side chain of lysin or tyrosine.

The reactive group specific to a side chain of a lysine residue is a group capable of specifically reacting with an amino group (NH₂) present in the side chain of the lysing residue; examples thereof include an activated ester residue (e.g., an N-hydroxysuccinimide residue), a vinylsulfone residue, a sulfonylchloride residue, an isocyanate residue, an isothiocyanate residue, an aldehyde residue, a 1,4,7,10-tetraazacyclodecane-1,4,7,10-tetraacetic acid residue, a 2-imino-2-methoxyethyl residue, and a diazonium terephthalic acid residue.

Examples of a linking portion formed by a reaction between the reactive group specific to a side chain of a lysine residue and the amino group (NH₂) present in the side chain of the lysine residue include an amide residue, a urea residue, a pyridine residue, a carbamate residue, and a sulfonamide residue.

More specifically, the reactive group specific to a side chain of a lysine residue may correspond to any one chemical structure selected from the group consisting of the following: where 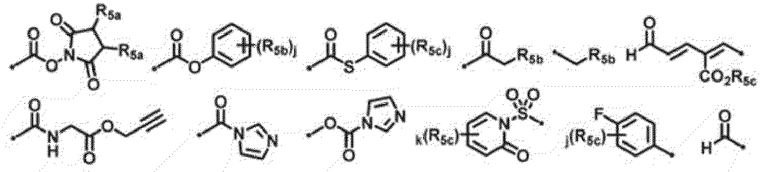 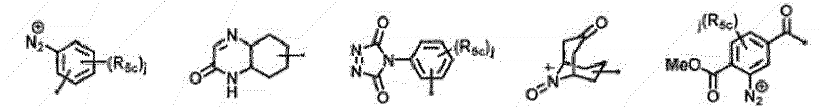 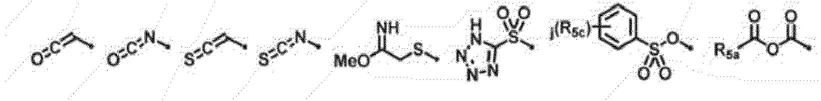
where R₅ₐ and R_{5c} are each a hydrogen atom or the substituent described above,
R_{5b} is an electron-withdrawing group,
j is any integer of 1 to 5, and
k is any integer of 1 to 4.

R₅ₐ and R_{5c} are each a hydrogen atom or the substituent described above. The definition, examples, and preferred examples of the substituent are similar to those described above.

R_{5b} is an electron-withdrawing group. Examples of the electron-withdrawing group include those described above; preferred are a halogen atom, a boronic acid residue, mesyl, tosyl, and triflate.

The letter j is any integer of 1 to 5, preferably an integer of 1 to 3, and more preferably 1 or 2.

The letter k is any integer of 1 to 4, preferably an integer of 1 to 3, and more preferably 1 or 2.

The linking portion formed by a reaction between the chemical structure as the reactive group specific to a side chain of a lysine residue and the amino group (NH₂) present in the side chain of the lysine residue may correspond to any one chemical structure selected from the group consisting of the following: 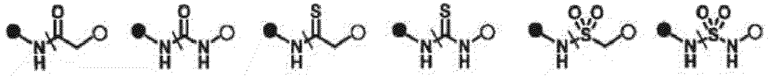 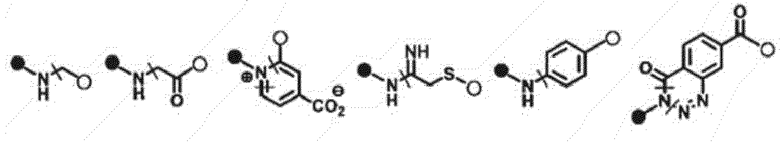 where a symbol of "black circle" indicates a bond to a T-side portion, and a symbol of "white circle" indicates a bond to a B-side portion, and a straight line orthogonal to a bond indicates a bond formed by the reaction.

The reactive group specific to a side chain of a tyrosine residue is a group capable of specifically reacting with an atom at the ortho-position of a phenolic hydroxy group (OH) present in the side chain of the tyrosine residue; examples thereof include a diazonium residue, a diazodicarboxylate residue, and a 2,3-dihydro-1H-pyrazin-6-one residue.

More specifically, the reactive group specific to a side chain of a tyrosine residue may correspond to any one chemical structure selected from the group consisting of the following: 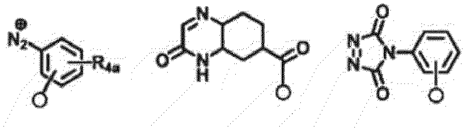 where R₄ₐ is a hydrogen atom or the substituent described above, and a symbol of "white circle" indicates a bond to B.

R₄ₐ is a hydrogen atom or the substituent described above. The definition, examples, and preferred examples of the substituent are similar to those described above.

A linking portion formed by a reaction between the chemical structure as the reactive group specific to a side chain of a tyrosine residue and the atom at the ortho-position of the phenolic hydroxy group (OH) present in the side chain of the tyrosine residue may correspond to any one chemical structure selected from the group consisting of the following: 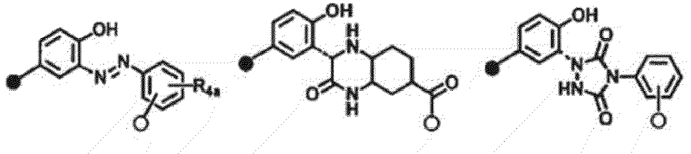 where R₄ₐ is a hydrogen atom or the substituent described above; and a symbol of "black circle" indicates a bond to T, and a symbol of "white circle" indicates a bond to B.

R₄ₐ is a hydrogen atom or the substituent described above. The definition, examples, and preferred examples of the substituent are similar to those described above.

The reactive group specific to a side chain of a tryptophan residue is a group capable of specifically reacting with a ring-forming atom at the 3-position of an indole group present in the side chain of the tryptophan residue; examples thereof include a 9-azabicyclo[3.3.1]nonan-3-one-N-oxyl residue.

More specifically, the reactive group specific to a side chain of a tryptophan residue may correspond to any one chemical structure selected from the group consisting of the following: 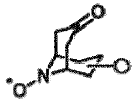 where a symbol of "white circle" indicates a bond to B.

A linking portion formed by a reaction between the chemical structure as the reactive group specific to a side chain of a tryptophan residue and the ring-forming atom at the 3-position of the indole group present in the side chain of the tryptophan residue may correspond to any one chemical structure selected from the group consisting of the following: 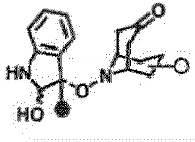 where a symbol of "black circle" indicates a bond to T, and a symbol of "white circle" indicates a bond to B.

The reactive group may be particularly preferably the reactive group specific to a side chain of lysine.

### 1-6. Partial Structure "L-B"

### 1-6-1. Length of Main Chain in Partial Structure "L-B" Linking A and R

In Formula (I), the length of a main chain linking A (the affinity substance) and R (the reactive group) (a linear chain portion in L-B) can be designed as appropriate in accordance with various factors such as the types of the antibody and the affinity substance and the relation between a target site of the affinity substance in the antibody and the positions and the number of the specific amino acid residues in the target region (e.g., the specific position) described above with which R reacts to be bound. The compound represented by Formula (I) can covalently bind to the antibody by causing the affinity substance to associate with the antibody and then causing the reactive group covalently binding to the affinity substance through L-B to react with a group in a side chain of the specific amino acid residue (e.g., an amino group in a side chain of a lysine residue) present near the target site. In this process, when another specific amino acid residue of the specific amino acid residue is not present in a region near the specific amino acid residue with which R reacts to be bound, a region near the target site, and a region between the specific amino acid residue and the target site, the reactive group can regioselectively bind to the specific amino acid residue without strictly controlling the length of the main chain. It is understood that even when another specific amino acid residue of the specific amino acid residue is present in such regions, the reactive group can regioselectively bind to the specific amino acid residue by controlling the length of the main chain.

The length of the main chain linking A and R, which can vary in accordance with factors such as the types of the antibody and the affinity substance thereto and the relation of the positions and the number of the specific amino acid residues in the target site in the antibody, may be about 5 angstroms or larger, preferably about 7.5 angstroms or larger, and more preferably about 10.5 angstroms or larger. The length of the main chain may be e.g., about 30 angstroms or smaller, preferably about 23 angstroms or smaller, and more preferably about 16.5 angstroms or smaller. More specifically, the length of the main chain may be e.g., about 5.0 to 30 angstroms, preferably about 7.5 to 23 angstroms, and more preferably about 10.5 to 16.5 angstroms.

By the way, it is a common technical knowledge in the technical field concerned that the relation of interatomic length (distance) is as the table below. Consequently, a person skilled in the art can design the main chain having atoms of the number corresponding to the length (angstrom) of the main chain described above as appropriate with reference to the interatomic lengths in the table below.

**Table 1. Relationship of length (distance) between carbon atoms present at both end in straight-chain alkylenes**

| | Length (Angstrom) |
|---|---|
| straight-chain alkylenes | |
| CH₂-CH₂ (C2) | about 1.5 |
| CH₂-CH₂-CH₂ (C3) | about 3.0 |
| C4 | about 4.5 |
| C5 | about 6.0 |
| C6 | about 7.5 |
| C7 | about 9.0 |
| C8 | about 10.5 |
| C9 | about 12.0 |
| C10 | about 13.5 |

| straight-chain alkenylene | |
|---|---|
| CH=CH | about 1.5 |

More specifically, the length of the main chain linking A and R can also be defined as the number of atoms forming the main chain (except hydrogen atoms and substituents). The number of atoms forming the main chain may be e.g., four (about 5.0 angstroms) or larger, preferably six (about 7.5 angstroms), and more preferably eight (about 10.5 angstroms) or larger. The number of atoms of the main chain may be e.g., 20 (about 30 angstroms) or smaller, preferably 16 (about 23 angstroms) or smaller, and more preferably 12 (about 16.5 angstroms) or smaller. More specifically, the number of atoms of the main chain may be e.g., 4 to 20, preferably 6 to 16, and more preferably 8 to 12.

When the main chain has a structure comprising no cyclic structure, the number of atoms of the main chain can be determined by counting the number of atoms in a chain structure.

On the other hand, when the main chain is a structure comprising a cyclic structure, the number of atoms of the main chain and the length described above do not necessarily correspond to each other, and the length that can be defined by the number of atoms of the main chain tends to be shorter than the length described above. Even in such a case, in view of defining the length of the main chain, the number of atoms of the main chain can be counted for convenience's sake. Specifically, the number of atoms of the main chain in such a case can be determined by counting the number of atoms of the shortest route connecting two bonds in the cyclic structure in addition to the number of atoms in a chain structure comprising no divalent cyclic structure in the main chain (e.g., refer to the (a) to (d) thick routes below). 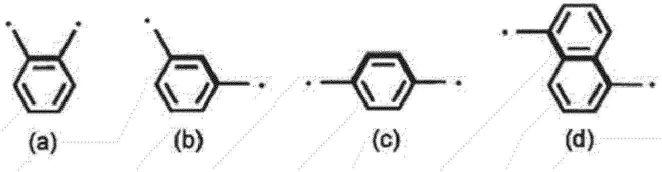 • is a bond.

In the case of (a), the shortest route is the thick route, and thus the number of atoms in the divalent cyclic structure counted as the number of atoms of the main chain is two.

In the case of (b), the shortest route is the thick route, and thus the number of atoms in the divalent cyclic structure counted as the number of atoms of the main chain is three.

In the case of (c), any route is the shortest route (the same distance), and thus the number of atoms in the divalent cyclic structure counted as the number of atoms of the main chain is four.

In the case of (d), the route of the condensed site is the shortest route, and thus the number of atoms in the divalent cyclic structure counted as the number of atoms of the main chain is four.

A linking portion of A and R represented by L-B (except a side chain) may be preferably a chain structure comprising no divalent cyclic structure. In this case, L and B can be designed as appropriate such that a linking chain portion of A and R represented by L-B comprises no divalent cyclic group.

### 1-6-2. Specific Structure of Partial Structure "L-B"

In Formula (I), L and B are structures that can be correlated with each other. Consequently, in Formula (I), L and B can be defined as a partial structure represented by "L-B."

In an embodiment, the cleavable linker may be (i) a cleavable linker which is a divalent group comprising a cleavable portion having the ability to form a bioorthogonal functional group on a reactive group side by cleavage or (ii) a cleavable linker which is a divalent group comprising a cleavable portion having no ability to form a bioorthogonal functional group on a reactive group side by cleavage.

In a specific embodiment, when L is the cleavable linker (i), B is (a) the divalent group comprising a bioorthogonal functional group or (b) the divalent group comprising no bioorthogonal functional group.

When L is the cleavable linker (i), B is preferably (a) the divalent group comprising a bioorthogonal functional group. In this case, the bioorthogonal functional group formed in (i) may be homogeneous or heterogeneous with respect to the bioorthogonal functional group in (a). In view of employing a simpler structure and/or improving reactivity to a single functional substance and the like, the bioorthogonal functional group formed in (i) may be homogeneous with respect to the bioorthogonal functional group in (a). On the other hand, in view of ensuring reactivity differentiated for two or more functional substances, non-use of a partial bioorthogonal functional group in the reaction, and the like, the bioorthogonal functional group formed in (i) may be heterogeneous with respect to the bioorthogonal functional group in (a).

Alternatively, when L is the cleavable linker (i), B may be (b) the divalent group comprising no bioorthogonal functional group. In this case, the compound represented by Formula (I) or a salt thereof has a simpler structure and is thus easily synthesized.

In another specific embodiment, when L is the cleavable linker (ii), B is (a) the divalent group comprising a bioorthogonal functional group.

In a specific embodiment, the partial structure represented by L-B preferably comprises no peptide portion. In this case, the antibody having a functional substance or functional substances according to the present invention (e.g., an antibody drug conjugate) obtained using the compound of the present invention has an advantage that it cannot comprise any peptide portion that can have immunogenicity as a linker.

In a specific embodiment, the partial structure represented by "L-B" may have a symmetrical structure [e.g., a cis form (that is, Z) and a trans form (that is E)] based on an atom present at the central position of the main chain linking A and R (the linear chain portion in L-B) (e.g., when the number of atoms forming the main chain is an odd number) or a bonding site present at the central position of the main chain (e.g., when the number of atoms forming the main chain is an even number). The bonding site present at the central position can be designed as the cleavable portion of the cleavable linker described above, for example. When the partial structure represented by "L-B" has a symmetrical structure, the partial structure represented by "L-B" can be easily synthesized. By reacting the same divalent groups each having a functional group capable of reacting to form a cleavable portion at one terminal (e.g., chain divalent groups each having an SH group at one terminal) with each other, a symmetrical structure comprising a cleavable portion at the central position of the main chain (chain divalent group-S-S-chain divalent group) can be achieved, for example.

Consequently, the partial structure represented by "L-B" may be a partial structure represented by "B2-L'-B1" (that is, L is a divalent group represented by B2-L', and B is B1). In this case, the compound represented by the above Formula (I) can be defined as a compound represented by the following Formula (I'):

A-B2-L'-B1-R (I')

wherein
A and R are the same as those of the above Formula (I),
   L' is a cleavable linker which is a divalent group comprising a cleavable portion,
B1 and B2 are the same as or different from each other, and are each (a) a divalent group comprising a bioorthogonal functional group or (b) a divalent group comprising no bioorthogonal functional group, and
   B1 and B2 may have a symmetrical structure with respect to L'.

B1 and B2 are the same as or different from each other; the definitions, examples, and preferred examples thereof are similar to those of B.

In a specific embodiment, L' may be represented by any one of the following Formulae (L1') to (L3'):

La'-C'-Lb' (L1')

La'-C' (L2')

C'-Lb' (L3')

wherein
La' and Lb' are each a divalent group, and
C' is a cleavable portion.

The definitions, examples, and preferred examples of the divalent groups represented by La' and Lb' are similar to those of the divalent groups represented by La and Lb, respectively.

The definition, examples, and preferred examples of the cleavable portion represented by C' are similar to those of the cleavable portion represented by C.

In another specific embodiment, a structural unit represented by L-B may be represented by the following Formula (LB'): 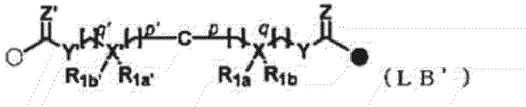 wherein
the definitions, examples, and preferred examples of C, p, p', q, q', X, X', R₁ₐ, 3R_{1a'}, R_{1b}, R_{1b'}, Y, Y', Z, and Z' are the same as those described above; and
a symbol of "white circle" indicates a bond to A, and a symbol of "black circle" indicates a bond to R.

Consequently, Formula (I) can be defined as the following Formula (I"): 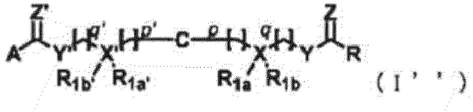 wherein
the definitions, examples, and preferred examples of A, R, C, p, p', q, q', X, X', R₁ₐ, R_{1b}, R_{1a'}, R_{1b'}, Y, Y', Z, and Z' are the same as those described above.

In Formulae (LB') and (I"), the length from C (a carbon atom) in C=Z to C (a carbon atom) in C=Z' is similar to the length of the main chain linking A and R. In these formulae, the length from C in C=Z to C in C=Z' can also be defined as the number of atoms forming a linking chain of a partial structure linking C in C=Z and C in C=Z' (except hydrogen atoms and substituents). The number of atoms is similar to the number of atoms forming the main chain linking A and R. The linking chain of a partial structure linking C in C=Z and C in C=Z' (except hydrogen atoms and substituents) may comprise no cyclic structure or comprise a cyclic structure and preferably comprises no cyclic structure. The linking chain (except hydrogen atoms and substituents) may preferably comprise no peptide portion.

### 1-7. Method of Production

The compound comprising an affinity substance to an antibody, a cleavable portion, and a reactive group, or a salt thereof can be prepared as appropriate. The compound comprising an affinity substance to an antibody, a cleavable portion, and a reactive group is represented by Formula (I), preferably Formula (I'), and more preferably Formula (I").

For the affinity substance, one having any functional group can be selected as appropriate. Consequently, using a reactive group capable of reacting with the functional group, the affinity substance is reacted with a structural unit represented by L-B-R or a structural unit represented by R-L-B-R (the two reactive groups are the same as or different from each other), whereby a structural unit represented by A-L-B-R can be prepared. Such a reaction can be conducted in an appropriate reaction system such as an organic solvent system or an aqueous solution system at an appropriate temperature (e.g., about 15°C to 200°C), for example. The reaction system may comprise an appropriate catalyst. The reaction time is e.g., 1 minute to 20 hours, preferably 10 minutes to 15 hours, more preferably 20 minutes to 10 hours, and even more preferably 30 minutes to 8 hours.

In the reaction system, a molar ratio (Y/X) of a structural unit represented by L-B-R or the structural unit represented by R-L-B-R (Y) to an affinity substance (X) is not limited to a particular ratio because it varies in accordance with the types of the structural unit and the affinity substance, the number of sites in the affinity substance to be modified with the structural unit, and the like; it is e.g., 0.1 to 50, preferably 0.5 to 40, more preferably 1 to 35, even more preferably 2 to 25, and particularly preferably 3 to 15.

Determination of the formation of the antibody comprising an affinity substance to the antibody and a cleavable portion, or a salt thereof, which depends on its specific raw materials and the molecular weight of a product, can be performed by electrophoresis, chromatography (e.g., gel permutation chromatography, ionexchange chromatography, reversed phase column chromatography, and high-performance liquid chromatography (HPLC)), or mass spectrometry, for example, and preferably mass spectrometry. The antibody comprising an affinity substance to the antibody and a cleavable portion, or a salt thereof can be purified as appropriate by any method such as chromatography (e.g., the pieces of chromatography described above and affinity chromatography).

### 1-8. Others

In the inventions described below [e.g., the inventions represented by Formulae (II) to (v), formulae having subordinate concepts thereof, and partial structural formulae (e.g., (L1) to (L3), (La'), (Lb'), and (B-1) to (B-4)], any symbols (e.g., A, L, B, and R), terms represented by the symbols, and the details thereof (e.g., definitions, examples, and preferred examples) are common to those of the invention of the compound represented by Formula (I) or a salt thereof. Specific portions (e.g., a cleavable portion and a portion having the ability to form a bioorthogonal functional group on a reactive group side by cleavage), specific groups (e.g., a bioorthogonal functional group, a divalent group, an alkyl group, a substituent, and an electron-withdrawing group), and specific values that can define the inventions described below and any technical elements such as a salt (e.g., definitions, examples, and preferred examples) can also be common to those described above. Consequently, these matters can be quoted as appropriate in the inventions described below without any special reference. Similarly, the technical elements of a specific invention described in the inventions described below can be quoted as appropriate as the technical elements of the present invention and other inventions.

### 2. Antibody Comprising Affinity Substance to Antibody and Cleavable Portion, or Salt thereof

### 2-1. Outline

The present invention provides an antibody comprising an affinity substance to the antibody and a cleavable portion, represented by Formula (II), or a salt thereof.

A-L-B-R'-T (II)

wherein
A is an affinity substance to an antibody,
L is a cleavable linker which is a divalent group comprising a cleavable portion,
B is (a) a divalent group comprising a bioorthogonal functional group or (b) a divalent group comprising no bioorthogonal functional group,
R' is a portion formed by a reaction between an antibody and a reactive group, and
T is an antibody.

### 2-2. Portion Formed by Reaction between Antibody and Reactive Group (R')

In a portion formed by a reaction between an antibody and a reactive group, the definitions, examples, and preferred examples of the antibody and the reactive group are as described above. The portion formed by a reaction between an antibody and a reactive group is a common technical knowledge in the technical field concerned and can be determined as appropriate in accordance with the types of the antibody and the reactive group.

The portion formed by a reaction between an antibody and a reactive group is preferably a portion formed by a reaction between a side chain of any one amino acid of 14 amino acids (asparagine, glutamine, methionine, proline, serine, threonine, tryptophan, tyrosine, aspartic acid, glutamic acid, arginine, histidine, and lysine) that can be contained in the antibody and a reactive group thereto.

The portion formed by a reaction between an antibody and a reactive group may be more preferably a portion formed by a reaction between a side chain of any one amino acid of lysine, tyrosine, tryptophan, and cysteine and a reactive group specific thereto.

The portion formed by a reaction between an antibody and a reactive group may be even more preferably a portion formed by a reaction between a side chain of any one amino acid of lysine, tyrosine, and tryptophan and a reactive group specific thereto. Examples of the portion formed by a reaction between a side chain of any one amino acid of lysine, tyrosine, and tryptophan and a reactive group specific thereto include the linking portion and/or chemical structure described in "1-5. Reactive Group (R)."

The portion formed by a reaction between an antibody and a reactive group may be still even more preferably a portion formed by a reaction between a side chain of lysine or tyrosine and a reactive group specific thereto (in particular, when the antibody is human IgG such as human IgG1). Examples of the portion formed by a reaction between a side chain of lysine or tyrosine and a reactive group specific thereto include the linking portion and/or chemical structure described in "1-5. Reactive Group (R)."

The portion formed by a reaction between an antibody and a reactive group may be particularly preferably a portion formed by a reaction between a side chain of lysine and a reactive group specific thereto.

### 2-3. Partial Structure "L-B"

The details of the partial structure "L-B" are as described in "1-6. Partial Structure "L-B"."

In a specific embodiment, the partial structure represented by "L-B" may be a partial structure represented by "B2-L'-B1" (that is, L is a divalent group represented by B2-L', and B is B1). In this case, the antibody comprising an affinity substance to the antibody and a cleavable portion, represented by the above Formula (II), or a salt thereof can be represented by the following Formula (II'):

A-B2-L'-B1-R'-T (II')

wherein
A, R', and T are the same as those of the above Formula (II),
L' is a cleavable linker which is a divalent group comprising the cleavable portion,
B1 and B2 are the same as or different from each other, and are each (a) a divalent group comprising a bioorthogonal functional group or (b) a divalent group comprising no bioorthogonal functional group, and
B1 and B2 may have a symmetrical structure with respect to L'.

In the above Formula (II'), L' may be represented by any one of Formulae (L1') to (L3') described above.

In another specific embodiment, a structural unit represented by L-B may be represented by the following Formula (LB'): 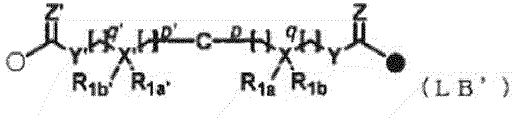 wherein
the definitions, examples, and preferred examples of C, p, p', q, q', X, X', R₁ₐ, R_{1a'}, R_{1b}, R_{1b'}, Y, Y', Z, and Z' are the same as those described above, and
a symbol of "white circle" indicates a bond to A, and a symbol of "black circle" indicates a bond to R'.

Consequently, the above Formula (II) can be defined as the following Formula (II"): 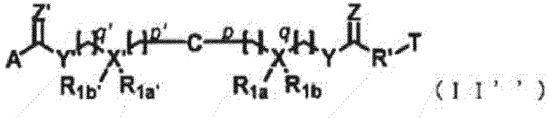 wherein
the definitions, examples, and preferred examples of A, R', T, C, p, p', q, q', X, X', R₁ₐ, R_{1b}, R_{1a'}, R_{1b'}, Y, Y', Z, and Z' are the same as those described above.

In Formulae (LB') and (II"), the length from C (a carbon atom) in C=Z to C (a carbon atom) in C=Z' is similar to the length of the main chain linking A and R described above. In these formulae, the length from C in C=Z to C in C=Z' can also be defined as the number of atoms forming a linking chain of a partial structure linking C in C=Z and C in C=Z' (except hydrogen atoms and substituents). The number of atoms is similar to the number of atoms forming the main chain linking A and R. The linking chain of a partial structure linking C in C=Z and C in C=Z' (except hydrogen atoms and substituents) may comprise no cyclic structure or comprise a cyclic structure and preferably comprises no cyclic structure. The linking chain (except hydrogen atoms and substituents) may preferably comprise no peptide portion.

### 2-4. Binding Site of Partial Structure Other Than Antibody, Possessed by Antibody (Regioselectivity)

A partial structure other than the antibody (e.g., A-L-B-R') can regioselectively bind to the target region described above in the antibody (T).

In the present specification, "regioselective" or "regioselectivity" refers to a state in which even though a specific amino acid residue is not present locally at a specific region in the antibody, a certain structural unit capable of binding to the specific amino acid residue in the antibody is present locally at a specific region in the antibody. Consequently, expressions related to regioselectivity such as "regioselectively having," "regioselective binding," and "binding with regioselectivity" mean that the possession rate or the binding rate of a certain structural unit in the target region comprising one or more specific amino acid residues is higher at a significant level than the possession rate or the binding rate of the structural unit in the non-target region comprising a plurality of amino acid residues homogeneous with respect to the specific amino acid residues in the target region. Such regioselective binding or possession can be achieved by the present invention, which enables the certain structural unit to preferentially react with the specific amino acid residues in the target region in the antibody, not causing the certain structural unit to randomly react with the specific amino acid residues in the antibody.

Specifically, when T comprises one or more specific amino acid residues in a target region consisting of 1 to 50 consecutive amino acid residues, and five or more of the specific amino acid residues in a non-target region other than the target region, the partial structure other than the antibody can be bound to the one or more specific amino acid residues contained in the target region with 30% or more regioselectivity. The definitions, examples, and preferred examples of the target region and regioselectivity are as described above.

### 2-5. Number of Partial Structures Other Than Antibody, Possessed by Antibody

The number of partial structures (e.g., A-L-B-R') other than the antibody, possessed by the antibody (T) can vary. When T is a multimeric protein comprising a plurality of monomeric proteins, for example, T can have the partial structure other than T in a plurality of corresponding target regions in the monomeric proteins. Consequently, T can have a plurality of partial structures other than T. Consequently, the structure represented by Formula (II), (II'), or (II") indicates that T may have one or a plurality of partial structures other than T. The number of partial structures other than T possessed by T can be adjusted by setting the type of the antibody and conditions such as a reaction ratio between the antibody and a structural unit to be introduced thereto as appropriate. Such a number, which varies depending on the type of the antibody, may be e.g., one to eight, preferably one to four, and more preferably one or two.

In a specific embodiment, when the antibody is a multimeric protein comprising a plurality of monomeric proteins, the antibody may possess a plurality of partial structures other than the antibody. The present invention can introduce a plurality of partial structures other than the antibody to the same target region of the monomeric proteins.

In a preferred embodiment, the antibody may be an antibody comprising a plurality of heavy chains. The definition, examples, and preferred examples of the antibody are as described above. The number of heavy chains varies depending on the type of the antibody. IgG, IgE, and IgD can have two heavy chains, for example. IgA can have two or four heavy chains. IgM can have eight heavy chains. The number of partial structures other than the antibody, possessed by the antibody (the antibody) can be considered to have the same meaning as DAR. In the present invention, the number of partial structures other than the antibody, possessed by the antibody may be one or two (preferably two) for IgG, IgE, and IgD, one to four (preferably four) for IgA, and one to eight (preferably eight) for IgM.

### 2.6 Method of Production

The present invention provides a method for producing an antibody comprising an affinity substance to the antibody and a cleavable portion, or a salt thereof.

(A1) Reacting a compound having an affinity substance to an antibody, a cleavable portion, and a reactive group, or a salt thereof with an antibody to form an antibody having an affinity substance to the antibody and a cleavable portion, or a salt thereof.

The compound comprising an affinity substance to an antibody, a cleavable portion, and a reactive group is represented by Formula (I), preferably Formula (I'), and more preferably Formula (I"). The antibody comprising an affinity substance to the antibody and a cleavable portion is represented by Formula (II), preferably Formula (II'), and more preferably Formula (II").

The compound comprising an affinity substance to an antibody, a cleavable portion, and a reactive group,or a salt thereof has the reactive group, and therefore can react with an antibody. Such a reaction can be conducted as appropriate under a condition incapable of causing denaturation or decomposition (e.g., cleavage of an amide bond) of proteins (a mild condition). Such a reaction can be conducted in an appropriate reaction system such as a buffer at room temperature (e.g., about 15°C to 30°C), for example. The pH of the buffer is e.g., 5 to 9, preferably 5.5 to 8.5, and more preferably 6.0 to 8.0. The buffer may comprise an appropriate catalyst. The reaction time is e.g., 1 minute to 20 hours, preferably 10 minutes to 15 hours, more preferably 20 minutes to 10 hours, and even more preferably 30 minutes to 8 hours. For the details of such a reaction, refer to G. J. L. Bernardes et al., Chem. Rev., 115, 2174 (2015); G. J. L. Bernardes et al., Chem. Asian. J., 4,630 (2009); B. G. Davies et al., Nat. Commun., 5, 4740 (2014); A. Wagner et al., Bioconjugate. Chem., 25,825 (2014), for example.

In the reaction system, a molar ratio (Y/X) of a compound comprising an affinity substance to an antibody, a cleavable portion, and a reactive group, or a salt thereof (Y) to an antibody (X) is not limited to a particular ratio because it varies in accordance with the types of the compound comprising an affinity substance to an antibody, a cleavable portion, and a reactive group and the antibody, the number of sites in the antibody to be modified with the compound comprising an affinity substance to an antibody, a cleavable portion, and a reactive group (e.g., DAR), and the like; it is e.g., 0.1 to 100, preferably 0.5 to 80, more preferably 1 to 70, even more preferably 2 to 50, and particularly preferably 3 to 30.

Determination of the formation of the antibody comprising an affinity substance to the antibody and a cleavable portion, or a salt thereof, which depends on its specific raw materials and the molecular weight of a product, can be performed by electrophoresis, chromatography (e.g., gel permutation chromatography, ionexchange chromatography, reversed phase column chromatography, and high-performance liquid chromatography (HPLC)), or mass spectrometry, for example, and preferably mass spectrometry. Determination of regioselectivity can be performed by peptide mapping, for example. Peptide mapping can be performed by protease (e.g., trypsin and chymotrypsin) treatment and mass spectrometry, for example. For the protease, an endoprotease is preferred. Examples of such an endoprotease include trypsin, chymotrypsin, Glu-C, Lys-N, Lys-C, and Asp-N. Determination of the number of partial structures other than the antibody, possessed by the antibody can be performed by electrophoresis, chromatography, or mass spectrometry, for example, and preferably mass spectrometry. The antibody comprising an affinity substance to the antibody and a cleavable portion, or a salt thereof can be purified as appropriate by any method such as chromatography (e.g., the pieces of chromatography described above and affinity chromatography).

### 3. Conjugate Having Affinity Substance to Antibody, Cleavable Portion, Functional Substance, and Antibody, or Salt thereof

### 3-1. Outline

The present invention provides a conjugate having an affinity substance to an antibody, a cleavable portion, a functional substance, and an antibody, represented by Formula (III), or a salt thereof.

A-L-B'(-F)-R'-T (III)

wherein A is an affinity substance to an antibody,
L is a cleavable linker which is a divalent group comprising a cleavable portion,
B' is a trivalent group comprising a portion formed by a reaction between a functional substance and a bioorthogonal functional group,
F is a functional substance,
R' is a portion formed by a reaction between an antibody and a reactive group, and
T is an antibody, or
a salt thereof.

In Formula (III) or other formulae, R' covalently binds to B' not F.

### 3-2. Functional substance (F)

The functional substance is not limited to a particular substance so long as it is a substance imparting any function to the antibody; examples thereof include drugs, labelling substances, and stabilizers; preferred are drugs and labelling substances. The functional substance may be a single functional substance or a substance in which two or more functional substances are coupled with each other.

The drug may be a drug to any disease. Examples of such a disease include cancer (e.g., lung cancer, stomach cancer, colon cancer, pancreatic cancer, renal cancer, liver cancer, thyroid cancer, prostatic cancer, bladder cancer, ovarian cancer, uterine cancer, bone cancer, skin cancer, a brain tumor, and melanoma), autoimmune diseases and inflammatory diseases (e.g., allergic diseases, articular rheumatism, and systemic lupus erythematosus), brain or nerve diseases (e.g., cerebral infarction, Alzheimer's disease, Parkinson disease, and amyotrophic lateral sclerosis), infectious diseases (e.g., microbial infectious diseases and viral infectious diseases), hereditary rare diseases (e.g., hereditary spherocytosis and nondystrophic myotonia), eye diseases (e.g., age-related macular degeneration, diabetic retinopathy, and retinitis pigmentosa), diseases in the bone and orthopedic field (e.g., osteoarthritis), blood diseases (e.g., leukosis and purpura), and other diseases (e.g., diabetes, metabolic diseases such as hyperlipidemia, liver diseases, renal diseases, lung diseases, circulatory system diseases, and digestive system diseases). When the antibody is an antibody to a target protein of a certain disease, the drug may be a drug related to the certain disease (e.g., a drug treating the certain disease and a drug relaxing side effects accompanying use of the antibody to the target protein of the certain disease).

More specifically, the drug is an anti-cancer agent. Examples of the anti-cancer agent include chemotherapeutic agents, toxins, and radioisotopes or substances comprising them. Examples of chemotherapeutic agents include DNA injuring agents, antimetabolites, enzyme inhibitors, DNA intercalating agents, DNA cleaving agents, topoisomerase inhibitors, DNA binding inhibitors, tubulin binding inhibitors, cytotoxic nucleosides, and platinum compounds. Examples of toxins include bacteriotoxins (e.g., diphtheria toxin) and phytotoxins (e.g., ricin). Examples of radioisotopes include radioisotopes of a hydrogen atom (e.g., ³H), radioisotopes of a carbon atom (e.g., ¹⁴C), radioisotopes of a phosphorous atom (e.g., ³²P), radioisotopes of a sulfur atom (e.g., 35^{S}), radioisotopes of yttrium (e.g., ⁹⁰Y), radioisotopes of technetium (e.g., ^{99m}Tc), radioisotopes of indium (e.g., ¹¹¹In), radioisotopes of an iodide atom (e.g., ¹²³I, ¹²⁵I, ¹²⁹I, and ¹³¹I), radioisotopes of samarium (e.g., ¹⁵³Sm), radioisotopes of rhenium (e.g., ¹⁸⁶Re), radioisotopes of astatine (e.g., ²¹¹At), and radioisotopes of bismuth (e.g., ²¹²Bi). More specifically, examples of the drug include auristatin (MMAE, MMAF), maytansine (DM1, DM4), pyrrolobenzodiazepine (PBD), IGN, camptothecin analog, calicheamicin, duocarmicin, eribulin, anthracycline, dmDNA31, and tubricin.

Examples of labelling substances include enzymes (e.g., peroxidase, alkaline phosphatase, luciferase, and β-galactosidase), affinity substances (e.g., streptavidin, biotin, digoxigenin, and aptamer), fluorescent substances (e.g., fluorescein, fluorescein isothiocyanate, rhodamine, green-fluorescent protein, and red-fluorescent protein), luminescent substances (e.g., luciferin, aequorin, acridinium ester, tris(2,2'-bipyridyl)ruthenium, and luminol), and radioisotopes (e.g., those described above) or substances comprising them.

The functional substance is a large compound, a middle compound, or a small compound and is preferably a small compound. The small compound refers to compounds with a molecular weight of 1,500 or lower. The small compound is a natural compound or a synthesized compound. The molecular weight of the small compound may be 1,200 or lower, 1,000 or lower, 900 or lower, 800 or lower, 700 or lower, 600 or lower, 500 or lower, 400 or lower, or 300 or lower. The molecular weight of the small compound may be 30 or higher, 40 or higher, or 50 or higher. The small compound may be any of the drugs or labelling substances described above. Examples of the small compound include amino acids, oligopeptides, vitamins, nucleosides, nucleotides, oligonucleotides, monosaccharides, oligosaccharides, lipids, fatty acids, and salts thereof.

### 3-3. Trivalent Group Comprising Portion Formed by Reaction between Functional substance and Bioorthogonal functional group (B')

B' is similar to (a) the divalent group comprising a bioorthogonal functional group in B described above except that the bioorthogonal functional group in (a) has reacted with the functional substance. Consequently, the definitions, examples, and preferred examples of the trivalent group and the orthogonal functional group in B' are similar to those in B except that the bioorthogonal functional group of (a) reacts with the functional substance.

The functional substance has various functional groups corresponding to its structure. When the functional substance has a functional group easily reacting with the bioorthogonal functional group, the functional group of the functional substance and the bioorthogonal functional group can be reacted with each other as appropriate. The function group easily reacting with the bioorthogonal functional group can vary depending on a specific type of the bioorthogonal functional group. A person skilled in the art can select an appropriate functional group as the functional group easily reacting with the bioorthogonal functional group as appropriate (e.g., Boutureira et al., Chem. Rev., 2015, 115, 2174-2195). Examples of the functional group easily reacting with the bioorthogonal functional group include, but are not limited to, a maleimide residue and a disulfide residue when the bioorthogonal functional group is a thiol residue, an azide residue when the bioorthogonal functional group is an alkyne residue, and a hydrazine residue when the bioorthogonal functional group is an aldehyde residue or a ketone residue. For example, when the bioorthogonal functional group is a thiol residue and the functional group easily reacting with the bioorthogonal functional group is a maleimide residue or a disulfide residue (or vice versa), the trivalent group comprising a portion formed by a reaction between the functional substance and the bioorthogonal functional group may be a trivalent group comprising a thiosuccinimide residue or a trivalent group comprising a disulfide residue; when the bioorthogonal functional group is an alkyne residue and the functional group easily reacting with the bioorthogonal functional group is an azide residue (or vice versa), the trivalent group comprising a portion formed by a reaction between the functional substance and the bioorthogonal functional group may be a trivalent group comprising a triazole residue (which may be condensed with another ring or is not necessarily condensed therewith); when the bioorthogonal functional group is an aldehyde residue or a ketone residue and the functional group easily reacting with the bioorthogonal functional group is a hydrazine residue (or vice versa), the trivalent group comprising a portion formed by a reaction between the functional substance and the bioorthogonal functional group may be a trivalent group comprising a hydrazone residue (e.g., Boutureira et al., Chem. Rev., 2015, 115, 2174-2195). The trivalent group comprising a thiosuccinimide residue, the trivalent group comprising a disulfide residue, the trivalent group comprising a triazole residue (which may be condensed with another ring or is not necessarily condensed therewith), or the trivalent group comprising a hydrazone residue is a preferred example of the trivalent group comprising a portion formed by a reaction between the functional substance and the bioorthogonal functional group.

On the other hand, when the functional substance has no functional group easily reacting with the bioorthogonal functional group, a substance derivatized so as to have a desired functional group can be used as the functional substance. For example, when the functional substance is an antibody, a substance derivatized so as to have a functional group that the antibody does not naturally have can be used. In this case, derivatization of the antibody may be performed by the method of the present invention. In this case, an antibody derivatized so as to regioselectively have a desired bioorthogonal functional group can be used as the functional substance.

Derivatization is a common technical knowledge in the field concerned (e.g., WO 2004/010957, United States Patent Application Publication No. 2006/0074008, and United States Patent Application Publication No. 2005/0238649). Derivatization may be performed using the cross-linking agent described above, for example. Alternatively, derivatization may be performed using a specific linker having a desired functional group. Such a linker may be able to separate the functional substance and the antibody from each other through the cleavage of the linker under an appropriate condition (e.g., intracellular or extracellular), for example. Examples of such a linker include peptidyl linkers decomposed by specific proteases [e.g., intracellular proteases (e.g., proteases present in lysosome or endosome) and extracellular proteases (e.g., secretory proteases)] (e.g., United States Patent No. 6,214,345; Dubowchik et al., Pharm. Therapeutics 83: 67-123 (1999)) and linkers capable of being cleaved at local acidic sites present in living bodies (e.g., Unites States Patent Nos. 5,622,929, 5,122,368, and 5,824,805). The linker may be self-immolative (e.g., WO 02/083180, WO 04/043493, and WO 05/112919). In the present invention, the derivatized functional substance can also be referred to simply as the "functional substance."

In the trivalent group comprising a portion formed by a reaction between a functional substance and a bioorthogonal functional group, the definitions, examples, and preferred examples of the functional substance and the bioorthogonal functional group are as described above. The portion formed by a reaction between a functional substance and a bioorthogonal functional group is a common technical knowledge in the technical field concerned and can be determined as appropriate in accordance with the types of the functional substance (when the functional substance is derivatized, its derivatized portion) and the bioorthogonal functional group.

The trivalent group comprising a portion formed by a reaction between a functional substance and a bioorthogonal functional group (1) may be a trivalent group comprising a thiosuccinimide residue, a triazole residue, or a hydrazone residue, referred to in the above-described preferred examples, or (2) which is not limited to a particular group, may be a trivalent group comprising a residue selected from the group consisting of a disulfide residue (this residue has been referred to in the above-described preferred examples), an acetal residue, a ketal residue, an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue, for example.

The trivalent group comprising a portion formed by a reaction between a functional substance and a bioorthogonal functional group is not particularly limited, but may be, for example, a trivalent group comprising a residue corresponding to any one chemical structure selected from the group of the following: 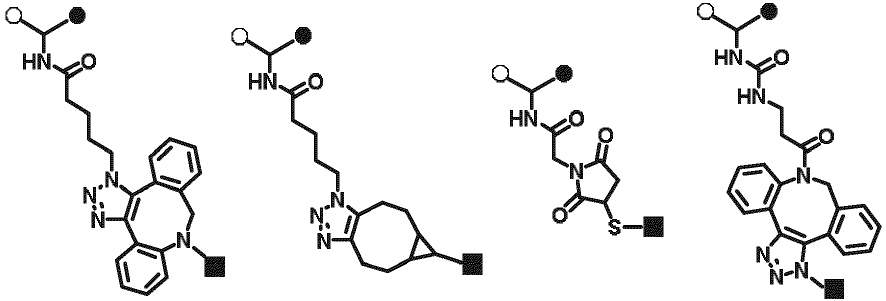 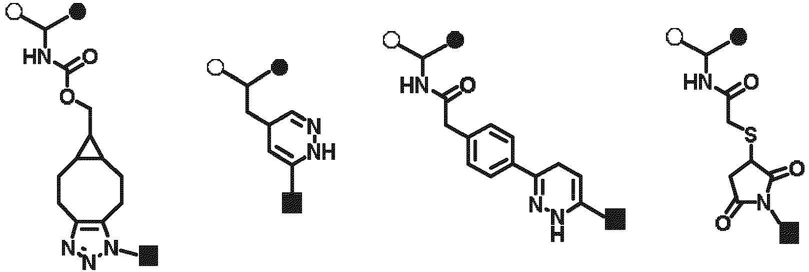
where a symbol of "black square" indicates a bond to a functional substance-side portion, and
a symbol of "white circle" indicates a bond to an L-side portion and a symbol of "black circle" indicates a bond to an R'-side portion, and
when the chemical structure is asymmetrical with respect to the cleavable portion, a symbol of "black circle" may indicate a bond to an L-side portion and a symbol of "white circle" may indicate a bond to an R'-side portion.

### 3-4. Partial Structure "L-B'(-F)"

The details of a partial structure "L-B'(-F)" are as described in "1-6. Partial Structure "L-B"" except that B is changed to B' (-F) .

In an embodiment, the cleavable linker may be (i) a cleavable linker which is a divalent group comprising a cleavable portion having the ability to form a bioorthogonal functional group on a reactive group side by cleavage or (ii) a cleavable linker which is a divalent group comprising a cleavable portion having no ability to form a bioorthogonal functional group on a reactive group side by cleavage.

In a specific embodiment, the cleavable linker may be the cleavable linker (ii). This is because B' already binds to the functional substance (F), and there is no need to form the bioorthogonal functional group on the reactive group side.

In another specific embodiment, the partial structure represented by L-B'(-F) may be a partial structure represented by B2'(-F2)-L'-B1'(-F1) (that is, L is B2' (-F2) -L', and B' is B1'). In this case, the conjugate having an affinity substance to an antibody, a cleavable portion, a functional substance, and an antibody, represented by the above Formula (III), or a salt thereof can be represented by the following Formula (III'):

A-B2'(-F2)-L'-B1'(-F1)-R'-T (III')

wherein A, R', and T are the same as those of the above Formula (III),
L' is a cleavable linker which is a divalent group comprising a cleavable portion,
B1' and B2' are the same as or different from each other, and are each a trivalent group comprising a portion formed by a reaction between a functional substance and a bioorthogonal functional group,
F1 and F2 are the same as or different from each other, and are each a functional substance, and
B1' (-F1) and B2' (-F2) may have a symmetrical structure with respect to L'.

B1' and B2' are the same as or different from each other; the definitions, examples, and preferred examples thereof are similar to those of B'.

In Formula (III'), L' may be represented by any one of Formulae (L1') to (L3') described above.

In another specific embodiment, a structural unit represented by L-B'(-F) may be represented by the following Formula (LB'(F)'): 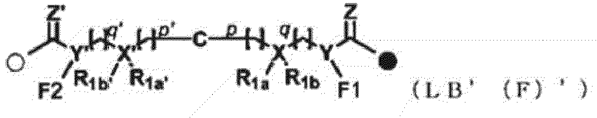 wherein
the definitions, examples, and preferred examples of C, F1, F2, p, p', q, q', X, X', R₁ₐ, R_{1a'}, R_{1b}, R_{1b'}, Z, and Z' are the same as those described above,
Y and Y' are the same as or different from each other, and are each a residue obtained by removing one hydrogen atom from Y of the above Formula (B-1),
a symbol of "white circle" indicates a bond to A, and a symbol of "black circle" indicates a bond to R'.

More specifically, the residue obtained by removing one hydrogen atom from Y of the above Formula (B-1) in Y and Y' is N(-)-, -CH(-)-, or a group obtained by removing one hydrogen atom from the above Formula (B-2). In view of simplifying the structure and the like, Y may be -N(-)- or -CH(-)-. Alternatively, in view of designing a carbon atom-based structure and the like, Y may be -CH(-)- or a group obtained by removing one hydrogen atom from Formula (B-2).

The group obtained by removing one hydrogen atom from the above Formula (B-2) is a group obtained by removing one hydrogen atom from the following Formula (B-2') : 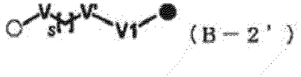
wherein V and V' are the same as or different from each other, and are each -NH-, -O-, -CH₂-, or a single bond,
V1 is (a) a divalent group comprising a bioorthogonal functional group or (b) a divalent group comprising no bioorthogonal functional group,
s is any integer of 0 to 10, and
a symbol of "white circle" and a symbol of "black circle" in Formula (B-2') have the same orientation as a symbol of "white circle" and a symbol of "black circle" in Formula (B-1), respectively. The definition, examples, and preferable examples of s in Formula (B-2') are similar to those in Formula (B-2). Consequently, in Formula (B-2'), one of V and V' may be -N(-)- or -CH(-)-. Alternatively, in Formula (B-2'), V1 may be a trivalent group obtained by removing one hydrogen atom from the divalent group (a) or (b) . Alternatively, in Formula (B-2'), one of s -CH₂-s may be -CH(-)-.

In this case, the above Formula (III) can be defined as the following Formula (III''): 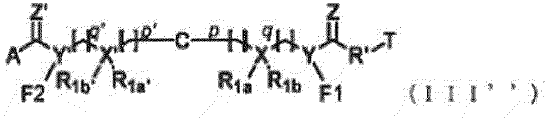 wherein
the definitions, examples, and preferred examples of A, R, C, F1, F2, p, p', q, q', X, X', R₁ₐ, R_{1b}, R₁ₐ., R_{1b'}, Y, Y', Z, and Z' are the same as those described above.

In the above Formulae (LB'(F)') and (III'), the length from C (a carbon atom) in C=Z to C (a carbon atom) in C=Z' is similar to the length of the main chain linking A and R described above. In these formulae, the length from C in C=Z to C in C=Z' can also be defined as the number of atoms forming a linking chain of a partial structure linking C in C=Z and C in C=Z' (except hydrogen atoms and substituents). The number of atoms is similar to the number of atoms forming the main chain linking A and R. The linking chain of a partial structure linking C in C=Z and C in C=Z' (except hydrogen atoms and substituents) may comprise no cyclic structure or comprise a cyclic structure and preferably comprises no cyclic structure. The linking chain (except hydrogen atoms and substituents) may preferably comprise no peptide portion.

### 3-5. Binding Site of Partial Structure Other Than Antibody, Possessed by Antibody (Regioselectivity)

A partial structure other than the antibody (e.g., A-L-B'(-F)-R') can regioselectively bind to the target region described above in the antibody (T). Specifically, when T comprises one or more specific amino acid residues in a target region consisting of 1 to 50 consecutive amino acid residues, and five or more of the specific amino acid residues in a non-target region other than the target region, the partial structure other than the antibody can be bound to the one or more specific amino acid residues contained in the target region with 30% or more regioselectivity. The definitions, examples, and preferred examples of the target region and regioselectivity are as described above.

### 3-6. Number of Partial Structures Other Than Antibody, Possessed by Antibody

The number of partial structures (e.g., A-L-B'(-F)-R') other than the antibody, possessed by the antibody (T) can vary. When T is a multimeric protein comprising a plurality of monomeric proteins, for example, T can have the partial structure other than T in a plurality of corresponding target regions in the monomeric proteins. Consequently, T can have a plurality of partial structures other than T. Consequently, the structure represented by Formula (III), (III'), or (III'') indicates that T may have one or a plurality of partial structures other than T. The number of partial structures other than T possessed by T can be adjusted by setting the type of the antibody and conditions such as a reaction ratio between the antibody and a structural unit to be introduced thereto as appropriate. Such a number, which varies depending on the type of the antibody, may be e.g., one to eight, preferably one to four, and more preferably one or two.

In a specific embodiment, when the antibody is a multimeric protein comprising a plurality of monomeric proteins, the antibody may possess a plurality of partial structures other than the antibody. The present invention can introduce a plurality of partial structures other than the antibody to the same target region of the monomeric proteins.

In a preferred embodiment, the antibody may be an antibody comprising a plurality of heavy chains. The definition, examples, and preferred examples of the antibody are as described above. In the present invention, the number of partial structures other than the antibody, possessed by the antibody may be one or two (preferably two) for IgG, IgE, and IgD, one to four (preferably four) for IgA, and one to eight (preferably eight) for IgM.

### 3-7. Method of Production

The present invention provides a method for producing a conjugate having an affinity substance to an antibody, a cleavable portion, a functional substance, and an antibody, or a salt thereof, the method comprising:
(B1) reacting an antibody comprising an affinity substance to the antibody and a cleavable portion, or a salt thereof with a functional substance to form a conjugate having an affinity substance to an antibody, a cleavable portion, a functional substance, and an antibody, or a salt thereof.

The antibody comprising an affinity substance to the antibody and a cleavable portion is represented by Formula (II), preferably Formula (II'), and more preferably Formula (II''). The conjugate having an affinity substance to an antibody, a cleavable portion, a functional substance, and an antibody is represented by Formula (III), preferably Formula (III'), and more preferably Formula (III'').

The antibody comprising an affinity substance to the antibody and a cleavable portion, or a salt thereof has a bioorthogonal functional group capable of reacting with a functional substance and can thus react with a functional substance. Such a reaction can be conducted as appropriate under a condition incapable of causing denaturation or decomposition (e.g., cleavage of an amide bond) of proteins (a mild condition) described in "2-6. Method of Production."

In the reaction system, a molar ratio (Y/X) of a functional substance (Y) to an antibody comprising an affinity substance to the antibody and a cleavable portion, or a salt thereof (X) is not limited to a particular ratio because it varies in accordance with the types of the antibody and the functional substance, the number of sites in the antibody to be modified with the functional substance (e.g., DAR), and the like; it is e.g., 0.1 to 100, preferably 0.5 to 80, more preferably 1 to 70, even more preferably 2 to 50, and particularly preferably 3 to 30.

Determination of the formation of the conjugate having an affinity substance to an antibody, a cleavable portion, a functional substance, and an antibody, or a salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity, determination of the number of partial structures other than the antibody, and purification can be performed as appropriate by the methods described in "2-6. Method of Production."

The present invention also provides a method for producing a conjugate having an affinity substance to an antibody, a cleavable portion, a functional substance, and an antibody, or a salt thereof, the method comprising:
(B2) reacting a compound having an affinity substance to an antibody, a cleavable portion, and a reactive group, or a salt thereof with an antibody to form an antibody having an affinity substance to the antibody and a cleavable portion, or a salt thereof; and
(B3) reacting the antibody comprising an affinity substance to the antibody and a cleavable portion, or a salt thereof with a functional substance to form a conjugate having an affinity substance to an antibody, a cleavable portion, a functional substance, and an antibody, or a salt thereof.

The process of (B2) can be performed in a manner similar to the process of (A1) described in "2-6. The process of (B3) can be performed in a manner similar to the process of (B1). The processes of (B2) and (B3) can be separately performed. Alternatively, the processes of (B2) and (B3) can be simultaneously performed in accordance with a combination of a reaction pair of a specific amino acid residue as a reaction target in the antibody and the reactive group and a reaction pair of a functional group in the functional substance and the bioorthogonal functional group. That is, when the processes of (B2) and (B3) are performed at the same time, a product-containing reaction solution obtained in the process of (B2) may be subjected to the process of (B3) without isolating the product obtained in the process of (B2).

### 4. Antibody Having Bioorthogonal Functional Group or Salt thereof

### 4-1. Outline of Method of Production

The present invention provides a method for producing an antibody having a bioorthogonal functional group or bioorthogonal functional groups, represented by Formula (IV), or a salt thereof.

L1-B-R'-T (IV)

wherein
L1 is (i') a monovalent group comprising a bioorthogonal functional group or (ii') a monovalent group comprising no bioorthogonal functional group,
B is (a) a divalent group comprising a bioorthogonal functional group or (b) a divalent group comprising no bioorthogonal functional group,
R' is a portion formed by a reaction between the antibody and a reactive group, and
T is the antibody.

### 4-2. (i') Monovalent Group Comprising Bioorthogonal functional group or (ii') Monovalent Group Comprising No Bioorthogonal functional group (L1)

In Formula (IV), L1 is (i') a monovalent group comprising a bioorthogonal functional group or (ii') a monovalent group comprising no bioorthogonal functional group. Such a monovalent group is a monovalent group that can be formed by cleavage of the cleavable linker which is a divalent group comprising a cleavable portion described above. Consequently, L1 may be a monovalent group that can be formed by cleavage of the residue or chemical structure of the cleavable portion described above. More specifically, (i') the monovalent group comprising a bioorthogonal functional group may be a monovalent group that can be formed by cleavage of (i) the cleavable linker which is a divalent group comprising a cleavable portion having the ability to form a bioorthogonal functional group on a reactive group side by cleavage described above; (ii') the monovalent group comprising no bioorthogonal functional group may be a monovalent group that can be formed by cleavage of the cleavable linker (ii) which is a divalent group comprising a cleavable portion having no ability to form a bioorthogonal functional group on a reactive group side by cleavage.

In a specific embodiment, L1 may be represented by the following Formula (L1-1) or (L1-2):

C1-Lb (L1-1)

C1 (L1-2)

wherein
Lb is a divalent group, and
C1 is a bioorthogonal functional group or a group other than the bioorthogonal functional group.

Examples of the divalent group include a divalent hydrocarbon group optionally having a substituent, a divalent heterocyclic group optionally having a substituent, -C(=O)-, -NRₐ- (Rₐ indicates a hydrogen atom or a substituent), -O-, -S-, -C(=S)-, and a group consisting of a combination of two or more (e.g., two to eight, preferably two to six, and more preferably two to four) of these. The definitions, examples, and preferred examples of the divalent hydrocarbon group, the divalent heterocyclic group, and the substituent are as described above.

The definition, examples, and preferred examples of the bioorthogonal functional group are as described above.

Examples of the group other than the bioorthogonal functional group include, among the substituents described above, those other than the bioorthogonal functional group. Examples of such a group other than the bioorthogonal functional group include alkyl, cycloalkyl, aralkyl, a monovalent heterocyclic group, hydroxy, amino, alkyloxy (alkoxy), cycloalkyloxy, and aralkyloxy. The definitions, examples, and preferred examples of these groups and the components of these groups (e.g., alkyl in alkyloxy (alkoxy), cycloalkyl in cycloalkyloxy, and aralkyl in aralkyloxy) are similar to those described above.

In a specific embodiment, Lb may be represented by the following (Lb'): 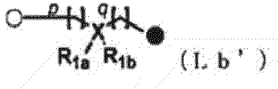
wherein p is any integer of 0 to 10,
q is any integer of 0 to 10,
X is a carbon atom, a nitrogen atom, or a single bond (when X is a nitrogen atom, R_{1b} is absent, and when X is a single bond, R₁ₐ and R_{1b} are absent) ,
R₁ₐ and R_{1b} are the same as or different from each other, and are each a hydrogen atom or selected from the group consisting of the above-described substituents, and
a symbol of "white circle" indicates a bond to C1, and a symbol of "black circle" indicates a bond to B.

The definitions, examples, and preferred examples of p, q, and X and R₁ₐ and R_{1b} (substituents) are as described above.

### 4-3. Partial Structure "L1-B"

### 4-3-1. Length of Partial Structure "L1-B" Linking L1 Terminal Portion and R'

In Formula (IV), the length of a partial structure linking an L1 terminal portion (or a C1 terminal portion) formed by cleavage and R' (a linear chain portion in "L1-B") (or the number of atoms of a main chain linking a bioorthogonal functional group and a side chain of a specific amino acid residue in an antibody regioselectively having a bioorthogonal functional group or bioorthogonal functional groups or a salt thereof not limited to a specific formula such as Formula (IV); hereinafter the same) can be designed as appropriate in accordance with the various factors described in "1-6-1. Length of Main Chain in Partial Structure "L-B" Linking A and R."

The length of the partial structure linking an L1 terminal portion (or a C1 terminal portion) formed by cleavage and R' may be e.g., about 2.5 angstroms or longer, preferably about 4 angstroms or longer, and more preferably about 5 angstroms or longer. The length of the partial structure may be e.g., about 15 angstroms or shorter, preferably about 12 angstroms or shorter, and more preferably about 8 angstroms or shorter. More specifically, the length of the partial structure may be e.g., about 2.5 to 15 angstroms, preferably about 4 to 12 angstroms, and more preferably about 5 to 8 angstroms.

More specifically, the length of the partial structure linking an L1 terminal portion (or a C1 terminal portion) formed by cleavage and R' can also be defined as the number of atoms forming a linking chain of the partial structure (except hydrogen atoms and substituents). The number of atoms forming the linking chain may be e.g., two (about 2.5 angstroms) or more, preferably three (about 4 angstroms) or more, and more preferably four (about 5 angstroms) or more. The number of atoms forming the linking chain may be e.g., ten (about 15 angstroms) or less, preferably eight (about 12 angstroms) or less, and more preferably six (about 8 angstroms) or less. More specifically, the number of atoms forming the linking chain may be e.g., two to ten, preferably three to eight, and more preferably four to six.

When the linking chain of the partial structure linking an L1 terminal portion (or a C1 terminal portion) formed by cleavage and R' is a chain structure comprising no divalent cyclic structure, the number of atoms of the linking chain can be determined by counting the number of atoms in the linking chain.

On the other hand, when the linking chain of the partial structure linking an L1 terminal portion (or a C1 terminal portion) formed by cleavage and R' is a structure comprising a divalent cyclic structure, the number of atoms of the linking chain and the length described above do not necessarily correspond to each other, and the length that can be defined by the number of atoms of the linking chain tends to be shorter than the length described above. Even in such a case, in view of defining the length of the linking chain by the number of atoms, the number of atoms of the linking chain can be counted for convenience's sake. Specifically, the number of atoms of the linking chain in such a case may be determined by counting the number of atoms of the shortest route connecting two bonds in the divalent cyclic structure in addition to the number of atoms in a chain structure comprising no divalent cyclic structure in the linking chain as described above.

The linking chain of the partial structure linking an L1 terminal portion (or a C1 terminal portion) formed by cleavage and R' may be preferably a chain structure comprising no divalent cyclic structure. In this case, L1-B can be designed as appropriate such that the linking chain of the partial structure linking a terminal portion and R' formed by cleavage comprises no divalent cyclic group.

In a specific embodiment, the partial structure represented by L1-B preferably comprises no peptide portion. In this case, the antibody having a functional substance or functional substances according to the present invention (e.g., an antibody drug conjugate) obtained using the antibody having a bioorthogonal functional group or bioorthogonal functional groups according to the present invention has an advantage that it cannot comprise any peptide portion, which can have immunogenicity, as a linker.

### 4-3-2. Specific Structure of Partial Structure "L1-B"

In Formula (IV), L1 and B are structures that can be correlated with each other. Consequently, in Formula (IV), L1 and B can be defined as a partial structure represented by "L1-B."

In a specific embodiment, when L1 is (i') the monovalent group comprising a bioorthogonal functional group, B is (a) the divalent group comprising a bioorthogonal functional group or (b) the divalent group comprising no bioorthogonal functional group.

When L1 is (i') the monovalent group comprising a bioorthogonal functional group, B is preferably (a) the divalent group comprising a bioorthogonal functional group. In this case, the bioorthogonal functional group in (i') may be homogeneous or heterogeneous with respect to the bioorthogonal functional group in (a). In view of employing a simpler structure and/or improving reactivity to a single functional substance and the like, the bioorthogonal functional group in (i') may be homogeneous with respect to the bioorthogonal functional group in (a). On the other hand, in view of ensuring reactivity differentiated for two or more functional substances and non-use of a partial bioorthogonal functional group in the reaction, the bioorthogonal functional group in (i') may be heterogeneous with respect to the bioorthogonal functional group in (a).

Alternatively, when L1 is (i') the monovalent group comprising a bioorthogonal functional group, B may be (b) the divalent group comprising no bioorthogonal functional group. In this case, the antibody having a bioorthogonal functional group or bioorthogonal functional groups represented by Formula (IV) or a salt thereof has a simpler structure and is thus easily synthesized.

In another specific embodiment, when L1 is (ii') the monovalent group comprising no bioorthogonal functional group, B is (a) the divalent group comprising a bioorthogonal functional group.

In a specific embodiment, a structural unit represented by L1-B may be represented by the following Formula (L1B'): 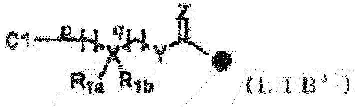 wherein
the definitions, examples, and preferred examples of C1, p, q, X, R₁ₐ, R_{1b}, Y, and Z are the same as those described above, and
a symbol of "black circle" indicates a bond to R'.

Consequently, Formula (IV) can be defined as the following Formula (IV'): 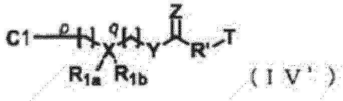 wherein
the definitions, examples, and preferred examples of C1, p, q, X, R₁ₐ, R_{1b'} Y, Z, R', and T are the same as those described above.

In Formulae (L1B') and (IV'), the length from C in C=Z to C1 is similar to the length of the partial structure linking an L1 terminal portion (or a C1 terminal portion) formed by cleavage and R'. In these formulae, the length from C in C=Z to C1 can also be defined as the number of atoms forming a linking chain of a partial structure from C in C=Z to C1 (except hydrogen atoms and substituents). The number of atoms is similar to the number of atoms constituting the partial structure linking an L1 terminal portion (or a C1 terminal) formed by cleavage and R' (except hydrogen atoms and substituents). The linking chain of the partial structure linking C in C=Z and C1 (except hydrogen atoms and substituents) may comprise no cyclic structure or comprise a cyclic structure and preferably comprises no cyclic structure. The linking chain (except hydrogen atoms and substituents) may preferably comprise no peptide portion.

### 4-4. Binding Site of Partial Structure Other Than Antibody, Possessed by Antibody (Regioselectivity)

A partial structure other than the antibody (e.g., L1-B-R') can regioselectively bind to the target region described above in the antibody (T). Specifically, when T comprises one or more specific amino acid residues in a target region consisting of 1 to 50 consecutive amino acid residues, and five or more of the specific amino acid residues in a non-target region other than the target region, the partial structure other than the antibody can be bound to the one or more specific amino acid residues contained in the target region with 30% or more regioselectivity. The definitions, examples, and preferred examples of the target region and regioselectivity are as described above.

### 4-5. Number of Partial Structures Other Than Antibody, Possessed by Antibody

The number of partial structures (e.g., L1-B-R') other than the antibody, possessed by the antibody (T) can vary. When T is a multimeric protein comprising a plurality of monomeric proteins, for example, T can have the partial structure other than T in a plurality of corresponding target regions in the monomeric proteins. Consequently, T can have a plurality of partial structures other than T. Consequently, the structure represented by Formula (IVI), (IV'), or IV'') indicates that T may have one or a plurality of partial structures other than T. The number of partial structures other than T possessed by T can be adjusted by setting the type of the antibody and conditions such as a reaction ratio between the antibody and a structural unit to be introduced thereto as appropriate. Such a number, which varies depending on the type of the antibody, may be e.g., one to eight, preferably one to four, and more preferably one or two.

In a specific embodiment, when the antibody is a multimeric protein comprising a plurality of monomeric proteins, the antibody may possess a plurality of partial structures other than the antibody. The present invention can introduce a plurality of partial structures other than the antibody to the same target region of the monomeric proteins.

In a preferred embodiment, the antibody may be an antibody comprising a plurality of heavy chains. The definition, examples, and preferred examples of the antibody are as described above. In the present invention, the number of partial structures other than the antibody, possessed by the antibody may be one or two (preferably two) for IgG, IgE, and IgD, one to four (preferably four) for IgA, and one to eight (preferably eight) for IgM.

### 4-6. Method of Production

The present invention provides a method for producing an antibody having a bioorthogonal functional group or bioorthogonal functional groups, or a salt thereof, the method comprising the following:
(C1) cleaving a cleavable portion of the antibody having an affinity substance to the antibody and a cleavable portion, or a salt thereof to form an antibody having a bioorthogonal functional group or bioorthogonal functional groups, or a salt thereof.

The antibody comprising an affinity substance to the antibody and a cleavable portion is represented by Formula (II), preferably Formula (II'), and more preferably Formula (II''). The antibody having a bioorthogonal functional group or bioorthogonal functional groups is represented by Formula (IV), preferably Formula (IV'), and more preferably Formula (IV'').

The antibody comprising an affinity substance to the antibody and a cleavable portion, or a salt thereof has the cleavable portion cleavable by the cleaving treatment described above and is thus cleavable. Such a cleaving reaction can be conducted as appropriate under a condition incapable of causing denaturation or decomposition (e.g., cleavage of an amide bond) of proteins (a mild condition) described in "2-6. Method of Production."

Examples of the cleaving treatment include (a) treatment with one or more substances selected from the group consisting of an acidic substance, a basic substance, a reducing agent, an oxidizing agent, and an enzyme, (b) treatment by physicochemical stimulus selected from the group consisting of light, and (c) being left when a cleavable linker comprising a self-decomposing cleavable portion is used. Conditions of such cleaving treatment are common technical knowledge in the field concerned (e.g., G. Leriche, L. Chisholm, A. Wagner, Bioorganic & Medicinal Chemistry, 20,571 (2012); Feng P. et al., Journal of American Chemical Society, 132,1500 (2010); Bessodes M. et al., Journal of Controlled Release, 99,423 (2004); DeSimone, J. M., Journal of American Chemical Society, 132,17928 (2010); Thompson, D. H., Journal of Controlled Release, 91,187 (2003); Schoenmarks, R. G., Journal of Controlled Release, 95,291 (2004)).

Determination of the formation of the antibody having a bioorthogonal functional group or bioorthogonal functional groups, or a salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity, determination of the number of partial structures other than the antibody, and purification can be performed as appropriate by the methods described in "2-6. Method of Production."

The present invention also provides a method for producing an antibody having a bioorthogonal functional group or bioorthogonal functional groups, or a salt thereof, the method comprising the following:
(C2) reacting a compound having an affinity substance to an antibody, a cleavable portion, and a reactive group, or a salt thereof with an antibody to form an antibody having an affinity substance to the antibody and a cleavable portion, or a salt thereof; and
(C3) cleaving a cleavable portion of the antibody having an affinity substance to the antibody and a cleavable portion, or a salt thereof to form an antibody having a bioorthogonal functional group or bioorthogonal functional groups, or a salt thereof.

The process of (C2) can be performed in a manner similar to the process of (A1) described in "2-6. Method of Production." The process of (C3) can be performed in a manner similar to the process of (C1). The processes of (C2) and (C3) can be separately performed. Alternatively, the processes of (C2) and (C3) can be simultaneously performed in accordance with factors such as a combination of the reactive group and the bioorthogonal functional group that can be formed by cleavage (non-reactivity). That is, when the processes of (C2) and (C3) are performed at the same time, a product-containing reaction solution obtained in the process of (C2) may be subjected to the process of (C3) without isolating the product obtained in the process of (C2) (for example, refer to Examples (81-9) and (84-8) in which a basic substance (hydroxylamine, which is a nucleophile) is added to the product-containing reaction solution obtained in the process of (C2) in order to cleave the cleavable portion in the cleavable linker present in the product].

### 5. Method for producing antibody having functional substance or salt thereof

### 5-1. Outline

The present invention provides a method for producing an antibody having a functional substance or functional substances, represented by Formula (V), or a salt thereof.

F-(L1-B)'-R'-T (V)

wherein
L1 is (i') a monovalent group comprising a bioorthogonal functional group or (ii') a monovalent group comprising no bioorthogonal functional group,
B is (a) a divalent group comprising a bioorthogonal functional group or (b) a divalent group comprising no bioorthogonal functional group,
a structural unit represented by (L1-B') is a divalent structural unit comprising a portion formed by a reaction between a functional substance and either one or both of the bioorthogonal functional groups in (i') and (a),
F is a functional substance,
R' is a portion formed by a reaction between an antibody and a reactive group, and
T is an antibody.

### 5-2. Partial Structure "F-(L1-B)'"

### 5-2-1. Length of Partial Structure "L1-B" Linking F and R'

In Formula (V), the length of a partial structure "L1-B" linking F and R' (a linear chain portion in "L1-B") (or the number of atoms of a main chain linking a functional substance and a side chain of a specific amino acid residue in an antibody regioselectively having a functional substance or functional substances or a salt thereof not limited to a specific formula such as Formula (V) or, when the a functional substance binds to the antibody through a bioorthogonal functional group, the number of atoms of a main chain linking the bioorthogonal functional group and a side chain of a specific amino acid residue; hereinafter the same) can be designed as appropriate in accordance with the various factors described in "1-6-1. Length of Main Chain in Partial Structure "L-B" Linking A and R." Length of Main Chain in Partial Structure "L-B" Linking A and R."

The length of the partial structure linking F and R' may be e.g., about 2.5 angstroms or longer, preferably about 4 angstroms or longer, and more preferably about 5 angstroms or longer. The length of the partial structure may be e.g., about 15 angstroms or shorter, preferably about 12 angstroms or shorter, and more preferably about 8 angstroms or shorter. More specifically, the length of the partial structure may be e.g., about 2.5 to 15 angstroms, preferably about 4 to 12 angstroms, and more preferably about 5 to 8 angstroms.

More specifically, the length of the partial structure linking F and R' can also be defined as the number of atoms forming a linking chain of the partial structure (except hydrogen atoms and substituents). The number of atoms forming the linking chain may be e.g., two (about 2.5 angstroms) or more, preferably three (about 4 angstroms) or more, and more preferably four (about 5 angstroms) or more. The number of atoms forming the linking chain may be e.g., ten (about 15 angstroms) or less, preferably eight (about 12 angstroms) or less, and more preferably six (about 8 angstroms) or less. More specifically, the number of atoms forming the linking chain may be e.g., two to ten, preferably three to eight, and more preferably four to six.

When the linking chain of the partial structure linking F and R' is a chain structure comprising no divalent cyclic structure, the number of atoms of the linking chain can be determined by counting the number of atoms in the linking chain.

On the other hand, when the linking chain of the partial structure linking F and R' is a structure comprising a divalent cyclic structure, the number of atoms of the linking chain and the length described above do not necessarily correspond to each other, and the length that can be defined by the number of atoms of the linking chain tends to be shorter than the length described above. Even in such a case, in view of defining the length of the linking chain by the number of atoms, the number of atoms of the linking chain can be counted for convenience's sake. Specifically, the number of atoms of the linking chain in such a case may be determined by counting the number of atoms of the shortest route connecting two bonds in the divalent cyclic structure in addition to the number of atoms in a chain structure comprising no divalent cyclic structure in the linking chain as described above.

The linking chain of the partial structure linking F and R' may be preferably a chain structure comprising no divalent cyclic structure. L1-B can be designed as appropriate such that the linking chain of the partial structure linking F and R' comprises no divalent cyclic group.

In a specific embodiment, the partial structure represented by L1-B preferably comprises no peptide portion. In this case, the antibody having a functional substance or functional substances according to the present invention (e.g., an antibody drug conjugate) has an advantage that it does not comprise any peptide portion, which can have immunogenicity, as a linker.

### 5-2-2. Specific Structure of Partial Structure "F-(L1-B)'"

In Formula (V), L1 and B in F-(L1-B)' are structures that can be correlated with each other. Consequently, in Formula (V), L1 and B can be defined as a partial structure represented by "L1-B."

A structural unit represented by F-(L1-B)' is a portion formed by a reaction between a functional substance and either one or both of the bioorthogonal functional groups in (i') and (a). A bioorthogonal functional group used for the reaction is not present in Formula (V). Consequently, in Formula (V), either one or both of the bioorthogonal functional groups in (i') and (a) are not present. The portion formed by a reaction between a functional substance and either one or both of the bioorthogonal functional groups in (i') and (a) is the same as the portion formed by a reaction between a functional substance and a bioorthogonal functional group. Consequently, in the present specification, examples (the names of residues) and specific examples (chemical structures) of the portion formed by a reaction between a functional substance and either one or both of the bioorthogonal functional groups in (i') and (a) are the same as those described above for the portion formed by a reaction between a functional substance and a bioorthogonal functional group.

In an embodiment, when L1 is (i') the monovalent group comprising a bioorthogonal functional group, B is (a) the divalent group comprising a bioorthogonal functional group or (b) the divalent group comprising no bioorthogonal functional group.

In a preferred embodiment, when L1 is (i') the monovalent group comprising a bioorthogonal functional group, B is (a) the divalent group comprising a bioorthogonal functional group. In this case, the bioorthogonal functional group in (i') may be homogeneous or heterogeneous with respect to the bioorthogonal functional group in (a). In view of employing a simpler structure and/or improving reactivity to a single functional substance and the like, the bioorthogonal functional group in (i') may be homogeneous with respect to the bioorthogonal functional group in (a). On the other hand, in view of ensuring reactivity differentiated for two or more functional substances, non-use of a partial bioorthogonal functional group in the reaction, and the like, the bioorthogonal functional group in (i') may be heterogeneous with respect to the bioorthogonal functional group in (a).

In another preferred embodiment, when L1 is (i') the monovalent group comprising a bioorthogonal functional group, B may be (b) the divalent group comprising no bioorthogonal functional group. In this case, the antibody having a functional substance or functional substances, represented by Formula (V), or a salt thereof has a simpler structure and is thus easily synthesized.

In another embodiment, when L1 is (ii') the monovalent group comprising no bioorthogonal functional group, B is (a) the divalent group comprising a bioorthogonal functional group.

In a specific embodiment, when the bioorthogonal functional groups in (i') and (a) are heterogeneous, the antibody having a functional substance or functional substances, represented by Formula (V), or a salt thereof may be represented by Formula (V1) or (V2).

L1-B'(-F)-R'-T (V1)

wherein
L1 is (i') a monovalent group comprising a bioorthogonal functional group or (ii') a monovalent group comprising no bioorthogonal functional group,
B' is a trivalent group comprising a portion formed by a reaction between a functional substance and a bioorthogonal functional group,
F is a functional substance,
R' is a portion formed by a reaction between an antibody and a reactive group, and
T is an antibody.

F-L1'-B-R'-T (V2)

wherein
L1' is a divalent group comprising a portion formed by a reaction between a functional substance and (i') a monovalent group comprising a bioorthogonal functional group,
B is (a) a divalent group comprising a bioorthogonal functional group or (b) a divalent group comprising no bioorthogonal functional group,
F is a functional substance,
R' is a portion formed by a reaction between an antibody and a reactive group, and
T is an antibody.

L1' is a divalent group comprising a portion formed by a reaction between a functional substance and (i') a monovalent group comprising a bioorthogonal functional group. The portion formed by a reaction between a functional substance and (i') a monovalent group comprising a bioorthogonal functional group is the same as the portion formed by a reaction between a functional substance and a bioorthogonal functional group. Consequently, in the present specification, examples (the names of residues) and specific examples (chemical structures) of the portion formed by a reaction between a functional substance and (i') a monovalent group comprising a bioorthogonal functional group are the same as those described above for the portion formed by a reaction between a functional substance and a bioorthogonal functional group. Consequently, the functional substance reacts with the bioorthogonal functional group, and thus the divalent group comprising a portion formed by a reaction between a functional substance and (i') a monovalent group comprising a bioorthogonal functional group (L1') can also be represented as a divalent group comprising a portion formed by a reaction between a functional substance and a bioorthogonal functional group (hereinafter the same). More specifically, the divalent group comprising a portion formed by a reaction between a functional substance and a bioorthogonal functional group (1) may be a divalent group comprising a thiosuccinimide residue, a triazole residue, or a hydrazone residue, referred to in the above-described preferred examples, or (2) which is not limited to a particular group, may be a divalent group comprising a residue selected from the group consisting of a disulfide residue (this residue has been referred to in the above-described preferred examples), an acetal residue, a ketal residue, an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue, for example, but is preferably (1) a divalent group comprising a thiosuccinimide residue, a triazole residue, or a hydrazone residue

In another specific embodiment, when the bioorthogonal functional groups in (i') and (a) are homogeneous or heterogeneous, the antibody having a functional substance or functional substances, represented by Formula (V), or a salt thereof may be represented by Formula (V3).

Fa-L1'-B'(-Fb)-R'-T (V3)

wherein
L1' is a divalent group comprising a portion formed by a reaction between a functional substance and (i') a monovalent group comprising a bioorthogonal functional group,
B' is a trivalent group comprising a portion formed by a reaction between a functional substance and a bioorthogonal functional group,
Fa and Fb are functional substances which are the same as or different from each other,
R' is a portion formed by a reaction between an antibody and a reactive group, and
T is an antibody.

In a specific embodiment, in the above Formula (V1), a structural unit represented by L1-B'(-F) may be represented by the following Formula (L1B'(F)). 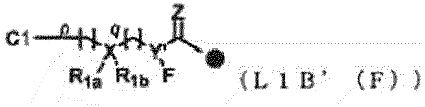 wherein
the definitions, examples, and preferred examples of F, C1, p, q, X, R₁ₐ, R_{1b}, and Z are the same as those described above,
Y' is a residue obtained by removing one hydrogen atom from Y of the above Formula (B-1), and
a symbol of "black circle" indicates a bond to R'.

The definition, examples, and preferred examples of the residue obtained by removing one hydrogen atom from Y of the above Formula (B-1) are similar to those describes above.

Consequently, Formula (V1) can be defined as the following Formula (V1'): 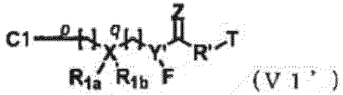 wherein
the definitions, examples, and preferred examples of F, C1, p, q, X, R₁ₐ, R_{1b}, Z, R', and T are the same as those described above, and
Y' is a residue obtained by removing one hydrogen atom from Y of the above Formula (B-1).

In a specific embodiment, in Formula (V2), a structural unit represented by F-L1'-B may be represented by the following Formula (FL1'B): 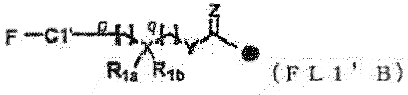 wherein
the definitions, examples, and preferred examples of F, p, q, X, R₁ₐ, R_{1b}, Y, and Z are the same as those described above,
C1' is a portion formed by a reaction between a functional substance and a bioorthogonal functional group, and
a symbol of "black circle" indicates a bond to R'.

C1' is a portion formed by a reaction between a functional substance and a bioorthogonal functional group. The definition, examples, and preferred examples of the portion formed by a reaction between a functional substance and a bioorthogonal functional group in C1' are similar to those described in the above "Divalent Group Comprising Portion Formed by Reaction between Functional substance and Bioorthogonal functional group" (hereinafter the same).

Consequently, Formula (V2) can be defined as the following Formula (V2'): 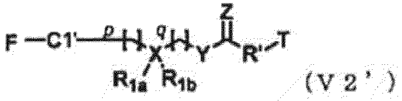 wherein
the definitions, examples, and preferred examples of F, p, q, X, R₁ₐ, R_{1b}, Y, Z, R', and T are the same as those described above, and
C1' is a portion formed by a reaction between a functional substance and a bioorthogonal functional group.

In a specific embodiment, in Formula (V3), a structural unit represented by Fa-L1'-B'(-Fb) may be represented by the following Formula (FaL1'B'(Fb)): 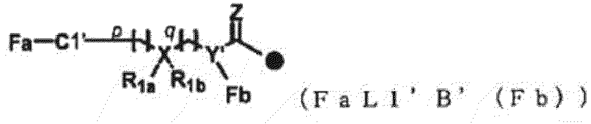 wherein
the definitions, examples, and preferred examples of Fa, Fb, p, q, X, R₁ₐ, R_{1b}, and Z are the same as those described above,
C1' is a portion formed by a reaction between a functional substance and a bioorthogonal functional group,
Y' is a residue obtained by removing one hydrogen atom from Y of the above Formula (B-1), and
a symbol of "black circle" indicates a bond to R'.

Consequently, Formula (V3) can be defined as the following Formula (V3'): 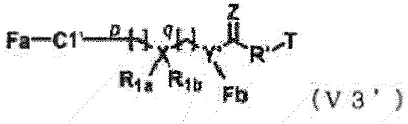 wherein
the definitions, examples, and preferred examples of Fa, Fb, p, q, X, R₁ₐ, R_{1b}, Z, R', and T are the same as those described above,
C1' is a portion formed by a reaction between a functional substance and a bioorthogonal functional group, and
Y' is a residue obtained by removing one hydrogen atom from Y of the above Formula (B-1).

In Formulae (L1B'(F), (FL1'B), and (FaL1'B'(F2)) and (V1'), (V2'), and (V3'), the length from C in C=Z to C1 is similar to the length of the partial structure linking an L1 terminal portion (or a C1 terminal portion) formed by cleavage and R'. In these formulae, the length from C in C=Z to C1 can also be defined as the number of atoms forming a linking chain of a partial structure from C in C=Z to C1 (except hydrogen atoms and substituents). The number of atoms is similar to the number of atoms constituting the partial structure linking an L1 terminal portion (or a C1 terminal) formed by cleavage and R' (except hydrogen atoms and substituents). The linking chain of the partial structure linking C in C=Z and C1 (except hydrogen atoms and substituents) may comprise no cyclic structure or comprise a cyclic structure and preferably comprises no cyclic structure. The linking chain (except hydrogen atoms and substituents) may preferably comprise no peptide portion.

### 5-3. Binding Site of Partial Structure Other Than Antibody, Possessed by Antibody (Regioselectivity)

A partial structure other than the antibody (e.g., F-(L1-B)'-R') can regioselectively bind to the target region described above in the antibody (T). Specifically, when T comprises one or more specific amino acid residues in a target region consisting of 1 to 50 consecutive amino acid residues, and five or more of the specific amino acid residues in a non-target region other than the target region, the partial structure other than the antibody can be bound to the one or more specific amino acid residues contained in the target region with 30% or more regioselectivity. The definitions, examples, and preferred examples of the target region and regioselectivity are as described above.

### 5-4. Number of Partial Structures Other Than Antibody, Possessed by Antibody

The number of partial structures (e.g., F-(L1-B)'-R') other than the antibody, possessed by the antibody (T) can vary. When T is a multimeric protein comprising a plurality of monomeric proteins, for example, T can have the partial structure other than T in a plurality of corresponding target regions in the monomeric proteins. Consequently, T can have a plurality of partial structures other than T. Consequently, the structure represented by Formula (V), (V1), (V2), (V3), (V1'), (V2'), or (V3') indicates that T may have one or a plurality of partial structures other than T. The number of partial structures other than T possessed by T can be adjusted by setting the type of the antibody and conditions such as a reaction ratio between the antibody and a structural unit to be introduced thereto as appropriate. Such a number, which varies depending on the type of the antibody, may be e.g., one to eight, preferably one to four, and more preferably one or two.

In a specific embodiment, when the antibody is a multimeric protein comprising a plurality of monomeric proteins, the antibody may possess a plurality of partial structures other than the antibody. The present invention can introduce a plurality of partial structures other than the antibody to the same target region of the monomeric proteins.

In a preferred embodiment, the antibody may be an antibody comprising a plurality of heavy chains. The definition, examples, and preferred examples of the antibody are as described above. In the present invention, the number of partial structures other than the antibody, possessed by the antibody may be one or two (preferably two) for IgG, IgE, and IgD, one to four (preferably four) for IgA, and one to eight (preferably eight) for IgM.

### 5-5. Method of Production

The present invention provides a method for producing an antibody having a functional substance or functional substances, or a salt thereof. The present method of production can be classified into (D) a method that uses a conjugate having an affinity substance to an antibody, a cleavable portion, a functional substance, and an antibody, or a salt thereof as a raw material (refer to Reaction (3) in FIG. 2) and (E) a method that uses an antibody having a bioorthogonal functional group or bioorthogonal functional groups, or a salt thereof as a raw material (refer to Reaction (5) in FIG. 2).

(D) The method that uses a conjugate having an affinity substance to an antibody, a cleavable portion, a functional substance, and an antibody, or a salt thereof as a raw material comprises the following:
(D1) cleaving a cleavable portion of a conjugate having an affinity substance to an antibody, a cleavable portion, a functional substance, and an antibody, or a salt thereof to form an antibody having a functional substance or functional substances, or a salt thereof.

The conjugate having an affinity substance to an antibody, a cleavable portion, a functional substance, and an antibody is represented by Formula (III), preferably Formula (III'), and more preferably Formula (III''). The antibody having a functional substance or functional substances is represented by Formula (V), preferably Formula (V1), (V2), or (V3), and more preferably Formula (V1'), (V2'), or (V3').

The conjugate having an affinity substance to an antibody, a cleavable portion, a functional substance, and an antibody, or a salt thereof has the cleavable portion cleavable by the cleaving treatment described above and is thus cleavable. Such a cleaving reaction can be conducted in a manner described in "4-6. Method of Production."

Determination of the formation of the antibody having a functional substance or functional substances, or a salt thereof, which depends on its specific raw materials and the molecular weight of a product, can be performed by electrophoresis, chromatography (e.g., gel permutation chromatography, ion-exchange chromatography, reversed phase column chromatography, and HPLC), or mass spectrometry, for example, and preferably mass spectrometry. Determination of regioselectivity, determination of the number of partial structures other than the antibody, and purification can be performed as appropriate by the methods described in "2-6. Method of Production."

The present invention also provides a method for producing an antibody having a functional substance or functional substances, or a salt thereof, the method comprising the following:
(D2) reacting an antibody comprising an affinity substance to the antibody and a cleavable portion, or a salt thereof with a functional substance to form a conjugate having an affinity substance to an antibody, a cleavable portion, a functional substance, and an antibody, or a salt thereof; and
(D3) cleaving a cleavable portion of a conjugate having an affinity substance to an antibody, a cleavable portion, a functional substance, and an antibody, or a salt thereof to form an antibody having a functional substance or functional substances, or a salt thereof.

The process of (D2) can be performed in a manner similar to the process of (B1) described in "3-7. Method of Production." The process of (D3) can be performed in a manner similar to the process of (D1). The processes of (D2) and (D3) can be separately performed. Alternatively, the processes of (D2) and (D3) can be simultaneously performed in accordance with factors such as a combination of the cleavable portion, the functional substance, and the bioorthogonal functional group. That is, when the processes of (D2) and (D3) are performed at the same time, a product-containing reaction solution obtained in the process of (D2) may be subjected to the process of (D3) without isolating the product obtained in the process of (D2) .

The present invention further provides a method for producing an antibody having a functional substance or functional substances, or a salt thereof, the method comprising the following:
(D4) reacting a compound comprising an affinity substance to an antibody, a cleavable portion, and a reactive group, or a salt thereof with an antibody to form an antibody comprising an affinity substance to the antibody and a cleavable portion, or a salt thereof;
(D5) reacting the antibody comprising an affinity substance to the antibody and a cleavable portion, or a salt thereof with a functional substance to form a conjugate having an affinity substance to an antibody, a cleavable portion, a functional substance, and an antibody, or a salt thereof; and
(D6) cleaving a cleavable portion of the conjugate having an affinity substance to an antibody, a cleavable portion, a functional substance, and an antibody, or a salt thereof to form an antibody having a functional substance or functional substances, or a salt thereof.

The process of (D4) can be performed in a manner similar to the process of (A1) described in "2-6. Method of Production." The processes of (D5) and (D6) can be performed in a manner similar to the processes of (D2) and (D3). The process of (D5) can be performed in a manner similar to the process of (B1) described in "3-7. Method of Production." The process of (D6) can be performed in a manner similar to the process of (D1). The processes of (D4) to (D6) can be separately performed. Alternatively, at least part of the processes of (D4) to (D6) can be simultaneously performed in accordance with factors such as a combination of the reactive group, the cleavable portion, the functional substance, and the bioorthogonal functional group. That is, when two or more processes out of (D4) to (D6) are performed at the same time, a product-containing reaction solution obtained in the former process may be subjected to the subsequent process without isolating the product obtained in the former process.

(E) The method that uses an antibody having a bioorthogonal functional group or bioorthogonal functional groups, or a salt thereof as a raw material comprises the following:
(E1) reacting an antibody having a bioorthogonal functional group or bioorthogonal functional groups, or a salt thereof with a functional substance to form an antibody having a functional substance or functional substances, or a salt thereof.

The antibody having a bioorthogonal functional group or bioorthogonal functional groups is represented by Formula (IV) and preferably Formula (IV'). The antibody having a functional substance or functional substances is represented by Formula (V), preferably Formula (V1), (V2), or (V3), and more preferably Formula (V1'), (V2'), or (V3').

The antibody having a bioorthogonal functional group or bioorthogonal functional groups, or a salt thereof has the bioorthogonal functional group and can thus react with a functional substance. Such a reaction can be conducted in a manner described in "3-7. Method of Production."

Determination of the formation of the antibody having a functional substance or functional substances, or a salt thereof can be performed by the method described above. Determination of regioselectivity, determination of the number of partial structures other than the antibody, and purification can be performed as appropriate by the methods described in "2-6. Method of Production."

The present invention also provides a method for producing an antibody having a functional substance or functional substances, or a salt thereof, the method comprising the following:
(E2) cleaving a cleavable portion of an antibody having an affinity substance to the antibody and a cleavable portion, or a salt thereof to form an antibody having a bioorthogonal functional group or bioorthogonal functional groups, or a salt thereof; and
(E3) reacting the antibody having a bioorthogonal functional group or bioorthogonal functional groups, or a salt thereof with a functional substance to form an antibody having a functional substance or functional substances, or a salt thereof.

The process of (E2) can be performed in a manner similar to the process of (C1) described in "4-6. Method of Production". The process of (E3) can be performed in a manner similar to the process of (E1). The processes of (E2) and (E3) can be separately performed. Alternatively, the processes of (E2) and (E3) can be simultaneously performed in accordance with factors such as a combination of the cleavable portion, the functional substance, and the bioorthogonal functional group. That is, when the processes of (E2) and (E3) are performed at the same time, a product-containing reaction solution obtained in the process of (E2) may be subjected to the process of (E3) without isolating the product obtained in the process of (E2) .

The present invention further provides a method for producing an antibody having a functional substance or functional substances, or a salt thereof, the method comprising the following:
(E4) reacting a compound comprising an affinity substance to an antibody, a cleavable portion, and a reactive group, or a salt thereof with an antibody to form an antibody comprising an affinity substance to the antibody and a cleavable portion, or a salt thereof;
(E5) cleaving a cleavable portion of the antibody comprising an affinity substance to the antibody and a cleavable portion, or a salt thereof to form an antibody having a bioorthogonal functional group or bioorthogonal functional groups, or a salt thereof; and
(E6) reacting the antibody comprising a bioorthogonal functional group, or a salt thereof with a functional substance to form an antibody having a functional substance or functional substances, or a salt thereof.

The process of (E4) can be performed in a manner similar to the process of (A1) described in "2-6. Method of Production." The processes of (E5) and (E6) can be performed in a manner similar to the processes of (E2) and (E3). The processes of (E4) to (E6) can be separately performed. Alternatively, at least part of the processes of (E4) to (E6) can be simultaneously performed in accordance with factors such as a combination of the reactive group, the cleavable portion, the functional substance, and the bioorthogonal functional group. That is, when two or more processes out of (E4) to (E6) are performed at the same time, a product-containing reaction solution obtained in the former process may be subjected to the subsequent process without isolating the product obtained in the former process.

### 6. Salt

In the present invention, examples of the salt include salts with inorganic acids, salts with organic acids, salts with inorganic bases, salts with organic bases, and salts with amino acids. Examples of salts with inorganic acids include salts with hydrogen chloride, hydrogen bromide, phosphoric acid, sulfuric acid, and nitric acid. Examples of salts with organic acids include salts with formic acid, acetic acid, trifluoroacetic acid, lactic acid, tartaric acid, fumaric acid, oxalic acid, maleic acid, citric acid, succinic acid, malic acid, benzenesulfonic acid, and p-toluenesulfonic acid. Examples of salts with inorganic bases include salts with alkali metals (e.g., sodium and potassium), alkaline-earth metals (e.g., calcium and magnesium), other metals such as zinc and aluminum, and ammonium. Examples of salts with organic bases include salts with trimethylamine, triethylamine, propylenediamine, ethylenediamine, pyridine, ethanolamine, monoalkyl ethanolamine, dialkyl ethanolamine, diethanolamine, and triethanolamine. Examples of salts with amino acids include salts with basic amino acids (e.g., arginine, histidine, lysine, and ornithine) and acidic amino acids (e.g., aspartic acid and glutamic acid). The salt is preferably a salt with an inorganic acid (e.g., hydrogen chloride) or a salt with an organic acid (e.g., trifluoroacetic acid).

### 7. Uses

The compound or salt thereof of the present invention having an affinity substance to an antibody, a cleavable portion, and a reactive group is useful for regioselective modification of an antibody, for example. Consequently, the present invention provides a reagent of regioselectively modifying an antibody, comprising a compound having an affinity substance to an antibody, a cleavable portion, and a reactive group, or a salt thereof.

The regioselective modifying reagent of the present invention may be provided in a form of a composition further comprising other components. Examples of such other compounds include solutions and stabilizers (e.g., antioxidants and preservatives). Among solutions, aqueous solutions are preferred. Examples of aqueous solutions include water (e.g., distilled water, sterilized distilled water, purified water, and a physiological saline solution) and buffers (e.g., an aqueous phosphoric acid solution, a Tris-hydrochloric acid buffer, a carbonic acid-bicarbonic acid buffer, an aqueous boric acid solution, a glycine-sodium hydroxide buffer, and a citric acid buffer); buffers are preferred. The pH of solutions is e.g., 5.0 to 9.0 and preferably 5.5 to 8.5. The regioselective modifying reagent of the present invention can be provided in a liquid form or a powder form (e.g., freeze-dried powder).

The antibody or salt thereof of the present invention (regioselectively) having an affinity substance to an antibody and a cleavable portion, the conjugate or salt thereof of the present invention (regioselectively) having an affinity substance to an antibody, a cleavable portion, a functional substance, and an antibody, and the antibody or salt thereof of the present invention (regioselectively) having a bioorthogonal functional group or bioorthogonal functional groups are useful as intermediates for preparing an antibody (regioselectively) having a functional substance or functional substances, or a salt thereof, for example.

The antibody or salt thereof of the present invention regioselectively having a functional substance or functional substances is useful as pharmaceuticals or reagents (e.g., diagnostic reagents and reagents for research), for example.

In particular, the antibody or salt thereof of the present invention (regioselectively) having a functional substance or functional substances is useful as pharmaceuticals. It is reported as described above that when the number of bonds and the bond positions of a drug of an antibody drug conjugate (ADC) are changed, pharmacokinetics, a releasing rate of the drug, and effects change. Given these circumstances, next-generation ADCs are required to control the number and positions of a drug to be conjugated. It is believed that when the number and positions are constant, the problems of expected efficacy, variations in conjugate medicines, and lot difference, or what is called regulation, will be solved. The antibody or salt thereof of the present invention having a functional substance or functional substances can solve such a problem of regulation. Consequently, the antibody or salt thereof of the present invention having a functional substance or functional substances may be provided in the form of a pharmaceutical composition. The pharmaceutical composition may comprise a pharmaceutically allowable carrier in addition to the antibody having a functional substance or functional substances, or a salt thereof. Examples of the pharmaceutically allowable carrier include, but are not limited to, excipients such as sucrose, starch, mannitol, sorbitol, lactose, glucose, cellulose, talc, calcium phosphate, and calcium carbonate; binders such as cellulose, methylcellulose, hydroxypropylcellulose, polypropylpyrrolidone, gelatin, gum arabic, polyethylene glycol, sucrose, and starch; disintegrators such as starch, carboxymethylcellulose, hydroxypropyl starch, sodium hydrogencarbonate, calcium phosphate, and calcium citrate; lubricants such as magnesium stearate, Aerosil, talc, sodium lauryl sulfate; aromatics such as citric acid, menthol, glycyl lysine ammonium salts, glycine, and orange powder; preservatives such as sodium benzoate, sodium hydrogen sulfite, methylparaben, and propylparaben; stabilizers such as citric acid, sodium citrate, and acetic acid, suspensions such as methylcellulose, polyvinylpyrrolidone, and aluminum stearate; dispersants such as surfactants; diluents such as water, a physiological saline solution, and orange juice; and base waxes such as cacao butter, polyethylene glycol, and refined kerosene. The antibody or salt thereof of the present invention having a functional substance or functional substances may also have any modification (e.g., PEGylation) achieving stability.

Examples of preparations suitable for oral administration include liquid medicines dissolving an effective amount of a ligand in a diluted solution such as water, a physiological saline solution, or orange juice; capsules, sachets, and tablets comprising an effective amount of a ligand as a solid or granules; suspension medicines suspending an effective amount of an active ingredient in an appropriate dispersion medium; and emulsions dispersing a solution dissolving an effective amount of an active ingredient in an appropriate dispersion medium to be emulsified.

The pharmaceutical composition is suitable for nonoral administration (e.g., intravenous injection, hypodermic injection, intramuscular injection, local injection, and intraperitoneal administration). Examples of the pharmaceutical composition suitable for such nonoral administration include aqueous or nonaqueous, isotonic, aseptic injection medicines, which may comprise an antioxidant, a buffer, a bacteriostat, a tonicity agent, or the like. Examples thereof also include aqueous or nonaqueous, aseptic suspension medicines, which may comprise a suspension, a solubilizing agent, a thickener, a stabilizer, an antiseptic, or the like.

The dose of the pharmaceutical composition, which varies by the type and activity of an active ingredient, the severity of diseases, an animal type as a subject to be dosed, the drug receptivity, body weight, and age of a subject to be dosed, or the like, can be set as appropriate.

### EXAMPLES

The present invention is explained by the following Examples in more detail, but the present invention is not limited to the following Examples.

### [Example 1: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (1-1) Synthesis of IgG1 Fc-Binding Peptide

The peptide of Ac-RGNCAYHKGQIIWCTYH-NH₂ (SEQ ID NO: 5) was synthesized by Fmoc solid phase synthesis method. For a peptide synthesizing apparatus, Liberty Blue manufactured by CEM was used. For all reagents, those manufactured by Watanabe Chemical Industries, Ltd. were used. Resin was Fmoc-NH-SAL-PEG Resin HL. Arginine (R), cysteine (C), and histidine (H) were subjected to double coupling. Cutting out from Resin was performed under a condition with three-hour stirring in a solution of trifluoroacetic acid:water:triisopropylsilane:ethanediol = 94:2.5:1.0:2.5. After cutting out, Resin was removed by filtration, and trifluoroacetic acid was removed. Diethyl ether was added to the formed crystals to perform ether precipitation, and the formed white crystals were collected by filtration. They were dissolved in a 0.1% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.1% of trifluoroacetic acid, and the fractions were determined by LC-MS. A fraction comprising a product was collected, was concentrated under reduced pressure to remove only acetonitrile, and was freeze-dried. A target product (215 mg, 103 µmol) was obtained.

MS (ESI) m/z:z=3 698.00[M+3H]³⁺, z=4 532.80[M+4H]⁴⁺

### (1-2) Formation of Intra-Molecular Disulfide Bond between Cys at Positions 4 and 14 of Ac-RGNCAYHKGQIIWCTYH-NH₂ (SEQ ID NO: 5)

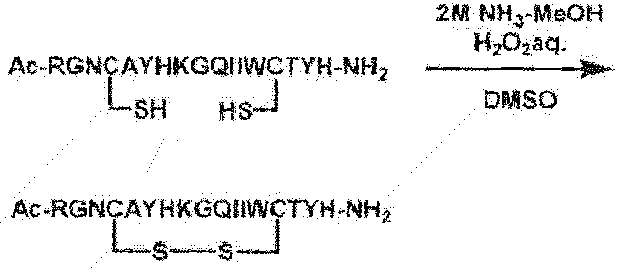

The amino acid sequence is SEQ ID NO: 5.

The peptide (215 mg, 103 µmol) synthesized in (1-1) was dissolved in DMSO to be 100 mM, then 2 equivalents of 2 M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (33.3 mg, 15.9 µmol).

MS (ESI) m/z:z=3 697.45[M+3H]³⁺, z=4 523.35 [M+4H]⁴⁺

### (1-3) Coupling between Disulfide Linker and Peptide

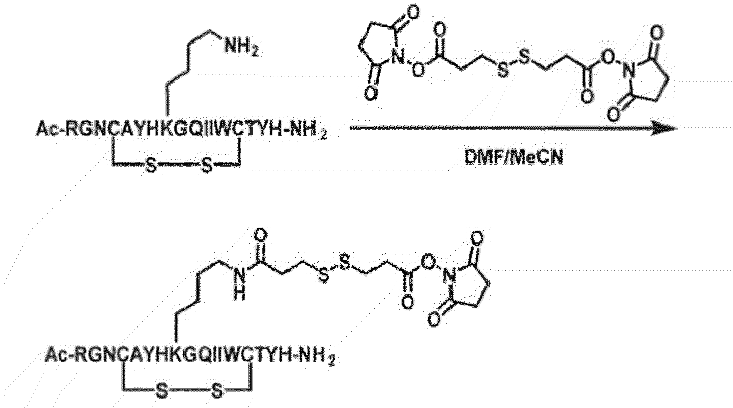

The amino acid sequence is SEQ ID NO: 5.

Ac-RGNCAYHKGQIIWCTYH-NH₂ (SEQ ID NO: 5) synthesized in (1-2) (33.3 mg, 15.9 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (257 mg, 636 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (9.20 mg, 3.87 µmol).

MS(ESI) m/z:z=3 793.75[M+3H]³⁺ HPLC purity: 79%

### (1-4) Specific Modification of Anti-HER2 IgG antibody Trastuzumab and Analysis by ESI-TOFMS

The peptide- and disulfide linker-coupled NHS-activation compound synthesized in (1-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152600.

### (1-5) Determination of Heavy Chain Selectivity of Specifically Modified Compound of Trastuzumab under Reduction Conditions by ESI-TOFMS Analysis

To the antibody-peptide conjugate formed in (1-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (1-6) Hydrophobic Interaction Chromatography (HIC)-UPLC Analysis of Specific Modification of Anti-HER2 IgG Antibody Trastuzumab

The antibody-peptide conjugate formed in (1-5), which had not been subjected to glycolysis, and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: trastuzumab raw material;
b: trastuzumab + 30 equivalents of the peptide- and disulfide linker-coupled NHS-activation compound;

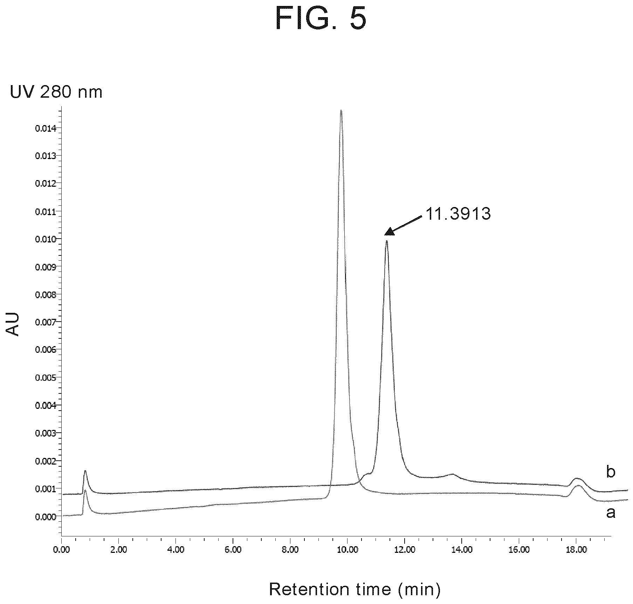

It is believed that a retension time of 11.3913 minutes is attributed to a compound with two peptides introduced to trastuzumab (FIG. 5).

### [Example 2: Peptide Mapping of Thiol-introduced Trastuzumab]

### (2-1) Treatment of Thiol-introduced Trastuzumab with Digestive Enzyme

The antibody-peptide conjugate obtained in (1-3) of Example 1 was glycosylated with PNGaseF (manufactured by New England BioLabs), and then subjected to peptide mapping. To a 1.5 mL low-adsorptive micro test tube, 10 µL of a sample solution, a 50 mM ammonium hydrogencarbonate buffer, and 10 µL of a 20 mM aqueous dithiothreitol solution dissolved in 40% trifluoroethanol were added, and the resulting solution was heated at 65°C for one hour. Thereafter, 10 µL of a 50 mM aqueous iodoacetamide solution was added thereto, and the resulting solution was reacted in a dark place at room temperature while being shaken at 300 rpm for 30 minutes. After the reaction, 40 µL of a 50 mM ammonium hydrogencarbonate buffer was added thereto, the resulting mixture was stirred, 10 µL of a 20 ng/µL aqueous trypsin solution or 12.5 µL of a 200 ng/µL aqueous Glu-C protease solution was added thereto, and the resulting mixture was subjected to enzyme digestion at 37°C for 18 hours. After the digestion, a 20% aqueous trifluoroacetic acid solution was added to the trypsin-digested sample in an amount of 2 µL and to the Glu-C protease digested sample in an amount of 2.12 µL to stop the reaction, and LC-MS/MS measurement was performed.

### (2-2) LC-MS/MS Measurement Conditions for Trastuzumab

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Acquisition
Activation Type:Collision Induced Dissociation(CID)
Data acquisition was performed using Xcalibur 3.0 (Thermo Fisher Scientific) and Thermo Orbitrap Fusion Tune Application 2.0 (Thermo Fisher Scientific) as accompanying software.

### (2-3) Analysis Condition of Modified Site of Trastuzumab

Modified site analysis of an LC-MS/MS measurement result was performed using Proteome Discoverer version 1.4 (Thermo Fisher Scientific).

For analysis with Proteome Discoverer, Sequest HT was used as a search engine, and the range of precursor ion was set to 350 Da to 5,000 Da. Trypsin or Glu-C protease was set as a digestive enzyme, and Maximum Missed Cleavage Sites was set to 3. Mass Tolerance was set to 5 ppm and 0.5 Da for precursor and fragment ion, respectively. For Static Modification, Carbamidomethyl (+57.021 Da) was set as modification of a cysteine residue with iodoacetamide. For Dynamic Modifications, oxidation of methionine (+15.995 Da) and a modified compound to a lysine residue (a thiol-introduced portion subjected to carbamidomethylation with iodoacetamide (+145.019 Da)) were set. Furthermore, a filter was applied so as to cause Peptide Confidence to be only High.

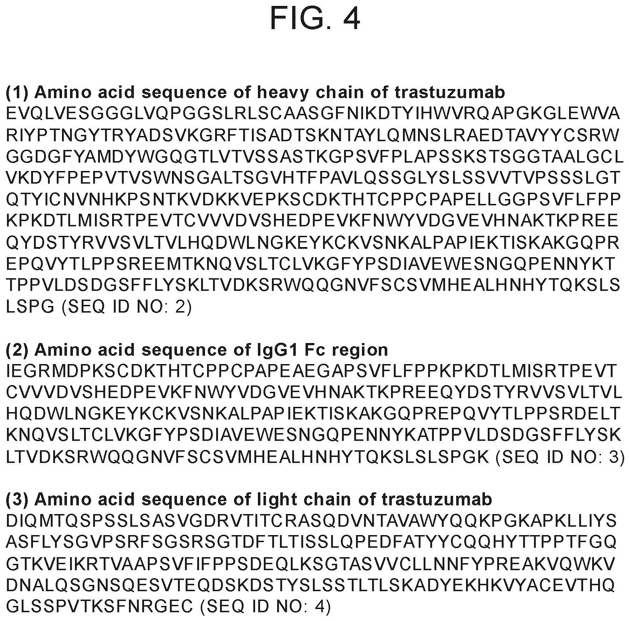

As data on amino acid sequences to be searched for a modified site, (1) and (3) illustrated in FIG. 4 were used.

### (2-4) Analysis Result of Modified Site of Trastuzumab by LC-MS/MS

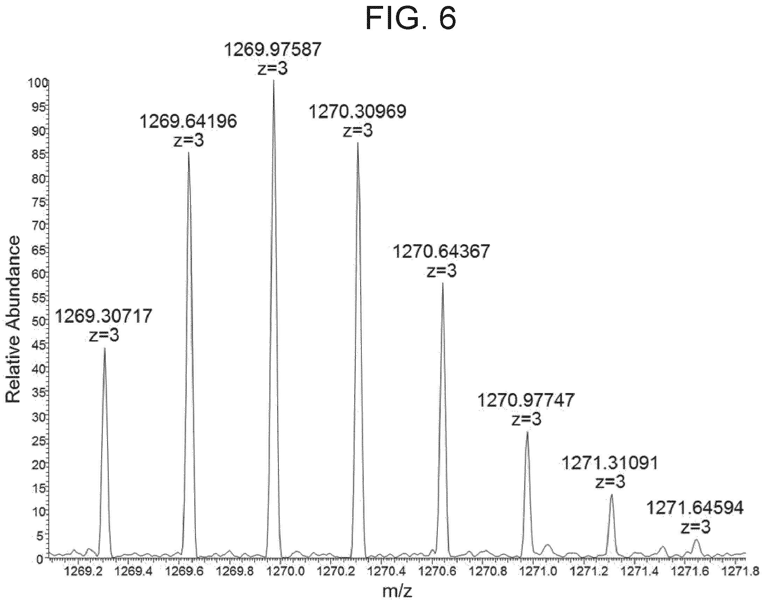

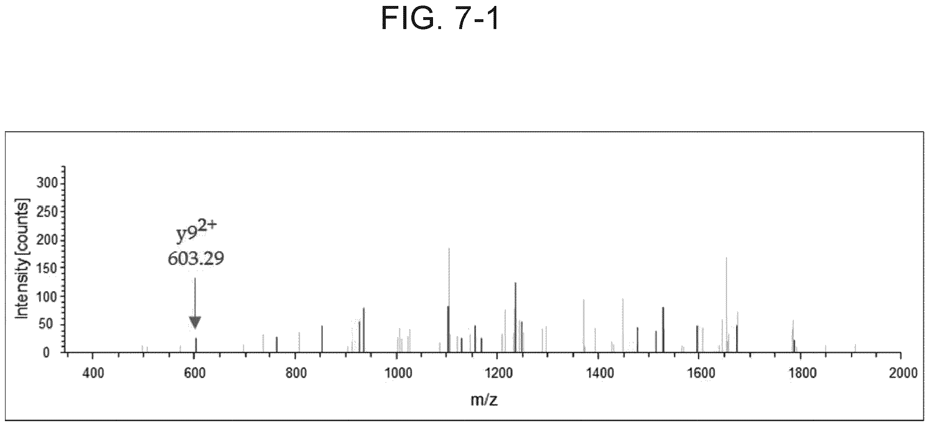

After analysis using LC-MS/MS, an MS spectrum of the peptide fragment of THTCPPCPAPELLGGPSVFLFPPKPKDTLMISR (SEQ ID NO: 6), which is a peptide consisting of 33 amino acid residues comprising a modified site to a lysin residue by trypsin digestion of trastuzumab (a thiol-introduced portion subjected to carbamidomethylation with iodoacetamide (+145.019 Da)) (measured value: m/z 1269.30717; theoretical value: 1269.30273; and trivalent) was observed (FIG. 6), and from a CID spectrum, a product ion of m/z 603.29 (theoretical value: 603.30) corresponding to divalent y9 indicating modification of a lysine residue at position 248 of a heavy chain in EU numbering was determined (FIG. 7-1). In addition, analysis with Proteome Discoverer indicated that modification to a lysine residue at position 246 or 248 occurred highly selectively (FIG. 7-2) .

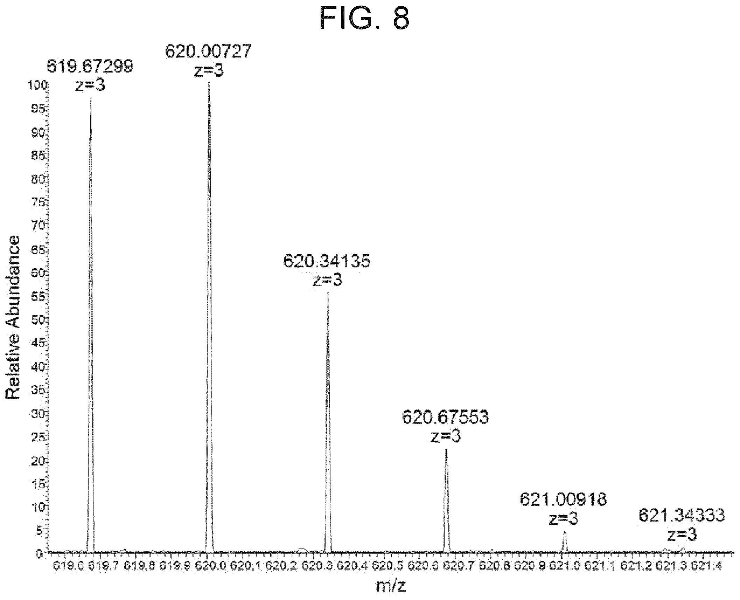

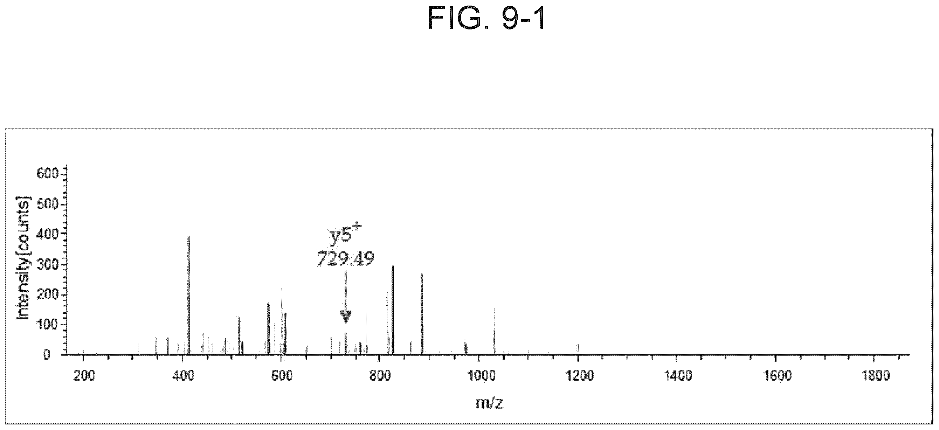

An MS spectrum of the peptide fragment of LLGGPSVFLFPPKPKD (SEQ ID NO: 7), which is a peptide consisting of 16 amino acid residues comprising a modified site to a lysine residue by Glu-C Protease digestion of trastuzumab (a thiol-introduced portion subjected to carbamidomethylation with iodoacetic acid (+145.019Da)) (measured value: m/z 619.67299; theoretical value: 619.67112; and trivalent) was observed (FIG. 8); and from a CID spectrum, a product ion of m/z 729.49 (theoretical value: 729.36) corresponding to monovalent y5 indicating modification of a lysine residue at position 246 or 248 of a heavy chain in EU numbering was determined (FIG. 9-1). In addition, analysis with Proteome Discoverer indicated that modification to a lysine residue at position 246 or 248 occurred highly selectively (FIG. 9-2).

From this result, it was found that in (1-3) of Example 1, conjugation progressed regioselectively at Lys246 and Lys248 in EU numbering of the heavy chain on the antibody.

### [Example 3: Disulfide Linker Cleavage, Reoxidation, and Fluorescent Labeling of Trastuzumab-Peptide Conjugate, and Analysis of Product Thereof]

### (3-1) Linker Cleavage and Reoxidation of Trastuzumab-Peptide Conjugate and Analysis of Product thereof by ESI-TOFMS

First, 1.9 mg of the trastuzumab-peptide conjugate synthesized in (1-4) of Example 1 was dissolved in a 60 mM phosphate buffer (pH 7.0) to be 18 µM, then 51.4 µL of 10 mM tris(2-carboxyethyl) phosphine (40 equivalents with respect to the trastuzumab-peptide conjugate) was added thereto, and the solution was stirred at room temperature for one hour to cleave the disulfide bond in the linker.

Next, to again form the disulfide bond in the antibody cleaved together with the disulfide bond in the linker, a process of reoxidation was performed (Jagath R Junutula et al., NATURE BIOTECHNOLOGY, 26(8), 925-932 (2008)). Specifically, the reaction solution was subjected to solvent substitution to a 9.57 mM PBS buffer (pH 7.0) with Amicon 10K to make the concentration of the trastuzumab-peptide conjugate 18 µM, then 48.6 µL of 10 mM dehydroascorbate (40 equivalents with respect to the trastuzumab-peptide conjugate) was added thereto, and the solution was stirred for three hours to perform reoxidation. The mass was measured by ESI-TOFMS; for a product, a peak was observed at 145329 with two thiopropionyl groups introduced.

Next, fluorescent labeling was performed on the regioselectively introduced thiopropionyl group. The trastuzumab having a thiopropionyl group introduced was subjected to solvent substitution to a 9.57 mM PBS buffer (pH 7.0) to make the concentration of the trastuzumab-peptide conjugate 18 µM, then 6.8 µL of 5 mM fluorescein-5-maleimide (10 equivalents with respect to the thiopropionyl group-introduced trastuzumab) was added thereto, and the solution was stirred for two hours to perform fluorescent labeling. To the formed fluorescently labeled trastuzumab, 5 µL of a 7 mM tris(2-carboxyethyl) phosphine hydrochloride solution (an equivalent with respect to the antibody) was added, the resulting mixture was stirred at room temperature for 20 minutes, and the mass was measured by ESI-TOFMS. For the raw material trastuzumab thiopropynyl, heavy chain peaks were observed at 50682 and 50844, and a light chain peak was observed at 23440, whereas for a product, peaks were observed at 51109 and 51272 with a fluorescently labeled portion introduced to the heavy chain, and a light chain peak was observed at 23440, the same as that of the raw material. Since the peaks at 50682 and 50844 of the heavy chain raw material disappeared during the measurement of the product, it is believed that fluorescein-5-maleimide reacted with all the heavy chains. From this result, it is believed that the drug antibody ratio (DAR) can be controlled to 2.0 even when the actual ADC is synthesized.

### (3-2) HIC-UPLC Analysis after Linker Cleavage and Reoxidation

The antibody-peptide conjugate formed in (3-1) and the raw material antibody were analyzed by HIC. For a column, Protein-Pak Hi Res HIC Column (Waters) 4.6 × 100 mm 2.5 µm was used. Detection was performed with A Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: trastuzumab + 30 equivalents of the peptide- and disulfide linker-coupled NHS-activation compound;
b: trastuzumab + sample after linker cleavage and reoxidation;

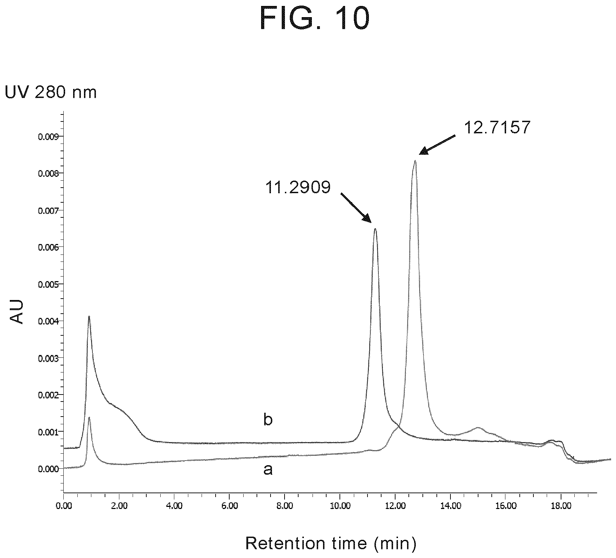

A retension time of 12.7157 minutes is attributed to a compound with two peptides introduced to trastuzumab, whereas that of 11.2909 minutes is attributed to a compound with two thiopropionyl groups introduced to trastuzumab (FIG. 10).

### [Example 4: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (4-1) Synthesis of IgG1 Fc-Binding Peptide

The peptide of Ac-RGNCAYHKGQIVWCTYH-NH₂ (SEQ ID NO: 8) was synthesized and purified in a similar method to (1-1) of Example 1 to obtain a target product (63.2 mg, 30.4 µπιοi) .

MS(ESI) m/z:z=3 693.35[M+3H]³⁺, z=4 520.30 [M+4H]⁴⁺

### (4-2) Formation of Intra-Molecular Disulfide Bond between Cys at Positions 4 and 14 of Ac-RGNCAYHKGQIVWCTYH-NH₂ (SEQ ID NO: 8)

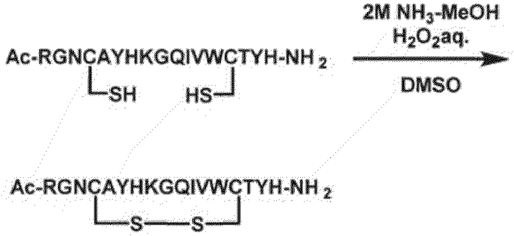

The amino acid sequence is SEQ ID NO: 8.

The peptide (63.2 mg, 30.4 µmol) synthesized in (4-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (25.3 mg, 12.2 µmol).

MS(ESI) m/z:z=3 692.70[M+3H]³⁺, z=4 520.30[M+4H]⁴⁺

### (4-3) Coupling between Disulfide Linker and Peptide

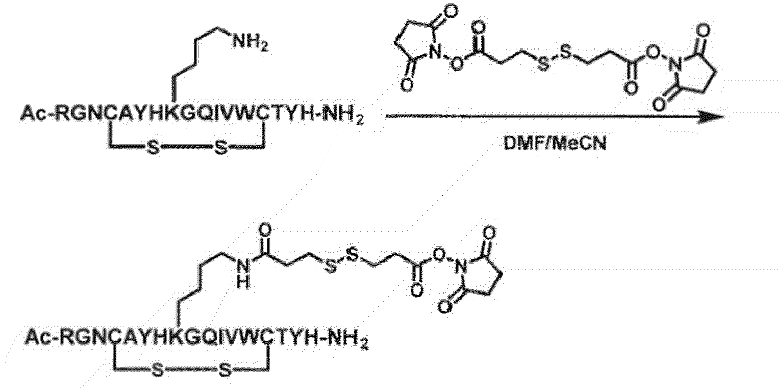

The amino acid sequence is SEQ ID NO: 8.

Ac-RGNCAYHKGQIVWCTYH-NH₂ (SEQ ID NO: 8) synthesized in (4-2) (25.3 mg, 12.2 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (197 mg, 488 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.0 mg, 2.54 µmol).

MS(ESI) m/z:z=3 789.20[M+3H]³⁺
HPLC purity: 89%

### (4-4) Specific Modification of Anti-HER2 IgG antibody Trastuzumab and Analysis by ESI-TOFMS

The peptide- and disulfide linker-coupled NHS-activation compound synthesized in (4-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150314, and a peak for a product with two binding peptides introduced was observed at 152568.

### (4-5) Determination of Heavy Chain Selectivity of Specifically Modified Compound of Trastuzumab under Reduction Conditions by ESI-TOFMS Analysis

To the antibody-peptide conjugate formed in (4-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23439, whereas for a product, peaks were observed at 50682 and 50844 with a thiopropionyl group introduced to the heavy chain, and a light chain peak was observed at 23439, the same as that of the raw material.

### (4-6) HIC-UPLC Analysis of Specific Modification of Anti-HER2 IgG Antibody Trastuzumab

The antibody-peptide conjugate formed in (4-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

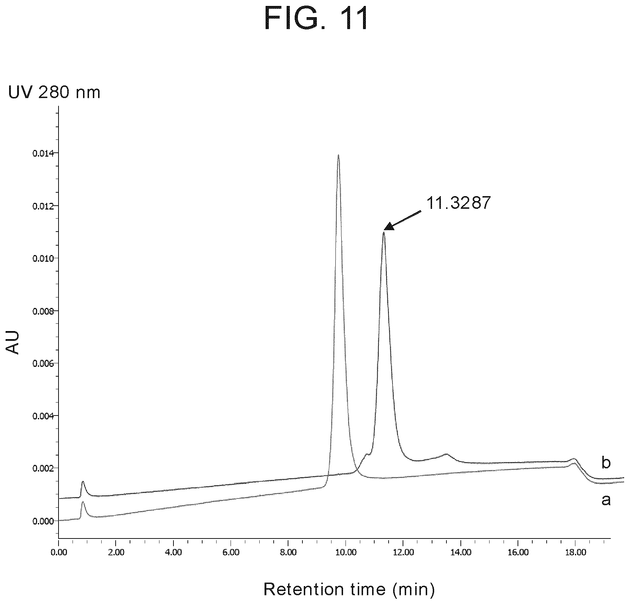

It is believed that a retension time of 10.6944 minutes is attributed to a compound with one peptide introduced to trastuzumab, whereas that of 11.3287 minutes is attributed to a compound with two peptides introduced to trastuzumab (FIG. 11).

### (4-7) Linker Cleavage and Reoxidation of Trastuzumab-Peptide Conjugate

By performing linker cleavage and reoxidation of the antibody-peptide conjugate obtained in (4-4) by the method described in (3-1) of Example 3, it is possible to obtain an antibody in which thiopropionyl groups are regioselectively introduced to Lys246 and Lys248 in EU numbering.

### [Example 5: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (5-1) Synthesis of IgG1 Fc-Binding Peptide

The peptide of Ac-RGNCAYHKGQVVWCTYH-NH₂ (SEQ ID NO: 9) was synthesized and purified in a similar method to (1-1) of Example 1 to obtain a target product (69.3 mg, 33.6 µπιοi) .

MS(ESI) m/z:z=3 688.80[M+3H]³⁺, z=4 516.85[M+4H]⁴⁺

### (5-2) Formation of Intra-Molecular Disulfide Bond between Cys at Positions 4 and 14 of Ac-RGNCAYHKGQVVWCTYH-NH₂ (SEQ ID NO: 9)

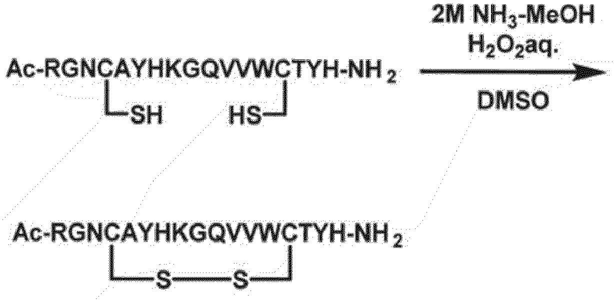

The amino acid sequence is SEQ ID NO: 9.

The peptide (69.3 mg, 33.6 µmol) synthesized in (5-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (35.8 mg, 17.3 µπιοi) .

MS(ESI) m/z:z=3 688.05[M+3H]³⁺, z=4 516.30[M+4H]⁴⁺

### (5-3) Coupling between Disulfide Linker and Peptide

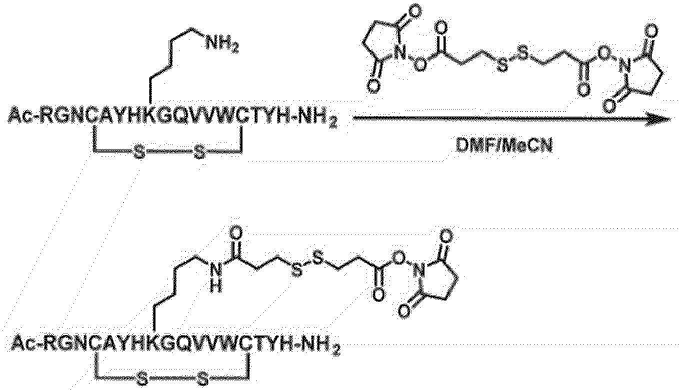

The amino acid sequence is SEQ ID NO: 9.

Ac-RGNCAYHKGQVVWCTYH-NH₂ (SEQ ID NO: 9) synthesized in (5-2) (35.8 mg, 17.3 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (280 mg, 692 µmol) in acetonitrile (2.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.5 mg, 2.34 µmol).

MS(ESI) m/z:z=3 784.55[M+3H]³⁺
HPLC purity: 71%

### (5-4) Specific Modification of Anti-HER2 IgG antibody Trastuzumab and Analysis by ESI-TOFMS

The peptide- and disulfide linker-coupled NHS-activation compound synthesized in (5-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150314, and a peak for a product with two binding peptides introduced was observed at 152568.

### (5-5) Determination of Heavy Chain Selectivity of Specifically Modified Compound of Trastuzumab under Reduction Conditions by ESI-TOFMS Analysis

To the antibody-peptide conjugate formed in (5-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23439, whereas for a product, peaks were observed at 50683 and 50845 with a thiopropionyl group introduced to the heavy chain, and a light chain peak was observed at 23439, the same as that of the raw material.

### (5-6) HIC-UPLC Analysis of Specific Modification of Anti-HER2 IgG Antibody Trastuzumab

The antibody-peptide conjugate formed in (5-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

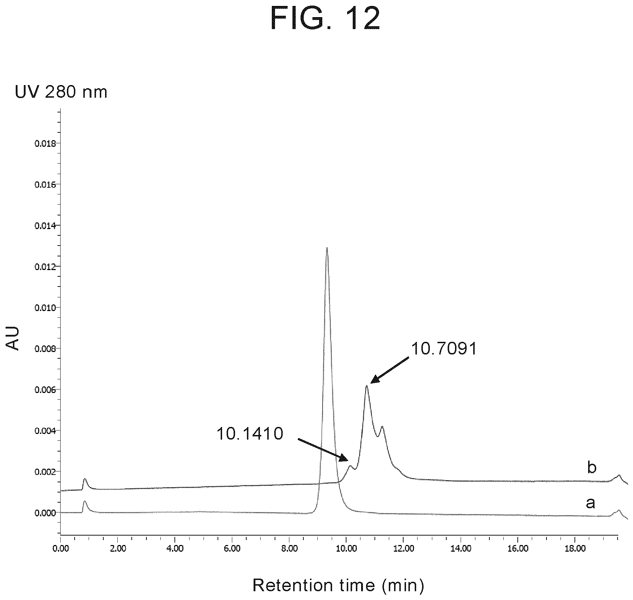

It is believed that a retension time of 10.1410 minutes is attributed to a compound with one peptide introduced to trastuzumab, whereas that of 10.7091 minutes is attributed to a compound with two peptides introduced to trastuzumab (FIG. 12).

### (5-7) Linker Cleavage and Reoxidation of Trastuzumab-Peptide Conjugate

By performing linker cleavage and reoxidation of the antibody-peptide conjugate obtained in (5-4) by the method described in (3-1) of Example 3, it is possible to obtain an antibody in which thiopropionyl groups are regioselectively introduced to Lys246 and Lys248 in EU numbering.

### [Example 6: Peptide Mapping of Thiol-introduced Trastuzumab]

### (6-1) Trypsin Treatment for Thiol-Introduced Trastuzumab

The antibody-peptide conjugate obtained in (5-3) of Example 5 was glycosylated with PNGaseF (manufactured by New England BioLabs), and then subjected to peptide mapping. To a 1.5 mL low-adsorptive micro test tube, 10 µL of a sample solution, a 50 mM ammonium hydrogencarbonate buffer, and 10 µL of a 20 mM aqueous dithiothreitol solution dissolved in 40% trifluoroethanol were added, and the resulting solution was heated at 65°C for one hour. Thereafter, 10 µL of a 50 mM aqueous iodoacetamide solution was added thereto, and the resulting solution was reacted in a dark place at room temperature while being shaken at 300 rpm for 30 minutes. After the reaction, 40 µL of a 50 mM ammonium hydrogencarbonate buffer was added thereto, the resulting mixture was stirred, 10 µL of a 20 ng/µL aqueous trypsin solution was added thereto, and the resulting mixture was subjected to enzyme digestion at 37°C for 18 hours. After digestion, 2 µL of a 20% aqueous trifluoroacetic acid solution was added thereto to stop the reaction, for which LC-MS/MS measurement was performed.

### (6-2) LC-MS/MS Measurement Conditions for Trastuzumab

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Acquisition
Activation Type:Collision Induced Dissociation(CID)
Data acquisition was performed using Xcalibur 3.0 (Thermo Fisher Scientific) and Thermo Orbitrap Fusion Tune Application 2.0 (Thermo Fisher Scientific) as accompanying software.

### (6-3) Analysis Condition of Modified Site of Trastuzumab

Modified site analysis of an LC-MS/MS measurement result was performed using Proteome Discoverer version 1.4 (Thermo Fisher Scientific).

For analysis with Proteome Discoverer, Sequest HT was used as a search engine, the range of precursor ion was set to 350 Da to 5,000 Da, Total Intensity Threshold was set to 50,000. Trypsin was set as a digestive enzyme, and Maximum Missed Cleavage Sites was set to 3. Mass Tolerance was set to 5 ppm and 0.5 Da for precursor and fragment ion, respectively. For Static Modification, Carbamidomethyl (+57.021 Da) was set as modification of a cysteine residue with iodoacetamide. For Dynamic Modifications, oxidation of methionine (+15.995 Da) and a modified compound to a lysine residue (a thiol-introduced portion subjected to carbamidomethylation with iodoacetamide (+145.019 Da)) were set. Furthermore, a filter was applied so as to cause Peptide Confidence to be only High.

As data on amino acid sequences to be searched for a modified site, (1) and (3) illustrated in FIG. 4 were used.

### (6-4) Analysis Result of Modified Site of Trastuzumab by LC-MS/MS

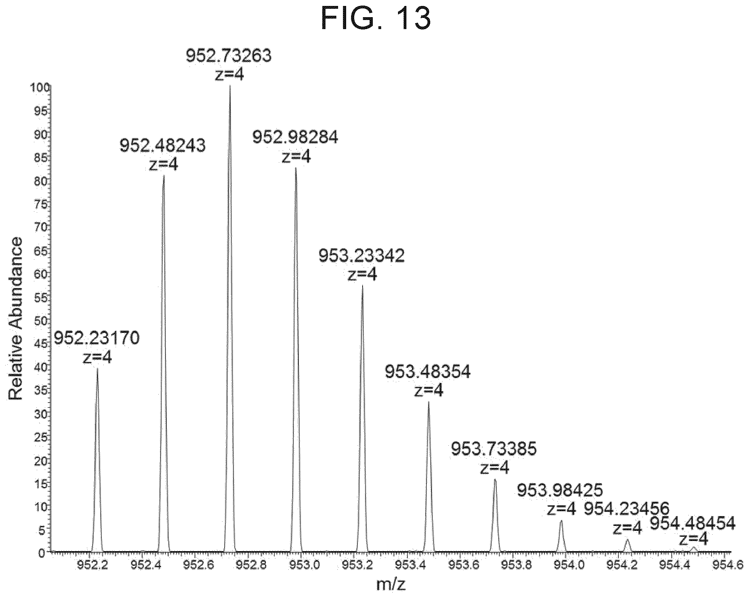

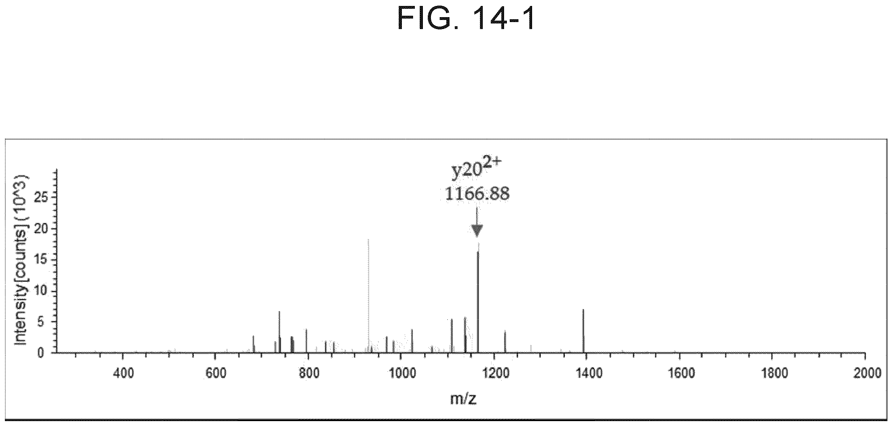

After analysis using LC-MS/MS, an MS spectrum of the peptide fragment of THTCPPCPAPELLGGPSVFLFPPKPKDTLMISR (SEQ ID NO: 6), which is a peptide consisting of 33 amino acid residues comprising a modified site to a lysin residue by trypsin digestion of trastuzumab (a thiol-introduced portion subjected to carbamidomethylation with iodoacetamide (+145.019 Da)) (measured value: m/z 952.23170; theoretical value: 952.22900; and tetravalent) was observed (FIG. 13), and from a CID spectrum, a product ion of m/z 1166.88 (theoretical value: 1166.61) corresponding to divalent y20 indicating modification of a lysine residue at position 246 or position 248 of a heavy chain in EU numbering was determined (FIG. 14-1). In addition, analysis with Proteome Discoverer indicated that modification to a lysine residue at position 246 or 248 occurred highly selectively (FIG. 14-2). From this result, it was found that in (5-3) of Example 5, conjugation progressed regioselectively at Lys246 and Lys248 in EU numbering on the heavy chain on the antibody.

### [Example 7: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (7-1) Synthesis of IgG1 Fc-Binding Peptide

The peptide of Ac-RGNCAYHKGQAVWCTYH-NH₂ (SEQ ID NO: 10) was synthesized and purified in a similar method to (1-1) of Example 1 to obtain a target product (73.1 mg, 35.9 µπιοi) .

MS(ESI) m/z:z=3 674.70[M+3H]³⁺, z=4 506.30[M+4H]⁴⁺

### (7-2) Formation of Intra-Molecular Disulfide Bond between Cys at Positions 4 and 14 of Ac-RGNCAYHKGQAVWCTYH-NH₂ (SEQ ID NO: 10)

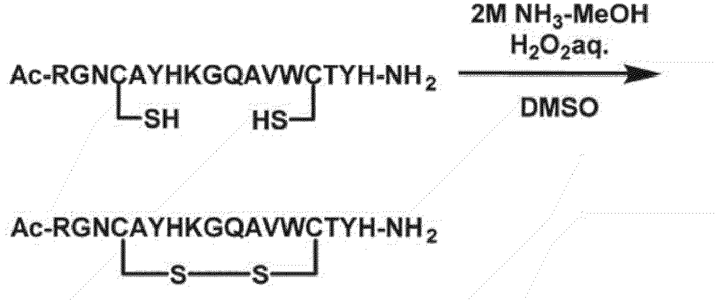

The amino acid sequence is SEQ ID NO: 10.

The peptide (73.1 mg, 35.9 µmol) synthesized in (7-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (30.5 mg, 15.0 µmol).

MS(ESI) m/z:z=3 678.75[M+3H]³⁺, z=4 509. 30 [M+4H]⁴⁺

### (7-3) Coupling between Disulfide Linker and Peptide

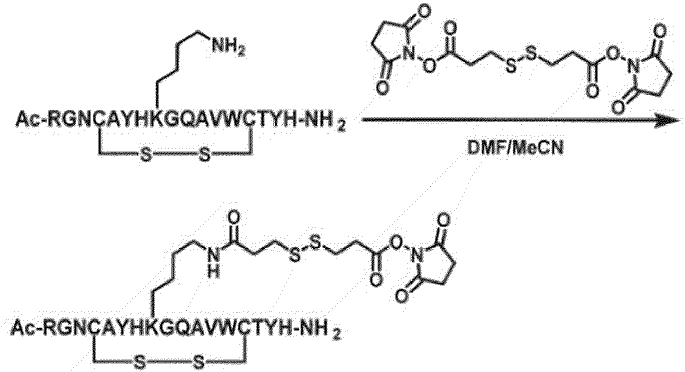

The amino acid sequence is SEQ ID NO: 10.

Ac-RGNCAYHKGQAVWCTYH-NH₂ (SEQ ID NO: 10) synthesized in (7-2) (30.5 mg, 15.0 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (243 mg, 600 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.4 mg, 2.32 µmol).

MS(ESI) m/z:z=3 775.15[M+3H]³⁺
HPLC purity: 89%

### (7-4) Specific Modification of Anti-HER2 IgG antibody Trastuzumab and Analysis by ESI-TOFMS

The peptide- and disulfide linker-coupled NHS-activation compound synthesized in (7-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150268, and a peak for a product with two binding peptides introduced was observed at 152630.

### (7-5) Determination of Heavy Chain Selectivity of Specifically Modified Compound of Trastuzumab under Reduction Conditions by ESI-TOFMS Analysis

To the antibody-peptide conjugate formed in (7-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50683 and 50844 with a thiopropionyl group introduced to the heavy chain, and a light chain peak was observed at 23439, the same as that of the raw material.

### (7-6) HIC-UPLC Analysis of Specific Modification of Anti-HER2 IgG antibody Trastuzumab

The antibody-peptide conjugate formed in (7-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

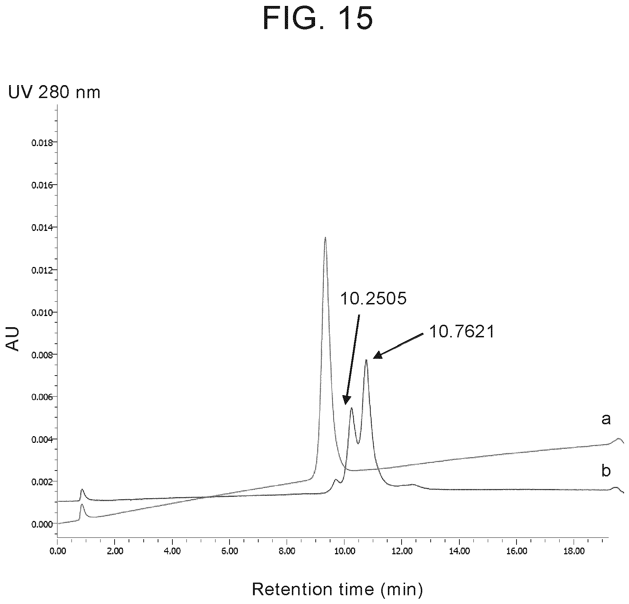

It is believed that a retension time of 10.2505 minutes is attributed to a compound with one peptide introduced to trastuzumab, whereas that of 10.7621 minutes is attributed to a compound with two peptides introduced to trastuzumab (FIG. 15).

### (7-7) Linker Cleavage and Reoxidation of Trastuzumab-Peptide Conjugate

By performing linker cleavage and reoxidation of the antibody-peptide conjugate obtained in (7-4) by the method described in (3-1) of Example 3, it is possible to obtain an antibody in which thiopropionyl groups are regioselectively introduced to Lys246 and Lys248 in EU numbering.

### [Example 8: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (8-1) Synthesis of IgG1 Fc-Binding Peptide

The peptide of Ac-RGNCAYHKGQLLWCTYH-NH₂ (SEQ ID NO: 11) was synthesized and purified in a similar method to (1-1) of Example 1 to obtain a target product t (30.3 mg, 14.5 µπιοi) .

MS(ESI) m/z:z=3 698.05 [M+3H]³⁺, z=4 532 . 80 [M+4H] ⁴⁺

### (8-2) Formation of Intra-Molecular Disulfide Bond between Cys at Positions 4 and 14 of Ac-RGNCAYHKGQLLWCTYH-NH₂ (SEQ ID NO: 11)

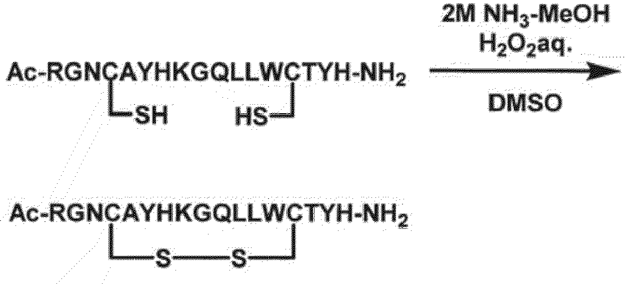

The amino acid sequence is SEQ ID NO: 11.

The peptide (30.3 mg, 14.5 µmol) synthesized in (8-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (11.9 mg, 5.70 µπιοi) .

MS(ESI) m/z:z=3 697.35 [M+3H]³⁺, z=4 523.30 [M+4H]⁴⁺

### (8-3) Coupling between Disulfide Linker and Peptide

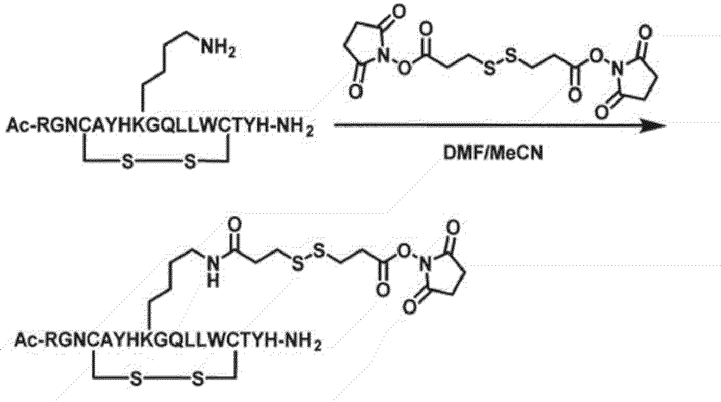

The amino acid sequence is SEQ ID NO: 11.

Ac-RGNCAYHKGQLLWCTYH-NH₂ (SEQ ID NO: 11) synthesized in (8-2) (11.9 mg, 5.70 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (92 mg, 228 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.0 mg, 2.52 µπιοi) .

MS(ESI) m/z:z=3 793.85[M+3H]³⁺
HPLC purity: 75%

### (8-4) Specific Modification of Anti-HER2 IgG antibody Trastuzumab and Analysis by ESI-TOFMS

The peptide- and disulfide linker-coupled NHS-activation compound synthesized in (8-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148073, whereas a peak for a product with one binding peptide introduced was observed at 150495, and a peak for a product with two binding peptides introduced was observed at 152752.

### (8-5) Determination of Heavy Chain Selectivity of Specifically Modified Compound of Trastuzumab under Reduction Conditions by ESI-TOFMS Analysis

To the antibody-peptide conjugate formed in (8-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (8-6) HIC-UPLC Analysis of Specific Modification of Anti-HER2 IgG antibody Trastuzumab

The antibody-peptide conjugate formed in (8-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

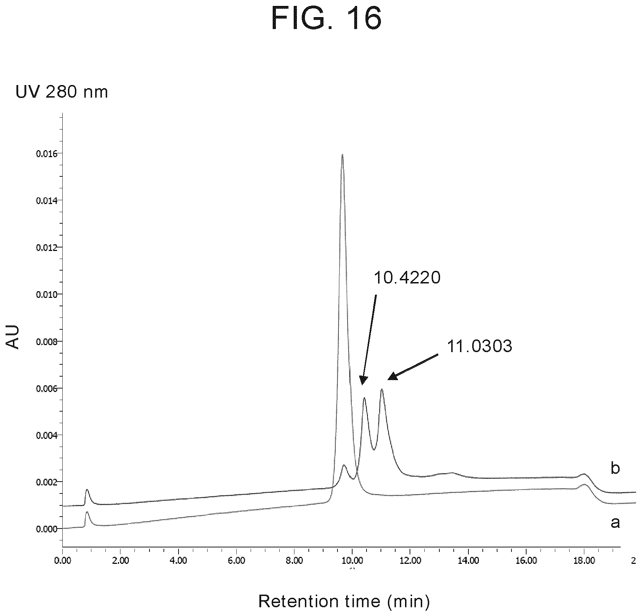

It is believed that a retension time of 10.4200 minutes is attributed to a compound with one peptide introduced to trastuzumab, whereas that of 11.0303 minutes is attributed to a compound with two peptides introduced to trastuzumab (FIG. 16).

### (8-7) Linker Cleavage and Reoxidation of Trastuzumab-Peptide Conjugate

By performing linker cleavage and reoxidation of the antibody-peptide conjugate obtained in (8-4) by the method described in (3-1) of Example 3, it is possible to obtain an antibody in which thiopropionyl groups are regioselectively introduced to Lys246 and Lys248 in EU numbering.

### [Example 9: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (9-1) Synthesis of IgG1 Fc-Binding Peptide

The peptide of Ac-RGNCAYHKGQLIWCTYH-NH₂ (SEQ ID NO: 12) was synthesized and purified in a similar method to (1-1) of Example 1 to obtain a target product (40.0 mg, 19.1 µπιοi) .

MS(ESI) m/z:z=3 698.10[M+3H]³⁺, z=4 532.80[M+4H]⁴⁺

### (9-2) Formation of Intra-Molecular Disulfide Bond between Cys at Positions 4 and 14 of Ac-RGNCAYHKGQLIWCTYH-NH₂ (SEQ ID NO: 12)

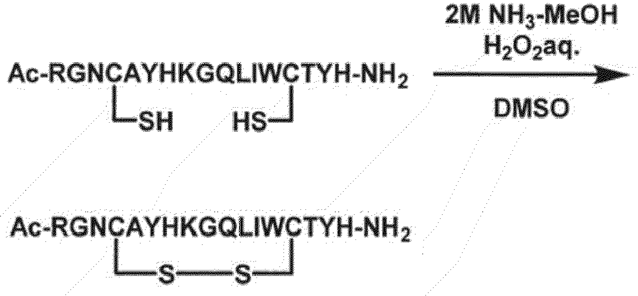

The amino acid sequence is SEQ ID NO: 12.

The peptide (40.0 mg, 19.1 µmol) synthesized in (9-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (13.5 mg, 6.46 µπιοi) .

MS(ESI) m/z:z=3 697.40[M+3H]³⁺, z=4 523.30[M+4H]⁴⁺

### (9-3) Coupling between Disulfide Linker and Peptide

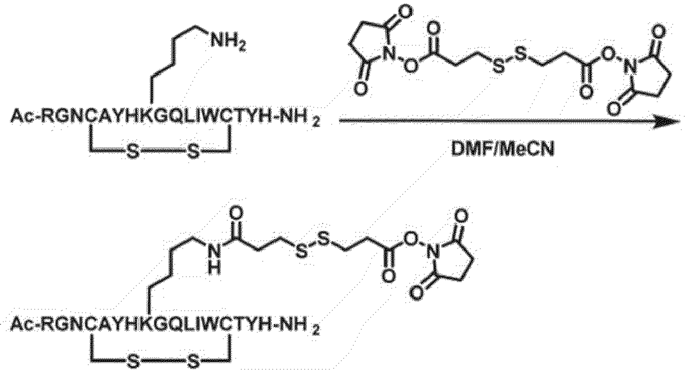

The amino acid sequence is SEQ ID NO: 12.

Ac-RGNCAYHKGQLIWCTYH-NH₂ (SEQ ID NO: 12) synthesized in (9-2) (13.5 mg, 6.46 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (104 mg, 258 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.5 mg, 2.31 µmol).

MS(ESI) m/z:z=3 793.75[M+3H]³⁺
HPLC purity: 90%

### (9-4) Specific Modification of Anti-HER2 IgG antibody Trastuzumab and Analysis by ESI-TOFMS

The peptide- and disulfide linker-coupled NHS-activation compound synthesized in (9-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152758.

### (9-5) Determination of Heavy Chain Selectivity of Specifically Modified Compound of Trastuzumab under Reduction Conditions by ESI-TOFMS Analysis

To the antibody-peptide conjugate formed in (9-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (9-6) HIC-UPLC Analysis of Specific Modification of Anti-HER2 IgG antibody Trastuzumab

The antibody-peptide conjugate formed in (9-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

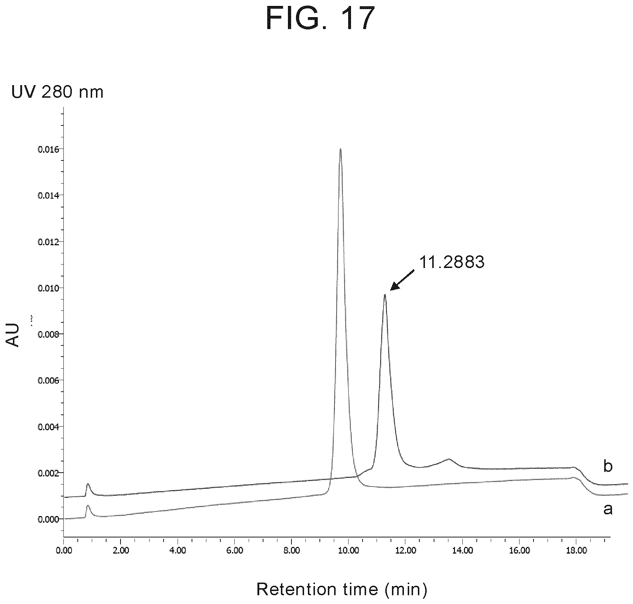

It is believed that a retension time of 11.2883 minutes is attributed to a compound with two peptides introduced to trastuzumab (FIG. 17).

### (9-7) Linker Cleavage and Reoxidation of Trastuzumab-Peptide Conjugate

By performing linker cleavage and reoxidation of the antibody-peptide conjugate obtained in (9-4) by the method described in (3-1) of Example 3, it is possible to obtain an antibody in which thiopropionyl groups are regioselectively introduced to Lys246 and Lys248 in EU numbering.

### [Example 10: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (10-1) Synthesis of IgG1 Fc-Binding Peptide

The peptide of Ac-DCAYHKGQIVWCT-NH₂ (SEQ ID NO: 13) was synthesized and purified in a similar method to (1-1) of Example 1 to obtain a target product (314 mg, 200 µmol).

MS(ESI) m/z:z=2 783.30[M+2H]²⁺

### (10-2) Formation of Intra-Molecular Disulfide Bond between Cys at Positions 2 and 12 of Ac-DCAYHKGQIVWCT-NH₂ (SEQ ID NO: 13)

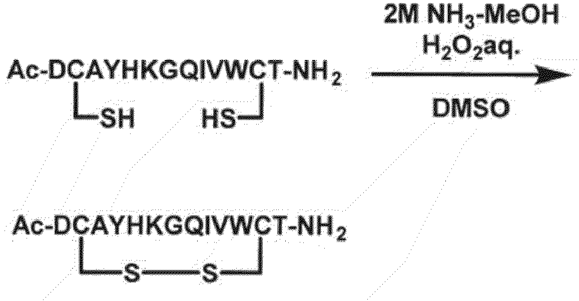

The amino acid sequence is SEQ ID NO: 13.

The peptide (314 mg, 200 µmol) synthesized in (10-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (47.8 mg, 30.6 µmol).

MS(ESI) m/z:z=2 782.20[M+2H]²⁺

### (10-3) Coupling between Disulfide Linker and Peptide

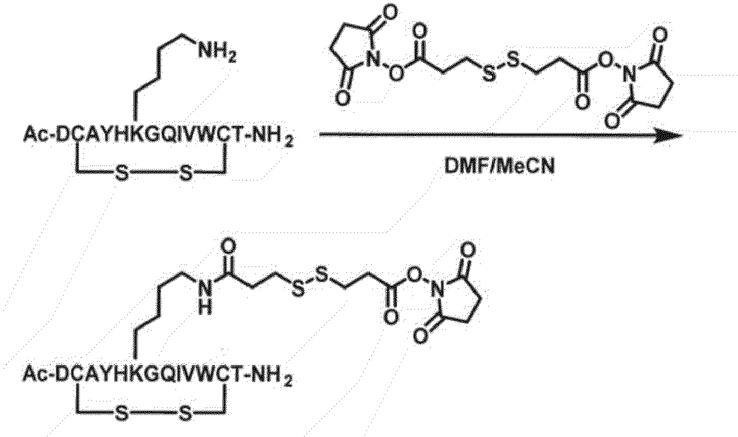

The amino acid sequence is SEQ ID NO: 13.

Ac-DCAYHKGQIVWCT-NH₂ (SEQ ID NO: 13) synthesized in (10-2) (47.8 mg, 30.6 µmol, the 2nd and 12th of two cysteines form a disulfide bond in the molecule) was dissolved in N,N'-dimethylformamide (1.00 mL), a solution dissolving 3,3'-dithiodipropionic acid di(N-succinimidyl) (493 mg, 1.22 mmol) in acetonitrile (2.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (14.0 mg, 7.59 µmol).

MS(ESI) m/z:z=2 926.70[M+2H]²⁺
HPLC purity: 90%

### (10-4) Specific Modification of Anti-HER2 IgG antibody Trastuzumab and Analysis by ESI-TOFMS

The peptide- and disulfide linker-coupled NHS-activation compound synthesized in (10-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152053.

### (10-5) Determination of Heavy Chain Selectivity of Specifically Modified Compound of Trastuzumab under Reduction Conditions by ESI-TOFMS Analysis

To the antibody-peptide conjugate formed in (10-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (10-6) HIC-UPLC Analysis of Specific Modification of Anti-HER2 IgG antibody Trastuzumab

The antibody-peptide conjugate formed in (10-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

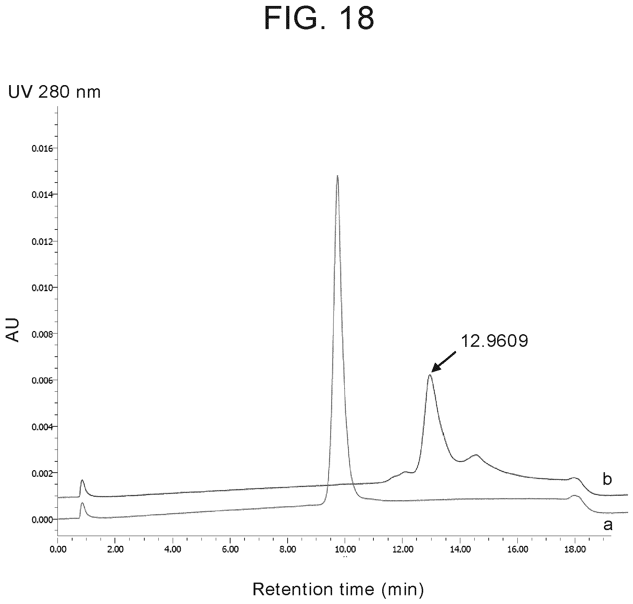

It is believed that a retension time of 12.9609 minutes is attributed to a compound with two peptides introduced to trastuzumab (FIG. 18).

### (10-7) Linker Cleavage and Reoxidation of Trastuzumab-Peptide Conjugate

By performing linker cleavage and reoxidation of the antibody-peptide conjugate obtained in (10-4) by the method described in (3-1) of Example 3, it is possible to obtain an antibody in which thiopropionyl groups are regioselectively introduced to Lys246 and Lys248 in EU numbering.

### [Example 11: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (11-1) Synthesis of IgG1 Fc-Binding Peptide

The peptide of Ac-DCAYHKGQVVWCT-NH₂ (SEQ ID NO: 14) was synthesized and purified in a similar method to (1-1) of Example 1 to obtain a target product (180 mg, 116 µmol).

MS(ESI) m/z:z=1 1551.0[M+H]⁺,z=2 776.30[M+2H]²⁺

### (11-2) Formation of Intra-Molecular Disulfide Bond between Cys at Positions 2 and 12 of Ac-DCAYHKGQWWCT-NH₂ (SEQ ID NO: 14)

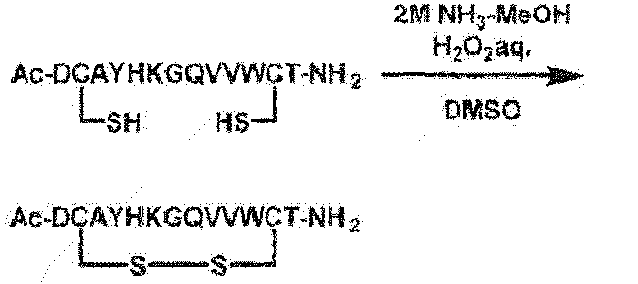

The amino acid sequence is SEQ ID NO: 14.

The peptide (180 mg, 116 µmol) synthesized in (11-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (36.4 mg, 23.5 µmol).

MS(ESI) m/z:z=1 1549.95[M+H]⁺,z=2 775.30[M+2H]²⁺

### (11-3) Coupling between Disulfide Linker and Peptide

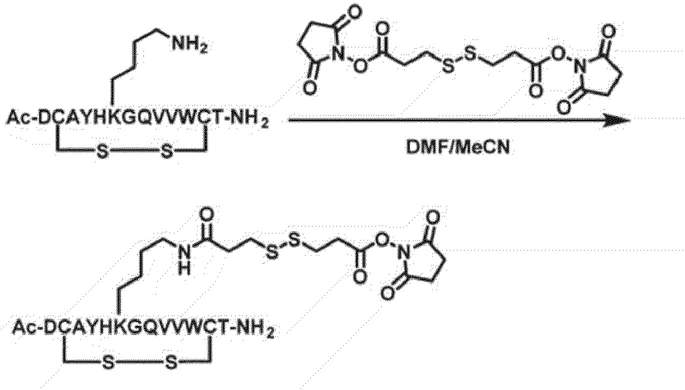

The amino acid sequence is SEQ ID NO: 14.

Ac-DCAYHKGQVVWCT-NH₂ (SEQ ID NO: 14) synthesized in (11-2) (36.4 mg, 23.5 µmol, the 2nd and 12th of two cysteines form a disulfide bond in the molecule) was dissolved in N,N'-dimethylformamide (1.50 mL), a solution dissolving 3,3'-dithiodipropionic acid di(N-succinimidyl) (380 mg, 0.94 mmol) in acetonitrile (2.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (16.0 mg, 8.70 µπιοi) .

MS(ESI) m/z:z=2 919.95[M+2H]²⁺
HPLC purity: 90%

### (11-4) Specific Modification of Anti-HER2 IgG antibody Trastuzumab and Analysis by ESI-TOFMS

The peptide- and disulfide linker-coupled NHS-activation compound synthesized in (11-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149220, and a peak for a product with two binding peptides introduced was observed at 151861.

### (11-5) Determination of Heavy Chain Selectivity of Specifically Modified Compound of Trastuzumab under Reduction Conditions by ESI-TOFMS Analysis

To the antibody-peptide conjugate formed in (11-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (11-6) HIC-UPLC Analysis of Specific Modification of Anti-HER2 IgG antibody Trastuzumab

The antibody-peptide conjugate formed in (11-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

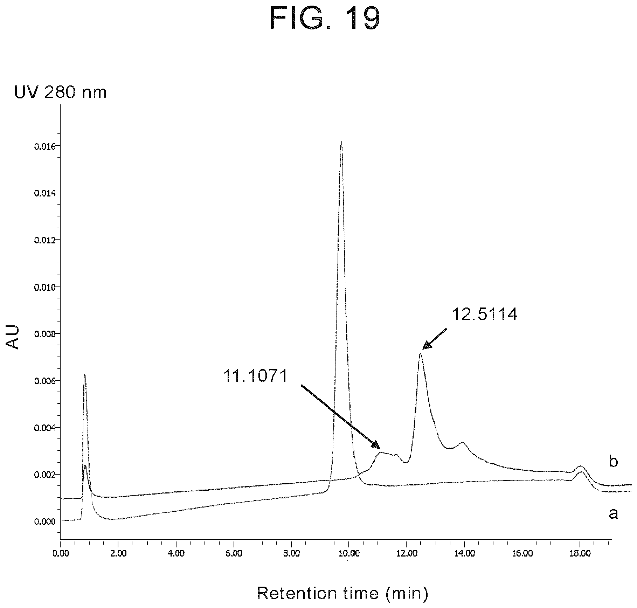

It is believed that a retension time of 11.1071 minutes is attributed to a compound with one peptide introduced to trastuzumab, whereas that of 12.5114 minutes is attributed to a compound with two peptides introduced to trastuzumab (FIG. 19).

### (11-7) Linker Cleavage and Reoxidation of Trastuzumab-Peptide Conjugate

By performing linker cleavage and reoxidation of the antibody-peptide conjugate obtained in (11-4) by the method described in (3-1) of Example 3, it is possible to obtain an antibody in which thiopropionyl groups are regioselectively introduced to Lys246 and Lys248 in EU numbering.

### [Example 12: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (12-1) Synthesis of IgG1 Fc-Binding Peptide

The peptide of Ac-DCAYHKGQAVWCT-NH₂ (SEQ ID NO: 15) was synthesized and purified in a similar method to (1-1) of Example 1 to obtain a target product (158 mg, 104 µmol).

MS(ESI) m/z:z=1 1523.95 [M+H]⁺, z=2 762.25[M+2H]²⁺

### (12-2) Formation of Intra-Molecular Disulfide Bond between Cys at Positions 2 and 12 of Ac-DCAYHKGQAVWCT-NH₂ (SEQ ID NO: 15)

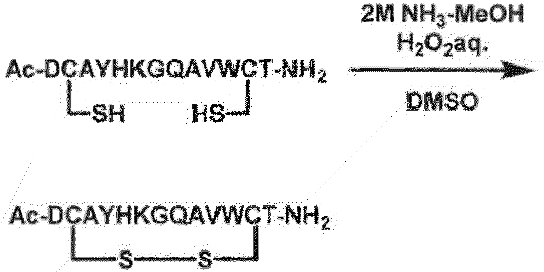

The amino acid sequence is SEQ ID NO: 15.

The peptide (158 mg, 104 µmol) synthesized in (12-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (26.7 mg, 17.6 µπιοi) .

MS(ESI) m/z:z=1 1521.80[M+H]⁺,z=2 761.20[M+2H]²⁺

### (12-3) Coupling between Disulfide Linker and Peptide

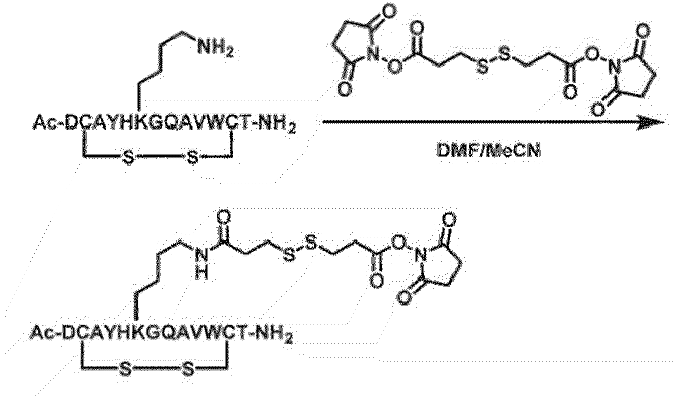

The amino acid sequence is SEQ ID NO: 15.

Ac-DCAYHKGQAVWCT-NH₂ (SEQ ID NO: 15) synthesized in (12-2) (26.7 mg, 17.6 µmol, the 2nd and 12th of two cysteines form a disulfide bond in the molecule) was dissolved in N,N'-dimethylformamide (1.50 mL), a solution dissolving 3,3'-dithiodipropionic acid di(N-succinimidyl) (380 mg, 0.94 mmol) in acetonitrile (2.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.50 mg, 3.59 µmol).

MS(ESI) m/z:z=2 906.10[M+2H]²⁺
HPLC purity: 93%

### (12-4) Specific Modification of Anti-HER2 IgG antibody Trastuzumab and Analysis by ESI-TOFMS

The peptide- and disulfide linker-coupled NHS-activation compound synthesized in (12-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 148897, and a peak for a product with two binding peptides introduced was observed at 151619.

### (12-5) Determination of Heavy Chain Selectivity of Specifically Modified Compound of Trastuzumab under Reduction Conditions by ESI-TOFMS Analysis

To the antibody-peptide conjugate formed in (12-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50684 and 50844 with a thiopropionyl group introduced to the heavy chain, and a light chain peak was observed at 23439, the same as that of the raw material.

### (12-6) HIC-UPLC Analysis of Specific Modification of Anti-HER2 IgG antibody Trastuzumab

The antibody-peptide conjugate formed in (12-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

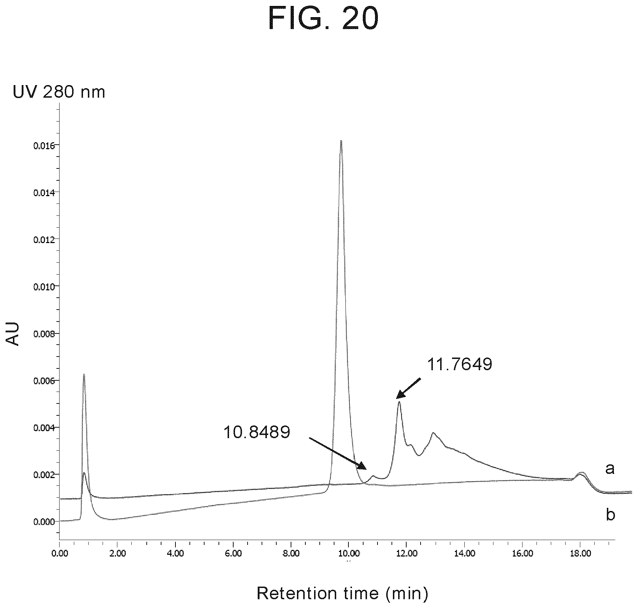

It is believed that a retension time of 10.8489 minutes is attributed to a compound with one peptide introduced to trastuzumab, whereas that of 11.7649 minutes is attributed to a compound with two peptides introduced to trastuzumab (FIG. 20).

### (12-7) Linker Cleavage and Reoxidation of Trastuzumab-Peptide Conjugate

By performing linker cleavage and reoxidation of the antibody-peptide conjugate obtained in (12-4) by the method described in (3-1) of Example 3, it is possible to obtain an antibody in which thiopropionyl groups are regioselectively introduced to Lys246 and Lys248 in EU numbering.

### [Example 13: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (13-1) Synthesis of IgG1 Fc-Binding Peptide

The peptide of Ac-RGNCAYHKSQIIWCTYH-NH₂ (SEQ ID NO: 16) was synthesized and purified in a similar method to (1-1) of Example 1 to obtain a target product (20.0 mg, 9.43 µπιοi) .

MS(ESI) m/z:z=3 708.00[M+3H]³⁺, z=4 531.35[M+4H]⁴⁺

### (13-2) Formation of Intra-Molecular Disulfide Bond between Cys at Positions 4 and 14 of Ac-RGNCAYHKSQIIWCTYH-NH₂ (SEQ ID NO: 16)

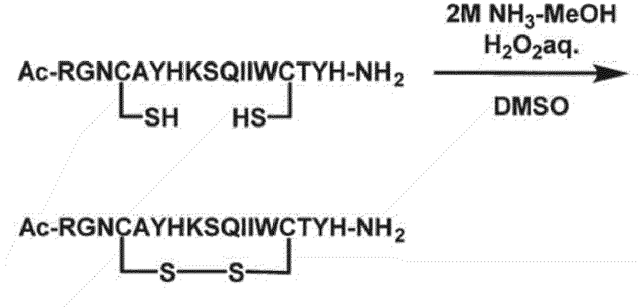

The amino acid sequence is SEQ ID NO: 16.

The peptide (20.0 mg, 9.43 µmol) synthesized in (13-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (12.0 mg, 5.66 µmol).

MS(ESI) m/z:z=3 707.50[M+3H]³⁺, z=4 530.85[M+4H]⁴⁺

### (13-3) Coupling between Disulfide Linker and Peptide

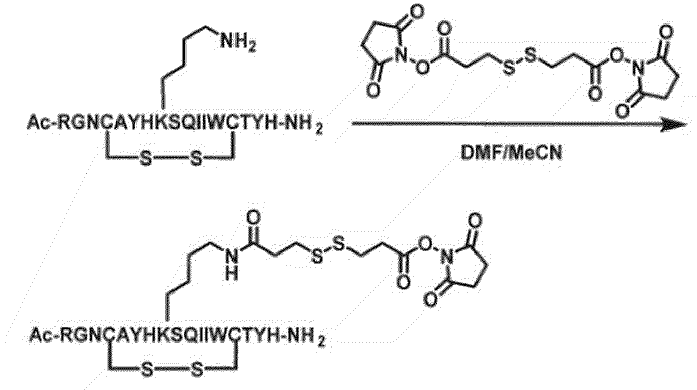

The amino acid sequence is SEQ ID NO: 16.

Ac-RGNCAYHKSQIIWCTYH-NH₂ (SEQ ID NO: 16) synthesized in (13-2) (12.0 mg, 5.66 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (45.7 mg, 113 µmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.20 mg, 1.33 µmol).

MS(ESI) m/z:z=3 803.80[M+3H]³⁺
HPLC purity: 92%

### (13-4) Specific Modification of Anti-HER2 IgG antibody Trastuzumab and Analysis by ESI-TOFMS

The peptide- and disulfide linker-coupled NHS-activation compound synthesized in (13-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152864.

### (13-5) Determination of Heavy Chain Selectivity of Specifically Modified Compound of Trastuzumab under Reduction Conditions by ESI-TOFMS Analysis

To the antibody-peptide conjugate formed in (13-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (13-6) HIC-UPLC Analysis of Specific Modification of Anti-HER2 IgG antibody Trastuzumab

The antibody-peptide conjugate formed in (13-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

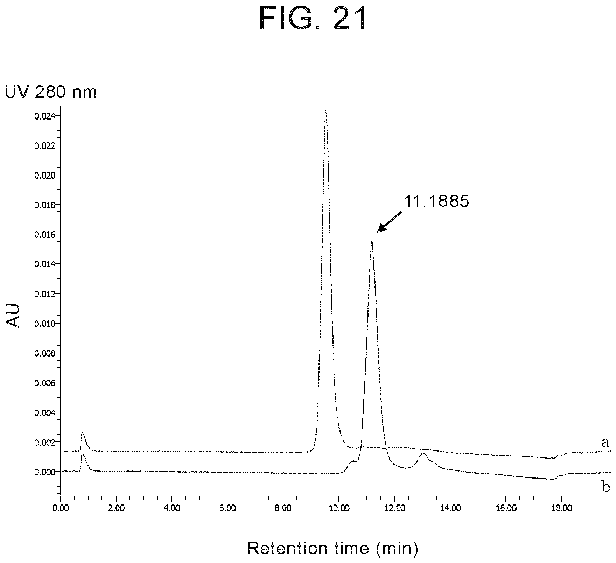

It is believed that a retension time of 11.1885 minutes is attributed to a compound with two peptides introduced to trastuzumab (FIG. 21).

### (13-7) Linker Cleavage and Reoxidation of Trastuzumab-Peptide Conjugate

By performing linker cleavage and reoxidation of the antibody-peptide conjugate obtained in (13-4) by the method described in (3-1) of Example 3, it is possible to obtain an antibody in which thiopropionyl groups are regioselectively introduced to Lys246 and Lys248 in EU numbering.

### [Example 14: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (14-1) Synthesis of IgG1 Fc-Binding Peptide

The peptide of Ac-RGNCAYHKNQIIWCTYH-NH₂ (SEQ ID NO: 17) was synthesized and purified in a similar method to (1-1) of Example 1 to obtain a target product (26.4 mg, 12.3 µπιοi) .

MS(ESI) m/z:z=3 717.00[M+3H]³⁺, z=4 537.95[M+4H]⁴⁺

### (14-2) Formation of Intra-Molecular Disulfide Bond between Cys at Positions 4 and 14 of Ac-RGNCAYHKNQIIWCTYH-NH₂ (SEQ ID NO: 17)

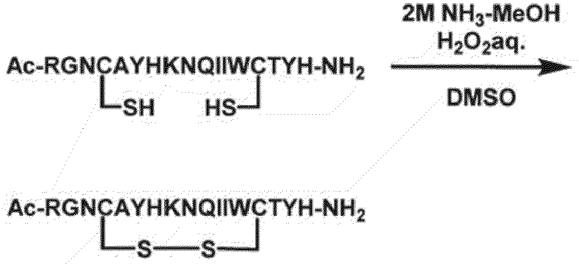

The amino acid sequence is SEQ ID NO: 17.

The peptide (26.4 mg, 12.3 µmol) synthesized in (14-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (13.0 mg, 6.06 µmol).

MS(ESI) m/z:z=3 716.15[M+3H]³⁺, z=4 537.55[M+4H]⁴⁺

### (14-3) Coupling between Disulfide Linker and Peptide

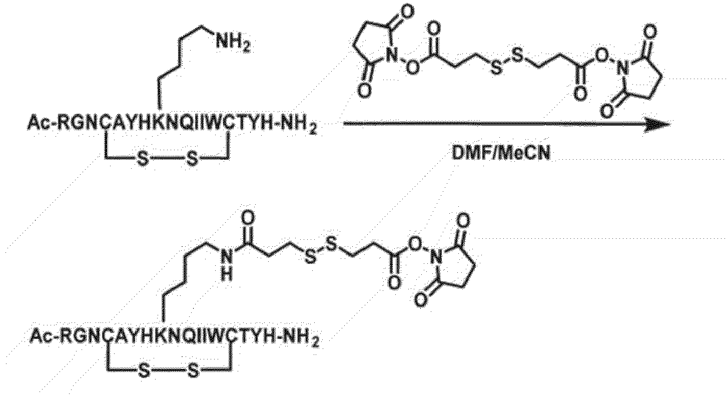

The amino acid sequence is SEQ ID NO: 17.

Ac-RGNCAYHKNQIIWCTYH-NH₂ (SEQ ID NO: 17) synthesized in (14-2) (13.0 mg, 6.06 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (49.0 mg, 121 µmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (1.30 mg, 0.53 µmol).

MS(ESI) m/z:z=3 812.85[M+3H]³⁺
HPLC purity: 100%

### (14-4) Specific Modification of Anti-HER2 IgG antibody Trastuzumab and Analysis by ESI-TOFMS

The peptide- and disulfide linker-coupled NHS-activation compound synthesized in (14-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150549, and a peak for a product with two binding peptides introduced was observed at 152869.

### (14-5) Determination of Heavy Chain Selectivity of Specifically Modified Compound of Trastuzumab under Reduction Conditions by ESI-TOFMS Analysis

To the antibody-peptide conjugate formed in (14-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (14-6) HIC-UPLC Analysis of Specific Modification of Anti-HER2 IgG antibody Trastuzumab

The antibody-peptide conjugate formed in (14-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

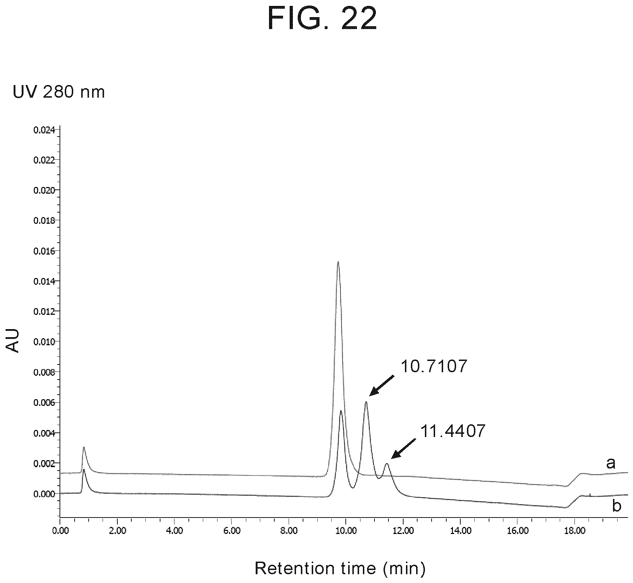

It is believed that a retension time of 9.8295 minutes is attributed to the trastuzumab raw material, whereas that of 10.7107 minutes is attributed to a compound with one peptide introduced to trastuzumab, and that of 11.4407 minutes is attributed to a compound with two peptides introduced to trastuzumab (FIG. 22).

### (14-7) Linker Cleavage and Reoxidation of Trastuzumab-Peptide Conjugate

By performing linker cleavage and reoxidation of the antibody-peptide conjugate obtained in (14-4) by the method described in (3-1) of Example 3, it is possible to obtain an antibody in which thiopropionyl groups are regioselectively introduced to Lys246 and Lys248 in EU numbering.

### [Example 15: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (15-1) Synthesis of IgG1 Fc-Binding Peptide

The peptide of Ac-RGNCAYHKDQIIWCTYH-NH₂ (SEQ ID NO: 18) was synthesized and purified in a similar method to (1-1) of Example 1 to obtain a target product (37.2 mg, 17.3 µπιοi) .

MS(ESI) m/z:z=4 538.20[M+4H]⁴⁺

### (15-2) Formation of Intra-Molecular Disulfide Bond between Cys at Positions 4 and 14 of Ac-RGNCAYHKDQIIWCTYH-NH₂ (SEQ ID NO: 18)

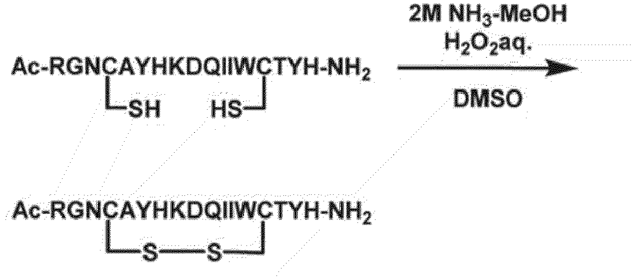

The amino acid sequence is SEQ ID NO: 18.

The peptide (37.2 mg, 17.3 µmol) synthesized in (15-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (17.2 mg, 8.01 µπιοi) .

MS(ESI) m/z:z=3 716.75[M+3H]³⁺, z=4 537.80 [M+4H]⁴⁺

### (15-3) Coupling between Disulfide Linker and Peptide

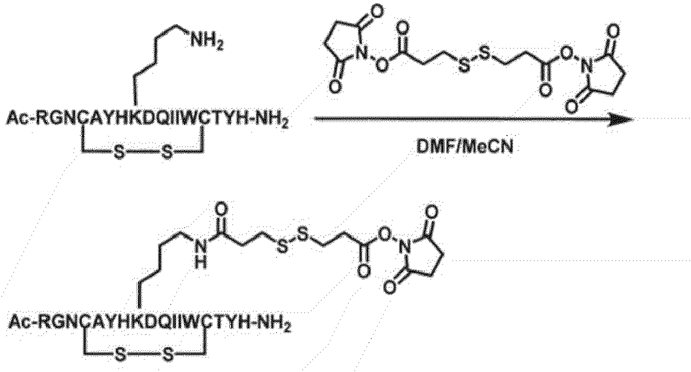

The amino acid sequence is SEQ ID NO: 18.

Ac-RGNCAYHKDQIIWCTYH-NH₂ (SEQ ID NO: 18) synthesized in (15-2) (17.2 mg, 8.01 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (65.0 mg, 160 µmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.00 mg, 1.23 µmol).

MS(ESI) m/z:z=3 813.05[M+3H]³⁺
HPLC purity: 100%

### (15-4) Specific Modification of Anti-HER2 IgG antibody Trastuzumab and Analysis by ESI-TOFMS

The peptide- and disulfide linker-coupled NHS-activation compound synthesized in (15-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152864.

### (15-5) Determination of Heavy Chain Selectivity of Specifically Modified Compound of Trastuzumab under Reduction Conditions by ESI-TOFMS Analysis

To the antibody-peptide conjugate formed in (15-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (15-6) HIC-UPLC Analysis of Specific Modification of Anti-HER2 IgG antibody Trastuzumab

The antibody-peptide conjugate formed in (15-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

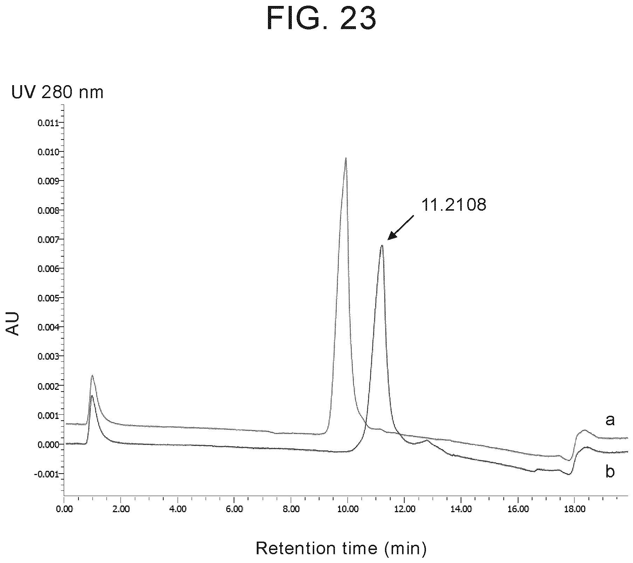

It is believed that a retension time of 11.2108 minutes is attributed to a compound with two peptides introduced to trastuzumab (FIG. 23).

### (15-7) Linker Cleavage and Reoxidation of Trastuzumab-Peptide Conjugate

By performing linker cleavage and reoxidation of the antibody-peptide conjugate obtained in (15-4) by the method described in (3-1) of Example 3, it is possible to obtain an antibody in which thiopropionyl groups are regioselectively introduced to Lys246 and Lys248 in EU numbering.

### [Example 16: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (16-1) Synthesis of IgG1 Fc-Binding Peptide

The peptide of Ac-RGNCAYHKQQIIWCTYH-NH₂ (SEQ ID NO: 19) was synthesized and purified in a similar method to (1-1) of Example 1 to obtain a target product (32.2 mg, 14.9 µmοl).

MS(ESI) m/z:z=3 541.55[M+3H]³⁺

### (16-2) Formation of Intra-Molecular Disulfide Bond between Cys at Positions 4 and 14 of Ac-RGNCAYHKQQIIWCTYH-NH₂ (SEQ ID NO: 19)

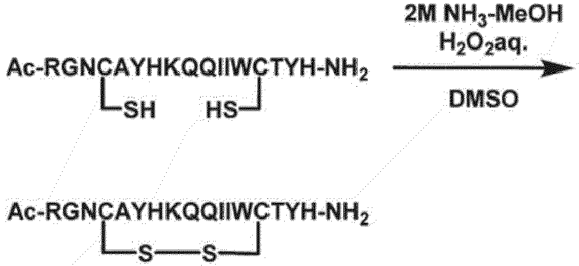

The amino acid sequence is SEQ ID NO: 19.

The peptide (32.2 mg, 14.9 µmol) synthesized in (16-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (14.3 mg, 6.62 µmοl).

MS(ESI) m/z:z=3 721.20 [M+3H]³⁺, z=4 541.00[M+4H]⁴⁺

### (16-3) Coupling between Disulfide Linker and Peptide

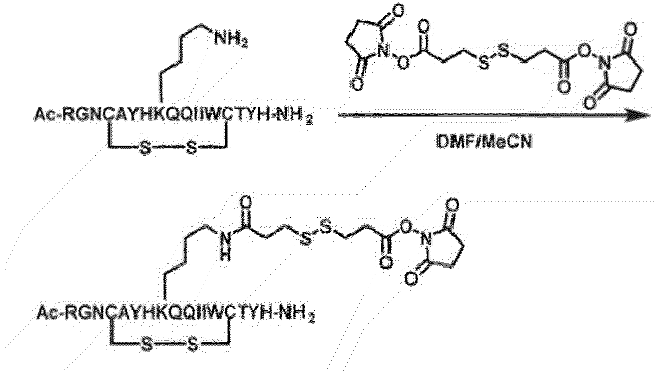

The amino acid sequence is SEQ ID NO: 19.

Ac-RGNCAYHKQQIIWCTYH-NH₂ (SEQ ID NO: 19) synthesized in (16-2) (14.3 mg, 6.62 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (53.0 mg, 132 µmol) in acetonitrile (0.30 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (4.00 mg, 1.63 µmol).

MS(ESI) m/z:z=3 817.50[M+3H]³⁺
HPLC purity: 89%

### (16-4) Specific Modification of Anti-HER2 IgG antibody Trastuzumab and Analysis by ESI-TOFMS

The peptide- and disulfide linker-coupled NHS-activation compound synthesized in (16-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150212, and a peak for a product with two binding peptides introduced was observed at 152897.

### (16-5) Determination of Heavy Chain Selectivity of Specifically Modified Compound of Trastuzumab under Reduction Conditions by ESI-TOFMS Analysis

To the antibody-peptide conjugate formed in (16-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50686 and 50844 with a thiopropionyl group introduced to the heavy chain, and a light chain peak was observed at 23439, the same as that of the raw material.

### (16-6) HIC-UPLC Analysis of Specific Modification of Anti-HER2 IgG antibody Trastuzumab

The antibody-peptide conjugate formed in (16-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

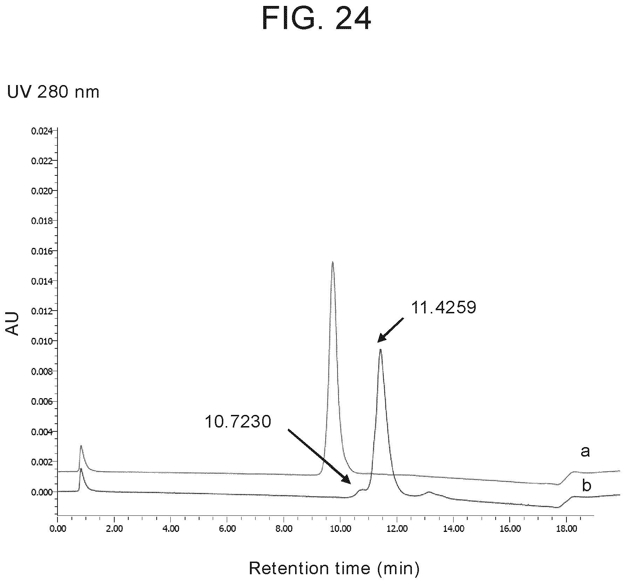

It is believed that a retension time of 10.7230 minutes is attributed to a compound with one peptide introduced to trastuzumab, whereas that of 11.4259 minutes is attributed to a compound with two peptides introduced to trastuzumab (FIG. 24).

### (16-7) Linker Cleavage and Reoxidation of Trastuzumab-Peptide Conjugate

By performing linker cleavage and reoxidation of the antibody-peptide conjugate obtained in (16-4) by the method described in (3-1) of Example 3, it is possible to obtain an antibody in which thiopropionyl groups are regioselectively introduced to Lys246 and Lys248 in EU numbering.

### [Example 17: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (17-1) Synthesis of IgG1 Fc-Binding Peptide

The peptide of Ac-RGNCAYHKEQIIWCTYH-NH₂ (SEQ ID NO: 20) was synthesized and purified in a similar method to (1-1) of Example 1 to obtain a target product (38.0 mg, 17.6 µmοl).

MS(ESI) m/z:z=3 722.00[M+3H]³⁺, z=4 541.75[M+4H]⁴⁺

### (17-2) Formation of Intra-Molecular Disulfide Bond between Cys at Positions 4 and 14 of Ac-RGNCAYHKEQIIWCTYH-NH₂ (SEQ ID NO: 20)

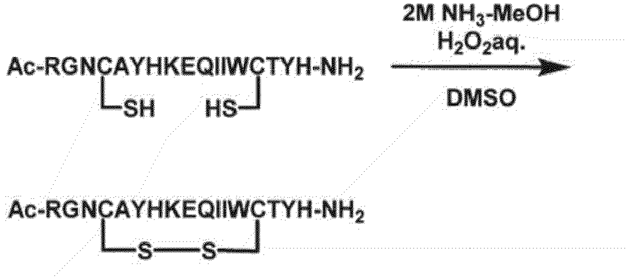

The amino acid sequence is SEQ ID NO: 20.

The peptide (38.0 mg, 17.6 µmol) synthesized in (17-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (16.0 mg, 7.40 µmοl).

MS(ESI) m/z:z=3 721.40[M+3H]³⁺, z=4 541.25[M+4H]⁴⁺

### (17-3) Coupling between Disulfide Linker and Peptide

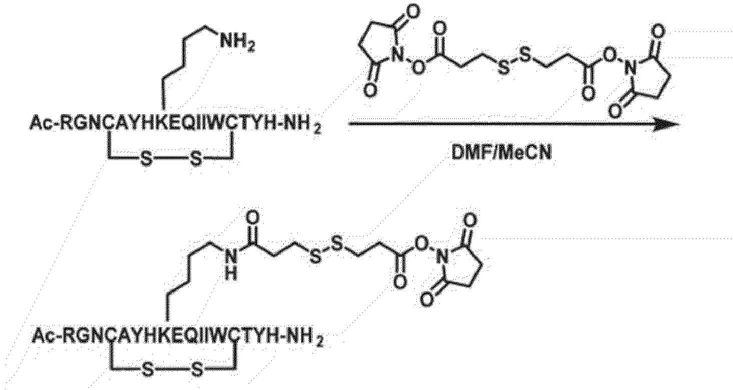

The amino acid sequence is SEQ ID NO: 20.

Ac-RGNCAYHKEQIIWCTYH-NH₂ (SEQ ID NO: 20) synthesized in (17-2) (16.0 mg, 7.40 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (60.0 mg, 148 µmol) in acetonitrile (0.30 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (4.50 mg, 1.84 µmol).

MS(ESI) m/z:z=3 817.80[M+3H]³⁺
HPLC purity: 100%

### (17-4) Specific Modification of Anti-HER2 IgG antibody Trastuzumab and Analysis by ESI-TOFMS

The peptide- and disulfide linker-coupled NHS-activation compound synthesized in (17-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152900.

### (17-5) Determination of Heavy Chain Selectivity of Specifically Modified Compound of Trastuzumab under Reduction Conditions by ESI-TOFMS Analysis

To the antibody-peptide conjugate formed in (17-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50686 and 50844 with a thiopropionyl group introduced to the heavy chain, and a light chain peak was observed at 23439, the same as that of the raw material.

### (17-6) HIC-UPLC Analysis of Specific Modification of Anti-HER2 IgG antibody Trastuzumab

The antibody-peptide conjugate formed in (17-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

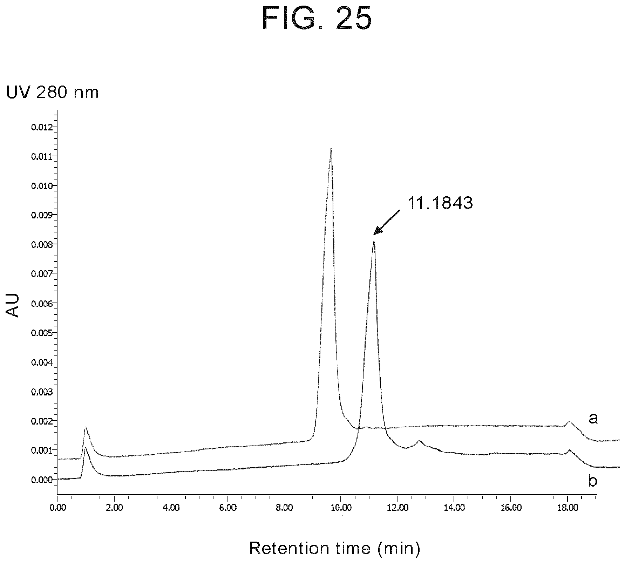

It is believed that a retension time of 11.1843 minutes is attributed to a compound with two peptides introduced to trastuzumab (FIG. 25).

### (17-7) Linker Cleavage and Reoxidation of Trastuzumab-Peptide Conjugate

By performing linker cleavage and reoxidation of the antibody-peptide conjugate obtained in (17-4) by the method described in (3-1) of Example 3, it is possible to obtain an antibody in which thiopropionyl groups are regioselectively introduced to Lys246 and Lys248 in EU numbering.

### [Example 18: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (18-1) Synthesis of IgG1 Fc-Binding Peptide

The peptide of Ac-RGNCAYHKFQIIWCTYH-NH₂ (SEQ ID NO: 21) was synthesized and purified in a similar method to (1-1) of Example 1 to obtain a target product (23.0 mg, 10.5 µmol).

MS(ESI) m/z:z=3 728.05[M+3H]³⁺, z=4 546.40[M+4H]⁴⁺

### (18-2) Formation of Intra-Molecular Disulfide Bond between Cys at Positions 4 and 14 of Ac-RGNCAYHKFQIIWCTYH-NH₂ (SEQ ID NO: 21)

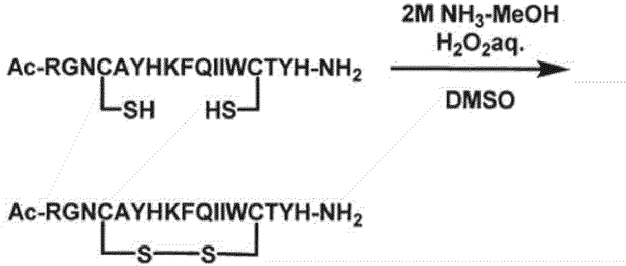

The amino acid sequence is SEQ ID NO: 21.

The peptide (23.0 mg, 10.5 µmol) synthesized in (18-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (9.80 mg, 4.50 µmοl).

MS(ESI) m/z:z=3 727.35[M+3H]³⁺, z=4 545.85[M+4H]⁴⁺

### (18-3) Coupling between Disulfide Linker and Peptide

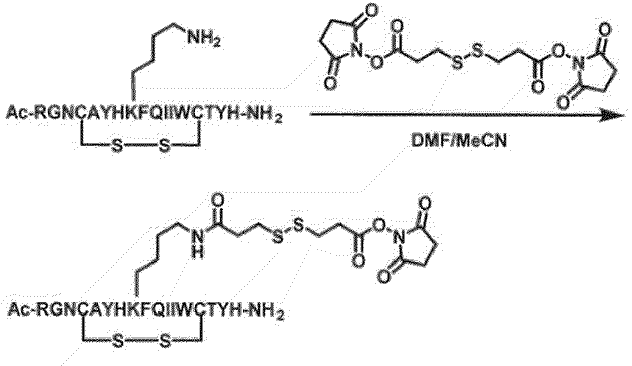

The amino acid sequence is SEQ ID NO: 21.

Ac-RGNCAYHKFQIIWCTYH-NH₂ (SEQ ID NO: 21) synthesized in (18-2) (9.80 mg, 4.50 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (36.0 mg, 90.0 µmol) in acetonitrile (0.30 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.50 mg, 1.01 µmοl).

MS(ESI) m/z:z=3 823.85[M+3H]³⁺
HPLC purity: 93%

### (18-4) Specific Modification of Anti-HER2 IgG antibody Trastuzumab and Analysis by ESI-TOFMS

The peptide- and disulfide linker-coupled NHS-activation compound synthesized in (18-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 153317.

### (18-5) Determination of Heavy Chain Selectivity of Specifically Modified Compound of Trastuzumab under Reduction Conditions by ESI-TOFMS Analysis

To the antibody-peptide conjugate formed in (18-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50686 and 50844 with a thiopropionyl group introduced to the heavy chain, and a light chain peak was observed at 23439, the same as that of the raw material.

### (18-6) HIC-UPLC Analysis of Specific Modification of Anti-HER2 IgG antibody Trastuzumab

The antibody-peptide conjugate formed in (18-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

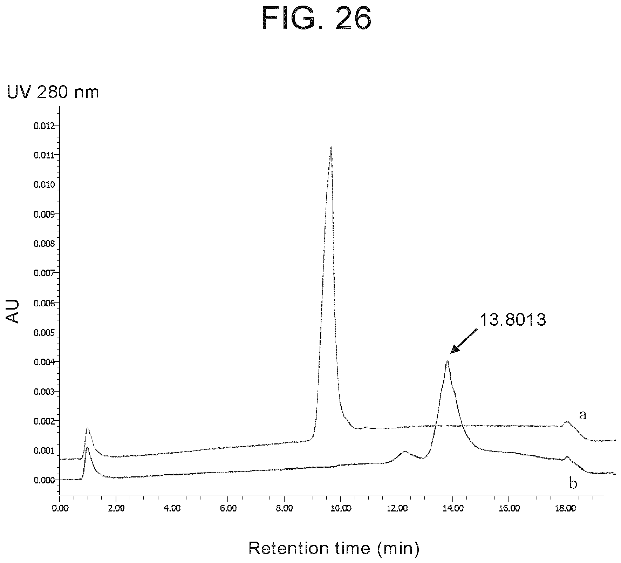

It is believed that a retension time of 13.8013 minutes is attributed to a compound with two peptides introduced to trastuzumab (FIG. 26).

### Linker Cleavage and Reoxidation of Trastuzumab-Peptide Conjugate

By performing linker cleavage and reoxidation of the antibody-peptide conjugate obtained in (18-4) by the method described in (3-1) of Example 3, it is possible to obtain an antibody in which thiopropionyl groups are regioselectively introduced to Lys246 and Lys248 in EU numbering.

### [Example 19: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (19-1) Synthesis of IgG1 Fc-Binding Peptide

The peptide of Ac-RGNCAYHKYQIIWCTYH-NH₂ (SEQ ID NO: 22) was synthesized and purified in a similar method to (1-1) of Example 1 to obtain a target product (30.8 mg, 14.0 µmοl).

MS(ESI) m/z:z=3 733.20[M+3H]³⁺, z=4 550.40[M+4H]⁴⁺

### (19-2) Formation of Intra-Molecular Disulfide Bond between Cys at Positions 4 and 14 of Ac-RGNCAYHKYQIIWCTYH-NH₂ (SEQ ID NO: 22)

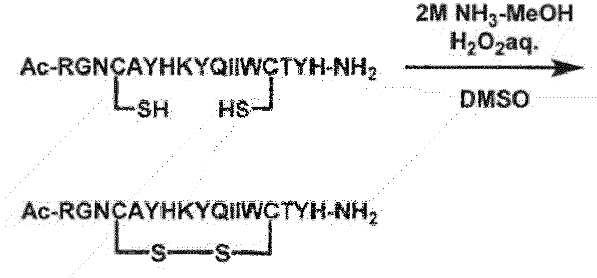

The amino acid sequence is SEQ ID NO: 22.

The peptide (30.8 mg, 14.0 µmol) synthesized in (19-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (17.5 mg, 7.97 µmοl).

MS(ESI) m/z:z=3 732.75[M+3H]³⁺, z=4 549.90[M+4H]⁴⁺

### (19-3) Coupling between Disulfide Linker and Peptide

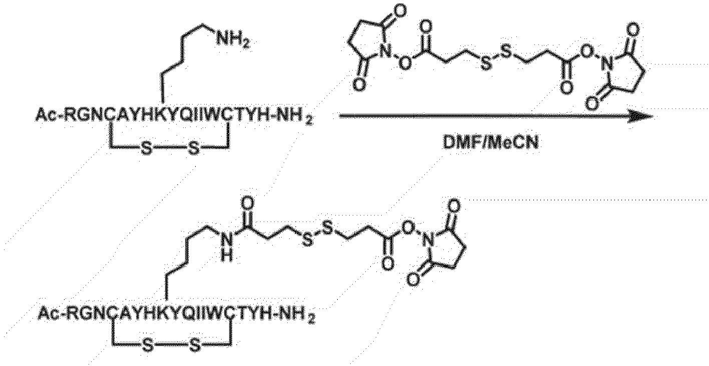

The amino acid sequence is SEQ ID NO: 22.

Ac-RGNCAYHKYQIIWCTYH-NH₂ (SEQ ID NO: 22) synthesized in (19-2) (17.5 mg, 7.97 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (97.0 mg, 239 µmol) in acetonitrile (0.30 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.50 mg, 1.41 µmοl).

MS(ESI) m/z:z=3 829.15[M+3H]³⁺
HPLC purity: 89%

### (19-4) Specific Modification of Anti-HER2 IgG antibody Trastuzumab and Analysis by ESI-TOFMS

The peptide- and disulfide linker-coupled NHS-activation compound synthesized in (19-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152965.

### (19-5) Determination of Heavy Chain Selectivity of Specifically Modified Compound of Trastuzumab under Reduction Conditions by ESI-TOFMS Analysis

To the antibody-peptide conjugate formed in (19-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50686 and 50844 with a thiopropionyl group introduced to the heavy chain, and a light chain peak was observed at 23439, the same as that of the raw material.

### (19-6) HIC-UPLC Analysis of Specific Modification of Anti-HER2 IgG antibody Trastuzumab

The antibody-peptide conjugate formed in (19-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

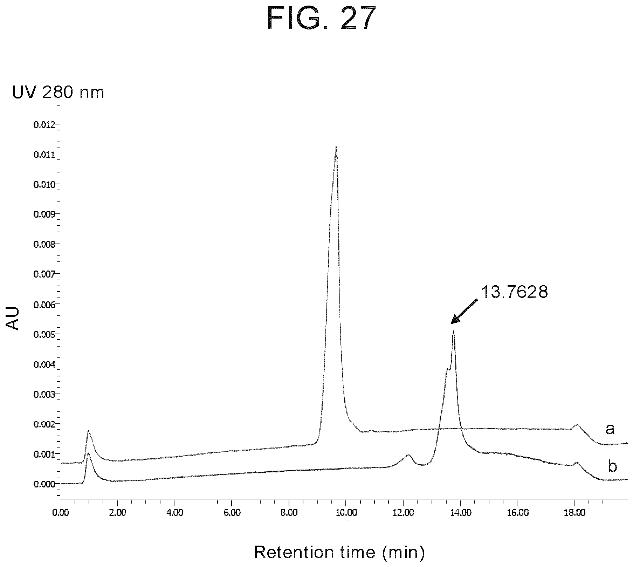

It is believed that a retension time of 13.7628 minutes is attributed to a compound with two peptides introduced to trastuzumab (FIG. 27).

### (19-7) Linker Cleavage and Reoxidation of Trastuzumab-Peptide Conjugate

By performing linker cleavage and reoxidation of the antibody-peptide conjugate obtained in (19-4) by the method described in (3-1) of Example 3, it is possible to obtain an antibody in which thiopropionyl groups are regioselectively introduced to Lys246 and Lys248 in EU numbering.

### [Example 20: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (20-1) Synthesis of IgG1 Fc-Binding Peptide

The peptide of Ac-RGNCAYHKWQIIWCTYH-NH₂ (SEQ ID NO: 23) was synthesized and purified in a similar method to (1-1) of Example 1 to obtain a target product (23.0 mg, 10.4 µmοl).

MS(ESI) m/z:z=3 741.15[M+3H]³⁺, z=4 556.00[M+4H]⁴⁺

### (20-2) Formation of Intra-Molecular Disulfide Bond between Cys at Positions 4 and 14 of Ac-RGNCAYHKWQIIWCTYH-NH₂ (SEQ ID NO: 23)

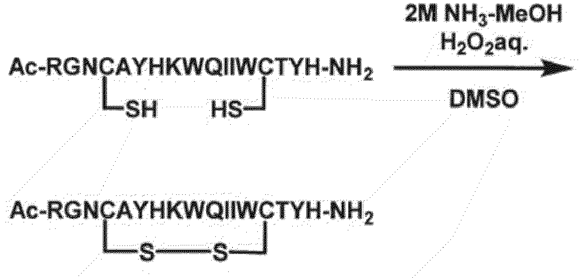

The amino acid sequence is SEQ ID NO: 23.

The peptide (23.0 mg, 10.4 µmol) synthesized in (20-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (8.20 mg, 3.70 µmοl).

MS(ESI) m/z:z=3 740.40[M+3H]³⁺, z=4 555.65[M+4H]⁴⁺

### (20-3) Coupling between Disulfide Linker and Peptide

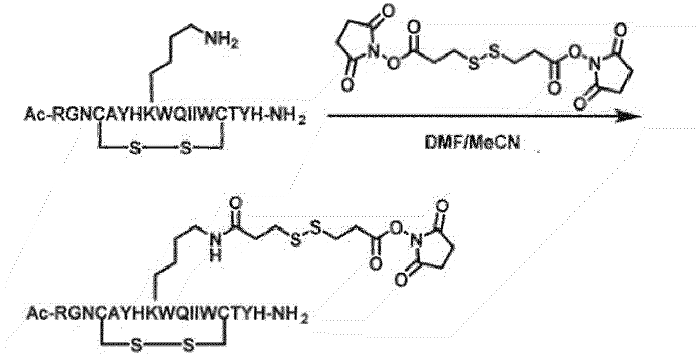

The amino acid sequence is SEQ ID NO: 23.

Ac-RGNCAYHKWQIIWCTYH-NH₂ (SEQ ID NO: 23) synthesized in (20-2) (8.20 mg, 3.70 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (60.0 mg, 148 µmol) in acetonitrile (0.30 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.2 mg, 0.88 µmol).

MS(ESI) m/z:z=3 836.80[M+3H]³⁺
HPLC purity: 93%

### (20-4) Specific Modification of Anti-HER2 IgG antibody Trastuzumab and Analysis by ESI-TOFMS

The peptide- and disulfide linker-coupled NHS-activation compound synthesized in (20-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 153121.

### (20-5) Determination of Heavy Chain Selectivity of Specifically Modified Compound of Trastuzumab under Reduction Conditions by ESI-TOFMS Analysis

To the antibody-peptide conjugate formed in (20-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23,440, whereas for a product, peaks were observed at 50682 and 50844 with a thiopropionyl group introduced to the heavy chain, and a light chain peak was observed at 23439, the same as that of the raw material.

### (20-6) HIC-UPLC Analysis of Specific Modification of Anti-HER2 IgG antibody Trastuzumab

The antibody-peptide conjugate formed in (20-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

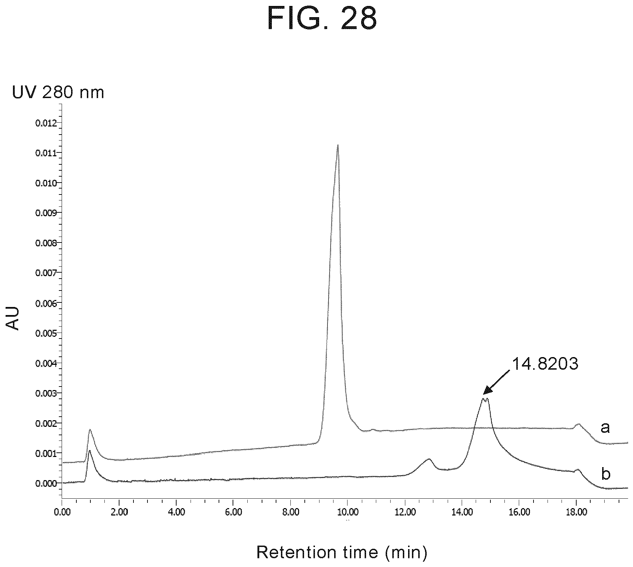

It is believed that a retension time of 14.8203 minutes is attributed to a compound with two peptides introduced to trastuzumab (FIG. 28).

### (20-7) Linker Cleavage and Reoxidation of Trastuzumab-Peptide Conjugate

By performing linker cleavage and reoxidation of the antibody-peptide conjugate obtained in (20-4) by the method described in (3-1) of Example 3, it is possible to obtain an antibody in which thiopropionyl groups are regioselectively introduced to Lys246 and Lys248 in EU numbering.

### [Example 21: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (21-1) Synthesis of IgG1 Fc-Binding Peptide

The peptide of Ac-RGNCAYHKHQIIWCTYH-NH₂ (SEQ ID NO: 24) was synthesized and purified in a similar method to (1-1) of Example 1 to obtain a target product (68.7 mg, 31.6 µmοl).

MS(ESI) m/z:z=3 724.70[M+3H]³⁺, z=4 543.85[M+4H]⁴⁺

### (21-2) Formation of Intra-Molecular Disulfide Bond between Cys at Positions 4 and 14 of Ac-RGNCAYHKHQIIWCTYH-NH₂ (SEQ ID NO: 24)

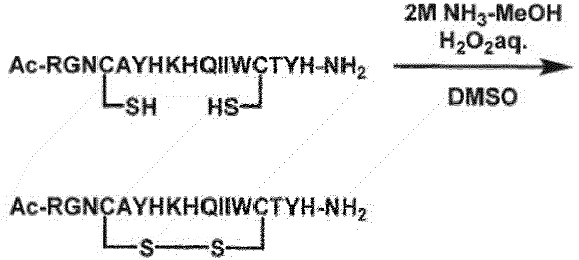

The amino acid sequence is SEQ ID NO: 24.

The peptide (68.7 mg, 31.6 µmol) synthesized in (21-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (44.9 mg, 20.7 µmol).

MS(ESI) m/z:z=4 543.55[M+4H]⁴⁺

### (21-3) Coupling between Disulfide Linker and Peptide

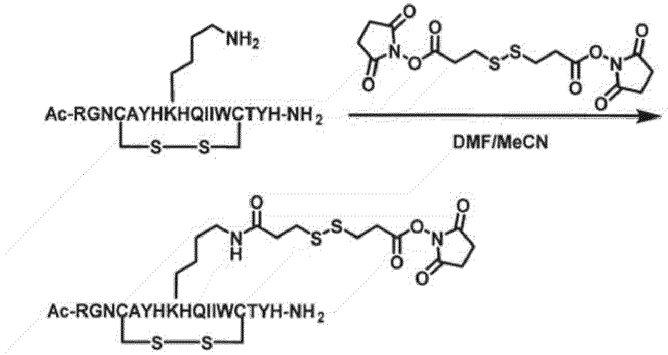

The amino acid sequence is SEQ ID NO: 24.

Ac-RGNCAYHKHQIIWCTYH-NH₂ (SEQ ID NO: 24) synthesized in (21-2) (44.9 mg, 20.7 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (60.0 mg, 148 µmol) in acetonitrile (0.60 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.30 mg, 1.34 µmol) .

MS(ESI) m/z:z=4 615.55[M+3H]⁴⁺
HPLC purity: 68%

### (21-4) Specific Modification of Anti-HER2 IgG antibody Trastuzumab and Analysis by ESI-TOFMS

The peptide- and disulfide linker-coupled NHS-activation compound synthesized in (21-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152909.

### (21-5) Determination of Heavy Chain Selectivity of Specifically Modified Compound of Trastuzumab under Reduction Conditions by ESI-TOFMS Analysis

To the antibody-peptide conjugate formed in (21-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50684 and 50844 with a thiopropionyl group introduced to the heavy chain, and a light chain peak was observed at 23439, the same as that of the raw material.

### (21-6) HIC-UPLC Analysis of Specific Modification of Anti-HER2 IgG antibody Trastuzumab

The antibody-peptide conjugate formed in (21-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

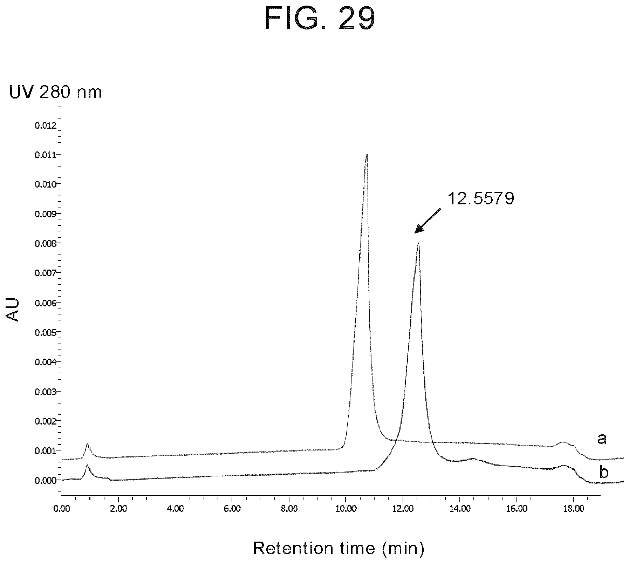

It is believed that a retension time of 12.5579 minutes is attributed to a compound with two peptides introduced to trastuzumab (FIG. 29).

### (21-7) Linker Cleavage and Reoxidation of Trastuzumab-Peptide Conjugate

By performing linker cleavage and reoxidation of the antibody-peptide conjugate obtained in (21-4) by the method described in (3-1) of Example 3, it is possible to obtain an antibody in which thiopropionyl groups are regioselectively introduced to Lys246 and Lys248 in EU numbering.

### [Example 22: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (22-1) Synthesis of IgG1 Fc-Binding Peptide

The peptide of Ac-RGNCAYHKTQIIWCTYH-NH₂ (SEQ ID NO: 25) was synthesized and purified in a similar method to (1-1) of Example 1 to obtain a target product (46.5 mg, 21.8 µmοl).

MS(ESI) m/z:z=3 712.80[M+3H]³⁺, z=4 534.85[M+4H]⁴⁺

### (22-2) Formation of Intra-Molecular Disulfide Bond between Cys at Positions 4 and 14 of Ac-RGNCAYHKTQIIWCTYH-NH₂ (SEQ ID NO: 25)

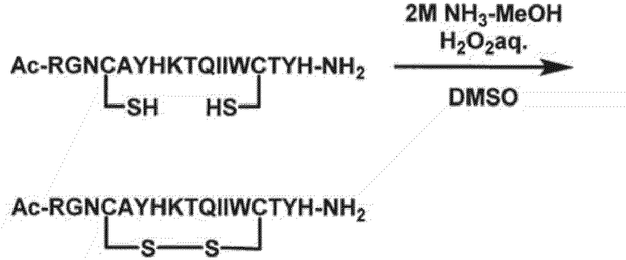

The amino acid sequence is SEQ ID NO: 25.

The peptide (46.5 mg, 21.8 µmol) synthesized in (22-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (31.6 mg, 14.8 µmol).

MS(ESI) m/z:z=3 711.95[M+3H]³⁺, z=4 534.30[M+4H]⁴⁺

### (22-3) Coupling between Disulfide Linker and Peptide

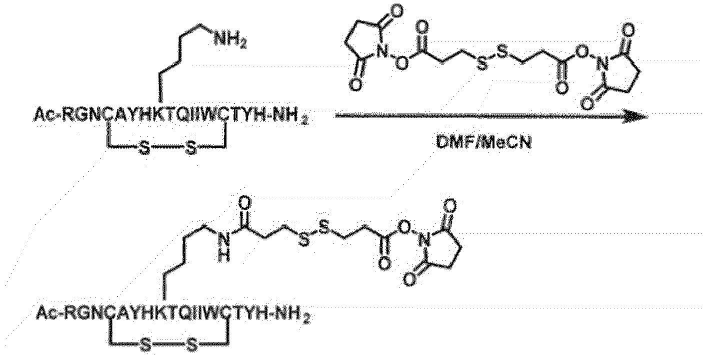

The amino acid sequence is SEQ ID NO: 25.

Ac-RGNCAYHKTQIIWCTYH-NH₂ (SEQ ID NO: 25) synthesized in (22-2) (31.6 mg, 14.8 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (120 mg, 296 µmol) in acetonitrile (0.60 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.50 mg, 2.27 µmol).

MS(ESI) m/z:z=3 808.45[M+3H]³⁺
HPLC purity: 89%

### (22-4) Specific Modification of Anti-HER2 IgG antibody Trastuzumab and Analysis by ESI-TOFMS

The peptide- and disulfide linker-coupled NHS-activation compound synthesized in (22-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152837.

### (22-5) Determination of Heavy Chain Selectivity of Specifically Modified Compound of Trastuzumab under Reduction Conditions by ESI-TOFMS Analysis

To the antibody-peptide conjugate formed in (19-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50686 and 50844 with a thiopropionyl group introduced to the heavy chain, and a light chain peak was observed at 23439, the same as that of the raw material.

### (22-6) HIC-UPLC Analysis of Specific Modification of Anti-HER2 IgG antibody Trastuzumab

The antibody-peptide conjugate formed in (22-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

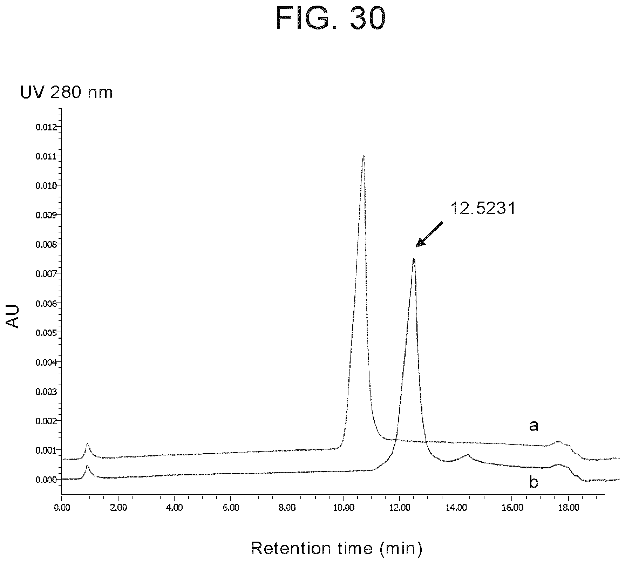

It is believed that a retension time of 12.5231 minutes is attributed to a compound with two peptides introduced to trastuzumab (FIG. 30).

### (22-7) Linker Cleavage and Reoxidation of Trastuzumab-Peptide Conjugate

By performing linker cleavage and reoxidation of the antibody-peptide conjugate obtained in (22-4) by the method described in (3-1) of Example 3, it is possible to obtain an antibody in which thiopropionyl groups are regioselectively introduced to Lys246 and Lys248 in EU numbering.

### [Example 23: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (23-1) Synthesis of IgG1 Fc-Binding Peptide

The peptide of Ac-RGNCAYHKLQIIWCTYH-NH₂ (SEQ ID NO: 26) was synthesized and purified in a similar method to (1-1) of Example 1 to obtain a target product (32.2 mg, 15.0 µmol).

MS(ESI) m/z:z=3 716.70[M+3H]³⁺, z=4 537.85[M+4H]⁴⁺

### (23-2) Formation of Intra-Molecular Disulfide Bond between Cys at Positions 4 and 14 of Ac-RGNCAYHKLQIIWCTYH-NH₂ (SEQ ID NO: 26)

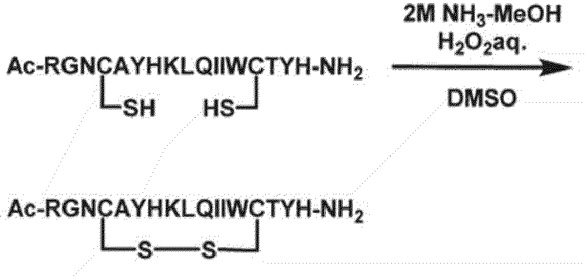

The amino acid sequence is SEQ ID NO: 26.

The peptide (32.2 mg, 15.0 µmol) synthesized in (23-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (9.40 mg, 4.38 µmol).

MS(ESI) m/z:z=3 716.10[M+3H]³⁺, z=4 537.35[M+4H]⁴⁺

### (23-3) Coupling between Disulfide Linker and Peptide

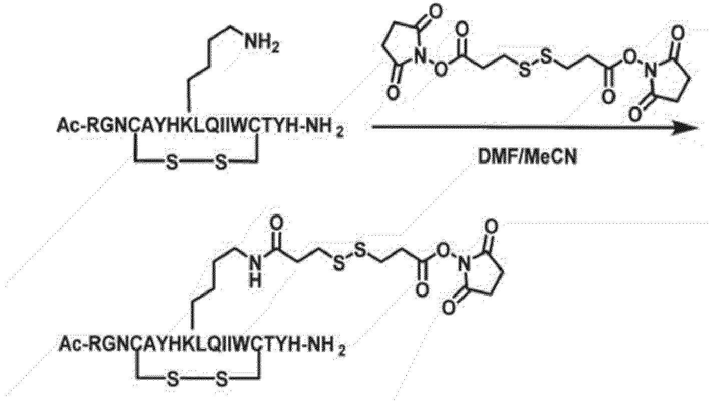

The amino acid sequence is SEQ ID NO: 26.

Ac-RGNCAYHKLQIIWCTYH-NH₂ (SEQ ID NO: 26) synthesized in (23-2) (9.40 mg, 4.38 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (70.7 mg, 175 µmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.80 mg, 1.15 µmol) .

MS(ESI) m/z:z=3 812.40[M+3H]³⁺
HPLC purity: 80%

### (23-4) Specific Modification of Anti-HER2 IgG antibody Trastuzumab and Analysis by ESI-TOFMS

The peptide- and disulfide linker-coupled NHS-activation compound synthesized in (23-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 153053.

### (23-5) Determination of Heavy Chain Selectivity of Specifically Modified Compound of Trastuzumab under Reduction Conditions by ESI-TOFMS Analysis

To the antibody-peptide conjugate formed in (23-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (23-6) HIC-UPLC Analysis of Specific Modification of Anti-HER2 IgG antibody Trastuzumab

The antibody-peptide conjugate formed in (23-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

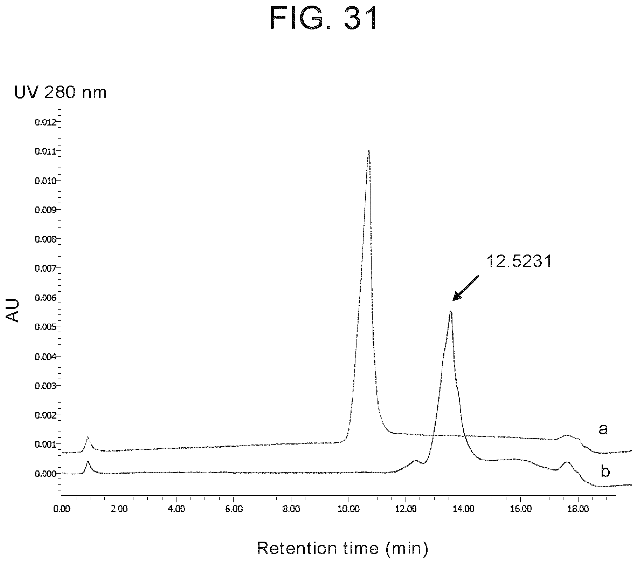

It is believed that a retension time of 13.5717 minutes is attributed to a compound with two peptides introduced to trastuzumab (FIG. 31).

### (23-7) Linker Cleavage and Reoxidation of Trastuzumab-Peptide Conjugate

By performing linker cleavage and reoxidation of the antibody-peptide conjugate obtained in (23-4) by the method described in (3-1) of Example 3, it is possible to obtain an antibody in which thiopropionyl groups are regioselectively introduced to Lys246 and Lys248 in EU numbering.

### [Example 24: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (24-1) Synthesis of IgG1 Fc-Binding Peptide

The peptide of Ac-CAYHKLQIVWC-NH₂ (SEQ ID NO: 27) was synthesized and purified in a similar method to (1-1) of Example 1 to obtain a target product (65.3 mg, 48.4 µmol).

MS(ESI) m/z:z=2 675.20[M+2H]²⁺

### (24-2) Formation of Intra-Molecular Disulfide Bond between Cys at Positions 1 and 11 of Ac-CAYHKLQIVWC-NH₂ (SEQ ID NO: 27)

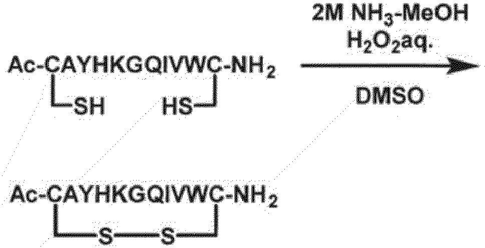

The amino acid sequence is SEQ ID NO: 27.

The peptide (65.3 mg, 48.4 µmol) synthesized in (24-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (36.8 mg, 27.3 µmol).

MS(ESI) m/z:z=2 674.35[M+2H]²⁺

### (24-3) Coupling between Disulfide Linker and Peptide

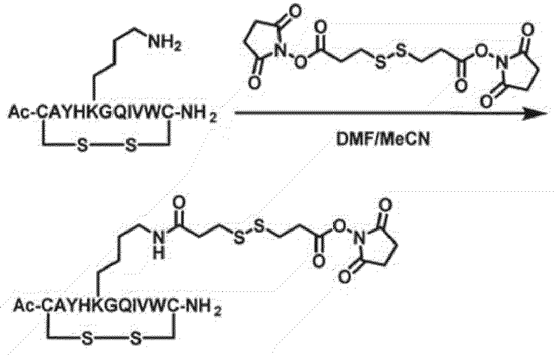

The amino acid sequence is SEQ ID NO: 27.

Ac-CAYHKLQIVWC-NH₂ (SEQ ID NO: 27) synthesized in (24-2) (36.8 mg, 27.3 µmol, the 1st and 11th of two cysteines form a disulfide bond in the molecule) was dissolved in N,N'-dimethylformamide (0.40 mL), a solution dissolving 3,3'-dithiodipropionic acid di(N-succinimidyl) (442 mg, 1.09 mmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (24.5 mg, 15.0 µmol).

MS(ESI) m/z:z=2 818.85[M+2H]²⁺
HPLC purity: 85%

### (24-4) Specific Modification of Anti-HER2 IgG antibody Trastuzumab and Analysis by ESI-TOFMS

The peptide- and disulfide linker-coupled NHS-activation compound synthesized in (24-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149590, and a peak for a product with two binding peptides introduced was observed at 151270.

### (24-5) Determination of Heavy Chain Selectivity of Specifically Modified Compound of Trastuzumab under Reduction Conditions by ESI-TOFMS Analysis

To the antibody-peptide conjugate formed in (24-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (24-6) HIC-UPLC Analysis of Specific Modification of Anti-HER2 IgG antibody Trastuzumab

The antibody-peptide conjugate formed in (24-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

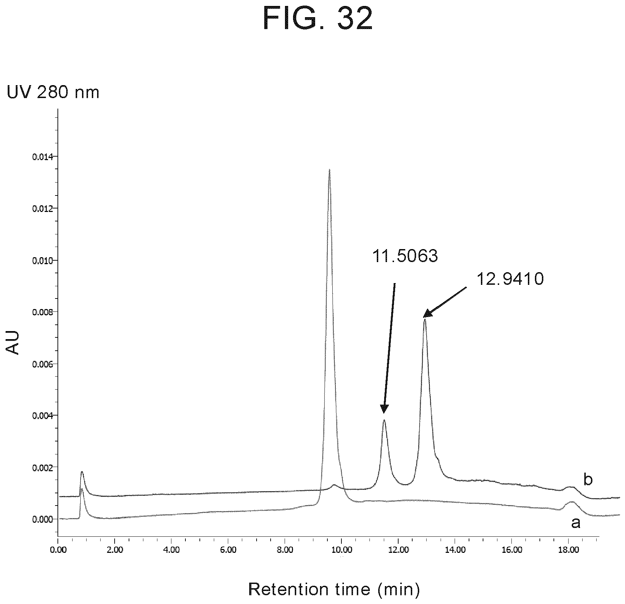

It is believed that a retension time of 11.5053 minutes is attributed to a compound with one peptide introduced to trastuzumab, whereas that of 12.9410 minutes is attributed to a compound with two peptides introduced to trastuzumab (FIG. 32).

### (24-7) Linker Cleavage and Reoxidation of Trastuzumab-Peptide Conjugate

By performing linker cleavage and reoxidation of the antibody-peptide conjugate obtained in (24-4) by the method described in (3-1) of Example 3, it is possible to obtain an antibody in which thiopropionyl groups are regioselectively introduced to Lys246 and Lys248 in EU numbering.

### [Example 25: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (25-1) Synthesis of IgG1 Fc-Binding Peptide

The peptide of Ac-CAYHKLQLIWC-NH₂ (SEQ ID NO: 28) was synthesized and purified in a similar method to (1-1) of Example 1 to obtain a target product (54.3 mg, 39.8 µmol).

MS(ESI) m/z:z=2 682.25[M+2H]²⁺

### (25-2) Formation of Intra-Molecular Disulfide Bond between Cys at Positions 1 and 11 of Ac-CAYHKLQLIWC-NH₂ (SEQ ID NO: 28)

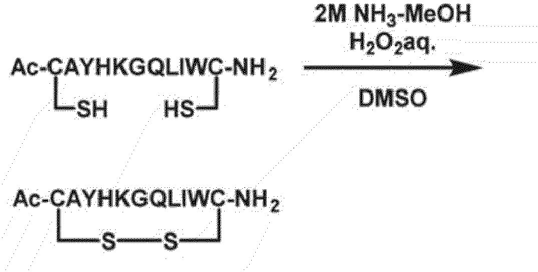

The amino acid sequence is SEQ ID NO: 28.

The peptide (54.3 mg, 39.8 µmol) synthesized in (25-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (34.6 mg, 25.4 µmol).

MS(ESI) m/z:z=2 681.20[M+2H]²⁺

### (25-3) Coupling between Disulfide Linker and Peptide

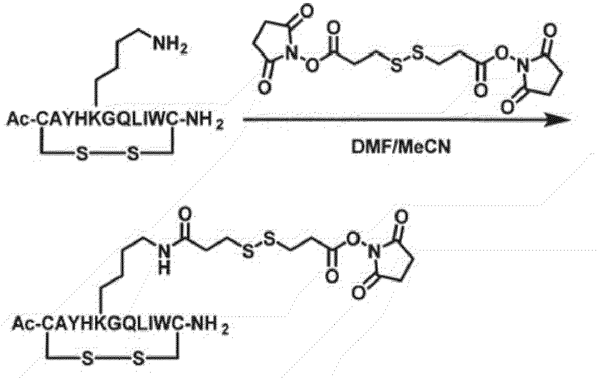

The amino acid sequence is SEQ ID NO: 28.

Ac-CAYHKLQLIWC-NH₂ (SEQ ID NO: 28) synthesized in (25-2) (34.6 mg, 25.4 µmol, the 1st and 11th of two cysteines form a disulfide bond in the molecule) was dissolved in N,N'-dimethylformamide (0.40 mL), a solution dissolving 3,3'-dithiodipropionic acid di(N-succinimidyl) (411 mg, 1.02 mmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (22.0 mg, 13.3 µmol) .

MS(ESI) m/z:z=2 835.85[M+2H]²⁺
HPLC purity: 88%

### (25-4) Specific Modification of Anti-HER2 IgG antibody Trastuzumab and Analysis by ESI-TOFMS

The peptide- and disulfide linker-coupled NHS-activation compound synthesized in (25-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149756, and a peak for a product with two binding peptides introduced was observed at 151131.

### (25-5) Determination of Heavy Chain Selectivity of Specifically Modified Compound of Trastuzumab under Reduction Conditions by ESI-TOFMS Analysis

To the antibody-peptide conjugate formed in (25-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (25-6) HIC-UPLC Analysis of Specific Modification of Anti-HER2 IgG antibody Trastuzumab

The antibody-peptide conjugate formed in (25-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

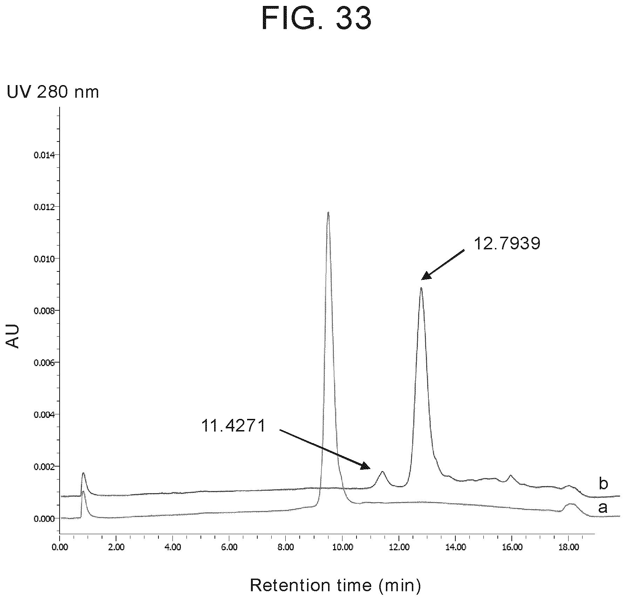

It is believed that a retension time of 11.4271 minutes is attributed to a compound with one peptide introduced to trastuzumab, whereas that of 12.7939 minutes is attributed to a compound with two peptides introduced to trastuzumab (FIG. 33).

### (25-7) Linker Cleavage and Reoxidation of Trastuzumab-Peptide Conjugate

By performing linker cleavage and reoxidation of the antibody-peptide conjugate obtained in (25-4) by the method described in (3-1) of Example 3, it is possible to obtain an antibody in which thiopropionyl groups are regioselectively introduced to Lys246 and Lys248 in EU numbering.

### [Example 26: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (26-1) Synthesis of IgG1 Fc-Binding Peptide

The peptide of Ac-CAYHKSQIVWC-NH₂ (SEQ ID NO: 29) was synthesized and purified in a similar method to (1-1) of Example 1 to obtain a target product (53.3 mg, 38.7 µmol).

MS(ESI) m/z:z=2 690.05[M+2H]²⁺

### (26-2) Formation of Intra-Molecular Disulfide Bond between Cys at Positions 1 and 11 of Ac-CAYHKSQIVWC-NH₂ (SEQ ID NO: 29)

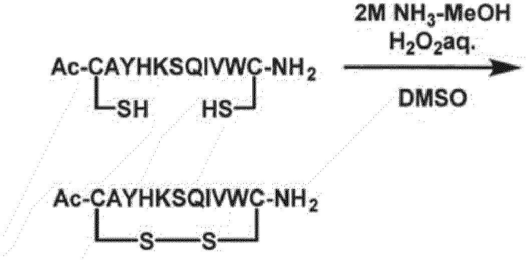

The amino acid sequence is SEQ ID NO: 29.

The peptide (53.3 mg, 38.7 µmol) synthesized in (26-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (22.8 mg, 16.6 µmol).

MS(ESI) m/z:z=2 689.20[M+2H]²⁺

### (26-3) Coupling between Disulfide Linker and Peptide

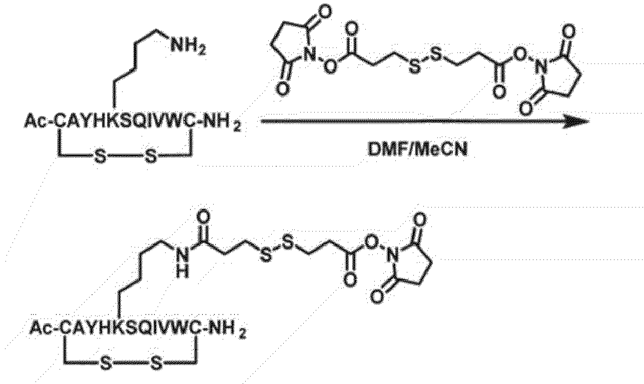

The amino acid sequence is SEQ ID NO: 29.

Ac-CAYHKSQIVWC-NH₂ (SEQ ID NO: 29) synthesized in (26-2) (22.8 mg, 16.6 µmol, the 1st and 11th of two cysteines form a disulfide bond in the molecule) was dissolved in N,N'-dimethylformamide (0.40 mL), a solution dissolving 3,3'-dithiodipropionic acid di(N-succinimidyl) (134 mg, 332 µmol) in acetonitrile (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (8.50 mg, 5.10 µmol).

MS(ESI) m/z:z=2 833.75[M+2H]²⁺
HPLC purity: 92%

### (26-4) Specific Modification of Anti-HER2 IgG antibody Trastuzumab and Analysis by ESI-TOFMS

The peptide- and disulfide linker-coupled NHS-activation compound synthesized in (26-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149772, and a peak for a product with two binding peptides introduced was observed at 151323.

### (26-5) Determination of Heavy Chain Selectivity of Specifically Modified Compound of Trastuzumab under Reduction Conditions by ESI-TOFMS Analysis

To the antibody-peptide conjugate formed in (26-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (26-6) HIC-UPLC Analysis of Specific Modification of Anti-HER2 IgG antibody Trastuzumab

The antibody-peptide conjugate formed in (26-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

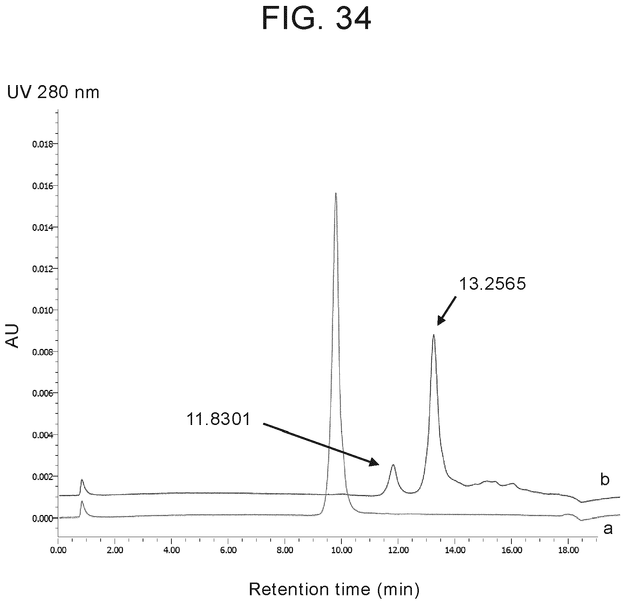

It is believed that a retension time of 11.8301 minutes is attributed to a compound with one peptide introduced to trastuzumab, whereas that of 13.2565 minutes is attributed to a compound with two peptides introduced to trastuzumab (FIG. 34).

### (26-7) Linker Cleavage and Reoxidation of Trastuzumab-Peptide Conjugate

By performing linker cleavage and reoxidation of the antibody-peptide conjugate obtained in (26-4) by the method described in (3-1) of Example 3, it is possible to obtain an antibody in which thiopropionyl groups are regioselectively introduced to Lys246 and Lys248 in EU numbering.

### [Example 27: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (27-1) Synthesis of IgG1 Fc-Binding Peptide

The peptide of Ac-RGNCAYHKGQLVFCTYH-NH₂ (SEQ ID NO: 30) was synthesized and purified in a similar method to (1-1) of Example 1 to obtain a target product (168 mg, 82.4 µmol).

MS(ESI) m/z:z=3 680.30[M+3H]³⁺, z=4 510.55[M+4H]⁴⁺

### (27-2) Formation of Intra-Molecular Disulfide Bond between Cys at Positions 4 and 14 of Ac-RGNCAYHKGQLVFCTYH-NH₂ (SEQ ID NO: 30)

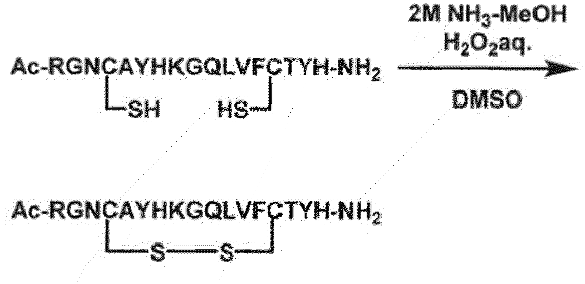

The amino acid sequence is SEQ ID NO: 30.

The peptide (168 mg, 82.4 µmol) synthesized in (27-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (22.1 mg, 10.9 µmol).

MS(ESI) m/z:z=3 679.55[M+3H]³⁺, z=4 510.10[M+4H]⁴⁺

### (27-3) Coupling between Disulfide Linker and Peptide

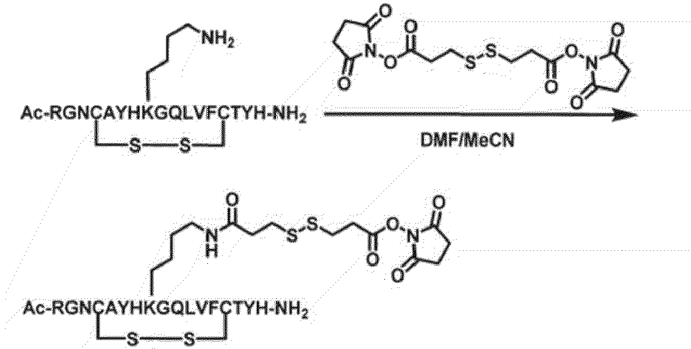

The amino acid sequence is SEQ ID NO: 30.

Ac-RGNCAYHKGQLVFCTYH-NH₂ (SEQ ID NO: 30) synthesized in (27-2) (22.1 mg, 10.9 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (176 mg, 436 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (8.00 mg, 3.34 µmol).

MS(ESI) m/z:z=3 776.05[M+3H]³⁺
HPLC purity: 89%

### (27-4) Specific Modification of Anti-HER2 IgG antibody Trastuzumab and Analysis by ESI-TOFMS

The peptide- and disulfide linker-coupled NHS-activation compound synthesized in (27-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148069, whereas a peak for a product with one binding peptide introduced was observed at 150279, and a peak for a product with two binding peptides introduced was observed at 152490.

### (27-5) Determination of Heavy Chain Selectivity of Specifically Modified Compound of Trastuzumab under Reduction Conditions by ESI-TOFMS Analysis

To the antibody-peptide conjugate formed in (27-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (27-6) HIC-UPLC Analysis of Specific Modification of Anti-HER2 IgG antibody Trastuzumab

The antibody-peptide conjugate formed in (27-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

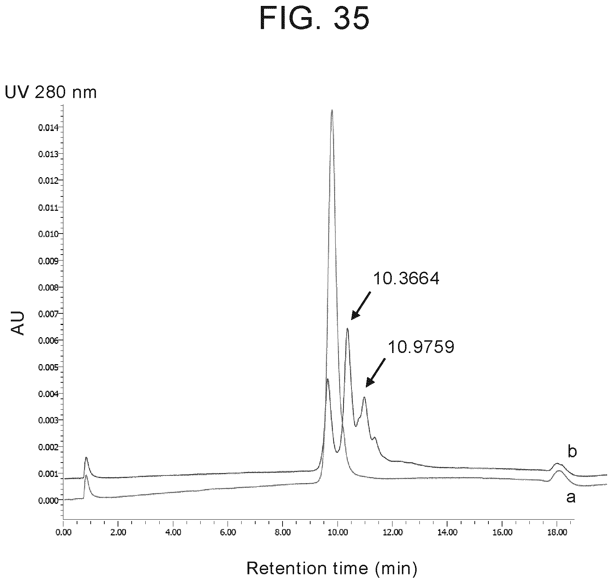

It is believed that a retension time of 9.6408 minutes is attributed to the trastuzumab raw material, whereas that of 10.3664 minutes is attributed to a compound with one peptide introduced to trastuzumab, and that of 10.9759 minutes is attributed to a compound with two peptides introduced to trastuzumab (FIG. 35).

### (27-7) Linker Cleavage and Reoxidation of Trastuzumab-Peptide Conjugate

By performing linker cleavage and reoxidation of the antibody-peptide conjugate obtained in (27-4) by the method described in (3-1) of Example 3, it is possible to obtain an antibody in which thiopropionyl groups are regioselectively introduced to Lys246 and Lys248 in EU numbering.

### [Example 28: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (28-1) Synthesis of IgG1 Fc-Binding Peptide

The peptide of Ac-RGNCAYHKGQQVWCTYH-NH₂ (SEQ ID NO: 31) was synthesized and purified in a similar method to (1-1) of Example 1 to obtain a target product (51.3 mg, 24.5 µmol).

MS(ESI) m/z:z=3 689.45[M+3H]³⁺,z=4 524.05[M+4H]⁴⁺

### (28-2) Formation of Intra-Molecular Disulfide Bond between Cys at Positions 4 and 14 of Ac-RGNCAYHKGQQVWCTYH-NH₂ (SEQ ID NO: 31)

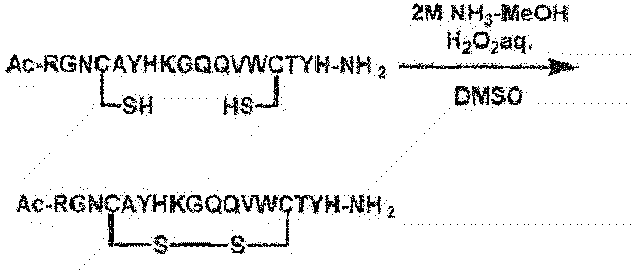

The amino acid sequence is SEQ ID NO: 31.

The peptide (51.3 mg, 24.5 µmol) synthesized in (28-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (31.6 mg, 15.1 µmol).

MS(ESI) m/z:z=3 697.60[M+3H]³⁺, z=4 523.60[M+4H]⁴⁺

### (28-3) Coupling between Disulfide Linker and Peptide

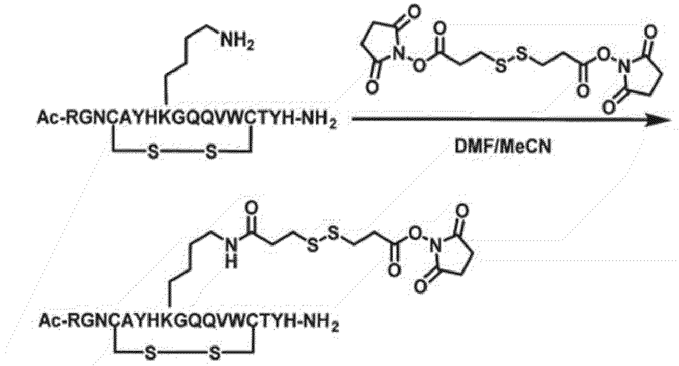

The amino acid sequence is SEQ ID NO: 31.

Ac-RGNCAYHKGQQVWCTYH-NH₂ (SEQ ID NO: 31) synthesized in (28-2) (31.6 mg, 15.1 µmol, the 4th and 14th of two cysteines form a disulfide bond in the molecule) was dissolved in N,N'-dimethylformamide (1.40 mL), a solution dissolving 3,3'-dithiodipropionic acid di(N-succinimidyl) (244 mg, 604 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.00 mg, 2.52 µmol).

MS(ESI) m/z:z=3 794.40[M+3H]³⁺
HPLC purity: 91%

### (28-4) Specific Modification of Anti-HER2 IgG antibody Trastuzumab and Analysis by ESI-TOFMS

The peptide- and disulfide linker-coupled NHS-activation compound synthesized in (28-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148057, whereas a peak for a product with one binding peptide introduced was observed at 150484, and a peak for a product with two binding peptides introduced was observed at 152587.

### (28-5) Determination of Heavy Chain Selectivity of Specifically Modified Compound of Trastuzumab under Reduction Conditions by ESI-TOFMS Analysis

To the antibody-peptide conjugate formed in (28-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (28-6) HIC-UPLC Analysis of Specific Modification of Anti-HER2 IgG antibody Trastuzumab

The antibody-peptide conjugate formed in (28-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

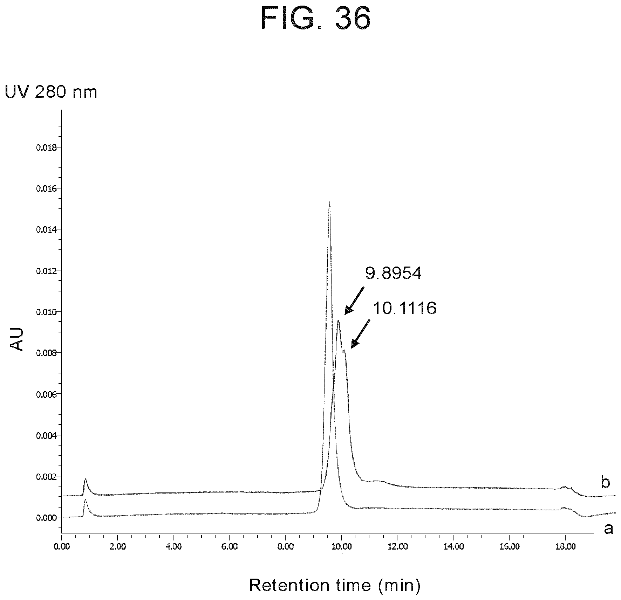

It is believed that a retension time of 9.8954 minutes is attributed to a compound with one peptide introduced to trastuzumab, whereas that of 10.1116 minutes is attributed to a compound with two peptides introduced to trastuzumab (FIG. 36).

### (28-7) Linker Cleavage and Reoxidation of Trastuzumab-Peptide Conjugate

By performing linker cleavage and reoxidation of the antibody-peptide conjugate obtained in (28-4) by the method described in (3-1) of Example 3, it is possible to obtain an antibody in which thiopropionyl groups are regioselectively introduced to Lys246 and Lys248 in EU numbering.

### [Example 29: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (29-1) Synthesis of IgG1 Fc-Binding Peptide

The peptide of Ac-RGNCAYHKGQEVWCTYH-NH₂ (SEQ ID NO: 32) was synthesized and purified in a similar method to (1-1) of Example 1 to obtain a target product (54.0 mg, 25.8 µmol).

MS(ESI) m/z:z=3 698.80[M+3H]³⁺,z=4 524.40[M+4H]⁴⁺

### (29-2) Formation of Intra-Molecular Disulfide Bond between Cys at Positions 4 and 14 of Ac-RGNCAYHKGQEVWCTYH-NH₂ (SEQ ID NO: 32)

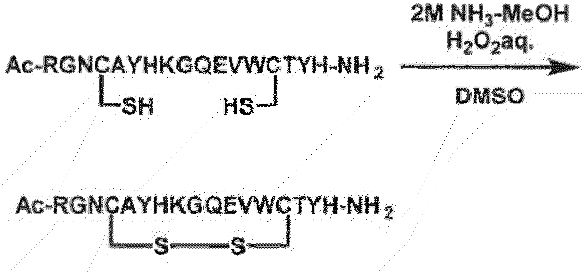

The amino acid sequence is SEQ ID NO: 32.

The peptide (54.0 mg, 25.8 µmol) synthesized in (29-1) was dissolved in DMSO to be 100 mM, then 2 equivalents of 2M NH₃-MeOH and 1 equivalent of a hydrogen peroxide solution were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (22.2 mg, 10.6 µmol).

MS(ESI) m/z:z=3 698.00[M+3H]³⁺, z=4 523.85[M+4H]⁴⁺

### (29-3) Coupling between Disulfide Linker and Peptide

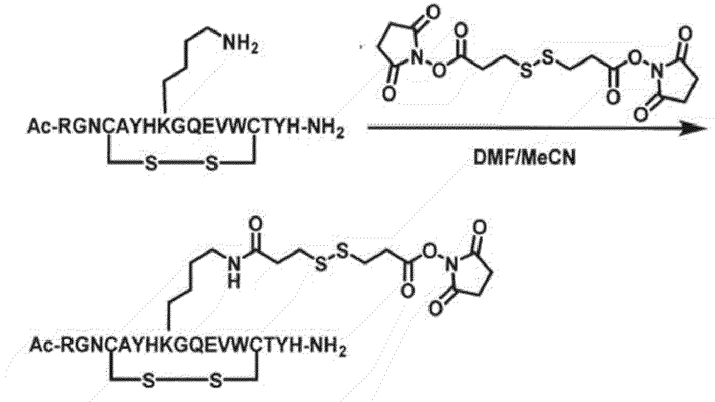

The amino acid sequence is SEQ ID NO: 32.

Ac-RGNCAYHKGQEVWCTYH-NH₂ (SEQ ID NO: 32) synthesized in (29-2) (22.2 mg, 10.6 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (171 mg, 424 µmol) in acetonitrile (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (4.50 mg, 1.89 µmol).

MS(ESI) m/z:z=3 794.45[M+3H]³⁺
HPLC purity: 92%

### (29-4) Specific Modification of Anti-HER2 IgG antibody Trastuzumab and Analysis by ESI-TOFMS

The peptide- and disulfide linker-coupled NHS-activation compound synthesized in (29-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150036, and a peak for a product with two binding peptides introduced was observed at 152599.

### (29-5) Determination of Heavy Chain Selectivity of Specifically Modified Compound of Trastuzumab under Reduction Conditions by ESI-TOFMS Analysis

To the antibody-peptide conjugate formed in (29-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (29-6) HIC-UPLC Analysis of Specific Modification of Anti-HER2 IgG antibody Trastuzumab

The antibody-peptide conjugate formed in (29-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

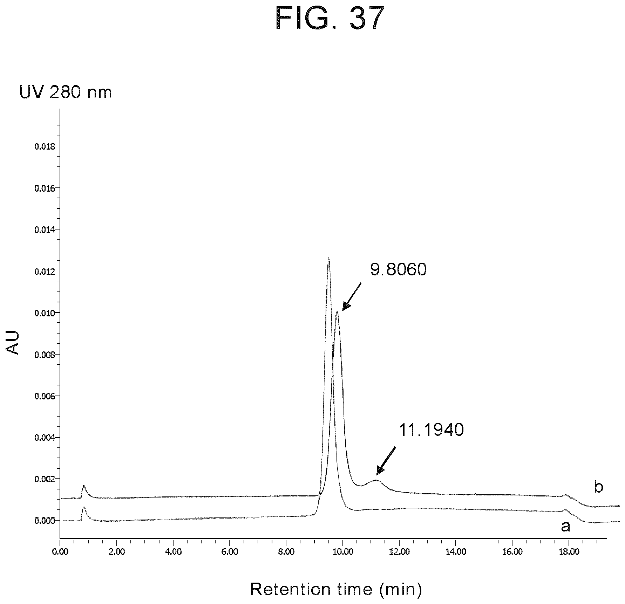

It is believed that a retension time of 9.8060 minutes is attributed to a compound with one peptide introduced to trastuzumab, whereas that of 11.1940 minutes is attributed to a compound with two peptides introduced to trastuzumab (FIG. 37).

### (29-7) Linker Cleavage and Reoxidation of Trastuzumab-Peptide Conjugate

By performing linker cleavage and reoxidation of the antibody-peptide conjugate obtained in (29-4) by the method described in (3-1) of Example 3, it is possible to obtain an antibody in which thiopropionyl groups are regioselectively introduced to Lys246 and Lys248 in EU numbering.

### [Example 30: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (30-1) Synthesis of IgG1 Fc-Binding Peptide

The peptide of Ac-CAYHKGQLVWC-NH₂ (SEQ ID NO: 33) was synthesized and purified in a similar method to (1-1) of Example 1 to obtain a target product (171 mg, 118 µmol).

MS(ESI)m/z:z=2 675.20 [M+2H]²⁺

### (30-2) Formation of Intra-Molecular Disulfide Bond between Cys at Positions 4 and 14 of Ac-CAYHKGQLVWC-NH₂ (SEQ ID NO: 33)

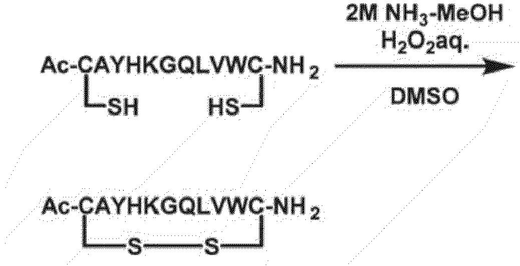

The amino acid sequence is SEQ ID NO: 33.

The peptide (171 mg, 118 µmol) synthesized in (30-1) was dissolved in DMSO (5.00 mL), 2 M NH₃-MeOH (118 µL, 236 mmol) and a hydrogen peroxide solution (241 µL, 2.36 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (23.2 mg, 17.2 µmol).

MS(ESI)m/z:z=1 1347.8[M+H]⁺,z=2 674.15[M+2H]²⁺

### (30-3) Coupling between Disulfide Linker and Peptide

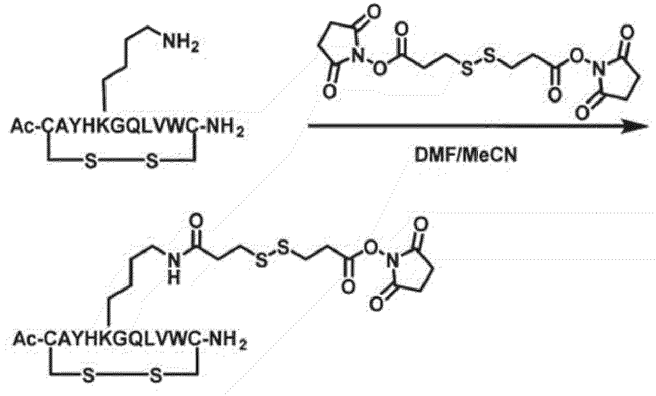

The amino acid sequence is SEQ ID NO: 33.

Ac-CAYHKGQLVWC-NH₂ (SEQ ID NO: 33) synthesized in (30-2) 23.2 mg, 17.2 µmol, the 1st and 11th of two cysteines form a disulfide bond in the molecule) was dissolved in N,N'-dimethylformamide (1.50 mL), a solution dissolving 3,3'-dithiodipropionic acid di(N-succinimidyl) (278 mg, 688 µmol) in acetonitrile (2.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (9.00 mg, 5.50 µmol).

MS(ESI)m/z:z=2 818.65[M+2H]²⁺
HPLC purity: 92%

### (30-4) Specific Modification of Anti-HER2 IgG antibody Trastuzumab and Analysis by ESI-TOFMS

The peptide- and disulfide linker-coupled NHS-activation compound synthesized in (30-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149591, and a peak for a product with two binding peptides introduced was observed at 151112.

### (30-5) Determination of Heavy Chain Selectivity of Specifically Modified Compound of Trastuzumab under Reduction Conditions by ESI-TOFMS Analysis

To the antibody-peptide conjugate formed in (30-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (30-6) HIC-UPLC Analysis of Specific Modification of Anti-HER2 IgG antibody Trastuzumab

The antibody-peptide conjugate formed in (30-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

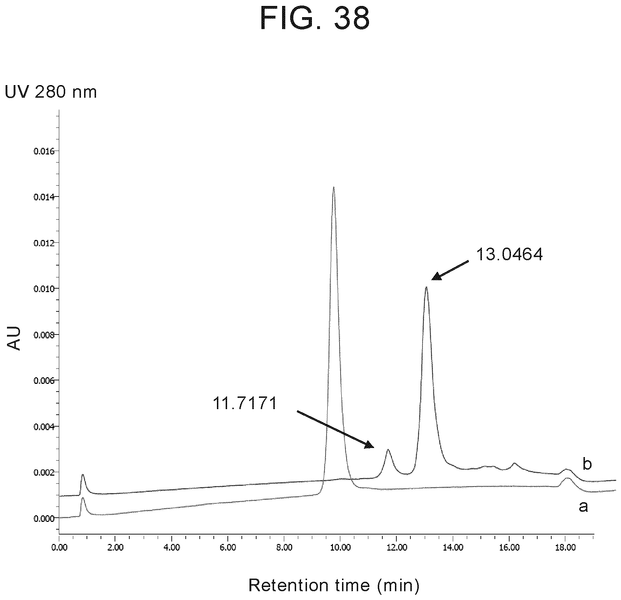

It is believed that a retension time of 11.7171 minutes is attributed to a compound with one peptide introduced to trastuzumab, whereas that of 13.0646 minutes is attributed to a compound with two peptides introduced to trastuzumab (FIG. 38).

### (30-7) Linker Cleavage and Reoxidation of Trastuzumab-Peptide Conjugate

By performing linker cleavage and reoxidation of the antibody-peptide conjugate obtained in (30-4) by the method described in (3-1) of Example 3, it is possible to obtain an antibody in which thiopropionyl groups are regioselectively introduced to Lys246 and Lys248 in EU numbering.

### [Example 31: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (31-1) Synthesis of IgG1 Fc-Binding Peptide

The peptide of Ac-RGNCAYHKAQLVWCTYH-NH₂ (SEQ ID NO: 34) was synthesized and purified in a similar method to (1-1) of Example 1 to obtain a target product (58.8 mg, 28.1 µmol).

MS(ESI)m/z:z=3 697.90[M+3H]³⁺

### (31-2) Formation of Intra-Molecular Disulfide Bond between Cys at Positions 4 and 14 of Ac-RGNCAYHKAQLVWCTYH-NH₂ (SEQ ID NO: 34)

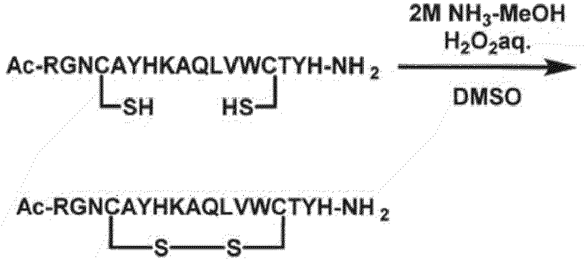

The amino acid sequence is SEQ ID NO: 34.

The peptide (58.8 mg, 28.1 µmol) synthesized in (31-1) was dissolved in DMSO (5.00 mL), 2 M NH₃-MeOH (28.1 µL, 56.2 µmol) and a hydrogen peroxide solution (57.4 µL, 562 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (21.2 mg, 10.1 µmol).

MS(ESI)m/z:z=3 697.50[M+3H]³⁺

### (31-3) Coupling between Disulfide Linker and Peptide

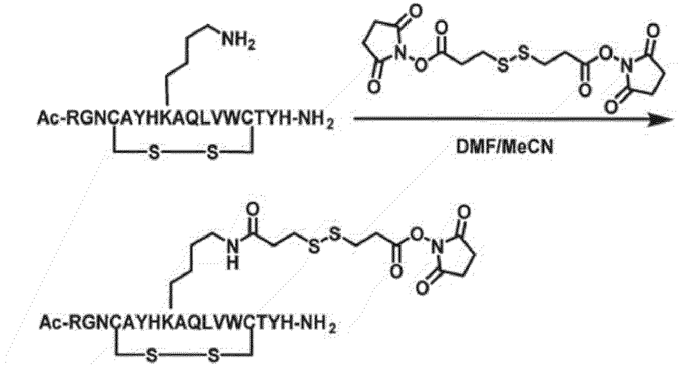

The amino acid sequence is SEQ ID NO: 34.

Ac-RGNCAYHKAQLVWCTYH-NH₂ (SEQ ID NO: 34) synthesized in (31-2) (21.2 mg, 10.1 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (163 mg, 404 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (8.00 mg, 3.36 µmol).

MS(ESI)m/z:z=3 794.00[M+3H]³⁺
HPLC purity: 91%

### (31-4) Specific Modification of Anti-HER2 IgG antibody Trastuzumab and Analysis by ESI-TOFMS

The peptide- and disulfide linker-coupled NHS-activation compound synthesized in (31-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148057, whereas a peak for a product with one binding peptide introduced was observed at 150492, and a peak for a product with two binding peptides introduced was observed at 152755.

### (31-5) Determination of Heavy Chain Selectivity of Specifically Modified Compound of Trastuzumab under Reduction Conditions by ESI-TOFMS Analysis

To the antibody-peptide conjugate formed in (31-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (31-6) HIC-UPLC Analysis of Specific Modification of Anti-HER2 IgG antibody Trastuzumab

The antibody-peptide conjugate formed in (31-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

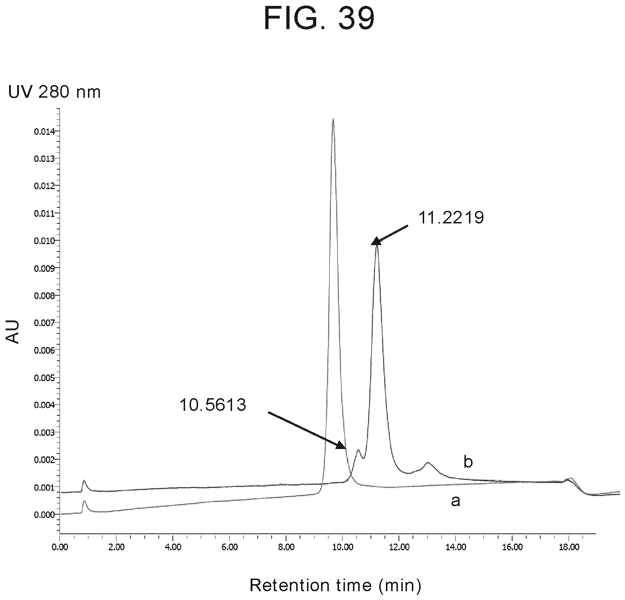

It is believed that a retension time of 10.5613 minutes is attributed to a compound with one peptide introduced to trastuzumab, whereas that of 11.2219 minutes is attributed to a compound with two peptides introduced to trastuzumab (FIG. 39).

### (31-7) Linker Cleavage and Reoxidation of Trastuzumab-Peptide Conjugate

By performing linker cleavage and reoxidation of the antibody-peptide conjugate obtained in (31-4) by the method described in (3-1) of Example 3, it is possible to obtain an antibody in which thiopropionyl groups are regioselectively introduced to Lys246 and Lys248 in EU numbering.

### [Example 32: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (32-1) Synthesis of IgG1 Fc-Binding Peptide

The peptide of Ac-RGNCAYHKVQLVWCTYH-NH₂ (SEQ ID NO: 35) was synthesized and purified in a similar method to (1-1) of Example 1 to obtain a target product (52.2 mg, 24.6 µmol).

MS(ESI)m/z:z=4 530.60[M+4H]⁴⁺

### (32-2) Formation of Intra-Molecular Disulfide Bond between Cys at Positions 4 and 14 of Ac-RGNCAYHKVQLVWCTYH-NH₂ (SEQ ID NO: 35)

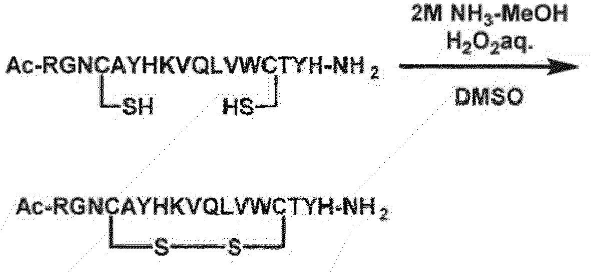

The amino acid sequence is SEQ ID NO: 35.

The peptide (52.2 mg, 24.6 µmol) synthesized in (32-1) was dissolved in DMSO (5.00 mL), 2 M NH₃-MeOH (24.6 µL, 49.2 µmol) and a hydrogen peroxide solution (50.2 µL, 492 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (17.3 mg, 8.17 µmol).

MS(ESI)m/z:z=4 530.30[M+4H]⁴⁺

### (32-3) Coupling between Disulfide Linker and Peptide

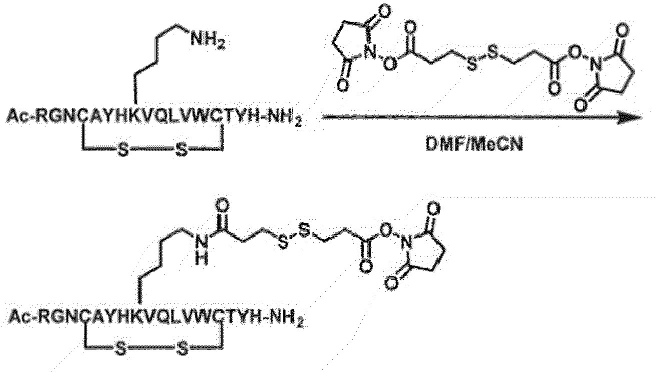

The amino acid sequence is SEQ ID NO: 35.

Ac-RGNCAYHKVQLVWCTYH-NH₂ (SEQ ID NO: 35) synthesized in (32-2) (17.3 mg, 8.17 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (132 mg, 327 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.80 mg, 2.83 µmol).

MS(ESI)m/z:z=3 803.45[M+3H]³⁺
HPLC purity: 82%

### (32-4) Specific Modification of Anti-HER2 IgG antibody Trastuzumab and Analysis by ESI-TOFMS

The peptide- and disulfide linker-coupled NHS-activation compound synthesized in (32-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148057, and for the raw material trastuzumab, a peak was observed at 148062, whereas a peak for a product with one binding peptide introduced was observed at 150517, and a peak for a product with two binding peptides introduced was observed at 152998.

### (32-5) Determination of Heavy Chain Selectivity of Specifically Modified Compound of Trastuzumab under Reduction Conditions by ESI-TOFMS Analysis

To the antibody-peptide conjugate formed in (32-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (32-6) HIC-UPLC Analysis of Specific Modification of Anti-HER2 IgG antibody Trastuzumab

The antibody-peptide conjugate formed in (32-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

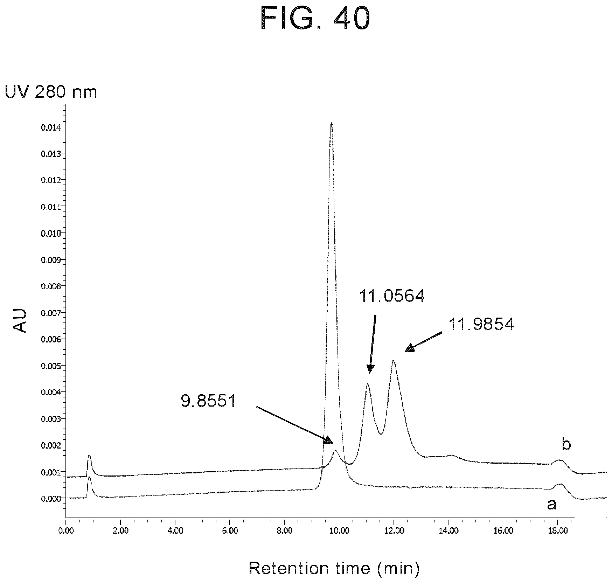

It is believed that a retension time of 9.8551 minutes is attributed to the trastuzumab raw material, whereas that of 11.0564 minutes is attributed to a compound with one peptide introduced to trastuzumab, and that of 11.9854 minutes is attributed to a compound with two peptides introduced to trastuzumab (FIG. 40).

### (32-7) Linker Cleavage and Reoxidation of Trastuzumab-Peptide Conjugate

By performing linker cleavage and reoxidation of the antibody-peptide conjugate obtained in (32-4) by the method described in (3-1) of Example 3, it is possible to obtain an antibody in which thiopropionyl groups are regioselectively introduced to Lys246 and Lys248 in EU numbering.

### [Example 33: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (33-1) Synthesis of IgG1 Fc-Binding Peptide

The peptide of Ac-RGNCAYHKLQLVWCTYH-NH₂ (SEQ ID NO: 36) was synthesized and purified in a similar method to (1-1) of Example 1 to obtain a target product (155 mg, 72.7 µmol).

MS(ESI)m/z:z=3 712.40[M+3H]³⁺

### (33-2) Formation of Intra-Molecular Disulfide Bond between Cys at Positions 4 and 14 of Ac-RGNCAYHKLQLVWCTYH-NH₂ (SEQ ID NO: 36)

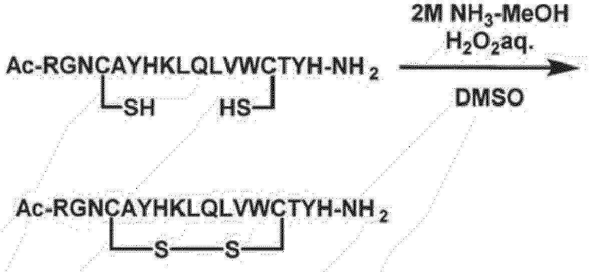

The amino acid sequence is SEQ ID NO: 36.

The peptide (155 mg, 72.7 µmol) synthesized in (33-1) was dissolved in DMSO (5.00 mL), 2 M NH₃-MeOH (72.7 µL, 145 µmol) and a hydrogen peroxide solution (148 µL, 1.45 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (13.3 mg, 6.24 µmol).

MS(ESI)m/z:z=3 711.50[M+3H]³⁺

### (33-3) Coupling between Disulfide Linker and Peptide

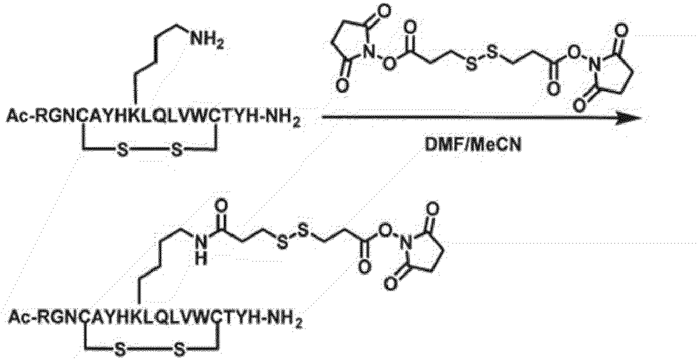

The amino acid sequence is SEQ ID NO: 36.

Ac-RGNCAYHKLQLVWCTYH-NH₂ (SEQ ID NO: 36) synthesized in (33-2) (13.3 mg, 6.24 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (101 mg, 250 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (7.00 mg, 2.89 µmol).

MS(ESI)m/z:z=3 807.80[M+3H]³⁺
HPLC purity: 89%

### (33-4) Specific Modification of Anti-HER2 IgG antibody Trastuzumab and Analysis by ESI-TOFMS

The peptide- and disulfide linker-coupled NHS-activation compound synthesized in (33-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148057, whereas a peak for a product with one binding peptide introduced was observed at 150899, and a peak for a product with two binding peptides introduced was observed at 153025.

### (33-5) Determination of Heavy Chain Selectivity of Specifically Modified Compound of Trastuzumab under Reduction Conditions by ESI-TOFMS Analysis

To the antibody-peptide conjugate formed in (33-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (33-6) HIC-UPLC Analysis of Specific Modification of Anti-HER2 IgG antibody Trastuzumab

The antibody-peptide conjugate formed in (33-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

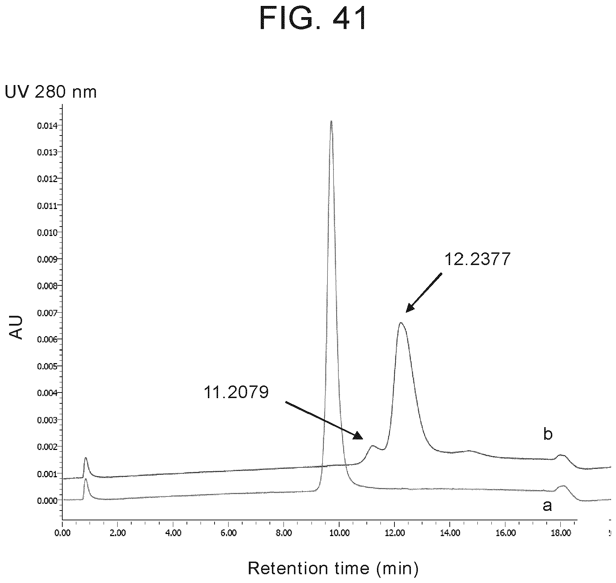

It is believed that a retension time of 11.2079 minutes is attributed to a compound with one peptide introduced to trastuzumab, whereas that of 12.2377 minutes is attributed to a compound with two peptides introduced to trastuzumab (FIG. 41).

### (33-7) Linker Cleavage and Reoxidation of Trastuzumab-Peptide Conjugate

By performing linker cleavage and reoxidation of the antibody-peptide conjugate obtained in (33-4) by the method described in (3-1) of Example 3, it is possible to obtain an antibody in which thiopropionyl groups are regioselectively introduced to Lys246 and Lys248 in EU numbering.

### [Example 34: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (34-1) Synthesis of IgG1 Fc-Binding Peptide

The peptide of Ac-RGNCAYHKIQLVWCTYH-NH₂ (SEQ ID NO: 37) was synthesized and purified in a similar method to (1-1) of Example 1 to obtain a target product (153 mg, 71.7 µmol).

MS(ESI)m/z:z=3 712.10[M+3H]³⁺

### (34-2) Formation of Intra-Molecular Disulfide Bond between Cys at Positions 4 and 14 of Ac-RGNCAYHKIQLVWCTYH-NH₂ (SEQ ID NO: 37)

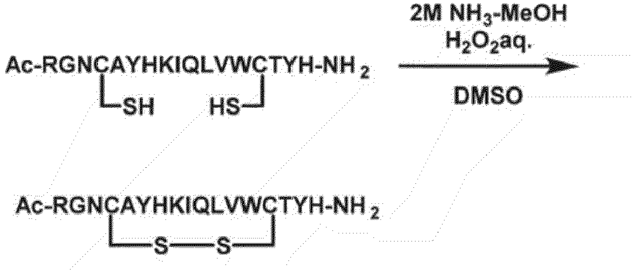

The amino acid sequence is SEQ ID NO: 37.

The peptide (153 mg, 71.7 µmol) synthesized in (34-1) was dissolved in DMSO (5.00 mL), 2 M NH₃-MeOH (71.7 µL, 143 µmol) and a hydrogen peroxide solution (146 µL, 1.43 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (15.7 mg, 7.37 µmol).

MS(ESI)m/z:z=3 711.40[M+3H]³⁺

### (34-3) Coupling between Disulfide Linker and Peptide

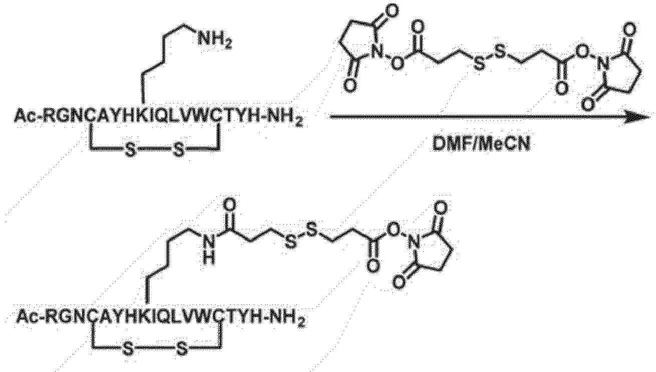

The amino acid sequence is SEQ ID NO: 37.

Ac-RGNCAYHKIQLVWCTYH-NH₂ (SEQ ID NO: 37) synthesized in (34-2) (15.7 mg, 7.37 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (119 mg, 295 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.00 mg, 2.07 µmol).

MS(ESI)m/z:z=3 807.75[M+3H]³⁺
HPLC purity: 94%

### (34-4) Specific Modification of Anti-HER2 IgG antibody Trastuzumab and Analysis by ESI-TOFMS

The peptide- and disulfide linker-coupled NHS-activation compound synthesized in (34-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150720, and a peak for a product with two binding peptides introduced was observed at 153210.

### (34-5) Determination of Heavy Chain Selectivity of Specifically Modified Compound of Trastuzumab under Reduction Conditions by ESI-TOFMS Analysis

To the antibody-peptide conjugate formed in (34-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (34-6) HIC-UPLC Analysis of Specific Modification of Anti-HER2 IgG antibody Trastuzumab

The antibody-peptide conjugate formed in (34-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

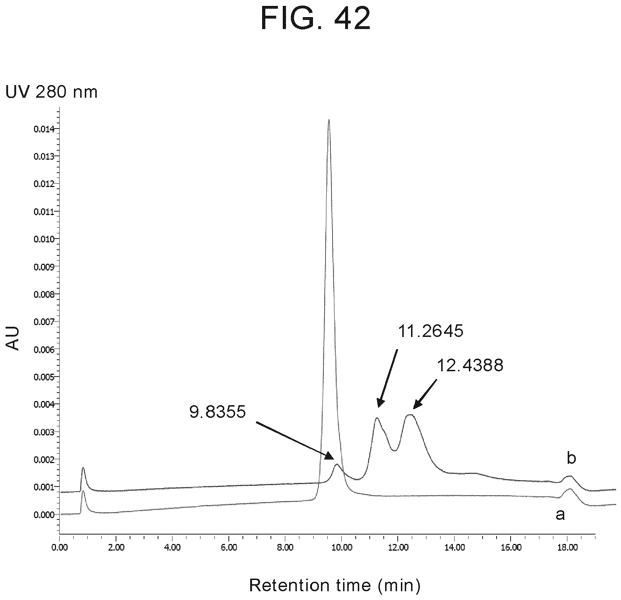

It is believed that a retension time of 9.8355 minutes is attributed to the trastuzumab raw material, whereas that of 11.2645 minutes is attributed to a compound with one peptide introduced to trastuzumab, and that of 12.4388 minutes is attributed to a compound with two peptides introduced to trastuzumab (FIG. 42).

### (34-7) Linker Cleavage and Reoxidation of Trastuzumab-Peptide Conjugate

By performing linker cleavage and reoxidation of the antibody-peptide conjugate obtained in (34-4) by the method described in (3-1) of Example 3, it is possible to obtain an antibody in which thiopropionyl groups are regioselectively introduced to Lys246 and Lys248 in EU numbering.

### [Example 35: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (35-1) Synthesis of IgG1 Fc-Binding Peptide

The peptide of Ac-RGNCAYHKSQLVWCTYH-NH₂ (SEQ ID NO: 38) was synthesized and purified in a similar method to (1-1) of Example 1 to obtain a target product (167 mg, 79.2 µmol).

MS(ESI)m/z:z=3 703.45[M+3H]³⁺

### (35-2) Formation of Intra-Molecular Disulfide Bond between Cys at Positions 4 and 14 of Ac-RGNCAYHKSQLVWCTYH-NH₂ (SEQ ID NO: 38)

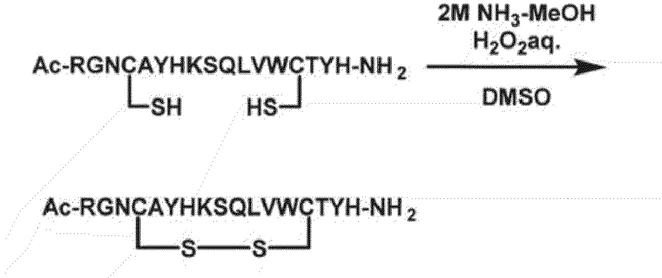

The amino acid sequence is SEQ ID NO: 38.

The peptide (167 mg, 79.2 µmol) synthesized in (35-1) was dissolved in DMSO (5.00 mL), 2 M NH₃-MeOH (79.2 µL, 158 µmol) and a hydrogen peroxide solution (161 µL, 1.58 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (19.6 mg, 9.31 µmol).

MS (ESI)m/z: z=3 702.90 [M+3H]³⁺

### (35-3) Coupling between Disulfide Linker and Peptide

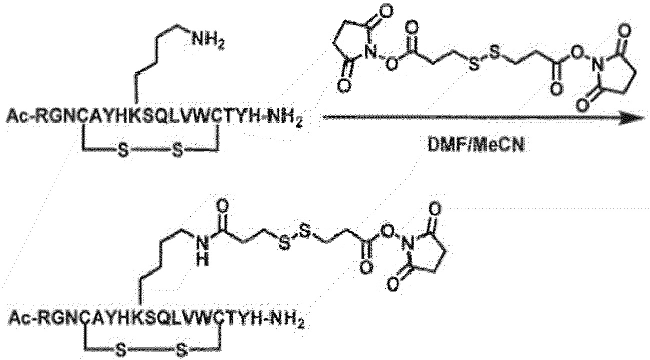

The amino acid sequence is SEQ ID NO: 38.

Ac-RGNCAYHKSQLVWCTYH-NH₂ (SEQ ID NO: 38) synthesized in (35-2) (19.6 mg, 9.31 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (150 mg, 372 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (7.50 mg, 3.13 µmol).

MS(ESI)m/z:z=3 799.10[M+3H]³⁺
HPLC purity: 92%

### (35-4) Specific Modification of Anti-HER2 IgG antibody Trastuzumab and Analysis by ESI-TOFMS

The peptide- and disulfide linker-coupled NHS-activation compound synthesized in (35-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150505, and a peak for a product with two binding peptides introduced was observed at 152784.

### (35-5) Determination of Heavy Chain Selectivity of Specifically Modified Compound of Trastuzumab under Reduction Conditions by ESI-TOFMS Analysis

To the antibody-peptide conjugate formed in (35-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (35-6) HIC-UPLC Analysis of Specific Modification of Anti-HER2 IgG antibody Trastuzumab

The antibody-peptide conjugate formed in (35-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

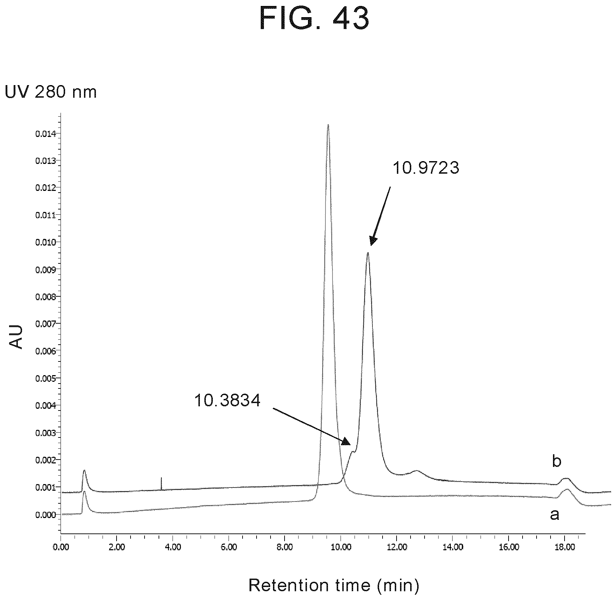

It is believed that a retension time of 10.3834 minutes is attributed to a compound with one peptide introduced to trastuzumab, whereas that of 10.9723 minutes is attributed to a compound with two peptides introduced to trastuzumab (FIG. 43).

### (35-7) Linker Cleavage and Reoxidation of Trastuzumab-Peptide Conjugate

By performing linker cleavage and reoxidation of the antibody-peptide conjugate obtained in (35-4) by the method described in (3-1) of Example 3, it is possible to obtain an antibody in which thiopropionyl groups are regioselectively introduced to Lys246 and Lys248 in EU numbering.

### [Example 36: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (36-1) Synthesis of IgG1 Fc-Binding Peptide

The peptide of Ac-RGNCAYHKTQLVWCTYH-NH₂ (SEQ ID NO: 39) was synthesized and purified in a similar method to (1-1) of Example 1 to obtain a target product (164 mg, 77.3 µmol).

MS(ESI)m/z:z=3 708.40[M+3H]³⁺

### (36-2) Formation of Intra-Molecular Disulfide Bond between Cys at Positions 4 and 14 of Ac-RGNCAYHKTQLVWCTYH-NH₂ (SEQ ID NO: 39)

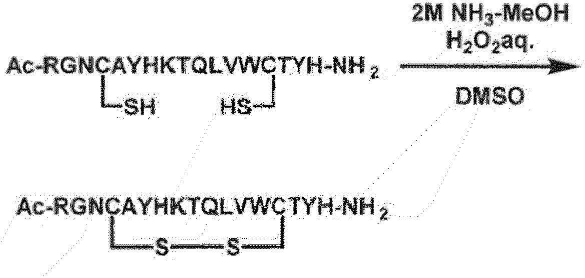

The amino acid sequence is SEQ ID NO: 39.

The peptide (164 mg, 77.3 µmol) synthesized in (36-1) was dissolved in DMSO (5.00 mL), 2 M NH₃-MeOH (77.3 µL, 155 µmol) and a hydrogen peroxide solution (158 µL, 1.55 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (16.3 mg, 7.69 µmol).

MS(ESI)m/z:z=3 707.60[M+3H]³⁺

### (36-3) Coupling between Disulfide Linker and Peptide

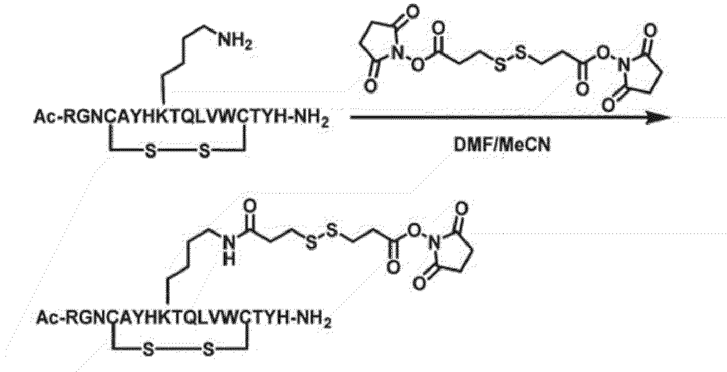

The amino acid sequence is SEQ ID NO: 39.

Ac-RGNCAYHKTQLVWCTYH-NH₂ (SEQ ID NO: 39) synthesized in (36-2) (16.3 mg, 7.69 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (125 mg, 308 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (4.50 mg, 1.87 µmol).

MS(ESI)m/z:z=3 803.70[M+3H]³⁺
HPLC purity: 89%

### (36-4) Specific Modification of Anti-HER2 IgG antibody Trastuzumab and Analysis by ESI-TOFMS

The peptide- and disulfide linker-coupled NHS-activation compound synthesized in (36-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 152814.

### (36-5) Determination of Heavy Chain Selectivity of Specifically Modified Compound of Trastuzumab under Reduction Conditions by ESI-TOFMS Analysis

To the antibody-peptide conjugate formed in (36-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (36-6) HIC-UPLC Analysis of Specific Modification of Anti-HER2 IgG antibody Trastuzumab

The antibody-peptide conjugate formed in (36-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

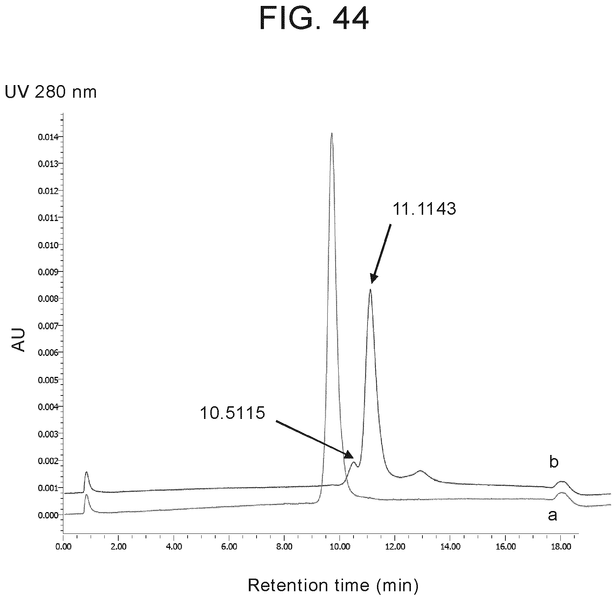

It is believed that a retension time of 10.5115 minutes is attributed to a compound with one peptide introduced to trastuzumab, whereas that of 11.1143 minutes is attributed to a compound with two peptides introduced to trastuzumab (FIG. 44).

### (36-7) Linker Cleavage and Reoxidation of Trastuzumab-Peptide Conjugate

By performing linker cleavage and reoxidation of the antibody-peptide conjugate obtained in (36-4) by the method described in (3-1) of Example 3, it is possible to obtain an antibody in which thiopropionyl groups are regioselectively introduced to Lys246 and Lys248 in EU numbering.

### [Example 37: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (37-1) Synthesis of IgG1 Fc-Binding Peptide

The peptide of Ac-RGNCAYHKNQLVWCTYH-NH₂ (SEQ ID NO: 40) was synthesized and purified in a similar method to (1-1) of Example 1 to obtain a target product (51.4 mg, 24.1 µmol).

MS(ESI)m/z:z=3 712.40[M+3H]³⁺,z=4 534.65[M+4H]⁴⁺

### (37-2) Formation of Intra-Molecular Disulfide Bond between Cys at Positions 4 and 14 of Ac-RGNCAYHKNQLVWCTYH-NH₂ (SEQ ID NO: 40)

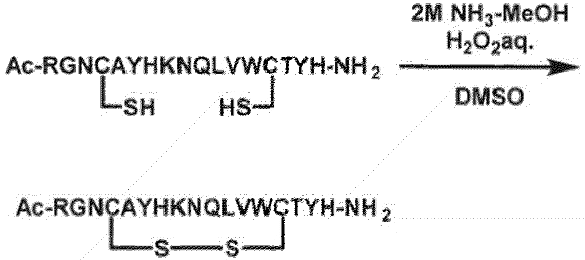

The amino acid sequence is SEQ ID NO: 40.

The peptide (51.4 mg, 24.1 µmol) synthesized in (37-1) was dissolved in DMSO (5.00 mL), 2 M NH₃-MeOH (24.1 µL, 48.2 µmol) and a hydrogen peroxide solution (49.2 µL, 482 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (20.0 mg, 9.38 µmol).

MS(ESI)m/z:z=3 711.75[M+3H]³⁺, z=4 534.10[M+4H]⁴⁺

### (37-3) Coupling between Disulfide Linker and Peptide

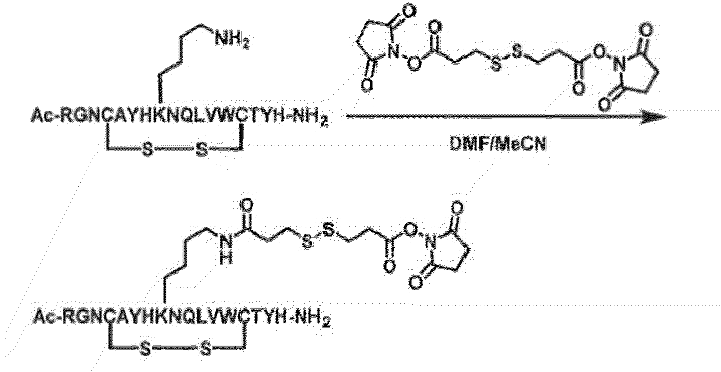

The amino acid sequence is SEQ ID NO: 40.

Ac-RGNCAYHKNQLVWCTYH-NH₂ (SEQ ID NO: 40) synthesized in (37-2) (20.0 mg, 9.38 µmol, the 4th and 14th of two cysteines form a disulfide bond in the molecule) was dissolved in N,N'-dimethylformamide (1.00 mL), a solution dissolving 3,3'-dithiodipropionic acid di(N-succinimidyl) (152 mg, 375 µmol) in acetonitrile (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (8.7 mg, 3.59 µmol).

MS(ESI)m/z:z=3 808.10[M+3H]³⁺
HPLC purity: 93%

### (37-4) Specific Modification of Anti-HER2 IgG antibody Trastuzumab and Analysis by ESI-TOFMS

The peptide- and disulfide linker-coupled NHS-activation compound synthesized in (37-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. A peak for a product with one binding peptide introduced was observed at 150370, and a peak for a product with two binding peptides introduced was observed at 152678.

### (37-5) Determination of Heavy Chain Selectivity of Specifically Modified Compound of Trastuzumab under Reduction Conditions by ESI-TOFMS Analysis

To the antibody-peptide conjugate formed in (37-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (37-6) HIC-UPLC Analysis of Specific Modification of Anti-HER2 IgG antibody Trastuzumab

The antibody-peptide conjugate formed in (37-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

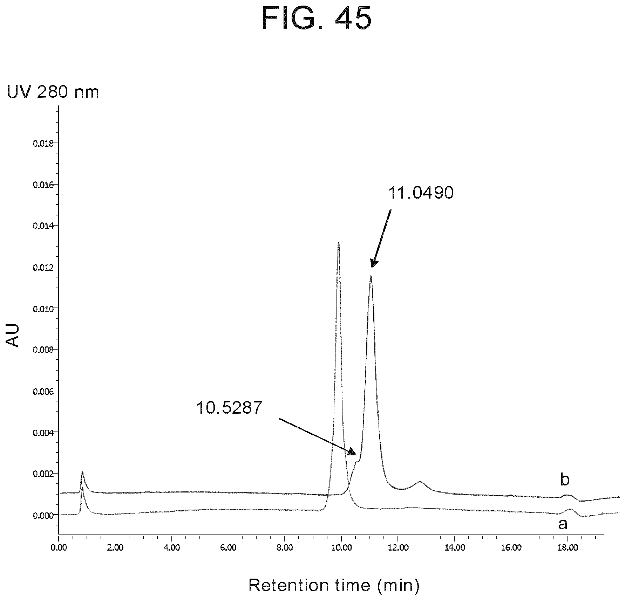

It is believed that a retension time of 10.5287 minutes is attributed to the trastuzumab raw material, whereas that of 11.0490 minutes is attributed to a compound with one peptide introduced to trastuzumab (FIG. 45).

### (37-7) Linker Cleavage and Reoxidation of Trastuzumab-Peptide Conjugate

By performing linker cleavage and reoxidation of the antibody-peptide conjugate obtained in (37-4) by the method described in (3-1) of Example 3, it is possible to obtain an antibody in which thiopropionyl groups are regioselectively introduced to Lys246 and Lys248 in EU numbering.

### [Example 38: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (38-1) Synthesis of IgG1 Fc-Binding Peptide

The peptide of Ac-RGNCAYHKDQLVWCTYH-NH₂ (SEQ ID NO: 41) was synthesized and purified in a similar method to (1-1) of Example 1 to obtain a target product (50.8 mg, 23.7 µmol).

MS(ESI)m/z:z=3 712.55[M+3H]³⁺,z=4 534.75[M+4H]⁴⁺

### (38-2) Formation of Intra-Molecular Disulfide Bond between Cys at Positions 4 and 14 of Ac-RGNCAYHKDQLVWCTYH-NH₂ (SEQ ID NO: 41)

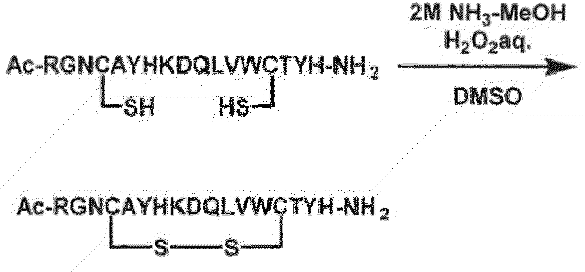

The amino acid sequence is SEQ ID NO: 41.

The peptide (50.8 mg, 23.7 µmol) synthesized in (38-1) was dissolved in DMSO (5.00 mL), 2 M NH₃-MeOH (23.8 µL, 47.4 µmol) and a hydrogen peroxide solution (48.6 µL, 474 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (36.8 mg, 17.2 µmol).

MS(ESI)m/z:z=3 712.05[M+3H]³⁺, z=4 534.35[M+4H]⁴⁺

### (38-3) Coupling between Disulfide Linker and Peptide

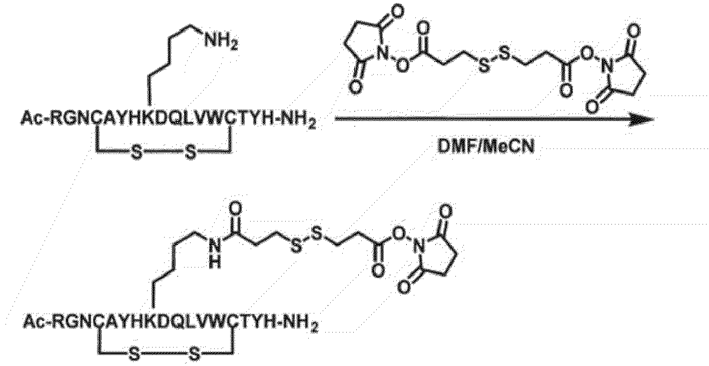

The amino acid sequence is SEQ ID NO: 41.

Ac-RGNCAYHKDQLVWCTYH-NH₂ (SEQ ID NO: 41) synthesized in (38-2) (36.8 mg, 17.2 µmol, the 4th and 14th of two cysteines form a disulfide bond in the molecule) was dissolved in N,N'-dimethylformamide (1.70 mL), a solution dissolving 3,3'-dithiodipropionic acid di(N-succinimidyl) (278 mg, 688 µmol) in acetonitrile (1.00 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.80 mg, 2.39 µmol).

MS(ESI)m/z:z=3 808.45[M+3H]³⁺
HPLC purity: 91%

### (38-4) Specific Modification of Anti-HER2 IgG antibody Trastuzumab and Analysis by ESI-TOFMS

The peptide- and disulfide linker-coupled NHS-activation compound synthesized in (38-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. A peak for a product with one binding peptide introduced was observed at 150367, and a peak for a product with two binding peptides introduced was observed at 152831.

### (38-5) Determination of Heavy Chain Selectivity of Specifically Modified Compound of Trastuzumab under Reduction Conditions by ESI-TOFMS Analysis

To the antibody-peptide conjugate formed in (38-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (38-6) HIC-UPLC Analysis of Specific Modification of Anti-HER2 IgG antibody Trastuzumab

The antibody-peptide conjugate formed in (38-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

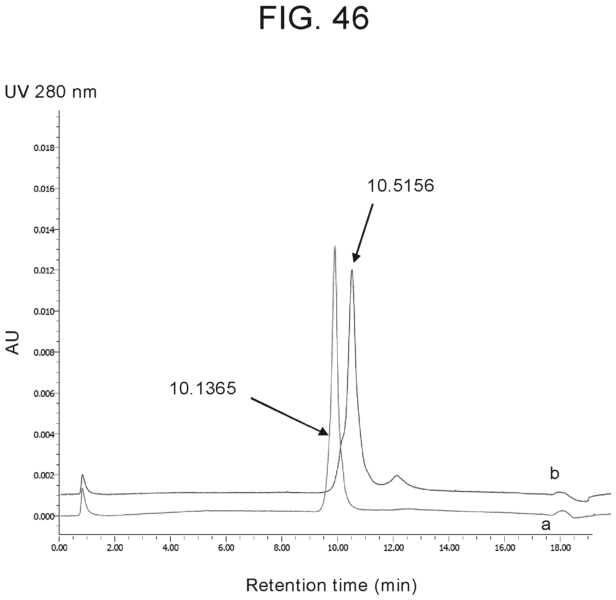

It is believed that a retension time of 10.1365 minutes is attributed to the trastuzumab raw material, whereas that of 10.5156 minutes is attributed to a compound with one peptide introduced to trastuzumab (FIG. 46).

### (38-7) Linker Cleavage and Reoxidation of Trastuzumab-Peptide Conjugate

By performing linker cleavage and reoxidation of the antibody-peptide conjugate obtained in (38-4) by the method described in (3-1) of Example 3, it is possible to obtain an antibody in which thiopropionyl groups are regioselectively introduced to Lys246 and Lys248 in EU numbering.

### [Example 39: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (39-1) Synthesis of IgG1 Fc-Binding Peptide

The peptide of Ac-RGNCAYHKQQLVWCTYH-NH₂ (SEQ ID NO: 42) was synthesized and purified in a similar method to (1-1) of Example 1 to obtain a target product (42.1 mg, 19.6 µmol).

MS(ESI)m/z:z=3 716.95[M+3H]³⁺,z=4 538.10[M+4H]⁴⁺

### (39-2) Formation of Intra-Molecular Disulfide Bond between Cys at Positions 4 and 14 of Ac-RGNCAYHKQQLVWCTYH-NH₂ (SEQ ID NO: 42)

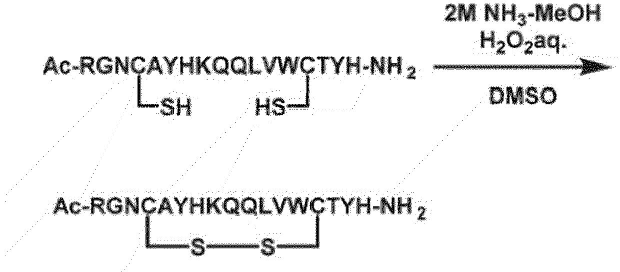

The amino acid sequence is SEQ ID NO: 42.

The peptide (42.1 mg, 19.6 µmol) synthesized in (39-1) was dissolved in DMSO (5.00 mL), 2 M NH₃-MeOH (19.6 µL, 39.2 µmol) and a hydrogen peroxide solution (40.0 µL, 392 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (18.1 mg, 8.39 µmol).

MS(ESI)m/z:z=3 716.30[M+3H]³⁺, z=4 537.50[M+4H]⁴⁺

### (39-3) Coupling between Disulfide Linker and Peptide

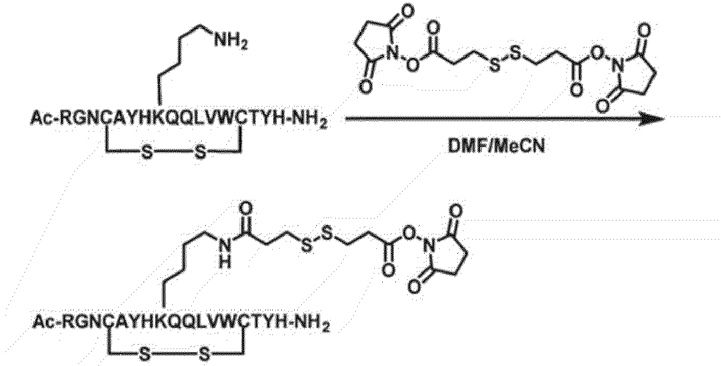

The amino acid sequence is SEQ ID NO: 42.

Ac-RGNCAYHKQQLVWCTYH-NH₂ (SEQ ID NO: 42) synthesized in (39-2) (18.1 mg, 8.39 µmol, the 4th and 14th of two cysteines form a disulfide bond in the molecule) was dissolved in N,N'-dimethylformamide (0.50 mL), a solution dissolving 3,3'-dithiodipropionic acid di(N-succinimidyl) (34.0 mg, 83.9 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.70 mg, 1.52 µmol).

MS(ESI)m/z:z=3 812.75[M+3H]³⁺
HPLC purity: 91%

### (39-4) Specific Modification of Anti-HER2 IgG antibody Trastuzumab and Analysis by ESI-TOFMS

The peptide- and disulfide linker-coupled NHS-activation compound synthesized in (39-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. A peak for a product with one binding peptide introduced was observed at 150540, and a peak for a product with two binding peptides introduced was observed at 152862.

### (39-5) Determination of Heavy Chain Selectivity of Specifically Modified Compound of Trastuzumab under Reduction Conditions by ESI-TOFMS Analysis

To the antibody-peptide conjugate formed in (39-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (39-6) HIC-UPLC Analysis of Specific Modification of Anti-HER2 IgG antibody Trastuzumab

The antibody-peptide conjugate formed in (39-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

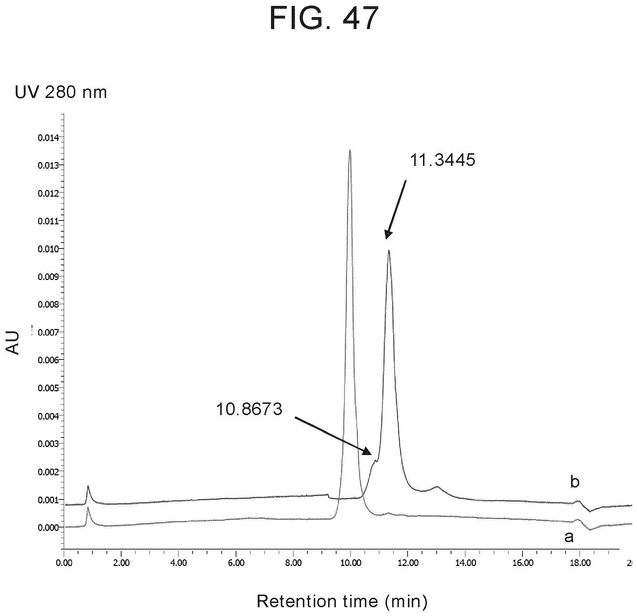

It is believed that a retension time of 10.8637 minutes is attributed to a compound with one peptide introduced to trastuzumab, whereas that of 11.3445 minutes is attributed to a compound with two peptides introduced to trastuzumab (FIG. 47).

### (39-7) Linker Cleavage and Reoxidation of Trastuzumab-Peptide Conjugate

By performing linker cleavage and reoxidation of the antibody-peptide conjugate obtained in (39-4) by the method described in (3-1) of Example 3, it is possible to obtain an antibody in which thiopropionyl groups are regioselectively introduced to Lys246 and Lys248 in EU numbering.

### [Example 40: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (40-1) Synthesis of IgG1 Fc-Binding Peptide

The peptide of Ac-RGNCAYHKEQLVWCTYH-NH₂ (SEQ ID NO: 43) was synthesized and purified in a similar method to (1-1) of Example 1 to obtain a target product (38.3 mg, 17.8 µmol) .

MS(ESI)m/z:z=3 717.30[M+3H]³⁺,z=4 538.30[M+4H]⁴⁺

### (40-2) Formation of Intra-Molecular Disulfide Bond between Cys at Positions 4 and 14 of Ac-RGNCAYHKEQLVWCTYH-NH₂ (SEQ ID NO: 43)

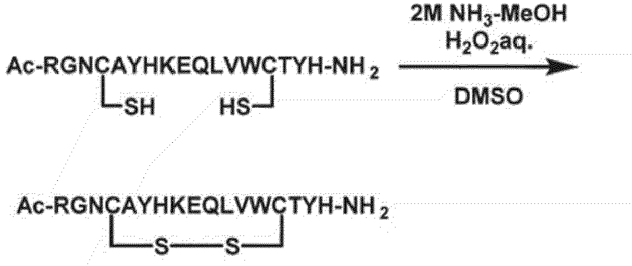

The amino acid sequence is SEQ ID NO: 43.

The peptide (38.3 mg, 17.8 µmol) synthesized in (40-1) was dissolved in DMSO (5.00 mL), 2 M NH₃-MeOH (17.8 µL, 35.6 µmol) and a hydrogen peroxide solution (36.4 µL, 356 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (18.6 mg, 8.70 µmol) .

MS(ESI)m/z:z=3 716.65[M+3H]³⁺, z=4 537.80[M+4H]⁴⁺

### (40-3) Coupling between Disulfide Linker and Peptide

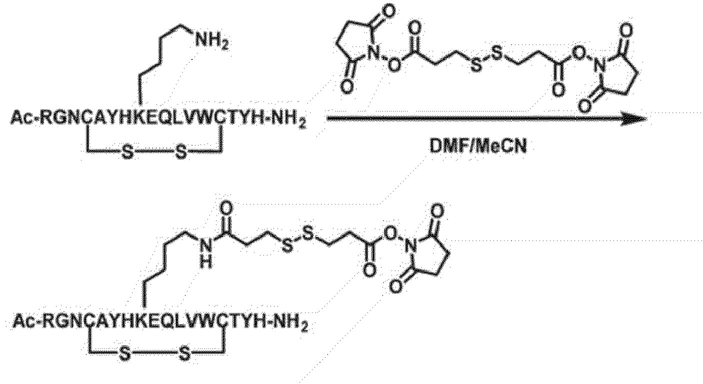

The amino acid sequence is SEQ ID NO: 43.

Ac-RGNCAYHKEQLVWCTYH-NH₂ (SEQ ID NO: 43) synthesized in (40-2) (18.6 mg, 8.70 µmol, the 4th and 14th of two cysteines form a disulfide bond in the molecule) was dissolved in N,N'-dimethylformamide (0.50 mL), a solution dissolving 3,3'-dithiodipropionic acid di(N-succinimidyl) (34.0 mg, 87.0 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.50 mg, 2.26 µmol).

MS(ESI)m/z:z=3 813.15[M+3H]³⁺
HPLC purity: 100%

### (40-4) Specific Modification of Anti-HER2 IgG antibody Trastuzumab and Analysis by ESI-TOFMS

The peptide- and disulfide linker-coupled NHS-activation compound synthesized in (40-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150535, and a peak for a product with two binding peptides introduced was observed at 152865.

### (40-5) Determination of Heavy Chain Selectivity of Specifically Modified Compound of Trastuzumab under Reduction Conditions by ESI-TOFMS Analysis

To the antibody-peptide conjugate formed in (40-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (40-6) HIC-UPLC Analysis of Specific Modification of Anti-HER2 IgG antibody Trastuzumab

The antibody-peptide conjugate formed in (40-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

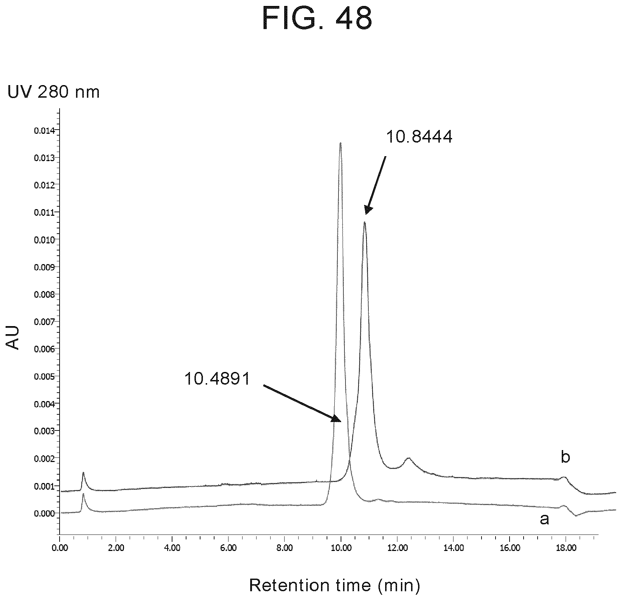

It is believed that a retension time of 10.4891 minutes is attributed to a compound with one peptide introduced to trastuzumab, whereas that of 10.8444 minutes is attributed to a compound with two peptides introduced to trastuzumab (FIG. 48).

### (40-7) Linker Cleavage and Reoxidation of Trastuzumab-Peptide Conjugate

By performing linker cleavage and reoxidation of the antibody-peptide conjugate obtained in (40-4) by the method described in (3-1) of Example 3, it is possible to obtain an antibody in which thiopropionyl groups are regioselectively introduced to Lys246 and Lys248 in EU numbering.

### [Example 41: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (41-1) Synthesis of IgG1 Fc-Binding Peptide

The peptide of Ac-RGNCAYHKFQLVWCTYH-NH₂ (SEQ ID NO: 44) was synthesized and purified in a similar method to (1-1) of Example 1 to obtain a target product (19.5 mg, 9.00 µmol) .

MS(ESI)m/z:z=3 723.25[M+3H]³⁺,z=4 542.90[M+4H]⁴⁺

### (41-2) Formation of Intra-Molecular Disulfide Bond between Cys at Positions 4 and 14 of Ac-RGNCAYHKFQLVWCTYH-NH₂ (SEQ ID NO: 44)

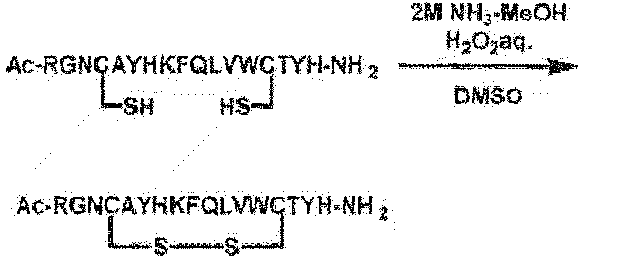

The amino acid sequence is SEQ ID NO: 44.

The peptide (19.5 mg, 9.00 µmol) synthesized in (41-1) was dissolved in DMSO (5.00 mL), 2 M NH₃-MeOH (9.00 µL, 18.0 µmol) and a hydrogen peroxide solution (18.4 µL, 180 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (8.60 mg, 3.97 µmol).

MS(ESI)m/z:z=3 722.75[M+3H]³⁺, z=4 542.30[M+4H]⁴⁺

### (41-3) Coupling between Disulfide Linker and Peptide

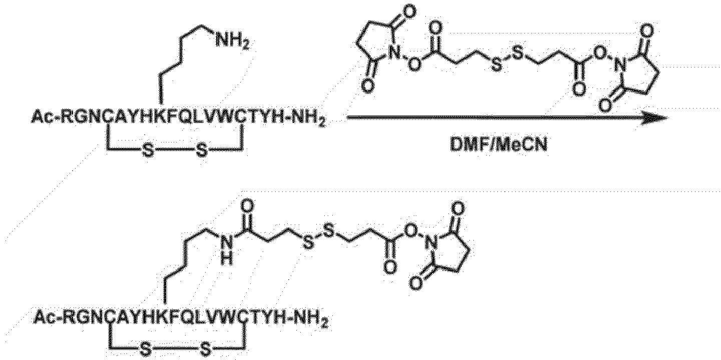

The amino acid sequence is SEQ ID NO: 44.

Ac-RGNCAYHKFQLVWCTYH-NH₂ (SEQ ID NO: 44) synthesized in (41-2) (8.60 mg, 3.97 µmol, the 4th and 14th of two cysteines form a disulfide bond in the molecule) was dissolved in N,N'-dimethylformamide (0.50 mL), a solution dissolving 3,3'-dithiodipropionic acid di(N-succinimidyl) (32.0 mg, 79.4 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.50 mg, 1.02 µmol).

MS(ESI)m/z:z=3 819.20[M+3H]³⁺
HPLC purity: 90%

### (41-4) Specific Modification of Anti-HER2 IgG antibody Trastuzumab and Analysis by ESI-TOFMS

The peptide- and disulfide linker-coupled NHS-activation compound synthesized in (41-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150972, and a peak for a product with two binding peptides introduced was observed at 153454.

### (41-5) Determination of Heavy Chain Selectivity of Specifically Modified Compound of Trastuzumab under Reduction Conditions by ESI-TOFMS Analysis

To the antibody-peptide conjugate formed in (41-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (41-6) HIC-UPLC Analysis of Specific Modification of Anti-HER2 IgG antibody Trastuzumab

The antibody-peptide conjugate formed in (41-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

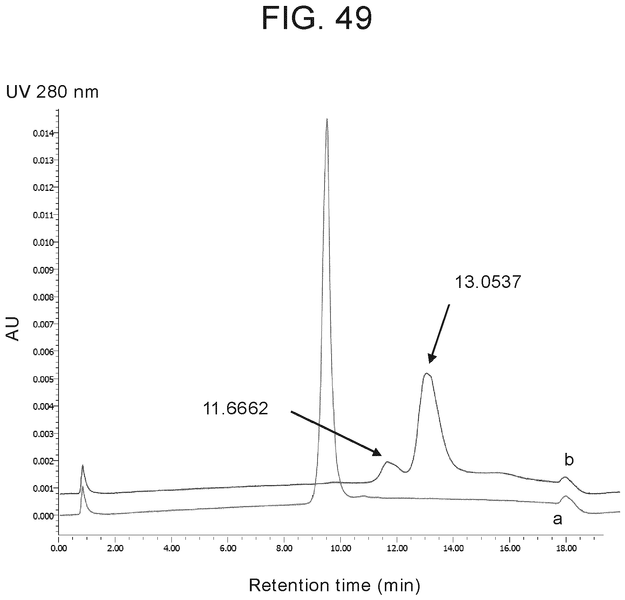

It is believed that a retension time of 11.6662 minutes is attributed to a compound with one peptide introduced to trastuzumab, whereas that of 13.0537 minutes is attributed to a compound with two peptides introduced to trastuzumab (FIG. 49).

### (41-7) Linker Cleavage and Reoxidation of Trastuzumab-Peptide Conjugate

By performing linker cleavage and reoxidation of the antibody-peptide conjugate obtained in (41-4) by the method described in (3-1) of Example 3, it is possible to obtain an antibody in which thiopropionyl groups are regioselectively introduced to Lys246 and Lys248 in EU numbering.

### [Example 42: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (42-1) Synthesis of IgG1 Fc-Binding Peptide

The peptide of Ac-RGNCAYHKRQLVWCTYH-NH₂ (SEQ ID NO: 45) was synthesized and purified in a similar method to (1-1) of Example 1 to obtain a target product (46.2 mg, 21.2 µmol) .

MS(ESI)m/z:z=3 726.30[M+3H]³⁺,z=4 545.10[M+4H]⁴⁺

### (42-2) Formation of Intra-Molecular Disulfide Bond between Cys at Positions 4 and 14 of Ac-RGNCAYHKRQLVWCTYH-NH₂ (SEQ ID NO: 45)

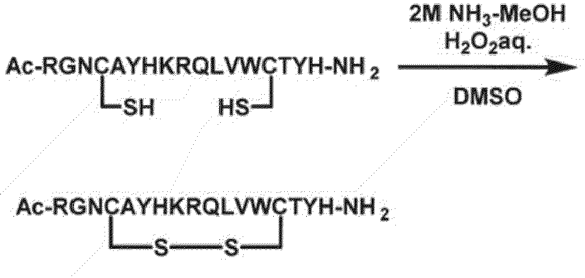

The amino acid sequence is SEQ ID NO: 45.

The peptide (46.2 mg, 21.2 µmol) synthesized in (42-1) was dissolved in DMSO (5.00 mL), 2 M NH₃-MeOH (21.2 µL, 42.4 µmol) and a hydrogen peroxide solution (43.4 µL, 424 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (29.6 mg, 13.6 µmol).

MS(ESI)m/z:z=3 725.10[M+3H]³⁺, z=4 544.65[M+4H]⁴⁺

### (42-3) Coupling between Disulfide Linker and Peptide

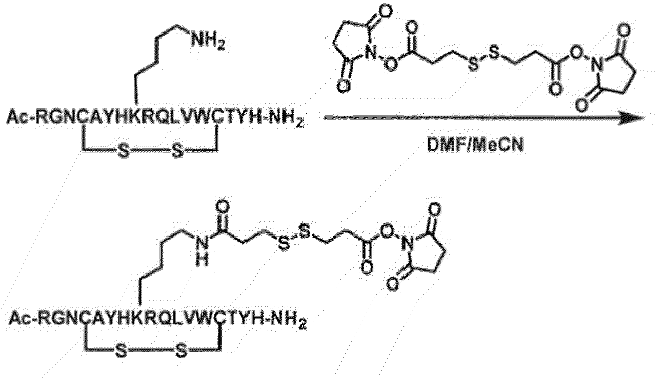

The amino acid sequence is SEQ ID NO: 45.

Ac-RGNCAYHKRQLVWCTYH-NH₂ (SEQ ID NO: 45) synthesized in (42-2) (29.6 mg, 13.6 µmol, the 4th and 14th of two cysteines form a disulfide bond in the molecule) was dissolved in N,N'-dimethylformamide (0.50 mL), a solution dissolving 3,3'-dithiodipropionic acid di(N-succinimidyl) (55.0 mg, 136 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.00 mg, 2.44 µmol).

MS(ESI)m/z:z=4 616.85[M+4H]⁴⁺
HPLC purity: 100%

### (42-4) Specific Modification of Anti-HER2 IgG antibody Trastuzumab and Analysis by ESI-TOFMS

The peptide- and disulfide linker-coupled NHS-activation compound synthesized in (42-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150577, and a peak for a product with two binding peptides introduced was observed at 152923.

### (42-5) Determination of Heavy Chain Selectivity of Specifically Modified Compound of Trastuzumab under Reduction Conditions by ESI-TOFMS Analysis

To the antibody-peptide conjugate formed in (42-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (42-6) HIC-UPLC Analysis of Specific Modification of Anti-HER2 IgG antibody Trastuzumab

The antibody-peptide conjugate formed in (42-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

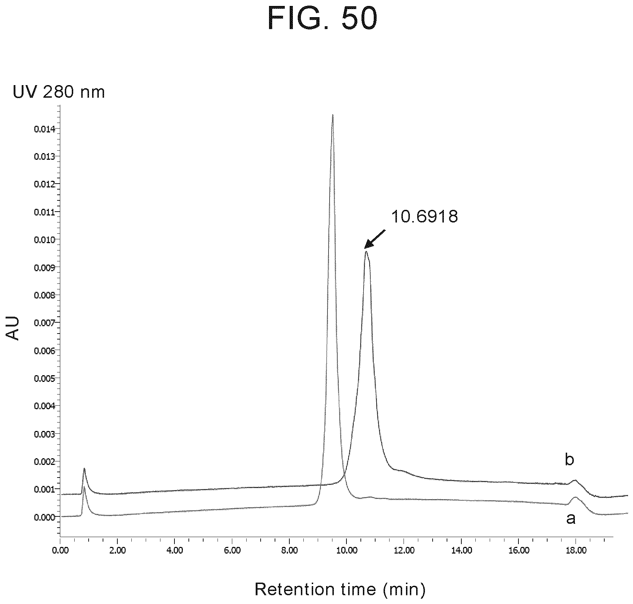

It is believed that a retension time of 10.6918 minutes is attributed to a compound with two peptides introduced to trastuzumab (FIG. 50).

### (42-7) Linker Cleavage and Reoxidation of Trastuzumab-Peptide Conjugate

By performing linker cleavage and reoxidation of the antibody-peptide conjugate obtained in (42-4) by the method described in (3-1) of Example 3, it is possible to obtain an antibody in which thiopropionyl groups are regioselectively introduced to Lys246 and Lys248 in EU numbering.

### [Example 43: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (43-1) Synthesis of IgG1 Fc-Binding Peptide

The peptide of Ac-RGNCAYHKHQLVWCTYH-NH₂ (SEQ ID NO: 46) was synthesized and purified in a similar method to (1-1) of Example 1 to obtain a target product (44.3 mg, 20.5 µmol) .

MS(ESI)m/z:z=3 720.05[M+3H]³⁺,z=4 540.30[M+4H]⁴⁺

### (43-2) Formation of Intra-Molecular Disulfide Bond between Cys at Positions 4 and 14 of Ac-RGNCAYHKHQLVWCTYH-NH₂ (SEQ ID NO: 46)

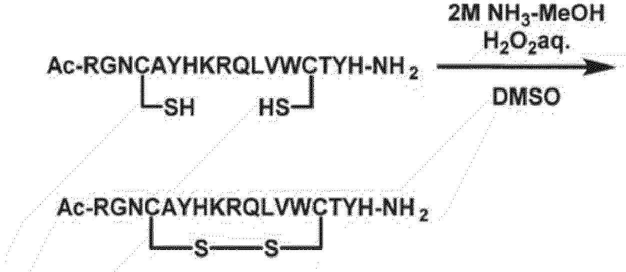

The amino acid sequence is SEQ ID NO: 46.

The peptide (44.3 mg, 20.5 µmol) synthesized in (43-1) was dissolved in DMSO (5.00 mL), 2 M NH₃-MeOH (20.5 µL, 41.0 µmol) and a hydrogen peroxide solution (41.9 µL, 410 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (26.0 mg, 12.0 µmol).

MS(ESI)m/z:z=3 719.60[M+3H]³⁺, z=4 540.10[M+4H]⁴⁺

### (43-3) Coupling between Disulfide Linker and Peptide

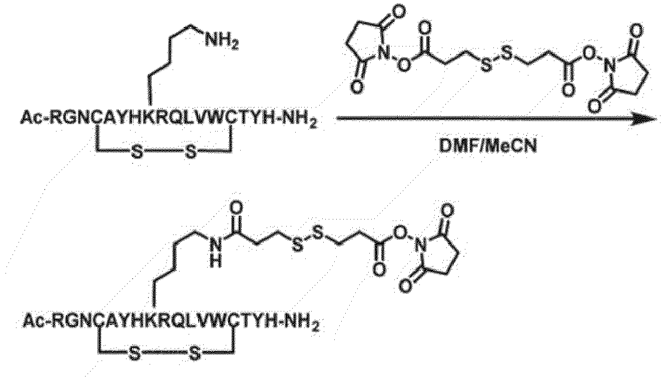

The amino acid sequence is SEQ ID NO: 46.

Ac-RGNCAYHKHQLVWCTYH-NH₂ (SEQ ID NO: 46) synthesized in (43-2) (26.0 mg, 12.0 µmol, the 4th and 14th of two cysteines form a disulfide bond in the molecule) was dissolved in N,N'-dimethylformamide (0.50 mL), a solution dissolving 3,3'-dithiodipropionic acid di(N-succinimidyl) (49.0 mg, 120 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (5.00 mg, 2.05 µmol).

MS(ESI)m/z:z=4 612.15[M+4H]⁴⁺
HPLC purity: 69%

### (43-4) Specific Modification of Anti-HER2 IgG antibody Trastuzumab and Analysis by ESI-TOFMS

The peptide- and disulfide linker-coupled NHS-activation compound synthesized in (43-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150549, and a peak for a product with two binding peptides introduced was observed at 152879.

### (43-5) Determination of Heavy Chain Selectivity of Specifically Modified Compound of Trastuzumab under Reduction Conditions by ESI-TOFMS Analysis

To the antibody-peptide conjugate formed in (43-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (43-6) HIC-UPLC Analysis of Specific Modification of Anti-HER2 IgG antibody Trastuzumab

The antibody-peptide conjugate formed in (43-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

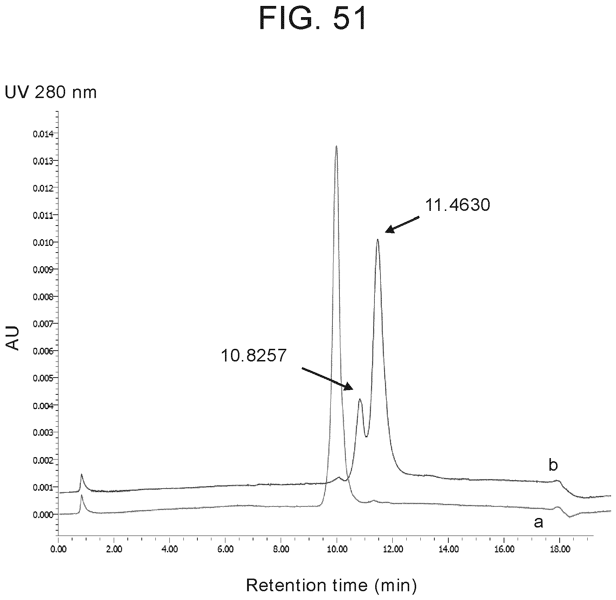

It is believed that a retension time of 10.8257 minutes is attributed to a compound with one peptide introduced to trastuzumab, whereas that of 11.4630 minutes is attributed to a compound with two peptides introduced to trastuzumab (FIG. 51).

### (43-7) Linker Cleavage and Reoxidation of Trastuzumab-Peptide Conjugate

By performing linker cleavage and reoxidation of the antibody-peptide conjugate obtained in (43-4) by the method described in (3-1) of Example 3, it is possible to obtain an antibody in which thiopropionyl groups are regioselectively introduced to Lys246 and Lys248 in EU numbering.

### [Example 44: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (44-1) Synthesis of IgG1 Fc-Binding Peptide

The peptide of Ac-RGNCAYHKWQLVWCTYH-NH₂ (SEQ ID NO: 47) was synthesized and purified in a similar method to (1-1) of Example 1 to obtain a target product (29.0 mg, 13.1 µmol) .

MS(ESI)m/z:z=3 736.25[M+3H]³⁺,z=4 552.65[M+4H]⁴⁺

### (44-2) Formation of Intra-Molecular Disulfide Bond between Cys at Positions 4 and 14 of Ac-RGNCAYHKWQLVWCTYH-NH₂ (SEQ ID NO: 47)

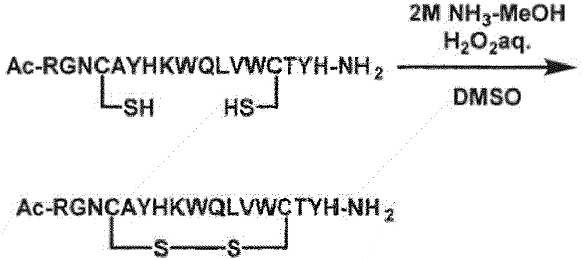

The amino acid sequence is SEQ ID NO: 47.

The peptide (29.0 mg, 13.1 µmol) synthesized in (44-1) was dissolved in DMSO (5.00 mL), 2 M NH₃-MeOH (13.1 µL, 26.2 µmol) and a hydrogen peroxide solution (26.8 µL, 262 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (11.5 mg, 5.22 µmol).

MS(ESI)m/z:z=3 735.80[M+3H]³⁺, z=4 552.10[M+4H]⁴⁺

### (44-3) Coupling between Disulfide Linker and Peptide

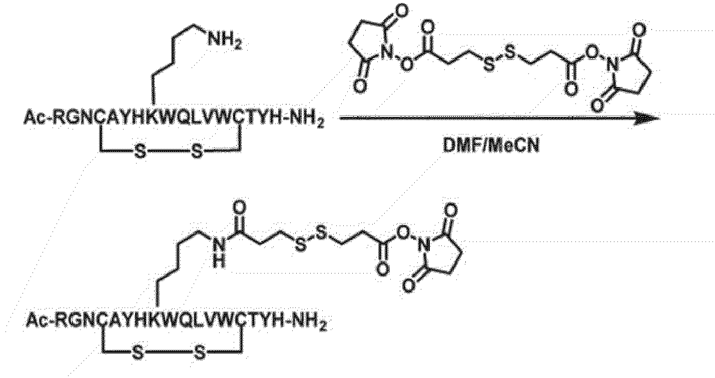

The amino acid sequence is SEQ ID NO: 47.

Ac-RGNCAYHKWQLVWCTYH-NH₂ (SEQ ID NO: 47) synthesized in (44-2) (11.5 mg, 5.22 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (21.0 mg, 52.0 µmol) in N,N'-dimethylformamide (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.00 mg, 1.20 µmol).

MS(ESI)m/z:z=3 832.05[M+3H]³⁺
HPLC purity: 93%

### (44-4) Specific Modification of Anti-HER2 IgG antibody Trastuzumab and Analysis by ESI-TOFMS

The peptide- and disulfide linker-coupled NHS-activation compound synthesized in (44-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 15.0595, and a peak for a product with two binding peptides introduced was observed at 15.3133.

### (44-5) Determination of Heavy Chain Selectivity of Specifically Modified Compound of Trastuzumab under Reduction Conditions by ESI-TOFMS Analysis

To the antibody-peptide conjugate formed in (44-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (44-6) HIC-UPLC Analysis of Specific Modification of Anti-HER2 IgG antibody Trastuzumab

The antibody-peptide conjugate formed in (44-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

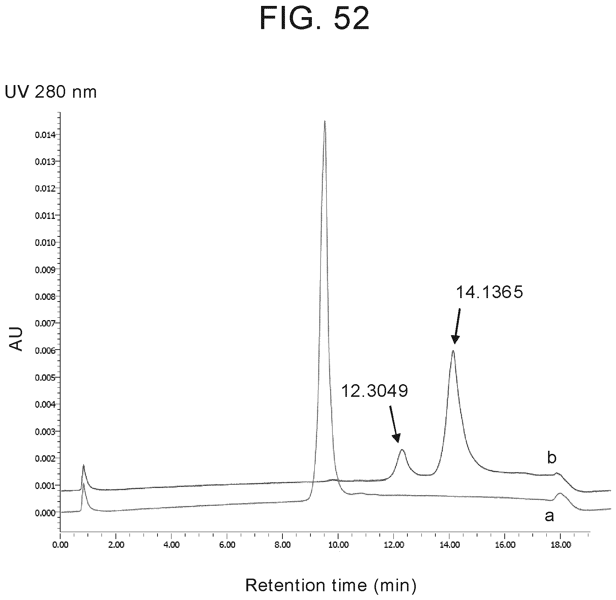

It is believed that a retension time of 12.3049 minutes is attributed to a compound with one peptide introduced to trastuzumab, whereas that of 14.1365 minutes is attributed to a compound with two peptides introduced to trastuzumab (FIG. 52).

### (44-7) Linker Cleavage and Reoxidation of Trastuzumab-Peptide Conjugate

By performing linker cleavage and reoxidation of the antibody-peptide conjugate obtained in (44-4) by the method described in (3-1) of Example 3, it is possible to obtain an antibody in which thiopropionyl groups are regioselectively introduced to Lys246 and Lys248 in EU numbering.

### [Example 45: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (45-1) Synthesis of IgG1 Fc-Binding Peptide

The peptide of Ac-RGNCAYHKYQLVWCTYH-NH₂ (SEQ ID NO: 48) was synthesized and purified in a similar method to (1-1) of Example 1 to obtain a target product (25.5 mg, 11.7 µmol) .

MS(ESI)m/z:z=3 728.65[M+3H]³⁺,z=4 546.85[M+4H]⁴⁺

### (45-2) Formation of Intra-Molecular Disulfide Bond between Cys at Positions 4 and 14 of Ac-RGNCAYHKYQLVWCTYH-NH₂ (SEQ ID NO: 48)

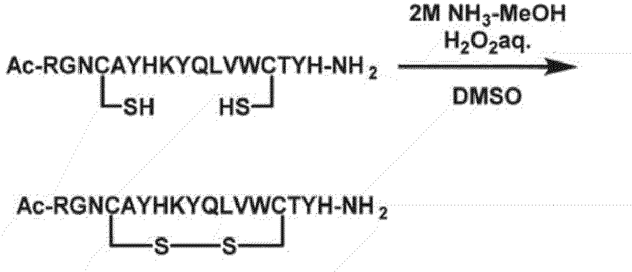

The amino acid sequence is SEQ ID NO: 48.

The peptide (25.5 mg, 11.7 µmol) synthesized in (45-1) was dissolved in DMSO (5.00 mL), 2 M NH₃-MeOH (11.7 µL, 23.4 µmol) and a hydrogen peroxide solution (23.9 µL, 234 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (13.0 mg, 6.00 µmol).

MS(ESI)m/z:z=3 728.05[M+3H]³⁺, z=4 546.35[M+4H]⁴⁺

### (45-3) Coupling between Disulfide Linker and Peptide

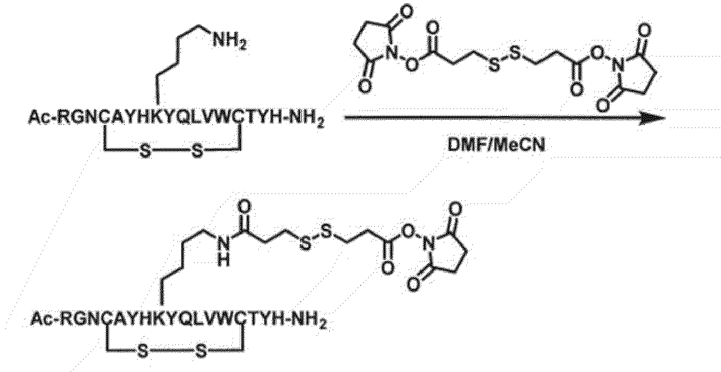

The amino acid sequence is SEQ ID NO: 48.

Ac-RGNCAYHKYQLVWCTYH-NH₂ (SEQ ID NO: 48) synthesized in (45-2) (13.0 mg, 6.00 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (24.0 mg, 60.0 µmol) in N,N'-dimethylformamide (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (3.80 mg, 1.54 µmol).

MS(ESI)m/z:z=3 824.50[M+3H]³⁺
HPLC purity: 100%

### (45-4) Specific Modification of Anti-HER2 IgG antibody Trastuzumab and Analysis by ESI-TOFMS

The peptide- and disulfide linker-coupled NHS-activation compound synthesized in (45-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150569, and a peak for a product with two binding peptides introduced was observed at 153086.

### (45-5) Determination of Heavy Chain Selectivity of Specifically Modified Compound of Trastuzumab under Reduction Conditions by ESI-TOFMS Analysis

To the antibody-peptide conjugate formed in (45-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (45-6) HIC-UPLC Analysis of Specific Modification of Anti-HER2 IgG antibody Trastuzumab

The antibody-peptide conjugate formed in (45-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

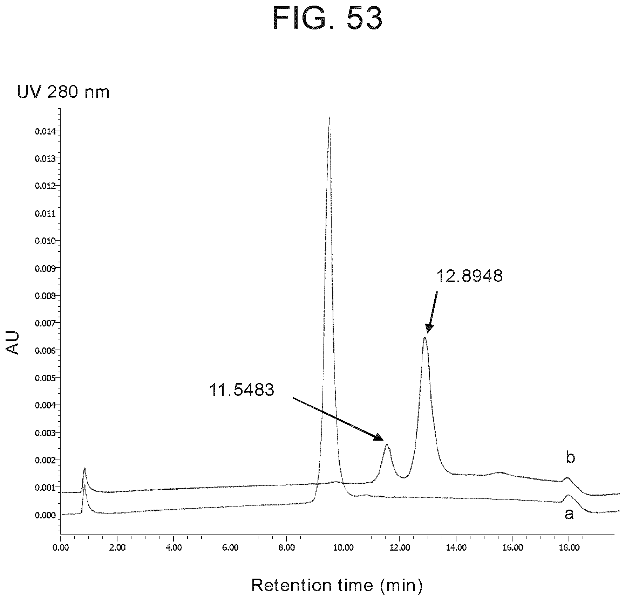

It is believed that a retension time of 11.5483 minutes is attributed to a compound with one peptide introduced to trastuzumab, whereas that of 12.8948 minutes is attributed to a compound with two peptides introduced to trastuzumab (FIG. 53).

### (45-7) Linker Cleavage and Reoxidation of Trastuzumab-Peptide Conjugate

By performing linker cleavage and reoxidation of the antibody-peptide conjugate obtained in (45-4) by the method described in (3-1) of Example 3, it is possible to obtain an antibody in which thiopropionyl groups are regioselectively introduced to Lys246 and Lys248 in EU numbering.

### [Example 46: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (46-1) Synthesis of IgG1 Fc-Binding Peptide

The peptide of Ac-RGNCAYFKGQLVWCTYH-NH₂ (SEQ ID NO: 49) was synthesized and purified in a similar method to (1-1) of Example 1 to obtain a target product (19.5 mg, 9.35 µmol) .

MS(ESI)m/z:z=3 696.70[M+3H]³⁺

### (46-2) Formation of Intra-Molecular Disulfide Bond between Cys at Positions 4 and 14 of Ac-RGNCAYFKGQLVWCTYH-NH₂ (SEQ ID NO: 49)

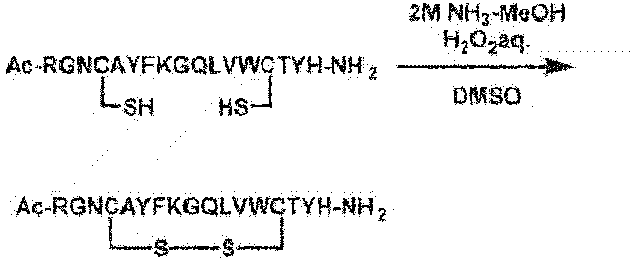

The amino acid sequence is SEQ ID NO: 49.

The peptide (19.5 mg, 9.35 µmol) synthesized in (46-1) was dissolved in DMSO (5.00 mL), 2 M NH₃-MeOH (9.35 µL, 18.7 µmol) and a hydrogen peroxide solution (19.1 µL, 187 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (9.20 mg, 4.41 µmol).

MS(ESI)m/z:z=3 696.05[M+3H]³⁺

### (46-3) Coupling between Disulfide Linker and Peptide

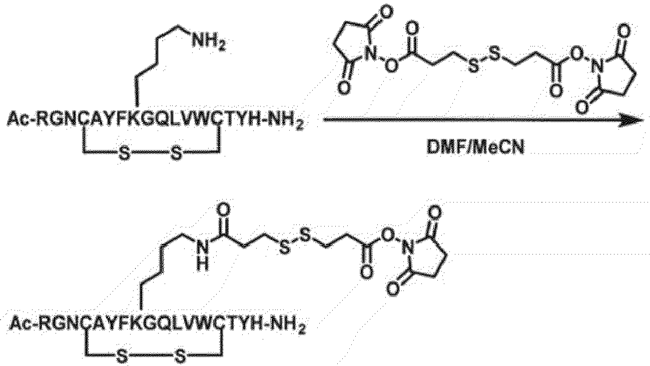

The amino acid sequence is SEQ ID NO: 49.

Ac-RGNCAYFKGQLVWCTYH-NH₂ (SEQ ID NO: 49) synthesized in (46-2) (9.20 mg, 4.41 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (71.4 mg, 176 µmol) in N,N'-dimethylformamide (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (1.50 mg, 0.63 µmol) .

MS(ESI)m/z:z=3 792.40[M+3H]³⁺
HPLC purity: 75%

### (46-4) Specific Modification of Anti-HER2 IgG antibody Trastuzumab and Analysis by ESI-TOFMS

The peptide- and disulfide linker-coupled NHS-activation compound synthesized in (46-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150661, and a peak for a product with two binding peptides introduced was observed at 153059.

### (46-5) Determination of Heavy Chain Selectivity of Specifically Modified Compound of Trastuzumab under Reduction Conditions by ESI-TOFMS Analysis

To the antibody-peptide conjugate formed in (46-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (46-6) HIC-UPLC Analysis of Specific Modification of Anti-HER2 IgG antibody Trastuzumab

The antibody-peptide conjugate formed in (46-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

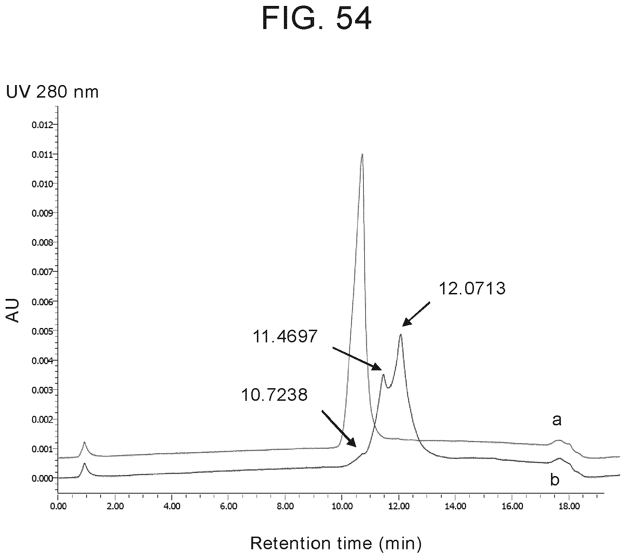

It is believed that a retension time of 10.7238 minutes is attributed to the trastuzumab raw material, whereas that of 11.4697 minutes is attributed to a compound with one peptide introduced to trastuzumab, and that of 12.0713 minutes is attributed to a compound with two peptides introduced to trastuzumab (FIG. 54).

### (46-7) Linker Cleavage and Reoxidation of Trastuzumab-Peptide Conjugate

By performing linker cleavage and reoxidation of the antibody-peptide conjugate obtained in (46-4) by the method described in (3-1) of Example 3, it is possible to obtain an antibody in which thiopropionyl groups are regioselectively introduced to Lys246 and Lys248 in EU numbering.

### [Example 47: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (47-1) Synthesis of IgG1 Fc-Binding Peptide

The peptide of Ac-RGNCAYYKGQLVWCTYH-NH₂ (SEQ ID NO: 50) was synthesized and purified in a similar method to (1-1) of Example 1 to obtain a target product (29.6 mg, 14.1 µmol) .

MS(ESI)m/z:z=3 702.05[M+3H]³⁺

### (47-2) Formation of Intra-Molecular Disulfide Bond between Cys at Positions 4 and 14 of Ac-RGNCAYYKGQLVWCTYH-NH₂ (SEQ ID NO: 50)

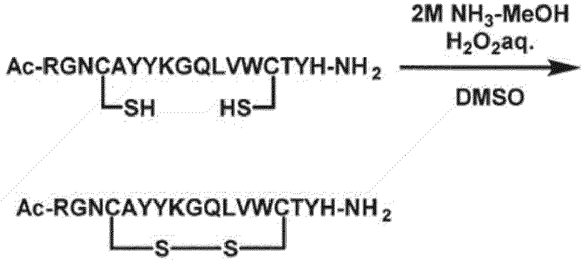

The amino acid sequence is SEQ ID NO: 50.

The peptide (29.6 mg, 14.1 µmol) synthesized in (47-1) was dissolved in DMSO (5.00 mL), 2 M NH₃-MeOH (14.1 µL, 28.2 µmol) and a hydrogen peroxide solution (28.8 µL, 282 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (12.1 mg, 5.76 µmol) .

MS(ESI)m/z:z=3 701.40[M+3H]³⁺

### (47-3) Coupling between Disulfide Linker and Peptide

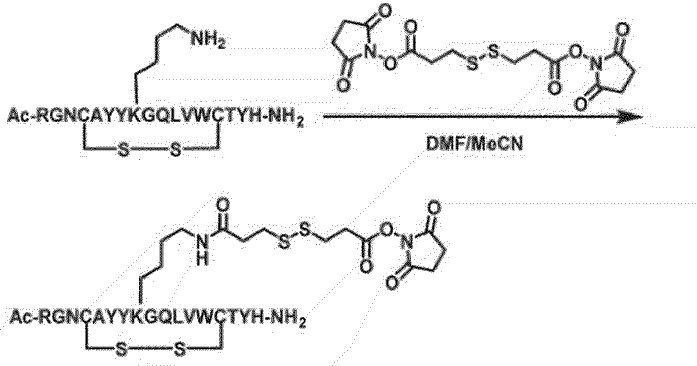

The amino acid sequence is SEQ ID NO: 50.

Ac-RGNCAYYKGQLVWCTYH-NH₂ (SEQ ID NO: 50) synthesized in (47-2) (12.1 mg, 5.76 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (70.0 mg, 173 µmol) in N,N'-dimethylformamide (0.40 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.70 mg, 1.13 µmol).

MS(ESI)m/z:z=3 797.65[M+3H]³⁺
HPLC purity: 77%

### (47-4) Specific Modification of Anti-HER2 IgG antibody Trastuzumab and Analysis by ESI-TOFMS

The peptide- and disulfide linker-coupled NHS-activation compound synthesized in (47-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150497, and a peak for a product with two binding peptides introduced was observed at 152779.

### (47-5) Determination of Heavy Chain Selectivity of Specifically Modified Compound of Trastuzumab under Reduction Conditions by ESI-TOFMS Analysis

To the antibody-peptide conjugate formed in (47-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (47-6) HIC-UPLC Analysis of Specific Modification of Anti-HER2 IgG antibody Trastuzumab

The antibody-peptide conjugate formed in (47-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

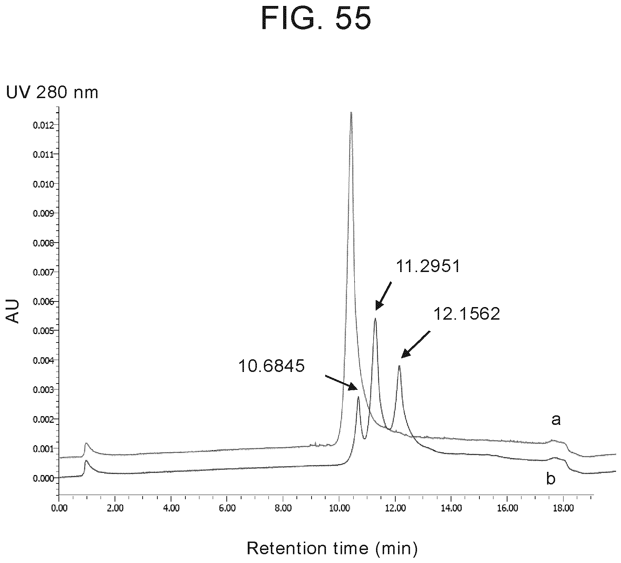

It is believed that a retension time of 10.6845 minutes is attributed to the trastuzumab raw material, whereas that of 11.2951 minutes is attributed to a compound with one peptide introduced to trastuzumab, and that of 12.1562 minutes is attributed to a compound with two peptides introduced to trastuzumab (FIG. 55).

### (47-7) Linker Cleavage and Reoxidation of Trastuzumab-Peptide Conjugate

By performing linker cleavage and reoxidation of the antibody-peptide conjugate obtained in (47-4) by the method described in (3-1) of Example 3, it is possible to obtain an antibody in which thiopropionyl groups are regioselectively introduced to Lys246 and Lys248 in EU numbering.

### [Example 48: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (48-1) Synthesis of IgG1 Fc-Binding Peptide

The peptide of Ac-RGNCAYWKGQLVWCTYH-NH₂ (SEQ ID NO: 51) was synthesized and purified in a similar method to (1-1) of Example 1 to obtain a target product (36.5 mg, 17.2 µmol) .

MS(ESI)m/z:z=3 709.65[M+3H]³⁺

### (48-2) Formation of Intra-Molecular Disulfide Bond between Cys at Positions 4 and 14 of Ac-RGNCAYWKGQLVWCTYH-NH₂ (SEQ ID NO: 51)

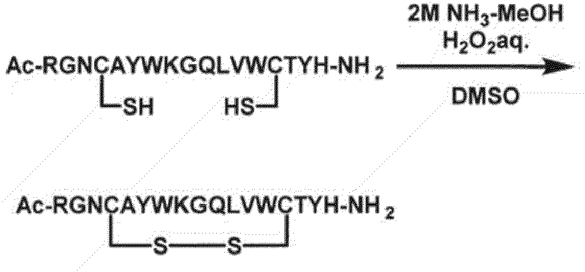

The amino acid sequence is SEQ ID NO: 51.

The peptide (36.5 mg, 17.2 µmol) synthesized in (48-1) was dissolved in DMSO (5.00 mL), 2 M NH₃-MeOH (17.2 µL, 34.4 µmol) and a hydrogen peroxide solution (35.1 µL, 344 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (15.9 mg, 7.48 µmol) .

MS(ESI)m/z:z=3 805.60[M+3H]³⁺

### (48-3) Coupling between Disulfide Linker and Peptide

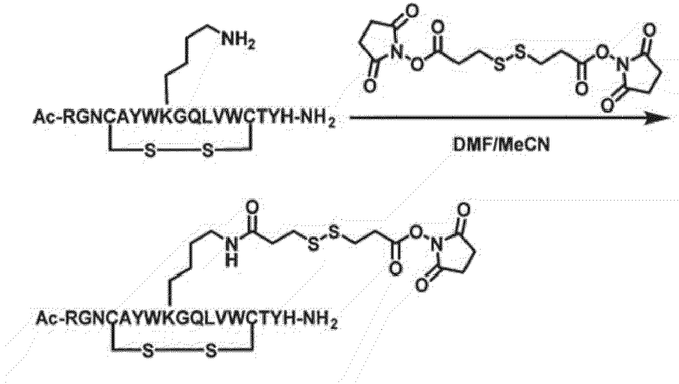

The amino acid sequence is SEQ ID NO: 51.

Ac-RGNCAYWKGQLVWCTYH-NH₂ (SEQ ID NO: 51) synthesized in (48-2) (15.9 mg, 7.48 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (60.7 mg, 150 µmol) in N,N'-dimethylformamide (0.60 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze- dried to obtain the peptide- and disulfide linker-coupled NHS-activation compound (6.0mg, 2.49 µmol).

MS(ESI)m/z:z=3 795.35[M+3H]³⁺
HPLC purity: 97%

### (48-4) Specific Modification of Anti-HER2 IgG antibody Trastuzumab and Analysis by ESI-TOFMS

The peptide- and disulfide linker-coupled NHS-activation compound synthesized in (48-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 150529, and a peak for a product with two binding peptides introduced was observed at 152819.

### (48-5) Determination of Heavy Chain Selectivity of Specifically Modified Compound of Trastuzumab under Reduction Conditions by ESI-TOFMS Analysis

To the antibody-peptide conjugate formed in (48-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (48-6) HIC-UPLC Analysis of Specific Modification of Anti-HER2 IgG antibody Trastuzumab

The antibody-peptide conjugate formed in (48-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

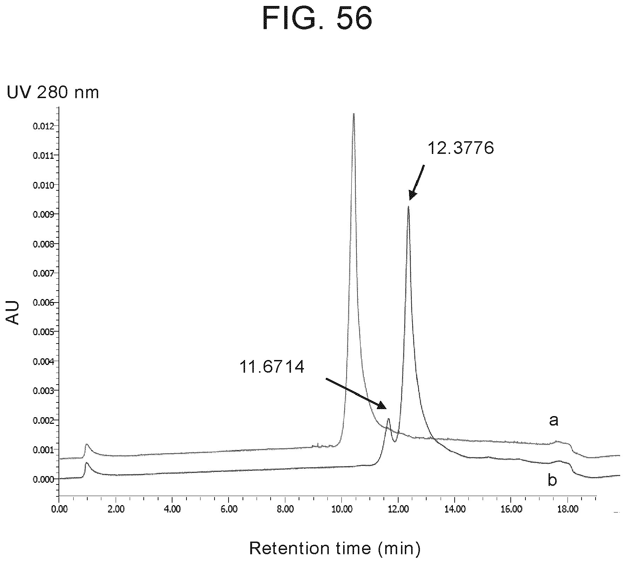

It is believed that a retension time of 11.6714 minutes is attributed to a compound with one peptide introduced to trastuzumab, whereas that of 12.3776 minutes is attributed to a compound with two peptides introduced to trastuzumab (FIG. 56).

### (48-7) Linker Cleavage and Reoxidation of Trastuzumab-Peptide Conjugate

By performing linker cleavage and reoxidation of the antibody-peptide conjugate obtained in (48-4) by the method described in (3-1) of Example 3, it is possible to obtain an antibody in which thiopropionyl groups are regioselectively introduced to Lys246 and Lys248 in EU numbering.

### [Example 49: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (49-1) Synthesis of IgG1 Fc-Binding Peptide

The peptide of Ac-RGNCAYRKGQLVWCTYH-NH₂ (SEQ ID NO: 52) was synthesized and purified in a similar method to (1-1) of Example 1 to obtain a target product (46.6 mg, 22.2 µmol) .

MS(ESI)m/z:z=3 699.65[M+3H]³⁺, z=4 525.15[M+4H]⁴⁺

### (49-2) Formation of Intra-Molecular Disulfide Bond between Cys at Positions 4 and 14 of Ac-RGNCAYRKGQLVWCTYH-NH₂ (SEQ ID NO: 52)

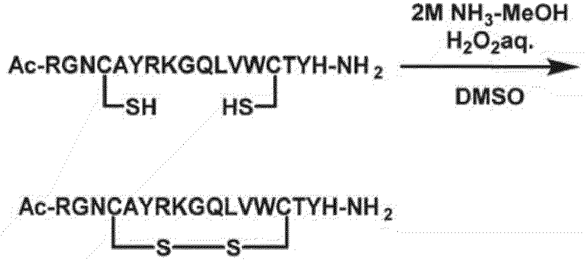

The amino acid sequence is SEQ ID NO: 52.

The peptide (46.6 mg, 22.2 µmol) synthesized in (49-1) was dissolved in DMSO (5.00 mL), 2 M NH₃-MeOH (22.2 µL, 44.4 µmol) and a hydrogen peroxide solution (45.4 µL, 444 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (32.2 mg, 15.4 µmol).

MS(ESI)m/z:z=3 699.00[M+3H]³⁺, z=4 524.50[M+4H]⁴⁺

### (49-3) Coupling between Disulfide Linker and Peptide

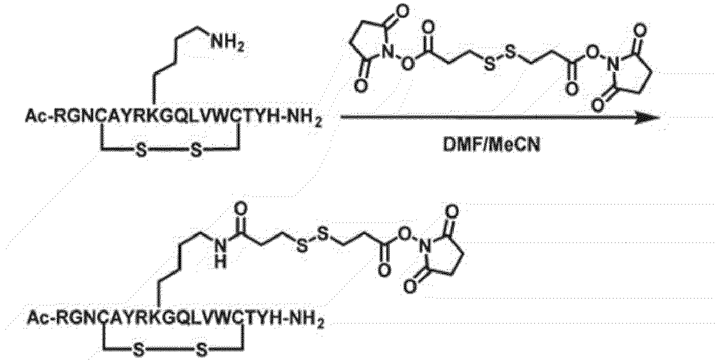

The amino acid sequence is SEQ ID NO: 52.

Ac-RGNCAYRKGQLVWCTYH-NH₂ (SEQ ID NO: 52) synthesized in (49-2) (32.2 mg, 15.4 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (125 mg, 308 µmol) in N,N'-dimethylformamide (0.60 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (10.2 mg, 4.28 µmol).

MS(ESI)m/z:z=3 795.35[M+3H]³⁺
HPLC purity: 97%

### (49-4) Specific Modification of Anti-HER2 IgG antibody Trastuzumab and Analysis by ESI-TOFMS

The peptide- and disulfide linker-coupled NHS-activation compound synthesized in (49-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148222, whereas a peak for a product with one binding peptide introduced was observed at 150492, and a peak for a product with two binding peptides introduced was observed at 152761.

### (49-5) Determination of Heavy Chain Selectivity of Specifically Modified Compound of Trastuzumab under Reduction Conditions by ESI-TOFMS Analysis

To the antibody-peptide conjugate formed in (49-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (49-6) HIC-UPLC Analysis of Specific Modification of Anti-HER2 IgG antibody Trastuzumab

The antibody-peptide conjugate formed in (49-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

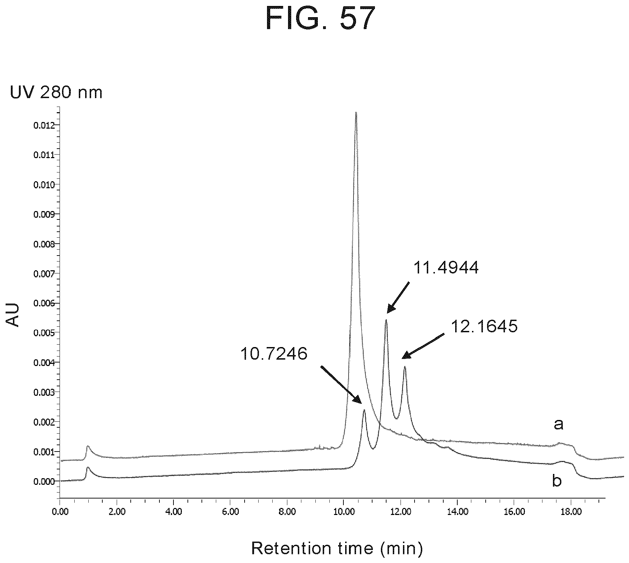

It is believed that a retension time of 10.7246 minutes is attributed to the trastuzumab raw material, whereas that of 11.4944 minutes is attributed to a compound with one peptide introduced to trastuzumab, and that of 12.1645 minutes is attributed to a compound with two peptides introduced to trastuzumab (FIG. 57).

### (49-7) Linker Cleavage and Reoxidation of Trastuzumab-Peptide Conjugate

By performing linker cleavage and reoxidation of the antibody-peptide conjugate obtained in (49-4) by the method described in (3-1) of Example 3, it is possible to obtain an antibody in which thiopropionyl groups are regioselectively introduced to Lys246 and Lys248 in EU numbering.

### [Example 50: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (50-1) Synthesis of IgG1 Fc-Binding Peptide

The peptide of Ac-RGNCAYGKGQLVWCTYH-NH₂ (SEQ ID NO: 53) was synthesized and purified in a similar method to (1-1) of Example 1 to obtain a target product (31.9 mg, 16.0 µmol) .

MS(ESI)m/z:z=3 666.70[M+3H]³⁺

### (50-2) Formation of Intra-Molecular Disulfide Bond between Cys at Positions 4 and 14 of Ac-RGNCAYGKGQLVWCTYH-NH₂ (SEQ ID NO: 53)

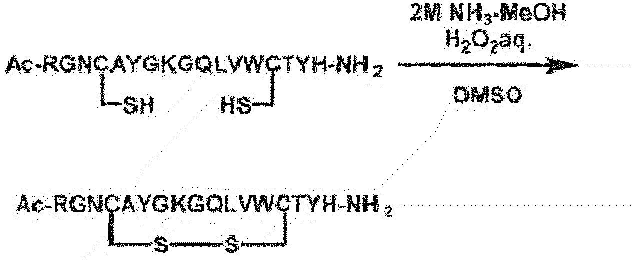

The amino acid sequence is SEQ ID NO: 53.

The peptide (31.9 mg, 16.0 µmol) synthesized in (50-1) was dissolved in DMSO (5.00 mL), 2 M NH₃-MeOH (16.0 µL, 32.0 µmol) and a hydrogen peroxide solution (32.7 µL, 320 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (17.4 mg, 8.72 µmol) .

MS(ESI)m/z:z=3 665.95[M+3H]³⁺

### (50-3) Coupling between Disulfide Linker and Peptide

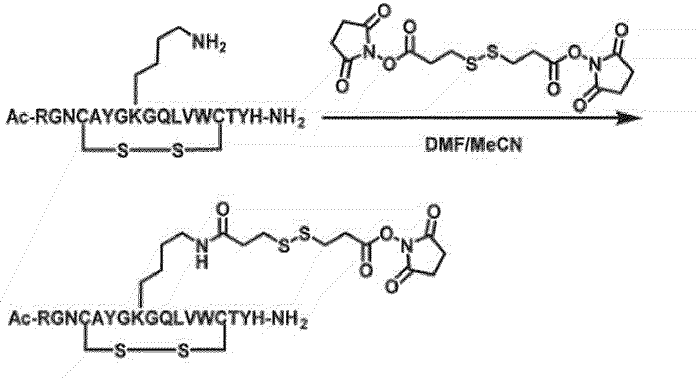

The amino acid sequence is SEQ ID NO: 53.

Ac-RGNCAYGKGQLVWCTYH-NH₂ (SEQ ID NO: 53) synthesized in (50-2) (17.4 mg, 8.72 µmol, the 4th and 14th of two cysteines form a disulfide bond in the molecule) was dissolved in N,N'-dimethylformamide (0.30 mL), a solution dissolving 3,3'-dithiodipropionic acid di(N-succinimidyl) (70.3 mg, 174 µmol) in N,N'-dimethylformamide (0.60 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (4.70 mg, 2.06 µmol).

MS(ESI)m/z:z=3 762.30[M+3H]³⁺
HPLC purity: 94%

### (50-4) Specific Modification of Anti-HER2 IgG antibody Trastuzumab and Analysis by ESI-TOFMS

The peptide- and disulfide linker-coupled NHS-activation compound synthesized in (50-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; for the raw material trastuzumab, a peak was observed at 148219, whereas a peak for a product with one binding peptide introduced was observed at 150402, and a peak for a product with two binding peptides introduced was observed at 152561.

### (50-5) Determination of Heavy Chain Selectivity of Specifically Modified Compound of Trastuzumab under Reduction Conditions by ESI-TOFMS Analysis

To the antibody-peptide conjugate formed in (50-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (50-6) HIC-UPLC Analysis of Specific Modification of Anti-HER2 IgG antibody Trastuzumab

The antibody-peptide conjugate formed in (50-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

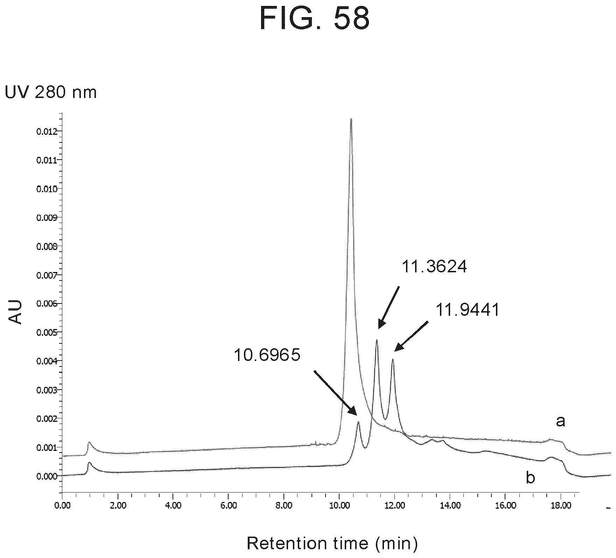

It is believed that a retension time of 10.6965 minutes is attributed to the trastuzumab raw material, whereas that of 11.3624 minutes is attributed to a compound with one peptide introduced to trastuzumab, and that of 11.9441 minutes is attributed to a compound with two peptides introduced to trastuzumab (FIG. 58).

### (50-7) Linker Cleavage and Reoxidation of Trastuzumab-Peptide Conjugate

By performing linker cleavage and reoxidation of the antibody-peptide conjugate obtained in (50-4) by the method described in (3-1) of Example 3, it is possible to obtain an antibody in which thiopropionyl groups are regioselectively introduced to Lys246 and Lys248 in EU numbering.

### [Example 51: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (51-1) Synthesis of IgG1 Fc-Binding Peptide

The peptide of Ac-DCAYHKGQLVWC-NH₂ (SEQ ID NO: 54) was synthesized and purified in a similar method to (1-1) of Example 1 to obtain a target product (76.9 mg, 52.5 µmol).

MS(ESI)m/z:z=2 732.65[M+2H]²⁺

### (51-2) Formation of Intra-Molecular Disulfide Bond between Cys at Positions 2 and 12 of Ac-DCAYHKGQLVWC-NH₂ (SEQ ID NO: 54)

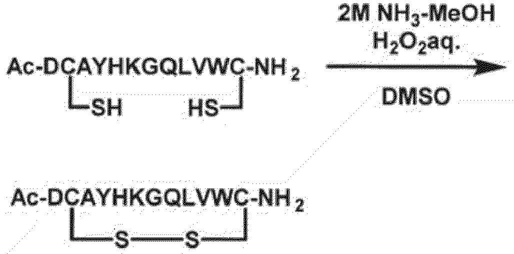

The amino acid sequence is SEQ ID NO: 54.

The peptide (76.9 mg, 52.5 µmol) synthesized in (51-1) was dissolved in DMSO (5.00 mL), 2 M NH₃-MeOH (52.5 µL, 105 µmol) and a hydrogen peroxide solution (107 µL, 1.05 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (35.9 mg, 24.6 µmol) .

MS(ESI)m/z:z=2 731.60[M+2H]²⁺

### (51-3) Coupling between Disulfide Linker and Peptide

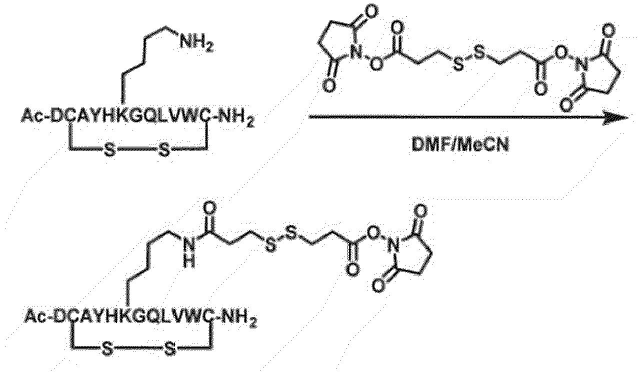

The amino acid sequence is SEQ ID NO: 54.

Ac-DCAYHKGQLVWC-NH₂ (SEQ ID NO: 54) synthesized in (51-2) (35.9 mg, 24.6 µmol, the 2nd and 12th of two cysteines form a disulfide bond in the molecule) was dissolved in N,N'-dimethylformamide (1.00 mL), a solution dissolving 3,3'-dithiodipropionic acid di(N-succinimidyl) (398 mg, 984 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (16.0 mg, 9.14 µmol).

MS(ESI)m/z:z=2 876.50[M+2H]²⁺
HPLC purity: 89%

### (51-4) Specific Modification of Anti-HER2 IgG antibody Trastuzumab and Analysis by ESI-TOFMS

The peptide- and disulfide linker-coupled NHS-activation compound synthesized in (51-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 151487.

### (51-5) Determination of Heavy Chain Selectivity of Specifically Modified Compound of Trastuzumab under Reduction Conditions by ESI-TOFMS Analysis

To the antibody-peptide conjugate formed in (51-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (51-6) HIC-UPLC Analysis of Specific Modification of Anti-HER2 IgG antibody Trastuzumab

The antibody-peptide conjugate formed in (51-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

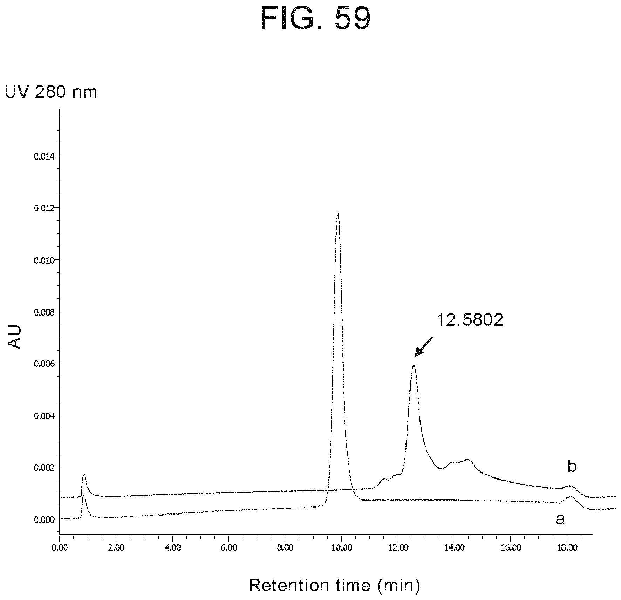

It is believed that a retension time of 12.5802 minutes is attributed to a compound with two peptides introduced to trastuzumab (FIG. 59).

### (51-7) Linker Cleavage and Reoxidation of Trastuzumab-Peptide Conjugate

By performing linker cleavage and reoxidation of the antibody-peptide conjugate obtained in (51-4) by the method described in (3-1) of Example 3, it is possible to obtain an antibody in which thiopropionyl groups are regioselectively introduced to Lys246 and Lys248 in EU numbering.

### [Example 52: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (52-1) Synthesis of IgG1 Fc-Binding Peptide

The peptide of Ac-NCAYHKGQLVWC-NH₂ (SEQ ID NO: 55) was synthesized and purified in a similar method to (1-1) of Example 1 to obtain a target product (78.6 mg, 53.7 µmοl).

MS(ESI)m/z:z=2 732.25 [M+2H]²⁺

### (52-2) Formation of Intra-Molecular Disulfide Bond between Cys at Positions 2 and 12 of Ac-NCAYHKGQLVWC-NH₂ (SEQ ID NO: 55)

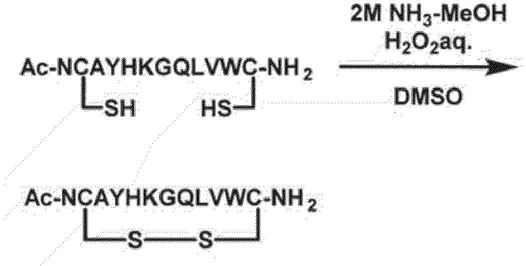

The amino acid sequence is SEQ ID NO: 55.

The peptide (78.6 mg, 53.7 µmol) synthesized in (52-1) was dissolved in DMSO (5.00 mL), 2 M NH₃-MeOH (53.7 µL, 107 µmol) and a hydrogen peroxide solution (109 µL, 1.07 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (28.0 mg, 19.2 µmοl).

MS(ESI)m/z:z=2 731.20 [M+2H]²⁺

### (52-3) Coupling between Disulfide Linker and Peptide

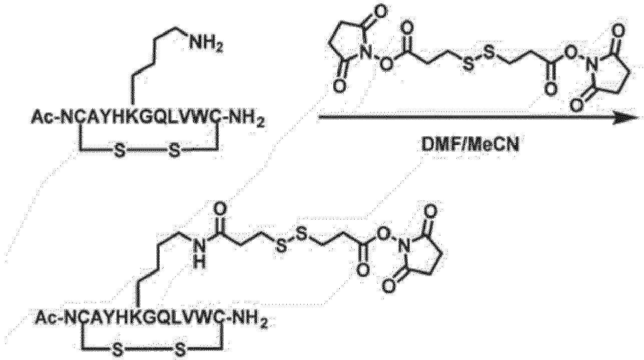

The amino acid sequence is SEQ ID NO: 55.

Ac-NCAYHKGQLVWC-NH₂ (SEQ ID NO: 55) synthesized in (52-2) (28.0 mg, 19.2 µmol, the 2nd and 12th of two cysteines form a disulfide bond in the molecule) was dissolved in N,N'-dimethylformamide (1.00 mL), a solution dissolving 3,3'-dithiodipropionic acid di(N-succinimidyl) (311 mg, 768 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (11.5 mg, 6.57 µmol) .

MS(ESI)m/z:z=2 875.75 [M+2H]²⁺
HPLC purity: 93%

### (52-4) Specific Modification of Anti-HER2 IgG antibody Trastuzumab and Analysis by ESI-TOFMS

The peptide- and disulfide linker-coupled NHS-activation compound synthesized in (52-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 151322.

### (52-5) Determination of Heavy Chain Selectivity of Specifically Modified Compound of Trastuzumab under Reduction Conditions by ESI-TOFMS Analysis

To the antibody-peptide conjugate formed in (52-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (52-6) HIC-UPLC Analysis of Specific Modification of Anti-HER2 IgG antibody Trastuzumab

The antibody-peptide conjugate formed in (52-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

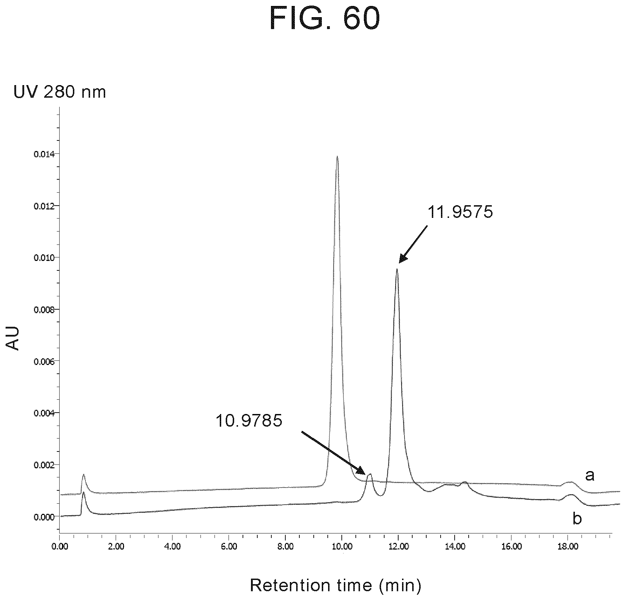

It is believed that a retension time of 10.9785 minutes is attributed to a compound with one peptide introduced to trastuzumab, whereas that of 11.9575 minutes is attributed to a compound with two peptides introduced to trastuzumab (FIG. 60).

### (52-7) Linker Cleavage and Reoxidation of Trastuzumab-Peptide Conjugate

By performing linker cleavage and reoxidation of the antibody-peptide conjugate obtained in (52-4) by the method described in (3-1) of Example 3, it is possible to obtain an antibody in which thiopropionyl groups are regioselectively introduced to Lys246 and Lys248 in EU numbering.

### [Example 53: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (53-1) Synthesis of IgG1 Fc-Binding Peptide

The peptide of Ac-CAYHKGQLVWCT-NH₂ (SEQ ID NO: 56) was synthesized and purified in a similar method to (1-1) of Example 1 to obtain a target product (63.8 mg, 44.0 µmol).

MS(ESI)m/z:z=2 725.70[M+2H]²⁺

### (53-2) Formation of Intra-Molecular Disulfide Bond between Cys at Positions 1 and 11 of Ac-CAYHKGQLVWCT-NH₂ (SEQ ID NO: 56)

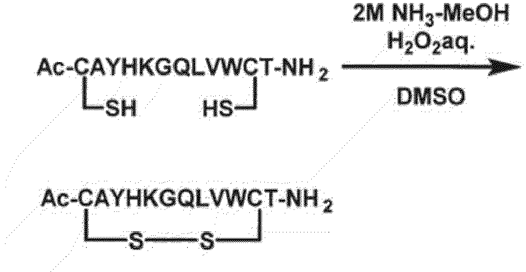

The amino acid sequence is SEQ ID NO: 56.

The peptide (63.8 mg, 44.0 µmol) synthesized in (53-1) was dissolved in DMSO (5.00 mL), 2 M NH₃-MeOH (44.0 µL, 88.0 µmol) and a hydrogen peroxide solution (89.9 µL, 880 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (24.7 mg, 17.1 µmol) .

MS(ESI)m/z:z=2 724.35[M+2H]²⁺

### (53-3) Coupling between Disulfide Linker and Peptide

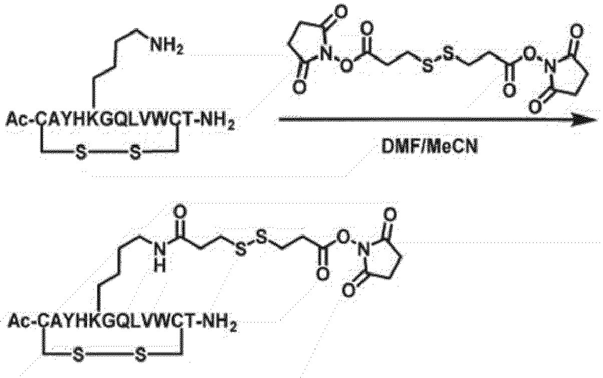

The amino acid sequence is SEQ ID NO: 56.

Ac-CAYHKGQLVWCT-NH₂ (SEQ ID NO: 56) synthesized in (53-2) (24.7 mg, 17.1 µmol, the 1st and 11th of two cysteines form a disulfide bond in the molecule) was dissolved in N,N'-dimethylformamide (1.00 mL), a solution dissolving 3,3'-dithiodipropionic acid di(N-succinimidyl) (277 mg, 684 µmol) in acetonitrile (1.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (9.00 mg, 5.18 µmol) .

MS(ESI)m/z:z=2 869.30 [M+2H]²⁺
HPLC purity: 77%

### (53-4) Specific Modification of Anti-HER2 IgG antibody Trastuzumab and Analysis by ESI-TOFMS

The peptide- and disulfide linker-coupled NHS-activation compound synthesized in (53-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS, and a peak for a product with two binding peptides introduced was observed at 151305.

### (53-5) Determination of Heavy Chain Selectivity of Specifically Modified Compound of Trastuzumab under Reduction Conditions by ESI-TOFMS Analysis

To the antibody-peptide conjugate formed in (53-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (53-6) HIC-UPLC Analysis of Specific Modification of Anti-HER2 IgG antibody Trastuzumab

The antibody-peptide conjugate formed in (53-5) and the raw material antibody were analyzed by HIC. For a column, Protein-Pak Hi Res HIC Column (Waters) 4.6 × 100 mm 2.5 µm was used. Detection was performed with A Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

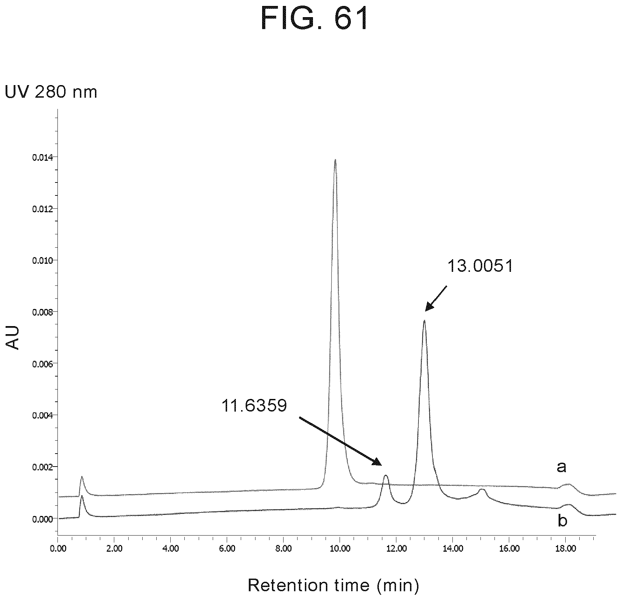

It is believed that a retension time of 11.6359 minutes is attributed to a compound with one peptide introduced to trastuzumab, whereas that of 13.0051 minutes is attributed to a compound with two peptides introduced to trastuzumab (FIG. 61).

### (53-7) Linker Cleavage and Reoxidation of Trastuzumab-Peptide Conjugate

By performing linker cleavage and reoxidation of the antibody-peptide conjugate obtained in (53-4) by the method described in (3-1) of Example 3, it is possible to obtain an antibody in which thiopropionyl groups are regioselectively introduced to Lys246 and Lys248 in EU numbering.

### [Example 54: Synthesis of Compound Having Affinity Substance to Antibody, Cleavable Portion, and Reactive Group (Peptide- and Disulfide Linker-Coupled NHS-Activation Compound), Modification of Anti-HER2 IgG Antibody Trastuzumab Using the Compound, and Analysis thereof]

### (54-1) Synthesis of IgG1 Fc-Binding Peptide

The peptide of Ac-CAYHKSQLVWC-NH₂ (SEQ ID NO: 57) was synthesized and purified in a similar method to (1-1) of Example 1 to obtain a target product (68.3 mg, 49.5 µmοl).

MS(ESI)m/z:z=2 690.20[M+2H]²⁺

### (54-2) Formation of Intra-Molecular Disulfide Bond between Cys at Positions 1 and 11 of Ac-CAYHKSQLVWC-NH₂ (SEQ ID NO: 57)

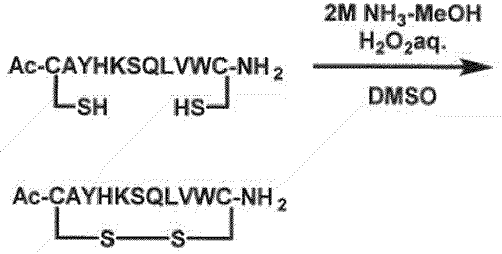

The amino acid sequence is SEQ ID NO: 57.

The peptide (68.3 mg, 49.5 µmol) synthesized in (54-1) was dissolved in DMSO (5.00 mL), 2 M NH₃-MeOH (49.5 µL, 99.0 µmol) and a hydrogen peroxide solution (101 µL, 990 mmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (30.4 mg, 22.1 µmol) .

MS(ESI)m/z:z=2 689.15[M+2H]²⁺

### (54-3) Coupling between Disulfide Linker and Peptide

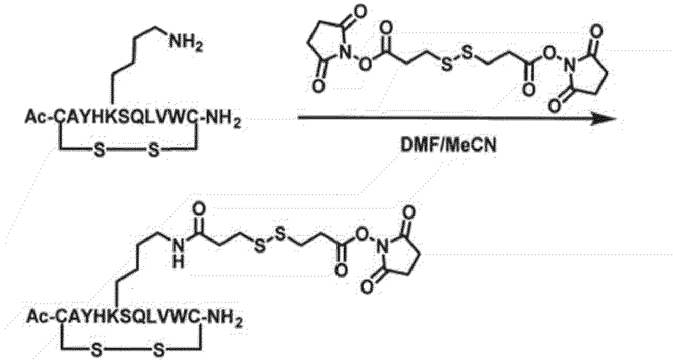

The amino acid sequence is SEQ ID NO: 57.

Ac-CAYHKSQLVWC-NH₂ (SEQ ID NO: 57) synthesized in (54-2) (30.4 mg, 22.1 µmol, the 1st and 11th of two cysteines form a disulfide bond in the molecule) was dissolved in N,N'-dimethylformamide (0.50 mL), a solution dissolving 3,3'-dithiodipropionic acid di(N-succinimidyl) (179 mg, 442 µmol) in N,N'-dimethylformamide (0.50 mL) was added thereto, and the solution was stirred at room temperature for 24 hours. Concentration under reduced pressure was performed to remove acetonitrile, which was then dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (12.0 mg, 7.20 µmol) .

MS(ESI)m/z:z=2 833.85[M+2H]²⁺
HPLC purity: 96%

### (54-4) Specific Modification of Anti-HER2 IgG antibody Trastuzumab and Analysis by ESI-TOFMS

The peptide- and disulfide linker-coupled NHS-activation compound synthesized in (54-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was added to a NAP-5 column to stop the reaction and was substituted with a 20 mM PBS buffer. The mass was measured by ESI-TOFMS; a peak for a product with one binding peptide introduced was observed at 149771, and a peak for a product with two binding peptides introduced was observed at 151326.

### (54-5) Determination of Heavy Chain Selectivity of Specifically Modified Compound of Trastuzumab under Reduction Conditions by ESI-TOFMS Analysis

To the antibody-peptide conjugate formed in (54-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50685 and 50844 with a thiopropionyl group introduced to the heavy chain, and a light chain peak was observed at 23439, the same as that of the raw material.

### (54-6) HIC-UPLC Analysis of Specific Modification of Anti-HER2 IgG antibody Trastuzumab

The antibody-peptide conjugate formed in (54-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

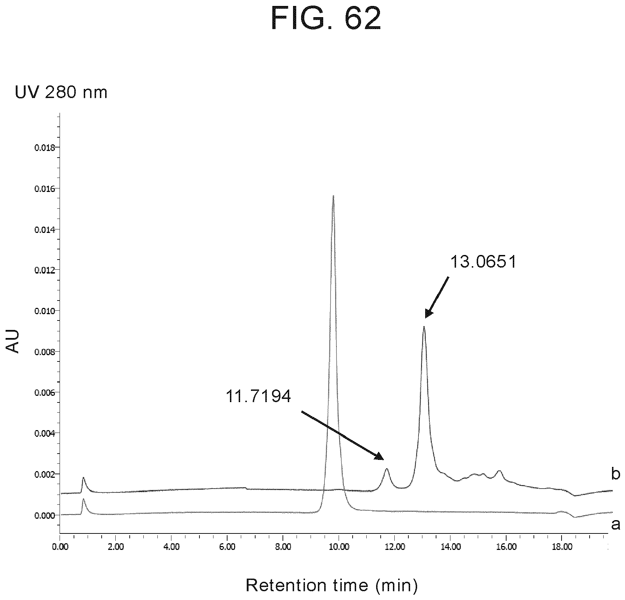

It is believed that a retension time of 11.7194 minutes is attributed to a compound with one peptide introduced to trastuzumab, whereas that of 13.0651 minutes is attributed to a compound with two peptides introduced to trastuzumab (FIG. 62).

### (54-7) Linker Cleavage and Reoxidation of Trastuzumab-Peptide Conjugate

By performing linker cleavage and reoxidation of the antibody-peptide conjugate obtained in (54-4) by the method described in (3-1) of Example 3, it is possible to obtain an antibody in which thiopropionyl groups are regioselectively introduced to Lys246 and Lys248 in EU numbering.

### [Example 55: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (55-1) Synthesis of IgG1 Fc-Binding Peptide

The peptide of Ac-RGNCAWHKGQIIWCTYH-NH₂ (SEQ ID NO: 68) was synthesized and purified in a similar method to (1-1) of Example 1 to obtain a target product (58.4 mg, 27.6 µmοl) .

MS(ESI)m/z:z=4 529.65[M+3H]³⁺

### (55-2) Formation of Intra-Molecular Disulfide Bond between Cys at Positions 4 and 14 of Ac-RGNCAWHKGQIIWCTYH-NH₂ (SEQ ID NO: 68)

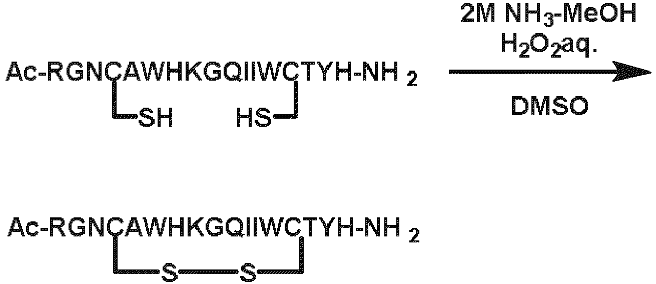

The amino acid sequence is SEQ ID NO: 68.

The peptide (58.4 mg, 27.6 µmol) synthesized in (55-1) was dissolved in DMSO (5.00 mL), 2 M NH₃-MeOH (27.6 µL, 55.2 µmol) and a hydrogen peroxide solution (56.4 µL, 552 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (29.3 mg, 13.9 µmοl).

MS(ESI)m/z:z=4 529.05[M+3H]³⁺

### (55-3) Coupling between Disulfide Linker and Peptide

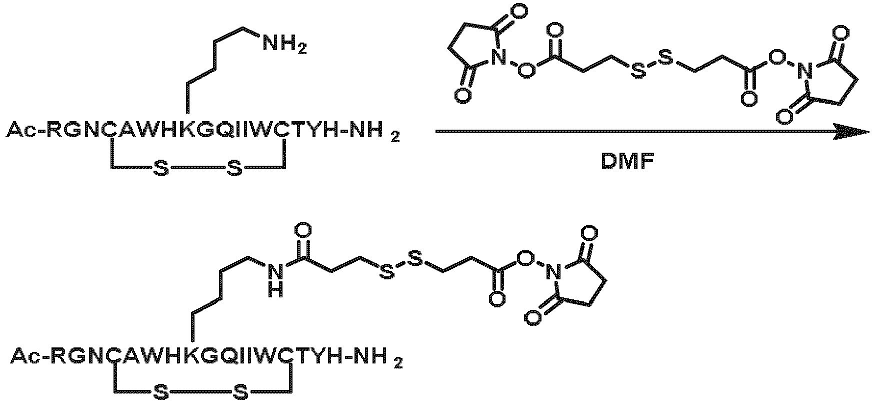

The amino acid sequence is SEQ ID NO: 68.

Ac-RGNCAWHKGQIIWCTYH-NH₂ (SEQ ID NO: 68) synthesized in (55-2) (29.3 mg, 13.9 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (112 mg, 278 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (14.5 mg, 6.04 mol).

MS(ESI)m/z:z=3 801.40[M+3H]³⁺
HPLC purity: 88%

### (55-4) Specific Modification of Anti-HER2 IgG antibody Trastuzumab and Analysis by ESI-TOFMS

The peptide- and disulfide linker-coupled NHS-activation compound synthesized in (55-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150690, and a peak for a product with two binding peptides introduced was observed at 152796.

### (55-5) Determination of Heavy Chain Selectivity of Specifically Modified Compound of Trastuzumab under Reduction Conditions by ESI-TOFMS Analysis

To the antibody-peptide conjugate formed in (55-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23439, whereas for a product, peaks were observed at 50682 and 50844 with a thiopropionyl group introduced to the heavy chain, and a light chain peak was observed at 23439, the same as that of the raw material.

### (55-6) HIC-UPLC Analysis of Specific Modification of Anti-HER2 IgG antibody Trastuzumab

The antibody-peptide conjugate formed in (55-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

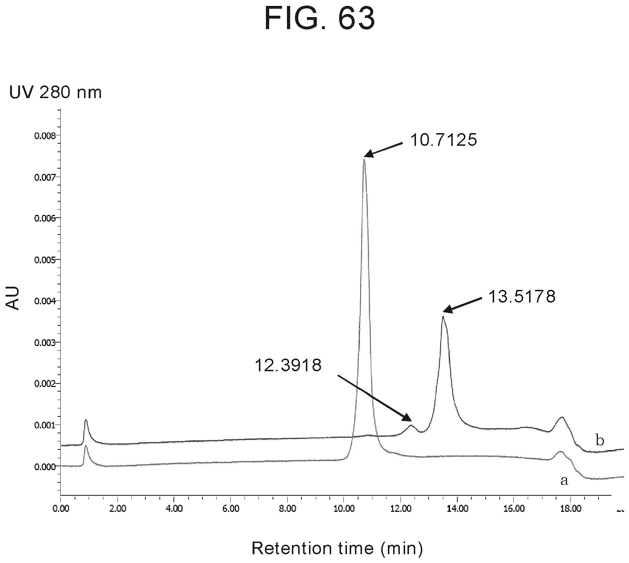

It is believed that a retension time of 12.3918 minutes is attributed to a compound with one peptide introduced to trastuzumab, whereas that of 13.5178 minutes is attributed to a compound with two peptides introduced to trastuzumab (FIG. 63).

### (55-7) Linker Cleavage and Reoxidation of Trastuzumab-Peptide Conjugate

By performing linker cleavage and reoxidation of the antibody-peptide conjugate obtained in (55-4) by the method described in (3-1) of Example 3, it is possible to obtain an antibody in which thiopropionyl groups are regioselectively introduced to Lys246 and Lys248 in EU numbering.

### [Example 56: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (56-1) Synthesis of IgG1 Fc-Binding Peptide

The peptide of Ac-RGNCAFHKGQIIWCTYH-NH₂ (SEQ ID NO: 69) was synthesized and purified in a similar method to (1-1) of Example 1 to obtain a target product (78.2 mg, 37.7 µmοl) .

MS(ESI)m/z:z=4 519.80[M+3H]³⁺

### (56-2) Formation of Intra-Molecular Disulfide Bond between Cys at Positions 4 and 14 of Ac-RGNCAFHKGQIIWCTYH-NH₂ (SEQ ID NO: 69)

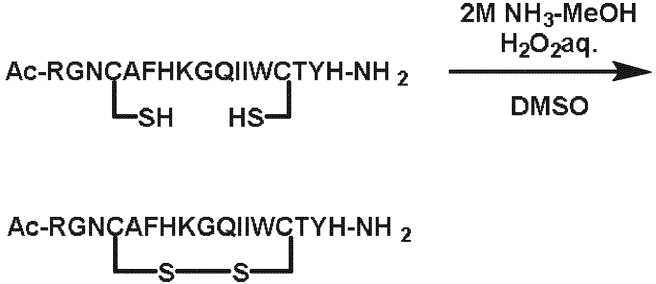

The amino acid sequence is SEQ ID NO: 69.

The peptide (78.2 mg, 37.7 µmol) synthesized in (56-1) was dissolved in DMSO (5.00 mL), 2 M NH₃-MeOH (37.7 µL, 75.4 µmol) and a hydrogen peroxide solution (77.0 µL, 754 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (46.9 mg, 22.6 µmol) .

MS(ESI)m/z:z=4 519.40[M+3H]³⁺

### (56-3) Coupling between Disulfide Linker and Peptide

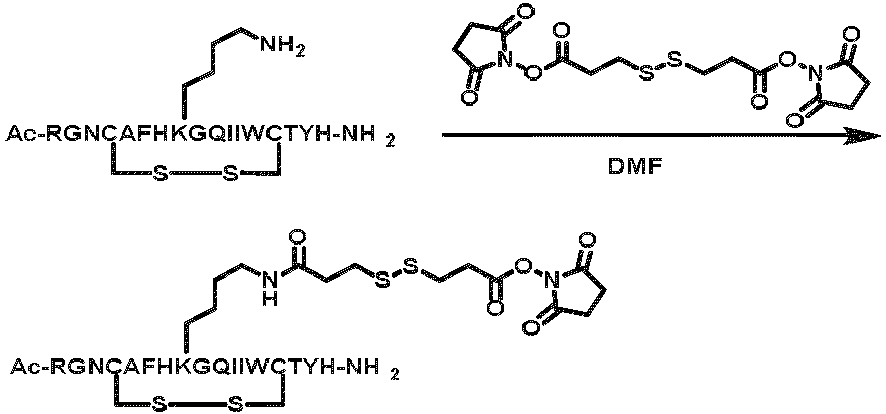

The amino acid sequence is SEQ ID NO: 69.

Ac-RGNCAFHKGQIIWCTYH-NH₂ (SEQ ID NO: 69) synthesized in (56-2) (46.9 mg, 22.6 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (183 mg, 452 µmol) in N,N'-dimethylformamide (0.80 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (18.5 mg, 7.83 mol).

MS(ESI)m/z:z=3 788.60[M+3H]³⁺
HPLC purity: 89%

### (56-4) Specific Modification of Anti-HER2 IgG antibody Trastuzumab and Analysis by ESI-TOFMS

The peptide- and disulfide linker-coupled NHS-activation compound synthesized in (56-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150368, and a peak for a product with two binding peptides introduced was observed at 152717.

### (56-5) Determination of Heavy Chain Selectivity of Specifically Modified Compound of Trastuzumab under Reduction Conditions by ESI-TOFMS Analysis

To the antibody-peptide conjugate formed in (56-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23440, whereas for a product, peaks were observed at 50682 and 50845 with a thiopropionyl group introduced to the heavy chain, and a light chain peak was observed at 23439, the same as that of the raw material.

### (56-6) HIC-UPLC Analysis of Specific Modification of Anti-HER2 IgG antibody Trastuzumab

The antibody-peptide conjugate formed in (56-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

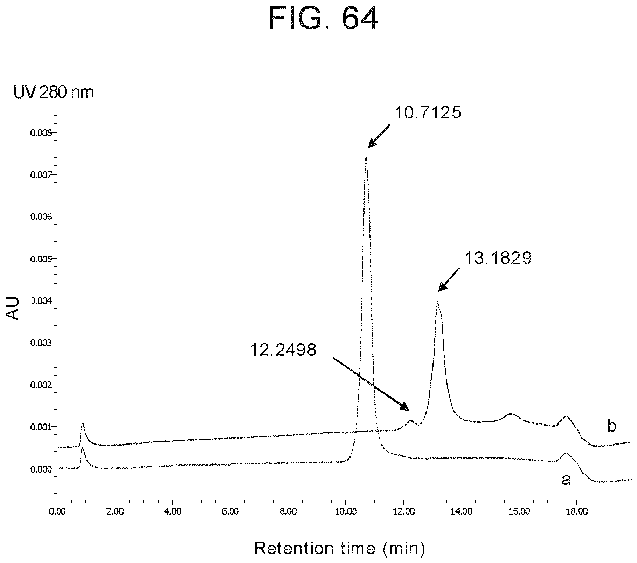

It is believed that a retension time of 12.2498 minutes is attributed to a compound with one peptide introduced to trastuzumab, whereas that of 13.1829 minutes is attributed to a compound with two peptides introduced to trastuzumab (FIG. 64).

### (56-7) Linker Cleavage and Reoxidation of Trastuzumab-Peptide Conjugate

By performing linker cleavage and reoxidation of the antibody-peptide conjugate obtained in (56-4) by the method described in (3-1) of Example 3, it is possible to obtain an antibody in which thiopropionyl groups are regioselectively introduced to Lys246 and Lys248 in EU numbering.

### [Example 57: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (57-1) Synthesis of IgG1 Fc-Binding Peptide

The peptide of Ac-RGNCAHHKGQIIWCTYH-NH₂ (SEQ ID NO: 70) was synthesized and purified in a similar method to (1-1) of Example 1 to obtain a target product (98.6 mg, 47.7 µmοl) .

MS(ESI)m/z:z=4 517.25[M+3H]³⁺

### (57-2) Formation of Intra-Molecular Disulfide Bond between Cys at Positions 4 and 14 of Ac-RGNCAHHKGQIIWCTYH-NH₂ (SEQ ID NO: 70)

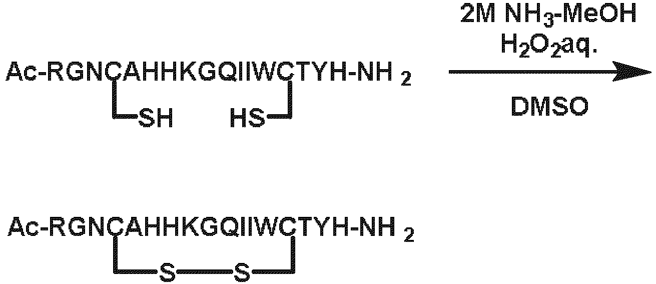

The amino acid sequence is SEQ ID NO: 70.

The peptide (98.6 mg, 47.7 µmol) synthesized in (57-1) was dissolved in DMSO (5.00 mL), 2 M NH₃-MeOH (47.7 µL, 95.4 µmol) and a hydrogen peroxide solution (97.4 µL, 954 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (90.3 mg, 43.7 µmol) .

MS(ESI)m/z:z=4 516.85[M+3H]³⁺

### (57-3) Coupling between Disulfide Linker and Peptide

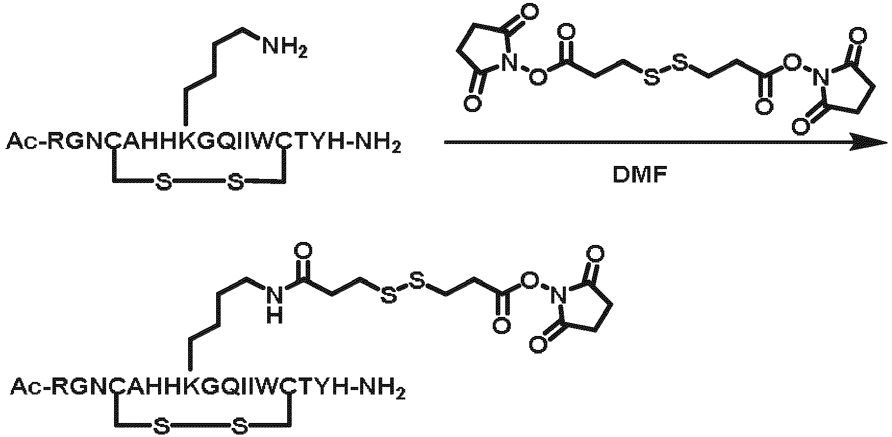

The amino acid sequence is SEQ ID NO: 70.

Ac-RGNCAHHKGQIIWCTYH-NH₂ (SEQ ID NO: 70) synthesized in (57-2) (90.3 mg, 43.7 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (353 mg, 874 µmol) in N,N'-dimethylformamide (1.20 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (22.5 mg, 9.56 mol).

MS(ESI)m/z:z=4 589.10[M+3H]³⁺
HPLC purity: 60%

### (57-4) Specific Modification of Anti-HER2 IgG antibody Trastuzumab and Analysis by ESI-TOFMS

The peptide- and disulfide linker-coupled NHS-activation compound synthesized in (57-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148236, whereas a peak for a product with one binding peptide introduced was observed at 150466, and a peak for a product with two binding peptides introduced was observed at 152702.

### (57-5) Determination of Heavy Chain Selectivity of Specifically Modified Compound of Trastuzumab under Reduction Conditions by ESI-TOFMS Analysis

To the antibody-peptide conjugate formed in (57-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23439, whereas for a product, peaks were observed at 50682 and 50844 with a thiopropionyl group introduced to the heavy chain, and a light chain peak was observed at 23439, the same as that of the raw material.

### (57-6) HIC-UPLC Analysis of Specific Modification of Anti-HER2 IgG antibody Trastuzumab

The antibody-peptide conjugate formed in (57-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

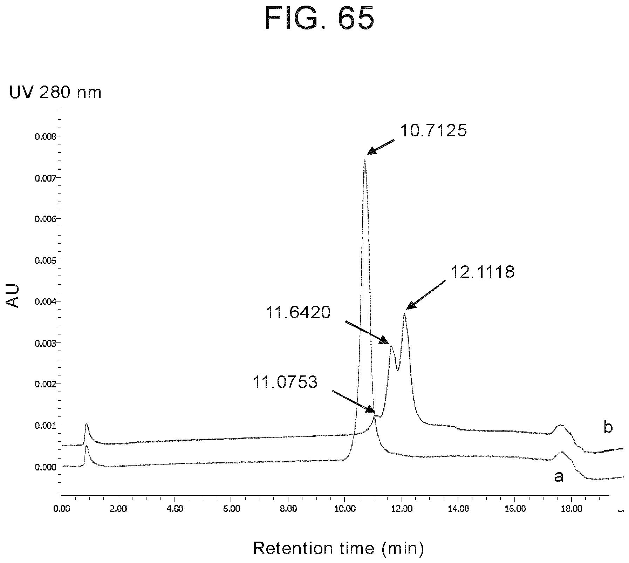

It is believed that a retension time of 11.0753 minutes is attributed to the trastuzumab raw material, whereas that of 11.6420 minutes is attributed to a compound with one peptide introduced to trastuzumab, and that of 12.1118 minutes is attributed to a compound with two peptides introduced to trastuzumab (FIG. 65).

### (57-7) Linker Cleavage and Reoxidation of Trastuzumab-Peptide Conjugate

By performing linker cleavage and reoxidation of the antibody-peptide conjugate obtained in (57-4) by the method described in (3-1) of Example 3, it is possible to obtain an antibody in which thiopropionyl groups are regioselectively introduced to Lys246 and Lys248 in EU numbering.

### [Example 58: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (58-1) Synthesis of IgG1 Fc-Binding Peptide

The peptide of Ac-RGNCGYHKGQIIWCTYH-NH₂ (SEQ ID NO: 71) was synthesized and purified in a similar method to (1-1) of Example 1 to obtain a target product (88.7 mg, 42.7 µmοl).

MS(ESI)m/z:z=4 520.25[M+3H]³⁺

### (58-2) Formation of Intra-Molecular Disulfide Bond between Cys at Positions 4 and 14 of Ac-RGNCGYHKGQIIWCTYH-NH₂ (SEQ ID NO: 71)

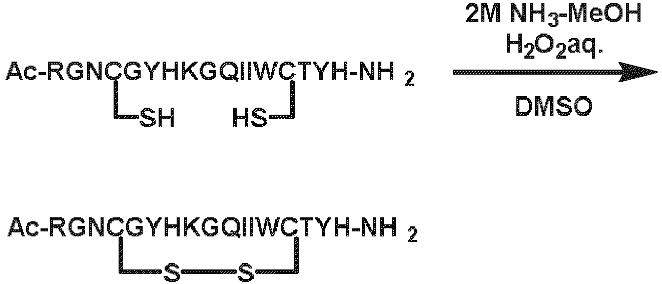

The amino acid sequence is SEQ ID NO: 71.

The peptide (88.7 mg, 42.7 µmol) synthesized in (58-1) was dissolved in DMSO (5.00 mL), 2 M NH₃-MeOH (42.7 µL, 85.4 µmol) and a hydrogen peroxide solution (87.2 µL, 854 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (66.3 mg, 31.9 µmol).

MS(ESI)m/z:z=4 519.75[M+3H]³⁺

### (58-3) Coupling between Disulfide Linker and Peptide

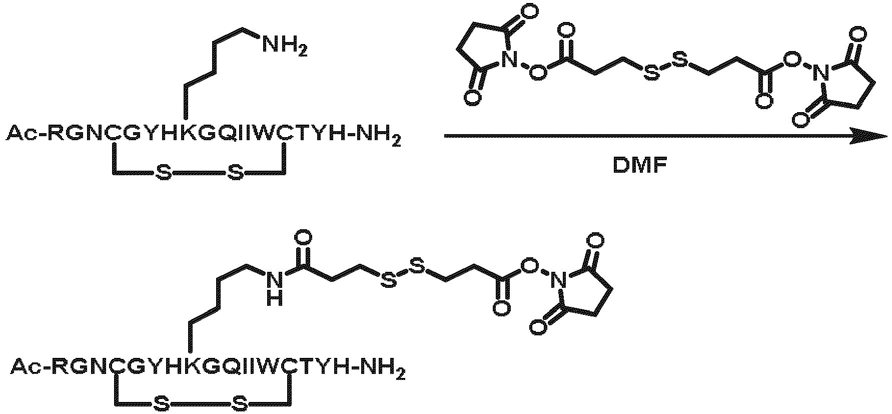

The amino acid sequence is SEQ ID NO: 71.

Ac-RGNCGYHKGQIIWCTYH-NH₂ (SEQ ID NO: 71) synthesized in (58-2) (66.3 mg, 31.9 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (258 mg, 638 µmol) in N,N'-dimethylformamide (0.80 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (24.5 mg, 10.4 mol).

MS(ESI)m/z:z=3 789.25[M+3H]³⁺
HPLC purity: 85%

### (58-4) Specific Modification of Anti-HER2 IgG antibody Trastuzumab and Analysis by ESI-TOFMS

The peptide- and disulfide linker-coupled NHS-activation compound synthesized in (58-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148229, whereas a peak for a product with one binding peptide introduced was observed at 150477, and a peak for a product with two binding peptides introduced was observed at 152892.

### (58-5) Determination of Heavy Chain Selectivity of Specifically Modified Compound of Trastuzumab under Reduction Conditions by ESI-TOFMS Analysis

To the antibody-peptide conjugate formed in (58-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50757, and a light chain peak was observed at 23439, whereas for a product, peaks were observed at 50685 and 50845 with a thiopropionyl group introduced to the heavy chain, and a light chain peak was observed at 23439, the same as that of the raw material.

### (58-6) HIC-UPLC Analysis of Specific Modification of Anti-HER2 IgG antibody Trastuzumab

The antibody-peptide conjugate formed in (58-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

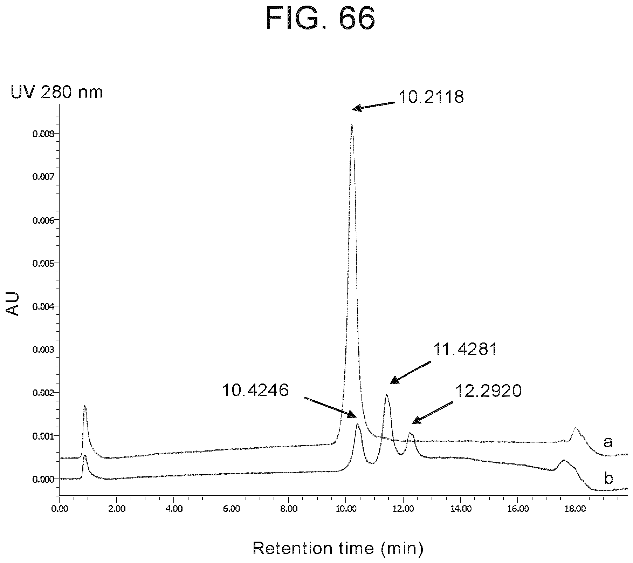

It is believed that a retension time of 10.4246 minutes is attributed to the trastuzumab raw material, whereas that of 11.4281 minutes is attributed to a compound with one peptide introduced to trastuzumab, and that of 12.2920 minutes is attributed to a compound with two peptides introduced to trastuzumab (FIG. 66).

### (58-7) Linker Cleavage and Reoxidation of Trastuzumab-Peptide Conjugate

By performing linker cleavage and reoxidation of the antibody-peptide conjugate obtained in (58-4) by the method described in (3-1) of Example 3, it is possible to obtain an antibody in which thiopropionyl groups are regioselectively introduced to Lys246 and Lys248 in EU numbering.

### [Example 59: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (59-1) Synthesis of IgG1 Fc-Binding Peptide

The peptide of Ac-RGNCLYHKGQIIWCTYH-NH₂ (SEQ ID NO: 72) was synthesized and purified in a similar method to (1-1) of Example 1 to obtain a target product (54.8 mg, 25.7 µmοl) .

MS(ESI)m/z:z=4 534.45[M+3H]³⁺

### (59-2) Formation of Intra-Molecular Disulfide Bond between Cys at Positions 4 and 14 of Ac-RGNCLYHKGQIIWCTYH-NH₂ (SEQ ID NO: 72)

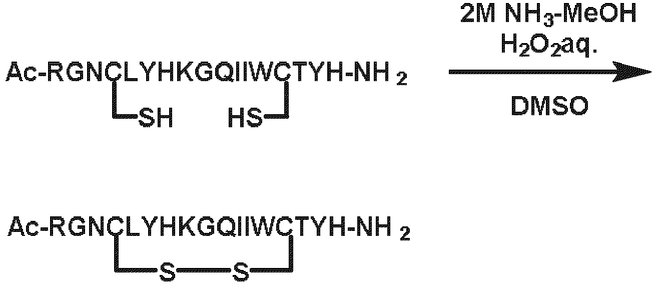

The amino acid sequence is SEQ ID NO: 72.

The peptide (54.8 mg, 25.7 µmol) synthesized in (59-1) was dissolved in DMSO (5.00 mL), 2 M NH₃-MeOH (25.7 µL, 51.4 µmol) and a hydrogen peroxide solution (52.5 µL, 514 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (27.8 mg, 13.0 µmοl).

MS(ESI)m/z:z=4 533.85[M+3H]³⁺

### (59-3) Coupling between Disulfide Linker and Peptide

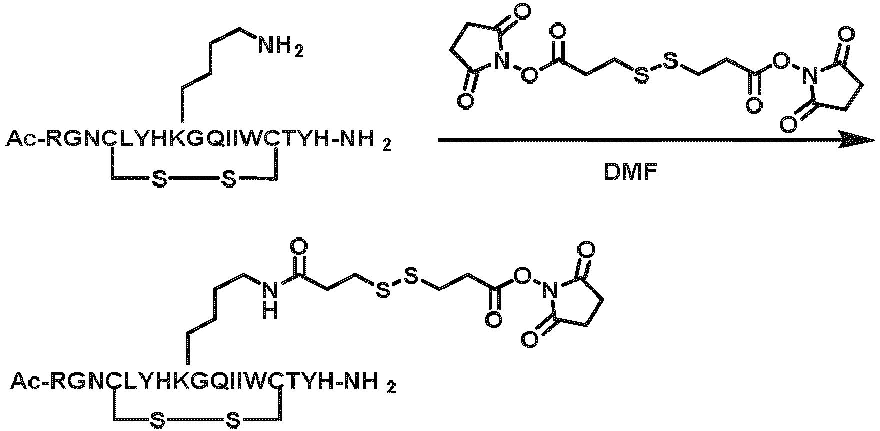

The amino acid sequence is SEQ ID NO: 72.

Ac-RGNCLYHKGQIIWCTYH-NH₂ (SEQ ID NO: 72) synthesized in (59-2) (27.8 mg, 13.0 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (105 mg, 260 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (18.8 mg, 7.77 mol).

MS(ESI)m/z:z=3 807.80[M+3H]³⁺
HPLC purity: 84%

### (59-4) Specific Modification of Anti-HER2 IgG antibody Trastuzumab and Analysis by ESI-TOFMS

The peptide- and disulfide linker-coupled NHS-activation compound synthesized in (59-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150691, and a peak for a product with two binding peptides introduced was observed at 152838.

### (59-5) Determination of Heavy Chain Selectivity of Specifically Modified Compound of Trastuzumab under Reduction Conditions by ESI-TOFMS Analysis

To the antibody-peptide conjugate formed in (59-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50757, and a light chain peak was observed at 23439, whereas for a product, peaks were observed at 50682 and 50844 with a thiopropionyl group introduced to the heavy chain, and a light chain peak was observed at 23439, the same as that of the raw material.

### (59-6) HIC-UPLC Analysis of Specific Modification of Anti-HER2 IgG antibody Trastuzumab

The antibody-peptide conjugate formed in (59-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

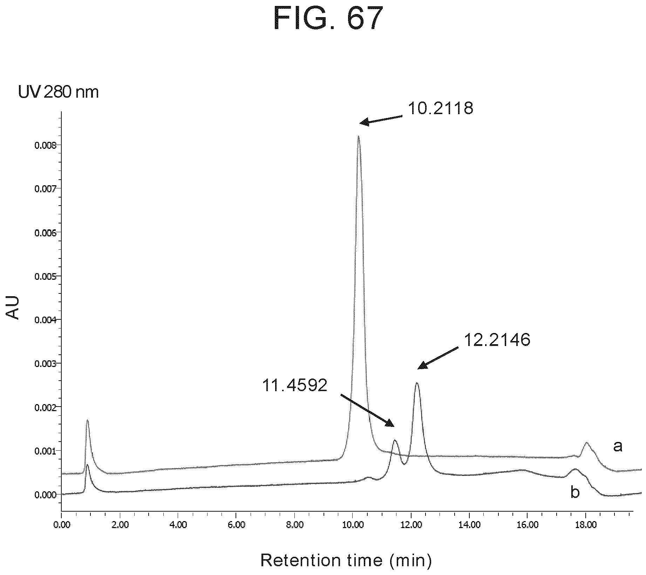

It is believed that a retension time of 11.4592 minutes is attributed to a compound with one peptide introduced to trastuzumab, whereas that of 12.2146 minutes is attributed to a compound with two peptides introduced to trastuzumab (FIG. 67).

### (59-7) Linker Cleavage and Reoxidation of Trastuzumab-Peptide Conjugate

By performing linker cleavage and reoxidation of the antibody-peptide conjugate obtained in (59-4) by the method described in (3-1) of Example 3, it is possible to obtain an antibody in which thiopropionyl groups are regioselectively introduced to Lys246 and Lys248 in EU numbering.

### [Example 60: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (60-1) Synthesis of IgG1 Fc-Binding Peptide

The peptide of Ac-RGNCPYHKGQIIWCTYH-NH₂ (SEQ ID NO: 73) was synthesized and purified in a similar method to (1-1) of Example 1 to obtain a target product (83.0 mg, 39.2 µmοl) .

MS(ESI)m/z:z=4 530.45[M+3H]³⁺

### (60-2) Formation of Intra-Molecular Disulfide Bond between Cys at Positions 4 and 14 of Ac-RGNCPYHKGQIIWCTYH-NH₂ (SEQ ID NO: 73)

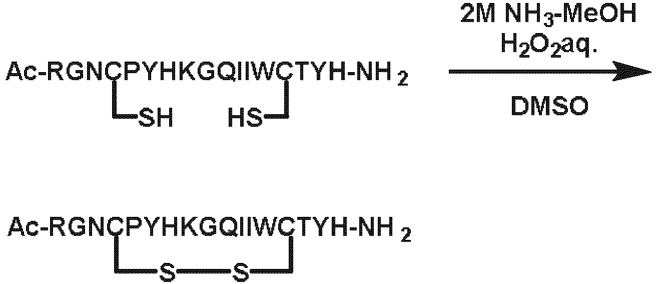

The amino acid sequence is SEQ ID NO: 73.

The peptide (83.0 mg, 39.2 µmol) synthesized in (60-1) was dissolved in DMSO (5.00 mL), 2 M NH₃-MeOH (39.2 µL, 78.4 µmol) and a hydrogen peroxide solution (80.1 µL, 784 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (49.1 mg, 23.2 µmοl).

MS(ESI)m/z:z=4 529.95[M+3H]³⁺

### (60-3) Coupling between Disulfide Linker and Peptide

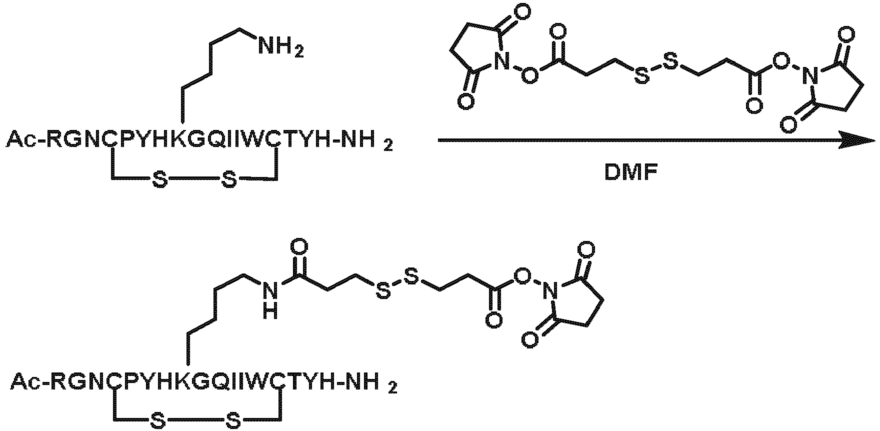

The amino acid sequence is SEQ ID NO: 73.

Ac-RGNCPYHKGQIIWCTYH-NH₂ (SEQ ID NO: 73) synthesized in (60-2) (49.1 mg, 23.2 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (188 mg, 464 µmol) in N,N'-dimethylformamide (0.80 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (19.4 mg, 8.07 mol).

MS(ESI)m/z:z=3 802.45[M+3H]³⁺
HPLC purity: 87%

### (60-4) Specific Modification of Anti-HER2 IgG antibody Trastuzumab and Analysis by ESI-TOFMS

The peptide- and disulfide linker-coupled NHS-activation compound synthesized in (60-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148225, whereas a peak for a product with one binding peptide introduced was observed at 150513, and a peak for a product with two binding peptides introduced was observed at 153398.

### (60-5) Determination of Heavy Chain Selectivity of Specifically Modified Compound of Trastuzumab under Reduction Conditions by ESI-TOFMS Analysis

To the antibody-peptide conjugate formed in (60-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23439, whereas for a product, peaks were observed at 50684 and 50845 with a thiopropionyl group introduced to the heavy chain, and a light chain peak was observed at 23439, the same as that of the raw material.

### (60-6) HIC-UPLC Analysis of Specific Modification of Anti-HER2 IgG antibody Trastuzumab

The antibody-peptide conjugate formed in (60-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

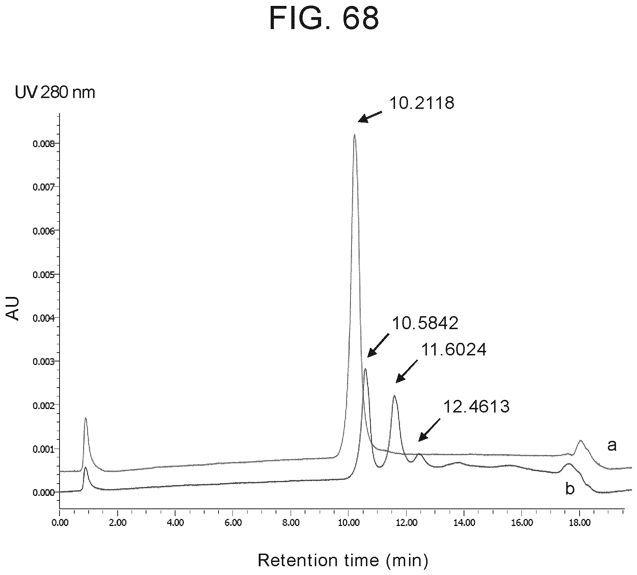

It is believed that a retension time of 10.5842 minutes is attributed to the trastuzumab raw material, whereas that of 11.6024 minutes is attributed to a compound with one peptide introduced to trastuzumab, and that of 12.4613 minutes is attributed to a compound with two peptides introduced to trastuzumab (FIG. 68).

### (60-7) Linker Cleavage and Reoxidation of Trastuzumab-Peptide Conjugate

By performing linker cleavage and reoxidation of the antibody-peptide conjugate obtained in (60-4) by the method described in (3-1) of Example 3, it is possible to obtain an antibody in which thiopropionyl groups are regioselectively introduced to Lys246 and Lys248 in EU numbering.

### [Example 61: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (61-1) Synthesis of IgG1 Fc-Binding Peptide

The peptide of Ac-RGNCRYHKGQIIWCTYH-NH₂ (SEQ ID NO: 74) was synthesized and purified in a similar method to (1-1) of Example 1 to obtain a target product (47.8 mg, 22.0 µmοl) .

MS(ESI)m/z:z=4 545.00[M+3H]³⁺

### (61-2) Formation of Intra-Molecular Disulfide Bond between Cys at Positions 4 and 14 of Ac-RGNCRYHKGQIIWCTYH-NH₂ (SEQ ID NO: 74)

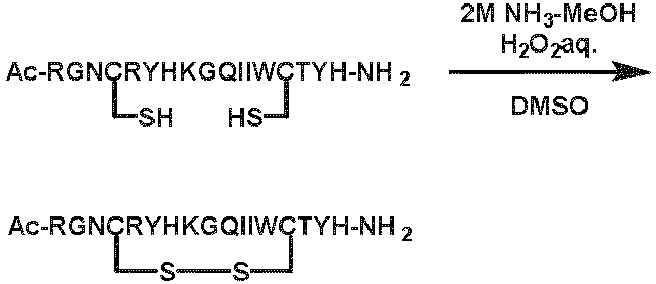

The amino acid sequence is SEQ ID NO: 74.

The peptide (47.8 mg, 22.0 µmol) synthesized in (61-1) was dissolved in DMSO (5.00 mL), 2 M NH₃-MeOH (22.0 µL, 44.0 µmol) and a hydrogen peroxide solution (44.9 µL, 440 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (52.5 mg, 24.1 µmol) .

MS(ESI)m/z:z=4 544.65[M+3H]³⁺

### (61-3) Coupling between Disulfide Linker and Peptide

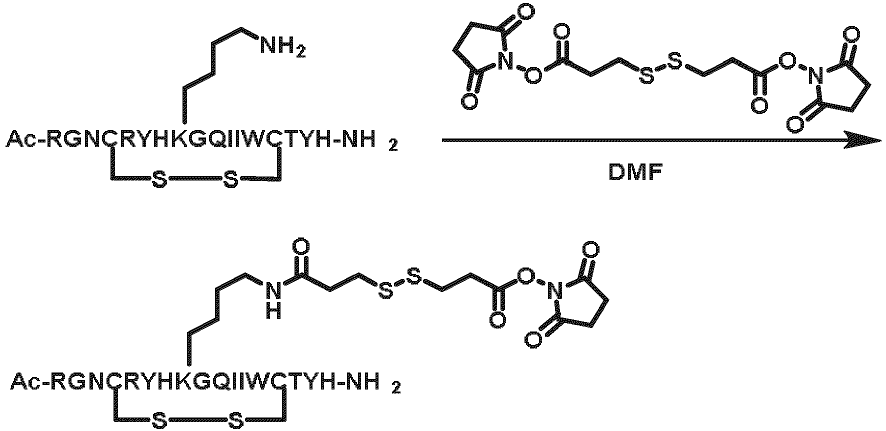

The amino acid sequence is SEQ ID NO: 74.

Ac-RGNCRYHKGQIIWCTYH-NH₂ (SEQ ID NO: 74) synthesized in (61-2) (52.5 mg, 24.1 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (195 mg, 482 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (20.0 mg, 8.12 mol).

MS(ESI)m/z:z=4 617.10[M+3H]³⁺
HPLC purity: 74%

### (61-4) Specific Modification of Anti-HER2 IgG antibody Trastuzumab and Analysis by ESI-TOFMS

The peptide- and disulfide linker-coupled NHS-activation compound synthesized in (61-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148387, whereas a peak for a product with one binding peptide introduced was observed at 150730, and a peak for a product with two binding peptides introduced was observed at 153089.

### (61-5) Determination of Heavy Chain Selectivity of Specifically Modified Compound of Trastuzumab under Reduction Conditions by ESI-TOFMS Analysis

To the antibody-peptide conjugate formed in (61-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50757, and a light chain peak was observed at 23440, whereas for a product, peaks were observed at 50682 and 50845 with a thiopropionyl group introduced to the heavy chain, and a light chain peak was observed at 23439, the same as that of the raw material.

### (61-6) HIC-UPLC Analysis of Specific Modification of Anti-HER2 IgG antibody Trastuzumab

The antibody-peptide conjugate formed in (61-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

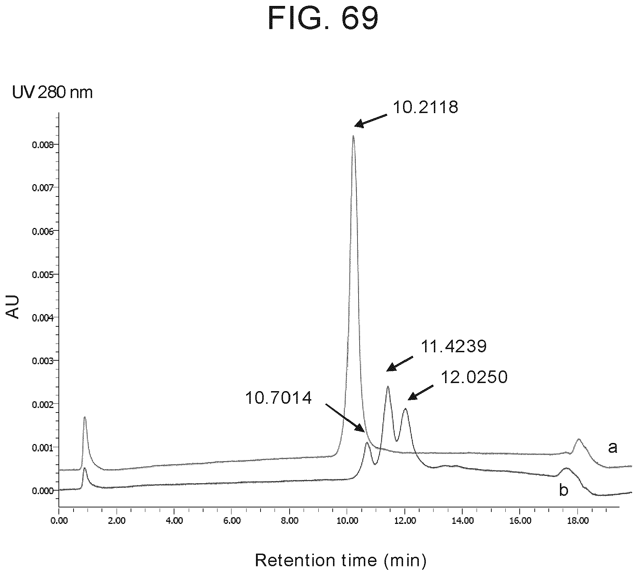

It is believed that a retension time of 10.7014 minutes is attributed to the trastuzumab raw material, whereas that of 11.4239 minutes is attributed to a compound with one peptide introduced to trastuzumab, and that of 12.0250 minutes is attributed to a compound with two peptides introduced to trastuzumab (FIG. 69).

### (61-7) Linker Cleavage and Reoxidation of Trastuzumab-Peptide Conjugate

By performing linker cleavage and reoxidation of the antibody-peptide conjugate obtained in (61-4) by the method described in (3-1) of Example 3, it is possible to obtain an antibody in which thiopropionyl groups are regioselectively introduced to Lys246 and Lys248 in EU numbering.

### [Example 62: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (62-1) Synthesis of IgG1 Fc-Binding Peptide

The peptide of Ac-RGNCVYHKGQIIWCTYH-NH₂ (SEQ ID NO: 75) was synthesized and purified in a similar method to (1-1) of Example 1 to obtain a target product (71.0 mg, 33.5 µmοl) .

MS(ESI)m/z:z=4 530.95[M+3H]³⁺

### (62-2) Formation of Intra-Molecular Disulfide Bond between Cys at Positions 4 and 14 of Ac-RGNCVYHKGQIIWCTYH-NH₂ (SEQ ID NO: 75)

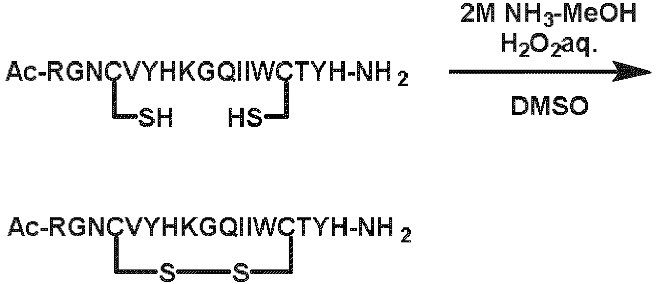

The amino acid sequence is SEQ ID NO: 75.

The peptide (71.0 mg, 33.5 µmol) synthesized in (62-1) was dissolved in DMSO (5.00 mL), 2 M NH₃-MeOH (33.5 µL, 67.0 µmol) and a hydrogen peroxide solution (68.0 µL, 670 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (38.9 mg, 18.4 µmol) .

MS(ESI)m/z:z=4 530.35[M+3H]³⁺

### (62-3) Coupling between Disulfide Linker and Peptide

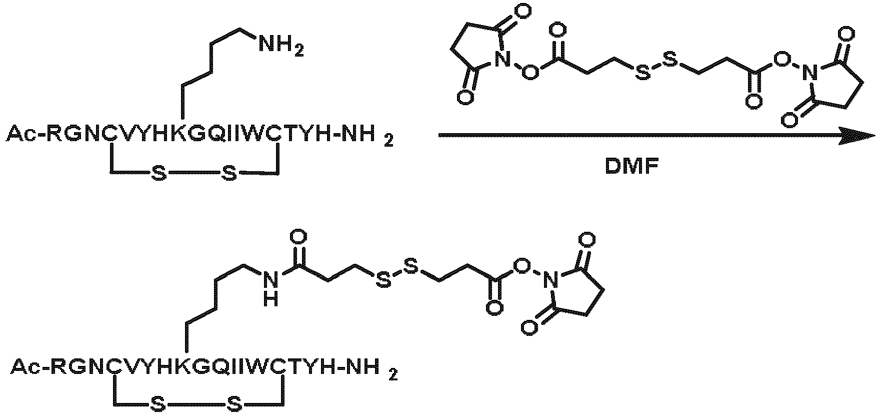

The amino acid sequence is SEQ ID NO: 75.

Ac-RGNCVYHKGQIIWCTYH-NH₂ (SEQ ID NO: 75) synthesized in (62-2) (38.9 mg, 18.4 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (149 mg, 368 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (11.2 mg, 4.65 mol).

MS(ESI)m/z:z=3 803.05[M+3H]³⁺
HPLC purity: 88%

### (62-4) Specific Modification of Anti-HER2 IgG antibody Trastuzumab and Analysis by ESI-TOFMS

The peptide- and disulfide linker-coupled NHS-activation compound synthesized in (62-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150358, and a peak for a product with two binding peptides introduced was observed at 152961.

### (62-5) Determination of Heavy Chain Selectivity of Specifically Modified Compound of Trastuzumab under Reduction Conditions by ESI-TOFMS Analysis

To the antibody-peptide conjugate formed in (62-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23439, whereas for a product, peaks were observed at 50684 and 50846 with a thiopropionyl group introduced to the heavy chain, and a light chain peak was observed at 23439, the same as that of the raw material.

### (62-6) HIC-UPLC Analysis of Specific Modification of Anti-HER2 IgG antibody Trastuzumab

The antibody-peptide conjugate formed in (62-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

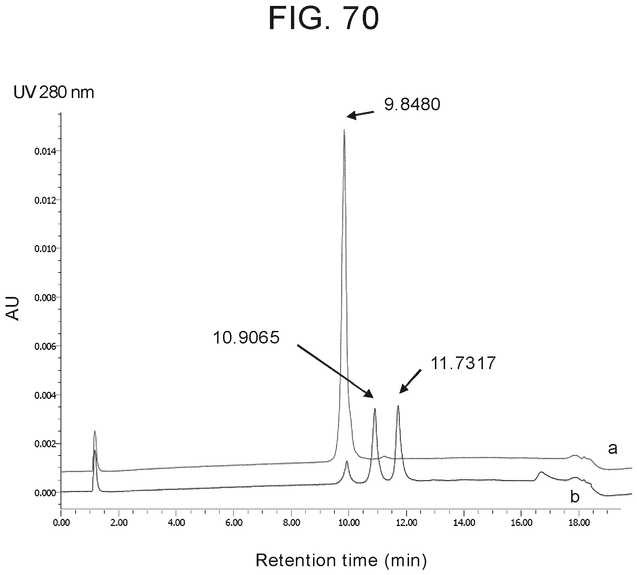

It is believed that a retension time of 10.9065 minutes is attributed to a compound with one peptide introduced to trastuzumab, whereas that of 11.7157 minutes is attributed to a compound with two peptides introduced to trastuzumab (FIG. 70).

### (62-7) Linker Cleavage and Reoxidation of Trastuzumab-Peptide Conjugate

By performing linker cleavage and reoxidation of the antibody-peptide conjugate obtained in (62-4) by the method described in (3-1) of Example 3, it is possible to obtain an antibody in which thiopropionyl groups are regioselectively introduced to Lys246 and Lys248 in EU numbering.

### [Example 63: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (63-1) Synthesis of IgG1 Fc-Binding Peptide

The peptide of Ac-RGNCNYHKGQIIWCTYH-NH₂ (SEQ ID NO: 76) was synthesized and purified in a similar method to (1-1) of Example 1 to obtain a target product (102 mg, 47.7 µmοl) .

MS(ESI)m/z:z=4 534.55[M+3H]³⁺

### (63-2) Formation of Intra-Molecular Disulfide Bond between Cys at Positions 4 and 14 of Ac-RGNCNYHKGQIIWCTYH-NH₂ (SEQ ID NO: 76)

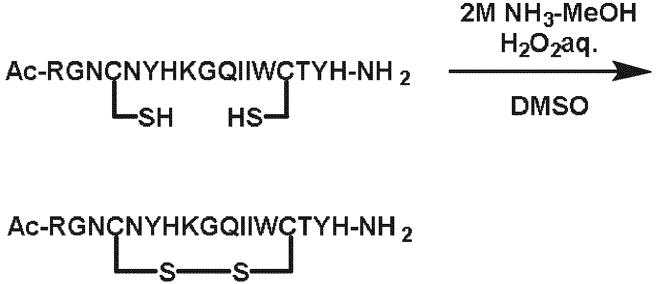

The amino acid sequence is SEQ ID NO: 76.

The peptide (102 mg, 47.7 µmol) synthesized in (63-1) was dissolved in DMSO (5.00 mL), 2 M NH₃-MeOH (47.7 µL, 95.4 µmol) and a hydrogen peroxide solution (97.4 µL, 954 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (51.0 mg, 23.9 µmοl).

MS(ESI)m/z:z=4 533.90[M+3H]³⁺

### (63-3) Coupling between Disulfide Linker and Peptide

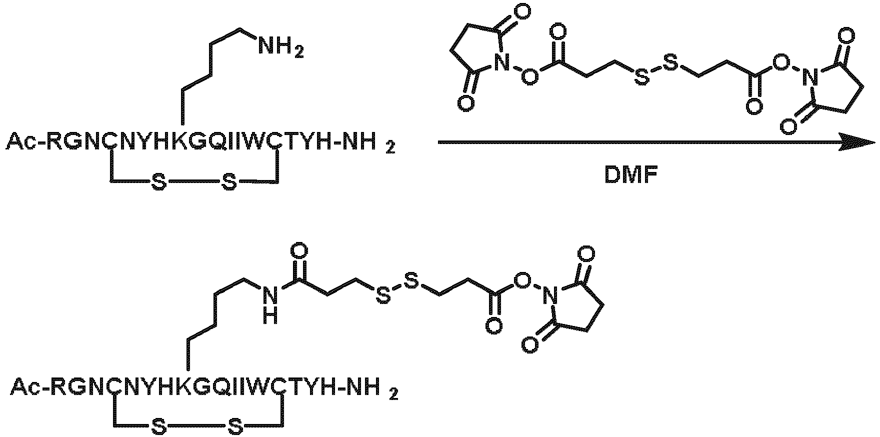

The amino acid sequence is SEQ ID NO: 76.

Ac-RGNCNYHKGQIIWCTYH-NH₂ (SEQ ID NO: 76) synthesized in (63-2) (51.0 mg, 23.9 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (193 mg, 478 µmol) in N,N'-dimethylformamide (1.00 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (19.5 mg, 8.05 mol).

MS(ESI)m/z:z=3 808.15[M+3H]³⁺
HPLC purity: 100%

### (63-4) Specific Modification of Anti-HER2 IgG antibody Trastuzumab and Analysis by ESI-TOFMS

The peptide- and disulfide linker-coupled NHS-activation compound synthesized in (63-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150530, and a peak for a product with two binding peptides introduced was observed at 152839.

### (63-5) Determination of Heavy Chain Selectivity of Specifically Modified Compound of Trastuzumab under Reduction Conditions by ESI-TOFMS Analysis

To the antibody-peptide conjugate formed in (63-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23439, whereas for a product, peaks were observed at 50682 and 50845 with a thiopropionyl group introduced to the heavy chain, and a light chain peak was observed at 23439, the same as that of the raw material.

### (63-6) HIC-UPLC Analysis of Specific Modification of Anti-HER2 IgG antibody Trastuzumab

The antibody-peptide conjugate formed in (63-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

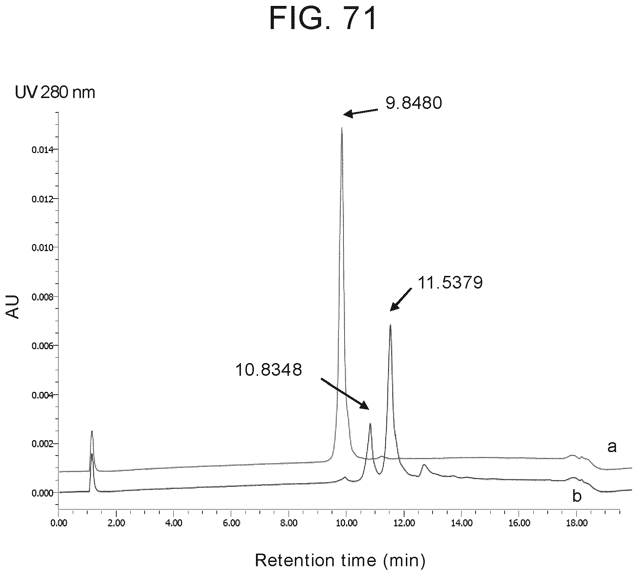

It is believed that a retension time of 10.8348 minutes is attributed to a compound with one peptide introduced to trastuzumab, whereas that of 11.5379 minutes is attributed to a compound with two peptides introduced to trastuzumab (FIG. 71).

### (63-7) Linker Cleavage and Reoxidation of Trastuzumab-Peptide Conjugate

By performing linker cleavage and reoxidation of the antibody-peptide conjugate obtained in (63-4) by the method described in (3-1) of Example 3, it is possible to obtain an antibody in which thiopropionyl groups are regioselectively introduced to Lys246 and Lys248 in EU numbering.

### [Example 64: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (64-1) Synthesis of IgG1 Fc-Binding Peptide

The peptide of Ac-RGNCEYHKGQIIWCTYH-NH₂ (SEQ ID NO: 77) was synthesized and purified in a similar method to (1-1) of Example 1 to obtain a target product (77.7 mg, 36.1 µmοl) .

MS(ESI)m/z:z=4 538.20[M+3H]³⁺

### (64-2) Formation of Intra-Molecular Disulfide Bond between Cys at Positions 4 and 14 of Ac-RGNCEYHKGQIIWCTYH-NH₂ (SEQ ID NO: 77)

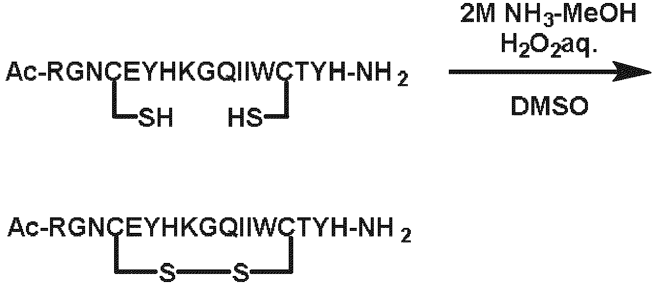

The amino acid sequence is SEQ ID NO: 77.

The peptide (77.7 mg, 36.1 µmol) synthesized in (64-1) was dissolved in DMSO (5.00 mL), 2 M NH₃-MeOH (36.1 µL, 72.2 µmol) and a hydrogen peroxide solution (73.7 µL, 722 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (47.5 mg, 22.1 µmol).

MS(ESI)m/z:z=4 537.70[M+3H]³⁺

### (64-3) Coupling between Disulfide Linker and Peptide

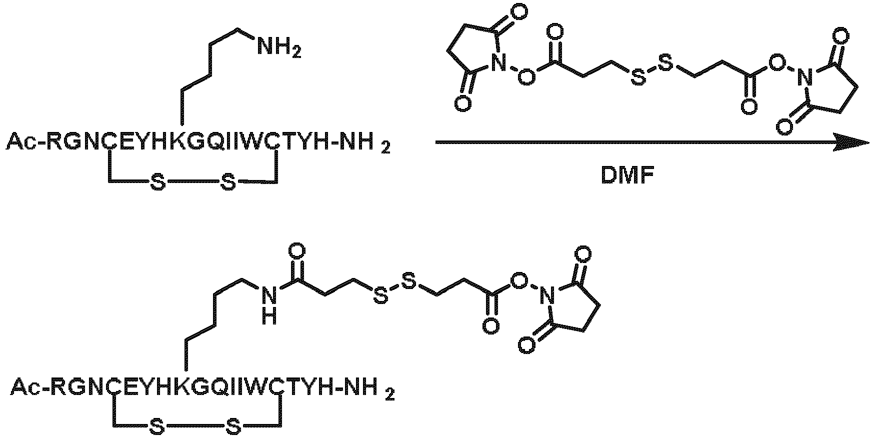

The amino acid sequence is SEQ ID NO: 77.

Ac-RGNCEYHKGQIIWCTYH-NH₂ (SEQ ID NO: 77) synthesized in (64-2) (47.5 mg, 22.1 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (179 mg, 442 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (20.0 mg, 8.21 mol).

MS(ESI)m/z:z=3 813.15[M+3H]³⁺
HPLC purity: 83%

### (64-4) Specific Modification of Anti-HER2 IgG antibody Trastuzumab and Analysis by ESI-TOFMS

The peptide- and disulfide linker-coupled NHS-activation compound synthesized in (64-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148073, whereas a peak for a product with one binding peptide introduced was observed at 150667, and a peak for a product with two binding peptides introduced was observed at 153031.

### (64-5) Determination of Heavy Chain Selectivity of Specifically Modified Compound of Trastuzumab under Reduction Conditions by ESI-TOFMS Analysis

To the antibody-peptide conjugate formed in (64-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50,595 and 50,756, and a light chain peak was observed at 23439, whereas for a product, peaks were observed at 50682 and 50844 with a thiopropionyl group introduced to the heavy chain, and a light chain peak was observed at 23439, the same as that of the raw material.

### (64-6) HIC-UPLC Analysis of Specific Modification of Anti-HER2 IgG antibody Trastuzumab

The antibody-peptide conjugate formed in (64-4) and the raw material antibody were analyzed by HIC. For a column, Protein-Pak Hi Res HIC Column (Waters) 4.6 x 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

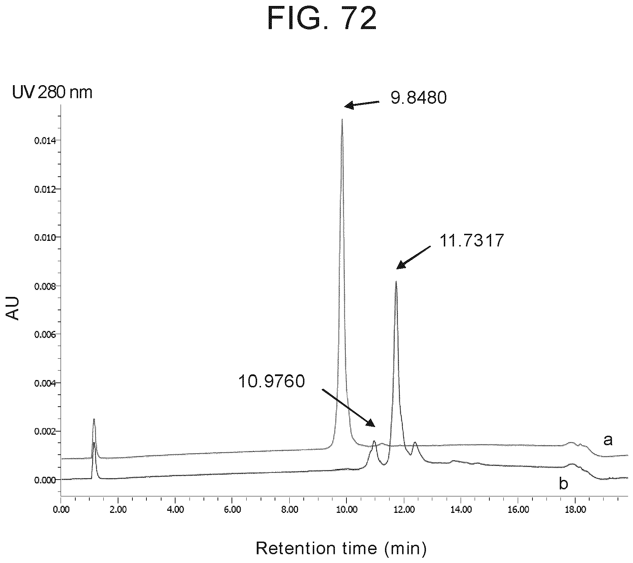

It is believed that a retension time of 10.9760 minutes is attributed to a compound with one peptide introduced to trastuzumab, whereas that of 11.7317 minutes is attributed to a compound with two peptides introduced to trastuzumab (FIG. 72).

### (64-7) Linker Cleavage and Reoxidation of Trastuzumab-Peptide Conjugate

By performing linker cleavage and reoxidation of the antibody-peptide conjugate obtained in (64-4) by the method described in (3-1) of Example 3, it is possible to obtain an antibody in which thiopropionyl groups are regioselectively introduced to Lys246 and Lys248 in EU numbering.

### [Example 65: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (65-1) Synthesis of IgG1 Fc-Binding Peptide

The peptide of Ac-RGNCFYHKGQIIWCTYH-NH₂ (SEQ ID NO: 78) was synthesized and purified in a similar method to (1-1) of Example 1 to obtain a target product (61.9 mg, 28.6 µmοl) .

MS(ESI)m/z:z=4 542.85[M+3H]³⁺

### (65-2) Formation of Intra-Molecular Disulfide Bond between Cys at Positions 4 and 14 of Ac-RGNCFYHKGQIIWCTYH-NH₂ (SEQ ID NO: 78)

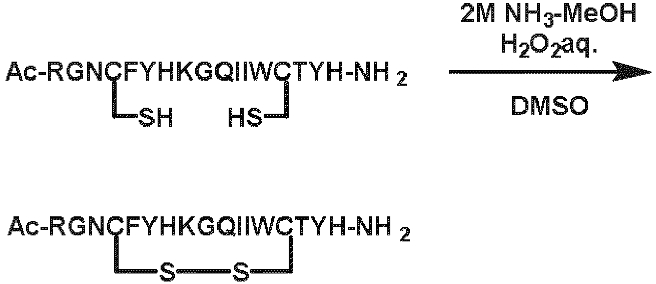

The amino acid sequence is SEQ ID NO: 78.

The peptide (61.9 mg, 28.6 µmol) synthesized in (65-1) was dissolved in DMSO (5.00 mL), 2 M NH₃-MeOH (28.6 µL, 57.2 µmol) and a hydrogen peroxide solution (58.4 µL, 572 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (30.2 mg, 13.9 µmοl).

MS(ESI)m/z:z=4 542.35[M+3H]³⁺

### (65-3) Coupling between Disulfide Linker and Peptide

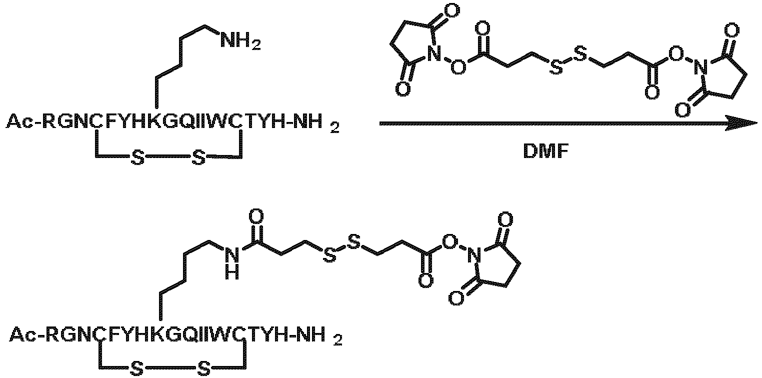

The amino acid sequence is SEQ ID NO: 78.

Ac-RGNCFYHKGQIIWCTYH-NH₂ (SEQ ID NO: 78) synthesized in (65-2) (30.2 mg, 13.9 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (112 mg, 278 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (12.0 mg, 4.89 mol).

MS(ESI)m/z:z=3 819.05[M+3H]³⁺
HPLC purity: 78%

### (65-4) Specific Modification of Anti-HER2 IgG antibody Trastuzumab and Analysis by ESI-TOFMS

The peptide- and disulfide linker-coupled NHS-activation compound synthesized in (65-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148164, whereas a peak for a product with one binding peptide introduced was observed at 150725, and a peak for a product with two binding peptides introduced was observed at 153072.

### (65-5) Determination of Heavy Chain Selectivity of Specifically Modified Compound of Trastuzumab under Reduction Conditions by ESI-TOFMS Analysis

To the antibody-peptide conjugate formed in (65-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50756, and a light chain peak was observed at 23439, whereas for a product, peaks were observed at 50678 and 50845 with a thiopropionyl group introduced to the heavy chain, and a light chain peak was observed at 23439, the same as that of the raw material.

### (65-6) HIC-UPLC Analysis of Specific Modification of Anti-HER2 IgG antibody Trastuzumab

The antibody-peptide conjugate formed in (65-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

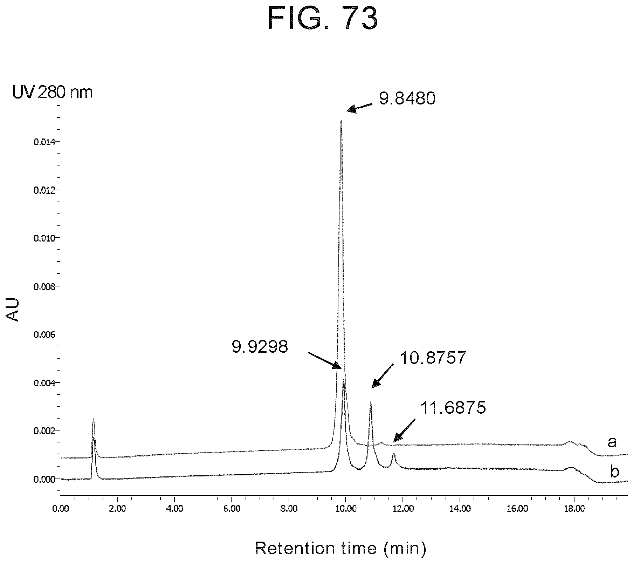

It is believed that a retension time of 9.9298 minutes is attributed to the trastuzumab raw material, whereas that of 10.8757 minutes is attributed to a compound with one peptide introduced to trastuzumab, and that of 11.6875 minutes is attributed to a compound with two peptides introduced to trastuzumab (FIG. 73).

### (65-7) Linker Cleavage and Reoxidation of Trastuzumab-Peptide Conjugate

By performing linker cleavage and reoxidation of the antibody-peptide conjugate obtained in (65-4) by the method described in (3-1) of Example 3, it is possible to obtain an antibody in which thiopropionyl groups are regioselectively introduced to Lys246 and Lys248 in EU numbering.

### [Example 66: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (66-1) Synthesis of IgG1 Fc-Binding Peptide

The peptide of Ac-RGNCAYHKGEIIWCTYH-NH₂ (SEQ ID NO: 79) was synthesized and purified in a similar method to (1-1) of Example 1 to obtain a target product (82.3 mg, 39.3 µmοl) .

MS(ESI)m/z:z=4 523.90[M+3H]³⁺

### (66-2) Formation of Intra-Molecular Disulfide Bond between Cys at Position 4 and Position 14 of Ac-RGNCAYHKGEIIWCTYH-NH₂ (SEQ ID NO: 79)

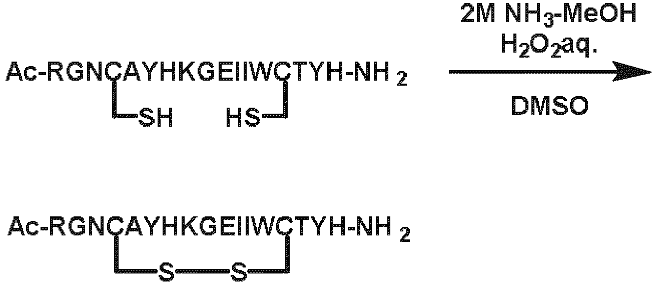

The amino acid sequence is SEQ ID NO: 79.

The peptide (82.3 mg, 39.3 µmol) synthesized in (66-1) was dissolved in DMSO (5.00 mL), 2 M NH₃-MeOH (39.3 µL, 78.6 µmol) and a hydrogen peroxide solution (80.3 µL, 786 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (48.7 mg, 23.3 µmοl).

MS(ESI)m/z:z=4 523.60[M+3H]³⁺

### (66-3) Coupling between Disulfide Linker and Peptide

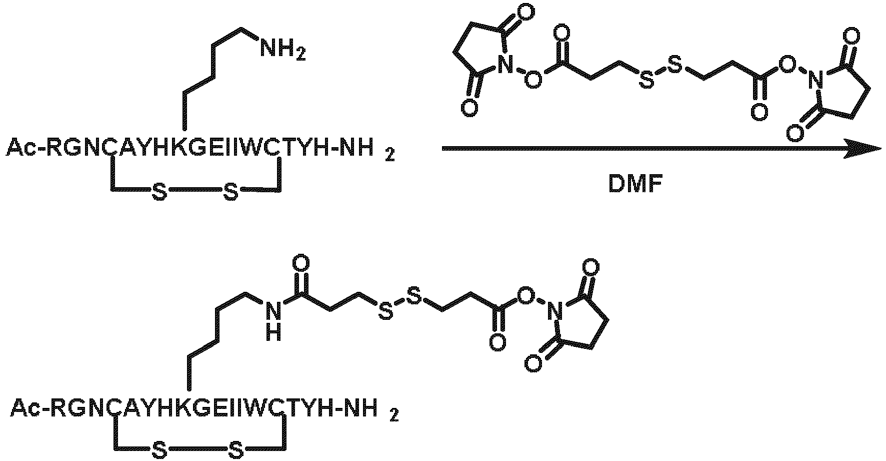

The amino acid sequence is SEQ ID NO: 79.

Ac-RGNCAYHKGEIIWCTYH-NH₂ (SEQ ID NO: 79) synthesized in (66-2) (48.7 mg, 23.3 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (188 mg, 466 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (18.0 mg, 7.56 mol).

MS(ESI)m/z:z=3 794.10[M+3H]³⁺
HPLC purity: 88%

### (66-4) Specific Modification of Anti-HER2 IgG antibody Trastuzumab and Analysis by ESI-TOFMS

The peptide- and disulfide linker-coupled NHS-activation compound synthesized in (66-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS, and a peak for a product with two binding peptides introduced was observed at 152753.

### (66-5) Determination of Heavy Chain Selectivity of Specifically Modified Compound of Trastuzumab under Reduction Conditions by ESI-TOFMS Analysis

To the antibody-peptide conjugate formed in (66-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50682 and 50844 with a thiopropionyl group introduced to the heavy chain, and a light chain peak was observed at 23439, the same as that of the raw material.

### (66-6) HIC-UPLC Analysis of Specific Modification of Anti-HER2 IgG antibody Trastuzumab

The antibody-peptide conjugate formed in (66-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

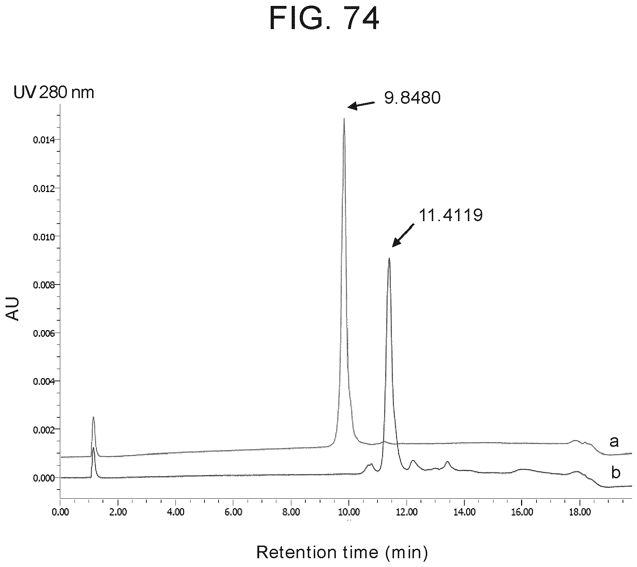

It is believed that a retension time of 11.4119 minutes is attributed to a compound with two peptides introduced to trastuzumab (FIG. 74).

### (66-7) Linker Cleavage and Reoxidation of Trastuzumab-Peptide Conjugate

By performing linker cleavage and reoxidation of the antibody-peptide conjugate obtained in (66-4) by the method described in (3-1) of Example 3, it is possible to obtain an antibody in which thiopropionyl groups are regioselectively introduced to Lys246 and Lys248 in EU numbering.

### [Example 67: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (67-1) Synthesis of IgG1 Fc-Binding Peptide

The peptide of Ac-RGNCAYHKGNIIWCTYH-NH₂ (SEQ ID NO: 80) was synthesized and purified in a similar method to (1-1) of Example 1 to obtain a target product (85.2 mg, 41.0 µmοl) .

MS(ESI)m/z:z=4 520.35[M+3H]³⁺

### (67-2) Formation of Intra-Molecular Disulfide Bond between Cys at Positions 4 and 14 of Ac-RGNCAYHKGNIIWCTYH-NH₂ (SEQ ID NO: 80)

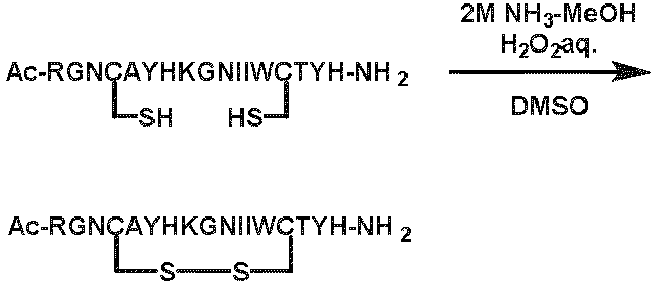

The amino acid sequence is SEQ ID NO: 80.

The peptide (85.2 mg, 41.0 µmol) synthesized in (67-1) was dissolved in DMSO (5.00 mL), 2 M NH₃-MeOH (41.0 µL, 82.0 µmol) and a hydrogen peroxide solution (83.8 µL, 820 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (100 mg, 48.2 µmol).

MS(ESI)m/z:z=4 519.65[M+3H]³⁺

### (67-3) Coupling between Disulfide Linker and Peptide

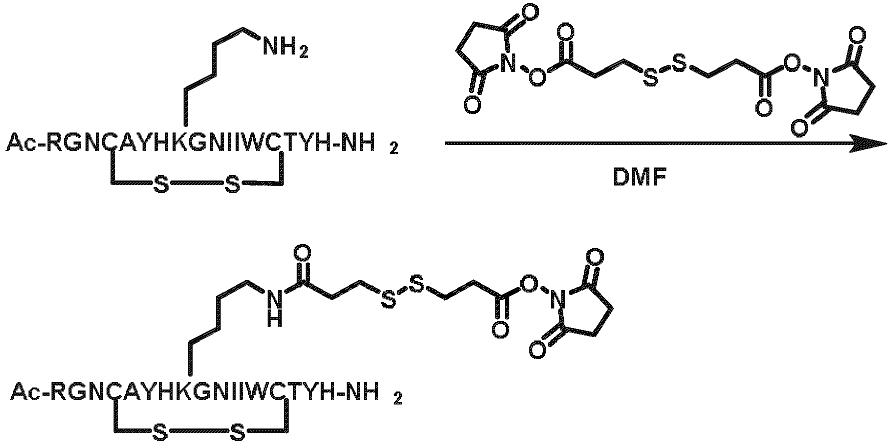

The amino acid sequence is SEQ ID NO: 80.

Ac-RGNCAYHKGNIIWCTYH-NH₂ (SEQ ID NO: 80) synthesized in (67-2) (100 mg, 48.2 µmol, the 4th and 14th of two cysteines form a disulfide bond in the molecule) was dissolved in N,N'-dimethylformamide (0.60 mL), a solution dissolving 3,3'-dithiodipropionic acid di(N-succinimidyl) (390 mg, 964 µmol) in N,N'-dimethylformamide (1.20 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (18.5 mg, 7.82 mol).

MS(ESI)m/z:z=3 789.20[M+3H]³⁺
HPLC purity: 89%

### (67-4) Specific Modification of Anti-HER2 IgG antibody Trastuzumab and Analysis by ESI-TOFMS

The peptide- and disulfide linker-coupled NHS-activation compound synthesized in (67-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS, and a peak for a product with two binding peptides introduced was observed at 152886.

### (67-5) Determination of Heavy Chain Selectivity of Specifically Modified Compound of Trastuzumab under Reduction Conditions by ESI-TOFMS Analysis

To the antibody-peptide conjugate formed in (67-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50682 and 50844 with a thiopropionyl group introduced to the heavy chain, and a light chain peak was observed at 23439, the same as that of the raw material.

### (67-6) HIC-UPLC Analysis of Specific Modification of Anti-HER2 IgG antibody Trastuzumab

The antibody-peptide conjugate formed in (67-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

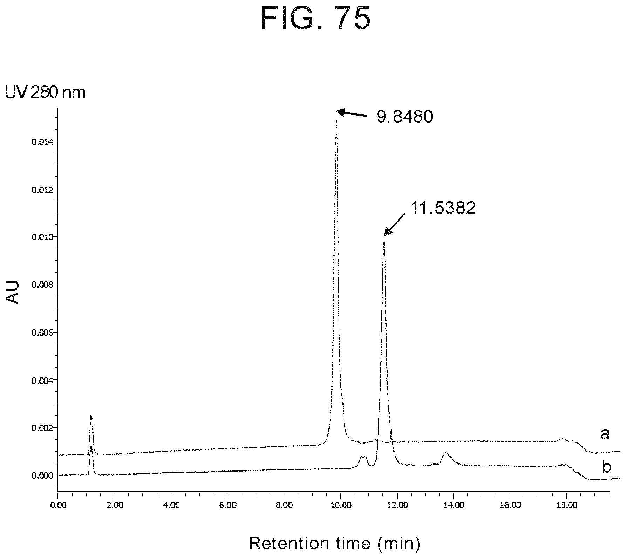

It is believed that a retension time of 11.5382 minutes is attributed to a compound with two peptides introduced to trastuzumab (FIG. 75).

### (67-7) Linker Cleavage and Reoxidation of Trastuzumab-Peptide Conjugate

By performing linker cleavage and reoxidation of the antibody-peptide conjugate obtained in (67-4) by the method described in (3-1) of Example 3, it is possible to obtain an antibody in which thiopropionyl groups are regioselectively introduced to Lys246 and Lys248 in EU numbering.

### [Example 68: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (68-1) Synthesis of IgG1 Fc-Binding Peptide

The peptide of Ac-RGNCAYHKGPIIWCTYH-NH₂ (SEQ ID NO: 81) was synthesized and purified in a similar method to (1-1) of Example 1 to obtain a target product (86.8 mg, 42.1 µmοl) .

MS(ESI)m/z:z=4 516.10[M+3H]³⁺

### (68-2) Formation of Intra-Molecular Disulfide Bond between Cys at Positions 4 and 14 of Ac-RGNCAYHKGPIIWCTYH-NH₂ (SEQ ID NO: 81)

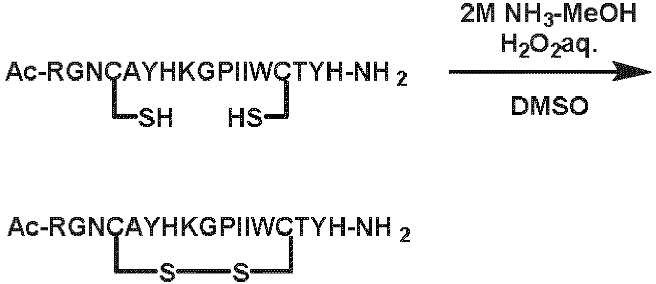

The amino acid sequence is SEQ ID NO: 81.

The peptide (86.8 mg, 42.1 µmol) synthesized in (68-1) was dissolved in DMSO (5.00 mL), 2 M NH₃-MeOH (42.1 µL, 84.2 µmol) and a hydrogen peroxide solution (86.0 µL, 842 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (44.1 mg, 21.4 µmol).

MS(ESI)m/z:z=4 515.55[M+3H]³⁺

### (68-3) Coupling between Disulfide Linker and Peptide

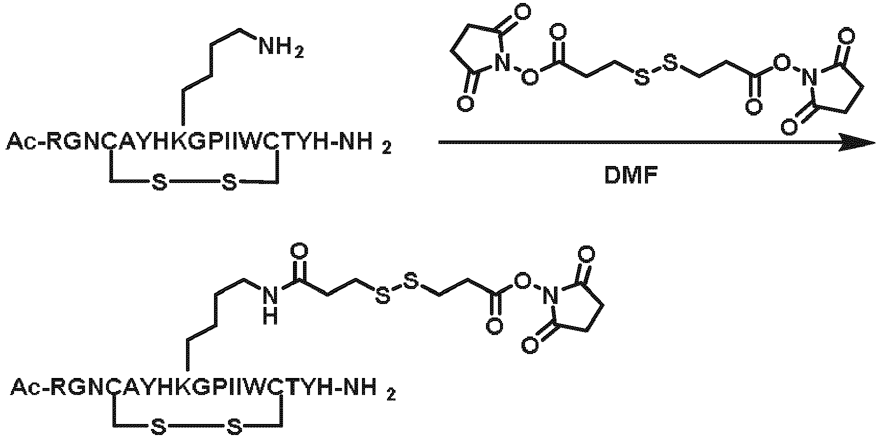

The amino acid sequence is SEQ ID NO: 81.

Ac-RGNCAYHKGPIIWCTYH-NH₂ (SEQ ID NO: 81) synthesized in (68-2) (44.1 mg, 21.4 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (173 mg, 428 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (12.5 mg, 5.32 mol).

MS(ESI)m/z:z=3 783.40[M+3H]³⁺
HPLC purity: 89%

### (68-4) Specific Modification of Anti-HER2 IgG antibody Trastuzumab and Analysis by ESI-TOFMS

The peptide- and disulfide linker-coupled NHS-activation compound synthesized in (68-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148394.

### (68-5) Determination of Heavy Chain Selectivity of Specifically Modified Compound of Trastuzumab under Reduction Conditions by ESI-TOFMS Analysis

To the antibody-peptide conjugate formed in (68-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, heavy chain peaks were observed at 50594 and 50756, and a light chain peak was observed at 23439, the same as those of the raw material.

### (68-6) HIC-UPLC Analysis of Specific Modification of Anti-HER2 IgG antibody Trastuzumab

The antibody-peptide conjugate formed in (68-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

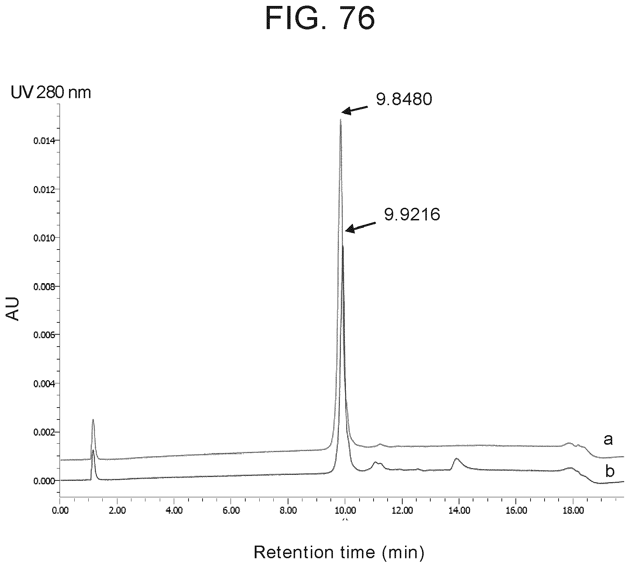

It is believed that a retension time of 9.9216 minutes is attributed to the trastuzumab raw material (FIG. 76) .

### (68-7) Linker Cleavage and Reoxidation of Trastuzumab-Peptide Conjugate

By performing linker cleavage and reoxidation of the antibody-peptide conjugate obtained in (68-4) by the method described in (3-1) of Example 3, it is possible to obtain an antibody in which thiopropionyl groups are regioselectively introduced to Lys246 and Lys248 in EU numbering.

### [Example 69: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (69-1) Synthesis of IgG1 Fc-Binding Peptide

The peptide of Ac-RGNCAYHKGGIIWCTYH-NH₂ (SEQ ID NO: 82) was synthesized and purified in a similar method to (1-1) of Example 1 to obtain a target product (68.9 mg, 34.1 µmοl) .

MS(ESI)m/z:z=4 506.00[M+3H]³⁺

### (69-2) Formation of Intra-Molecular Disulfide Bond between Cys at Positions 4 and 14 of Ac-RGNCAYHKGGIIWCTYH-NH₂ (SEQ ID NO: 82)

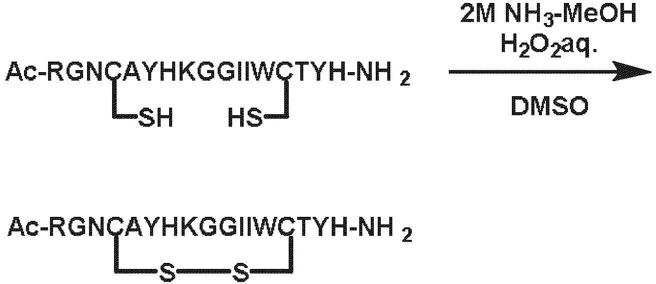

The amino acid sequence is SEQ ID NO: 82.

The peptide (68.9 mg, 34.1 µmol) synthesized in (69-1) was dissolved in DMSO (5.00 mL), 2 M NH₃-MeOH (34.1 µL, 68.2 µmol) and a hydrogen peroxide solution (69.7 µL, 682 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (34.6 mg, 17.1 µmοl) .

MS(ESI)m/z:z=4 506.00[M+3H]³⁺

### (69-3) Coupling between Disulfide Linker and Peptide

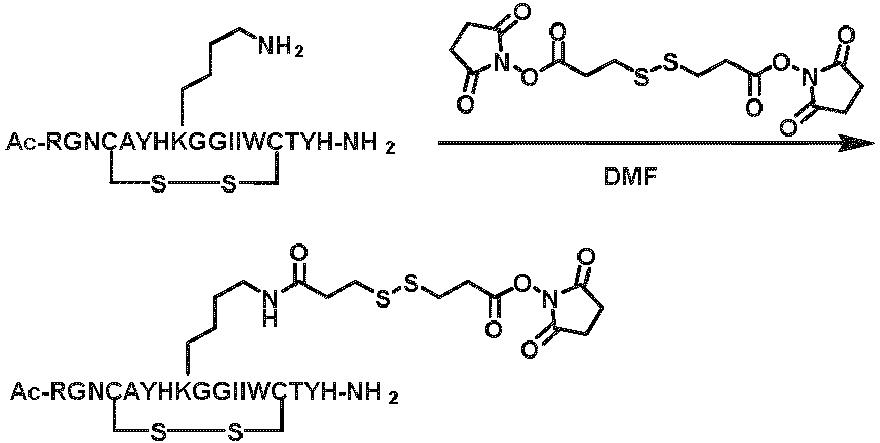

The amino acid sequence is SEQ ID NO: 82.

Ac-RGNCAYHKGGIIWCTYH-NH₂ (SEQ ID NO: 82) synthesized in (69-2) (34.6 mg, 17.1 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (138 mg, 342 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (10.5 mg, 4.55 mol).

MS(ESI)m/z:z=3 770.15[M+3H]³⁺
HPLC purity: 91%

### (69-4) Specific Modification of Anti-HER2 IgG antibody Trastuzumab and Analysis by ESI-TOFMS

The peptide- and disulfide linker-coupled NHS-activation compound synthesized in (69-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150075, and a peak for a product with two binding peptides introduced was observed at 152612.

### (69-5) Determination of Heavy Chain Selectivity of Specifically Modified Compound of Trastuzumab under Reduction Conditions by ESI-TOFMS Analysis

To the antibody-peptide conjugate formed in (69-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50683 and 50845 with a thiopropionyl group introduced to the heavy chain, and a light chain peak was observed at 23439, the same as that of the raw material.

### (69-6) HIC-UPLC Analysis of Specific Modification of Anti-HER2 IgG antibody Trastuzumab

The antibody-peptide conjugate formed in (69-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

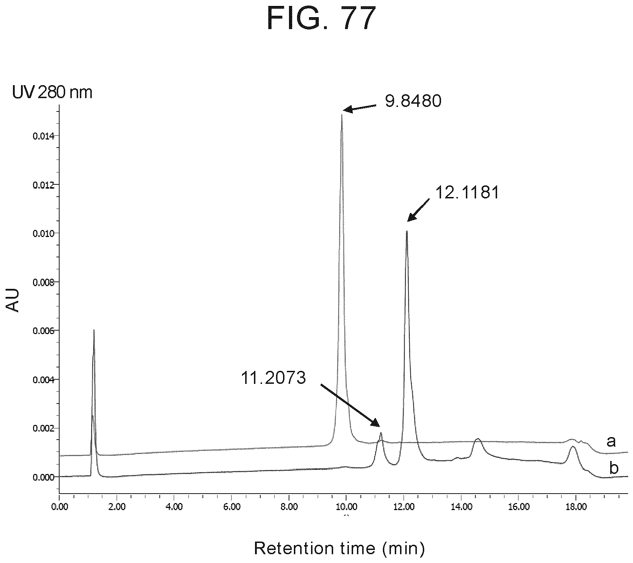

It is believed that a retension time of 11.2073 minutes is attributed to a compound with one peptide introduced to trastuzumab, whereas that of 12.1181 minutes is attributed to a compound with two peptides introduced to trastuzumab (FIG. 77).

### (69-7) Linker Cleavage and Reoxidation of Trastuzumab-Peptide Conjugate

By performing linker cleavage and reoxidation of the antibody-peptide conjugate obtained in (69-4) by the method described in (3-1) of Example 3, it is possible to obtain an antibody in which thiopropionyl groups are regioselectively introduced to Lys246 and Lys248 in EU numbering.

### [Example 70: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (70-1) Synthesis of IgG1 Fc-Binding Peptide

The peptide of Ac-RGNCAYHKGDIIWCTYH-NH₂ (SEQ ID NO: 83) was synthesized and purified in a similar method to (1-1) of Example 1 to obtain a target product (78.1 mg, 37.6 µmοl) .

MS(ESI)m/z:z=4 520.65[M+3H]³⁺

### (70-2) Formation of Intra-Molecular Disulfide Bond between Cys at Positions 4 and 14 of Ac-RGNCAYHKGDIIWCTYH-NH₂ (SEQ ID NO: 83)

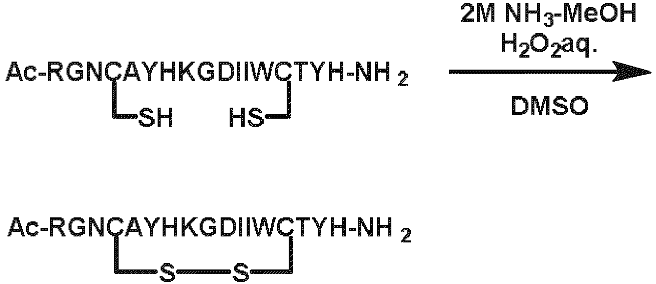

The amino acid sequence is SEQ ID NO: 83.

The peptide (78.1 mg, 37.6 µmol) synthesized in (70-1) was dissolved in DMSO (5.00 mL) , 2 M NH₃-MeOH (37.6 µL, 75.2 µmol) and a hydrogen peroxide solution (76.8 µL, 752 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (38.8 mg, 18.7 µmοl) .

MS(ESI)m/z:z=4 520.20[M+3H]³⁺

### (70-3) Coupling between Disulfide Linker and Peptide

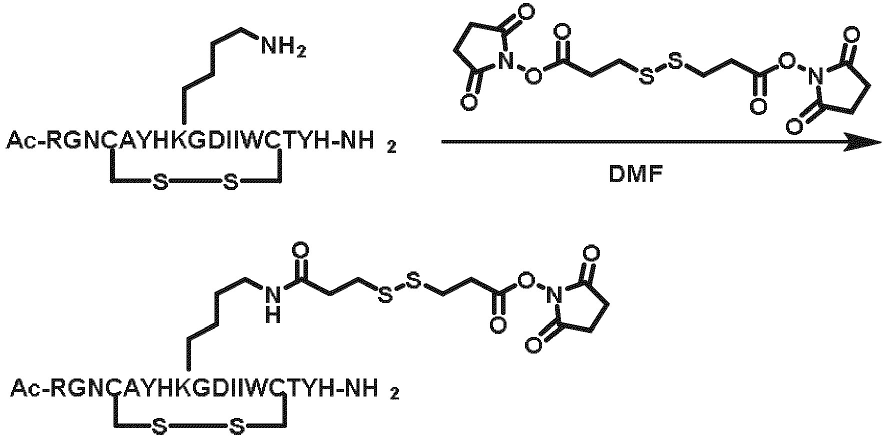

The amino acid sequence is SEQ ID NO: 83.

Ac-RGNCAYHKGDIIWCTYH-NH₂ (SEQ ID NO: 83) synthesized in (70-2) (38.8 mg, 18.7 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (151 mg, 374 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (17.5 mg, 7.40 mol).

MS(ESI)m/z:z=3 789.60[M+3H]³⁺
HPLC purity: 88%

### (70-4) Specific Modification of Anti-HER2 IgG antibody Trastuzumab and Analysis by ESI-TOFMS

The peptide- and disulfide linker-coupled NHS-activation compound synthesized in (70-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150682, and a peak for a product with two binding peptides introduced was observed at 153085.

### (70-5) Determination of Heavy Chain Selectivity of Specifically Modified Compound of Trastuzumab under Reduction Conditions by ESI-TOFMS Analysis

To the antibody-peptide conjugate formed in (70-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50845 with a thiopropionyl group introduced to the heavy chain, and a light chain peak was observed at 23439, the same as that of the raw material.

### (70-6) HIC-UPLC Analysis of Specific Modification of Anti-HER2 IgG antibody Trastuzumab

The antibody-peptide conjugate formed in (70-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

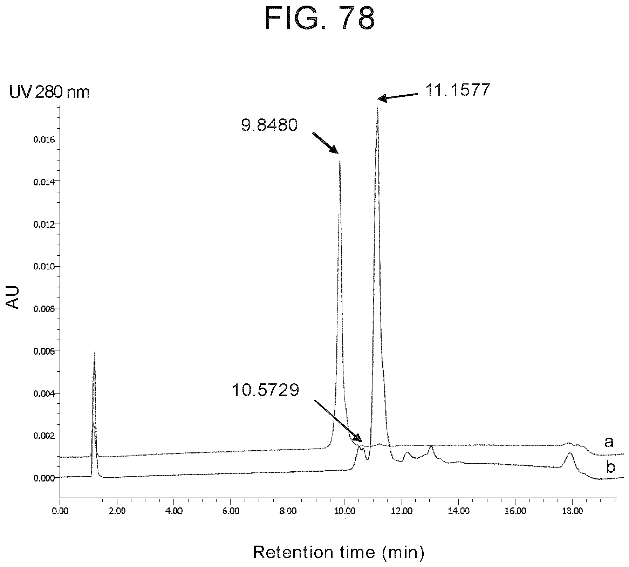

It is believed that a retension time of 10.5729 minutes is attributed to a compound with one peptide introduced to trastuzumab, whereas that of 11.1577 minutes is attributed to a compound with two peptides introduced to trastuzumab (FIG. 78).

### (70-7) Linker Cleavage and Reoxidation of Trastuzumab-Peptide Conjugate

By performing linker cleavage and reoxidation of the antibody-peptide conjugate obtained in (70-4) by the method described in (3-1) of Example 3, it is possible to obtain an antibody in which thiopropionyl groups are regioselectively introduced to Lys246 and Lys248 in EU numbering.

### [Example 71: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (71-1) Synthesis of IgG1 Fc-Binding Peptide

The peptide of Ac-RGNCAYHKGRIIWCTYH-NH₂ (SEQ ID NO: 84) was synthesized and purified in a similar method to (1-1) of Example 1 to obtain a target product (70.1 mg, 33.1 µmοl) .

MS(ESI)m/z:z=4 530.75[M+3H]³⁺

### (71-2) Formation of Intra-Molecular Disulfide Bond between Cys at Positions 4 and 14 of Ac-RGNCAYHKGRIIWCTYH-NH₂ (SEQ ID NO: 84)

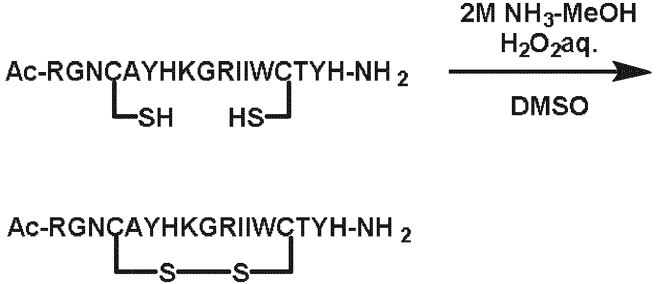

The amino acid sequence is SEQ ID NO: 84.

The peptide (70.1 mg, 33.1 µmol) synthesized in (71-1) was dissolved in DMSO (5.00 mL), 2 M NH₃-MeOH (33.1 µL, 66.2 µmol) and a hydrogen peroxide solution (67.6 µL, 662 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (36.8 mg, 17.4 µmοl) .

MS(ESI)m/z:z=4 530.40[M+3H]³⁺

### (71-3) Coupling between Disulfide Linker and Peptide

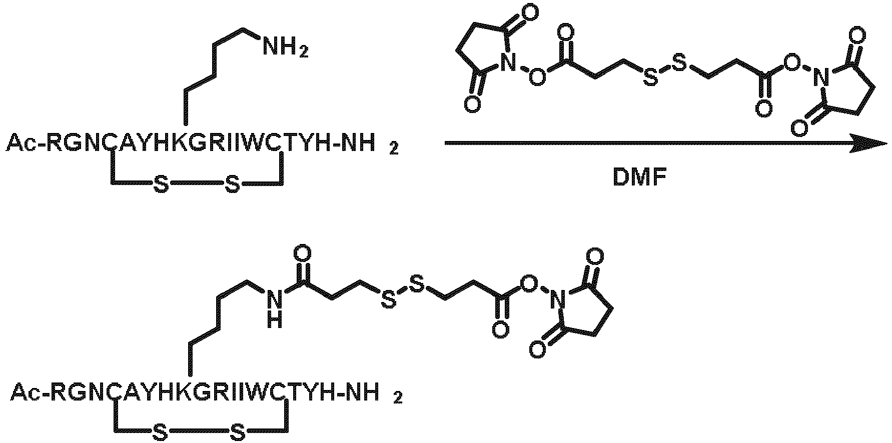

The amino acid sequence is SEQ ID NO: 84.

Ac-RGNCAYHKGRIIWCTYH-NH₂ (SEQ ID NO: 84) synthesized in (71-2) (36.8 mg, 17.4 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (141 mg, 348 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (6.90 mg, 2.87 mol).

MS(ESI)m/z:z=3 803.10[M+3H]³⁺
HPLC purity: 81%

### (71-4) Specific Modification of Anti-HER2 IgG antibody Trastuzumab and Analysis by ESI-TOFMS

The peptide- and disulfide linker-coupled NHS-activation compound synthesized in (71-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150674, and a peak for a product with two binding peptides introduced was observed at 153338.

### (71-5) Determination of Heavy Chain Selectivity of Specifically Modified Compound of Trastuzumab under Reduction Conditions by ESI-TOFMS Analysis

To the antibody-peptide conjugate formed in (71-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50844 with a thiopropionyl group introduced to the heavy chain, and a light chain peak was observed at 23439, the same as that of the raw material.

### (71-6) HIC-UPLC Analysis of Specific Modification of Anti-HER2 IgG antibody Trastuzumab

The antibody-peptide conjugate formed in (71-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

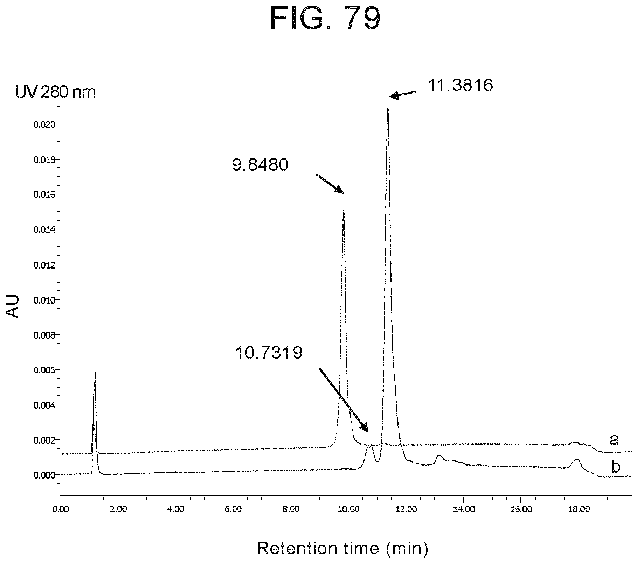

It is believed that a retension time of 10.7319 minutes is attributed to a compound with one peptide introduced to trastuzumab, whereas that of 11.3816 minutes is attributed to a compound with two peptides introduced to trastuzumab (FIG. 79).

### (71-7) Linker Cleavage and Reoxidation of Trastuzumab-Peptide Conjugate

By performing linker cleavage and reoxidation of the antibody-peptide conjugate obtained in (71-4) by the method described in (3-1) of Example 3, it is possible to obtain an antibody in which thiopropionyl groups are regioselectively introduced to Lys246 and Lys248 in EU numbering.

### [Example 72: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (72-1) Synthesis of IgG1 Fc-Binding Peptide

The peptide of Ac-RGNCAYHKGFIIWCTYH-NH₂ (SEQ ID NO: 85) was synthesized and purified in a similar method to (1-1) of Example 1 to obtain a target product (61.2 mg, 29.0 µmοl) .

MS(ESI)m/z:z=4 528.65[M+3H]³⁺

### (72-2) Formation of Intra-Molecular Disulfide Bond between Cys at Positions 4 and 14 of Ac-RGNCAYHKGFIIWCTYH-NH₂ (SEQ ID NO: 85)

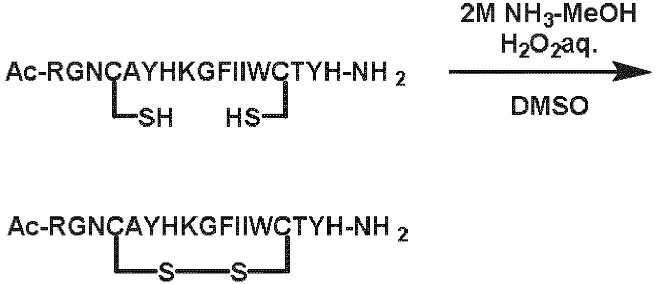

The amino acid sequence is SEQ ID NO: 85.

The peptide (61.2 mg, 29.0 µmol) synthesized in (72-1) was dissolved in DMSO (5.00 mL) , 2 M NH₃-MeOH (29.0 µL, 58.0 µmol) and a hydrogen peroxide solution (59.2 µL, 580 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (33.3 mg, 15.8 µmοl) .

MS(ESI)m/z:z=4 528.00[M+3H]³⁺

### (72-3) Coupling between Disulfide Linker and Peptide

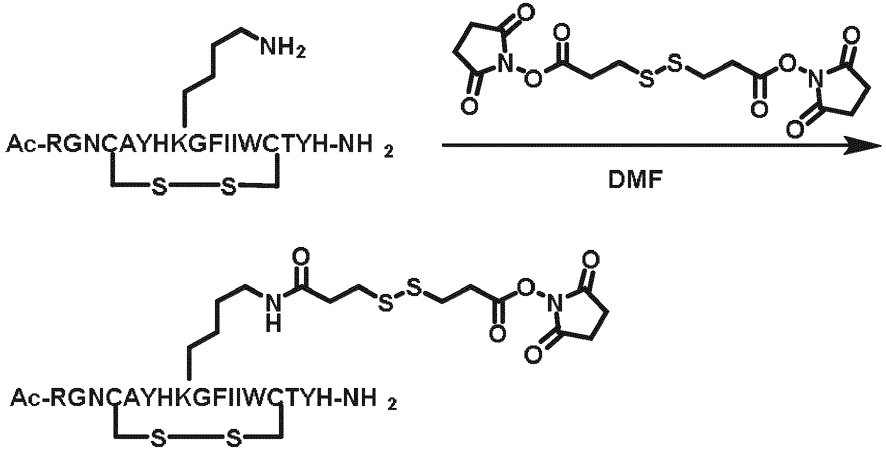

The amino acid sequence is SEQ ID NO: 85.

Ac-RGNCAYHKGFIIWCTYH-NH₂ (SEQ ID NO: 85) synthesized in (72-2) (33.3 mg, 15.8 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (128 mg, 316 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (14.0 mg, 5.84 mol).

MS(ESI)m/z:z=3 800.20[M+3H]³⁺
HPLC purity: 83%

### (72-4) Specific Modification of Anti-HER2 IgG antibody Trastuzumab and Analysis by ESI-TOFMS

The peptide- and disulfide linker-coupled NHS-activation compound synthesized in (72-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150706, and a peak for a product with two binding peptides introduced was observed at 152979.

### (72-5) Determination of Heavy Chain Selectivity of Specifically Modified Compound of Trastuzumab under Reduction Conditions by ESI-TOFMS Analysis

To the antibody-peptide conjugate formed in (72-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50845 with a thiopropionyl group introduced to the heavy chain, and a light chain peak was observed at 23439, the same as that of the raw material.

### (72-6) HIC-UPLC Analysis of Specific Modification of Anti-HER2 IgG antibody Trastuzumab

The antibody-peptide conjugate formed in (72-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

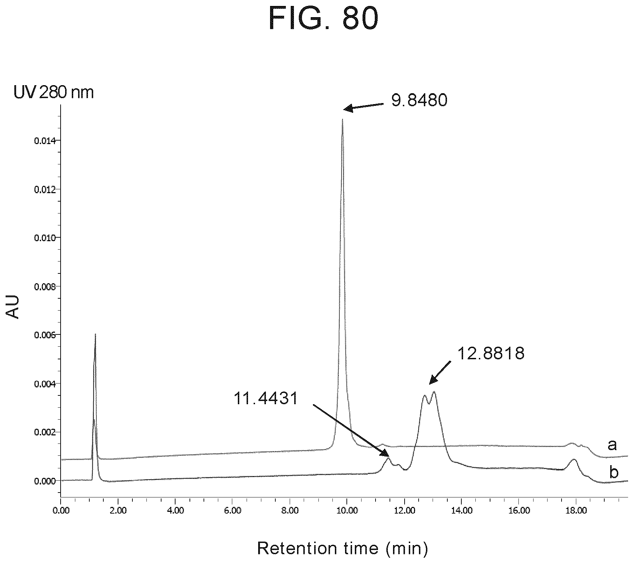

It is believed that a retension time of 9.8295 minutes is attributed to the trastuzumab raw material, whereas that of 12.2498 minutes is attributed to a compound with one peptide introduced to trastuzumab, and that of 13.1829 minutes is attributed to a compound with two peptides introduced to trastuzumab (FIG. 80).

### (72-7) Linker Cleavage and Reoxidation of Trastuzumab-Peptide Conjugate

By performing linker cleavage and reoxidation of the antibody-peptide conjugate obtained in (72-4) by the method described in (3-1) of Example 3, it is possible to obtain an antibody in which thiopropionyl groups are regioselectively introduced to Lys246 and Lys248 in EU numbering.

### [Example 73: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (73-1) Synthesis of IgG1 Fc-Binding Peptide

The peptide of Ac-RGNCAYHKGHIIWCTYH-NH₂ (SEQ ID NO: 86) was synthesized and purified in a similar method to (1-1) of Example 1 to obtain a target product (94.0 mg, 44.8 µmοl) .

MS(ESI)m/z:z=4 526.20[M+3H]³⁺

### (73-2) Formation of Intra-Molecular Disulfide Bond between Cys at Positions 4 and 14 of Ac-RGNCAYHKGHIIWCTYH-NH₂ (SEQ ID NO: 86)

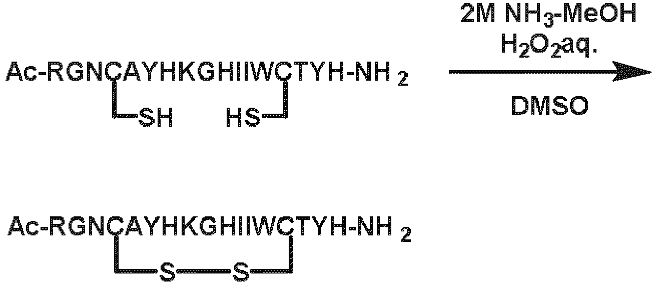

The amino acid sequence is SEQ ID NO: 86.

The peptide (94.0 mg, 44.8 µmol) synthesized in (73-1) was dissolved in DMSO (5.00 mL) , 2 M NH₃-MeOH (44.8 µL, 89.6 µmol) and a hydrogen peroxide solution (91.5 µL, 896 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (56.0 mg, 26.7 µmοl) .

MS(ESI)m/z:z=4 525.70[M+3H]³⁺

### (73-3) Coupling between Disulfide Linker and Peptide

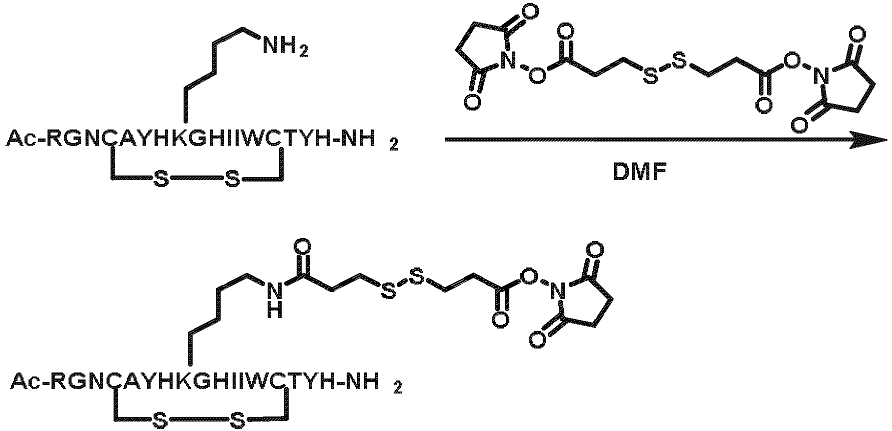

The amino acid sequence is SEQ ID NO: 86.

Ac-RGNCAYHKGHIIWCTYH-NH₂ (SEQ ID NO: 86) synthesized in (73-2) (56.0 mg, 26.7 µmol, the 4th and 14th of two cysteines form a disulfide bond in the molecule) was dissolved in N,N'-dimethylformamide (0.40 mL), a solution dissolving 3,3'-dithiodipropionic acid di(N-succinimidyl) (216 mg, 534 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (16.8 mg, 7.04 mol).

MS(ESI)m/z:z=3 796.75[M+3H]³⁺
HPLC purity: 100%

### (73-4) Specific Modification of Anti-HER2 IgG antibody Trastuzumab and Analysis by ESI-TOFMS

The peptide- and disulfide linker-coupled NHS-activation compound synthesized in (73-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 150686, and a peak for a product with two binding peptides introduced was observed at 152951.

### (73-5) Determination of Heavy Chain Selectivity of Specifically Modified Compound of Trastuzumab under Reduction Conditions by ESI-TOFMS Analysis

To the antibody-peptide conjugate formed in (73-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50845 with a thiopropionyl group introduced to the heavy chain, and a light chain peak was observed at 23439, the same as that of the raw material.

### (73-6) HIC-UPLC Analysis of Specific Modification of Anti-HER2 IgG antibody Trastuzumab

The antibody-peptide conjugate formed in (73-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

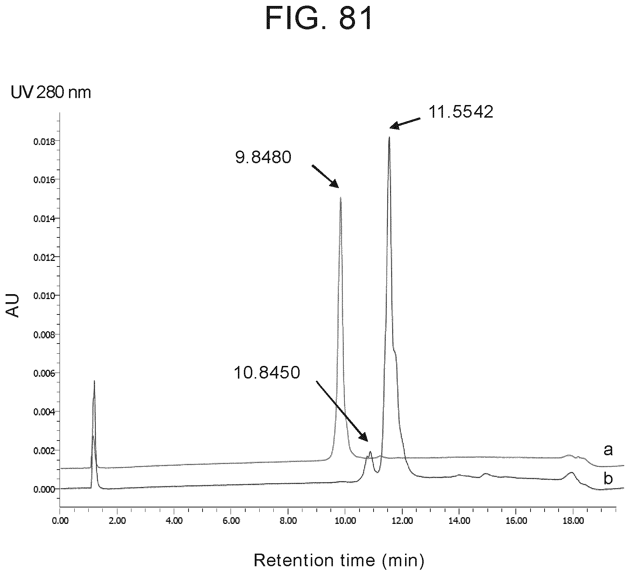

It is believed that a retension time of 10.8450 minutes is attributed to a compound with one peptide introduced to trastuzumab, whereas that of 11.5542 minutes is attributed to a compound with two peptides introduced to trastuzumab (FIG. 81).

### (73-7) Linker Cleavage and Reoxidation of Trastuzumab-Peptide Conjugate

By performing linker cleavage and reoxidation of the antibody-peptide conjugate obtained in (73-4) by the method described in (3-1) of Example 3, it is possible to obtain an antibody in which thiopropionyl groups are regioselectively introduced to Lys246 and Lys248 in EU numbering.

### [Example 74: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (74-1) Synthesis of IgG1 Fc-Binding Peptide

The peptide of Ac-DCAYHKGQIIWCT-NH₂ (SEQ ID NO: 87) was synthesized and purified in a similar method to (1-1) of Example 1 to obtain a target product (66.7 mg, 42.2 µmοl) .

MS(ESI)m/z:z=2 790.20[M+3H]³⁺

### (74-2) Formation of Intra-Molecular Disulfide Bond between Cys at Positions 2 and 12 of Ac-DCAYHKGQIIWCT-NH₂ (SEQ ID NO: 87)

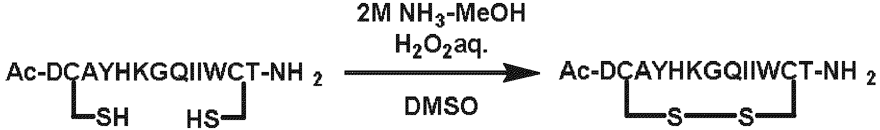

The amino acid sequence is SEQ ID NO: 87.

The peptide (66.7 mg, 42.2 µmol) synthesized in (74-1) was dissolved in DMSO (5.00 mL) , 2 M NH₃-MeOH (42.2 µL, 84.4 µmol) and a hydrogen peroxide solution (86.2 µL, 844 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (25.0 mg, 15.9 µmοl).

MS(ESI)m/z:z=2 788.95[M+3H]³⁺

### (74-3) Coupling between Disulfide Linker and Peptide

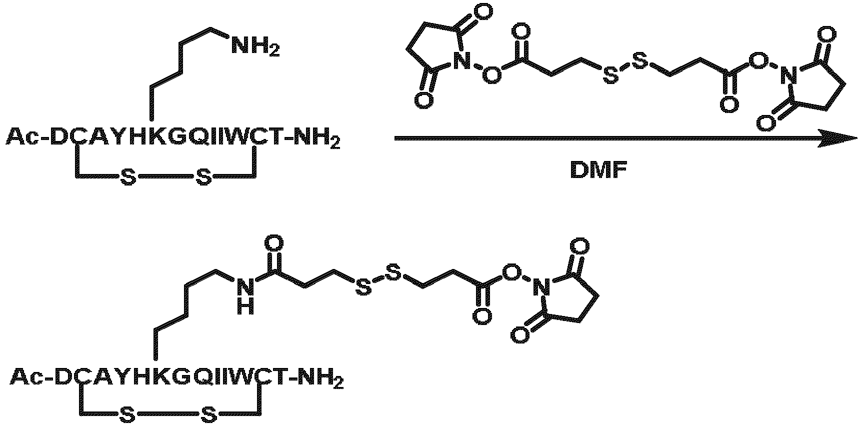

The amino acid sequence is SEQ ID NO: 87.

Ac-DCAYHKGQIIWCT-NH₂ (SEQ ID NO: 87) synthesized in (74-2) (25.0 mg, 15.9 µmol, the 2nd and 12th of two cysteines form a disulfide bond in the molecule) was dissolved in N,N'-dimethylformamide (0.40 mL), a solution dissolving 3,3'-dithiodipropionic acid di(N-succinimidyl) (129 mg, 318 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (11.0 mg, 5.89 mol).

MS(ESI)m/z:z=2 933.80[M+3H]³⁺
HPLC purity: 92%

### (74-4) Specific Modification of Anti-HER2 IgG antibody Trastuzumab and Analysis by ESI-TOFMS

The peptide- and disulfide linker-coupled NHS-activation compound synthesized in (74-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 149462, and a peak for a product with two binding peptides introduced was observed at 152084.

### (74-5) Determination of Heavy Chain Selectivity of Specifically Modified Compound of Trastuzumab under Reduction Conditions by ESI-TOFMS Analysis

To the antibody-peptide conjugate formed in (74-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50845 with a thiopropionyl group introduced to the heavy chain, and a light chain peak was observed at 23439, the same as that of the raw material.

### (74-6) HIC-UPLC Analysis of Specific Modification of Anti-HER2 IgG antibody Trastuzumab

The antibody-peptide conjugate formed in (74-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

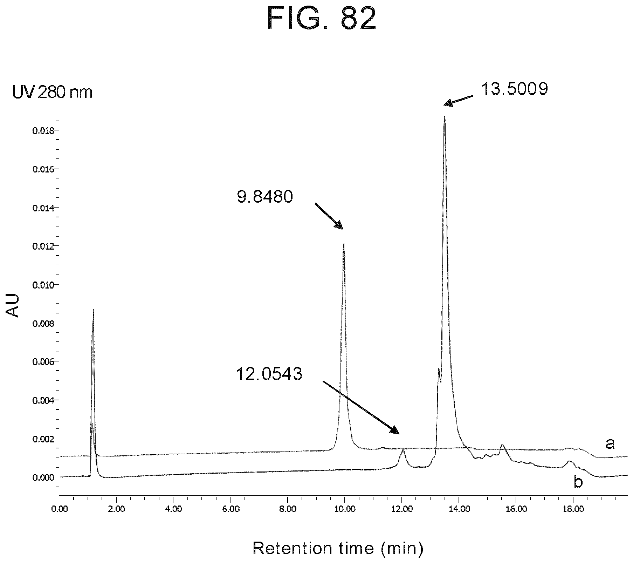

It is believed that a retension time of 12.0543 minutes is attributed to a compound with one peptide introduced to trastuzumab, whereas that of 13.5009 minutes is attributed to a compound with two peptides introduced to trastuzumab (FIG. 82).

### (74-7) Linker Cleavage and Reoxidation of Trastuzumab-Peptide Conjugate

By performing linker cleavage and reoxidation of the antibody-peptide conjugate obtained in (74-4) by the method described in (3-1) of Example 3, it is possible to obtain an antibody in which thiopropionyl groups are regioselectively introduced to Lys246 and Lys248 in EU numbering.

### [Example 75: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (75-1) Synthesis of IgG1 Fc-Binding Peptide

The peptide of Ac-NCAYHKGQIIWCT-NH₂ (SEQ ID NO: 88) was synthesized and purified in a similar method to (1-1) of Example 1 to obtain a target product (75.1 mg, 47.6 µmοl) .

MS(ESI)m/z:z=2 789.65[M+3H]³⁺

### (75-2) Formation of Intra-Molecular Disulfide Bond between Cys at Positions 2 and 12 of Ac-NCAYHKGQIIWCT-NH₂ (SEQ ID NO: 88)

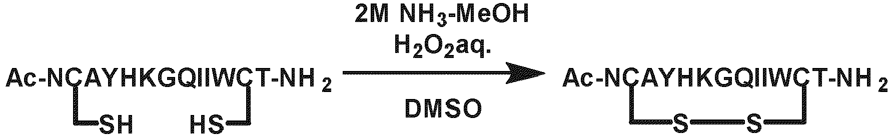

The amino acid sequence is SEQ ID NO: 88.

The peptide (75.1 mg, 47.6 µmol) synthesized in (75-1) was dissolved in DMSO (5.00 mL) , 2 M NH₃-MeOH (47.6 µL, 95.2 µmol) and a hydrogen peroxide solution (97.2 µL, 952 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (27.7 mg, 17.6 µmοl) .

MS(ESI)m/z:z=2 788.90[M+3H]³⁺

### (75-3) Coupling between Disulfide Linker and Peptide

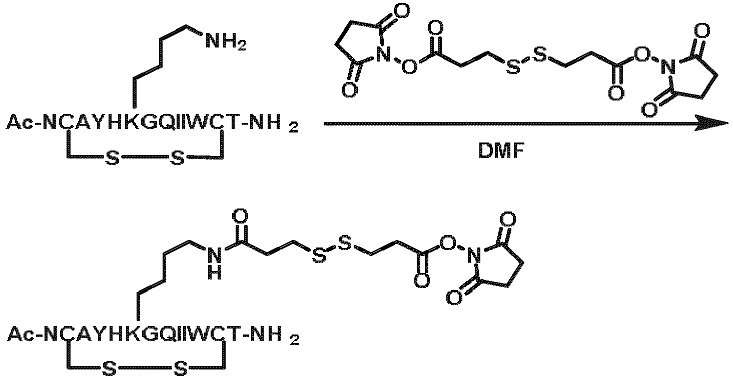

The amino acid sequence is SEQ ID NO: 88.

Ac-NCAYHKGQIIWCT-NH₂ (SEQ ID NO: 88) synthesized in (75-2) (27.7 mg, 17.6 µmol, the 2nd and 12th of two cysteines form a disulfide bond in the molecule) was dissolved in N,N'-dimethylformamide (0.40 mL), a solution dissolving 3,3'-dithiodipropionic acid di(N-succinimidyl) (142 mg, 352 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (10.0 mg, 5.36 mol).

MS(ESI)m/z:z=2 933.10[M+3H]³⁺
HPLC purity: 89%

### (75-4) Specific Modification of Anti-HER2 IgG antibody Trastuzumab and Analysis by ESI-TOFMS

The peptide- and disulfide linker-coupled NHS-activation compound synthesized in (75-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; a peak for a product with one binding peptide introduced was observed at 149662, and a peak for a product with two binding peptides introduced was observed at 152266.

### (75-5) Determination of Heavy Chain Selectivity of Specifically Modified Compound of Trastuzumab under Reduction Conditions by ESI-TOFMS Analysis

To the antibody-peptide conjugate formed in (75-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50845 with a thiopropionyl group introduced to the heavy chain, and a light chain peak was observed at 23439, the same as that of the raw material.

### (75-6) HIC-UPLC Analysis of Specific Modification of Anti-HER2 IgG antibody Trastuzumab

The antibody-peptide conjugate formed in (75-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

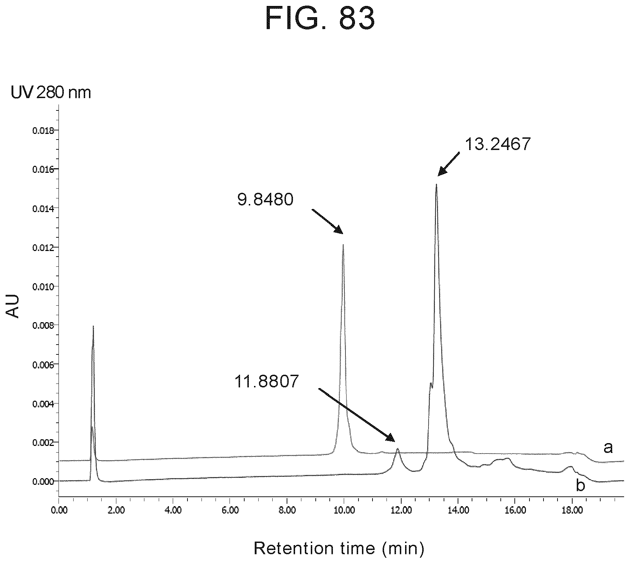

It is believed that a retension time of 11.8807 minutes is attributed to a compound with one peptide introduced to trastuzumab, whereas that of 13.2467 minutes is attributed to a compound with two peptides introduced to trastuzumab (FIG. 83).

### (75-7) Linker Cleavage and Reoxidation of Trastuzumab-Peptide Conjugate

By performing linker cleavage and reoxidation of the antibody-peptide conjugate obtained in (75-4) by the method described in (3-1) of Example 3, it is possible to obtain an antibody in which thiopropionyl groups are regioselectively introduced to Lys246 and Lys248 in EU numbering.

### [Example 76: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (76-1) Synthesis of IgG1 Fc-Binding Peptide

The peptide of Ac-GNCAYHKGQIIWCTY-NH₂ (SEQ ID NO: 89) was synthesized and purified in a similar method to (1-1) of Example 1 to obtain a target product (44.7 mg, 24.9 µmοl) .

MS(ESI)m/z:z=2 899.70[M+3H]³⁺

### (76-2) Formation of Intra-Molecular Disulfide Bond between Cys at Positions 3 and 13 of Ac-GNCAYHKGQIIWCTY-NH₂ (SEQ ID NO: 89)

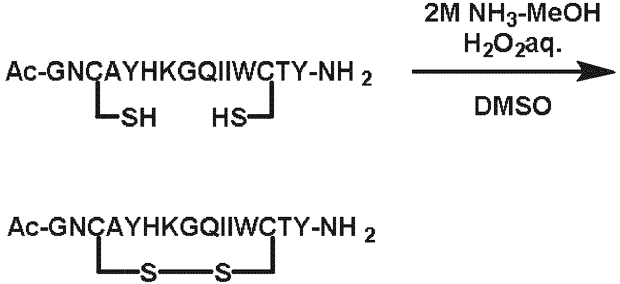

The amino acid sequence is SEQ ID NO: 89.

The peptide (44.7 mg, 24.9 µmol) synthesized in (76-1) was dissolved in DMSO (5.00 mL) , 2 M NH₃-MeOH (24.9 µL, 49.8 µmol) and a hydrogen peroxide solution (50.9 µL, 498 µmοl) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (20.0 mg, 11.1 µmοl) .

MS(ESI)m/z:z=2 899.00[M+3H]³⁺

### (76-3) Coupling between Disulfide Linker and Peptide

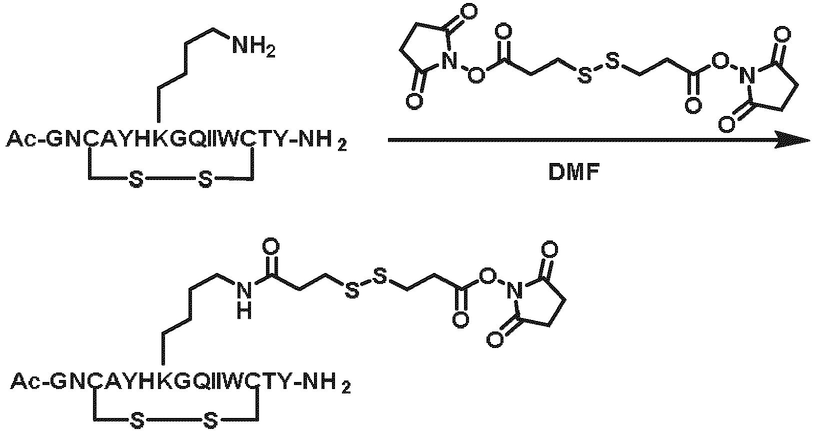

The amino acid sequence is SEQ ID NO: 89.

Ac-GNCAYHKGQIIWCTY-NH₂ (SEQ ID NO: 89) synthesized in (76-2) (20.0 mg, 11.1 µmol, the 3rd and 13th of two cysteines form a disulfide bond in the molecule) was dissolved in N,N'-dimethylformamide (0.20 mL), a solution dissolving 3,3'-dithiodipropionic acid di(N-succinimidyl) (90 mg, 222 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (9.40 mg, 4.51 mol).

MS(ESI)m/z:z=3 696.00[M+3H]³⁺
HPLC purity: 95%

### (76-4) Specific Modification of Anti-HER2 IgG antibody Trastuzumab and Analysis by ESI-TOFMS

The peptide- and disulfide linker-coupled NHS-activation compound synthesized in (76-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS, and a peak for a product with two binding peptides introduced was observed at 152165.

### (76-5) Determination of Heavy Chain Selectivity of Specifically Modified Compound of Trastuzumab under Reduction Conditions by ESI-TOFMS Analysis

To the antibody-peptide conjugate formed in (76-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50844 with a thiopropionyl group introduced to the heavy chain, and a light chain peak was observed at 23439, the same as that of the raw material.

### (76-6) HIC-UPLC Analysis of Specific Modification of Anti-HER2 IgG antibody Trastuzumab

The antibody-peptide conjugate formed in (76-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

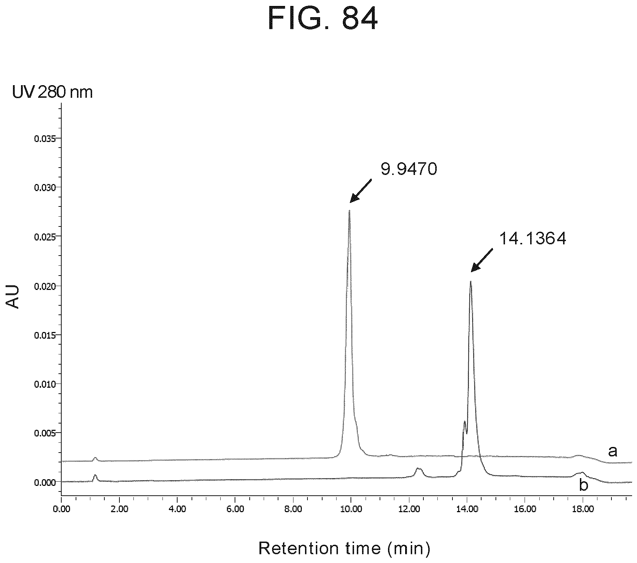

It is believed that a retension time of 14.1364 minutes is attributed to a compound with two peptides introduced to trastuzumab (FIG. 84).

### (76-7) Linker Cleavage and Reoxidation of Trastuzumab-Peptide Conjugate

By performing linker cleavage and reoxidation of the antibody-peptide conjugate obtained in (76-4) by the method described in (3-1) of Example 3, it is possible to obtain an antibody in which thiopropionyl groups are regioselectively introduced to Lys246 and Lys248 in EU numbering.

### [Example 77: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (77-1) Synthesis of IgG1 Fc-Binding Peptide

The peptide of Ac-GCAYHKGQIIWCG-NH₂ (SEQ ID NO: 90) was synthesized and purified in a similar method to (1-1) of Example 1 to obtain a target product (53.5 mg, 36.2 µmol).

MS(ESI)m/z:z=2 739.20[M+3H]³⁺

### (77-2) Formation of Intra-Molecular Disulfide Bond between Cys at Positions 2 and 12 of Ac-GCAYHKGQIIWCG-NH₂ (SEQ ID NO: 90)

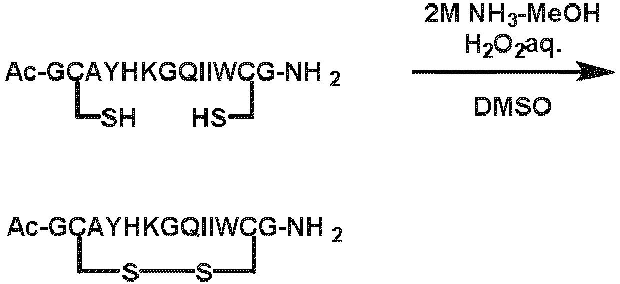

The amino acid sequence is SEQ ID NO: 90.

The peptide (53.5 mg, 36.2 µmol) synthesized in (77-1) was dissolved in DMSO (5.00 mL), 2 M NH₃-MeOH (36.2 µL, 72.4 µmol) and a hydrogen peroxide solution (74.0 µL, 724 µmol)were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (28.7 mg, 19.5 µmol).

MS(ESI)m/z:z=2 738.20[M+3H]³⁺

### (77-3) Coupling between Disulfide Linker and Peptide

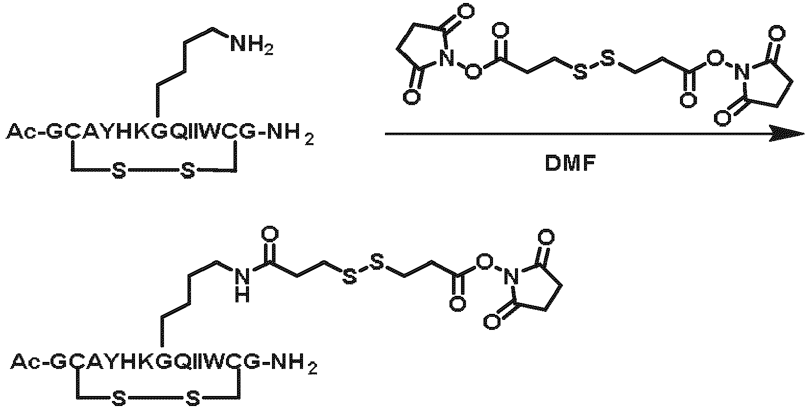

The amino acid sequence is SEQ ID NO: 90.

Ac-GCAYHKGQIIWCG-NH₂ (SEQ ID NO: 90) synthesized in (77-2) (28.7 mg, 19.5 µmol, the 2nd and 12th of two cysteines form a disulfide bond in the molecule) was dissolved in N,N'-dimethylformamide (0.40 mL), a solution dissolving 3,3'-dithiodipropionic acid di(N-succinimidyl) (158 mg, 390 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (11.0 mg, 6.24 mol).

MS(ESI)m/z:z=2 882.90[M+3H]³⁺
HPLC purity: 85%

### (77-4) Specific Modification of Anti-HER2 IgG antibody Trastuzumab and Analysis by ESI-TOFMS

The peptide- and disulfide linker-coupled NHS-activation compound synthesized in (77-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148398, whereas a peak for a product with one binding peptide introduced was observed at 149874, and a peak for a product with two binding peptides introduced was observed at 151522.

### (77-5) Determination of Heavy Chain Selectivity of Specifically Modified Compound of Trastuzumab under Reduction Conditions by ESI-TOFMS Analysis

To the antibody-peptide conjugate formed in (77-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50757, and a light chain peak was observed at 23439, whereas for a product, peaks were observed at 50682 and 50844 with a thiopropionyl group introduced to the heavy chain, and a light chain peak was observed at 23439, the same as that of the raw material.

### (77-6) HIC-UPLC Analysis of Specific Modification of Anti-HER2 IgG antibody Trastuzumab

The antibody-peptide conjugate formed in (77-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

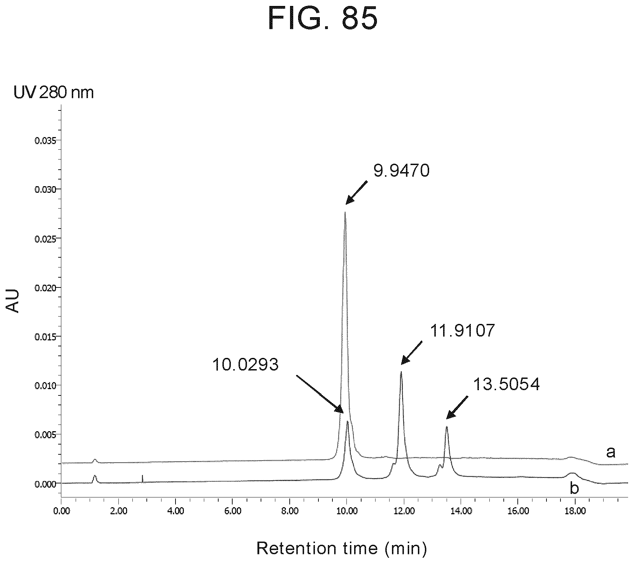

It is believed that a retension time of 10.0293 minutes is attributed to the trastuzumab raw material, whereas that of 11.9107 minutes is attributed to a compound with one peptide introduced to trastuzumab, and that of 13.5054 minutes is attributed to a compound with two peptides introduced to trastuzumab (FIG. 85).

### (77-7) Linker Cleavage and Reoxidation of Trastuzumab-Peptide Conjugate

By performing linker cleavage and reoxidation of the antibody-peptide conjugate obtained in (77-4) by the method described in (3-1) of Example 3, it is possible to obtain an antibody in which thiopropionyl groups are regioselectively introduced to Lys246 and Lys248 in EU numbering.

### [Example 78: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (78-1) Synthesis of IgG1 Fc-Binding Peptide

The peptide of Ac-GGCAYHKGQIIWCGG-NH₂ (SEQ ID NO: 91) was synthesized and purified in a similar method to (1-1) of Example 1 to obtain a target product (16.5 mg, 10.4 µmol).

MS(ESI)m/z:z=2 796.20[M+3H]³⁺

### (78-2) Formation of Intra-Molecular Disulfide Bond between Cys at Positions 3 and 13 of Ac-GGCAYHKGQIIWCGG-NH₂ (SEQ ID NO: 91)

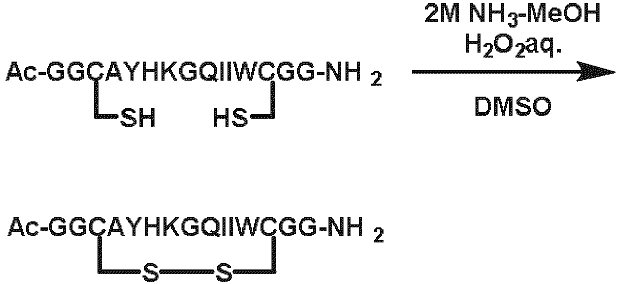

The amino acid sequence is SEQ ID NO: 91.

The peptide (16.5 mg, 10.4 µmol) synthesized in (78-1) was dissolved in DMSO (5.00 mL), 2 M NH₃-MeOH (10.4 µL, 20.8 µmol) and a hydrogen peroxide solution (21.2 µL, 208 µmol) were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (3.70 mg, 2.33 µmol).

MS(ESI)m/z:z=2 795.35[M+3H]³⁺

### (78-3) Coupling between Disulfide Linker and Peptide

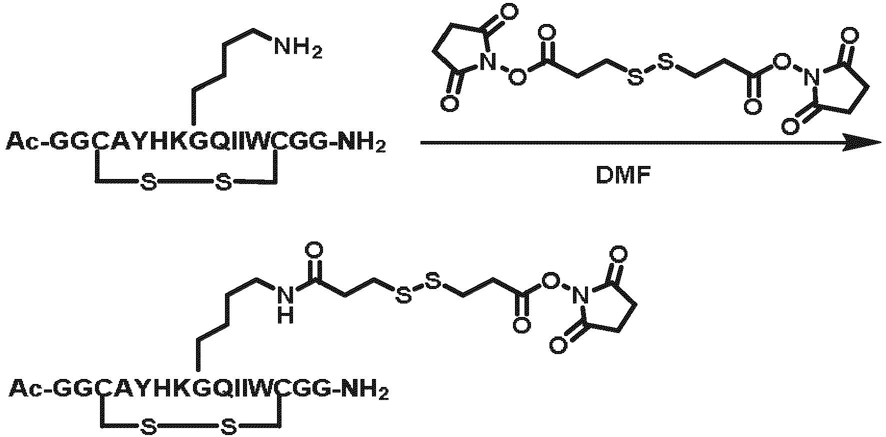

The amino acid sequence is SEQ ID NO: 91.

Ac-GGCAYHKGQIIWCGG-NH₂ (SEQ ID NO: 91) synthesized in (78-2) (3.70 mg, 2.33 µmol, the 3rd and 13th of two cysteines form a disulfide bond in the molecule) was dissolved in N,N'-dimethylformamide (0.40 mL), a solution dissolving 3,3'-dithiodipropionic acid di(N-succinimidyl) (19.0 mg, 46.6 µmol) in N,N'-dimethylformamide (0.20 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (1.20 mg, 0.64 mol).

MS(ESI)m/z:z=2 939.95[M+3H]³⁺
HPLC purity: 60%

### (78-4) Specific Modification of Anti-HER2 IgG antibody Trastuzumab and Analysis by ESI-TOFMS

The peptide- and disulfide linker-coupled NHS-activation compound synthesized in (78-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148790, whereas a peak for a product with one binding peptide introduced was observed at 150561, and a peak for a product with two binding peptides introduced was observed at 152305.

### (78-5) Determination of Heavy Chain Selectivity of Specifically Modified Compound of Trastuzumab under Reduction Conditions by ESI-TOFMS Analysis

To the antibody-peptide conjugate formed in (78-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50682 and 50844 with a thiopropionyl group introduced to the heavy chain, and a light chain peak was observed at 23439, the same as that of the raw material.

### (78-6) HIC-UPLC Analysis of Specific Modification of Anti-HER2 IgG antibody Trastuzumab

The antibody-peptide conjugate formed in (78-5) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

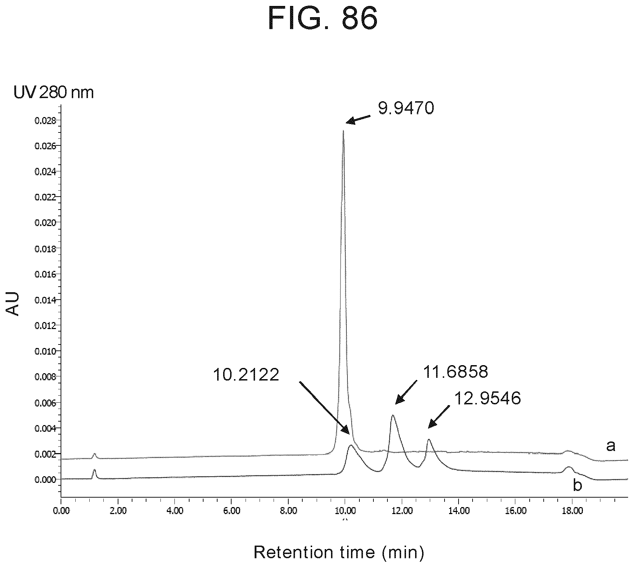

It is believed that a retension time of 10.2122 minutes is attributed to the trastuzumab raw material, whereas that of 11.6858 minutes is attributed to a compound with one peptide introduced to trastuzumab, and that of 12.9546 minutes is attributed to a compound with two peptides introduced to trastuzumab (FIG. 86).

### (78-7) Linker Cleavage and Reoxidation of Trastuzumab-Peptide Conjugate

By performing linker cleavage and reoxidation of the antibody-peptide conjugate obtained in (78-4) by the method described in (3-1) of Example 3, it is possible to obtain an antibody in which thiopropionyl groups are regioselectively introduced to Lys246 and Lys248 in EU numbering.

### [Example 79: Synthesis of Compound Having Affinity Substance to Soluble Protein, Cleavable Portion, and Reactive Group (Peptide- and Disulfide Linker-Coupled NHS-Activation Compound), Modification of Anti-HER2 IgG Antibody Trastuzumab Using Compound, and Analysis thereof]

### (79-1) Synthesis of IgG1 Fc-Binding Peptide

The peptide of Ac-GGGCAYHKGQIIWCGGG-NH₂ (SEQ ID NO: 92) was synthesized and purified in a similar method to (1-1) of Example 1 to obtain a target product (69.9 mg, 41.0 µmol).

MS(ESI)m/z:z=2 853.25[M+3H]³⁺

### (79-2) Formation of Intra-Molecular Disulfide Bond between Cys at Positions 4 and 14 of Ac-GGGCAYHKGQIIWCGGG-NH₂ (SEQ ID NO: 92)

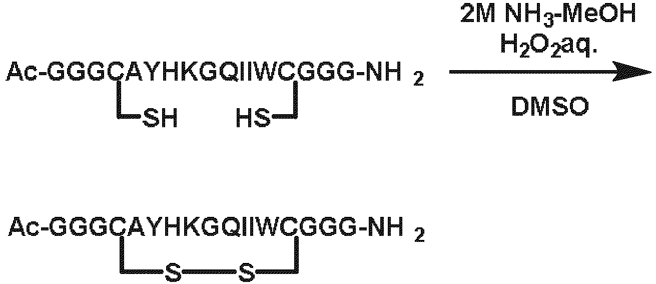

The amino acid sequence is SEQ ID NO: 92.

The peptide (69.9 mg, 41.0 µmol) synthesized in (79-1) was dissolved in DMSO (5.00 mL), 2 M NH₃-MeOH (41.0 µL, 82.0 µmol) and a hydrogen peroxide solution (83.8 µL, 820 µmol)were added thereto, and the solution was stirred at room temperature for 20 hours. A 2 M aqueous trifluoroacetic acid solution was added to the reaction solution to stop the reaction, which was dissolved in a 0.05% aqueous trifluoroacetic acid solution and was subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide (28.3 mg, 16.6 µmol).

MS(ESI)m/z:z=2 852.30[M+3H]³⁺

### (79-3) Coupling between Disulfide Linker and Peptide

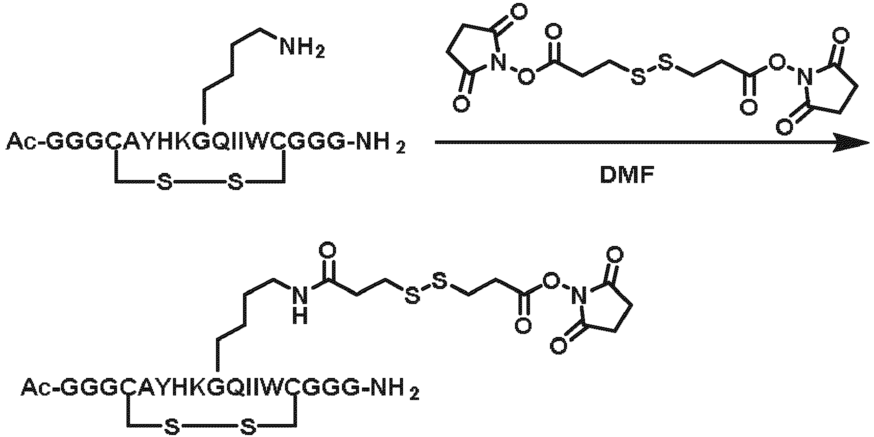

The amino acid sequence is SEQ ID NO: 92.

Ac-GGGCAYHKGQIIWCGGG-NH₂ (SEQ ID NO: 92) synthesized in (79-2) (28.3 mg, 16.6 µmol, the 4th and 14th of two cysteines form a disulfide bond in the molecule) was dissolved in N,N'-dimethylformamide (0.80 mL), a solution dissolving 3,3'-dithiodipropionic acid di(N-succinimidyl) (134 mg, 332 µmol) in N,N'-dimethylformamide (0.60 mL) was added thereto, and the solution was stirred at room temperature for 24 hours. They were dissolved in a 0.05% aqueous trifluoroacetic acid solution and were subjected to reversed phase high-speed liquid chromatography with octadodecyl group-chemically bound type silica gel as a filler, fractions were eluted with a mixed solution of water and acetonitrile containing 0.05% of trifluoroacetic acid, and the fractions were determined by LC-MS. A fraction comprising a product was collected, was concentrated under reduced pressure to remove acetonitrile, and was then freeze-dried to obtain the peptide- and disulfide linker-coupled NHS-activation compound (2.00 mg, 1.00 mol).

MS (ESI)m/z: z=3 664.75 [M+3H]³⁺
HPLC purity: 62%

### (79-4) Specific Modification of Anti-HER2 IgG antibody Trastuzumab and Analysis by ESI-TOFMS

The peptide- and disulfide linker-coupled NHS-activation compound synthesized in (79-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material trastuzumab, a peak was observed at 148225, whereas a peak for a product with one binding peptide introduced was observed at 150102, and a peak for a product with two binding peptides introduced was observed at 151977.

### (79-5) Determination of Heavy Chain Selectivity of Specifically Modified Compound of Trastuzumab under Reduction Conditions by ESI-TOFMS Analysis

To the antibody-peptide conjugate formed in (79-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50595 and 50757, and a light chain peak was observed at 23439, whereas for a product, peaks were observed at 50682 and 50844 with a thiopropionyl group introduced to the heavy chain, and a light chain peak was observed at 23439, the same as that of the raw material.

### (79-6) HIC-UPLC Analysis of Specific Modification of Anti-HER2 IgG antibody Trastuzumab

The antibody-peptide conjugate formed in (79-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

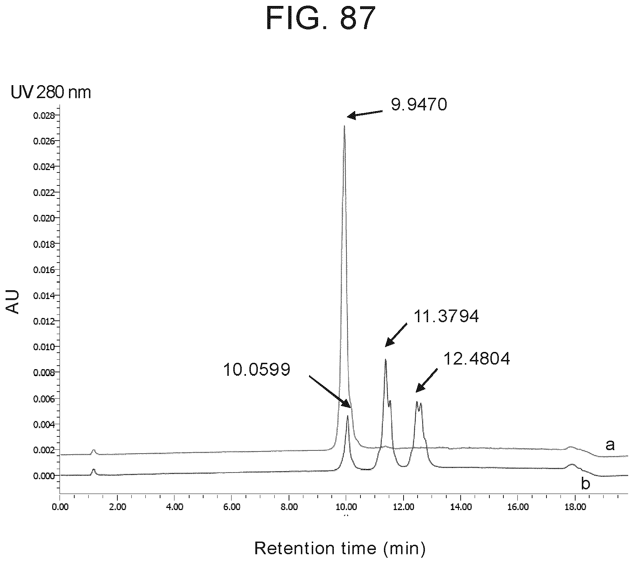

It is believed that a retension time of 10.0599 minutes is attributed to the trastuzumab raw material, whereas that of 11.3794 minutes is attributed to a compound with one peptide introduced to trastuzumab, and that of 12.4804 minutes is attributed to a compound with two peptides introduced to trastuzumab (FIG. 87).

### (79-7) Linker Cleavage and Reoxidation of Trastuzumab-Peptide Conjugate

By performing linker cleavage and reoxidation of the antibody-peptide conjugate obtained in (79-4) by the method described in (3-1) of Example 3, it is possible to obtain an antibody in which thiopropionyl groups are regioselectively introduced to Lys246 and Lys248 in EU numbering.

### [Example 80: Synthesis of Cleavable Linker and Coupling thereof with IgG1 Fc-Binding Peptide]

### (80) Synthesis of Cleavable Linker and Coupling thereof with Peptide

### (80-1) Synthesis of Thioester Linker and Coupling thereof with Peptide

### (80-1-1) Synthesis of Thioester Linker

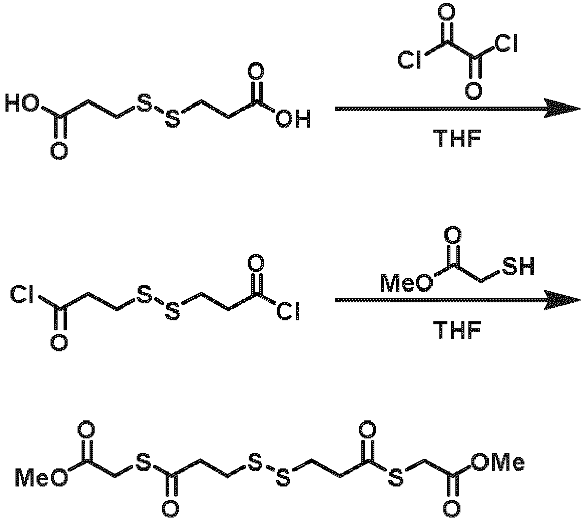

3,3'-Dithiopropionic acid (1.0 g, 5.0 mmol) was dissolved in 10 mL of THF and 100 µL of DMF, pyridine (4.0 mL, 50 mmol) and oxalyl chloride (1.5 mL, 15.0 mmol) were added dropwise thereto under ice cooling, and the resulting mixture was stirred at room temperature for two hours. After two hours, methylthioglycolate (1.1 mL, 15.0 mmol) was added dropwise thereto, and the resulting mixture was further stirred at room temperature for two hours. The reaction solution was concentrated under reduced pressure and purified by normal phase chromatography with hexane/ethyl acetate to obtain methyl 2-[3-[[3-(2-methoxy-2-oxo-ethyl) sulfanyl-3-oxo-propyl] disulfanyl] propanoylsulfanyl] acetate (1.74 g, 4.5 mmol) as a target product.

MS(ESI)m/z:387.0[M+H]⁺

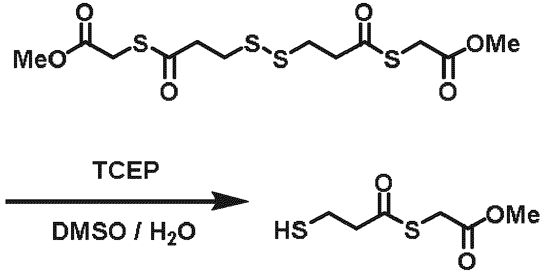

Methyl 2-[3-[[3-(2-methoxy-2-oxo-ethyl) sulfanyl-3-oxo-propyl] disulfanyl] propanoylsulfanyl] acetate (1.74 g,4.5 mmol) was dissolved in 5.0 mL of H₂O and 1.0 mL of DMSO, tris(2-carboxyethyl) phosphine hydrochloride (1.93 g, 6.75 mmol) was added thereto, and the resulting mixture was stirred at room temperature for two hours. After 2 hours, the solution was extracted with ethyl acetate to obtain methyl 2-(3-sulfanylpropanoylsulfanyl) acetate (1.20 g, 6.2 mmol) as a target product.

MS(ESI)m/z:195.0[M+H]⁺

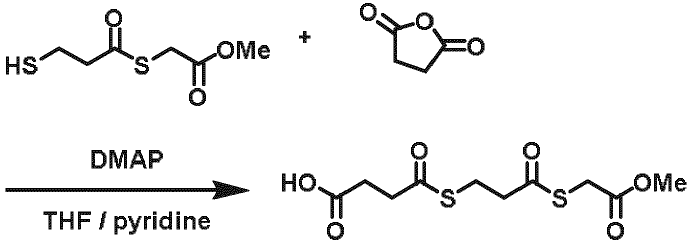

2.7 mL of acetonitrile and 0.3 mL of pyridine were added to and dissolved in methyl 2-(3-sulfanylpropanoylsulfanyl) acetate (1.20 g, 6.2 mmol). Thereafter, succinic anhydride (0.65 g, 6.5 mmol) and 4-dimethylaminopyridine (12.2 mg, 0.1 mmol) were added thereto, and the resulting mixture was stirred for one hour. 20 mL of 1 M aqueous hydrochloric acid solution was added thereto, and the resulting mixture was extracted with ethyl acetate. The solution was concentrated under reduced pressure to obtain 4-[3-(2-methoxy-2-oxo-ethyl) sulfanyl-3-oxo-propyl] sulfanyl-4-oxo-butanoic acid (1.18 g, 4.0 mmol).

MS(ESI)m/z:295.0[M+H]⁺

### (80-1-2) Coupling between Thioester Linker and Peptide

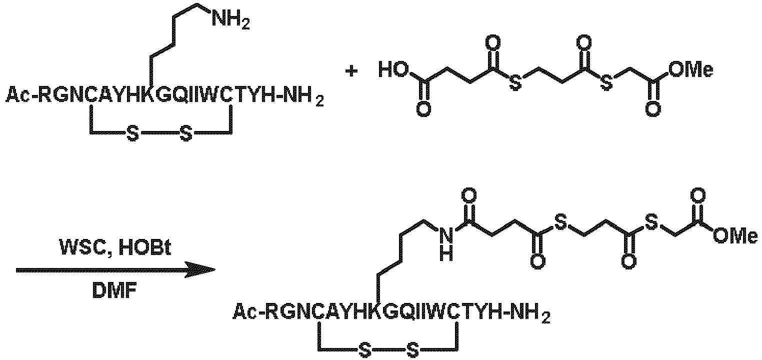

The amino acid sequence is SEQ ID NO: 5.

Ac-RGNCAYHKGQIIWCTYH-NH₂ (SEQ ID NO: 5) synthesized in (80-1) (30 mg, 14.4 µmol, the 4th and 14th of two cysteines form a disulfide bond in the molecule) was dissolved in 1 mL of N,N'-dimethylformamide, and 4-[3-(2-methoxy-2-oxo-ethyl) sulfanyl-3-oxo-propyl] sulfanyl-4-oxo-butanoic acid (65 mg, 0.22 mmol) and WSC·HCl (41 mg, 0.22 mmol) were added thereto. The solution was stirred at room temperature for 12 hours, a 0.1% trifluoroacetic acid solution was added thereto, and fractions were eluted by reversed phase preparative chromatography. A fraction comprising a product was collected, was concentrated under reduced pressure to remove only acetonitrile, and was then freeze-dried to obtain the peptide- and linker-coupled thiophenol-activation compound (24.1 mg, 10.2 µmol).

MS(ESI)m/z:z=3 789 [M+3H]³⁺, z=4 592 [M+4H]⁴⁺

### (80-4) Specific Modification of Anti-HER2 IgG antibody Trastuzumab and Analysis by ESI-TOFMS

The peptide- and linker-coupled thiophenol-activation compound synthesized in (80-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 50 mM sodium acetate buffer (pH 4.7), 4.7 µL of a 21.6 mM peptide reagent (10 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer.

### (80-5) Determination of Heavy Chain Selectivity of Specifically Modified Compound of Trastuzumab under Reduction Conditions by ESI-TOFMS Analysis

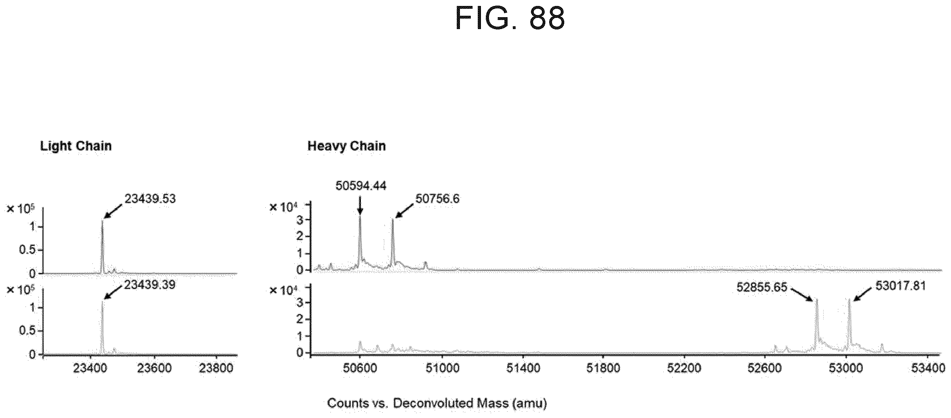

To the antibody-peptide conjugate formed in (80-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 52855 and 53017 with a peptide- and linker-coupled compound introduced to the heavy chain, and a light chain peak was observed at 23439, the same as that of the raw material (FIG. 88).

### (80-6) HIC-UPLC Analysis of Specific Modification of Anti-HER2 IgG antibody Trastuzumab

The antibody-peptide conjugate formed in (80-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 10 equivalents of the peptide- and linker-coupled thiophenol-activation compound;

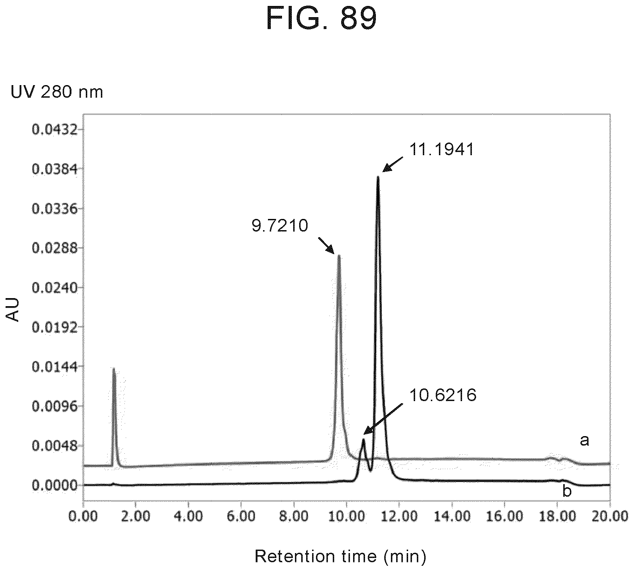

It is believed that a retension time of 10.6216 minutes is attributed to a compound with one peptide introduced to trastuzumab, whereas that of 11.1941 minutes is attributed to a compound with two peptides introduced to trastuzumab (FIG. 89).

### (80-7) Linker Cleavage of Trastuzumab-Peptide Conjugate

The trastuzumab-peptide conjugate (20 mM PBS buffer) obtained in (80-4) was substituted with a 0.5 M hydroxylamine 10 mM EDTA (pH 5.5) solution by Amicon 3K, and the solution was allowed to stand at room temperature for two hours. After two hours, the solution was substituted with 20 mM PBS buffer, 10 mM EDTA (pH 7.4) to obtain thiol-introduced trastuzumab.

### (80-8) Determination of Heavy Chain Selectivity of Specifically Modified Compound of Thiol-introduced Trastuzumab under Reduction Conditions by ESI-TOFMS Analysis

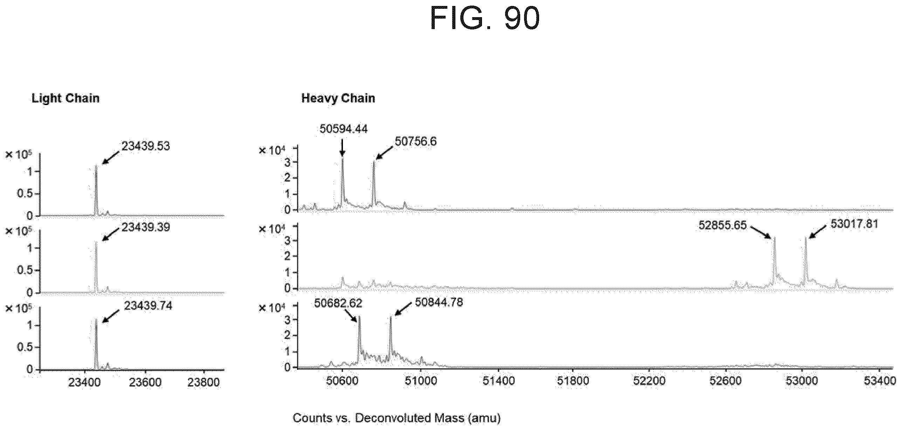

To the thiol-introduced trastuzumab formed in (80-7), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50682 and 50844 with a thiopropionyl group introduced to the heavy chain, and a light chain peak was observed at 23439, the same as that of the raw material (FIG. 90)

### (80-9) HIC-UPLC Analysis of Specific Modification of Anti-HER2 IgG antibody Trastuzumab

The thiol-introduced trastuzumab and the trastuzumab-peptide conjugate antibody formed in (80-4) were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: trastuzumab-peptide conjugate
b: thiol-introduced trastuzumab;

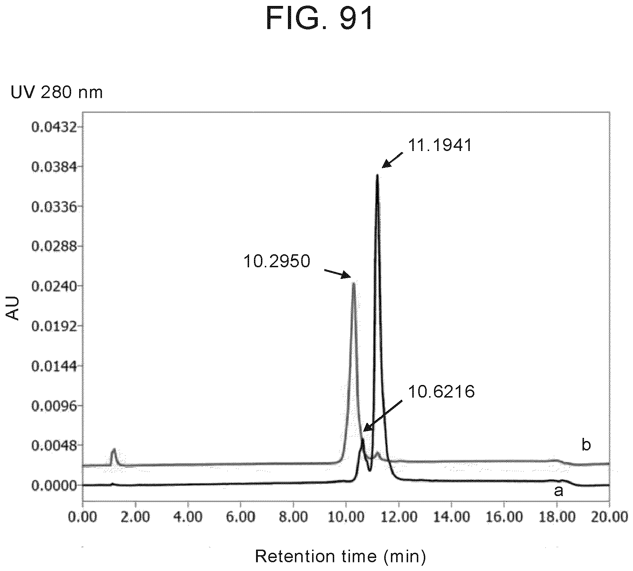

It is believed that a retension time of 10.2950 minutes is attributed to a compound with two thiopropionyl groups introduced to trastuzumab (FIG. 91).

### (80-10) Fluorescent Labeling of Thiol-Introduced Trastuzumab

To the thiol-introduced trastuzumab (20 mM PBS buffer, 10 mM EDTA) obtained in (80-7), 5 equivalents of fluorescein-5-maleimide (10 mM, dissolved in DMSO) were added. The solution was allowed to stand at room temperature for one hour and then substituted with a 20 mM PBS buffer to obtain fluorescently labeled trastuzumab (ADC mimic).

### (80-11) Determination of Heavy Chain Selectivity of Fluorescently Labeled Trastuzumab under Reduction Conditions by ESI-TOFMS Analysis

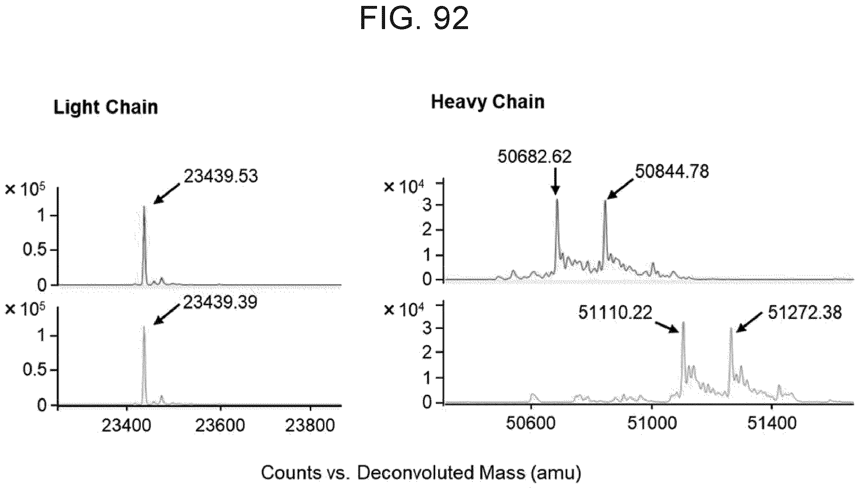

To the fluorescently labeled trastuzumab (ADC mimic) formed in (80-10), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material thiol-introduced trastuzumab, heavy chain peaks were observed at 50682 and 50844, and a light chain peak was observed at 23439, whereas for a product, peaks were observed at 51110 and 51272 with fluorescein introduced to the heavy chain, and a light chain peak was observed at 23439, the same as that of the raw material (FIG. 92).

### (80-12) Synthesis of ADC mimic using click reaction from thiol-introduced trastuzumab

To the thiol-introduced trastuzumab (20 mM PBS buffer, 10 mM EDTA) obtained in (80-7), 10 equivalents of N-(4-azidophenyl)-2-iodo-acetamide (10 mM, dissolved in DMSO) were added. The solution was allowed to stand at room temperature for one hour and then substituted with a 20 mM PBS buffer, and 5 equivalents of dibenzocyclooctynoic acid (DBCO-Acid, 10 mM, dissolved in DMSO) were added thereto to perform a click reaction, thus obtaining ADC mimic.

### (80-13) Determination of Heavy Chain Selectivity of ADC mimic under Reduction Conditions by ESI-TOFMS Analysis

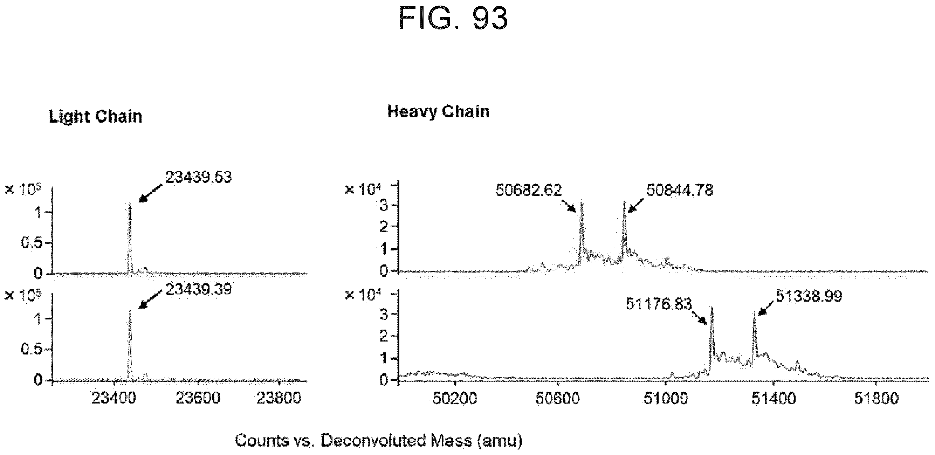

To the ADC mimic formed in (80-12), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material thiol-introduced trastuzumab, heavy chain peaks were observed at 50682 and 50844, and a light chain peak was observed at 23439, whereas for a product, peaks were observed at 51176 and 51338 with a click reaction product introduced to the heavy chain, and a light chain peak was observed at 23439, the same as that of the raw material (FIG. 93).

### (80-14) Summary

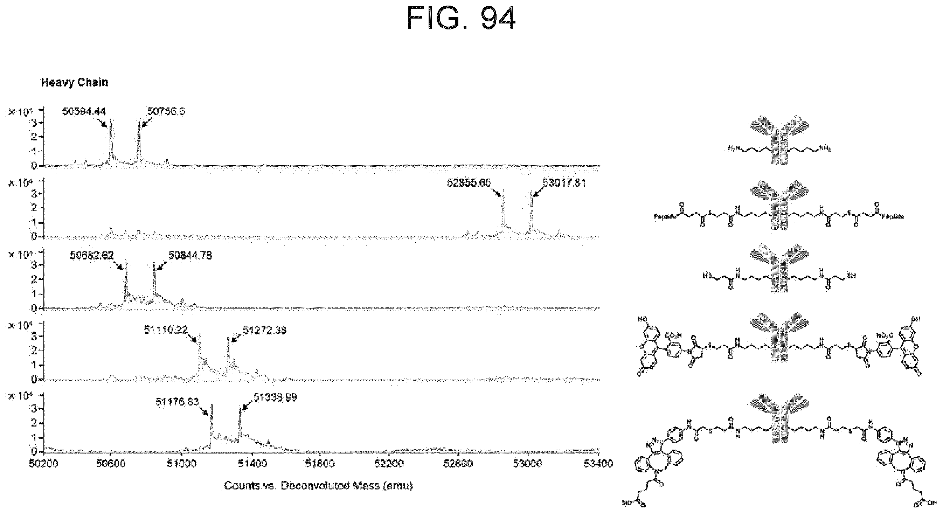

The results of trastuzumab, (80-5), (80-8), (80-10), and (80-12) are summarized (FIG. 94).

### [Example 81: Synthesis of Cleavable Linker and Coupling thereof with IgG1 Fc-Binding Peptide]

### (81) Synthesis of Cleavable Linker and Coupling thereof with Peptide

### (81-1) Synthesis of Thioester Linker and Coupling thereof with Peptide

### (81-1-1) Synthesis of Thioester Linker

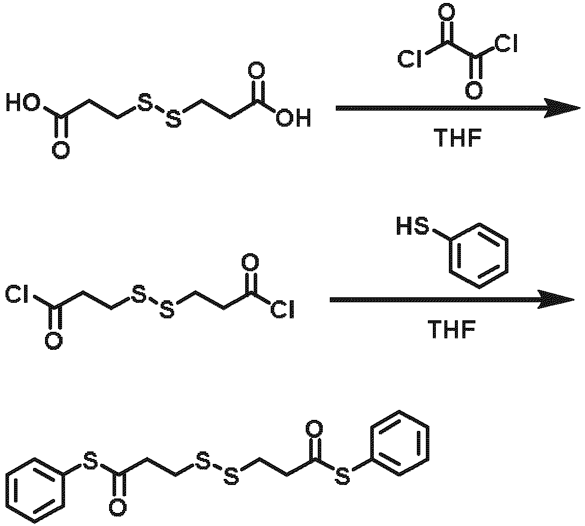

3,3'-Dithiopropionic acid (1.0 g, 5.0 mmol) was dissolved in 10 mL of THF and 100 µL of DMF, pyridine (4.0 mL, 50 mmol) and oxalyl chloride (1.5 mL, 15.0 mmol) were added dropwise thereto under ice cooling, and the resulting mixture was stirred at room temperature for two hours. After two hours, thiophenol (1.53 mL, 15.0 mmol) was added dropwise thereto, and the resulting mixture was further stirred at room temperature for two hours. The reaction solution was concentrated under reduced pressure and purified by normal phase chromatography with hexane/ethyl acetate to obtain S-phenyl 3-[(3-oxo-3-phenylsulfanyl-propyl) disulfanyl] propanethioate (1.77 g, 4.5 mmol) as a target product.

MS(ESI)m/z:395.1[M+H]⁺

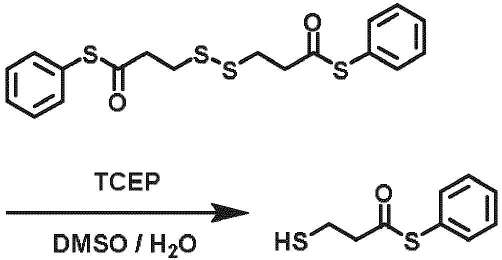

S-Phenyl 3-[(3-oxo-3-phenylsulfanyl-propyl) disulfanyl] propanethioate (1.77 g, 4.5 mmol) was dissolved in 5.0 mL of H₂O and 1.0 mL of DMSO, tris(2-carboxyethyl) phosphine hydrochloride (1.93 g, 6.75 mmol) was added thereto, and the resulting mixture was stirred at room temperature for two hours. After 2 hours, the solution was extracted with ethyl acetate to obtain S-phenyl 3-sulfanylpropanethioate (1.29 g, 6.5 mmol) as a target product.

MS(ESI)m/z:199.2[M+H]⁺

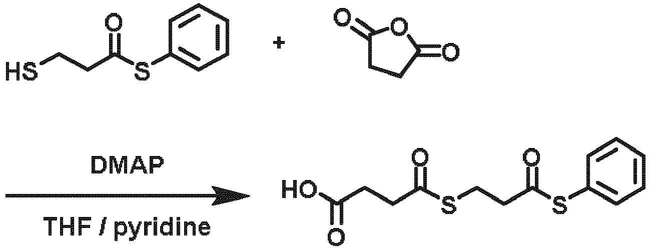

2.7 mL of acetonitrile and 0.3 mL of pyridine were added to and dissolved in S-phenyl 3-sulfanyl propanethioate (1.29 g, 6.5 mmol). Thereafter, succinic anhydride (0.65 g, 6.5 mmol) and 4-dimethylaminopyridine (12.2 mg, 0.1 mmol) were added thereto, and the resulting mixture was stirred for one hour. 20 mL of 1 M aqueous hydrochloric acid solution was added thereto, and the resulting mixture was extracted with ethyl acetate. The solution was concentrated under reduced pressure to obtain (4-oxo-4-(3-oxo-3-phenylsulfanyl-propyl) sulfanyl-butanoic acid (1.25 g, 4.2 mmol).

MS(ESI)m/z:299.0[M+H]⁺

### (81-1-2) Coupling between Thioester Linker and Peptide

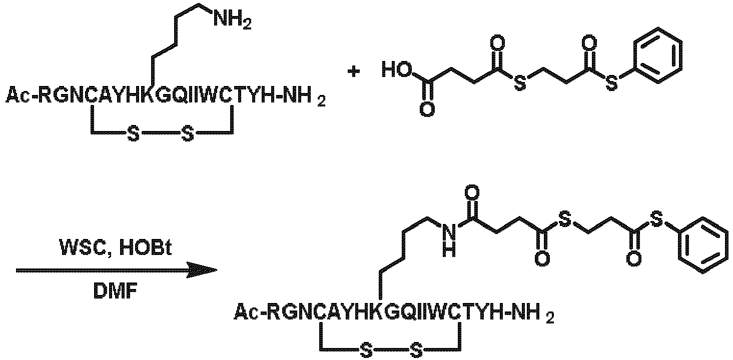

The amino acid sequence is SEQ ID NO: 5.

Ac-RGNCAYHKGQIIWCTYH-NH₂ (SEQ ID NO: 5) synthesized in (81-1) (30 mg, 14.4 µmol, the 4th and 14th of two cysteines form a disulfide bond in the molecule) was dissolved in 1 mL of N,N'-dimethylformamide, and ((4-oxo-4-(3-oxo-3-phenylsulfanyl-propyl) sulfanyl-butanoic acid (64 mg, 0.22 mmol) and WSC·HCl (41 mg, 0.22 mmol) were added thereto. The solution was stirred at room temperature for 12 hours, a 0.1% trifluoroacetic acid solution was added thereto, and fractions were eluted by reversed phase preparative chromatography. A fraction comprising a product was collected, was concentrated under reduced pressure to remove only acetonitrile, and was then freeze-dried to obtain the peptide- and linker-coupled thiophenol-activation compound (22.3 mg, 9.4 µmol).

MS(ESI)m/z:z=3 790[M+3H]³⁺,z=4 593[M+4H]⁴⁺

### (81-4) Specific Modification of Anti-HER2 IgG antibody Trastuzumab and Analysis by ESI-TOFMS

The peptide- and linker-coupled thiophenol-activation compound synthesized in (81-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 4.7), 4.7 µL of a 21.6 mM peptide reagent (30 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer.

### (81-5) Determination of Heavy Chain Selectivity of Specifically Modified Compound of Trastuzumab under Reduction Conditions by ESI-TOFMS Analysis

To the antibody-peptide conjugate formed in (81-4), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 52855 and 53017 with a peptide- and linker-coupled compound introduced to the heavy chain, and a light chain peak was observed at 23439, the same as that of the raw material.

### (81-6) HIC-UPLC Analysis of Specific Modification of Anti-HER2 IgG antibody Trastuzumab

The antibody-peptide conjugate formed in (81-4) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 30 equivalents of the peptide- and disulfide linker-coupled NHS-activation compound;

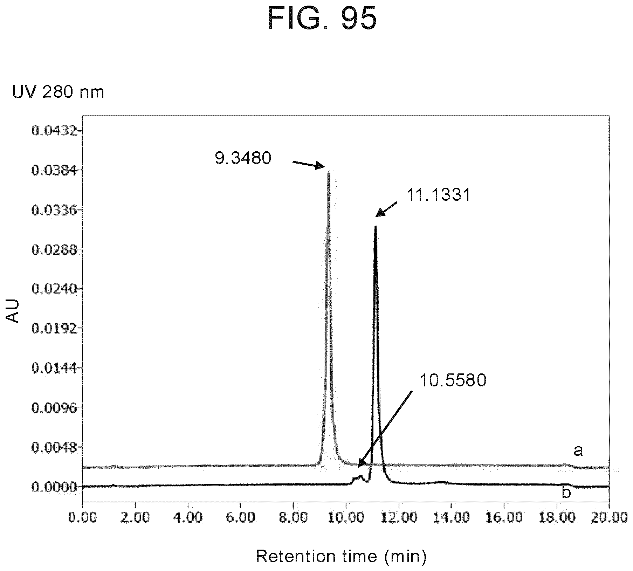

It is believed that a retension time of 10.5580 minutes is attributed to a compound with one peptide introduced to trastuzumab, whereas that of 11.1331 minutes is attributed to a compound with two peptides introduced to trastuzumab (FIG. 95).

### (81-7) Linker Cleavage of Trastuzumab-Peptide Conjugate

The trastuzumab-peptide conjugate (20 mM PBS buffer) obtained in (81-4) was substituted with a 0.5 M hydroxylamine 10 mM EDTA (pH 5.5) solution by Amicon 3K, and the solution was allowed to stand at room temperature for two hours. After two hours, the solution was substituted with 20 mM PBS buffer, 10 mM EDTA (pH 7.4) to obtain thiol-introduced trastuzumab.

### (81-8) Determination of Heavy Chain Selectivity of Specifically Modified Compound of Thiol-introduced Trastuzumab under Reduction Conditions by ESI-TOFMS Analysis

To the thiol-introduced trastuzumab formed in (81-7), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50682 and 50844 with a thiopropionyl group introduced to the heavy chain, and a light chain peak was observed at 23439, the same as that of the raw material.

### (81-9) Synthesis of Thiol-introduced Trastuzumab by One-pot Method

The peptide- and linker-coupled thiophenol-activation compound synthesized in (81-3) was dissolved in N,N'-dimethylformamide to be 21.6 mM. Anti-HER2 IgG antibody trastuzumab (Chugai Pharmaceutical) in an amount of 500 µg was dissolved in 46.9 µL of a 60 mM sodium acetate buffer (pH 5.5), 4.7 µL of a 21.6 mM peptide reagent (30 equivalents with respect to the antibody) was added thereto, and the solution was stirred at room temperature for one hour. Thereafter, a 0.5 M hydroxylamine 10 mM EDTA (pH 5.5) solution was added in the same amount as sodium acetate, and the resulting mixture was allowed to stand at room temperature for two hours. After two hours, the solution was substituted with 20 mM PBS buffer, 10 mM EDTA (pH 7.4) to obtain thiol-introduced trastuzumab.

### (81-10) Trypsin Treatment for Thiol-introduced Trastuzumab

The thiol-introduced trastuzumab obtained in (81-9) was glycosylated with PNGaseF (manufactured by New England BioLabs), and then subjected to peptide mapping. To a 1.5 mL low-adsorptive micro test tube, 10 µL of a sample solution, a 50 mM ammonium hydrogencarbonate buffer, and 10 µL of a 20 mM aqueous dithiothreitol solution dissolved in 40% trifluoroethanol were added, and the resulting solution was heated at 65°C for one hour. Thereafter, 10 µL of a 50 mM aqueous iodoacetamide solution was added thereto, and the resulting solution was reacted in a dark place at room temperature for 30 minutes. After the reaction, 40 µL of a 50 mM ammonium hydrogencarbonate buffer was added thereto, the resulting mixture was stirred, 10 µL of a 20 ng/µL aqueous trypsin solution was added thereto, and the resulting mixture was subjected to enzyme digestion at 37°C for 16 hours. After digestion, 2 µL of a 20% aqueous trifluoroacetic acid solution was added thereto to stop the reaction, for which LC-MS/MS measurement was performed.

### (81-11) LC-MS/MS Measurement Conditions for Trypsin Digestion

### (Analyzer)

Nano HPLC: EASY-nLC 1000 (Thermo Fisher Scientific)
Mass Spectrometer: Tribrid Mass Spectrometer Orbitrap Fusion (Thermo Fisher Scientific)

### (HPLC Analysis Conditions)

Trap column: Acclaim PepMap (registered trademark) 100, 75 µm × 2 cm, (Thermo Fisher Scientific)
Analysis column: ESI-column (NTCC-360/75-3-125, 75 µm × 12.5 cm, 3 µm (Nikkyo Technos Co., Ltd.))
Mobile Phase A: a 0.1% aqueous formate solution
Mobile Phase B: a 0.1% formate acetonitrile solution
Loading solution: a 0.1% aqueous trifluoroacetic acid solution
Flow rate: 300 nL/min
Sample injection amount: 1 µL
Gradient condition (B%): 2% (0.0 minute to 0.5 minute), 2% → 30% (0.5 minute to 23.5 minutes), 30% → 75% (23.5 minutes to 25.5 minutes), and 75% (25.5 minutes to 35.0 minutes).

### (Mass Spectrometer Analysis Conditions)

Ionization: ESI, Positive mode
Scan type: Data Dependent Acquisition
Activation Type:Collision Induced Dissociation(CID)
Data acquisition was performed using Xcalibur 3.0 (Thermo Fisher Scientific) and Thermo Orbitrap Fusion Tune Application 2.0 (Thermo Fisher Scientific) as accompanying software.

### (81-12) Analysis Condition of Modified Site of Trastuzumab

Modified site analysis of an LC-MS/MS measurement result was performed using Proteome Discoverer version 1.4 (Thermo Fisher Scientific).

For analysis with Proteome Discoverer, Sequest HT was used as a search engine, the range of precursor ion was set to 350 Da to 5,000 Da, and Total Intensity Threshold was set so as to be 0.01% of the intensity of a peak top. Trypsin was set as a digestive enzyme, and Maximum Missed Cleavage Sites was set to 3. Mass Tolerance was set to 5 ppm and 0.5 Da for precursor and fragment ion, respectively. For Static Modification, Carbamidomethyl (+57.021 Da) was set as modification of a cysteine residue with iodoacetamide. For Dynamic Modifications, oxidation of methionine residue (+15.995 Da) and a modified compound to a lysine residue (a thiol-introduced portion subjected to carbamidomethylation with iodoacetamide (+145.019 Da)) were set. Furthermore, a filter was applied so as to cause Peptide Confidence to be only High.

As data on amino acid sequences to be searched for a modified site, (1) and (3) illustrated in FIG. 4 were used.

### (81-13) Analysis Result of Modified Site of Trastuzumab by LC-MS/MS

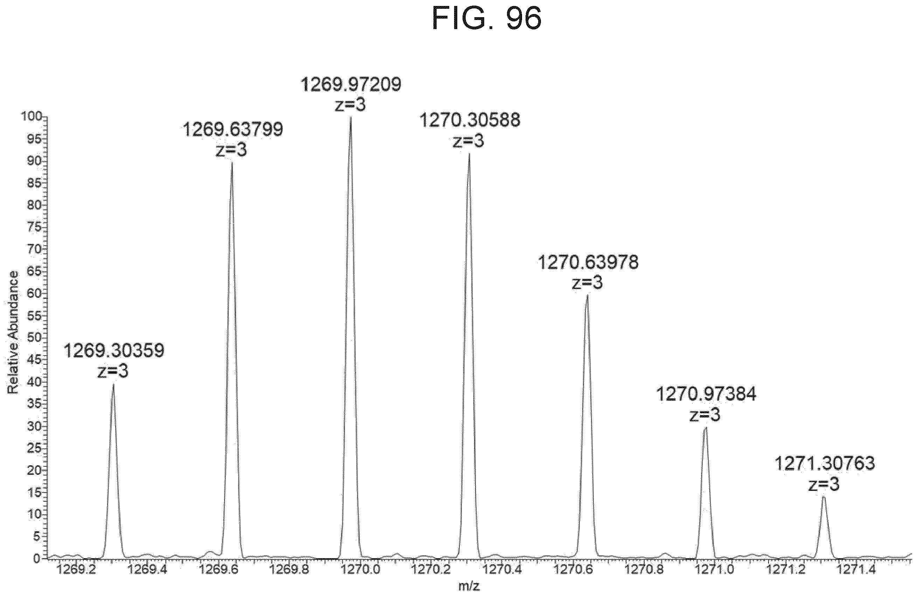

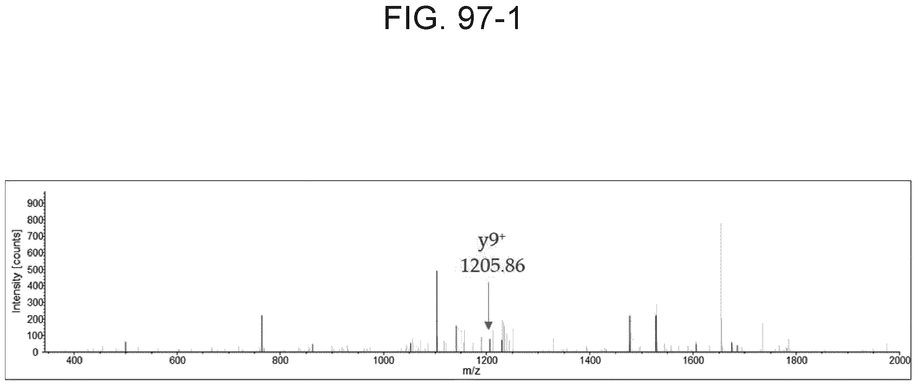

After analysis using LC-MS/MS, an MS spectrum of the peptide fragment of THTCPPCPAPELLGGPSVFLFPPKPKDTLMISR (SEQ ID NO: 6), which is a peptide consisting of 33 amino acid residues comprising a modified site to a lysin residue by trypsin digestion of trastuzumab (a thiol-introduced portion subjected to carbamidomethylation with iodoacetamide (+145.019 Da)) (measured value: m/z 1269.30359; theoretical value: 1269.30273; and trivalent) was observed (FIG. 96), and from a CID spectrum, a product ion of m/z 1205.86 (theoretical value: 1205.60) corresponding to monovalent y9 indicating modification of a lysine residue at position 246 or position 248 of a heavy chain in EU numbering was determined (FIG. 97-1). In addition, analysis with Proteome Discoverer indicated that modification to a lysine residue at position 246 or 248 occurred highly selectively (FIG. 97-2).

From this result, it was found that in (81-9), conjugation progressed regioselectively at Lys246 and Lys248 in EU numbering on the heavy chain on the antibody.

### Example 82: Synthesis of Antibody-Nucleic Acid Conjugate

### (82-1-1) Introduction of Linker to Nucleic Acid

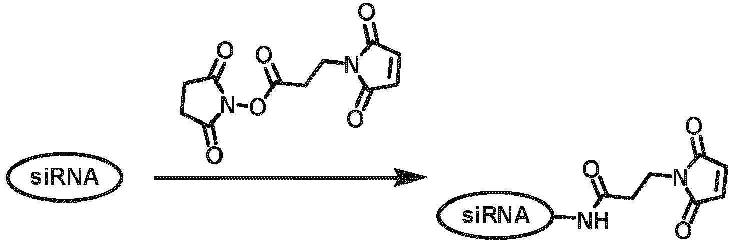

To siRNA (pH 7.4 PBS buffer), 100 equivalents of 3-(2,5-dioxopyrrol-1-yl) propanoic acid (2,5-dioxopyrrolidin-1-yl) (200 mM, dissolved in DMF) were added. The solution was allowed to stand at 4°C for 20 hours and then purified using NAP-5 Columns (manufactured by GE Healthcare).

siRNA having the following sequence was used.
Sense strand: 5'-6-FAM-A(M)C(M)AGC(M)AAAU(M)U(M)C(M)C(M)AU(M)C(M)GU(M)GU(M)-N(6) (SEQ ID NO: 93)
Antisense strand: 5'-p-AC(F)AC(F)GAU(F)GGAAU(F)U(F)U(F)GC(F)U(F)GU(F)UU (SEQ ID NO: 94)
· p: Phosphorylation, (F): 2'-Fluoro, (M): 2'-O-Me (methyl), and N(6): amino C6 linker were added to a 3' terminal.

### (82-1-2) Synthesis of Thiol-introduced Trastuzumab-Nucleic Acid Conjugate

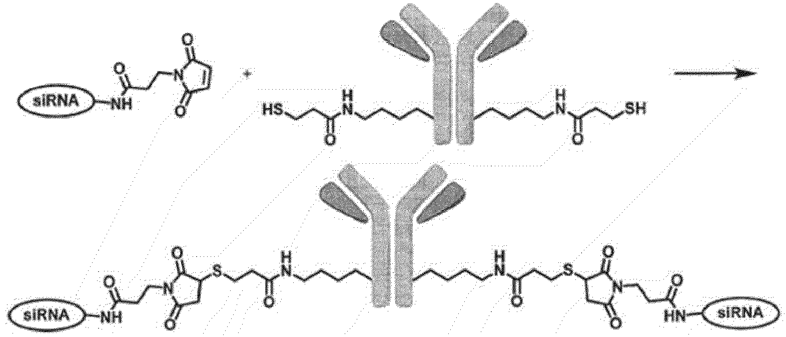

To the thiol-introduced trastuzumab (20 mM PBS buffer, 10 mM EDTA) obtained in (81-7), 10 equivalents of the linker-introduced siRNA obtained in (210-1-1) were added, and
the resulting mixture was allowed to stand at 4°C for 40 hours. The mixed solution was purified using Amicon Ultra-0.5 mL 3 kDa (manufactured by Merck Millipore) and then further purified using AKTA purifier (manufactured by GE Healthcare). The purified conjugate was analyzed by SDS-PAG.

The purification using AKTA purifier (manufactured by GE Healthcare) was performed under the following conditions.
Column: Superdex 200 Increase10/300GL (manufactured by GE Healthcare)
Buffer: pH 7.4 PBS buffer
Flow rate: 0.4 ml/min
Detector: detected at wavelengths of 215 and 280 nm.

### (82-2) Analysis of Antibody-Nucleic Acid Conjugate by SDS-PAGE

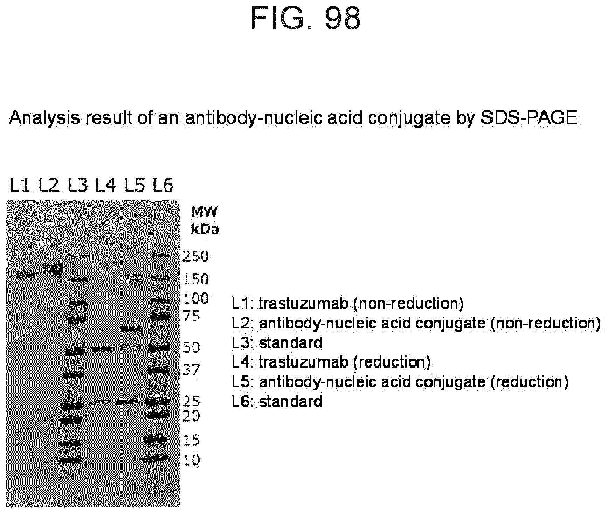

The conjugate synthesized in (82-1-2) was analyzed by SDS-PAGE. Precision Plus Protein™ Prestained Standard (manufactured by BIO-RAD) was used as a standard, and staining was performed with Barrett CBB Stain One (manufactured by Nacalai Tesque) (FIG. 98).

As a result, it was found from SDS-PAGE that the nucleic acid could be conjugated to the unmodified antibody regioselectively. That is, it was indicated that not only a small molecule but also a nucleic acid could be introduced as Payload.

### Example 83: Introduction of Azide Group to Thiol-introduced Trastuzumab and Synthesis of ADC mimic Using Click Reaction

### (83-1) Introduction of Azide Group to Thiol-introduced Trastuzumab and Synthesis of ADC mimic

### (83-1-1) Introduction of Azide Group to Thiol-introduced Trastuzumab

To the thiol-introduced trastuzumab (20 mM PBS buffer, 10 mM EDTA) obtained in (81-7), 50 equivalents of N-[2-[2-(2-azidoethoxy) ethoxy] ethyl]-2-iodo-acetamide (50 mM, dissolved in DMF) were added. The solution was allowed to stand at 37°C for three hours and then substituted with a 20 mM PBS buffer to obtain azide-introduced trastuzumab.

### (83-1-2) Determination of Heavy Chain Selectivity of Azide-introduced Trastuzumab under Reduction Conditions by ESI-TOFMS Analysis

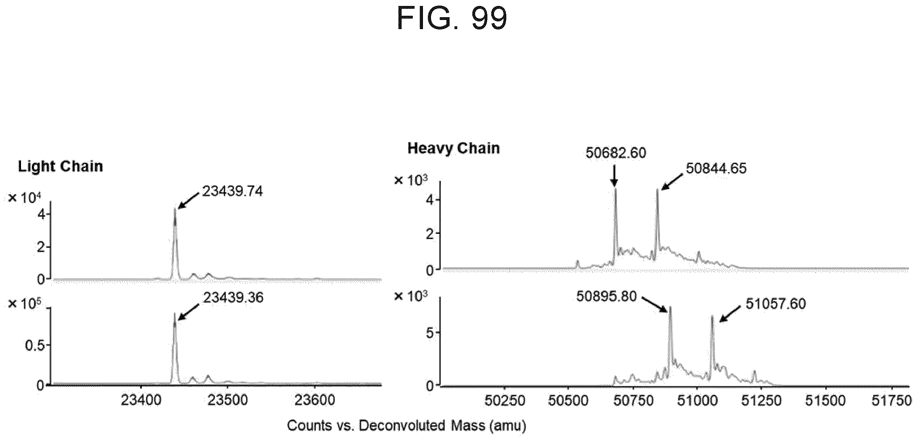

To the azide-introduced trastuzumab formed in (83-1-1), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material thiol-introduced trastuzumab, heavy chain peaks were observed at 50682 and 50844, and a light chain peak was observed at 23439, whereas for a product, peaks were observed at 50895 and 51057 with a click reaction product introduced to the heavy chain, and a light chain peak was observed at 23439, the same as that of the raw material (FIG. 99).

### (83-1-3) Conversion of Azide-introduced Trastuzumab to ADC mimic

To the azide-introduced trastuzumab (20 mM PBS buffer, 10 mM EDTA) obtained in (83-1-1), 10 equivalents of dibenzocyclooctynoic acid (DBCO-Acid, 10 mM, dissolved in DMF) were added to perform a click reaction, thus obtaining ADC mimic.

### (83-1-4) Determination of Heavy Chain Selectivity of ADC mimic under Reduction Conditions by ESI-TOFMS Analysis

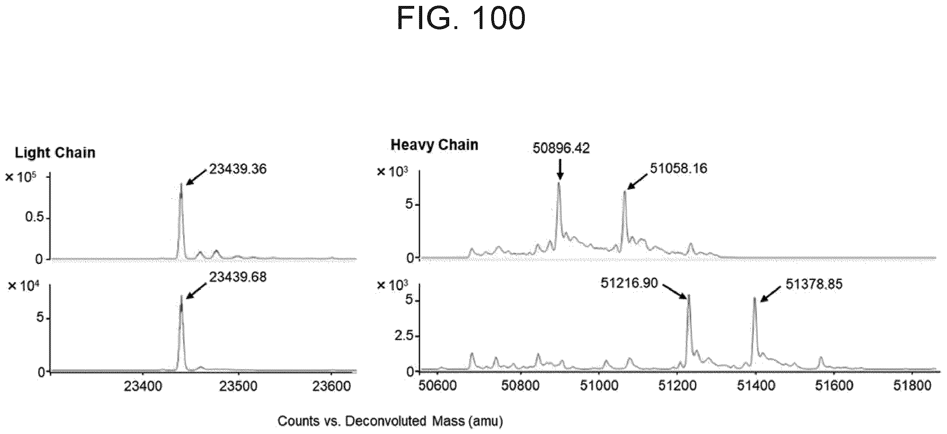

To the ADC mimic formed in (83-1-3), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material azide-introduced trastuzumab, heavy chain peaks were observed at 50896 and 51058, and a light chain peak was observed at 23439, whereas for a product, peaks were observed at 51216 and 51378 with a click reaction product introduced to the heavy chain, and a light chain peak was observed at 23439, the same as that of the raw material (FIG. 100).

As a result, it was indicated that ADC mimic could be synthesized even if a linker was inserted between the thiol-introduced trastuzumab and Payload.

### [Example 84: Synthesis of Cleavable Linker and Coupling thereof with IgG1 Fc-Binding Peptide]

### (84) Synthesis of Azide-Containing Cleavable Linker and Coupling thereof with Peptide

### (84-1) Synthesis of Azide-Containing Thioester Linker and Coupling thereof with Peptide

### (84-1-1) Synthesis of Azide-Containing Thioester Linker

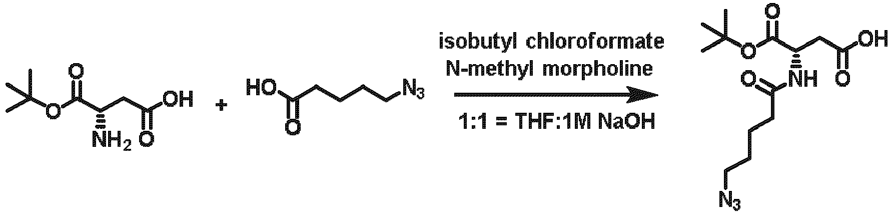

5-Azidopentanoic acid (200 mg, 1.40 mmol) was dissolved in a THF solvent (7.0 mL). Isobutyl chloroformate (0.220 mL, 1.68 mmol) and N-methylmorpholine (0.230 mL, 2.10 mmol) were added thereto at 0°C, and the solution was stirred for 30 minutes to prepare a mixed anhydride of 5-azidopentanoic acid.(3S)-3-Amino-4-tert-butoxy-4-oxo-butanoic acid (0.397 mg, 2.10 mmol) was dissolved in a 1 M aqueous sodium hydroxide solution (7.0 mL), and the mixed solution was added dropwise to the above-described THF solution of the mixed anhydride of 5-azidopentanoic acid at room temperature. The solution was stirred at room temperature for 16 hours, and then a 6 M aqueous hydrochloric acid solution was added thereto to adjust the pH within the system to 3.0. After the adjustment, the solution was subjected to liquid separation extraction with ethyl acetate, and the organic phase was concentrated under reduced pressure to obtain a crude product, which was purified by column chromatography (dichloromethane : methanol = 10 : 1). A fraction comprising a product was collected and was concentrated under reduced pressure to obtain (3S)-3-(5-azidopentanoylamino)-4-tert-butoxy-4-oxo-butanoic acid (0.380 g, 1.21 mmol).

MS(ESI) m/z:337[M+Na]⁺

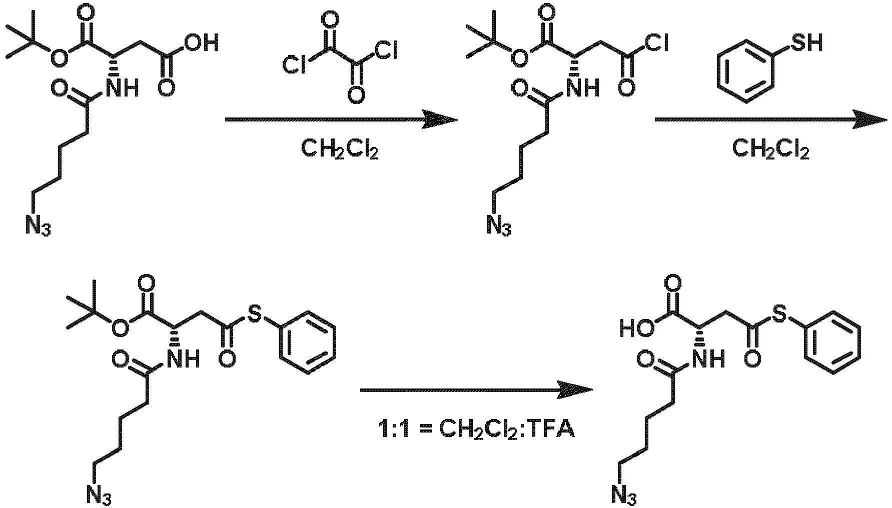

(3S)-3-(5-azidopentanoylamino)-4-tert-butoxy-4-oxo-butanoic acid (0.380 g, 1.21 mmol) was dissolved in 6.0 mL of dichloromethane. Oxalyl chloride (0.125 mL, 1.45 mmol) and N,N'-dimethylformamide (4.68 µL, 0.0605 mmol) were added dropwise thereto under ice cooling, and the resulting mixture was stirred for 30 minutes. After 30 minutes, a mixed solution of pyridine (0.196 mL, 2.42 mmol), thiophenol (0.185 mL, 1.81 mmol), and dimethylaminopyridine (14.8 mg, 0.121 mmol) was added dropwise thereto, and the resulting mixture was stirred at room temperature for two hours. After two hours, 6.0 mL of a 0.1 M aqueous hydrochloric acid solution was added thereto, and the resulting mixture was subjected to liquid separation extraction with dichloromethane. Thereafter, the organic layer was concentrated under reduced pressure and purified by column chromatography (hexane/ethyl acetate). A fraction comprising a product was collected and was concentrated under reduced pressure to obtain a residue. To the residue, 3.0 mL of dichloromethane and 3.0 mL of trifluoroacetic acid were added, and the resulting mixture was stirred at room temperature for one hour. After one hour, the reaction solution was concentrated under reduced pressure to obtain (2S)-2-(5-azidopentanoylamino)-4-oxo-4-phenylsulfanyl-butanoic acid (150 mg, 0.428 mmol).

MS(ESI)m/z:351[M+H]⁺

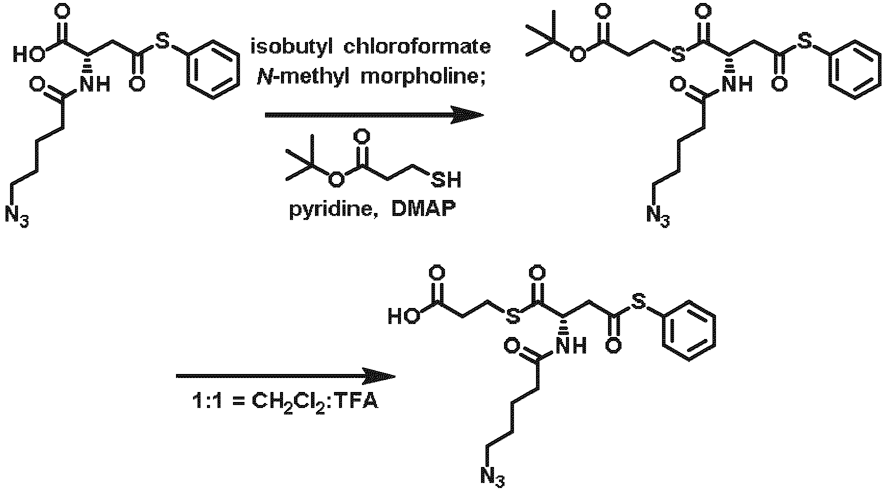

(2S)-2-(5-Azidopentanoylamino)-4-oxo-4-phenylsulfanyl-butanoic acid (0.150 g, 0.428 mmol) was dissolved in a THF solvent (4.3 mL), isobutyl chloroformate (0.0674 mL, 0.514 mmol) and N-methylmorpholine (0.0706 mL, 0.642 mmol) were added thereto at 0°C, and the solution was stirred for 30 minutes to prepare a mixed anhydride. Pyridine (0.0695 mL, 0.857 mmol), tert-butyl 3-sulfanylpropanoate (0.139 mL, 0.857 mmol), and dimethylaminopyridine (5.20 mg, 0.0428 mmol) were dissolved in a THF solvent (1.0 mL), and the mixed solution was added dropwise to the above-described THF solution of the mixed anhydride at room temperature. The solution was stirred at room temperature for three hours. Thereafter, 4.3 mL of a 0.1 M aqueous hydrochloric acid solution was added thereto, and the resulting mixture was subjected to liquid separation extraction with ethyl acetate. Thereafter, the organic layer was concentrated under reduced pressure and purified by column chromatography (hexane/ethyl acetate). A fraction comprising a product was collected and was concentrated under reduced pressure to obtain a residue. To the residue, 2.0 mL of dichloromethane and 2.0 mL of trifluoroacetic acid were added, and the resulting mixture was stirred at room temperature for one hour. After one hour, the reaction solution was concentrated under reduced pressure to obtain 3-[(2S)-2-(5-azidopentanoylamino)-4-oxo-4-phenylsulfanyl-butanoyl] sulfanylpropanoic acid (43.8 mg, 0.100 mmol) .

MS(ESI)m/z:439[M+H]⁺

### (84-1-2) Coupling between Thioester Linker and Peptide

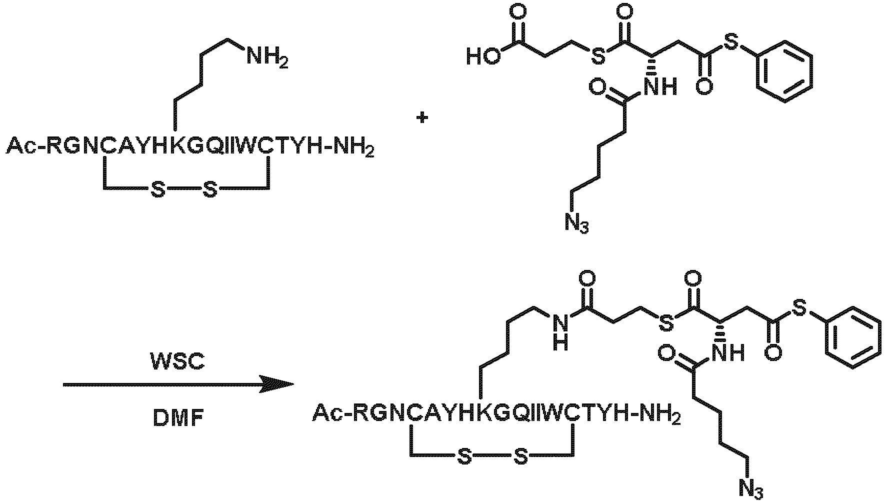

The amino acid sequence is SEQ ID NO: 5.

Ac-RGNCAYHKGQIIWCTYH-NH₂ (SEQ ID NO: 5) synthesized in (84-1) (9.8 mg, 4.7 µmol, the 4th and 14th of two cysteines form a disulfide bond in the molecule) was dissolved in 1 mL of N,N'-dimethylformamide, and 3-[(2S)-2-(5-azidopentanoylamino)-4-oxo-4-phenylsulfanyl-butanoyl] sulfanylpropanoic acid (30.9 mg, 0.0704 mmol) and WSC·HCl (13.5 mg, 0.0704 mmol) were added thereto. The solution was stirred at room temperature for three hours, a 0.1% trifluoroacetic acid solution was added thereto, and fractions were eluted by reversed phase preparative chromatography. A fraction comprising a product was collected, was concentrated under reduced pressure to remove only acetonitrile, and was then freeze-dried to obtain the peptide- and linker-coupled thiophenol-activation compound (3.76 mg, 1.50 µmol).

MS(ESI)m/z:z=2 1255 [M+2H]²⁺, z=3 837[M+3H]³⁺

### (84-2) Specific Modification of Anti-HER2 IgG antibody Trastuzumab and Analysis by ESI-TOFMS

The peptide- and linker-coupled thiophenol-activation compound synthesized in (84-1) was dissolved in N,N'-dimethylformamide to be 5.01 mM. Anti-HER2 IgG antibody trastuzumab (Chugai Pharmaceutical) in an amount of 707 µg was dissolved in 220 µL of a 60 mM sodium acetate buffer (pH 5.5), 19.1 µL of a 5.01 mM peptide reagent (10 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer.

### (84-3) Determination of Heavy Chain Selectivity of Specifically Modified Compound of Trastuzumab under Reduction Conditions by ESI-TOFMS Analysis

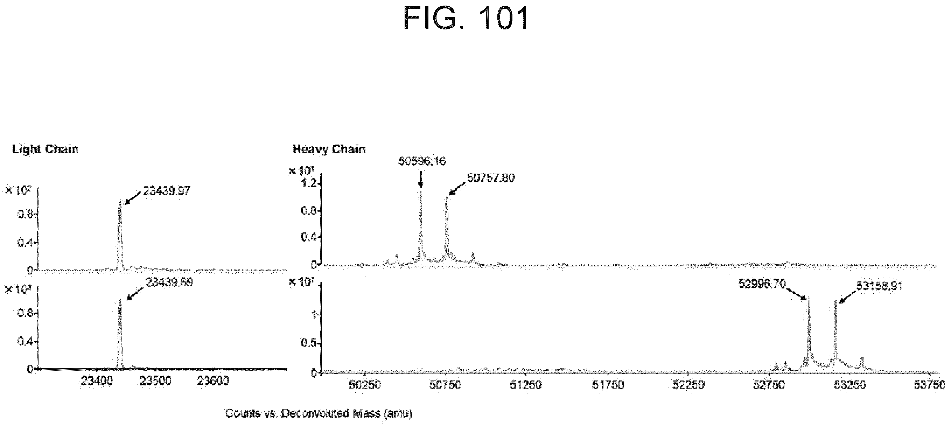

To the antibody-peptide conjugate formed in (84-2), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 52996 and 53158 with a peptide- and linker-coupled compound introduced to the heavy chain, and a light chain peak was observed at 23439, the same as that of the raw material (FIG. 101).

### (84-4) HIC-UPLC Analysis of Specific Modification of Anti-HER2 IgG antibody Trastuzumab

The antibody-peptide conjugate formed in (84-2) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: a trastuzumab raw material;
b: trastuzumab + 10 equivalents of the peptide- and azide linker-coupled NHS-activation compound;

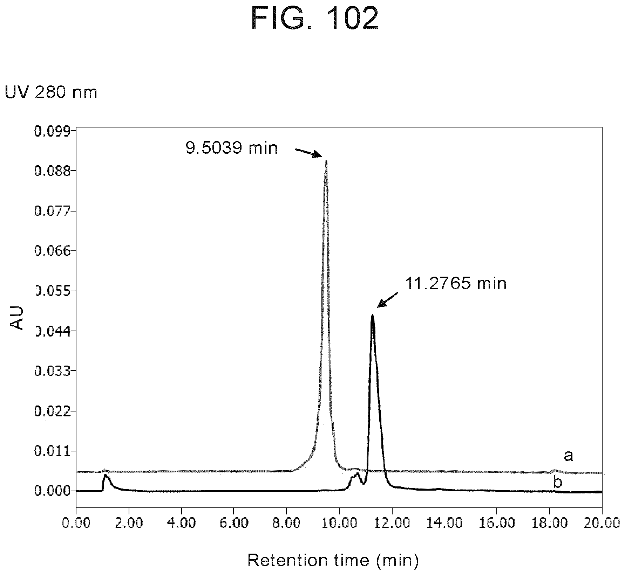

It is believed that a retension time of 11.2765 minutes is attributed to a compound with two peptides introduced to trastuzumab (FIG. 102).

### (84-5) Linker Cleavage of Trastuzumab-Peptide Conjugate

The trastuzumab-peptide conjugate (20 mM PBS buffer) obtained in (84-2) was substituted with a 0.5 M hydroxylamine 10 mM EDTA (pH 6.0) solution by Amicon 10K, and the solution was allowed to stand at room temperature for two hours. After two hours, the solution was substituted with 20 mM PBS buffer, 10 mM EDTA (pH 7.4) to obtain thiol-introduced trastuzumab.

### (84-6) Determination of Heavy Chain Selectivity of Specifically Modified Compound of Azide-introduced Trastuzumab under Reduction Conditions by ESI-TOFMS Analysis

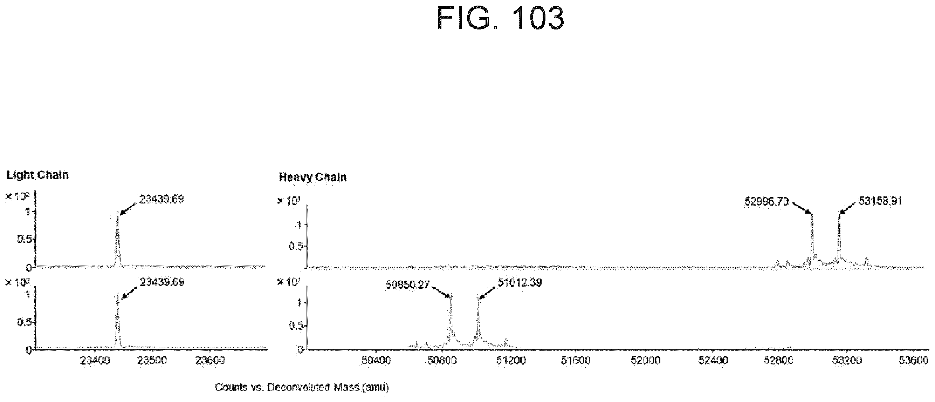

To the thiol-introduced trastuzumab formed in (84-5), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material trastuzumab, heavy chain peaks were observed at 50594 and 50756, and a light chain peak was observed at 23439, whereas for a product, peaks were observed at 50850 and 51012 with a thiopropionyl group introduced to the heavy chain, and a light chain peak was observed at 23439, the same as that of the raw material (FIG. 103)

### (84-7) HIC-UPLC analysis of Specific Modification of Azide-introduced Trastuzumab

The azide-introduced antibody formed in (84-5) and the raw material antibody-peptide conjugate were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: trastuzumab-peptide conjugate
b: azide-introduced trastuzumab;

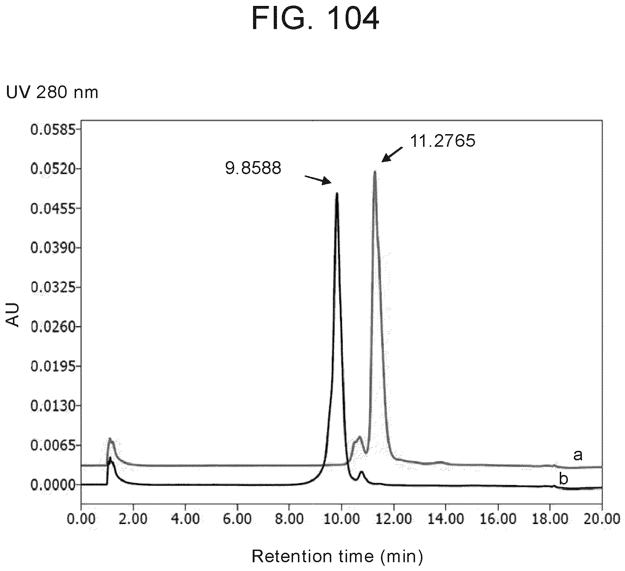

It is believed that a retension time of 9.8588 minutes is attributed to a compound with two azides introduced to trastuzumab (FIG. 104).

### (84-8) Synthesis of Azide-introduced Trastuzumab by One-pot Method

The peptide- and linker-coupled thiophenol-activation compound synthesized in (84-1) was dissolved in N,N'-dimethylformamide to be 21.1 mM. Anti-HER2 IgG antibody trastuzumab (Chugai Pharmaceutical) in an amount of 1.29 mg was dissolved in 123.8 µL of a 60 mM sodium acetate buffer (pH 5.5), 10.8 µL of a 21.1 mM peptide reagent (15 equivalents with respect to the antibody) was added thereto, and the solution was stirred at room temperature for one hour. Thereafter, a 0.5 M hydroxylamine 10 mM EDTA (pH 5.5) solution was added in the same amount as sodium acetate, and the resulting mixture was allowed to stand at room temperature for two hours. After two hours, the solution was substituted with 20 mM PBS buffer, 10 mM EDTA (pH 7.4) to obtain azide-introduced trastuzumab.

### (84-9) Trypsin Treatment for Azide-introduced Trastuzumab

The azide-introduced trastuzumab obtained in (84-8) was treated in a manner similar to the above (81-10), for which LC-MS/MS measurement was performed.

### (84-10) LC-MS/MS Measurement Conditions for Trypsin Digestion

An analyzer, HPLC analysis conditions, and mass spectrometer analysis conditions are similar to those in the above (81-11).

### (84-11) Analysis Condition of Modified Site of Trastuzumab

Modified site analysis for the LC-MS/MS measurement result is similar to that of the above (81-12) except for setting of Dynamic Modifications. For Dynamic Modifications, oxidation of methionine residue (+15.995 Da) and modified compound to a lysine residue (azide-introduced portion (+255.097 Da), amine-introduced portion (+229.106), azidocarboxylic acid-introduced portion (+240.086), aminecarboxylic acid-introduced portion (+214.095)) were set. Furthermore, a filter was applied so as to cause Peptide Confidence to be only High.

### (84-12) Analysis Result of Modified Site of Trastuzumab by LC-MS/MS

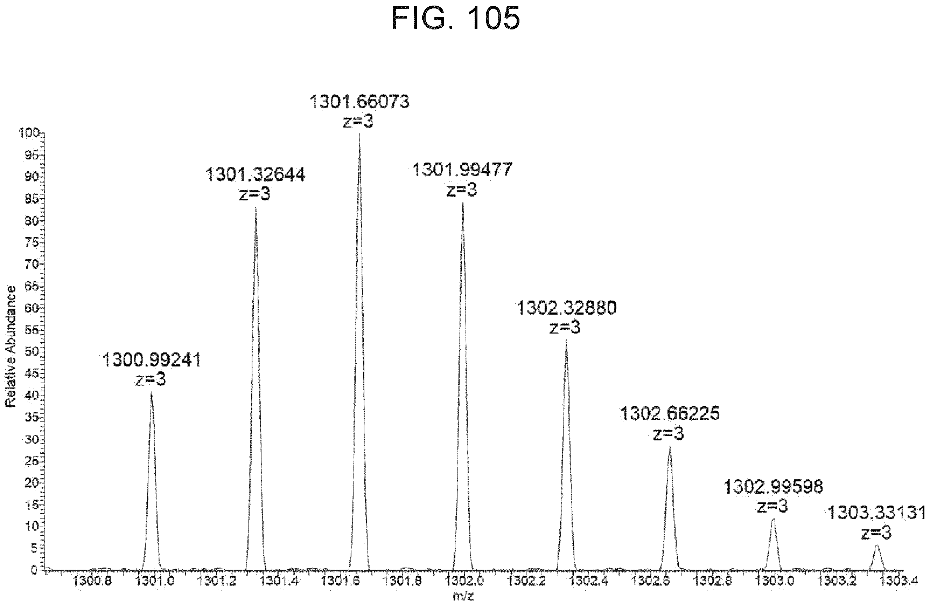

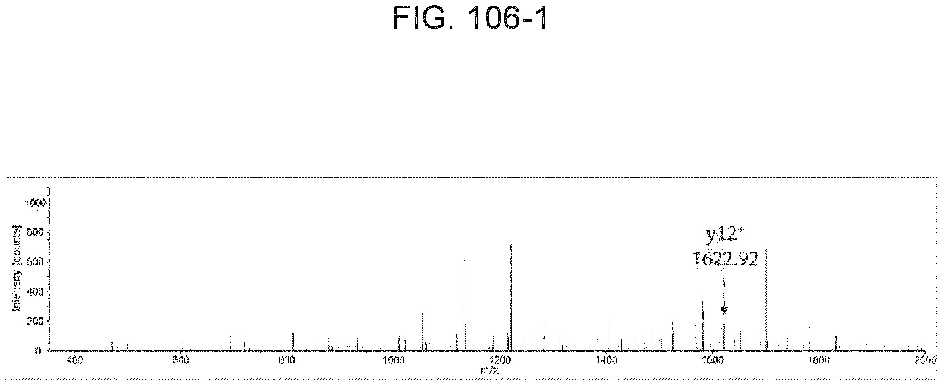

After analysis using LC-MS/MS, an MS spectrum of the peptide fragment of THTCPPCPAPELLGGPSVFLFPPKPKDTLMISR (SEQ ID NO: 6), which is a peptide consisting of 33 amino acid residues comprising a modified site to a lysine residue by trypsin digestion of trastuzumab (an azidocarboxylic acid-introduced portion (+240.086 Da)) (measured value: m/z 1300.99241; theoretical value: 1300.99173; and trivalent) was observed (FIG. 105); and from a CID spectrum, a product ion of m/z 1622.92 (theoretical value: 1622.87) corresponding to monovalent y12 indicating modification of a lysine residue at position 246 or position 248 of a heavy chain in EU numbering was determined (FIG. 106-1). In addition, analysis with Proteome Discoverer indicated that modification to a lysine residue at position 246 or 248 occurred highly selectively (FIG. 106-2).

From this result, it was found that in (84-8), conjugation progressed regioselectively at Lys246 and Lys248 in EU numbering on the heavy chain on the antibody.

### Example 85: Synthesis of ADC mimic using Click Reaction to Azide-introduced Trastuzumab

### (85-1) Small Molecule Introduction to Azide-introduced Trastuzumab

To the azide-introduced trastuzumab (20 mM PBS buffer, 10 mM EDTA) obtained in (84-5), 10 equivalents of dibenzocyclooctynoic acid (DBCO-Acid, 10 mM, dissolved in DMF) were added to perform a click reaction, thus obtaining ADC mimic.

### (85-2) Determination of Heavy Chain Selectivity of ADC mimic under Reduction Conditions by ESI-TOFMS Analysis

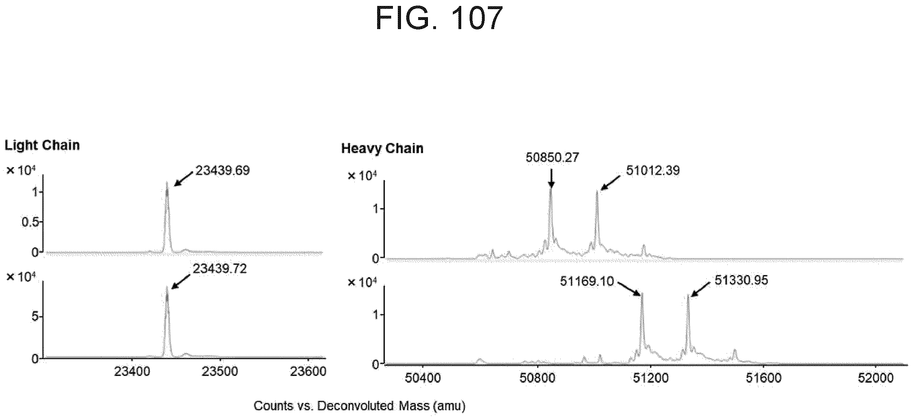

To the ADC mimic formed in (85-1), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material azide-introduced trastuzumab, heavy chain peaks were observed at 50850 and 51012, and a light chain peak was observed at 23439, whereas for a product, peaks were observed at 51169 and 51330 with a click reaction product introduced to the heavy chain, and a light chain peak was observed at 23439, the same as that of the raw material (FIG. 107).

### (85-3) HIC-UPLC analysis of Specific Modification of Azide-introduced Trastuzumab

The azide-introduced antibody formed in (85-1) and the raw material antibody-peptide conjugate were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: azide-introduced trastuzumab
b: ADC mimic;

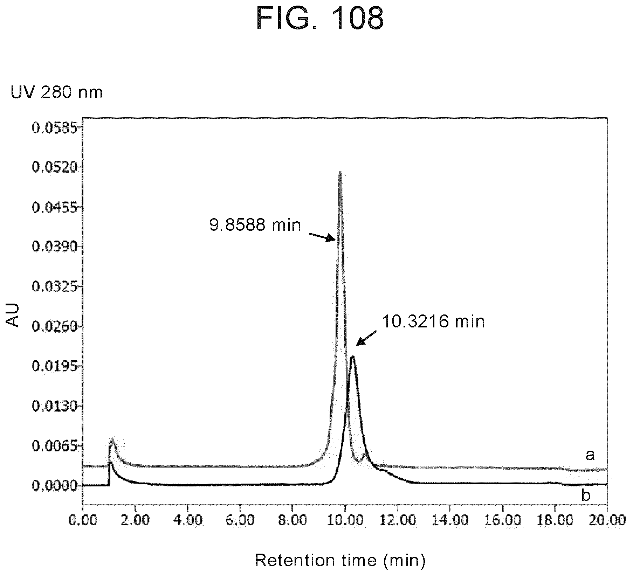

It is believed that a retension time of 10.3216 minutes is attributed to a compound with two small molecules introduced to trastuzumab (FIG. 108).

### (85-4) Trypsin Treatment for ADC mimic

The ADC mimic obtained in (85-1) was treated in a manner similar to the above (81-10), for which LC-MS/MS measurement was performed.

### (85-5) LC-MS/MS Measurement Conditions for Trastuzumab

An analyzer, HPLC analysis conditions, and mass spectrometer analysis conditions are similar to those in the above (81-11).

### (85-6) Analysis Condition of Modified Site of Trastuzumab

Modified site analysis for the LC-MS/MS measurement result is similar to that of the above (81-12) except for setting of Dynamic Modifications. For Dynamic Modifications, oxidation of methionine residue (+15.995 Da) and modified compound to a lysine residue (DBCO-Acid-introduced portion (+574.218), DBCO-Acid carboxylic acid-introduced portion (+559.207), azide-introduced portion (+255.097), azidocarboxylic acid-introduced portion (+240.086)) were set. Furthermore, a filter was applied so as to cause Peptide Confidence to be only High.

### (85-7) Analysis Result of Modified Site of Trastuzumab by LC-MS/MS

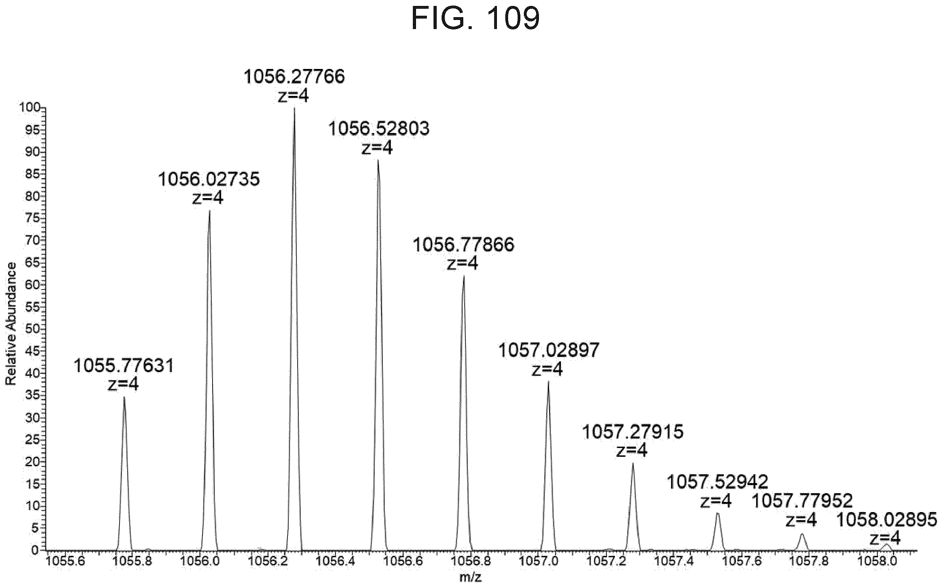

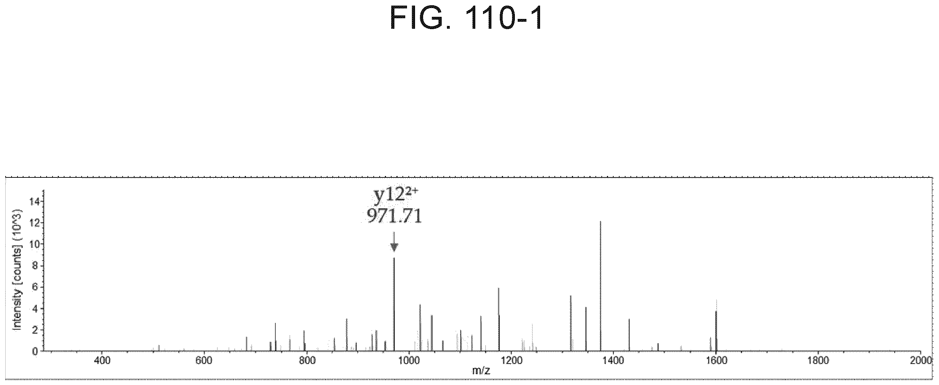

After analysis using LC-MS/MS, an MS spectrum of the peptide fragment of THTCPPCPAPELLGGPSVFLFPPKPKDTLMISR (SEQ ID NO: 6), which is a peptide consisting of 33 amino acid residues comprising a modified site to a lysine residue by trypsin digestion of trastuzumab (a DBCO-Acid carboxylic acid-introduced portion (+559.207)) (measured value: m/z 1055.77631; theoretical value: 1055.77600; and tetravalent) was observed (FIG. 109); and from a CID spectrum, a product ion of m/z 971.71 (theoretical value: 971.50) corresponding to divalent y12 indicating modification of a lysine residue at position 246 or position 248 of a heavy chain in EU numbering was determined (FIG. 110-1). In addition, analysis with Proteome Discoverer indicated that modification to a lysine residue at position 246 or 248 occurred highly selectively (FIG. 110-2).

From this result, it was found that in (85-1), conjugation progressed regioselectively at Lys246 and Lys248 in EU numbering on the heavy chain on the antibody.

### Example 86: Specific Modification of Anti-TNF-α IgG1 Antibody Adalimumab

### (86-1) Specific Modification of Anti-TNF-α IgG1 Antibody Adalimumab and Analysis by ESI-TOFMS

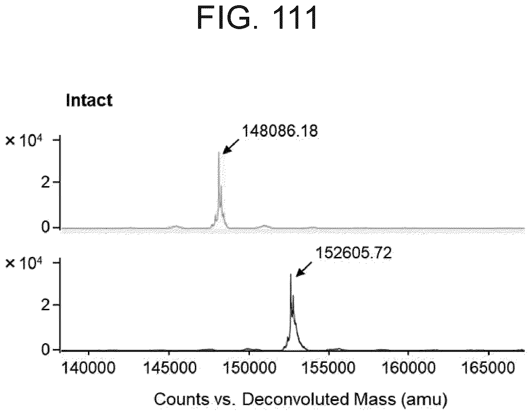

The peptide- and linker-coupled thiophenol-activation compound synthesized in (81-3) was dissolved in N,N'-dimethylformamide to be 21.6 mM. Anti-TNF-α IgG1 antibody adalimumab (Eisai Co., Ltd.) in an amount of 500 µg was dissolved in 200 µL of a 50 mM sodium acetate buffer (pH 5.5), 4.7 µL of a 4 mM peptide reagent (10 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material adalimumab, a peak was observed at 148086, whereas for a product with two binding peptides introduced, a peak was observed at 152605 (FIG. 111).

### (86-2) HIC-UPLC analysis of Specific Modification of Anti-TNF-α IgG1 Antibody Adalimumab

The adalimumab-peptide conjugate formed in (86-1) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: adalimumab raw material
b: adalimumab + 10 equivalents of peptide- and linker-coupled thiophenol-activation compound;

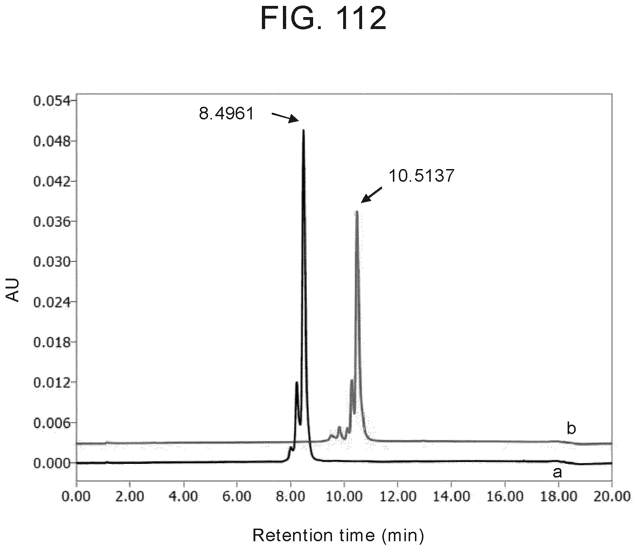

It is believed that a retension time of 10.5137 minutes is attributed to a compound with two peptides introduced to trastuzumab (FIG. 112).

### (86-3) Linker Cleavage of Adalimumab-Peptide Conjugate

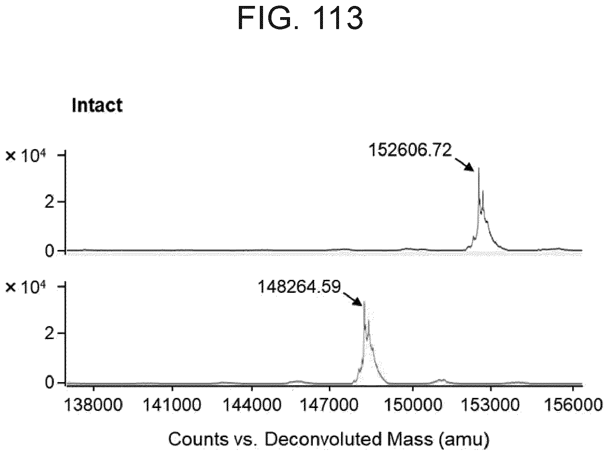

The adalimumab-peptide conjugate (20 mM PBS buffer) obtained in (86-2) was substituted with a 0.5 M hydroxylamine 10 mM EDTA (pH 5.5) solution by Amicon 3K, and the solution was allowed to stand at room temperature for two hours. After two hours, the solution was substituted with 20 mM PBS buffer, 10 mM EDTA (pH 7.4) to obtain thiol-introduced adalimumab. The mass was measured by ESI-TOFMS; for the raw material adalimumab-peptide conjugate, a peak was observed at 152606, whereas for thiol-introduced adalimumab as a product, a peak was observed at 148264 (FIG. 113).

### (86-4) HIC-UPLC Analysis of Thiol-introduced Adalimumab

The adalimumab-peptide conjugate formed in (863) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: adalimumab-peptide- and linker-coupled compound
b: thiol-introduced adalimumab;

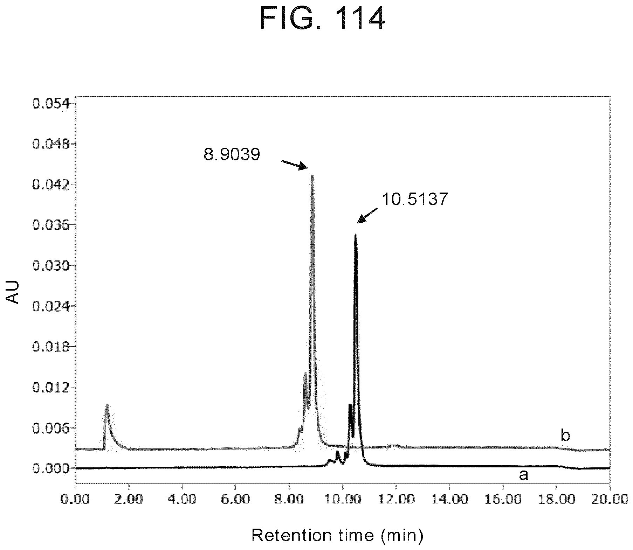

It is believed that a retension time of 8.9039 minutes is attributed to a compound with two thiols introduced to trastuzumab (FIG. 114).

### (86-5) Fluorescent labeling of Thiol-Introduced Adalimumab

To the thiol-introduced adalimumab (20 mM PBS buffer, 10 mM EDTA) obtained in (86-3), 5 equivalents of fluorescein-5-maleimide (10 mM, dissolved in DMSO) were added. The solution was allowed to stand at room temperature for one hour and then substituted with a 20 mM PBS buffer to obtain fluorescently labeled adalimumab (ADC mimic).

### (86-6) Determination of Heavy Chain Selectivity of Fluorescently Labeled Adalimumab under Reduction Conditions by ESI-TOFMS Analysis

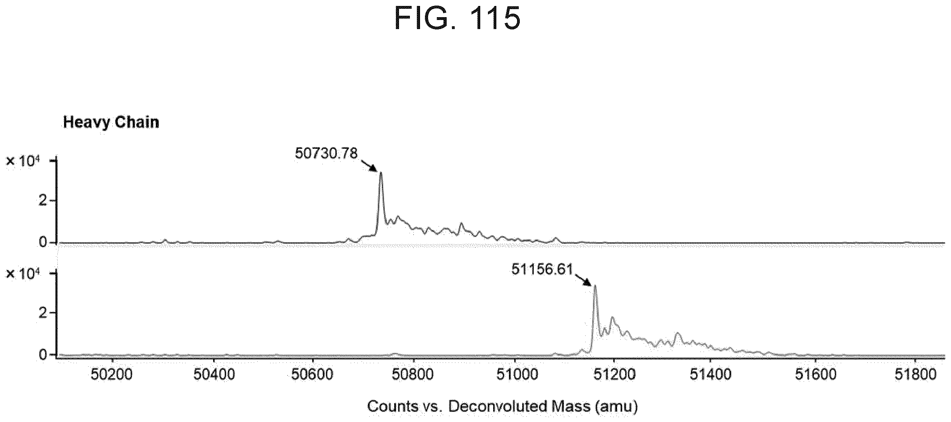

To the fluorescently labeled adalimumab (ADC mimic) formed in (86-5), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material and a product, the same light chain peak was observed, whereas for the raw material adalimumab, a heavy chain peak was observed at 50730, and for the product, a peak was observed at 51156 with fluorescein introduced to the heavy chain (FIG. 115).

### Example 87: Specific Modification of Anti-RANKL IgG2 Antibody Denosumab

### (87-1) Specific Modification of Anti-RANKL IgG2 Antibody Denosumab and Analysis by ESI-TOFMS

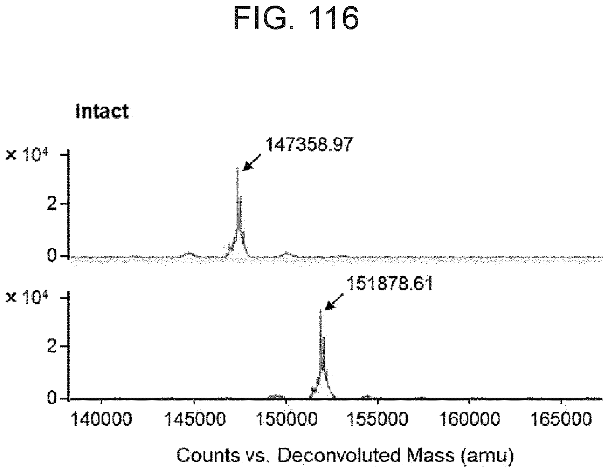

The peptide- and linker-coupled thiophenol-activation compound synthesized in (81-3) was dissolved in N,N'-dimethylformamide to be 21.6 mM. Anti-RANKL IgG2 antibody denosumab (Daiichi Sankyo Co., Ltd.) in an amount of 500 µg was dissolved in 200 µL of a 50 mM sodium acetate buffer (pH 5.5), 4.7 µL of a 4 mM peptide reagent (10 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material denosumab, a peak was observed at 147358.97, whereas for a product with two binding peptides introduced, a peak was observed at 151878.61 (FIG. 116).

### (87-2) HIC-UPLC analysis of Specific Modification of Anti-RANKL IgG2 Antibody Denosumab

The denosumab-peptide conjugate formed in (87-1) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: denosumab raw material
b: denosumab + 10 equivalents of peptide- and linker-coupled thiophenol-activation compound;

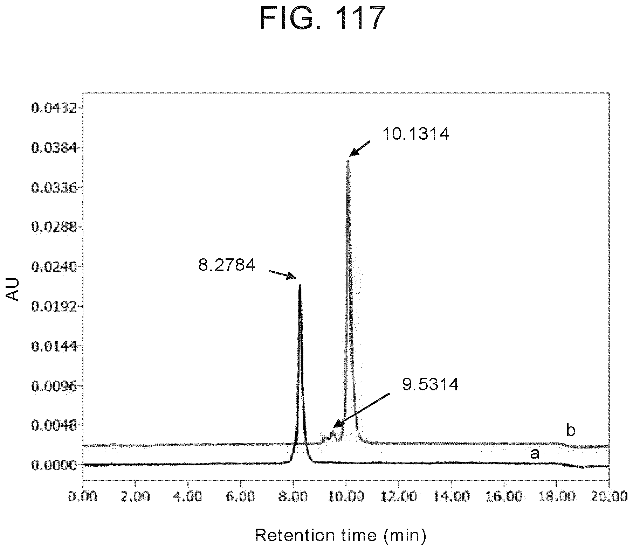

It is believed that a retension time of 10.1314 minutes is attributed to a compound with two peptides introduced to trastuzumab (FIG. 117).

### (87-3) Linker Cleavage of Denosumab-Peptide Conjugate

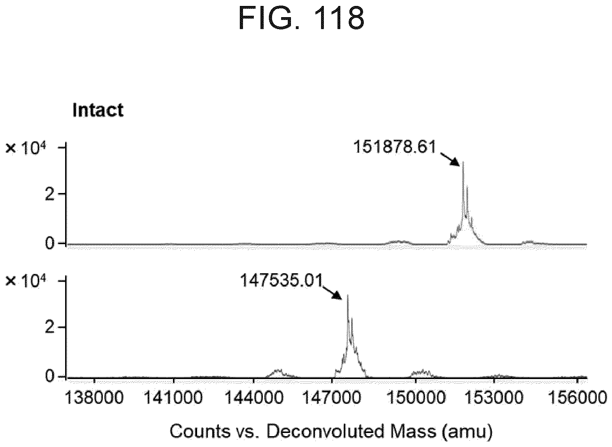

The denosumab-peptide conjugate (20 mM PBS buffer) obtained in (87-2) was substituted with a 0.5 M hydroxylamine 10 mM EDTA (pH 5.5) solution by Amicon 3K, and the solution was allowed to stand at room temperature for two hours. After two hours, the solution was substituted with 20 mM PBS buffer, 10 mM EDTA (pH 7.4) to obtain thiol-introduced denosumab. The mass was measured by ESI-TOFMS; for the raw material denosumab-peptide conjugate, a peak was observed at 151878, whereas for thiol-introduced denosumab as a product, a peak was observed at 147535 (FIG. 118).

### (87-4) HIC-UPLC Analysis of Thiol-introduced Denosumab

The denosumab-peptide conjugate formed in (87-3) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: denosumab-peptide- and linker-coupled compound
b: thiol-introduced denosumab;

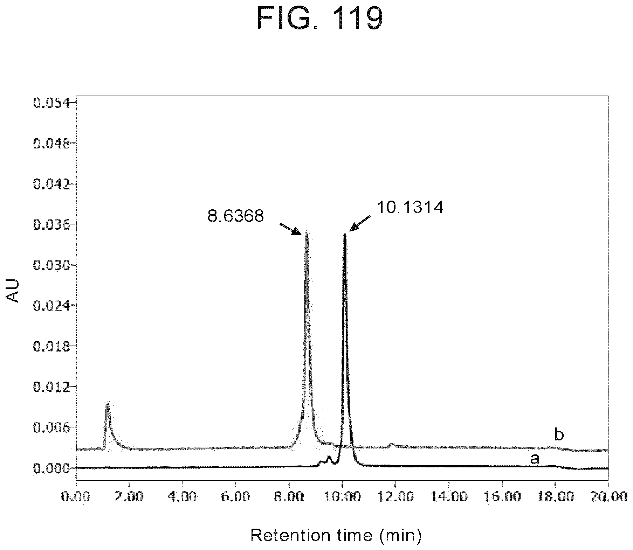

It is believed that a retension time of 8.6368 minutes is attributed to a compound with two thiols introduced to trastuzumab (FIG. 119).

### (87-5) Fluorescent labeling of Thiol-Introduced Denosumab

To the thiol-introduced denosumab (20 mM PBS buffer, 10 mM EDTA) obtained in (87-3), 5 equivalents of fluorescein-5-maleimide (10 mM, dissolved in DMSO) were added. The solution was allowed to stand at room temperature for one hour and then substituted with a 20 mM PBS buffer to obtain fluorescently labeled denosumab (ADC mimic).

### (87-6) Determination of Heavy Chain Selectivity of Fluorescently Labeled Denosumab under Reduction Conditions by ESI-TOFMS Analysis

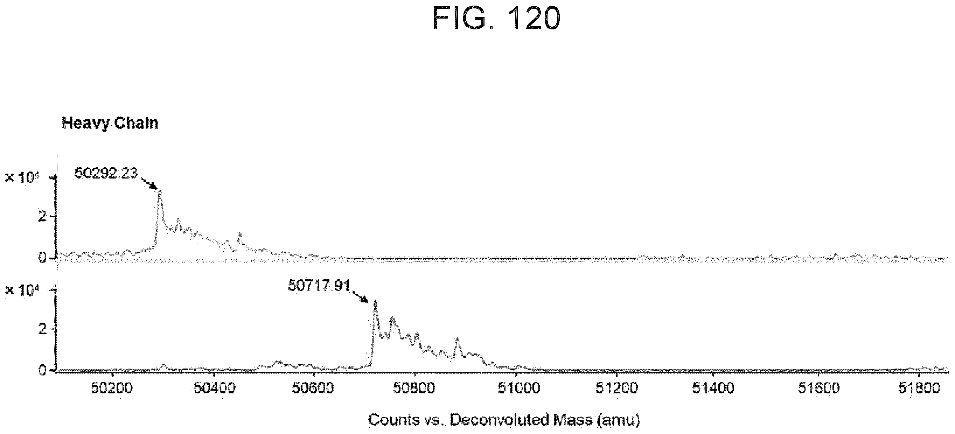

To the fluorescently labeled denosumab (ADC mimic) formed in (87-5), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material and a product, the same light chain peak was observed, whereas for the raw material denosumab, a heavy chain peak was observed at 50292, and for the product, a peak was observed at 50717 with fluorescein introduced to the heavy chain (FIG. 120).

### Example 88: Specific Modification of Anti-IL-4/13 Receptor IgG4 Antibody Dupilumab

### (88-1) Specific Modification of Anti-IL-4/13 Receptor IgG4 Antibody Dupilumab and Analysis by ESI-TOFMS

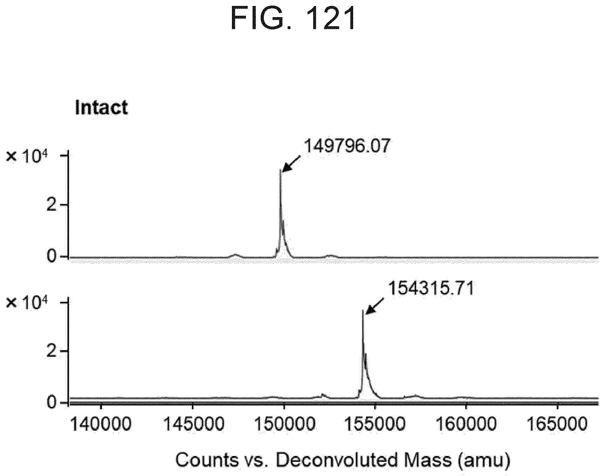

The peptide- and linker-coupled thiophenol-activation compound synthesized in (81-3) was dissolved in N,N'-dimethylformamide to be 21.6 mM. Anti-IL-4/13 receptor IgG4 antibody dupilumab (Sanofi Regeneron) in an amount of 500 µg was dissolved in 200 µL of a 50 mM sodium acetate buffer (pH 5.5), 4.7 µL of a 4 mM peptide reagent (10 equivalents with respect to the antibody) was added thereto, and the solution was stirred at room temperature for one hour. The reaction solution was substituted with a 100 mM sodium citrate buffer (pH 2.9) to stop the reaction and was further substituted with a 20 mM PBS buffer. The mass was measured by ESI-TOFMS; for the raw material dupilumab, a peak was observed at 149796, whereas for a product with two binding peptides introduced, a peak was observed at 154315 (FIG. 121).

### (88-2) HIC-UPLC analysis of Specific Modification of Anti-IL-4/13 Receptor IgG4 Antibody Dupilumab

The dupilumab-peptide conjugate formed in (88-1) and the raw material antibody were analyzed by HIC. For a column, Protein-Pak Hi Res HIC Column (Waters) 4.6 × 100 mm 2.5 µm was used. Detection was performed with A_ Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: dupilumab raw material
b: dupilumab + 10 equivalents of peptide- and linker-coupled thiophenol-activation compound;

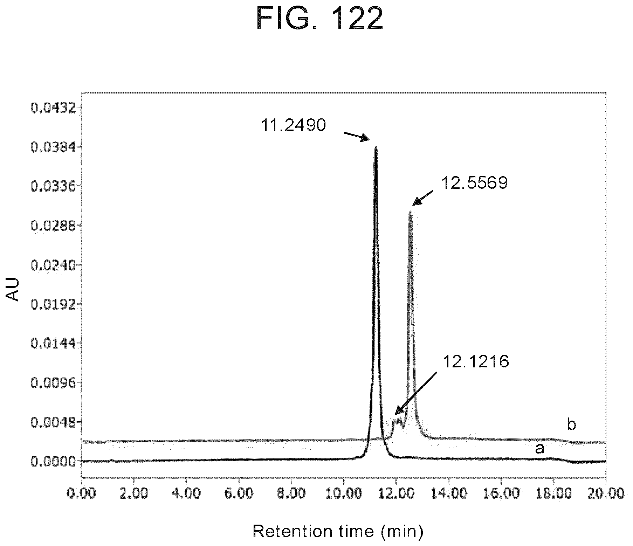

It is believed that a retension time of 12.5569 minutes is attributed to a compound with two peptides introduced to trastuzumab (FIG. 122).

### (88-3) Linker Cleavage of Dupilumab-Peptide Conjugate

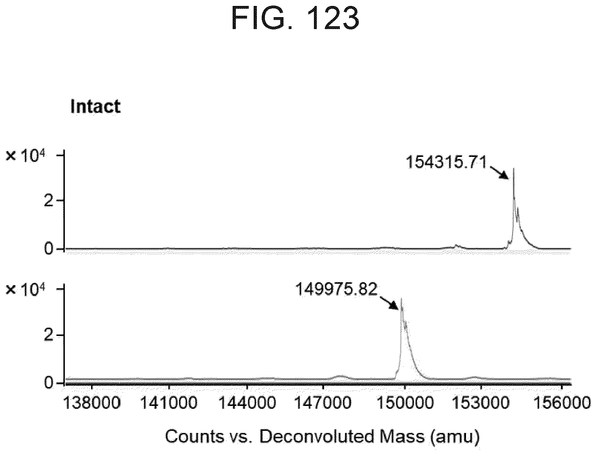

The dupilumab-peptide conjugate (20 mM PBS buffer) obtained in (88-2) was substituted with a 0.5 M hydroxylamine 10 mM EDTA (pH 5.5) solution by Amicon 3K, and the solution was allowed to stand at room temperature for two hours. After two hours, the solution was substituted with 20 mM PBS buffer, 10 mM EDTA (pH 7.4) to obtain thiol-introduced dupilumab. The mass was measured by ESI-TOFMS; for the raw material dupilumab-peptide conjugate, a peak was observed at 154315, whereas for thiol-introduced dupilumab as a product, a peak was observed at 149975 (FIG. 123).

### (88-4) HIC-UPLC Analysis of Thiol-introduced Dupilumab

The dupilumab-peptide conjugate formed in (88-3) and the raw material antibody were analyzed by HIC. For a column, Protein-Pak Hi Res HIC Column (Waters) 4.6 × 100 mm 2.5 µm was used. Detection was performed with A_Buffer: 0.1 M PiNa, 2.3 M (NH₄)₂SO₄, pH 7.0, B_Buffer: 0.1 M PiNa, pH 7.0, a flow rate of 0.6 ml/min, a gradient of A 60% B 40% → A 0% B 100%, 16 minutes (data collection 20 minutes), a column temperature of 40°C, a thermostat temperature of 40°C, and a detector with a wavelength of 280 nm.

Samples were reacted under the following conditions:
a: dupilumab-peptide- and linker-coupled compound
b: thiol-introduced dupilumab;

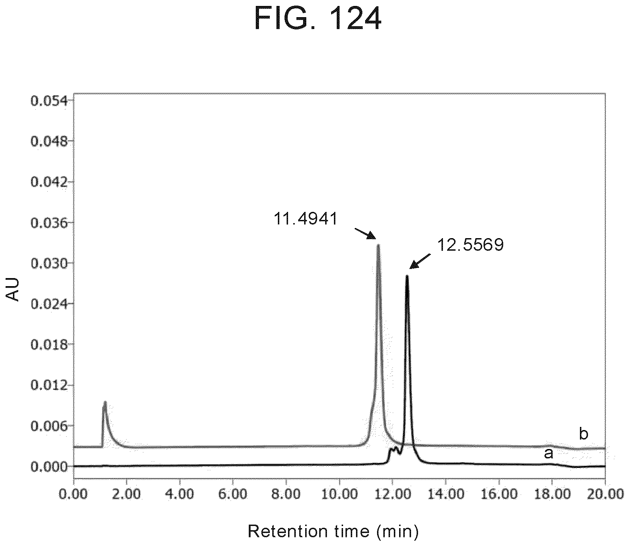

It is believed that a retension time of 11.4941 minutes is attributed to a compound with two thiols introduced to trastuzumab (FIG. 124).

### (88-5) Fluorescent labeling of Thiol-Introduced Dupilumab

To the thiol-introduced dupilumab (20 mM PBS buffer, 10 mM EDTA) obtained in (88-3), 5 equivalents of fluorescein-5-maleimide (10 mM, dissolved in DMSO) were added. The solution was allowed to stand at room temperature for one hour and then substituted with a 20 mM PBS buffer to obtain fluorescently labeled dupilumab (ADC mimic).

### (88-6) Determination of Heavy Chain Selectivity of Fluorescently Labeled Dupilumab under Reduction Conditions by ESI-TOFMS Analysis

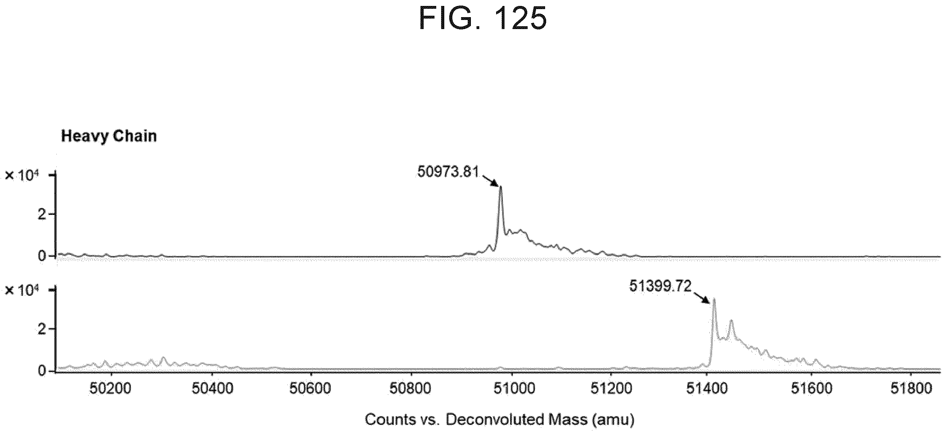

To the fluorescently labeled dupilumab (ADC mimic) formed in (88-5), 5 µL of a 7 mM tris(2-carboxyethyl) phosphine hydrochloride solution (an equivalent with respect to the antibody) was added, and the solution was stirred at room temperature for 20 minutes. The mass was measured by ESI-TOFMS; for the raw material and a product, the same light chain peak was observed, whereas for the raw material thiol-introduced dupilumab, a heavy chain peak was observed at 50973, and for the product, a peak was observed at 51399 with fluorescein introduced to the heavy chain (FIG. 125).

### (Summary)

The foregoing results are summarized as follows.

**Table 2. Summary of affinity peptide (1)**

| Amino Acid Sequence | SEQ ID NO | EXAMPLE |
|---|---|---|
| RGNCAYHKGQIIWCTYH | 5 | 1-3, 80, 81, 84 |
| RGNCAYHKGQIVWCTYH | 8 | 4 |
| RGNCAYHKGQVVWCTYH | 9 | 5, 6 |
| RGNCAYHKGQAVWCTYH | 10 | 7 |
| RGNCAYHKGQLLWCTYH | 11 | 8 |
| RGNCAYHKGQLIWCTYH | 12 | 9 |
| DCAYHKGQIVWCT | 13 | 10 |
| DCAYHKGQVVWCT | 14 | 11 |
| DCAYHKGQAVWCT | 15 | 12 |
| RGNCAYHKSQIIWCTYH | 16 | 13 |
| RGNCAYHKNQIIWCTYH | 17 | 14 |
| RGNCAYHKDQIIWCTYH | 18 | 15 |
| RGNCAYHKQQIIWCTYH | 19 | 16 |
| RGNCAYHKEQIIWCTYH | 20 | 17 |
| RGNCAYHKFQIIWCTYH | 21 | 18 |
| RGNCAYHKYQIIWCTYH | 22 | 19 |
| RGNCAYHKWQIIWCTYH | 23 | 20 |
| RGNCAYHKHQIIWCTYH | 24 | 21 |
| RGNCAYHKTQIIWCTYH | 25 | 22 |
| RGNCAYHKLQIIWCTYH | 26 | 23 |
| CAYHKLQIVWC | 27 | 24 |
| CAYHKLQLIWC | 28 | 25 |
| CAYHKSQIVWC | 29 | 26 |
| RGNCAYHKGQLVFCTYH | 30 | 27 |
| RGNCAYHKGQQVWCTYH | 31 | 28 |
| RGNCAYHKGQEVWCTYH | 32 | 29 |

**Table 3. Summary of affinity peptide (2)**

| Amino Acid Sequence | SEQ ID NO | EXAMPLE |
|---|---|---|
| CAYHKGQLVWC | 33 | 30 |
| RGNCAYHKAQLVWCTYH | 34 | 31 |
| RGNCAYHKVQLVWCTYH | 35 | 32 |
| RGNCAYHKLQLVWCTYH | 36 | 33 |
| RGNCAYHKIQLVWCTYH | 37 | 34 |
| RGNCAYHKSQLVWCTYH | 38 | 35 |
| RGNCAYHKTQLVWCTYH | 39 | 36 |
| RGNCAYHKNQLVWCTYH | 40 | 37 |
| RGNCAYHKDQLVWCTYH | 41 | 38 |
| RGNCAYHKQQLVWCTYH | 42 | 39 |
| RGNCAYHKEQLVWCTYH | 43 | 40 |
| RGNCAYHKFQLVWCTYH | 44 | 41 |
| RGNCAYHKRQLVWCTYH | 45 | 42 |
| RGNCAYHKHQLVWCTYH | 46 | 43 |
| RGNCAYHKWQLVWCTYH | 47 | 44 |
| RGNCAYHKYQLVWCTYH | 48 | 45 |
| RGNCAYFKGQLVWCTYH | 49 | 46 |
| RGNCAYYKGQLVWCTYH | 50 | 47 |
| RGNCAYWKGQLVWCTYH | 51 | 48 |
| RGNCAYRKGQLVWCTYH | 52 | 49 |
| RGNCAYGKGQLVWCTYH | 53 | 50 |
| DCAYHKGQLVWC | 54 | 51 |
| NCAYHKGQLVWC | 55 | 52 |
| CAYHKGQLVWCT | 56 | 53 |
| CAYHKSQLVWC | 57 | 54 |

**Table 4. Summary of affinity peptide (3)**

| Amino Acid Sequence | SEQ ID NO | EXAMPLE |
|---|---|---|
| RGNCAWHKGQIIWCTYH | 68 | 55 |
| RGNCAFHKGQIIWCTYH | 69 | 56 |
| RGNCAHHKGQIIWCTYH | 70 | 57 |
| RGNCGYHKGQIIWCTYH | 71 | 58 |
| RGNCLYHKGQIIWCTYH | 72 | 59 |
| RGNCPYHKGQIIWCTYH | 73 | 60 |
| RGNCRYHKGQIIWCTYH | 74 | 61 |
| RGNCVYHKGQIIWCTYH | 75 | 62 |
| RGNCNYHKGQIIWCTYH | 76 | 63 |
| RGNCEYHKGQIIWCTYH | 77 | 64 |
| RGNCFYHKGQIIWCTYH | 78 | 65 |
| RGNCAYHKGEIIWCTYH | 79 | 66 |
| RGNCAYHKGNIIWCTYH | 80 | 67 |
| RGNCAYHKGPIIWCTYH | 81 | 68 |
| RGNCAYHKGGIIWCTYH | 82 | 69 |
| RGNCAYHKGDIIWCTYH | 83 | 70 |
| RGNCAYHKGRIIWCTYH | 84 | 71 |
| RGNCAYHKGFIIWCTYH | 85 | 72 |
| RGNCAYHKGHIIWCTYH | 86 | 73 |
| DCAYHKGQIIWCT | 87 | 74 |
| NCAYHKGQIIWCT | 88 | 75 |
| GNCAYHKGQIIWCTY | 89 | 76 |
| GCAYHKGQIIWCG | 90 | 77 |
| GGCAYHKGQIIWCGG | 91 | 78 |
| GGGCAYHKGQIIWCGGG | 92 | 79 |

For the antibody, it has been indicated that a specific amino acid residue including the lysine residue at position 246 and/or 248 (e.g., Examples 2, 6, 81, 84, and 85) can be modified regioselectively.

Furthermore, the following compound having an affinity substance to an antibody, a cleavable portion, and a reactive group, represented by the following Formula (I), is described with reference to part of Examples as Table 5 below:

A-L-B-R (I)

wherein
A is the affinity substance to an antibody,
L is a cleavable linker which is a divalent group comprising the cleavable portion,
B is (a) a divalent group comprising a bioorthogonal functional group or (b) a divalent group comprising no bioorthogonal functional group, and
R is the reactive group to the antibody.
L is (i) a cleavable linker which is a divalent group comprising a cleavable portion having an ability to produce a bioorthogonal functional group on a reactive group side by cleavage or (ii) a cleavable linker which is a divalent group comprising a cleavable portion having no ability to produce a bioorthogonal functional group on a reactive group side by cleavage.

**Table 5. Relationship between compound having affinity substance to antibody, cleavable portion, and reactive group, and antibody having bioorthogonal functional groups which can be produced by using the compound**

| | | | |
|---|---|---|---|
| Example | Specific structure of compound | | Antibody having bioorthogonal functional groups which can be produced by using the compound* |
| | □ | : Affinity substance (A) | |
| | 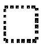 | : Cleavable portion (in L) | |
| | ○ | : Bioorthogonal functional group B(a) or L(ii) | |
| | 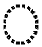 | : Reactive group (R) | |
| 1 | 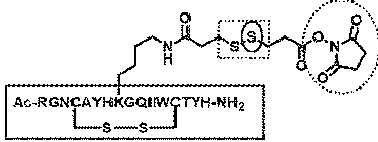 | | 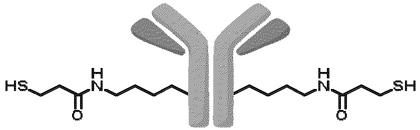 |
| | The above-amino acid sequence: SEQ ID NO:5 | | |
| 80 | 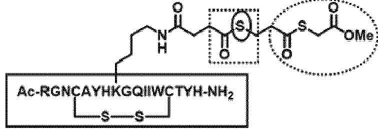 | | 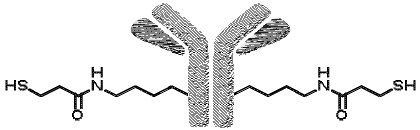 |
| | The SEQ | above-amino acid sequence: ID NO:5 | |
| 81 | 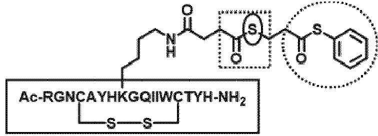 | | 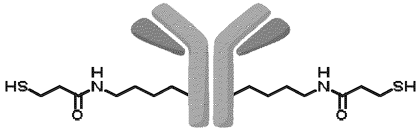 |
| | The above-amino acid sequence: SEQ ID NO:5 | | |
| 84 | 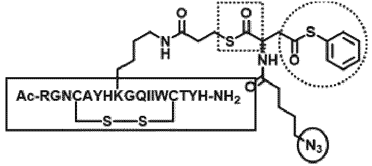 | | |
| | The above-amino acid sequence: SEQ ID NO:5 | | |

| | | | |
|---|---|---|---|
| * An NH-C4 alkyl portion extending from an antibody is derived from side chain of lysine residue in the antibody (hereafter the same applies). | | | |

Various compounds each having an affinity substance to an antibody, a cleavable portion, and a reactive group can be prepared according to well-known methods of organic synthesis by changing the types of (A) the affinity substance to an antibody, (L) the cleavable linker which is a divalent group comprising a cleavable portion, (B) (a) the divalent group comprising a bioorthogonal functional group or (b) the divalent group comprising no bioorthogonal functional group, and (R) the reactive group to the antibody as appropriate as described above. A relation between additional examples of the compound having an affinity substance to an antibody, a cleavable portion, and a reactive group that can be prepared in the present invention and the antibody having a bioorthogonal functional group or bioorthogonal functional groups that can be produced by using the same is as follows.

**Table 6. Relationship between additional examples of compound having affinity substance to antibody, cleavable portion, and reactive group, and antibody having bioorthogonal functional groups which can be produced by using the compound (1)**

| | | | |
|---|---|---|---|
| Example | Specific structure of compound | | Antibody having bioorthogonal functional groups which can be produced by using the compound* |
| | □ | : Affinity substance (A) | |
| | 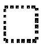 | : Cleavable portion (in L) | |
| | ○ | : Bioorthogonal functional group B(a) or L(ii) | |
| | 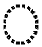 | : Reactive group (R) | |
| 89 | 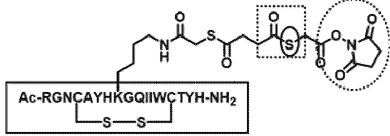 | | 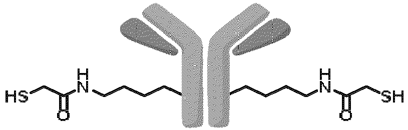 |
| | The above-amino acid sequence: SEQ ID NO:5 | | |
| 90 | 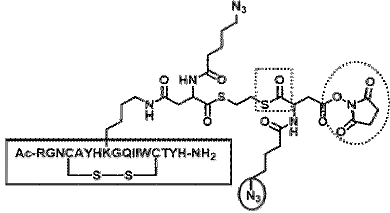 | | 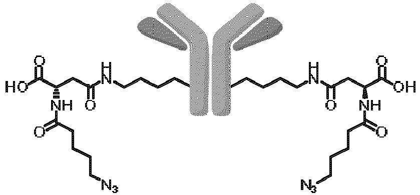 |
| | The above-amino acid sequence: SEQ ID NO:5 | | |
| 91 | 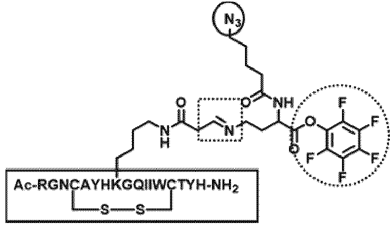 | | 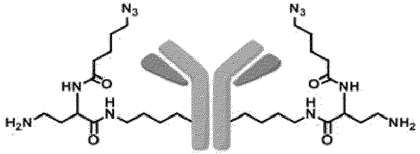 |
| | The above-amino acid sequence: SEQ ID NO:5 | | |
| 92 | 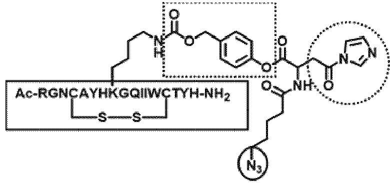 | | 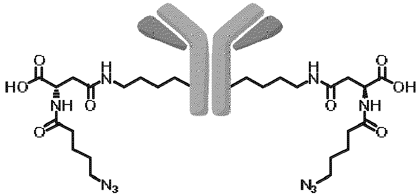 |
| | The above-amino acid sequence: SEQ ID NO:5 | | |

**Table 7. Relationship between additional examples of compound having affinity substance to antibody, cleavable portion, and reactive group, and antibody having bioorthogonal functional groups which can be produced by using the compound (2)**

| | | | |
|---|---|---|---|
| Example | Specific structure of compound | | Antibody having bioorthogonal functional groups which can be produced by using the compound* |
| | □ | : Affinity substance (A) | |
| | 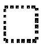 | : Cleavable portion (in L) | |
| | ○ | : Bioorthogonal functional group B(a) or L(ii) | |
| | 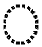 | : Reactive group (R) | |
| 93 | 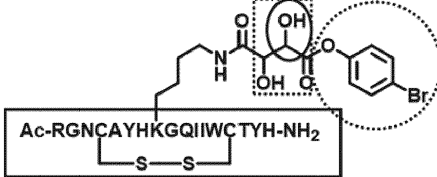 | | 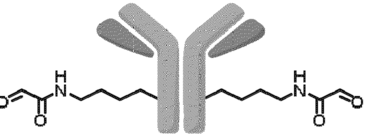 |
| | The above-amino acid sequence: SEQ ID NO:5 | | |
| 94 | 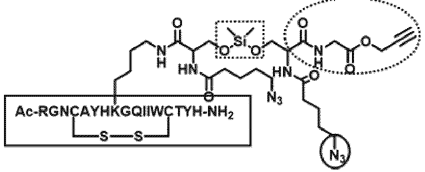 | | 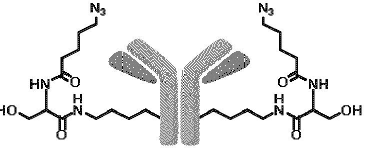 |
| | The above-amino acid sequence: SEQ ID NO:5 | | |
| 95 | 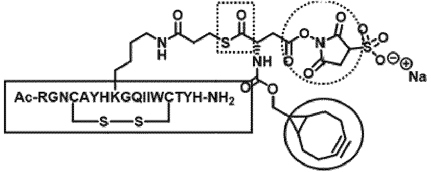 | | 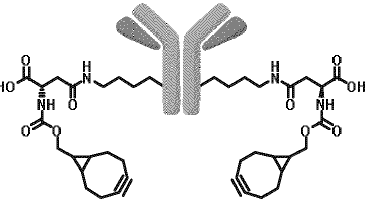 |
| | The above-amino acid sequence: SEQ ID NO:5 | | |
| 96 | 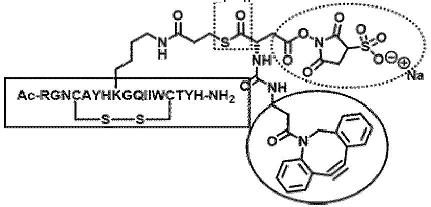 | | 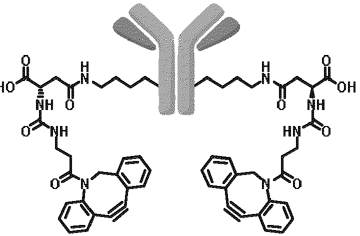 |
| | The above-amino acid sequence: SEQ ID NO:5 | | |

**Table 8. Relationship between additional examples of compound having affinity substance to antibody, cleavable portion, and reactive group, and antibody having bioorthogonal functional groups which can be produced by using the compound (3)**

| | | | |
|---|---|---|---|
| Example | Specific structure of compound | | Antibody having bioorthogonal functional groups which can be produced by using the compound* |
| | □ | : Affinity substance (A) | |
| | 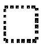 | : Cleavable portion (in L) | |
| | ○ | : Bioorthogonal functional group B(a) or L(ii) | |
| | 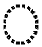 | : Reactive group (R) | |
| 97 | 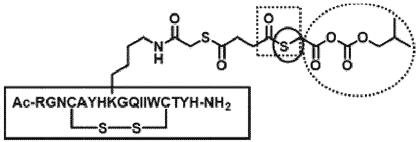 | | 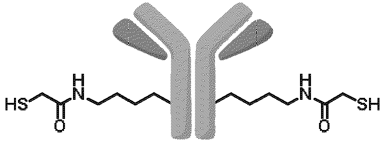 |
| | The above-amino acid sequence: SEQ ID NO:5 | | |
| 98 | 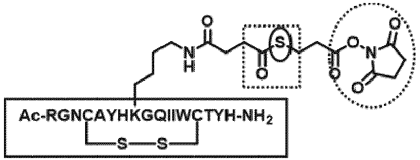 | | 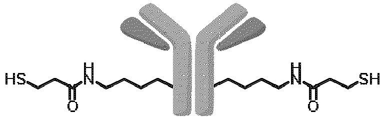 |
| | The above-amino acid sequence: SEQ ID NO:5 | | |
| 99 | 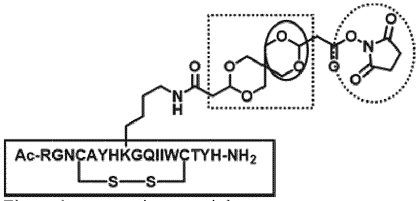 | | 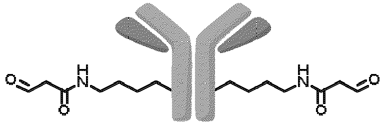 |
| | The above-amino acid sequence: SEQ ID NO:5 | | |
| 100 | 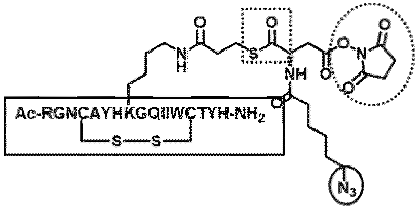 | | 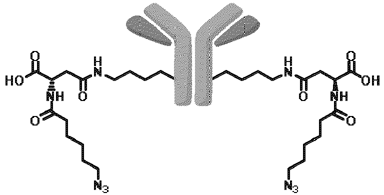 |
| | The above-amino acid sequence: SEQ ID NO:5 | | |

**Table 9. Relationship between additional examples of compound having affinity substance to antibody, cleavable portion, and reactive group, and antibody having bioorthogonal functional groups which can be produced by using the compound (4)**

| | | | |
|---|---|---|---|
| Example | Specific structure of compound | | Antibody having bioorthogonal functional groups which can be produced by using the compound* |
| | □ | : Affinity substance (A) | |
| | 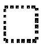 | : Cleavable portion (in L) | |
| | ○ | : Bioorthogonal functional group B(a) or L(ii) | |
| | 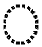 | : Reactive group (R) | |
| 101 | 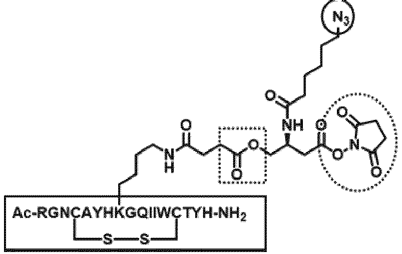 | | 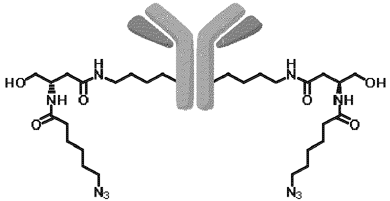 |
| | The above-amino acid sequence: SEQ ID NO:5 | | |
| 102 | 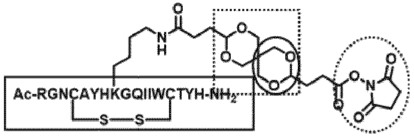 | | 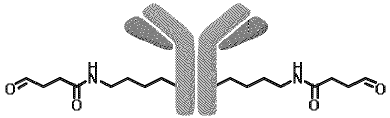 |
| | The above-amino acid sequence: SEQ ID NO:5 | | |
| 103 | 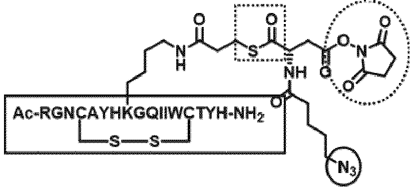 | | 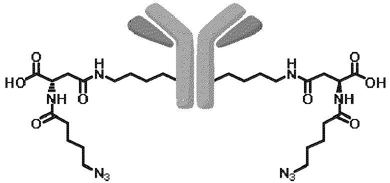 |
| | The above-amino acid sequence: SEQ ID NO:5 | | |

**Table 10. Relationship between additional examples of compound having affinity substance to antibody, cleavable portion, and reactive group, and antibody having bioorthogonal functional groups which can be produced by using the compound (5)**

| | | | |
|---|---|---|---|
| Example | Specific structure of compound | | Antibody having bioorthogonal functional groups which can be produced by using the compound* |
| | □ | : Affinity substance (A) | |
| | 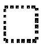 | : Cleavable portion (in L) | |
| | ○ | : Bioorthogonal functional group B(a) or L(ii) | |
| | 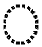 | : Reactive group (R) | |
| 104 | 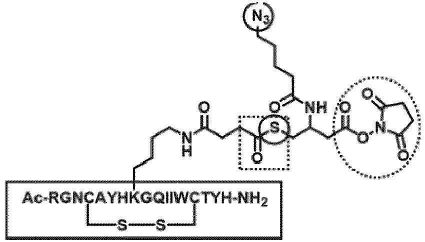 | | |
| | The above-amino acid sequence: SEQ ID NO:5 | | |
| 105 | 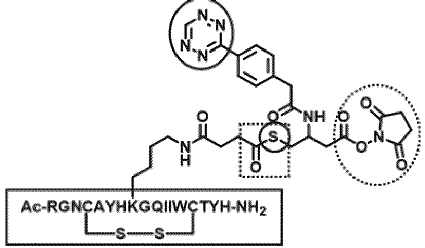 | | |
| | The above-amino acid sequence: SEQ ID NO:5 | | |
| 106 | 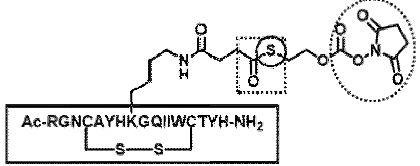 | | 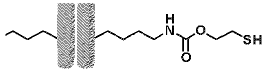 |
| | The above-amino acid sequence: SEQ ID NO:5 | | |
| 107 | 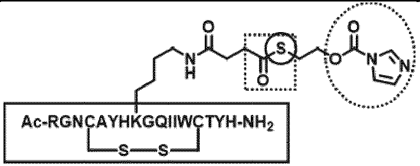 | | |
| | The above-amino acid sequence: SEQ ID NO:5 | | |

It has been demonstrated from the foregoing that in a compound having an affinity substance to an antibody, a cleavable portion, and a reactive group, the antibody can be modified regioselectively by adjusting factors such as the type of the affinity substance, the length of a linker between the affinity substance and the reactive group, and the position in the affinity substance to which the linker is introduced as appropriate.

### Industrial Applicability

For example, the present invention is useful for production of a regioselectively modified antibody.

### [Sequence Listing]

SEQUENCE LISTING

<110>  Ajinomoto Co., Inc.

<120>  Compounds and salts thereof comprising an affinity substance to
       an antibody, a clevable moiety, and a reactive group

<130>  PAMA-31637

<150>  JP2018-113953
<151>  2018-06-14

<150>  JP2018-205446
<151>  2018-10-31

<160>  94

<170>  PatentIn version 3.5

<210>  1
<211>  230
<212>  PRT
<213>  Artificial Sequence

<220>
<223>  Fc region of human IgG1


<220>
<221>  misc_feature
<222>  (18)..(19)
<223>  Xaa can be any naturally occurring amino acid

<220>
<221>  misc_feature
<222>  (21)..(21)
<223>  Xaa can be any naturally occurring amino acid

<220>
<221>  misc_feature
<222>  (140)..(140)
<223>  Xaa can be any naturally occurring amino acid

<220>
<221>  misc_feature
<222>  (142)..(142)
<223>  Xaa can be any naturally occurring amino acid

<220>
<221>  misc_feature
<222>  (177)..(177)
<223>  Xaa can be any naturally occurring amino acid

<400>  1

Pro Lys Ser Cys Asp Lys Thr His Thr Cys Pro Pro Cys Pro Ala Pro
1               5                   10                  15


Glu Xaa Xaa Gly Xaa Pro Ser Val Phe Leu Phe Pro Pro Lys Pro Lys
            20                  25                  30

Asp Thr Leu Met Ile Ser Arg Thr Pro Glu Val Thr Cys Val Val Val
        35                  40                  45

Asp Val Ser His Glu Asp Pro Glu Val Lys Phe Asn Trp Tyr Val Asp
    50                  55                  60

Gly Val Glu Val His Asn Ala Lys Thr Lys Pro Arg Glu Glu Gln Tyr
65                  70                  75                  80

Asp Ser Thr Tyr Arg Val Val Ser Val Leu Thr Val Leu His Gln Asp
                85                  90                  95

Trp Leu Asn Gly Lys Glu Tyr Lys Cys Lys Val Ser Asn Lys Ala Leu
            100                 105                 110

Pro Ala Pro Ile Glu Lys Thr Ile Ser Lys Ala Lys Gly Gln Pro Arg
        115                 120                 125

Glu Pro Gln Val Tyr Thr Leu Pro Pro Ser Arg Xaa Glu Xaa Thr Lys
    130                 135                 140

Asn Gln Val Ser Leu Thr Cys Leu Val Lys Gly Phe Tyr Pro Ser Asp
145                 150                 155                 160

Ile Ala Val Glu Trp Glu Ser Asn Gly Gln Pro Glu Asn Asn Tyr Lys
                165                 170                 175

Xaa Thr Pro Pro Val Leu Asp Ser Asp Gly Ser Phe Phe Leu Tyr Ser
            180                 185                 190

Lys Leu Thr Val Asp Lys Ser Arg Trp Gln Gln Gly Asn Val Phe Ser
        195                 200                 205

Cys Ser Val Met His Glu Ala Leu His Asn His Tyr Thr Gln Lys Ser
    210                 215                 220

Leu Ser Leu Ser Pro Gly
225                 230

<210>  2
<211>  449
<212>  PRT
<213>  Artificial Sequence

<220>
<223>  Heavy chain of Trastuzumab (humanized IgG1 monoclonal antibody)

<400>  2

Glu Val Gln Leu Val Glu Ser Gly Gly Gly Leu Val Gln Pro Gly Gly

1               5                   10                  15

Ser Leu Arg Leu Ser Cys Ala Ala Ser Gly Phe Asn Ile Lys Asp Thr
            20                  25                  30

Tyr Ile His Trp Val Arg Gln Ala Pro Gly Lys Gly Leu Glu Trp Val
        35                  40                  45

Ala Arg Ile Tyr Pro Thr Asn Gly Tyr Thr Arg Tyr Ala Asp Ser Val
    50                  55                  60

Lys Gly Arg Phe Thr Ile Ser Ala Asp Thr Ser Lys Asn Thr Ala Tyr
65                  70                  75                  80

Leu Gln Met Asn Ser Leu Arg Ala Glu Asp Thr Ala Val Tyr Tyr Cys
                85                  90                  95

Ser Arg Trp Gly Gly Asp Gly Phe Tyr Ala Met Asp Tyr Trp Gly Gln
            100                 105                 110

Gly Thr Leu Val Thr Val Ser Ser Ala Ser Thr Lys Gly Pro Ser Val
        115                 120                 125

Phe Pro Leu Ala Pro Ser Ser Lys Ser Thr Ser Gly Gly Thr Ala Ala
    130                 135                 140

Leu Gly Cys Leu Val Lys Asp Tyr Phe Pro Glu Pro Val Thr Val Ser
145                 150                 155                 160

Trp Asn Ser Gly Ala Leu Thr Ser Gly Val His Thr Phe Pro Ala Val
                165                 170                 175

Leu Gln Ser Ser Gly Leu Tyr Ser Leu Ser Ser Val Val Thr Val Pro
            180                 185                 190

Ser Ser Ser Leu Gly Thr Gln Thr Tyr Ile Cys Asn Val Asn His Lys
        195                 200                 205

Pro Ser Asn Thr Lys Val Asp Lys Lys Val Glu Pro Lys Ser Cys Asp
    210                 215                 220

Lys Thr His Thr Cys Pro Pro Cys Pro Ala Pro Glu Leu Leu Gly Gly
225                 230                 235                 240

Pro Ser Val Phe Leu Phe Pro Pro Lys Pro Lys Asp Thr Leu Met Ile
                245                 250                 255

Ser Arg Thr Pro Glu Val Thr Cys Val Val Val Asp Val Ser His Glu
            260                 265                 270

Asp Pro Glu Val Lys Phe Asn Trp Tyr Val Asp Gly Val Glu Val His
        275                 280                 285

Asn Ala Lys Thr Lys Pro Arg Glu Glu Gln Tyr Asp Ser Thr Tyr Arg
    290                 295                 300

Val Val Ser Val Leu Thr Val Leu His Gln Asp Trp Leu Asn Gly Lys
305                 310                 315                 320

Glu Tyr Lys Cys Lys Val Ser Asn Lys Ala Leu Pro Ala Pro Ile Glu
                325                 330                 335

Lys Thr Ile Ser Lys Ala Lys Gly Gln Pro Arg Glu Pro Gln Val Tyr
            340                 345                 350

Thr Leu Pro Pro Ser Arg Glu Glu Met Thr Lys Asn Gln Val Ser Leu
        355                 360                 365

Thr Cys Leu Val Lys Gly Phe Tyr Pro Ser Asp Ile Ala Val Glu Trp
    370                 375                 380

Glu Ser Asn Gly Gln Pro Glu Asn Asn Tyr Lys Thr Thr Pro Pro Val
385                 390                 395                 400

Leu Asp Ser Asp Gly Ser Phe Phe Leu Tyr Ser Lys Leu Thr Val Asp
                405                 410                 415

Lys Ser Arg Trp Gln Gln Gly Asn Val Phe Ser Cys Ser Val Met His
            420                 425                 430

Glu Ala Leu His Asn His Tyr Thr Gln Lys Ser Leu Ser Leu Ser Pro
        435                 440                 445

Gly

<210>  3
<211>  237
<212>  PRT
<213>  Artificial Sequence

<220>
<223>  Fc region of human IgG1 antibody

<400>  3

Ile Glu Gly Arg Met Asp Pro Lys Ser Cys Asp Lys Thr His Thr Cys

1                   5                   10                  15

Pro Pro Cys Pro Ala Pro Glu Ala Glu Gly Ala Pro Ser Val Phe Leu
            20                  25                  30

Phe Pro Pro Lys Pro Lys Asp Thr Leu Met Ile Ser Arg Thr Pro Glu
        35                  40                  45

Val Thr Cys Val Val Val Asp Val Ser His Glu Asp Pro Glu Val Lys
    50                  55                  60

Phe Asn Trp Tyr Val Asp Gly Val Glu Val His Asn Ala Lys Thr Lys
65                  70                  75                  80

Pro Arg Glu Glu Gln Tyr Asp Ser Thr Tyr Arg Val Val Ser Val Leu
                85                  90                  95

Thr Val Leu His Gln Asp Trp Leu Asn Gly Lys Glu Tyr Lys Cys Lys
            100                 105                 110

Val Ser Asn Lys Ala Leu Pro Ala Pro Ile Glu Lys Thr Ile Ser Lys
        115                 120                 125

Ala Lys Gly Gln Pro Arg Glu Pro Gln Val Tyr Thr Leu Pro Pro Ser
    130                 135                 140

Arg Asp Glu Leu Thr Lys Asn Gln Val Ser Leu Thr Cys Leu Val Lys
145                 150                 155                 160

Gly Phe Tyr Pro Ser Asp Ile Ala Val Glu Trp Glu Ser Asn Gly Gln
                165                 170                 175

Pro Glu Asn Asn Tyr Lys Ala Thr Pro Pro Val Leu Asp Ser Asp Gly
            180                 185                 190

Ser Phe Phe Leu Tyr Ser Lys Leu Thr Val Asp Lys Ser Arg Trp Gln
        195                 200                 205

Gln Gly Asn Val Phe Ser Cys Ser Val Met His Glu Ala Leu His Asn
    210                 215                 220

His Tyr Thr Gln Lys Ser Leu Ser Leu Ser Pro Gly Lys
225                 230                 235

<210>  4
<211>  214
<212>  PRT
<213>  Artificial Sequence

<220>
<223>  Light chain of Trastuzumab (humanized IgG1 monoclonal antibody)

<400>  4

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Val Asn Thr Ala
            20                  25                  30

Val Ala Trp Tyr Gln Gln Lys Pro Gly Lys Ala Pro Lys Leu Leu Ile
        35                  40                  45

Tyr Ser Ala Ser Phe Leu Tyr Ser Gly Val Pro Ser Arg Phe Ser Gly
    50                  55                  60

Ser Arg Ser Gly Thr Asp Phe Thr Leu Thr Ile Ser Ser Leu Gln Pro
65                  70                  75                  80

Glu Asp Phe Ala Thr Tyr Tyr Cys Gln Gln His Tyr Thr Thr Pro Pro
                85                  90                  95

Thr Phe Gly Gln Gly Thr Lys Val Glu Ile Lys Arg Thr Val Ala Ala
            100                 105                 110

Pro Ser Val Phe Ile Phe Pro Pro Ser Asp Glu Gln Leu Lys Ser Gly
        115                 120                 125

Thr Ala Ser Val Val Cys Leu Leu Asn Asn Phe Tyr Pro Arg Glu Ala
    130                 135                 140

Lys Val Gln Trp Lys Val Asp Asn Ala Leu Gln Ser Gly Asn Ser Gln
145                 150                 155                 160

Glu Ser Val Thr Glu Gln Asp Ser Lys Asp Ser Thr Tyr Ser Leu Ser
                165                 170                 175

Ser Thr Leu Thr Leu Ser Lys Ala Asp Tyr Glu Lys His Lys Val Tyr
            180                 185                 190

Ala Cys Glu Val Thr His Gln Gly Leu Ser Ser Pro Val Thr Lys Ser
        195                 200                 205

Phe Asn Arg Gly Glu Cys
    210

<210>  5

<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  5

Arg Gly Asn Cys Ala Tyr His Lys Gly Gln Ile Ile Trp Cys Thr Tyr
1               5                   10                  15

His

<210>  6
<211>  33
<212>  PRT
<213>  Artificial Sequence

<220>
<223>  Peptide fragment of Trastuzumab

<400>  6

Thr His Thr Cys Pro Pro Cys Pro Ala Pro Glu Leu Leu Gly Gly Pro
1               5                   10                  15

Ser Val Phe Leu Phe Pro Pro Lys Pro Lys Asp Thr Leu Met Ile Ser
            20                  25                  30

Arg

<210>  7
<211>  16
<212>  PRT
<213>  Artificial Sequence

<220>
<223>  Peptide fragment of Trastuzumab

<400>  7

Leu Leu Gly Gly Pro Ser Val Phe Leu Phe Pro Pro Lys Pro Lys Asp
1               5                   10                  15

<210>  8
<211>  17
<212>  PRT
<213>  Artificial Sequence

<220>
<223>  Peptide having an affinity to Fc region of human IgG.  The amino

acid residue at N-terminus may be acetylated, the amino acid
      residue at C-terminus may be aminated and two cysteine residues
      may be linked by disulfide bridge.

<400>  8

Arg Gly Asn Cys Ala Tyr His Lys Gly Gln Ile Val Trp Cys Thr Tyr
1               5                   10                  15

His

<210>  9
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  9

Arg Gly Asn Cys Ala Tyr His Lys Gly Gln Val Val Trp Cys Thr Tyr
1               5                   10                  15

His

<210>  10
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  10

Arg Gly Asn Cys Ala Tyr His Lys Gly Gln Ala Val Trp Cys Thr Tyr
1               5                   10                  15

His

<210>  11
<211>  17
<212>  PRT
<213>  Artificial Sequence

<220>
<223>  Peptide having an affinity to Fc region of human IgG.  The amino

acid residue at N-terminus may be acetylated, the amino acid
      residue at C-terminus may be aminated and two cysteine residues
      may be linked by disulfide bridge.

<400>  11

Arg Gly Asn Cys Ala Tyr His Lys Gly Gln Leu Leu Trp Cys Thr Tyr
1               5                   10                  15

His

<210>  12
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  12

Arg Gly Asn Cys Ala Tyr His Lys Gly Gln Leu Ile Trp Cys Thr Tyr
1               5                   10                  15

His

<210>  13
<211>  13
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  13

Asp Cys Ala Tyr His Lys Gly Gln Ile Val Trp Cys Thr
1               5                   10

<210>  14
<211>  13
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  14

Asp Cys Ala Tyr His Lys Gly Gln Val Val Trp Cys Thr
1               5                   10

<210>  15
<211>  13
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  15

Asp Cys Ala Tyr His Lys Gly Gln Ala Val Trp Cys Thr
1               5                   10

<210>  16
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  16

Arg Gly Asn Cys Ala Tyr His Lys Ser Gln Ile Ile Trp Cys Thr Tyr
1               5                   10                  15

His

<210>  17
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  17

Arg Gly Asn Cys Ala Tyr His Lys Asn Gln Ile Ile Trp Cys Thr Tyr
1               5                   10                  15

His

<210>  18
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  18

Arg Gly Asn Cys Ala Tyr His Lys Asp Gln Ile Ile Trp Cys Thr Tyr
1               5                   10                  15

His

<210>  19
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  19

Arg Gly Asn Cys Ala Tyr His Lys Gln Gln Ile Ile Trp Cys Thr Tyr
1               5                   10                  15

His

<210>  20
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  20

Arg Gly Asn Cys Ala Tyr His Lys Glu Gln Ile Ile Trp Cys Thr Tyr
1               5                   10                  15

His

<210>  21
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  21

Arg Gly Asn Cys Ala Tyr His Lys Phe Gln Ile Ile Trp Cys Thr Tyr
1               5                   10                  15

His

<210>  22
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  22

Arg Gly Asn Cys Ala Tyr His Lys Tyr Gln Ile Ile Trp Cys Thr Tyr
1               5                   10                  15

His

<210>  23
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  23

Arg Gly Asn Cys Ala Tyr His Lys Trp Gln Ile Ile Trp Cys Thr Tyr
1               5                   10                  15

His

<210>  24
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  24

Arg Gly Asn Cys Ala Tyr His Lys His Gln Ile Ile Trp Cys Thr Tyr
1               5                   10                  15

His

<210>  25
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  25

Arg Gly Asn Cys Ala Tyr His Lys Thr Gln Ile Ile Trp Cys Thr Tyr
1               5                   10                  15

His

<210>  26
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  26

Arg Gly Asn Cys Ala Tyr His Lys Leu Gln Ile Ile Trp Cys Thr Tyr
1               5                   10                  15

His

<210>  27
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  27

Cys Ala Tyr His Lys Leu Gln Ile Val Trp Cys
1               5                   10


<210>  28
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  28

Cys Ala Tyr His Lys Leu Gln Leu Ile Trp Cys
1               5                   10


<210>  29
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  29

Cys Ala Tyr His Lys Ser Gln Ile Val Trp Cys
1               5                   10


<210>  30
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  30

Arg Gly Asn Cys Ala Tyr His Lys Gly Gln Leu Val Phe Cys Thr Tyr
1               5                   10                  15

His

<210>  31
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  31

Arg Gly Asn Cys Ala Tyr His Lys Gly Gln Gln Val Trp Cys Thr Tyr
1               5                   10                  15

His

<210>  32
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  32

Arg Gly Asn Cys Ala Tyr His Lys Gly Gln Glu Val Trp Cys Thr Tyr
1               5                   10                  15

His

<210>  33
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  33

Cys Ala Tyr His Lys Gly Gln Leu Val Trp Cys
1               5                   10

<210>  34
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  34

Arg Gly Asn Cys Ala Tyr His Lys Ala Gln Leu Val Trp Cys Thr Tyr
1               5                   10                  15

His

<210>  35
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  35

Arg Gly Asn Cys Ala Tyr His Lys Val Gln Leu Val Trp Cys Thr Tyr
1               5                   10                  15

His

<210>  36
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  36

Arg Gly Asn Cys Ala Tyr His Lys Leu Gln Leu Val Trp Cys Thr Tyr

1               5                   10                  15

His

<210>  37
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  37

Arg Gly Asn Cys Ala Tyr His Lys Ile Gln Leu Val Trp Cys Thr Tyr
1               5                   10                  15

His

<210>  38
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  38

Arg Gly Asn Cys Ala Tyr His Lys Ser Gln Leu Val Trp Cys Thr Tyr
1               5                   10                  15

His

<210>  39
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  39

Arg Gly Asn Cys Ala Tyr His Lys Thr Gln Leu Val Trp Cys Thr Tyr

1               5                   10                  15

His

<210>  40
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  40

Arg Gly Asn Cys Ala Tyr His Lys Asn Gln Leu Val Trp Cys Thr Tyr
1               5                   10                  15

His

<210>  41
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  41

Arg Gly Asn Cys Ala Tyr His Lys Asp Gln Leu Val Trp Cys Thr Tyr
1               5                   10                  15

His

<210>  42
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  42

Arg Gly Asn Cys Ala Tyr His Lys Gln Gln Leu Val Trp Cys Thr Tyr

1               5                   10                  15

His

<210>  43
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  43

Arg Gly Asn Cys Ala Tyr His Lys Glu Gln Leu Val Trp Cys Thr Tyr
1               5                   10                  15

His

<210>  44
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  44

Arg Gly Asn Cys Ala Tyr His Lys Phe Gln Leu Val Trp Cys Thr Tyr
1               5                   10                  15

His

<210>  45
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  45

Arg Gly Asn Cys Ala Tyr His Lys Arg Gln Leu Val Trp Cys Thr Tyr

1               5                   10                  15


His




<210>  46
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  46

Arg Gly Asn Cys Ala Tyr His Lys His Gln Leu Val Trp Cys Thr Tyr
1               5                   10                  15


His




<210>  47
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  47

Arg Gly Asn Cys Ala Tyr His Lys Trp Gln Leu Val Trp Cys Thr Tyr
1               5                   10                  15


His




<210>  48
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  48

Arg Gly Asn Cys Ala Tyr His Lys Tyr Gln Leu Val Trp Cys Thr Tyr

1               5                   10                  15

His

<210>  49
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  49

Arg Gly Asn Cys Ala Tyr Phe Lys Gly Gln Leu Val Trp Cys Thr Tyr
1               5                   10                  15

His

<210>  50
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  50

Arg Gly Asn Cys Ala Tyr Tyr Lys Gly Gln Leu Val Trp Cys Thr Tyr
1               5                   10                  15

His

<210>  51
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  51

Arg Gly Asn Cys Ala Tyr Trp Lys Gly Gln Leu Val Trp Cys Thr Tyr

1               5                   10                  15

His

<210>  52
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  52

Arg Gly Asn Cys Ala Tyr Arg Lys Gly Gln Leu Val Trp Cys Thr Tyr
1               5                   10                  15

His

<210>  53
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  53

Arg Gly Asn Cys Ala Tyr Gly Lys Gly Gln Leu Val Trp Cys Thr Tyr
1               5                   10                  15

His

<210>  54
<211>  12
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  54

Asp Cys Ala Tyr His Lys Gly Gln Leu Val Trp Cys

1               5                   10


<210>  55
<211>  12
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  55

Asn Cys Ala Tyr His Lys Gly Gln Leu Val Trp Cys
1               5                   10


<210>  56
<211>  12
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  56

Cys Ala Tyr His Lys Gly Gln Leu Val Trp Cys Thr
1               5                   10


<210>  57
<211>  11
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  57

Cys Ala Tyr His Lys Ser Gln Leu Val Trp Cys
1               5                   10


<210>  58
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues

may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE

<222>  (17)..(17)
<223>  absent, or histidine

<400>  58

Xaa Xaa Xaa Cys Xaa Xaa Xaa Xaa Xaa Xaa Ile Ile Trp Cys Xaa Xaa
1               5                   10                  15

Xaa

<210>  59
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE

<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  59

Xaa Xaa Xaa Cys Xaa Xaa Xaa Xaa Xaa Xaa Ile Val Trp Cys Xaa Xaa
1               5                   10                  15


Xaa




<210>  60
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>

<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  60

Xaa Xaa Xaa Cys Xaa Xaa Xaa Xaa Xaa Xaa Val Val Trp Cys Xaa Xaa
1               5                   10                  15

Xaa

<210>  61
<211>  17
<212>  PRT
<213>  Artificial Sequence

<220>

<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  61

Xaa Xaa Xaa Cys Xaa Xaa Xaa Xaa Xaa Xaa Ala Val Trp Cys Xaa Xaa
1               5                   10                  15

Xaa

<210>  62
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
      glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
      acide, phenylalanine, tyrosine, tryptophan, histidine, threonine,
      leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  62

Xaa Xaa Xaa Cys Xaa Xaa Xaa Xaa Xaa Xaa Leu Leu Trp Cys Xaa Xaa
1               5                   10                  15


Xaa




<210>  63
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)

<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  63

Xaa Xaa Xaa Cys Xaa Xaa Xaa Xaa Xaa Xaa Leu Ile Trp Cys Xaa Xaa
1               5                   10                  15


Xaa




<210>  64
<211>  17
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>

<221>  MISC_FEATURE
<222>  (16)..(16)
<223>  absent, or tyrosine

<220>
<221>  MISC_FEATURE
<222>  (17)..(17)
<223>  absent, or histidine

<400>  64

Xaa Xaa Xaa Cys Xaa Xaa Xaa Xaa Xaa Xaa Leu Val Phe Cys Xaa Xaa
1               5                   10                  15

Xaa

<210>  65
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>

<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  65

Xaa Xaa Xaa Cys Xaa Xaa Xaa Xaa Xaa Xaa Gln Val Trp Cys Xaa Xaa
1               5                   10                  15


Xaa




<210>  66
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)
<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  66

Xaa Xaa Xaa Cys Xaa Xaa Xaa Xaa Xaa Xaa Glu Val Trp Cys Xaa Xaa
1               5                   10                  15


Xaa

<210>  67
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.


<220>
<221>  MISC_FEATURE
<222>  (1)..(1)
<223>  absent, or arginine

<220>
<221>  MISC_FEATURE
<222>  (2)..(2)
<223>  absent, or glycine

<220>
<221>  MISC_FEATURE
<222>  (3)..(3)
<223>  absent, aspartic acid, or asparagine

<220>
<221>  MISC_FEATURE
<222>  (5)..(5)
<223>  alanine

<220>
<221>  MISC_FEATURE
<222>  (6)..(6)
<223>  tyrosine, tryptophan, or histidine

<220>
<221>  MISC_FEATURE
<222>  (7)..(7)
<223>  histidine, phenylalanine, tyrosine, tryptophan, arginine, or
       glycine

<220>
<221>  MISC_FEATURE
<222>  (8)..(8)
<223>  lysine

<220>
<221>  MISC_FEATURE
<222>  (9)..(9)
<223>  glycine, serine, asparagine, glutamine, aspartic acid, glutamic
       acide, phenylalanine, tyrosine, tryptophan, histidine, threonine,
       leucine, alanine, valine, isoleucine, or arginine

<220>
<221>  MISC_FEATURE
<222>  (10)..(10)
<223>  glutamine, glutamic acid, asparagine, or aspartic acid

<220>
<221>  MISC_FEATURE
<222>  (15)..(15)

<223>  absent, or threonine

<220>
<221>  MISC_FEATURE
<222>  (16)..(16)
<223>  absent, or tyrosine

<220>
<221>  MISC_FEATURE
<222>  (17)..(17)
<223>  absent, or histidine

<400>  67

Xaa Xaa Xaa Cys Xaa Xaa Xaa Xaa Xaa Xaa Leu Val Trp Cys Xaa Xaa
1               5                   10                  15

Xaa

<210>  68
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  68

Arg Gly Asn Cys Ala Trp His Lys Gly Gln Ile Ile Trp Cys Thr Tyr
1               5                   10                  15

His

<210>  69
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  69

Arg Gly Asn Cys Ala Phe His Lys Gly Gln Ile Ile Trp Cys Thr Tyr
1               5                   10                  15

His

<210>  70
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  70

Arg Gly Asn Cys Ala His His Lys Gly Gln Ile Ile Trp Cys Thr Tyr
1               5                   10                  15

His

<210>  71
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  71

Arg Gly Asn Cys Gly Tyr His Lys Gly Gln Ile Ile Trp Cys Thr Tyr
1               5                   10                  15

His

<210>  72
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  72

Arg Gly Asn Cys Leu Tyr His Lys Gly Gln Ile Ile Trp Cys Thr Tyr
1               5                   10                  15

His

<210>  73
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  73

Arg Gly Asn Cys Pro Tyr His Lys Gly Gln Ile Ile Trp Cys Thr Tyr
1               5                   10                  15

His

<210>  74
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  74

Arg Gly Asn Cys Arg Tyr His Lys Gly Gln Ile Ile Trp Cys Thr Tyr
1               5                   10                  15

His

<210>  75
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  75

Arg Gly Asn Cys Val Tyr His Lys Gly Gln Ile Ile Trp Cys Thr Tyr
1               5                   10                  15

His

<210>  76
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  76

Arg Gly Asn Cys Asn Tyr His Lys Gly Gln Ile Ile Trp Cys Thr Tyr
1               5                   10                  15

His

<210>  77
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  77

Arg Gly Asn Cys Glu Tyr His Lys Gly Gln Ile Ile Trp Cys Thr Tyr
1               5                   10                  15

His

<210>  78
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  78

Arg Gly Asn Cys Phe Tyr His Lys Gly Gln Ile Ile Trp Cys Thr Tyr
1               5                   10                  15

His

<210>  79
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  79

Arg Gly Asn Cys Ala Tyr His Lys Gly Glu Ile Ile Trp Cys Thr Tyr
1               5                   10                  15

His

<210>  80
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  80

Arg Gly Asn Cys Ala Tyr His Lys Gly Asn Ile Ile Trp Cys Thr Tyr
1               5                   10                  15

His

<210>  81
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  81

Arg Gly Asn Cys Ala Tyr His Lys Gly Pro Ile Ile Trp Cys Thr Tyr
1               5                   10                  15

His

<210>  82
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  82

Arg Gly Asn Cys Ala Tyr His Lys Gly Gly Ile Ile Trp Cys Thr Tyr
1               5                   10                  15

His

<210>  83
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  83

Arg Gly Asn Cys Ala Tyr His Lys Gly Asp Ile Ile Trp Cys Thr Tyr
1               5                   10                  15

His

<210>  84
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  84

Arg Gly Asn Cys Ala Tyr His Lys Gly Arg Ile Ile Trp Cys Thr Tyr
1               5                   10                  15

His

<210>  85
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  85

Arg Gly Asn Cys Ala Tyr His Lys Gly Phe Ile Ile Trp Cys Thr Tyr
1               5                   10                  15

His

<210>  86
<211>  17
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  86

Arg Gly Asn Cys Ala Tyr His Lys Gly His Ile Ile Trp Cys Thr Tyr
1               5                   10                  15

His

<210>  87
<211>  13
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  87

Asp Cys Ala Tyr His Lys Gly Gln Ile Ile Trp Cys Thr
1               5                   10

<210>  88
<211>  13
<212>  PRT

<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  88

Asn Cys Ala Tyr His Lys Gly Gln Ile Ile Trp Cys Thr
1               5                   10


<210>  89
<211>  15
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  89

Gly Asn Cys Ala Tyr His Lys Gly Gln Ile Ile Trp Cys Thr Tyr
1               5                   10                  15


<210>  90
<211>  13
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  90

Gly Cys Ala Tyr His Lys Gly Gln Ile Ile Trp Cys Gly
1               5                   10


<210>  91
<211>  15
<212>  PRT
<213>  Artificial Sequence

<220>
<223>  Peptide having an affinity to Fc region of human IgG.  The amino
       acid residue at N-terminus may be acetylated, the amino acid
       residue at C-terminus may be aminated and two cysteine residues
       may be linked by disulfide bridge.

<400>  91

Gly Gly Cys Ala Tyr His Lys Gly Gln Ile Ile Trp Cys Gly Gly
1               5                   10                  15

<210>  92
<211>  17
<212>  PRT
<213>  Artificial Sequence

<220>
<223>  Peptide having an affinity to Fc region of human IgG.  The amino
      acid residue at N-terminus may be acetylated, the amino acid
      residue at C-terminus may be aminated and two cysteine residues
      may be linked by disulfide bridge.

<400>  92

Gly Gly Gly Cys Ala Tyr His Lys Gly Gln Ile Ile Trp Cys Gly Gly
1               5                   10                  15

Gly

<210>  93
<211>  19
<212>  RNA
<213>  Artificial Sequence

<220>
<223>  Nucleotide sequence of sense strand of siRNA

<400>  93
acagcaaauu ccaucgugu                                                    19

<210>  94
<211>  21
<212>  RNA
<213>  Artificial Sequence

<220>
<223>  Nucleotide sequence of antisense strand of siRNA

<400>  94
acacgaugga auuugcuguu u                                                 21

## Claims

1. A compound having an affinity substance to an antibody, a cleavable portion, and a reactive group, or a salt thereof, the compound being represented by the following formula (I):
A-L-B-R (I)
wherein
A is the affinity substance an antibody,
L is a cleavable linker which is a divalent group comprising the cleavable portion,
B is (a) a divalent group comprising a bioorthogonal functional group or (b) a divalent group comprising no bioorthogonal functional group, and
R is the reactive group to the antibody, wherein
the affinity substance to an antibody is a peptide comprising any of the following amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58),
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59),
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60),
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61),
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62),
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63),
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64),
Formula 1-8 (X₀₋₃)ₐ-C-Xaa1-Xaa2-Xaa3-Xaa4-Xaa5-Xaa6-Q-V-W-C- (X₀₋₃)_{b} (SEQ ID NO: 65),
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive any amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.

2. The compound or salt thereof according to claim 1, wherein
(X₀₋₃)ₐ is absent, an arginine residue-glycine residue-asparagine residue, an aspartic acid residue, or an asparagine residue,
(X₀₋₃)_{b} is absent, a threonine residue-tyrosine residue-histidine residue, or a threonine residue,
Xaa1 is an alanine residue,
Xaa2 is a tyrosine residue, a tryptophan residue, or a histidine residue, and
Xaa6 is a glutamine residue, a glutamic acid residue, an asparagine residue, or an aspartic acid residue.

3. The compound or salt thereof according to claim 1, wherein
(X₀₋₃)ₐ is a glycine residue-asparagine residue, a glycine residue, a glycine residue-glycine residue, or a glycine residue-glycine residue-glycine residue,
(X₀₋₃)_{b} is a threonine residue-tyrosine residue, a glycine residue, a glycine residue-glycine residue, or a glycine residue-glycine residue-glycine residue,
Xaa1 is a glycine residue, a leucine residue, a proline residue, an arginine residue, a valine residue, an asparagine residue, a glutamic acid residue, or a phenylalanine residue,
Xaa2 is a phenylalanine residue, and
Xaa6 is a proline residue, a glycine residue, an arginine residue, a phenylalanine residue, or a histidine residue.

4. The compound or salt thereof according to any one of claims 1 to 3, wherein the affinity substance to an antibody is a peptide comprising an amino acid sequence selected from the group consisting of the following:
(1) RGNCAYHKGQIIWCTYH (SEQ ID NO: 5);
(2)RGNCAYHKGQIVWCTYH(SEQ ID NO: 8);
(3) RGNCAYHKGQVVWCTYH (SEQ ID NO: 9);
(4)RGNCAYHKGQAVWCTYH(SEQ ID NO: 10);
(5)RGNCAYHKGQLLWCTYH(SEQ ID NO: 11);
(6)RGNCAYHKGQLIWCTYH(SEQ ID NO: 12);
(7) DCAYHKGQIVWCT (SEQ ID NO: 13);
(8) DCAYHKGQWWCT (SEQ ID NO: 14);
(9) DCAYHKGQAVWCT (SEQ ID NO: 15);
(10)RGNCAYHKSQIIWCTYH(SEQ ID NO: 16);
(11) RGNCAYHKNQIIWCTYH (SEQ ID NO: 17);
(12)RGNCAYHKDQIIWCTYH(SEQ ID NO: 18);
(13)RGNCAYHKQQIIWCTYH(SEQ ID NO: 19);
(14)RGNCAYHKEQIIWCTYH(SEQ ID NO: 20);
(15)RGNCAYHKFQIIWCTYH(SEQ ID NO: 21);
(16)RGNCAYHKYQIIWCTYH(SEQ ID NO: 22);
(17)RGNCAYHKWQIIWCTYH(SEQ ID NO: 23);
(18)RGNCAYHKHQIIWCTYH(SEQ ID NO: 24);
(19)RGNCAYHKTQIIWCTYH(SEQ ID NO: 25);
(20)RGNCAYHKLQIIWCTYH(SEQ ID NO: 26);
(21)CAYHKLQIVWC(SEQ ID NO: 27);
(22)CAYHKLQLIWC(SEQ ID NO: 28);
(23)CAYHKSQIVWC(SEQ ID NO: 29);
(24)RGNCAYHKGQLVFCTYH(SEQ ID NO: 30);
(25)RGNCAYHKGQQVWCTYH(SEQ ID NO: 31);
(26)RGNCAYHKGQEVWCTYH(SEQ ID NO: 32);
(27)CAYHKGQLVWC(SEQ ID NO: 33);
(28)RGNCAYHKAQLVWCTYH(SEQ ID NO: 34);
(29)RGNCAYHKVQLVWCTYH(SEQ ID NO: 35);
(30)RGNCAYHKLQLVWCTYH(SEQ ID NO: 36);
(31)RGNCAYHKIQLVWCTYH(SEQ ID NO: 37);
(32)RGNCAYHKSQLVWCTYH(SEQ ID NO: 38);
(33)RGNCAYHKTQLVWCTYH(SEQ ID NO: 39);
(34)RGNCAYHKNQLVWCTYH(SEQ ID NO: 40);
(35)RGNCAYHKDQLVWCTYH(SEQ ID NO: 41);
(36)RGNCAYHKQQLVWCTYH(SEQ ID NO: 42);
(37)RGNCAYHKEQLVWCTYH(SEQ ID NO: 43);
(38)RGNCAYHKFQLVWCTYH(SEQ ID NO: 44);
(39)RGNCAYHKRQLVWCTYH(SEQ ID NO: 45);
(40)RGNCAYHKHQLVWCTYH(SEQ ID NO: 46);
(41)RGNCAYHKWQLVWCTYH(SEQ ID NO: 47);
(42)RGNCAYHKYQLVWCTYH(SEQ ID NO: 48);
(43)RGNCAYFKGQLVWCTYH(SEQ ID NO: 49);
(44)RGNCAYYKGQLVWCTYH(SEQ ID NO: 50);
(45)RGNCAYWKGQLVWCTYH(SEQ ID NO: 51);
(46)RGNCAYRKGQLVWCTYH(SEQ ID NO: 52);
(47)RGNCAYGKGQLVWCTYH(SEQ ID NO: 53);
(48)DCAYHKGQLVWC(SEQ ID NO: 54);
(49)NCAYHKGQLVWC(SEQ ID NO: 55);
(50)CAYHKGQLVWCT(SEQ ID NO: 56);
(51)CAYHKSQLVWC(SEQ ID NO: 57);
(52)RGNCAWHKGQIIWCTYH(SEQ ID NO: 68);
(53)RGNCAFHKGQIIWCTYH(SEQ ID NO: 69);
(54)RGNCAHHKGQIIWCTYH(SEQ ID NO: 70);
(55)RGNCGYHKGQIIWCTYH(SEQ ID NO: 71);
(56)RGNCLYHKGQIIWCTYH(SEQ ID NO: 72);
(57)RGNCPYHKGQIIWCTYH(SEQ ID NO: 73);
(58)RGNCRYHKGQIIWCTYH(SEQ ID NO: 74);
(59)RGNCVYHKGQIIWCTYH(SEQ ID NO: 75);
(60)RGNCNYHKGQIIWCTYH(SEQ ID NO: 76);
(61)RGNCEYHKGQIIWCTYH(SEQ ID NO: 77);
(62)RGNCFYHKGQIIWCTYH(SEQ ID NO: 78);
(63)RGNCAYHKGEIIWCTYH(SEQ ID NO: 79);
(64)RGNCAYHKGNIIWCTYH(SEQ ID NO: 80);
(65)RGNCAYHKGPIIWCTYH(SEQ ID NO: 81);
(66)RGNCAYHKGGIIWCTYH(SEQ ID NO: 82);
(67)RGNCAYHKGDIIWCTYH(SEQ ID NO: 83);
(68)RGNCAYHKGRIIWCTYH(SEQ ID NO: 84);
(69)RGNCAYHKGFIIWCTYH(SEQ ID NO: 85);
(70)RGNCAYHKGHIIWCTYH(SEQ ID NO: 86);
(71)DCAYHKGQIIWCT(SEQ ID NO: 87);
(72)NCAYHKGQIIWCT(SEQ ID NO: 88);
(73)GNCAYHKGQIIWCTY(SEQ ID NO: 89);
(74)GCAYHKGQIIWCG(SEQ ID NO: 90);
(75)GGCAYHKGQIIWCGG(SEQ ID NO: 91); and
(76) GGGCAYHKGQIIWCGGG (SEQ ID NO: 92).

5. The compound or salt thereof according to any one of claims 1 to 4, wherein L is (i) a cleavable linker which is a divalent group comprising a cleavable portion having an ability to form a bioorthogonal functional group on a reactive group side by cleavage or (ii) a cleavable linker which is a divalent group comprising a cleavable portion having no ability to form a bioorthogonal functional group on a reactive group side by cleavage.

6. The compound or salt thereof according to any one of claims 1 to 5, wherein the cleavable portion is a portion cleavable by any of (a) treatment with one or more substances selected from the group consisting of an acidic substance, a basic substance, a reducing agent, an oxidizing agent, and an enzyme, (b) treatment by physicochemical stimulus selected from the group consisting of light, and (c) being left when a cleavable linker comprising a self-decomposing cleavable portion is used.

7. The compound or salt thereof according to any one of claims 1 to 6, wherein the cleavable portion is selected from the group consisting of a disulfide residue, an acetal residue, a ketal residue, an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.

8. The compound or salt thereof according to any one of claims 5 to 7, wherein the cleavable portion of (i) is selected from the group consisting of a disulfide residue, an ester residue, an acetal residue, a ketal residue, an imine residue, and a vicinal diol residue.

9. The compound or salt thereof according to any one of claims 5 to 7, wherein the cleavable portion of (ii) is selected from the group consisting of an ester residue, a carbamoyl residue, an alkoxyalkyl residue, an imine residue, a tertiary alkyloxy carbamate residue, a silane residue, a hydrazone-containing residue, a phosphoramidate residue, an aconityl residue, a trityl residue, an azo residue, a vicinal diol residue, a selenium residue, an aromatic ring-containing residue having an electron-withdrawing group, a coumarin-containing residue, a sulfone-containing residue, an unsaturated bond-containing chain residue, and a glycosyl residue.

10. The compound or salt thereof according to any one of claims 1 to 9, wherein the bioorthogonal functional group is selected from the group consisting of an azide residue, an aldehyde residue, a thiol residue, an alkyne residue, an alkene residue, a halogen residue, a tetrazine residue, a nitron residue, a hydroxylamine residue, a nitrile residue, a hydrazine residue, a ketone residue, a boronic acid residue, a cyanobenzothiazole residue, an allyl residue, a phosphine residue, a maleimide residue, a disulfide residue, a thioester residue, an α-halocarbonyl residue, an isonitrile residue, a sydnone residue, and a selenium residue.

11. The compound or salt thereof according to any one of claims 1 to 10, wherein the reactive group is a reactive group specific to any one side chain of a lysine residue, a tyrosine residue, and a tryptophan residue.

12. The compound or salt thereof according to claim 11, wherein the reactive group is a reactive group specific to a side chain of a lysine residue.

13. The compound or salt thereof according to any one of claims 1 to 12, having one or more characteristics selected from the following:
(a) a main chain linking A and R has 4 to 20 atoms;
(b) the main chain linking A and R comprises no cyclic structure; and
(c) a partial structure represented by L-B comprises no peptide portion.

14. A reagent of regioselectively modifying an antibody, the reagent comprising a compound having an affinity substance to an antibody, a cleavable portion, and a reactive group, represented by the following Formula (I):
A-L-B-R (I)
wherein
A is the affinity substance to an antibody,
L is a cleavable linker which is a divalent group comprising the cleavable portion,
B is (a) a divalent group comprising a bioorthogonal functional group or (b) a divalent group comprising no bioorthogonal functional group, and
R is the reactive group to the antibody, or
a salt thereof, wherein
the affinity substance to an antibody is a peptide comprising any of the amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive arbitrary amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.

15. An antibody having an affinity substance to the antibody and a cleavable portion, or a salt thereof, represented by the following Formula (II):
A-L-B-R'-T (II)
wherein
A is the affinity substance to the antibody,
L is a cleavable linker which is a divalent group comprising the cleavable portion,
B is (a) a divalent group comprising a bioorthogonal functional group or (b) a divalent group comprising no bioorthogonal functional group,
R' is a portion formed by a reaction between the antibody and a reactive group, and
T is the antibody, wherein
the affinity substance to an antibody is a peptide comprising any of the amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive arbitrary amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.

16. The antibody or salt thereof according to claim 15, wherein the antibody is a monoclonal antibody.

17. The antibody or salt thereof according to claim 15 or 16, wherein the antibody is an IgG antibody.

18. The antibody or salt thereof according to any one of claims 15 to 17, wherein the antibody is derived from a human.

19. The antibody or salt thereof according to any one of claims 15 to 18, wherein
the antibody comprises any one Fc region protein selected from the group consisting of the following (A) to (C) and has antigen-binding ability:
(A) an Fc region protein comprising the amino acid sequence of SEQ ID NO: 1;
(B) an Fc region protein comprising an amino acid sequence with one or several amino acid residues inserted, added, deleted, or substituted in the amino acid sequence of SEQ ID NO: 1; and
(C) an Fc region protein comprising an amino acid sequence having 90% or more identity to the amino acid sequence of SEQ ID NO: 1.

20. The antibody or salt thereof according to any one of claims 15 to 19, wherein
the antibody comprises one or more specific amino acid residues in a target region consisting of 1 to 50 consecutive amino acid residues, and five or more of the specific amino acid residues in a non-target region other than the target region, and
a structural unit represented by A-L-B-R' binds to the one or more of the specific amino acid residues contained in the target region with 30% or more regioselectivity.

21. The antibody or salt thereof according to claim 20, wherein the target region is a region consisting of one to ten consecutive amino acid residues.

22. The antibody or salt thereof according to claim 21, wherein the target region is a region consisting of one to three consecutive amino acid residues.

23. The antibody or salt thereof according to claim 22, wherein the target region is a region consisting of amino acid residues at positions 246 to 248 in a human IgG Fc region.

24. The antibody or salt thereof according to any one of claims 20 to 23, wherein the regioselectivity is 50% or more.

25. The antibody or salt thereof according to claim 24, wherein the regioselectivity is 70% or more.

26. The antibody or salt thereof according to claim 25, wherein the regioselectivity is 90% or more.

27. The antibody or salt thereof according to any one of claims 15 to 26, wherein the portion formed by a reaction between the antibody and a reactive group is a portion formed by a reaction of a reactive group specific to any one side chain of a lysine residue, a tyrosine residue, and a tryptophan residue to a lysine residue, a tyrosine residue, or a tryptophan residue.

28. The antibody or salt thereof according to any one of claims 15 to 27, wherein the portion formed by a reaction between the antibody and a reactive group is a portion formed by a reaction between a lysine residue and a reactive group specific to a side chain of the lysine residue.

29. A method for producing an antibody having a bioorthogonal functional group or bioorthogonal functional groups, or a salt thereof, the method comprising
cleaving a cleavable portion of an antibody having an affinity substance to the antibody and a cleavable portion, represented by the following Formula (II):
A-L-B-R'-T (II)
wherein
A is the affinity substance to the antibody,
L is a cleavable linker which is a divalent group comprising the cleavable portion,
B is (a) a divalent group comprising a bioorthogonal functional group or (b) a divalent group comprising no bioorthogonal functional group,
R' is a portion formed by a reaction between the antibody and a reactive group, and
T is the antibody, or a salt thereof to form an antibody having a bioorthogonal functional group or bioorthogonal functional groups, represented by the following Formula (IV):
L1-B-R'-T (IV)
wherein
B, R', and T are the same as those of the above Formula (II), and
L1 is (i') a monovalent group comprising a bioorthogonal functional group or (ii') a monovalent group comprising no bioorthogonal functional group, or a salt thereof, wherein
the affinity substance to the antibody is a peptide comprising any of the amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive arbitrary amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.

30. A method for producing an antibody having a functional substance or functional substances, or a salt thereof, the method comprising:
cleaving a cleavable portion of a conjugate having an affinity substance to an antibody, a cleavable portion, a functional substance, and an antibody, represented by the following Formula (III):
A-L-B' (-F)-R'-T (III)
wherein
A is the affinity substance to an antibody,
L is a cleavable linker which is a divalent group comprising the cleavable portion,
B' is a trivalent group comprising a portion formed by a reaction between the functional substance and a bioorthogonal functional group,
F is the functional substance,
R' is a portion formed by a reaction between the antibody and a reactive group, and
T is the antibody, or a salt thereof to form an antibody having a functional substance or functional substances, represented by the following Formula (V1):
L1-B'(-F)-R'-T (V1)
wherein
L1 is (i') a monovalent group comprising a bioorthogonal functional group or (ii') a monovalent group comprising no bioorthogonal functional group, and
B', F, R', and T are the same as those of the above Formula (III), or a salt thereof, wherein
the affinity substance to an antibody is a peptide comprising any of the amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive arbitrary amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.

31. A method for producing an antibody having a functional substance or functional substances, or a salt thereof, the method comprising:
(A) cleaving a cleavable portion of an antibody having an affinity substance to the antibody and a cleavable portion, represented by the following Formula (II):
A-L-B-R'-T (II)
wherein
A is the affinity substance to the antibody,
L is a cleavable linker which is a divalent group comprising the cleavable portion,
B is (a) a divalent group comprising a bioorthogonal functional group or (b) a divalent group comprising no bioorthogonal functional group, and
R' is a portion formed by a reaction between the antibody and a reactive group, and
T is the antibody, or a salt thereof to form an antibody having a bioorthogonal functional group or bioorthogonal functional groups, represented by the following Formula (IV):
L1-B-R'-T (IV)
wherein
B, R', and T are the same as those of the above Formula (II), and
L1 is (i') a monovalent group comprising a bioorthogonal functional group or (ii') a monovalent group comprising no bioorthogonal functional group, or a salt thereof; and
(B) reacting the antibody having a bioorthogonal functional group or bioorthogonal functional groups, or salt thereof with one or more functional substances to form an antibody having a functional substance or functional substances, represented by the following Formula (V2):
F-L1'-B-R'-T (V2)
wherein
B, R', and T are the same as those of the above Formula (IV),
L1' is a divalent group comprising a portion formed by a reaction between the functional substance and (i') the monovalent group comprising a bioorthogonal functional group, and
F is the functional substance, or the following Formula (V3): Fa-L1'-B'(-Fb)-R'-T (V3)
wherein
R' and T are the same as those of the above Formula (IV),
L1' is the same as that of the above Formula (V2),
B' is a trivalent group comprising a portion formed by a reaction between the functional substance and a bioorthogonal functional group, and
Fa and Fb are functional substances which are the same as or different from each other, or a salt thereof, wherein
the affinity substance to the antibody is a peptide comprising any of the amino acid sequences of
Formula 1-1: (X₀₋₃)ₐ-C-Xaa1-Xaa2-Xaa3-Xaa4-Xaa5-Xaa6-I-I-W-C- (X₀₋₃)_{b} (SEQ ID NO: 58)
Formula 1-2: (X₀₋₃)ₐ-C-Xaa1-Xaa2-Xaa3-Xaa4-Xaa5-Xaa6-I-V-W-C- (X₀₋₃)_{b} (SEQ ID NO: 59)
Formula 1-3: (X₀₋₃)ₐ-C-Xaa1-Xaa2-Xaa3-Xaa4-Xaa5-Xaa6-V-V-W-C- (X₀₋₃)_{b} (SEQ ID NO: 60)
Formula 1-4: (X₀₋₃)ₐ-C-Xaa1-Xaa2-Xaa3-Xaa4-Xaa5-Xaa6-A-V-W-C- (X₀₋₃)_{b} (SEQ ID NO: 61)
Formula 1-5: (X₀₋₃)ₐ-C-Xaa1-Xaa2-Xaa3-Xaa4-Xaa5-Xaa6-L-L-W-C- (X₀₋₃)_{b} (SEQ ID NO: 62)
Formula 1-6: (X₀₋₃)ₐ-C-Xaa1-Xaa2-Xaa3-Xaa4-Xaa5-Xaa6-L-I-W-C- (X₀₋₃)_{b} (SEQ ID NO: 63)
Formula 1-7: (X₀₋₃)ₐ-C-Xaa1-Xaa2-Xaa3-Xaa4-Xaa5-Xaa6-L-V-F-C- (X₀₋₃)_{b} (SEQ ID NO: 64)
Formula 1-8: (X₀₋₃)ₐ-C-Xaa1-Xaa2-Xaa3-Xaa4-Xaa5-Xaa6-Q-V-W-C- (X₀₋₃)_{b} (SEQ ID NO: 65)
Formula 1-9: (X₀₋₃)ₐ-C-Xaa1-Xaa2-Xaa3-Xaa4-Xaa5-Xaa6-E-V-W-C- (X₀₋₃)_{b} (SEQ ID NO: 66)
wherein
(X₀₋₃)ₐ is absent or one to three consecutive arbitrary amino acid residues (other than a lysine residue and a cysteine residue) which are the same or different,
(X₀₋₃)_{b} is absent or one to three consecutive arbitrary amino acid residues (other than a lysine residue and a cysteine residue) which are the same or different,
Xaa1 is an alanine residue, a glycine residue, a leucine residue, a proline residue, an arginine residue, a valine residue, an asparagine residue, a glutamic acid residue, or a phenylalanine residue,
Xaa2 is a tyrosine residue, a tryptophan residue, a histidine residue, or a phenylalanine residue,
Xaa3 is a histidine residue, a phenylalanine residue, a tyrosine residue, a tryptophan residue, an arginine residue, or a glycine residue,
Xaa4 is a lysine residue,
Xaa5 is a glycine residue, a serine residue, an asparagine residue, a glutamine residue, an aspartic acid residue, a glutamic acid residue, a phenylalanine residue, a tyrosine residue, a tryptophan residue, a histidine residue, a threonine residue, a leucine residue, an alanine residue, a valine residue, an isoleucine residue, or an arginine residue, and
Xaa6 is a glutamine residue, a glutamic acid residue, an asparagine residue, an aspartic acid residue, a proline residue, a glycine residue, an arginine residue, a phenylalanine residue, or a histidine residue, and
Formula 2-1: (X₀₋₃')ₐ-C-Xaa1' -Xaa2' -Xaa3' -Xaa4 '-Xaa5'-Xaa6'-L-V-W-C-(X₀₋₃')_{b} (SEQ ID NO: 67)
wherein
(X₀₋₃')ₐ and (X₀₋₃')_{b} are the same as the above (X₀₋₃)ₐ and (X₀₋₃)_{b}, respectively, and
Xaa1', Xaa2', Xaa3', Xaa4', Xaa5', and Xaa6' are the same as the above Xaa1, Xaa2, Xaa3, Xaa4, Xaa5, and Xaa6, respectively,
except for a case where (X₀₋₃')ₐ is one to three consecutive arbitrary amino acid residues which are the same or different, (X₀₋₃')_{b} is one to three consecutive arbitrary amino acid residues which are the same or different, Xaa3' is a histidine residue, and Xaa5' is a glycine residue.
